# EUROPEAN PATENT APPLICATION

(11) **EP 3 010 065 A1**
(43) Date of publication of application: **20.04.2016**
(21) Application number: 15190326.7
(22) Date of filing: 19.10.2015
(51) Int. Cl.: H01L 51/54

(54) **ORGANIC LIGHT-EMITTING DEVICE**

(30) Priority: 17.10.2014 KR 20140141200
(71) Applicant: Samsung Display Co., Ltd., Gyeonggi-do (KR)
(72) Inventor: ITO, Naoyuki, Yongin-si, Gyeonggi-do (KR); KIM, Myeong-Suk, Yongin-si, Gyeonggi-do (KR); KIM, Youn-Sun, Yongin-si, Gyeonggi-do (KR); KIM, Sung-Wook, Yongin-si, Gyeonggi-do (KR); CHO, Hwan-Hee, Yongin-si, Gyeonggi-do (KR); CHU, Chang-Woong, Yongin-si, Gyeonggi-do (KR)
(74) Representative: Gulde & Partner

(57) **Abstract**

An organic light-emitting device having high efficiency and long lifespan and showing little change in the efficiency at an x-coordinate (CIEx) value of 0.21 is disclosed.

## Description

The present invention relates to an organic light-emitting device.

### BACKGROUND

Organic light-emitting devices are self-emitting devices that have wide viewing angles, high contrast, quick response times, high brightness, low driving voltage characteristics, and can provide multicolored images.

For example, an organic light-emitting device may include a first electrode disposed on a substrate, and may have a structure in which a hole transport region, an emission layer, an electron transport region, and a second electrode in which that are sequentially stacked on the first electrode. Holes injected from the first electrode move to an emission layer via the hole transport region while electrons injected from the second electrode move to an emission layer via the electron transport region. Carriers, e,g., the holes and the electrons, recombine in the emission layer to generate excitons, When the excitons drop from an excited state to a ground state, light is emitted.

### SUMMARY

Provided is an organic light-emitting device as defined in claim 1.

Additional aspects will be set forth in part in the description which follows and, in part, will be apparent from the description, or may be learned by practice of the presented exemplary embodiments.

According to another aspect of an exemplary embodiment, there is provided an organic light-emitting device including: a first electrode, a second electrode, and an organic layer that is disposed between the first electrode and the second electrode and includes an emission layer,
wherein the emission layer may include a Host I and a dopant,
the Host I may be represented by Formula 1, and
the dopant may be represented by Formula 7:

<Formula 11 > Ar₁₁₁⁅(L₁₁₁)ₐ₁₁₁-(R₁₁₁)_{b111}]ₙ₁₁₁

<Formula 7> M(L₁)ₙ₇₁(L₂)ₙ₇₂

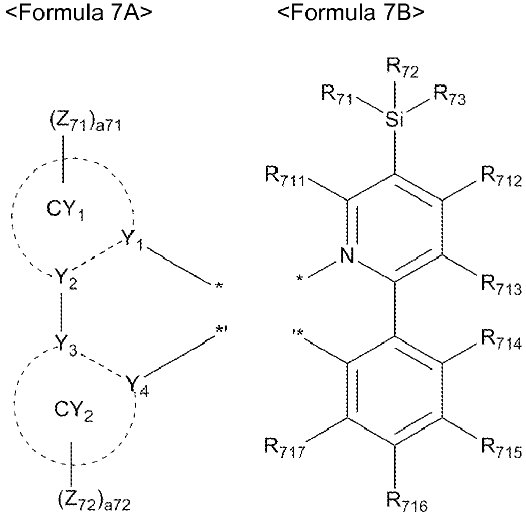

In Formulae above,
Ar₁₁₁ may be selected from a substituted or unsubstituted C₄-C₃₀ pyrrolidine-based core and a substituted or unsubstituted C₇-C₃₀ condensed polycyclic-based core,
L₁₁₁ may be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
a111 may be selected from 0, 1, 2, and 3,
R₁₁₁ may be selected from a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, wherein at least one R₁₁₁ may be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group,
b111 may be selected from 1, 2, and 3,
n 111 may be selected from 1, 2, 3, and 4,
M may be selected from iridium (Ir), platinum (Pt), osmium (Os), titanium (Ti), zirconium (Zr), hafnium (Hf), europium (Eu), terbium (Tb), thulium (Tm), and rhodium (Rd),
L₁ may be a ligand represented by Formula 7A above, and L₂ may be a ligand represented by Formula 7B above, wherein L₁ and L₂ may be different from each other,
n71 and n72 may each be independently 1 or 2, a sum of n71 and n72 (n71+n72) may be 2 or 3, and when n71 is 2 or more, a plurality of L₁s may be identical to or different from each other, and when n72 is 2 or more, a plurality of L₂s may be identical to or different from each other,
Y₁ to Y₄ may each be independently C or N, wherein Y₁ and Y₂ may be linked to each other via a single bond or a double bond, and Y₃ and Y₄ may be linked to each other via a single bond or a double bond,
CY₁ and CY₂ may each be independently selected from a C₅-C₆₀ cyclic group and a C₂-C₆₀ heterocyclic group, wherein CY₁ and CY₂ may be optionally linked to each other via a single bond or a first linking group,
R₇₁ to R₇₃ may each be independently selected from:
a C₁-C₁₀ alkyl group; and
a C₁-C₁₀ alkyl group substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, and a phosphoric acid group or a salt thereof,
Z₇₁, Z₇₂, and R₇₁₁ to R₇₁₇ may each be independently selected from a hydrogen, a deuterium, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂),-Si(Q₃)(Q₄)(Q₅), and -B(Q₆)(Q₇), wherein R₇₁₂ is not a hydrogen, and two adjacent substituents selected from R₇₁₄ to R₇₁₇ may be optionally linked to each other to form a condensed ring,
a71 and a72 may each be independently selected from integers of 1 to 5, wherein when a71 is 2 or more, a plurality of Z₇₁s may be identical to or different from each other, and when a72 is 2 or more, a plurality of Z₇₂s may be identical to or different from each other,
* and *' may each independently indicate a binding site to M of Formula 1, and
at least one substituent of the substituted C₄-C₃₀ pyrrolidine-based core, the substituted C₇-C₃₀ condensed polycyclic-based core, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the subtstituted divalent non-aromatic condensed polycyclic group, the subtstituted divalent non-aromatic condensed heteropolycyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted or unsubstituted monovalent non-aromatic condensed heteropolycyclic group may be selected from:
a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), and -B(Q₁₆)(Q₁₇);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), and -B(Q₂₆)(Q₂₇); and
-N(Q₃₁)(Q₃₂), -Si(Q₃₃)(Q₃₄)(Q₃₅), and -B(Q₃₆)(Q₃₇),
wherein Q₁ to Q₇, Q₁₁ to Q₁₇, Q₂₁ to Q₂₇, and Q₃₁ to Q₃₇ may each be independently selected from a hydrogen, a C₁-C₆₀ alkyl group, a C₁-C₆₀ alkoxy group, a C₆-C₆₀ aryl group, a C₁-C₆₀ heteroaryl group, a monovalent non-aromatic condensed polycyclic group, and a monovalent non-aromatic condensed heteropolycyclic group.

According to another aspect of an exemplary embodiment, there is provided an organic light-emitting device including: a first electrode, a second electrode, and an organic layer that is disposed between the first electrode and the second electrode and includes an emission layer,
wherein the emission layer may include a Host I, a Host II, and a dopant,
the Host I and the Host II may be different from each other,
the Host I and the Host II may each be independently represented by Formula 11, and
the dopant may be represented by Formula 7:

<Formula 11 > Ar₁₁₁⁅(L₁₁₁)ₐ₁₁₁-(R₁₁₁)_{b111}]ₙ₁₁₁

<Formula 7> M(L₁)ₙ₇₁(L₂)ₙ₇₂

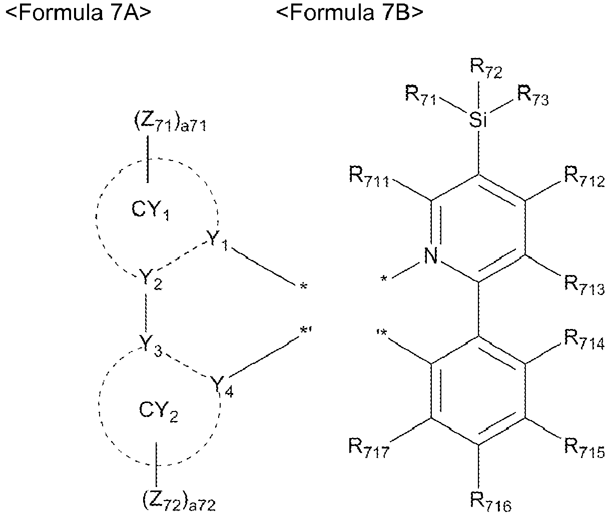

In Formulae above,
Ar₁₁₁ may be selected from a substituted or unsubstituted C₄-C₃₀ pyrrolidine-based core and a substituted or unsubstituted C₇-C₃₀ condensed polycyclic-based core,
L₁₁₁ may be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
a111 may be selected from 0, 1, 2, and 3,
R₁₁₁ may be selected from a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, wherein at least one R₁₁₁ is selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group,
b111 may be selected from 1, 2, and 3,
n 111 may be selected from 1, 2, 3, and 4,
M may be selected from iridium (Ir), platinum (Pt), osmium (Os), titanium (Ti), zirconium (Zr), hafnium (Hf), europium (Eu), terbium (Tb), thulium (Tm), and rhodium (Rd),
L₁ may be a ligand represented by Formula 7A above, and L₂ may be a ligand represented by Formula 7B above, wherein L₁ and L₂ may be different from each other,
n71 and n72 may each be independently 1 or 2, a sum of n71 and n72 (n71+n72) may be 2 or 3, and when n71 is 2 or more, a plurality of L₁s may be identical to or different from each other, and when n72 is 2 or more, a plurality of L₂s may be identical to or different from each other,
Y₁ to Y₄ may each be independently C or N, wherein Y₁ and Y₂ may be linked to each other via a single bond or a double bond, and Y₃ and Y₄ may be linked to each other via a single bond or a double bond,
CY₁ and CY₂ may each be independently selected from a C₅-C₆₀ cyclic group and a C₂-C₆₀ heterocyclic group wherein CY₁ and CY₂ may be optionally linked to each other via a single bond or a first linking group,
R₇₁ to R₇₃ may each be independently selected from:
a C₁-C₁₀ alkyl group; and
a C₁-C₁₀ alkyl group substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, and a phosphoric acid group or a salt thereof,
Z₇₁, Z₇₂, and R₇₁₁ to R₇₁₇ may each be independently selected from a hydrogen, a deuterium, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂),-Si(Q₃)(Q₄)(Q₅), and -B(Q₆)(Q₇), wherein R₇₁₂ is not a hydrogen, and two adjacent substituents selected from R₇₁₄ to R₇₁₇ may be optionally linked to each other to form a condensed ring,
a71 and a72may each be independently selected from integers of 1 to 5, wherein when a71 is 2 or more, a plurality of Z₇₁s may be identical to or different from each other, and when a72 is 2 or more, a plurality of Z₇₂s may be identical to or different from each other,
* and *' may each independently indicate a binding site to M of Formula 1, and
at least one substituent of the substituted C₄-C₃₀ pyrrolidine-based core, the substituted C₇-C₃₀ condensed polycyclic-based core, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the subtstituted divalent non-aromatic condensed polycyclic group, the subtstituted divalent non-aromatic condensed heteropolycyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted or unsubstituted monovalent non-aromatic condensed heteropolycyclic group may be selected from:
a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), and -B(Q₁₆)(Q₁₇);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), and -B(Q₂₆)(Q₂₇); and
-N(Q₃₁)(Q₃₂), -Si(Q₃₃)(Q₃₄)(Q₃₅), and -B(Q₃₆)(Q₃₇),
wherein Q₁ to Q₇, Q₁₁ to Q₁₇, Q₂₁ to Q₂₇, and Q₃₁ to Q₃₇ may each be independently selected from a hydrogen, a C₁-C₆₀ alkyl group, a C₁-C₆₀ alkoxy group, a C₆-C₆₀ aryl group, a C₁-C₆₀ heteroaryl group, a monovalent non-aromatic condensed polycyclic group, and a monovalent non-aromatic condensed heteropolycyclic group.

### BRIEF DESCRIPTION OF THE DRAWINGS

These and/or other aspects will become apparent and more readily appreciated from the following description of the exemplary embodiments, taken in conjunction with the accompanying drawings in which:
FIG. 1 is a schematic view of a structure of an organic light-emitting device according to an exemplary embodiment;
FIG. 2 is a diagram showing a photoluminescence (PL) spectrum of a dopant solution according to an exemplary embodiment; and
FIG. 3 is a graph plotting color coordinates associated with efficiency according to an exemplary embodiment.

### DETAILED DESCRIPTION

Reference will now be made in detail to exemplary embodiments, examples of which are illustrated in the accompanying drawings, wherein like reference numerals refer to like elements throughout.

It will be understood that when a layer, region, or component is referred to as being "formed on" another layer, region, or component, it can be directly or indirectly on or formed on the other layer, region, or component. That is, for example, intervening layers, regions, or components may be present.

Sizes of components in the accompanying drawing may be exaggerated for convenience of explanation. In other words, since sizes and thicknesses of components in the accompanying drawing may be arbitrarily illustrated for convenience of explanation, the following embodiments are not limited thereto.

As used herein, the expression "(an organic layer) includes at least one first host" may be construed as meaning "(an organic layer) may include one first host or at least two different first hosts in a range of Formula 1".

As used herein, the term "organic layer" refers to a single layer and/or a plurality of layers disposed between a first electrode and a second electrode of an organic light-emitting device. A material included in the "organic layer" is not limited to an organic material.

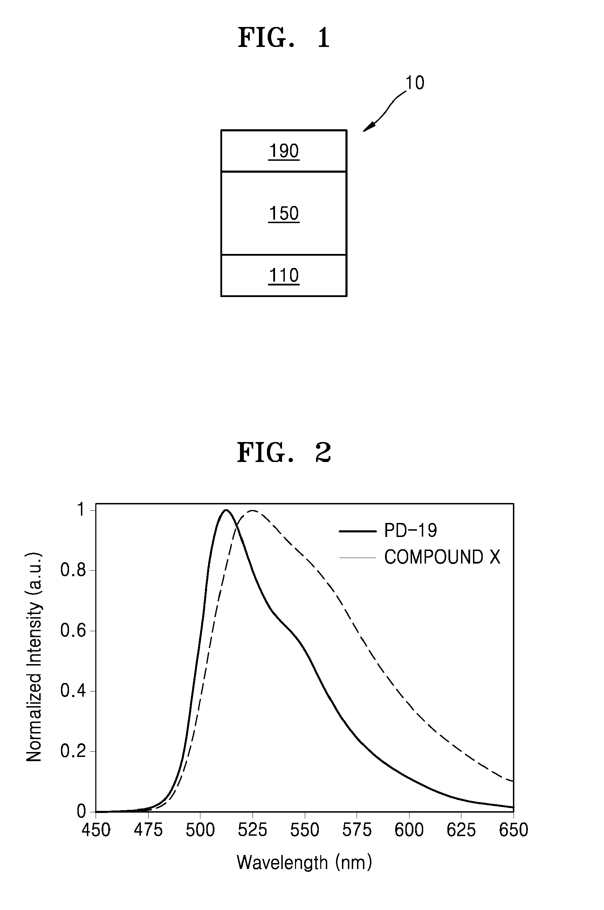

FIG. 1 is a schematic view of a structure of an organic light-emitting device 10 according to an exemplary embodiment.

In FIG. 1, a substrate may be additionally disposed under a first electrode 110 or on a second electrode 190. The substrate may be a glass substrate or a transparent plastic substrate, each with excellent mechanical strength, thermal stability, transparency, surface smoothness, ease of handling, and water-resistance.

The first electrode 110 may be formed by, e.g., depositing or sputtering a material for forming the first electrode 110 on the substrate. When the first electrode 110 is an anode, the material for forming the first electrode 110 may be selected from materials having a high work function to facilitate hole injection. The first electrode may be a reflective electrode, a semi-transparent electrode, or a transparent electrode. The material for forming the first electrode 110 may be a transparent and highly conductive material, and examples of such a material include indium tin oxide (ITO), indium zinc oxide (IZO), tin oxide (SnO₂), and zinc oxide (ZnO). When the first electrode 110 is a semi-transparent electrode or a reflective electrode, at least one of magnesium (Mg), aluminum (Al), aluminum-lithium (Al-Li), calcium (Ca), magnesium-indium (Mg-ln), and magnesium-silver (Mg-Ag) may be utilized as a material for forming the first electrode 110.

The first electrode 110 may have a single-layered structure or a multi-layered structure including a plurality of layers. For example, the first electrode 110 may have a three-layered structured of ITO/Ag/ITO, but the structure of the first electrode 110 is not limited thereto.

An organic layer 150 including an emission layer (EML) may be disposed on the first electrode 110. The organic layer 150 may further include a hole transport region disposed between the first electrode 110 and the EML and an electron transport region disposed between the EML and the second electrode 190.

The hole transport region may include at least one of a hole injection layer (HIL), a hole transport layer (HTL), a buffer layer, and an electron blocking layer (EBL); and the electron transport region may include at least one of a hole blocking layer (HBL), an electron transport layer (ETL), and an electron injection layer (EIL), but the hole transport region and the electron transport region are not limited thereto.

The hole transport region may have a single-layered structure formed of a single material, a single-layered structure formed of a plurality of different materials, or a multi-layered structure having a plurality of layers formed of a plurality of different materials.

For example, the hole transport region have a single-layered structure formed of a plurality of different materials, or a multi-layered structure such as a structure of HIL/HTL, a structure of HIL/HTL/buffer layer, a structure of HIL/buffer layer, a structure of HTL/buffer layer, or a structure of HIL/HTL/EBL. Layers of each structure are sequentially stacked from the first electrode 110 in this stated order, but the hole transport region is not limited thereto.

When the hole transport region includes an HIL, the HIL may be formed on the first electrode 110 by utilizing various suitable methods, such as vacuum deposition, spin coating, casting, a Langmuir-Blodgett (LB) method, ink-jet printing, laser-printing, or a laser-induced thermal imaging (LITI) method.

When an HIL is formed by vacuum deposition, the vacuum deposition may be performed, e.g., at a deposition temperature of about 100 °C to about 500 °C, at a vacuum degree of about 10⁻⁸ torr to about 10⁻³ torr, and a deposition rate of about 0.01 Å/sec to about 100 Å/sec, depending upon a composition of a compound for forming the HIL to be deposited and a structure of the HIL to be formed.

When an HIL is formed by spin coating, the coating may be performed, e.g., at a coating speed of about 2,000 rpm to about 5,000 rpm and at a temperature of about 80 °C to about 200 °C, depending upon a composition of a compound for forming the HIL to be deposited and a structure of the HIL to be formed.

When the hole transport region includes an HTL, the HTL may be formed on the first electrode 110 or the HIL by utilizing various suitable methods, such as vacuum deposition, spin coating, casting, an LB method, an ink-jet printing, a laser-printing, or an LITI method. When the HTL is formed by vacuum deposition and/or spin coating, the deposition and coating conditions for the HTL may be determined by referring to the deposition and coating conditions for the HIL.

The hole transport region may include at least one of m-MTDATA, TDATA, 2-TNATA, NPB, β-NPB, TPD, Spiro-TPD, Spiro-NPB, α-NPB, TAPC, HMTPD, 4,4',4"-tris(N-carbazolyl)triphenylamine (TCTA), polyaniline/dodecylbenzenesulfonic acid (Pani/DBSA), poly(3,4-ethylenedioxythiophene)/poly(4-styrenesulfonate) (PEDOT/PSS), polyaniline/camphor sulfonicacid:polyaniline (Pani/CSA), polyaniline)/poly(4-styrenesulfonate) (PANI/PSS), a compound represented by Formula 201, and a compound represented by Formula 202:

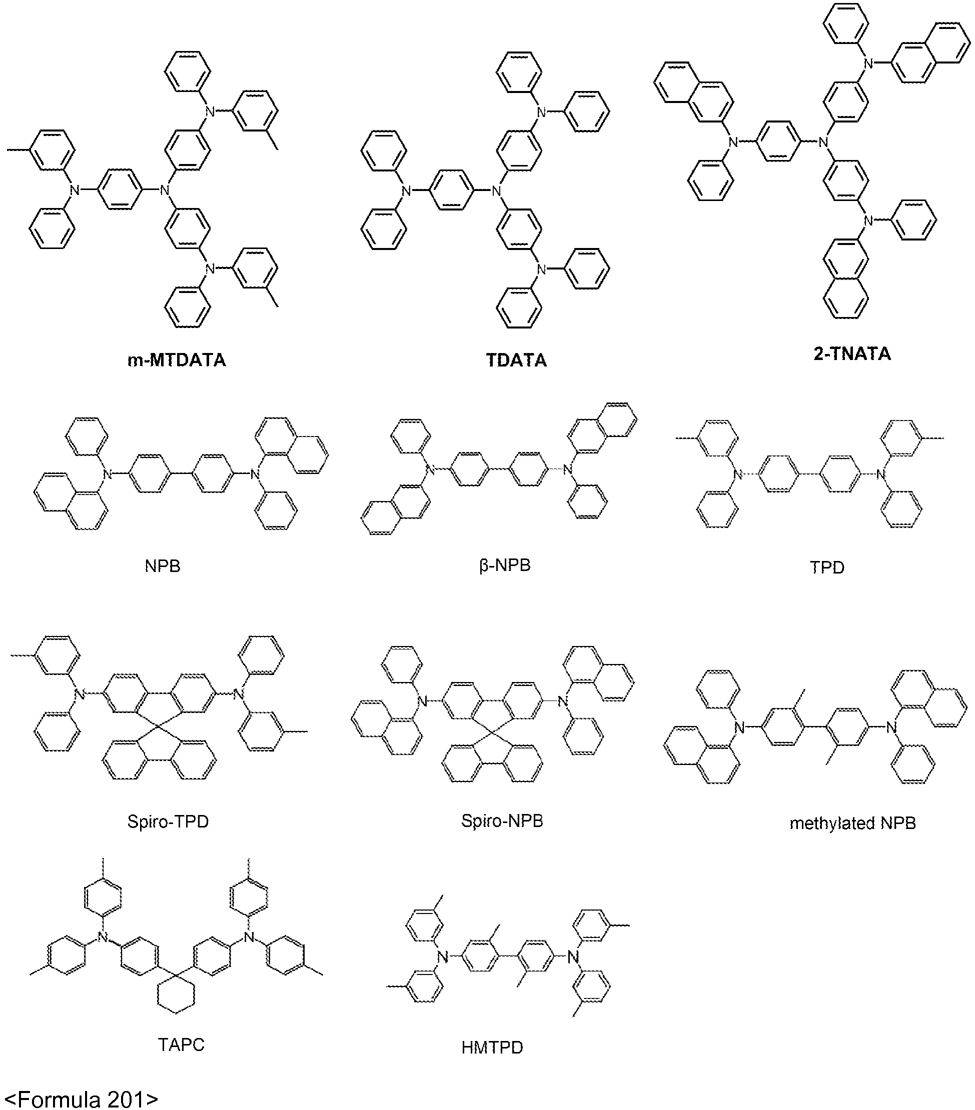

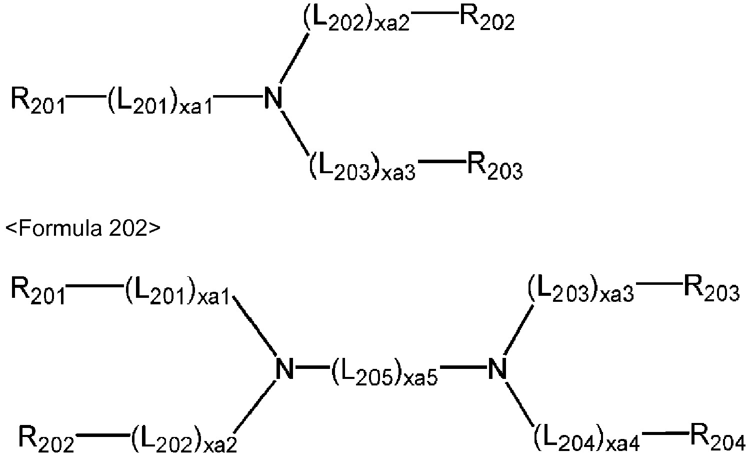

In Formulae 201 and 202,
L₂₀₁ to L₂₀₅ may each be independently selected from:
a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
at least one substituent of the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the subtstituted divalent non-aromatic condensed polycyclic group, and the subtstituted divalent non-aromatic condensed heteropolycyclic group may be selected from:
   a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
   a C₁-C₆₀ alkyl group, a C₂-C₆₀ alkenyl group, a C₂-C₆₀ alkynyl group, and a C₁-C₆₀ alkoxy group, each substituted with at least one of a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₀₁)(Q₂₀₂), -Si(Q₂₀₃)(Q₂₀₄)(Q₂₀₅), and -B(Q₂₀₆)(Q₂₀₇);
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, - N(Q₂₁₁)(Q₂₁₂), -Si(Q₂₁₃)(Q₂₁₄)(Q₂₁₅), and -B(Q₂₁₆)(Q₂₁₇); and
   -N(Q₂₂₁)(Q₂₂₂), -Si(Q₂₂₃)(Q₂₂₄)(Q₂₂₅), and -B(Q₂₂₆)(Q₂₂₇),
   xa1 to xa4 may each be independently selected from 0, 1,2, and 3;
   xa5 may be selected from 1, 2, 3, 4, and 5, and
   R₂₀₁ to R₂₀₅ may each be independently selected from:
      a C₁-C₆₀ alkyl group, a C₂-C₆₀ alkenyl group, a C₂-C₆₀ alkynyl group, and a C₁-C₆₀ alkoxy group;
      a C₁-C₆₀ alkyl group, a C₂-C₆₀ alkenyl group, a C₂-C₆₀ alkynyl group, and a C₁-C₆₀ alkoxy group, each substituted with at least one of a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₃₁)(Q₂₃₂), -Si(Q₂₃₃)(Q₂₃₄)(Q₂₃₅), and -B(Q₂₃₆)(Q₂₃₇);
      a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group; and
      a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, - N(Q₂₄₁)(Q₂₄₂), -Si(Q₂₄₃)(Q₂₄₄)(Q₂₄₅), and -B(Q₂₄₆)(Q₂₄₇),
      wherein Q₂₀₁ to Q₂₀₇, Q₂₁₁ to Q₂₁₇, Q₂₂₁ to Q₂₂₇, Q₂₃₁ to Q₂₃₇, and Q₂₄₁ to Q₂₄₇ may each be independently selected from:
         a hydrogen, a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
         a C₁-C₆₀ alkyl group, a C₂-C₆₀ alkenyl group, a C₂-C₆₀ alkynyl group, and a C₁-C₆₀ alkoxy group, each substituted with at least one of a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
         a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group; and
         a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group.

For example, in Formulae 201 and 202,
L₂₀₁ to L₂₀₅ may each be independently selected from:
a phenylene group, a naphthylenylene group, a fluorenylene group, a spiro-fluorenylene group, a benzofluorenylene group, a dibenzofluorenylene group, a phenanthrenylene group, an anthracenylene group, a pyrenylene group, a chrysenylene group, a pyridinylene group, a pyrazinylene group, a pyrimidinylene group, a pyridazinylene group, a quinolinylene group, an isoquinolinylene group, a quinoxalinylene group, a quinazolinylene group, a carbazolylene group, and a triazinylene group; and
a phenylene group, a naphthylenylene group, a fluorenylene group, a spiro-fluorenylene group, a benzofluorenylene group, a dibenzofluorenylene group, a phenanthrenylene group, an anthracenylene group, a pyrenylene group, a chrysenylene group, a pyridinylene group, a pyrazinylene group, a pyrimidinylene group, a pyridazinylene group, a quinolinylene group, an isoquinolinylene group, a quinoxalinylene group, a quinazolinylene group, a carbazolylene group, and a triazinylene group, each substituted with at least one of a deuterium, -F, -CI, - Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, an isoindolyl group, a quinolinyl group, an isoquinolinyl group, a quinoxalinyl group, a quinazolinyl group, a carbazolyl group, and a triazinyl group,
xa1 to xa4 may each be independently 0, 1, or 2,
xa5 may be 1, 2, or 3,
R₂₀₁ to R₂₀₅ may each be independently selected from:
   a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group; and
   a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group, each substituted with at least one of a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, an azulenyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group, but embodiments are not limited thereto.

The compound of Formula 201 may be represented by Formula 201A:

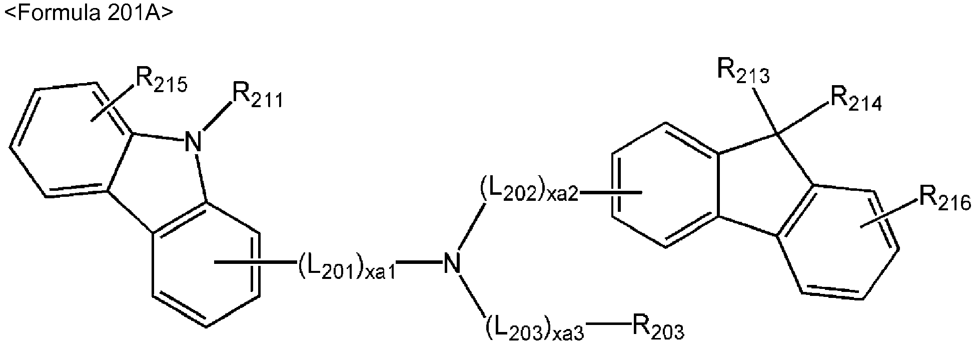

For example, the compound of Formula 201 may be represented by Formula 201A-1, but is not limited thereto:

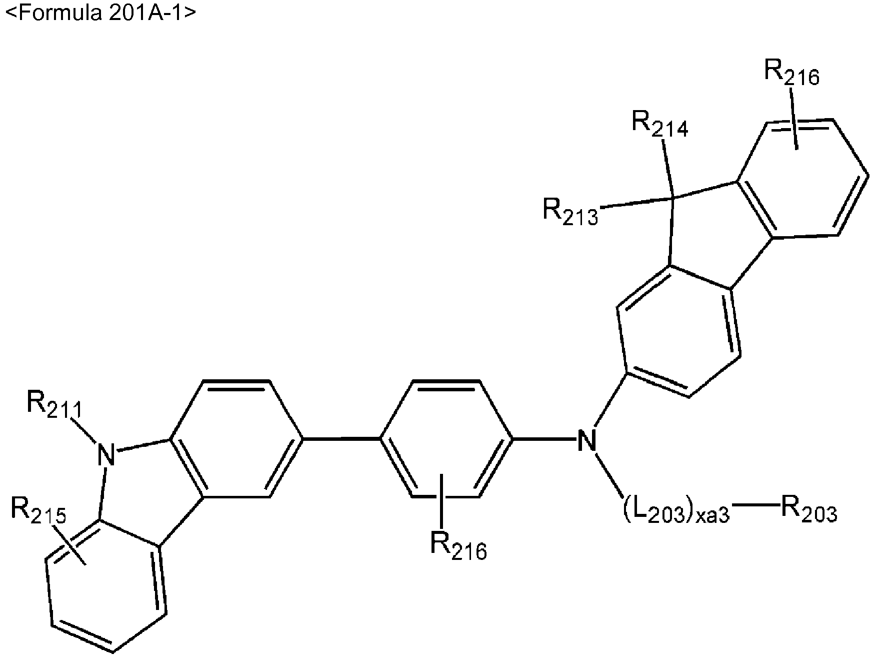

The compound of Formula 202 may be represented by Formula 202A, but is not limited thereto:

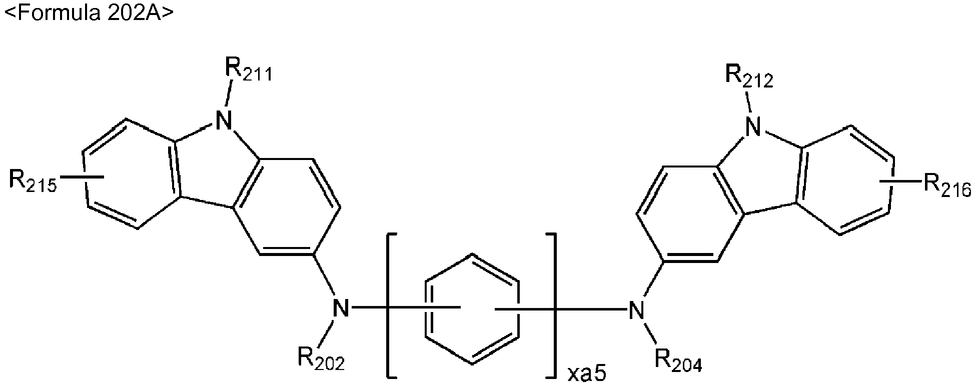

In Formulae 201A, 201A-1, and 202A, descriptions of L₂₀₁ to L₂₀₃, xa1 to xa3, xa5, and R₂₀₂ to R₂₀₄ may each be independently as referred to in the descriptions provided above, descriptions of R₂₁₁ and R₂₁₂ may each be independently as referred to in the description provided in connection with R₂₀₃, and R₂₁₃ to R₂₁₆ may each be independently selected from: a hydrogen, a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group.

For example, in Formulae 201A, 201A-1, and 202A, L₂₀₁ to L₂₀₃ may each be independently selected from:
a phenylene group, a naphthylenylene group, a fluorenylene group, a spiro-fluorenylene group, a benzofluorenylene group, a dibenzofluorenylene group, a phenanthrenylene group, an anthracenylene group, a pyrenylene group, a chrysenylene group, a pyridinylene group, a pyrazinylene group, a pyrimidinylene group, a pyridazinylene group, a quinolinylene group, an isoquinolinylene group, a quinoxalinylene group, a quinazolinylene group, a carbazolylene group, and a triazinylene group; and
a phenylene group, a naphthylenylene group, a fluorenylene group, a spiro-fluorenylene group, a benzofluorenylene group, a dibenzofluorenylene group, a phenanthrenylene group, an anthracenylene group, a pyrenylene group, a chrysenylene group, a pyridinylene group, a pyrazinylene group, a pyrimidinylene group, a pyridazinylene group, a quinolinylene group, an isoquinolinylene group, a quinoxalinylene group, a quinazolinylene group, a carbazolylene group, and a triazinylene group, each substituted with at least one of a deuterium, -F, -CI, - Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group,
xa1 to xa3 may each be independently 0 or 1,
R₂₀₃, R₂₁₁, and R₂₁₂ may each be independently selected from:
   a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group; and
   a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group, each substituted with at least one of a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group,
   R₂₁₃ and R₂₁₄ may each be independently selected from:
      a C₁-C₂₀ alkyl group and a C₁-C₂₀ alkoxy group;
      a C₁-C₂₀ alkyl group and a C₁-C₂₀ alkoxy group, each substituted with at least one of a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group;
      a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group; and
      a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group, each substituted with at least one of a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group,
      R₂₁₅ and R₂₁₆ may each be independently selected from a hydrogen, a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof,
      a C₁-C₂₀ alkyl group, and a C₁-C₂₀ alkoxy group;
      a C₁-C₂₀ alkyl group and a C₁-C₂₀ alkoxy group, each substituted with at least one of a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group;
      a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, and a triazinyl group; and
      a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, and a triazinyl group, each substituted with at least one of a deuterium, - F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group, and
      xa5 may be 1 or 2.

In Formulae 201A and 201A-1, R₂₁₃ and R₂₁₄ may bind to each other to form a saturated or unsaturated ring.

The compound of Formula 201 and the compound of Formula 202 may each include one of Compounds HT1 to HT20, but the compound of Formula 201 and the compound of Formula 202 are not limited thereto:

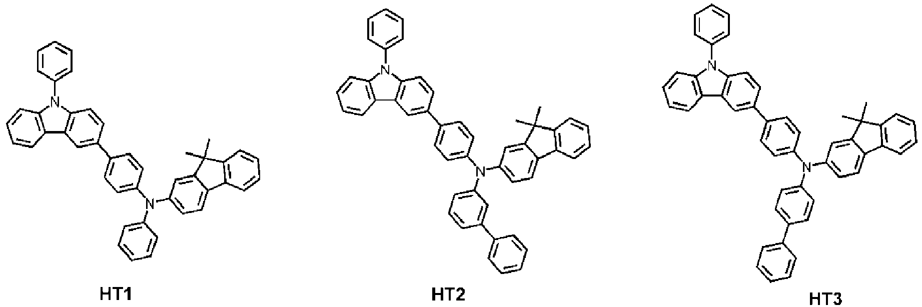

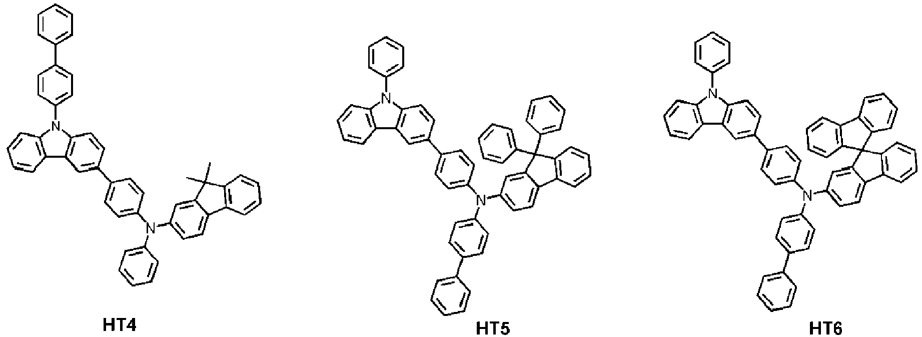

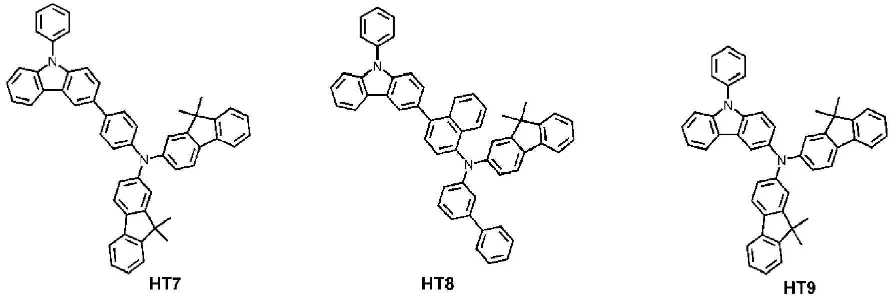

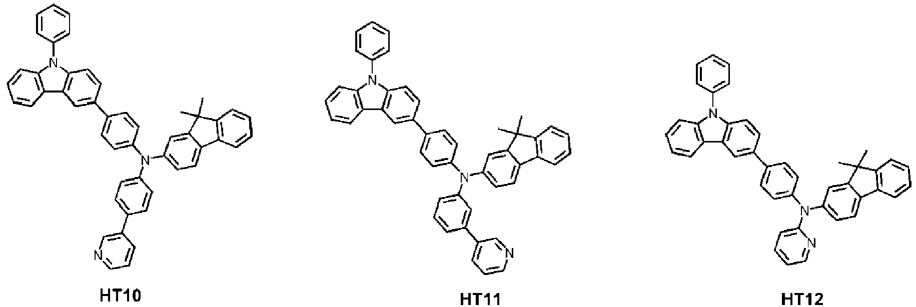

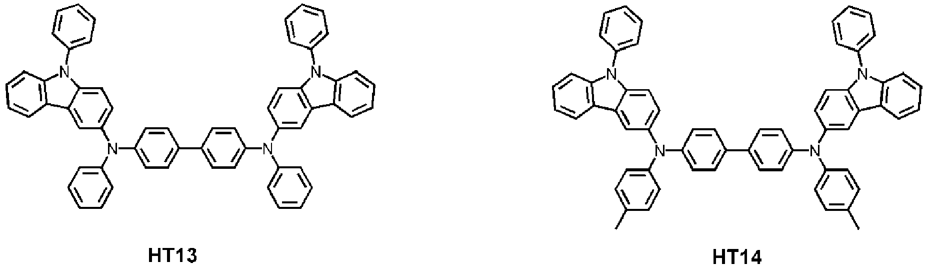

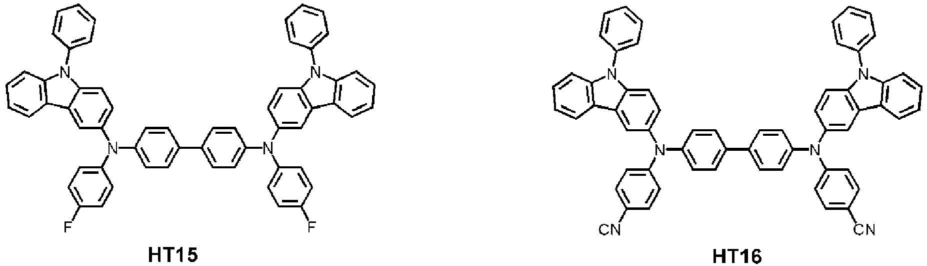

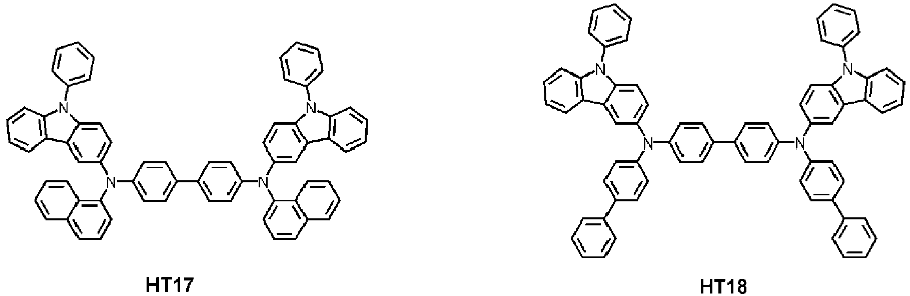

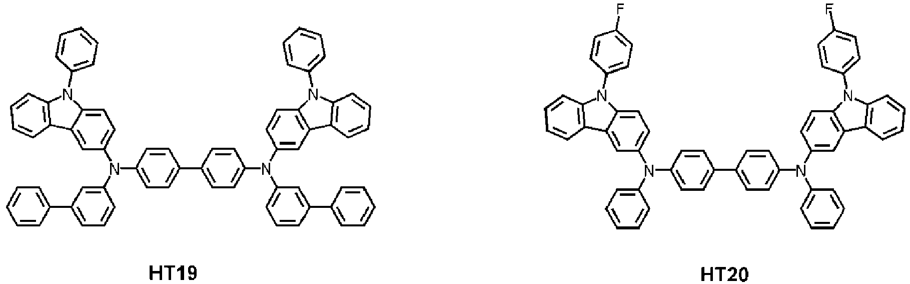

A thickness of the hole transport region may be in a range of about 100 Å to about 10,000 Å, for example, about 100 Å to about 1,000 Å. When the hole transport region includes both an HIL and an HTL, a thickness of the HIL may be in a range of about 100 Å to about 10,000 Å, for example, about 100 Å to about 1,000 Å; and a thickness of the HTL may be in a range of about 50 Å to about 2,000 Å, for example about 100 Å to about 1,500 Å. In one examplary embodiment, when the thickness of the hole transport region, the HIL, and the HTL are within these ranges, satisfactory or suitable hole transporting characteristics are obtained without a substantial increase in driving voltage.

The hole transport region may further include, in addition to these materials, a charge-generation material for the improvement of conductive properties. The charge-generation material may be homogeneously or inhomogeneously dispersed in the hole transport region.

The charge-generation material may be, for example, a p-dopant. The p-dopant may be one of a quinone derivative, a metal oxide, and a cyano group-containing compound, but the p-dopant is not limited thereto. For example, non-limiting examples of the p-dopant are a quinone derivative such as tetracyano-quinonedimethane (TCNQ) and 2,3,5,6-tetrafluoro-tetracyano-1,4-benzoquinonedimethane (F4-TCNQ); a metal oxide such as a tungsten oxide and a molybdenum oxide; and Compound HT-D1, but the p-dopant is not limited thereto.

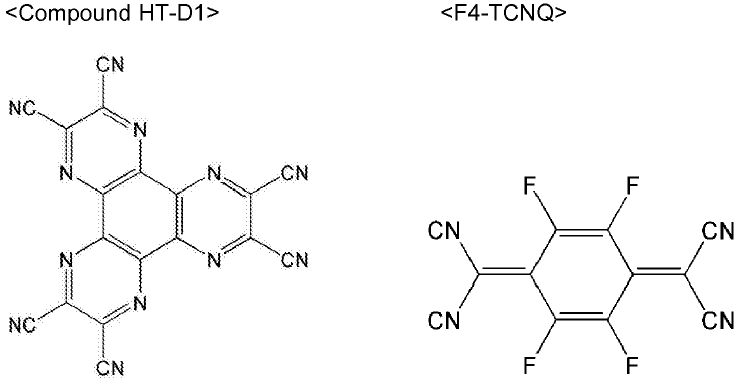

The hole transport region may further include, in addition to the HIL and the HTL, at least one of a buffer layer and an EBL. Since the buffer layer may compensate for an optical resonance distance according to a wavelength of light emitted from the EML, light-emission efficiency of a formed organic light-emitting device may be improved. For usage as a material included in the buffer layer, materials that are included in the hole transport region may be utilized. The EBL may reduce or prevent injection of electrons from the electron transport region.

The EML may be formed on the first electrode 110 or on the hole transport region by utilizing various suitable methods, such as vacuum deposition, spin coating, casting, an LB method, an ink-jet printing, a laser-printing, or an LITI method. When the EML is formed by vacuum deposition and spin coating, the deposition and coating conditions for the emission layer may be determined by referring to the deposition and coating conditions for the HIL.

When the organic light-emitting device 10 is a full color organic light-emitting device, the EML may be patterned into a red EML, a green EML, or a blue EML, according to individual sub pixels, respectively. The EML may have various modifications in the structure, and for example, may have a structure of a red EML, a green EML, and a blue EML, each of which layers are sequentially stacked in the stated order, or a structure in which a red light-emitting material, a green light-emitting material, and a blue light-emitting material are mixed without distinction between layers, and accordingly the EML may emit white light. Alternatively, the EML may be a white EML, and may further include a color converting layer, which converts white light to light of desired color, or a color filter.

Hereinafter, an exemplary embodiment in which an EML of an organic light-emitting includes the first host will be described (Embodiment 1).

In an exemplary embodiment, the EML may include the first host represented by one of Formulae 1 and 2:

<Formula 1> Ar₁₁⁅(L₁₁)ₐ₁₁-(R₁₁)_{b11}]ₙ₁₁

<Formula 2> Ar₂₁⁅(L₂₁)ₐ₂₁-(R₂₁)_{b21}]ₙ₂₁

In Formulae 1 and 2, Ar₁₁ and Ar₂₁ may each be independently selected from: a substituted or unsubstituted C₄-C₃₀ pyrrolidine-based core and a substituted or unsubstituted C₇-C₃₀ condensed polycyclic-based core,
at least one substituent of the substituted C₄-C₃₀ pyrrolidine-based core and the substituted C₇-C₃₀ condensed polycyclic-based core may be selected from:
a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), and -B(Q₁₆)(Q₁₇);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), and -B(Q₂₆)(Q₂₇); and
-N(Q₃₁)(Q₃₂), -Si(Q₃₃)(Q₃₄)(Q₃₅), and -B(Q₃₆)(Q₃₇),
wherein Q₁ to Q₇, Q₁₁ to Q₁₇, Q₂₁ to Q₂₇, and Q₃₁ to Q₃₇ may each independently be selected from a hydrogen, a C₁-C₆₀ alkyl group, a C₁-C₆₀ alkoxy group, a C₆-C₆₀ aryl group, a C₁-C₆₀ heteroaryl group, a monovalent non-aromatic condensed polycyclic group, and a monovalent non-aromatic condensed heteropolycyclic group.

For example, the substituted or unsubstituted C₄-C₃₀ pyrrolidine-based core may have a partial pyrrolidine structure represented by Formula 10-1, but the substituted or unsubstituted C₄-C₃₀ pyrrolidine-based core is not limited thereto:

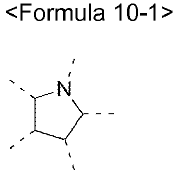

In Formula 10-1,
the line '- - -' indicates a covalent bond with an adjacent atom, wherein the adjacent atom may be a hydrogen atom (H), a nitrogen atom (N), an oxygen atom (O), a carbon atom (C), or a sulfur atom (S).

For example, the substituted or unsubstituted C₇-C₃₀ condensed polycyclic-based core may have a partial condensed polycyclic group represented by one of Formulae 10-2 or 10-2, but the substituted or unsubstituted C₇-C₃₀ condensed polycyclic-based core is not limited thereto:

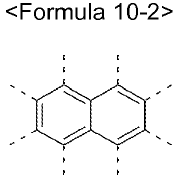

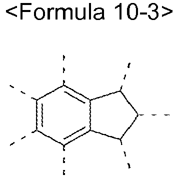

In Formulae 10-2 and 10-3,
the line '- - -' indicates a covalent bond with an adjacent atom, wherein the adjacent atom may be H, N, or C.

For example, in Formulae 1 and 2, Ar₁₁ may be represented by one of Formulae 8A-1 to 8A-4, 8B-1 to 8B-19, and 8C-1 to 8C-19, and
Ar₂₁ may be a group represented by one of Formulae 9A-1 to 9A-4, 9B-1 to 9B-19, and 9C-1 to 9C-19, but Ar₁₁ and Ar₂₁ are not limited thereto:

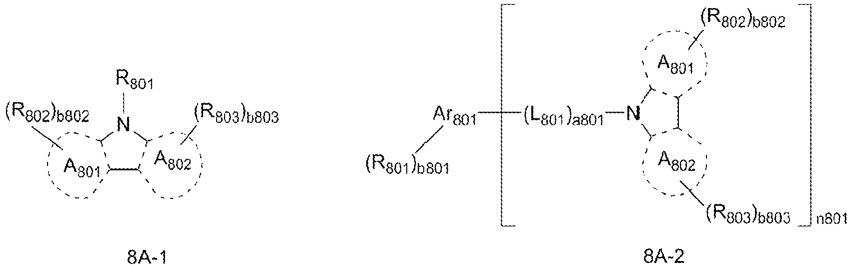

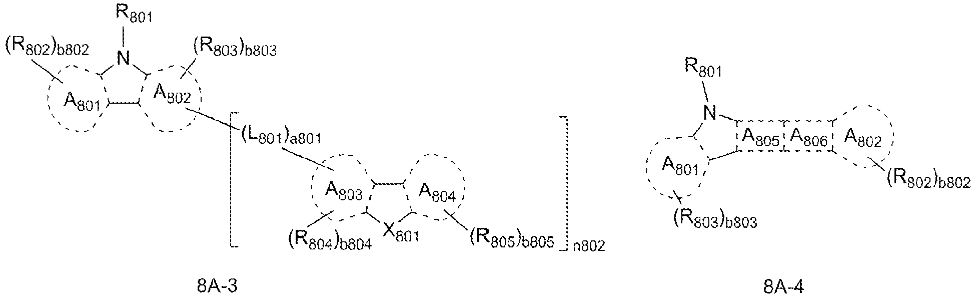

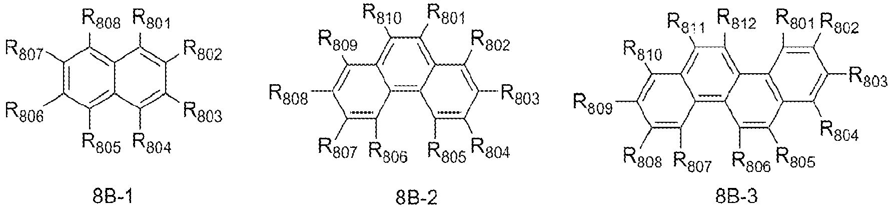

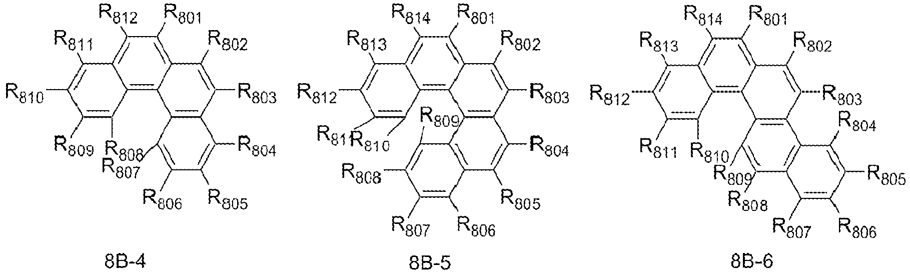

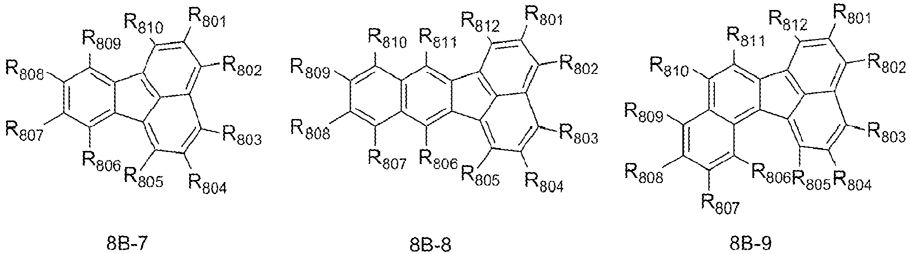

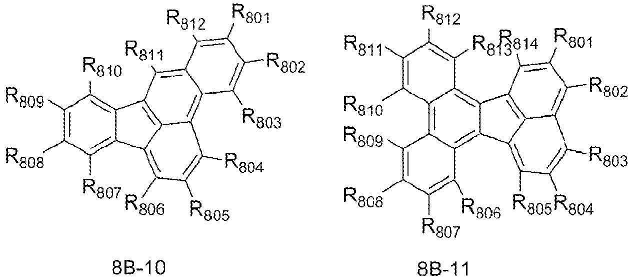

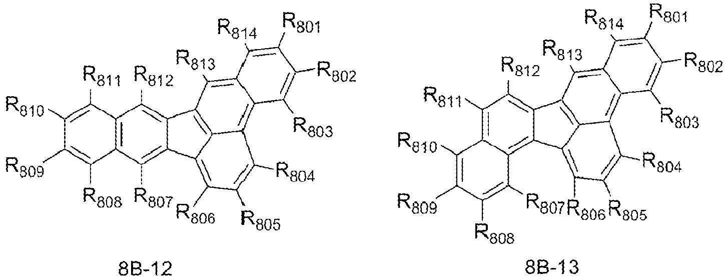

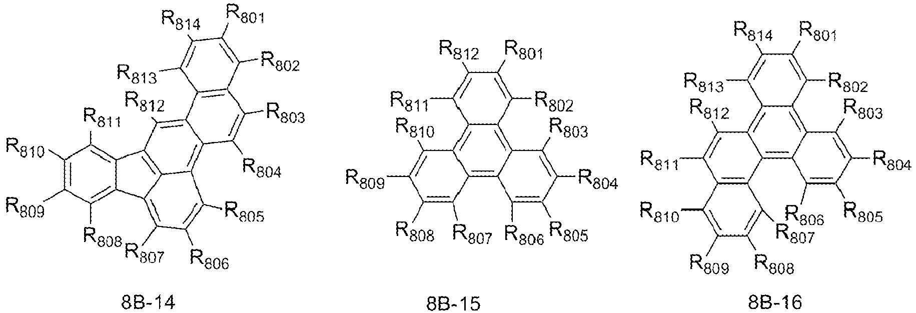

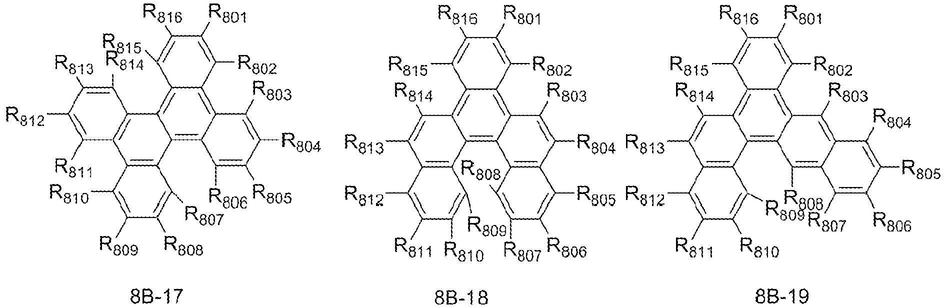

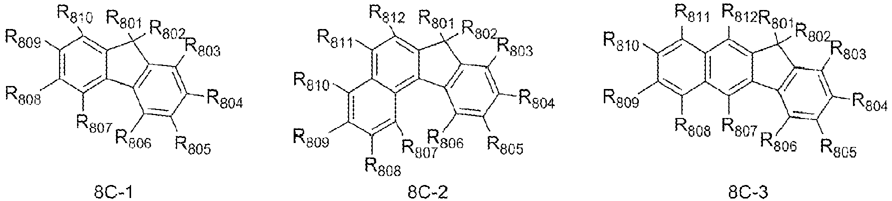

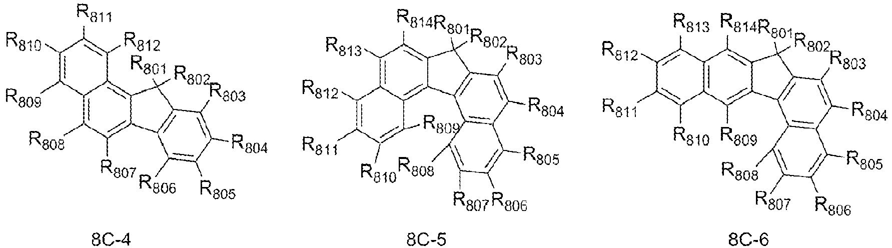

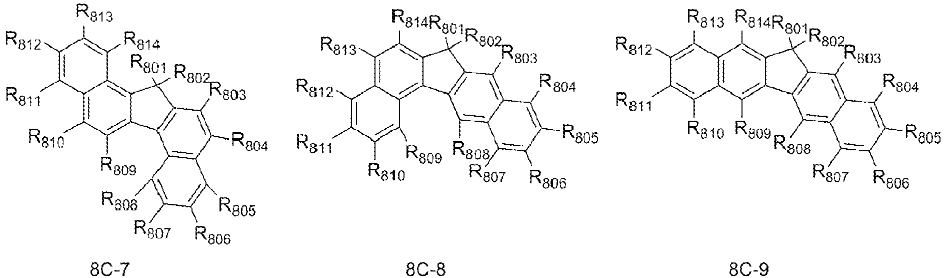

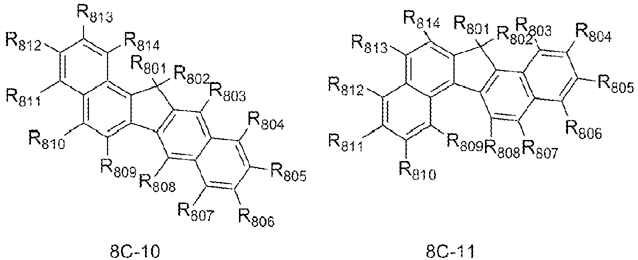

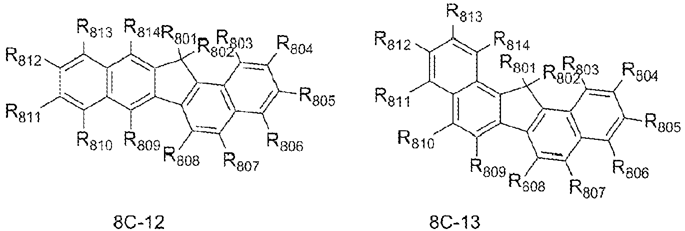

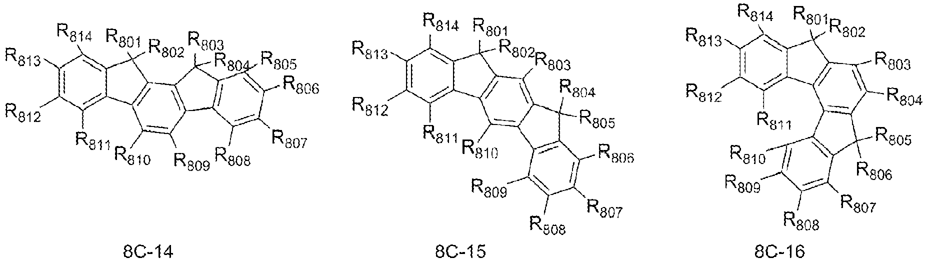

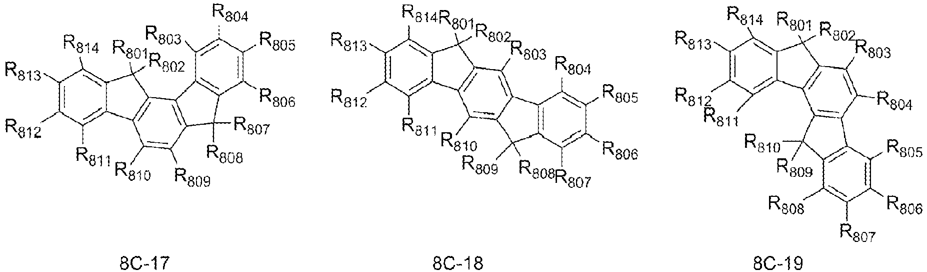

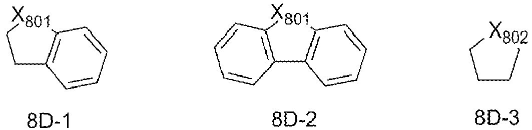

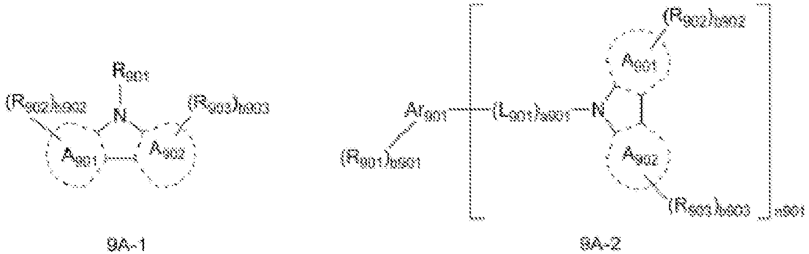

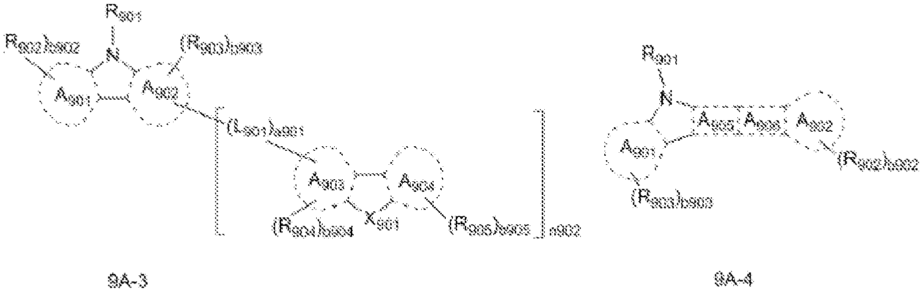

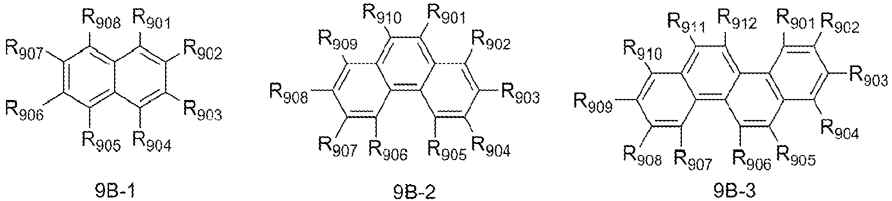

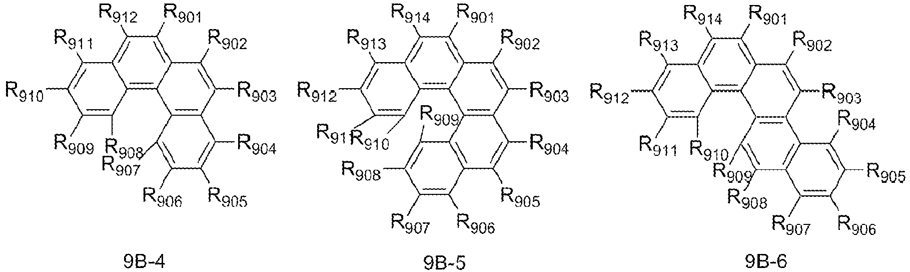

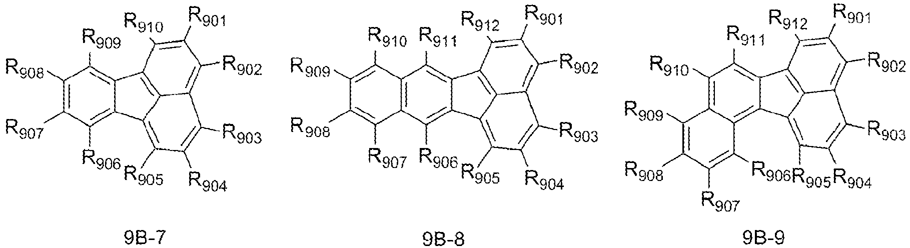

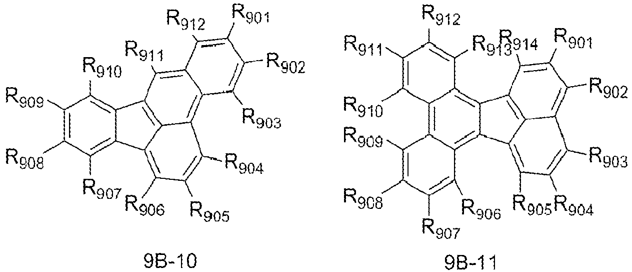

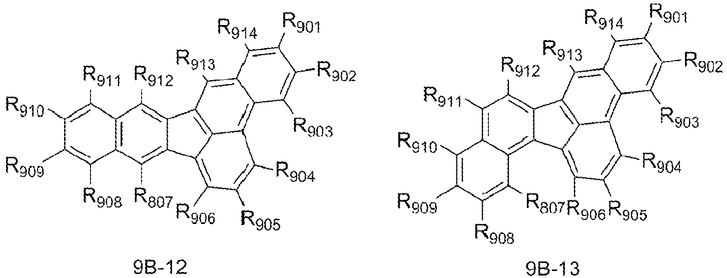

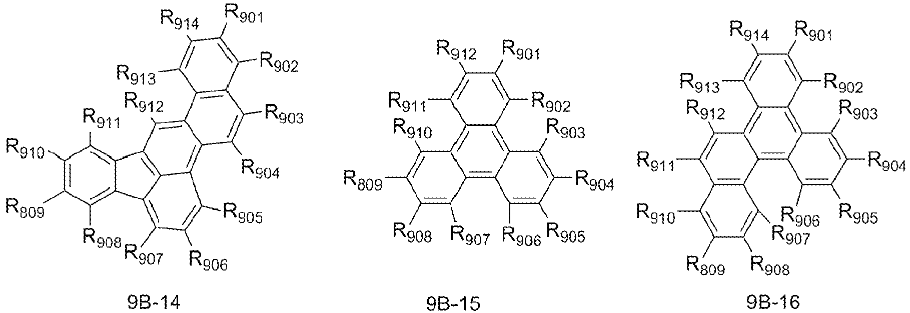

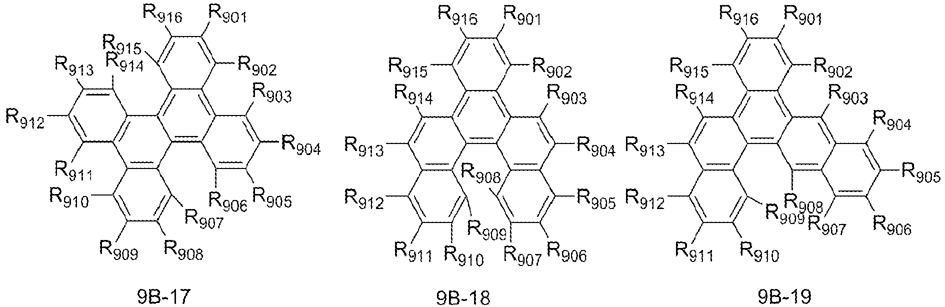

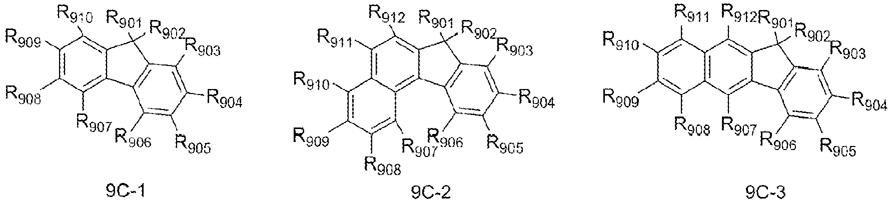

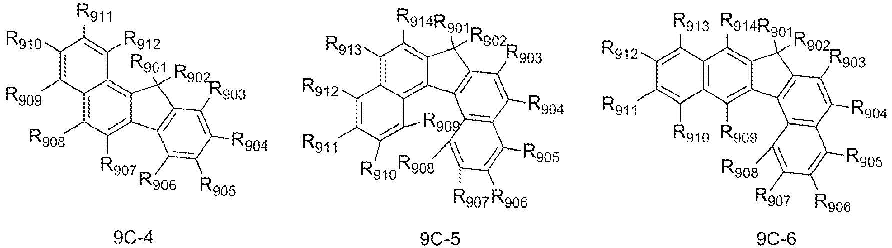

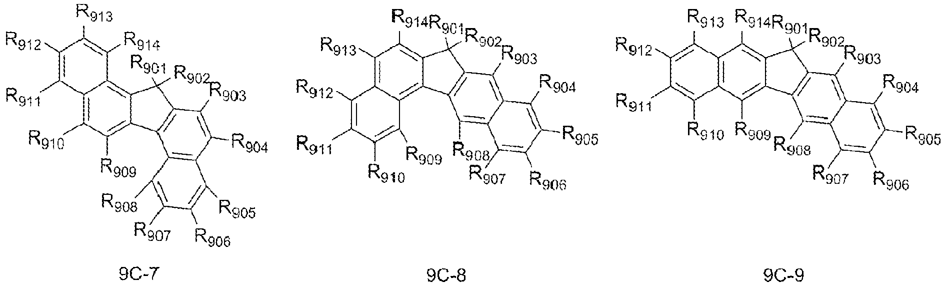

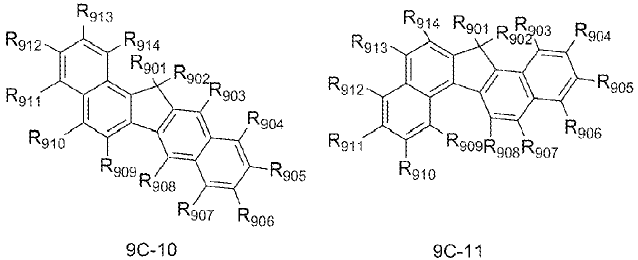

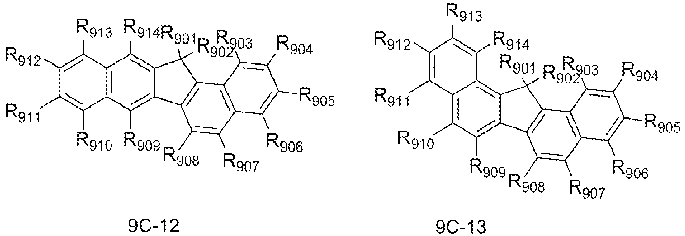

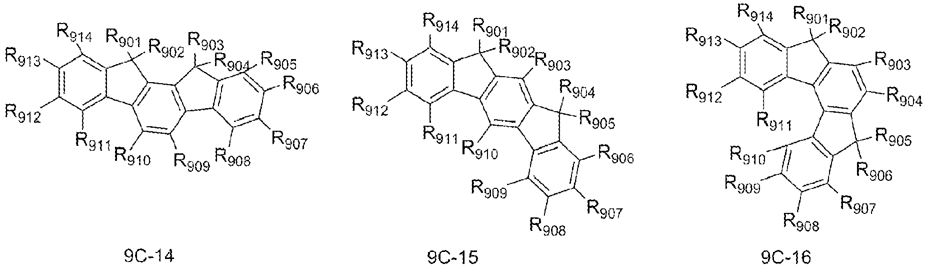

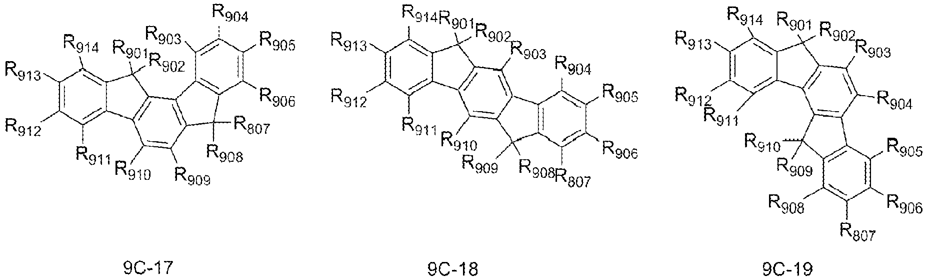

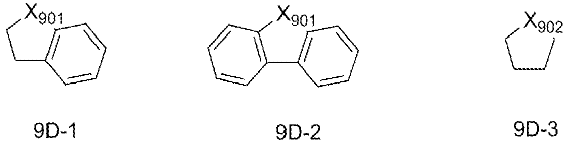

In Formulae 8A-1 to 8A-4, 8B-1 to 8B-19, 8D-1 to 8D-3, 8C-1 to 8C-19, 9A-1 to 9A-4, 9B-1 to 9B-19, 9C-1 to 9C-19, and 9D-1 to 9D-3,
Ar₈₀₁ and Ar₉₀₁, may each be independently selected from a substituted or unsubstituted C₃-C₁₀ cycloalkane group, a substituted or unsubstituted C₁-C₁₀ heterocycloalkane group, a substituted or unsubstituted C₃-C₁₀ cycloalkene group, a substituted or unsubstituted C₁-C₁₀ heterocycloalkene group, a substituted or unsubstituted C₆-C₆₀ arene group, a substituted or unsubstituted C₁-C₆₀ heteroarene group, a substituted or unsubstituted non-aromatic condensed polycyclic group, and a substituted or unsubstituted non-aromatic condensed heteropolycyclic group,
L₈₀₁, and L₉₀₁ may each be independently selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
a801 and a901 may each be independently selected from 0, 1, 2, and 3,
A₈₀₁ to A₈₀₄ may each be independently selected from a benzene, a naphthalene, a pyridine, a pyrimidine, a pyrazine, a quinoline, an isoquinoline, a 2,6-naphthyridine, a 1,8-naphthyridine, a 1,5-naphthyridine, a 1,6-naphthyridine, a 1,7-naphthyridine, a 2,7-naphthyridine, a quinoxaline, a phthalazine, a quinazoline, the group of Formula 8D-1, and the group of Formula 8D-2,
A₉₀₁ to A₉₀₄ may each be independently selected from a benzene, a naphthalene, a pyridine, a pyrimidine, a pyrazine, a quinoline, an isoquinoline, a 2,6-naphthyridine, a 1,8-naphthyridine, a 1,5-naphthyridine, a 1,6-naphthyridine, a 1,7-naphthyridine, a 2,7-naphthyridine, a quinoxaline, a phthalazine, a quinazoline, the compound of Formula 9D-1, and the compound of Formula 9D-2,
A₈₀₅ and A₉₀₅ may each be independently selected from a benzene and a naphthalene,
A₈₀₆ may be presented by Formula 8D-3, and A₉₀₆ may be represented by Formula 9D-3,
X₈₀₁ and X₈₀₂ may each be independently selected from N(R₈₀₆), O, S, C(R₈₀₆)(R₈₀₇), Si(R₈₀₆)(R₈₀₇), B(R₈₀₆), P(R₈₀₆), and P(=O)(R₈₀₆), and X₉₀₁ and X₉₀₂ may each be independently selected from N(R₉₀₆), O, S, C(R₉₀₆)(R₉₀₇), Si(R₉₀₆)(R₉₀₇), B(R₉₀₆), P(R₉₀₆), and P(=O)(R₉₀₆),
R₈₀₁ to R₈₁₆ may each be independently selected from *-[(L₁₁)ₐ₁₁-(R₁₁)_{b11}], a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, wherein R₈₀₁ to R₈₁₆ in the number of n11 may be *-[(L₁₁)ₐ₁₁-(R₁₁)_{b11}],
R₉₀₁ to R₉₁₆ may each be independently selected from *-[(L₂₁)ₐ₂₁-(R₂₁)_{b21}], a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, wherein R₉₀₁ to R₉₁₆ in the number of n21 may be *-[(L₂₁)ₐ₂₁-(R₂₁)_{b21}],
b801 to b805 and b901 to b905 may each be independently selected from 1, 2, 3, and 4,
n801 and n901 may each be independently 2, 3, and 4,
n802 and n902 may each be independently selected from 1, 2, and 3, and
at least one substituent of the substituted C₃-C₁₀ cycloalkane group, the substituted C₁-C₁₀ heterocycloalkane group, the substituted C₃-C₁₀ cycloalkene group, the substituted C₁-C₁₀ heterocycloalkene group, the substituted C₆-C₆₀ arene group, the substituted C₁-C₆₀ heteroarene group, the substituted non-aromatic condensed polycyclic group, the substituted non-aromatic condensed heteropolycyclic group, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the subtstituted divalent non-aromatic condensed polycyclic group, the subtstituted divalent non-aromatic condensed heteropolycyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted or unsubstituted monovalent non-aromatic condensed heteropolycyclic group may be selected from:
a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), and -B(Q₁₆)(Q₁₇);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), and -B(Q₂₆)(Q₂₇); and
-N(Q₃₁)(Q₃₂), -Si(Q₃₃)(Q₃₄)(Q₃₅), and -B(Q₃₆)(Q₃₇),
wherein Q₁₁ to Q₁₇, Q₂₁ to Q₂₇, and Q₃₁ to Q₃₇ may each be independently selected from a hydrogen, a C₁-C₆₀ alkyl group, a C₁-C₆₀ alkoxy group, a C₆-C₆₀ aryl group, a C₁-C₆₀ heteroaryl group, a monovalent non-aromatic condensed polycyclic group, and a monovalent non-aromatic condensed heteropolycyclic group, but embodiments are not limited thereto.

In an exemplary embodiment, in Formulae 8A-2 and 9A-2, Ar₈₀₁ and Ar₉₀₁ may each be independently selected from a cyclohexane, a benzene, a naphthalene, a pyridine, a pyrimidine, a triazine, a fluorene, and a spiro-fluorene, but Ar₈₀₁ and Ar₉₀₁ are not limited thereto.

In another exemplary embodiment, in Formulae 8A-2 and 9A-2, Ar₈₀₁ and Ar₉₀₁ may each be independently selected from a cyclohexane, a benzene, a pyridine, and a fluorene, but Ar₈₀₁ and Ar₉₀₁ are not limited thereto.

For example, in Formulae 8A-2 and 8A-3, descriptions of L₈₀₁ and L₉₀₁ may each be independently as referred to in the descriptions provided in connection with L₁₁.

For example, in Formulae 8A-2 and 8A-3, descriptions of a801 and a901 may each be independently as referred to in the descriptions provided in connection with a11.

In an exemplary embodiment, in Formulae 8A-1 to 8A-4 and 9A-1 to 9A-4, A₈₀₁ to A₈₀₄ and A₉₀₁ to A₉₀₄ may each be independently selected from a benzene, a naphthalene, a pyridine, a pyrimidine, a pyrazine, a quinoline, an isoquinoline, a 2,6-naphthyridine, a 1,8-naphthyridine, a 1,5-naphthyridine, a 1,6-naphthyridine, a 1,7-naphthyridine, a 2,7-naphthyridine, a quinoxaline, a phthalazine, and a quinazoline, but A₈₀₁ to A₈₀₄ and A₉₀₁ to A₉₀₄ are not limited thereto.

In another exemplary embodiment, in Formulae 8A-1 to 8A-4 and 9A-1 to 9A-4, A₈₀₁ to A₈₀₄ and A₉₀₁ to A₉₀₄ may each be independently selected from a benzene, a naphthalene, a pyridine, a quinoline, and an isoquinoline, but A₈₀₁ to A₈₀₄ and A₉₀₁ to A₉₀₄ are not limited thereto.

In another exemplary embodiment, in Formulae 8A-1 to 8A-4 and 9A-1 to 9A-4, A₈₀₁ to A₈₀₄ and A₉₀₁ to A₉₀₄ may each be independently selected from a benzene and a naphthalene, but A₈₀₁ to A₈₀₄ and A₉₀₁ to A₉₀₄ are not limited thereto.

For example, in Formulae 8A-4 and 9A-4, A₈₀₅ and A₉₀₅ may each be independently a benzene, but A₈₀₅ and A₉₀₅ are not limited thereto.

For example, in Formulae 8D-1 to 8D-3 and 9D-1 to 9D-3, X₈₀₁ and X₈₀₂ may each be independently selected from N(R₈₀₆), O, S, and C(R₈₀₆)(R₈₀₇), and
X₉₀₁ and X₉₀₂ may each be independently selected from N(R₉₀₆), O, S, and C(R₉₀₆)(R₉₀₇), but embodiments are not limited thereto.

For example, in Formulae 8A-1 to 8A-4, 8B-1 to 8B-19, 8D-1 to 8D-3, 8C-1 to 8C-19, 9A-1 to 9A-4, 9B-1 to 9B-19, 9C-1 to 9C-19, and 9D-1 to 9D-3, R₈₀₁ to R₈₁₆ may each be independently selected from *-[(L₁₁)ₐ₁₁-(R₁₁)_{b11}], a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₆-C₆₀ aryl group, and a C₁-C₆₀ heteroaryl group, wherein R₈₀₁ to R₈₁₆ in the number of n11 may be *-[(L₁₁)ₐ₁₁-(R₁₁)_{b11}],

R₉₀₁ to R₉₁₆ may each be independently selected from *-[(L₂₁)ₐ₂₁-(R₂₁)_{b21}], a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₆-C₆₀ aryl group, and a C₁-C₆₀ heteroaryl group, wherein R₉₀₁ to R₉₁₆ in the number of n21 may be *-[(L₂₁)ₐ₂₁-(R₂₁)_{b21}], but embodiments are not limited thereto.

In an examplry embodiment, in Formulae 8A-1 to 8A-4, 8B-1 to 8B-19, 8D-1 to 8D-3, 8C-1 to 8C-19, 9A-1 to 9A-4, 9B-1 to 9B-19, 9C-1 to 9C-19, and 9D-1 to 9D-3, R₈₀₁ to R₈₁₆ may each be independently selected from *-[(L₁₁)ₐ₁₁-(R₁₁)_{b11}], a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a methyl group, an ethyl group, an n-propyl group, an n-butyl group, an iso-butyl group, a sec-butyl group, a tert-butyl group, a phenyl group, a naphthyl group, and a pyridinyl group, wherein R₈₀₁ to R₈₁₆ in the number of n11 may be *-[(L₁₁)ₐ₁₁(R₁₁)_{b11}],
R₉₀₁ to R₉₁₆ may each be independently selected from a hydrogen, a deuterium, -F, -Cl, -Br,-I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a methyl group, an ethyl group, an n-propyl group, an n-butyl group, an iso-butyl group, a sec-butyl group, a tert-butyl group, a phenyl group, a naphthyl group, and a pyridinyl group, wherein R₉₀₁ to R₉₁₆ in the number of n21 may be *-[(L₂₁)ₐ₂₁-(R₂₁)_{b21]}, but embodiments are not limited thereto.

For example, in Formulae 8A-2 and 9A-2, n801 and n901 may each be independently selected from 2 and 3, but n801 and n901 are not limited thereto. When n801 and n901 each are independently 2 or more, a plurality of moieties indicated in [ ] may be identical to or different from each other.

For example, in Formulae 8A-2 and 9A-2, n802 and n902 may each be independently selected from 1 and 2, but n802 and n902 are not limited thereto. When n801 and n901 each are independently 2 or more, a plurality of moieties indicated in [ ] may be identical to or different from each other.

In Formulae 1 and 2, L₁₁ and L₂₁ may each be independently selected from:
a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group, and
at least one substituent of the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the subtstituted divalent non-aromatic condensed polycyclic group, and the subtstituted divalent non-aromatic condensed heteropolycyclic group may be selected from:
   a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
   a C₁-C₆₀ alkyl group, a C₂-C₆₀ alkenyl group, a C₂-C₆₀ alkynyl group, and a C₁-C₆₀ alkoxy group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), and -B(Q₁₆)(Q₁₇);
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), and -B(Q₂₆)(Q₂₇); and
   -N(Q₃₁)(Q₃₂), -Si(Q₃₃)(Q₃₄)(Q₃₅), and -B(Q₃₆)(Q₃₇),
   wherein Q₁ to Q₇, Q₁₁ to Q₁₇, Q₂₁ to Q₂₇, and Q₃₁ to Q₃₇ may each be independently selected from a hydrogen, a C₁-C₆₀ alkyl group, a C₁-C₆₀ alkoxy group, a C₆-C₆₀ aryl group, a C₁-C₆₀ heteroaryl group, a monovalent non-aromatic condensed polycyclic group, and a monovalent non-aromatic condensed heteropolycyclic group.

In an exemplary embodiment, in Formulae 1 and 2, L₁₁ and L₂₁ may each be independently selected from a phenylene group, a pentalenylene group, an indenylene group, a naphthylene group, an azulenylene group, a heptalenylene group, an indacenylene group, an acenaphthylene group, a fluorenylene group, a spiro-fluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a pyrrolylene group, a thiophenylene group, a furanylene group, an imidazolylene group, a pyrazolylene group, a thiazolylene group, an isothiazolylene group, an oxazolylene group, an isoxazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzimidazolylene group, a benzofuranylene group, a benzothiophenylene group, an isobenzothiazolylene group, a benzoxazolylene group, an isobenzoxazolylene group, a triazolylene group, a tetrazolylene group, an oxadiazolylene group, a triazinylene group, a dibenzofuranylene group, a dibenzothiophenylene group, a benzocarbazolylene group, and a dibenzocarbazolylene group; and
a phenylene group, a pentalenylene group, an indenylene group, a naphthylene group, an azulenylene group, a heptalenylene group, an indacenylene group, an acenaphthylene group, a fluorenylene group, a spiro-fluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a pyrrolylene group, a thiophenylene group, a furanylene group, an imidazolylene group, a pyrazolylene group, a thiazolylene group, an isothiazolylene group, an oxazolylene group, an isoxazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzimidazolylene group, a benzofuranylene group, a benzothiophenylene group, an isobenzothiazolylene group, a benzoxazolylene group, an isobenzoxazolylene group, a triazolylene group, a tetrazolylene group, an oxadiazolylene group, a triazinylene group, a dibenzofuranylene group, a dibenzothiophenylene group, a benzocarbazolylene group, and a dibenzocarbazolylene group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, a thiadiazolyl group, and an imidazopyridinyl, but embodiments are not limited thereto.

In another exemplary embodiment, in Formulae 1 and 2, L₁₁ and L₂₁ may each be independently selected from a phenylene group, a naphthylene group, a fluorenylene group, a phenanthrenylene group, an anthracenylene group, a triphenylenylene group, a pyrrolylene group, a thiophenylene group, a furanylene group, a pyridinylene group, a pyrazinylene group, a pyrimidinylene group, an indolylene group, a quinolinylene group, an isoquinolinylene group, a benzoquinolinylene group, a naphthyridinylene group, a quinoxalinylene group, a quinazolinylene group, a cinnolinylene group, a carbazolylene group, a phenanthridinylene group, a benzimidazolylene group, a benzofuranylene group, a benzothiophenylene group, a triazolylene group, a dibenzofuranylene group, and a dibenzothiophenylene group; and

a phenylene group, a naphthylene group, a fluorenylene group, a phenanthrenylene group, an anthracenylene group, a triphenylenylene group, a pyrrolylene group, a thiophenylene group, a furanylene group, a pyridinylene group, a pyrazinylene group, a pyrimidinylene group, an indolylene group, a quinolinylene group, an isoquinolinylene group, a benzoquinolinylene group, a naphthyridinylene group, a quinoxalinylene group, a quinazolinylene group, a cinnolinylene group, a carbazolylene group, a phenanthridinylene group, a benzimidazolylene group, a benzofuranylene group, a benzothiophenylene group, a triazolylene group, a dibenzofuranylene group, and a dibenzothiophenylene group, each substituted with at least one of a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group, but embodiments are not limited thereto.

In another exemplary embodiment, in Formulae 1 and 2, L₁₁ and L₂₁ may each be independently a group represented by one of Formulae 3-1 to 3-18, but L₁₁ and L₂₁ are not limited thereto:

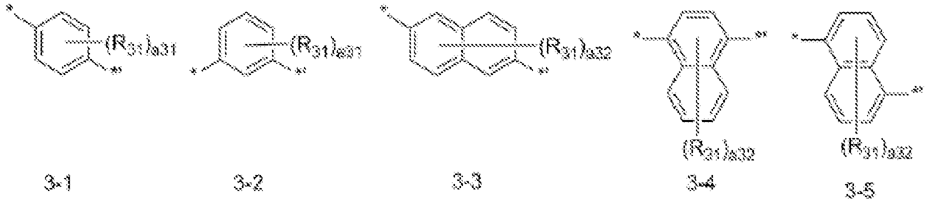

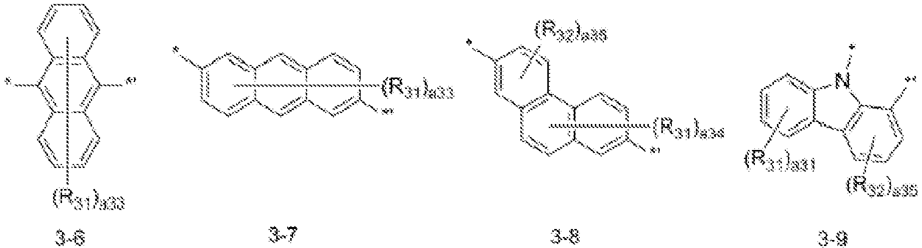

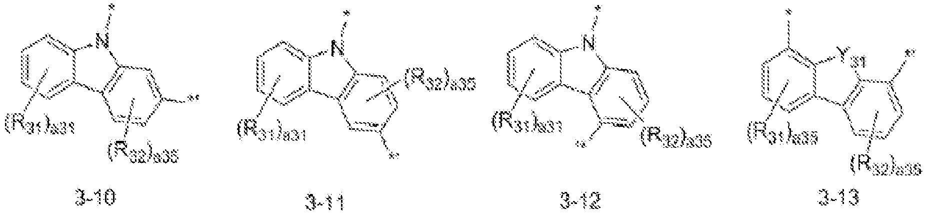

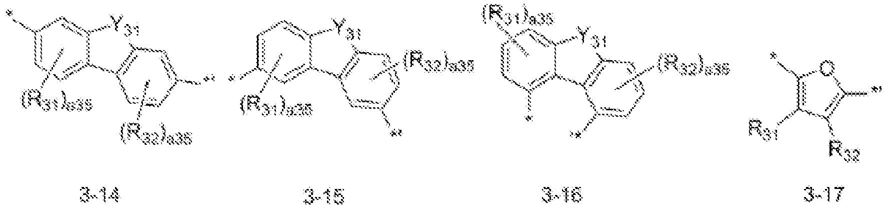

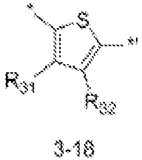

In Formulae 3-1 to 3-18,
Y₃₁ may be selected from C(R₃₃)(R₃₄), N(R₃₃), O, S, and Si(R₃₃)(R₃₄),
R₃₁ to R₃₄ may each be independently selected from a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group,
a31 may be selected from 1, 2, 3, and 4,
a32 may be selected from 1, 2, 3, 4, 5, and 6,
a33 may be selected from 1, 2, 3, 4, 5, 6, 7, and 8,
a34 may be selected from 1, 2, 3, 4, and 5,
a35 may be selected from 1, 2, and 3, and
* and *' may each independently indicate a binding site to an adjacent atom.

In another exemplary embodiment, in Formulae 1 and 2, L₁₁ and L₂₁ may each be independently a group represented by one of Formulae 4-1 to 4-36, but L₁₁ and L₂₁ are not limited thereto:

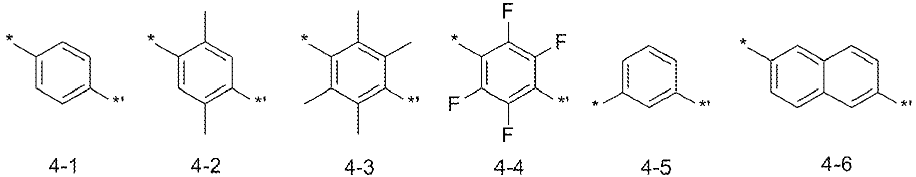

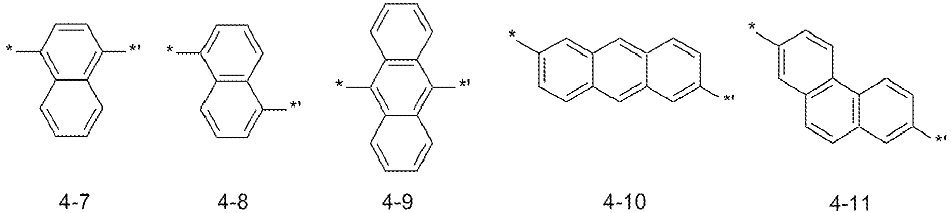

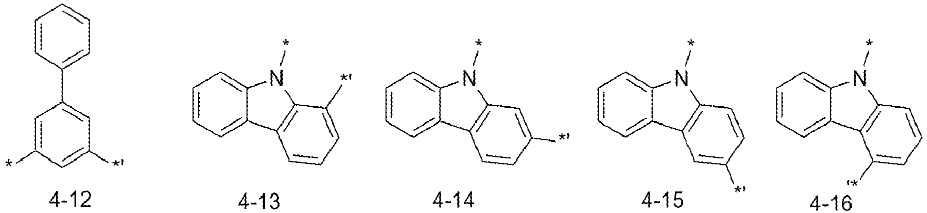

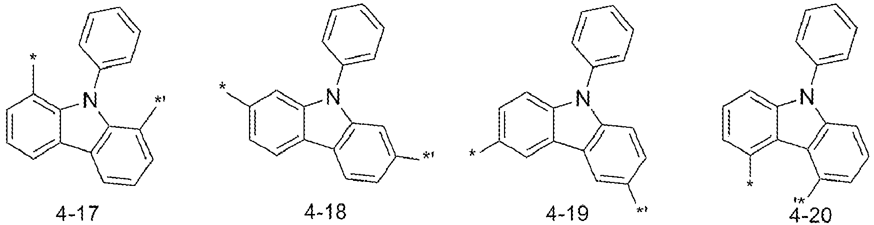

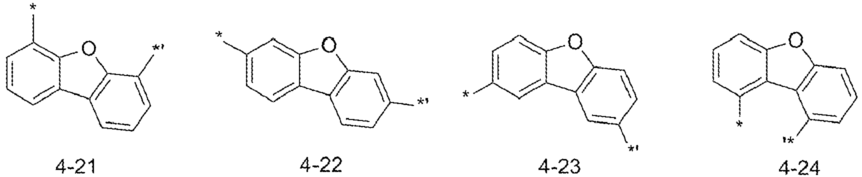

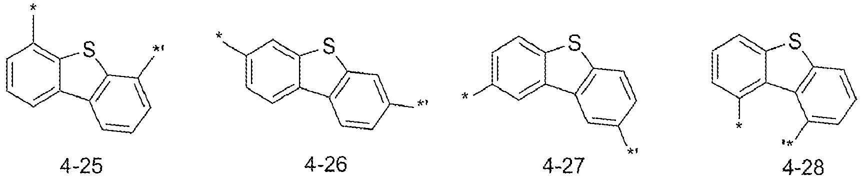

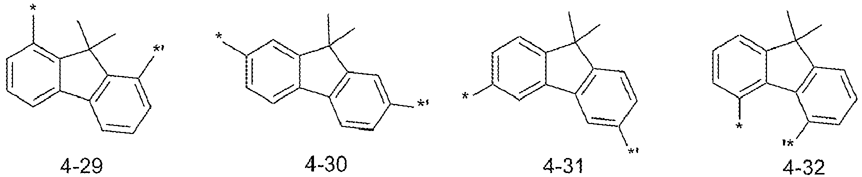

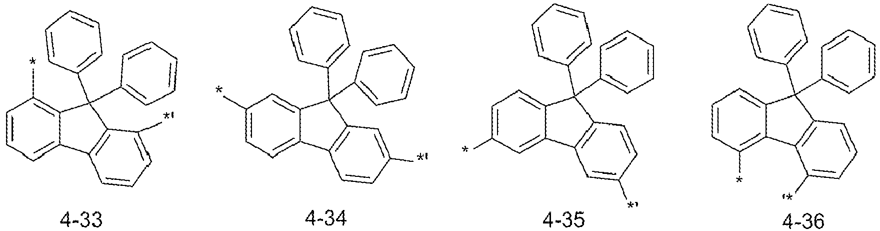

In Formulae 4-1 to 4-36,
* and *' may each independently indicate a binding stie to an adjacent atom.

In Formula 1, a11 indicates the number of L₁₁, and may be selected from 0, 1,2, and 3. For example, in Formula 1, a11 may be selected from 0 and 1, but a11 is not limited thereto. When a11 is 0, (L₁₁)ₐ₁₁ indicates a single bond. When a11 is 2 or more, a plurality of L₁₁s may be identical to or different from each other. For example, In Formulae 2, 8-2, and 9-2, descriptions of a21, a801, and a901 may each be independently as referred to in the description provided in connection with a11 and Formulae above.

In Formula 2, a21 may be selected from 0, 1,2, and 3. For example, in Formula 2, a21 may be selected from 0 and 1, but a21 is not limited thereto.

In Formulae 1 and 2, R₁₁ may be a hole-transporting group, and R₂₁ may be an electron-transporting group.

For example, in Formula 1, R₁₁ may be selected from:
a phenyl group, a naphthyl group, a fluorenyl group, a benzofluorenyl group, a dibenzofluorenyl group, a phenanthrenyl group, an anthracenyl group, a triphenylenyl group, a benzofuranyl group, a benzothiophenyl group, a dibenzofuranyl group, a dibenzothiophenyl group, a carbazolyl group, and -N(R₅₆)(R₅₇);
a phenyl group, a naphthyl group, a fluorenyl group, a benzofluorenyl group, a dibenzofluorenyl group, a phenanthrenyl group, an anthracenyl group, a triphenylenyl group, a benzofuranyl group, a benzothiophenyl group, a dibenzofuranyl group, a dibenzothiophenyl group, and a carbazolyl group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₆-C₂₀ aryl group, a C₁-C₂₀ heteroaryl group, a monovalent non-aromatic condensed polycyclic group, a monovalent non-aromatic condensed heteropolycyclic group, -N(Q₄₁)(Q₄₂), -Si(Q₄₃)(Q₄₄)(Q₄₅), and -B(Q₄₆)(Q₄₇); and
a phenyl group, a naphthyl group, a fluorenyl group, a benzofluorenyl group, a dibenzofluorenyl group, a phenanthrenyl group, an anthracenyl group, a triphenylenyl group, a benzofuranyl group, a benzothiophenyl group, a dibenzofuranyl group, a dibenzothiophenyl group, and a carbazolyl group, each substituted with at least one of a C₆-C₂₀ aryl group, a C₁-C₂₀ heteroaryl group, a monovalent non-aromatic condensed polycyclic group, and a monovalent non-aromatic condensed heteropolycyclic group that are each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, and a C₁-C₂₀ alkoxy group,
R₅₆ and R₅₇ may each be independently selected from:
   a C₁-C₂₀ alkyl group, a C₆-C₂₀ aryl group, a C₁-C₂₀ heteroaryl group, a monovalent non-aromatic condensed polycyclic group, and a monovalent non-aromatic condensed heteropolycyclic group; and
   a C₁-C₂₀ alkyl group, a C₆-C₂₀ aryl group, a C₁-C₂₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₆-C₂₀ aryl group, a C₁-C₂₀ heteroaryl group, a monovalent non-aromatic condensed polycyclic group, and a monovalent non-aromatic condensed heteropolycyclic group,
   wherein Q₄₁ to Q₄₇ may each be independently selected from a C₁-C₂₀ alkyl group, a C₆-C₂₀ aryl group, a C₁-C₂₀ heteroaryl group, a monovalent non-aromatic condensed polycyclic group, and a monovalent non-aromatic condensed heteropolycyclic group, but embodiments are not limited thereto.

In another exemplary embodiment, in Formula 1, R₁₁ may be selected from groups represented by Formulae 5-1 to 5-13, but R₁₁ is not limited thereto:

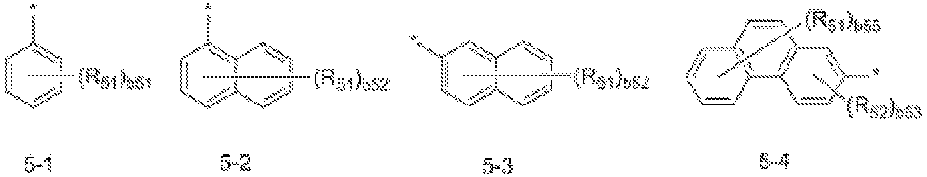

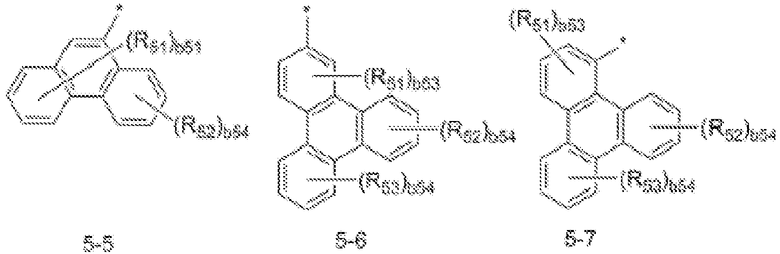

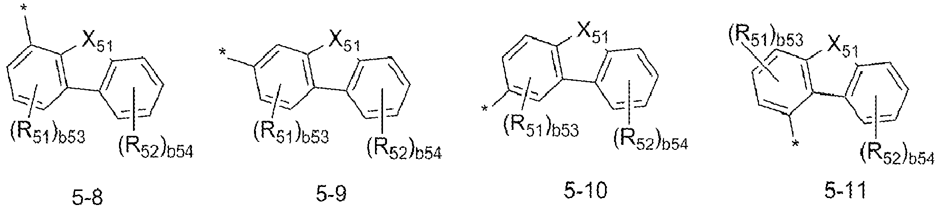

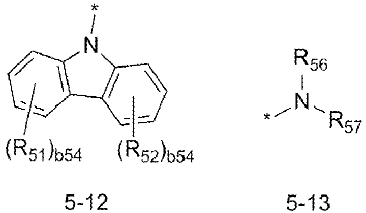

In Formulae 5-1 to 5-13,
X₅₁ may be selected from O, S, N(R₅₄), and C(R₅₄)(R₅₅),
R₅₁ to R₅₅ may each be independently selected from:
a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₆-C₂₀ aryl group, a C₁-C₂₀ heteroaryl group, a monovalent non-aromatic condensed polycyclic group, a monovalent non-aromatic condensed heteropolycyclic group, -N(Q₄₁)(Q₄₂), -Si(Q₄₃)(Q₄₄)(Q₄₅), and - B(Q₄₆)(Q₄₇); and
a C₆-C₂₀ aryl group, a C₁-C₂₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, and a C₁-C₂₀ alkoxy group,
R₅₆ and R₅₇ may each be independently selected from:
   a C₁-C₂₀ alkyl group, a C₆-C₂₀ aryl group, a C₁-C₂₀ heteroaryl group, a monovalent non-aromatic condensed polycyclic group, and a monovalent non-aromatic condensed heteropolycyclic group; and
   a C₁-C₂₀ alkyl group, a C₆-C₂₀ aryl group, a C₁-C₂₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₆-C₂₀ aryl group, a C₁-C₂₀ heteroaryl group, a monovalent non-aromatic condensed polycyclic group, and a monovalent non-aromatic condensed heteropolycyclic group,
   wherein Q₄₁ to Q₄₇ may each be independently selected from a C₁-C₂₀ alkyl group, a C₆-C₂₀ aryl group, a C₁-C₂₀ heteroaryl group, a monovalent non-aromatic condensed polycyclic group, and a monovalent non-aromatic condensed heteropolycyclic group,
   b51 may be selected from 1, 2, 3, 4, and 5,
   b52 may be selected from 1, 2, 3, 4, 5, 6, and 7,
   b53 may be selected from 1, 2, and 3,
   b54 may be selected from 1, 2, 3, and 4,
   b55 may be selected from 1, 2, 3, 4, 5, and 6, and
   * indicates a binding site to an adjacent atom.

In another exemplary embodiment, in Formula 1, R₁₁ may be selected from groups represented by Formulae 6-1 to 6-59, but R₁₁ is not limited thereto:

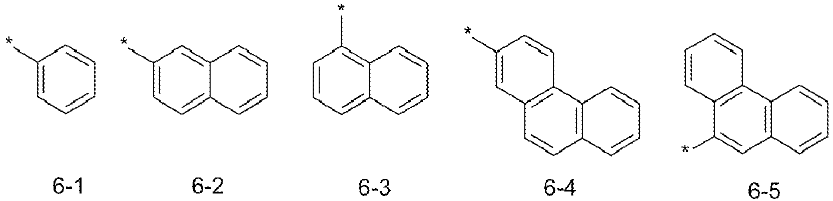

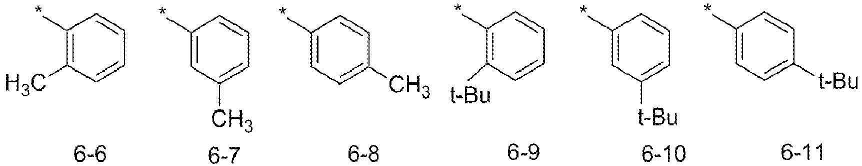

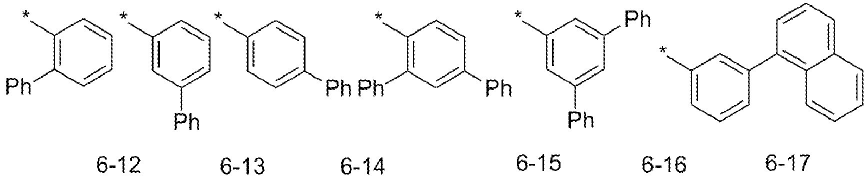

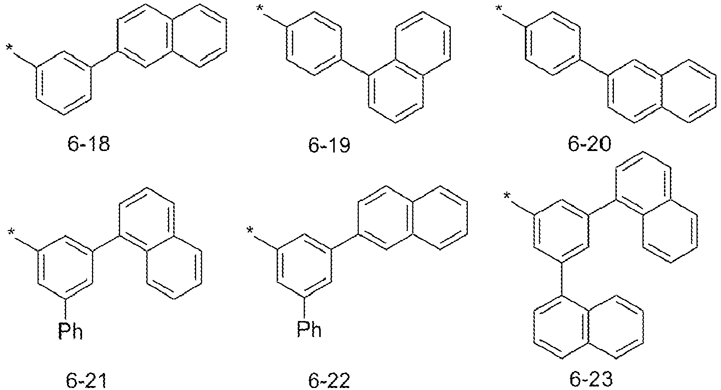

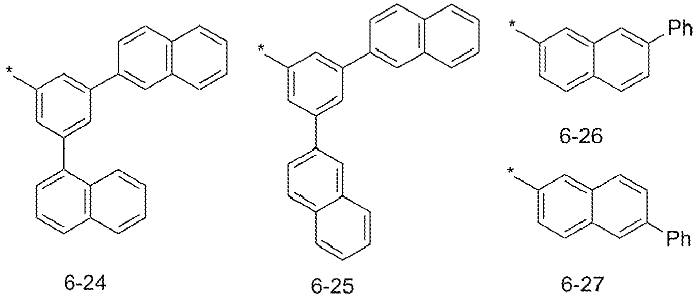

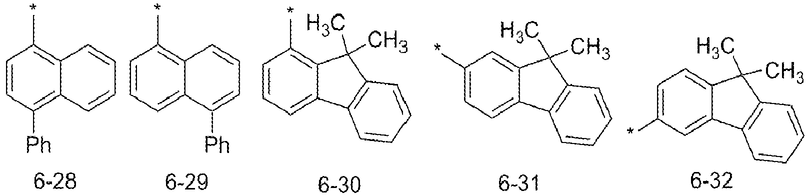

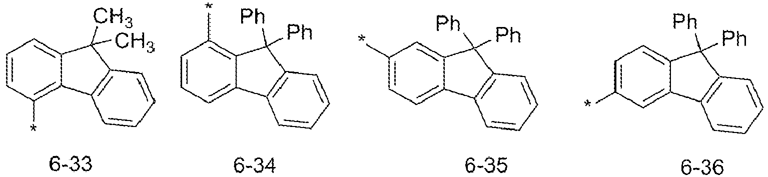

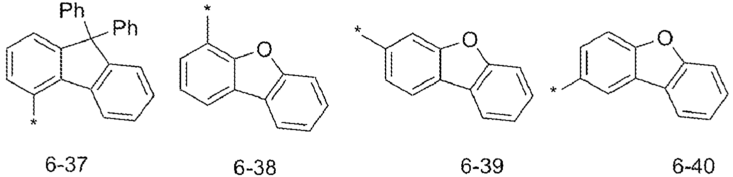

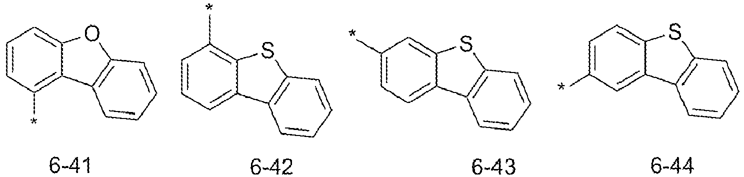

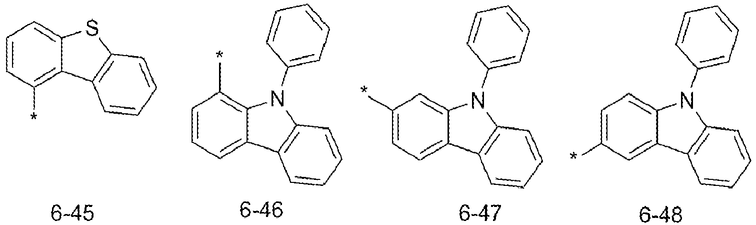

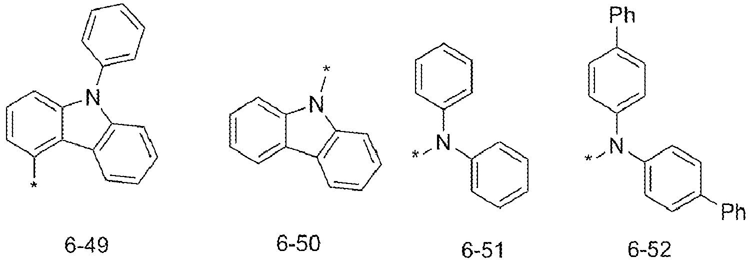

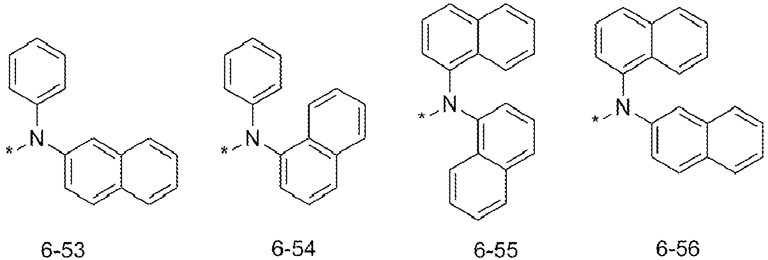

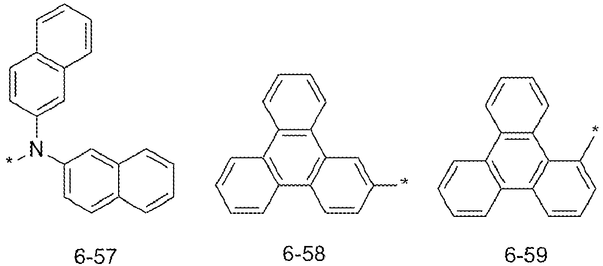

In Formulae 6-1 to 6-59,
t-Bu indicates a tert-butyl group,
Ph indicates a phenyl group, and
* indicate a binding site to an adjacent atom.

In an exemplary embodiment, in Formula 2, R₂₁ may be selected from:
a pyrrolyl group, an indolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a quinolinyl group, an isoquinolinyl group, a benzoquinolinyl group, a naphthyridinyl group, a quinoxalinyl group, a quinazolinyl group, a phenanthridinyl group, an acridinyl group, a phenanthrolinyl group, a phenazinyl group, a benzothiazolyl group, a benzoxazolyl group, a benzimidazolyl group, a triazolyl group, a triazinyl group, an imidazopyridinyl group, an imidazopyrimidinyl group, a pyridobenzofuranyl group, a pyrimidobenzofuranyl group, a pyridobenzothiophenyl group, and a pyrimidobenzothiophenyl group;
a pyrrolyl group, an indolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a quinolinyl group, an isoquinolinyl group, a benzoquinolinyl group, a naphthyridinyl group, a quinoxalinyl group, a quinazolinyl group, a phenanthridinyl group, an acridinyl group, a phenanthrolinyl group, a phenazinyl group, a benzothiazolyl group, a benzoxazolyl group, a benzimidazolyl group, a triazolyl group, a triazinyl group, an imidazopyridinyl group, an imidazopyrimidinyl group, a pyridobenzofuranyl group, a pyrimidobenzofuranyl group, a pyridobenzothiophenyl group, and a pyrimidobenzothiophenyl group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₆-C₂₀ aryl group, a C₁-C₂₀ heteroaryl group, a monovalent non-aromatic condensed polycyclic group, a monovalent non-aromatic condensed heteropolycyclic group, - N(Q₄₁)(Q₄₂), -Si(Q₄₃)(Q₄₄)(Q₄₅), and -B(Q₄₆)(Q₄₇); and
a pyrrolyl group, an indolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a quinolinyl group, an isoquinolinyl group, a benzoquinolinyl group, a naphthyridinyl group, a quinoxalinyl group, a quinazolinyl group, a phenanthridinyl group, an acridinyl group, a phenanthrolinyl group, a phenazinyl group, a benzothiazolyl group, a benzoxazolyl group, a benzimidazolyl group, a triazolyl group, a triazinyl group, an imidazopyridinyl group, an imidazopyrimidinyl group, a pyridobenzofuranyl group, a pyrimidobenzofuranyl group, a pyridobenzothiophenyl group, and a pyrimidobenzothiophenyl group, each substituted with at least one of a C₆-C₂₀ aryl group, a C₁-C₂₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, and a C₁-C₂₀ alkoxy group,
wherein Q₄₁ to Q₄₇ may each be independently selected from a C₁-C₂₀ alkyl group, a C₆-C₂₀ aryl group, a C₁-C₂₀ heteroaryl group, a monovalent non-aromatic condensed polycyclic group, and a monovalent non-aromatic condensed heteropolycyclic group, but embodiments are not limited thereto.

In another exemplary embodiment, in Formula 2, R₂₁ may be selected from groups represented by Formulae 5-21 to 5-79, but R₂₁ are not limited thereto:

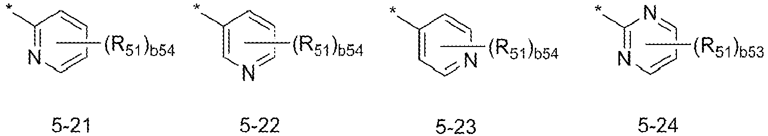

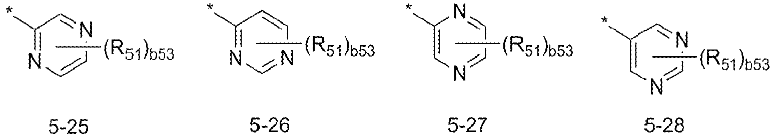

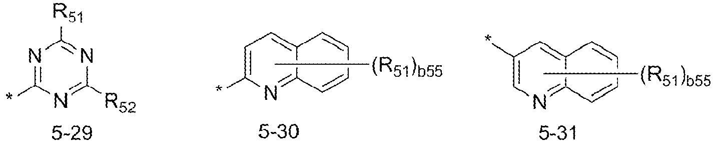

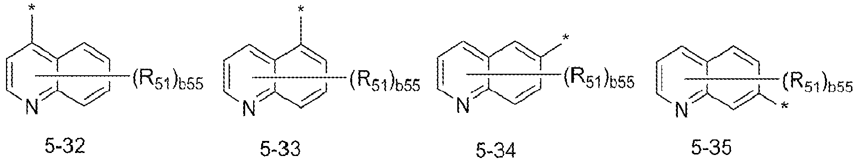

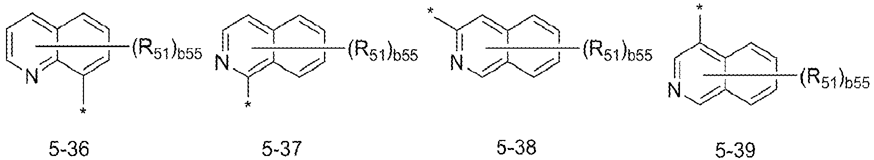

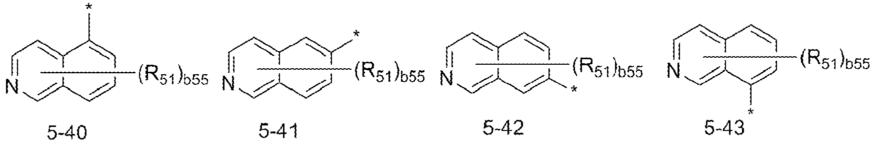

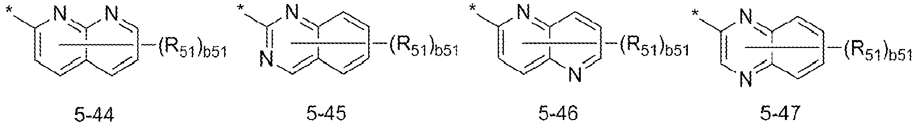

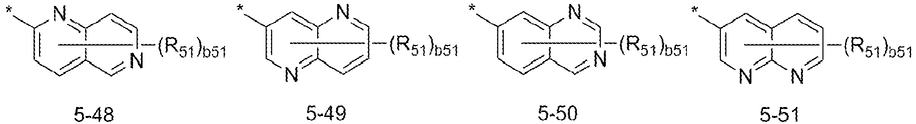

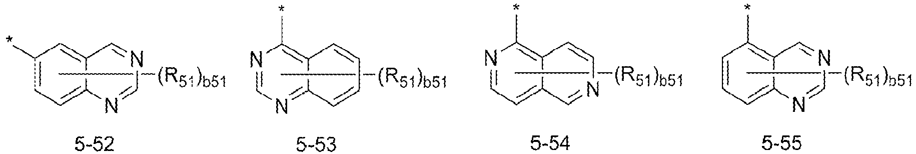

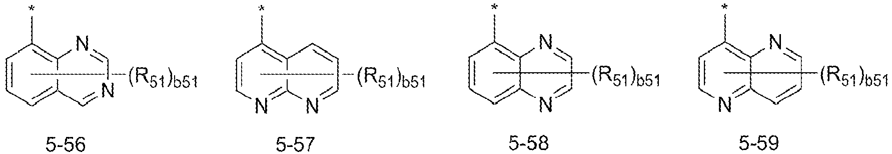

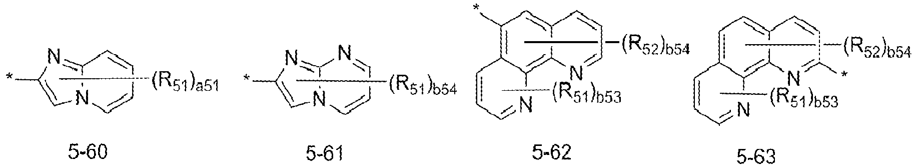

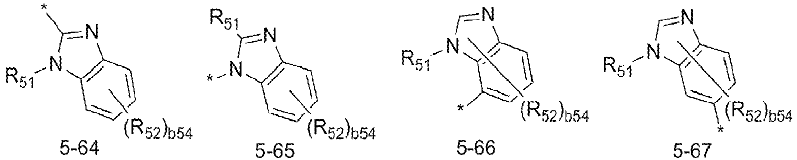

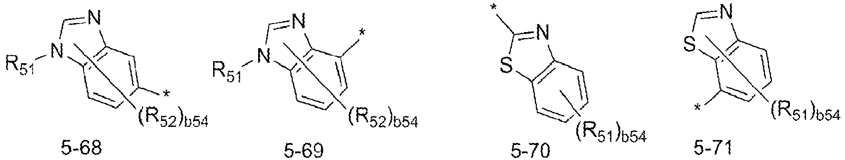

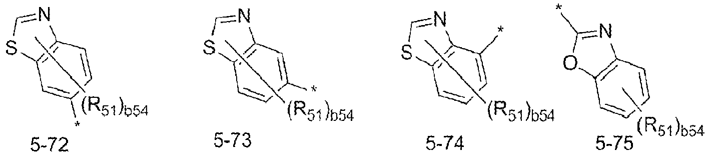

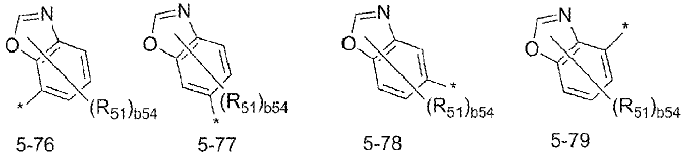

In Formulae 5-21 to 5-79,
R₅₁ and R₅₂ may each be independently selected from:
a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₆-C₂₀ aryl group, a C₁-C₂₀ heteroaryl group, a monovalent non-aromatic condensed polycyclic group, a monovalent non-aromatic condensed heteropolycyclic group, -N(Q₄₁)(Q₄₂), -Si(Q₄₃)(Q₄₄)(Q₄₅), and - B(Q₄₆)(Q₄₇); and
a C₆-C₂₀ aryl group, a C₁-C₂₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, and a C₁-C₂₀ alkoxy group;
Q₄₁ to Q₄₇ may each be independently selected from a C₁-C₂₀ alkyl group, a C₆-C₂₀ aryl group, a C₁-C₂₀ heteroaryl group, a monovalent non-aromatic condensed polycyclic group, and a monovalent non-aromatic condensed heteropolycyclic group;
b51 may be selected from 1, 2, 3, 4, and 5,
b53 may be selected from selected from 1, 2, and 3,
b54 may be selected from selected from 1,2,3, and 4,
b55 may be selected from 1, 2, 3, 4, 5, and 6, and
* indicates a binding site to an adjacent atom.

In another exemplary embodiment, in Formula 2, R₂₁ may be selected from groups represented by Formulae 6-61 to 6-219, but R₂₁ is not limited thereto:

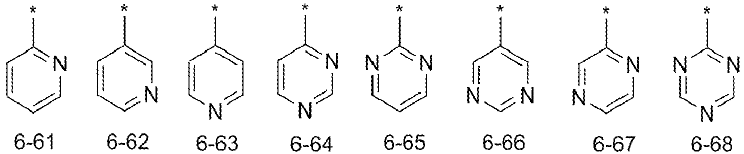

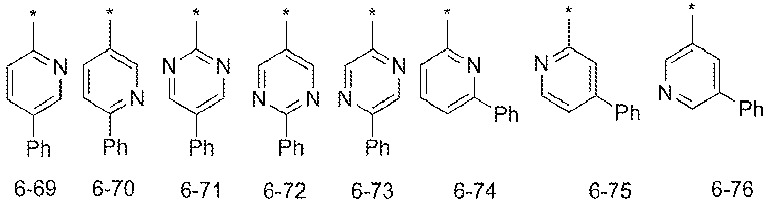

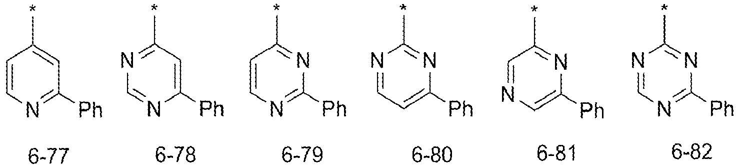

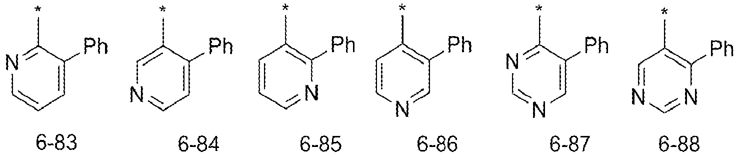

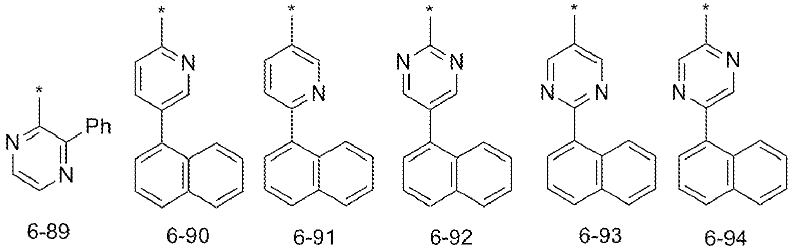

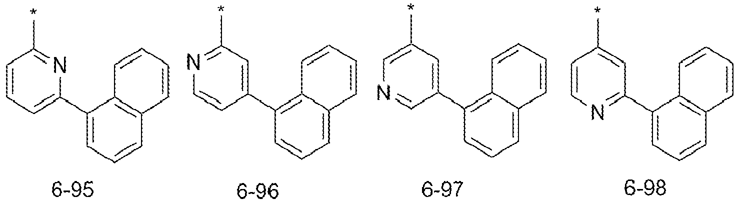

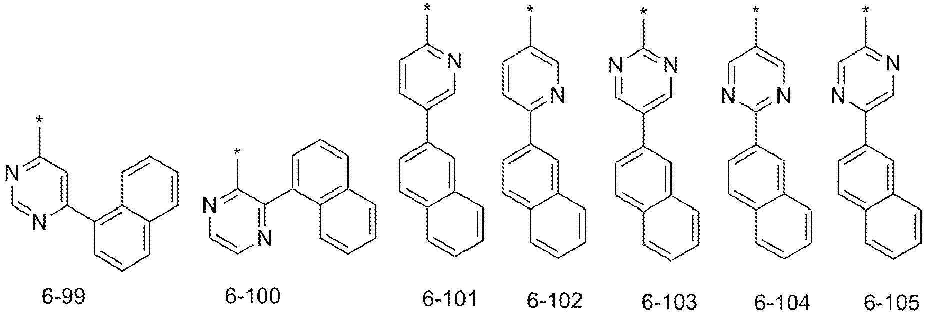

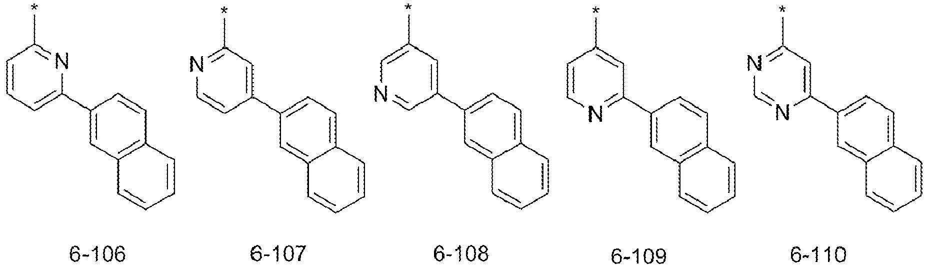

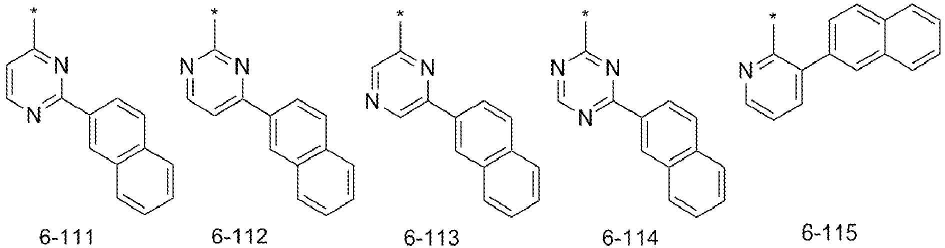

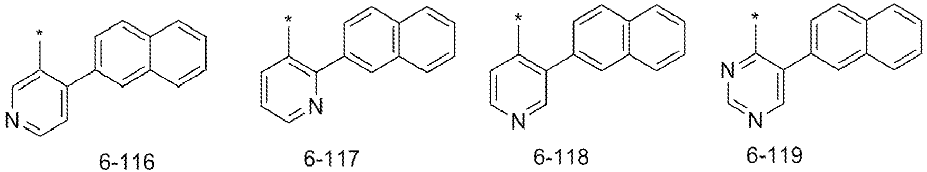

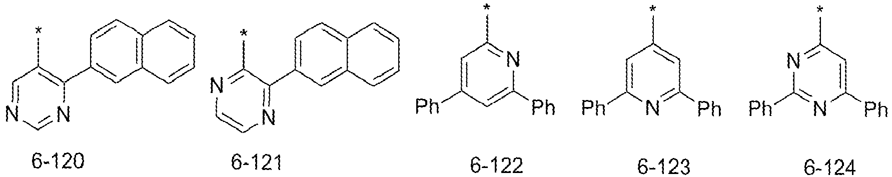

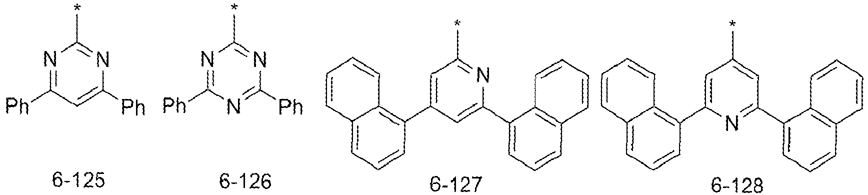

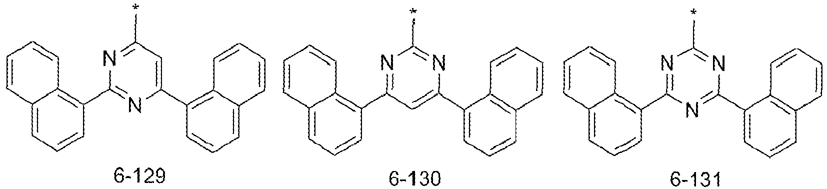

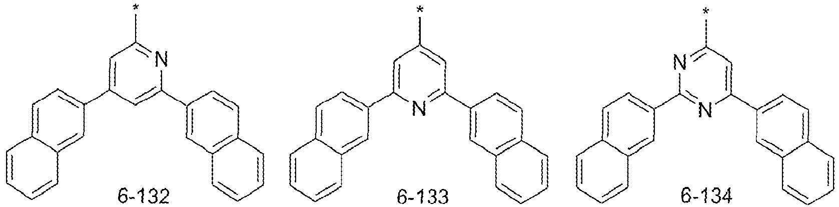

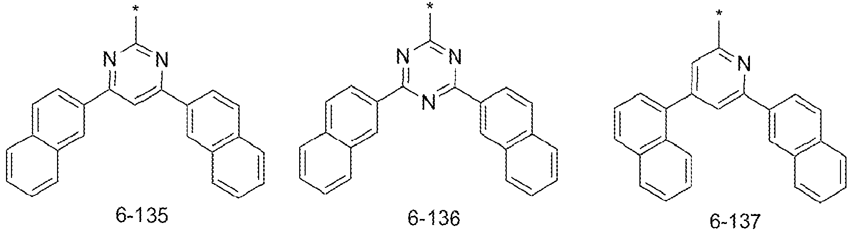

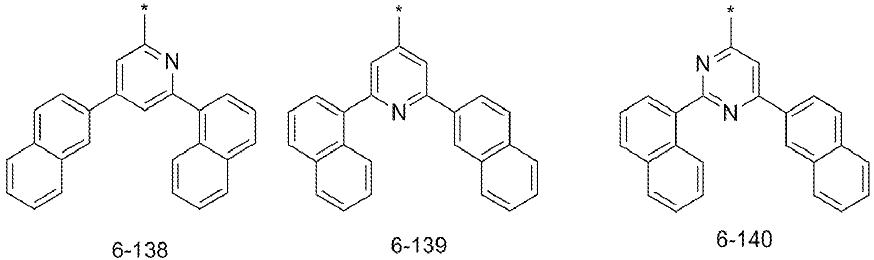

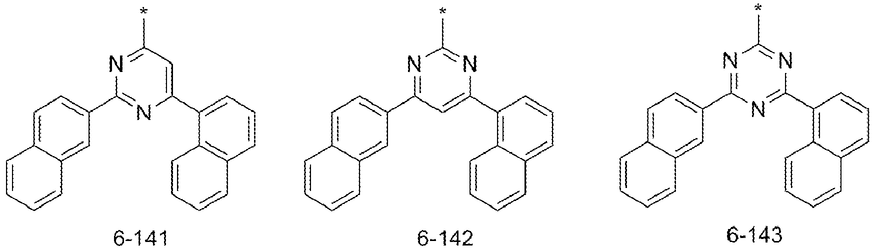

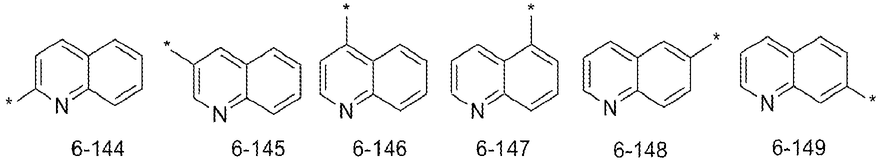

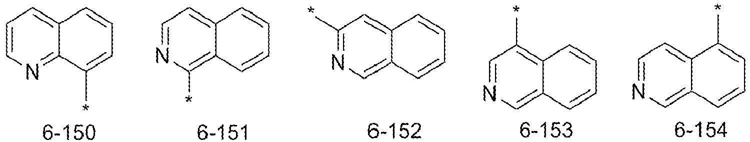

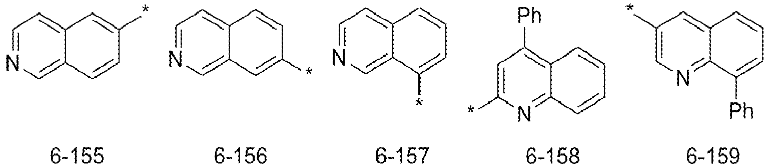

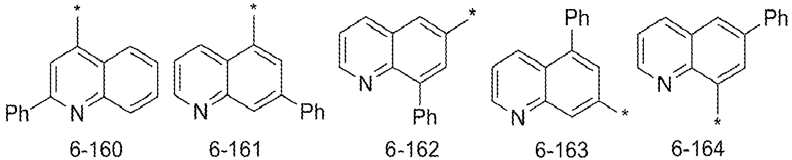

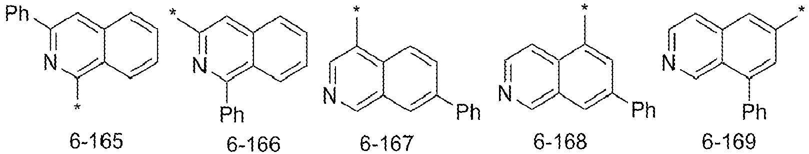

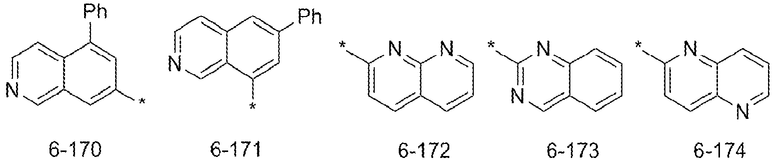

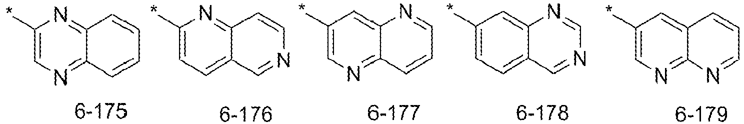

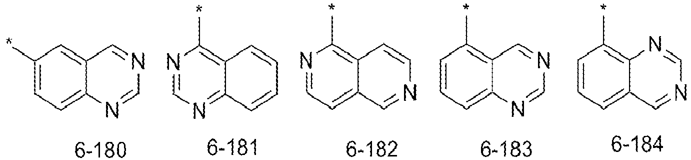

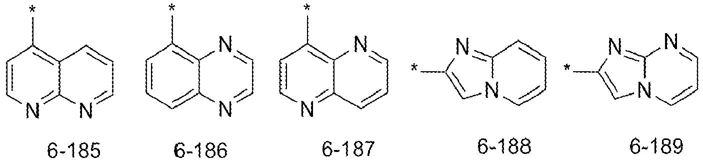

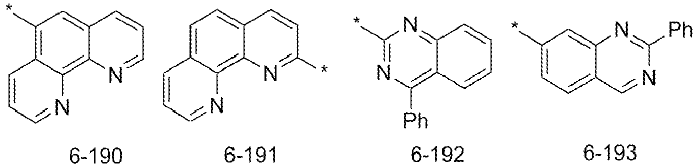

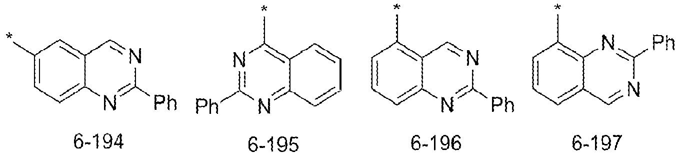

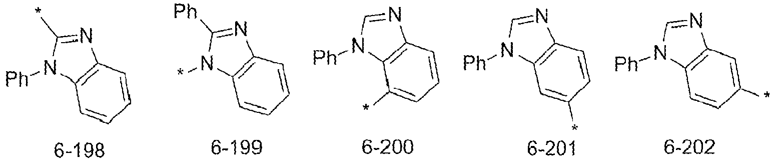

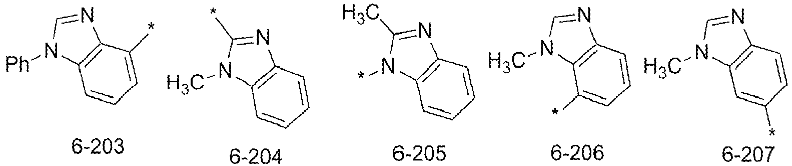

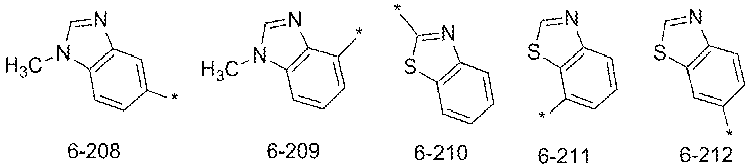

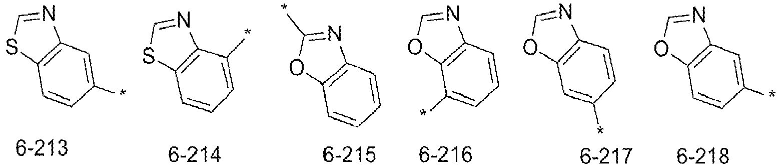

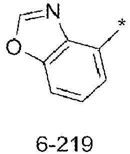

In Formulae 6-61 to 6-219,
Ph indicates a phenyl group, and
* indicates a binding site to an adjacent atom.

In Formula 1, b11 indicates the number of R₁₁, and may be selected from 1, 2, and 3. For example, in Formula 1, b11 may be selected from 1 and 2, but b11 is not limited thereto. When b11 is 2 or more, a plurality of R₁₁s may be identical to or different from each other.

In Formula 2, b21 indicates the number of R₂₁, and may be selected from 1, 2, and 3. For example, in Formula 1, b21 may be selected from 1 and 2, but b21 is not limited thereto. When b21 is 2 or more, a plurality of R₂₁s may be identical to or different from each other.

In Formula 1, n11 indicates the number of *-[(L₁₁)ₐ₁₁-(R₁₁)_{b11}], and may be selected from 1, 2, 3, and 4. For example, in Formula 1, n11 may be selected from 1 and 2, but n11 is not limited thereto. When n11 is 2 or more, a plurality of *-[(L₁₁)ₐ₁₁-(R₁₁)_{b11}]s may be identical to or different from each other.

In Formula 2, n21 indicates the number of *-[(L₂₁)ₐ₂₁-(R₂₁)_{b21}], and may be selected from 1, 2, 3, and 4. For example, in Formula 2, n21 may be selected from 1 and 2, but n21 is not limited thereto. When n21 is 2 or more, a plurality of *-[(L₂₁)ₐ₂₁-(R₂₁)_{b21}]s may be identical to or different from each other.

For example, the first host may be represented by one of Formulae 1-1 to 1-14 and 2-1 to 2-14, but the first host is not limited thereto:

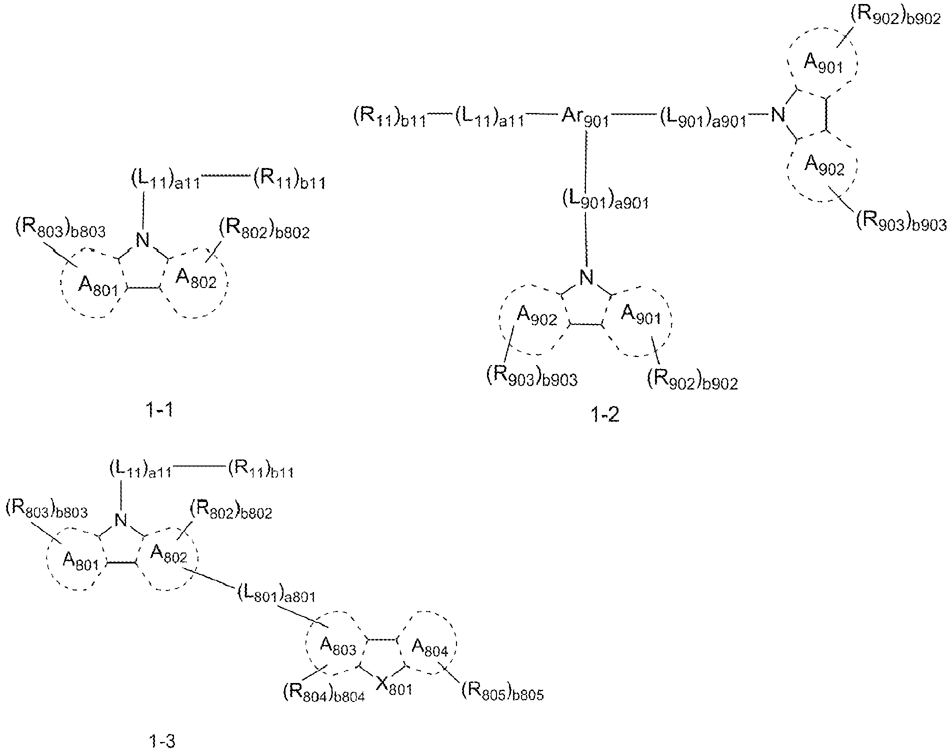

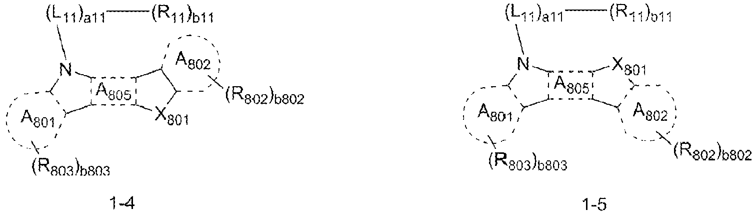

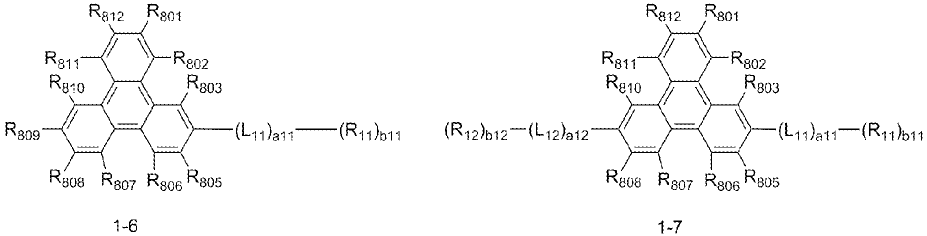

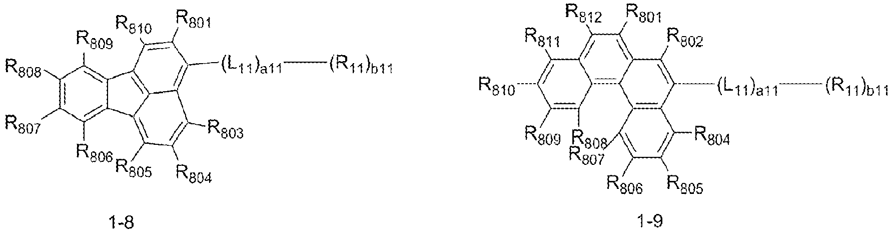

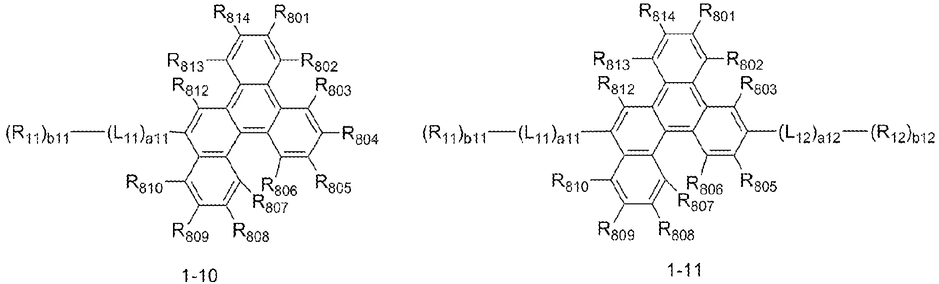

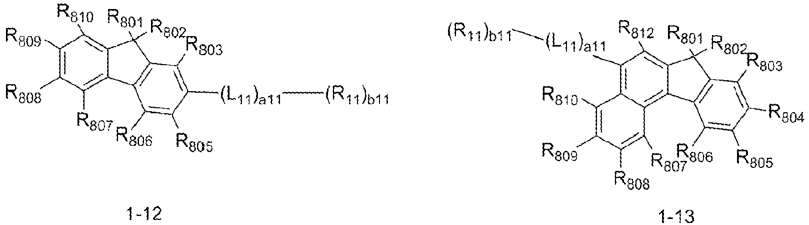

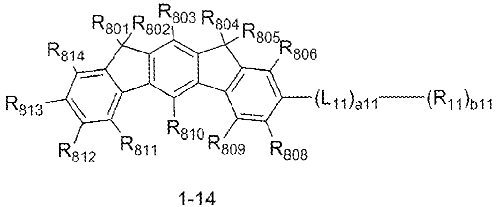

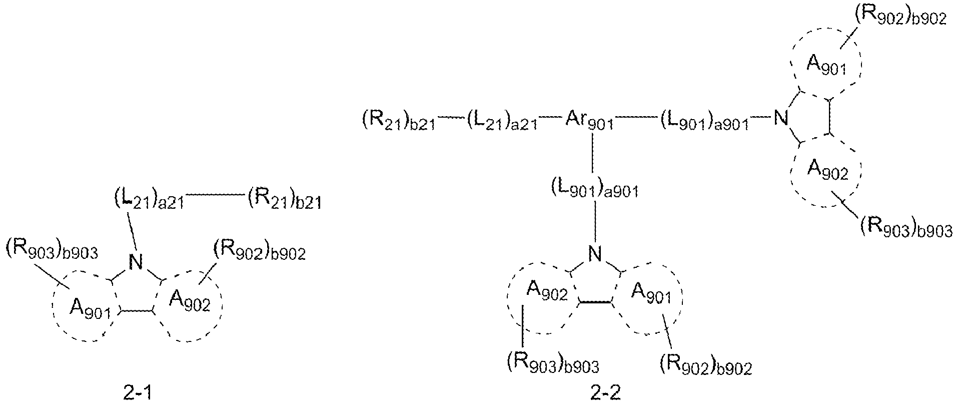

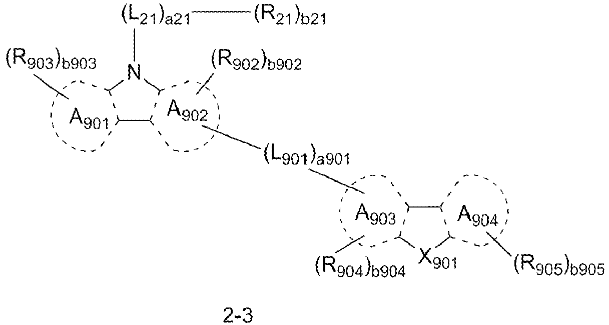

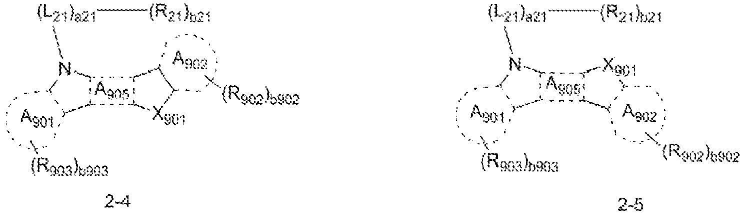

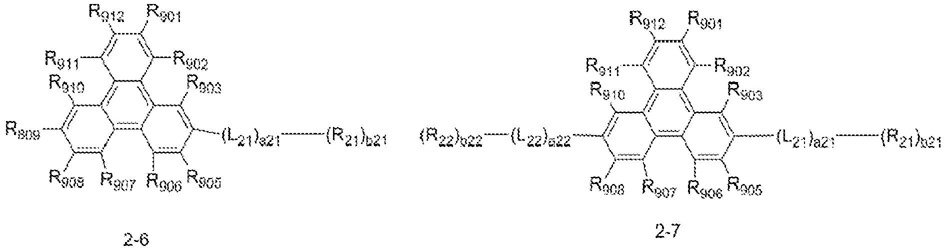

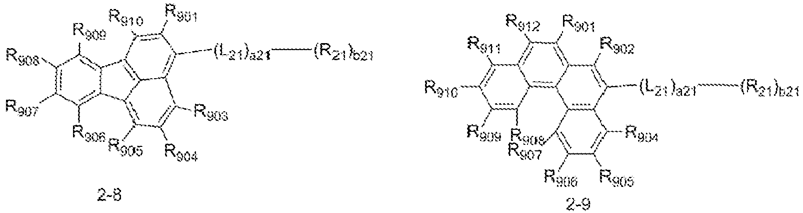

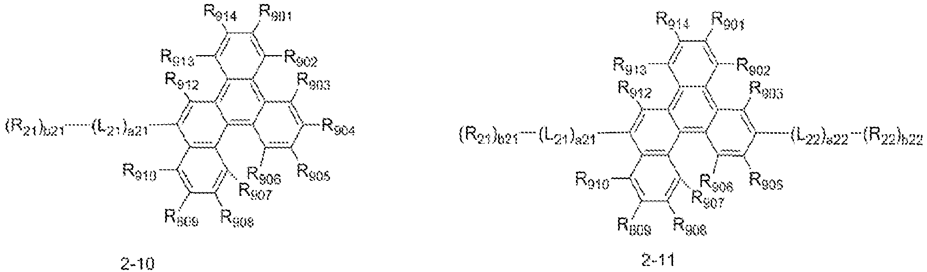

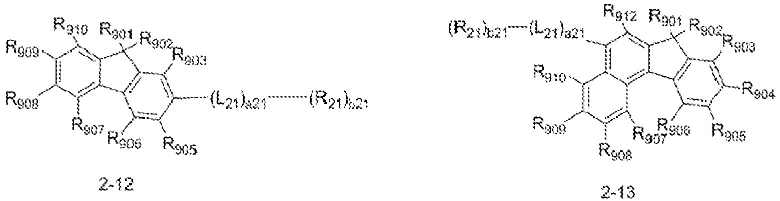

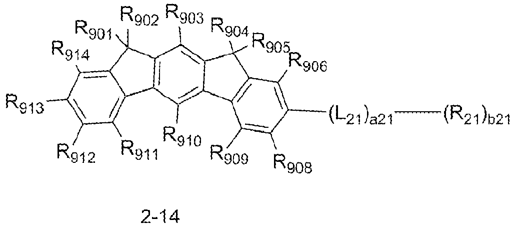

In Formulae 1-1 to 1-14 and 2-1 to 2-14,
descriptions of L₁₁, a11, R₁₁, b11, L₈₀₁, a801, A₈₀₁ to A₈₀₅, X₈₀₁, R₈₀₁ to R₈₁₄, and b801 to b805 may each be independently as referred to in the descriptions provided above, a description of L₁₂ may be as referred to in the description provided in connection with L₁₁ in Formula 1, a description of a12 may be as referred to in the description provided in connection with a11 in Formula 1, a description of R₁₂ may be as referred to in the description provided in connection with R₁₁ in Formula 1, and a description of b21 may be as referred to in the description provided in connection with b11 in Formula 1, and
descriptions of L₂₁, a21, R₂₁, b21, L₉₀₁, a901, A₉₀₁ to A₉₀₅, X₉₀₁, R₉₀₁ to R₉₁₂, and b901 to b905 may each be independently as referred to in the description provided in connection with those in Formula 2, a description of L₂₂ may be as referred to in the description provided in connection with L₂₁ in Formula 2, a description of a22 may be as referred to in the description provided in connection with a21 in Formula 2, a description of R₂₂ may be as referred to in the description provided in connection with R₂₁ in Formula 2, and a description of b22 may be as referred to in the description provided in connection with b21 in Formula 2.

In another exemplary embodiment, the first host may be selected from compounds below, but the first host is not limited thereto:

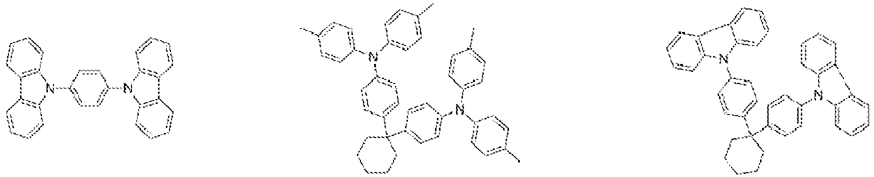

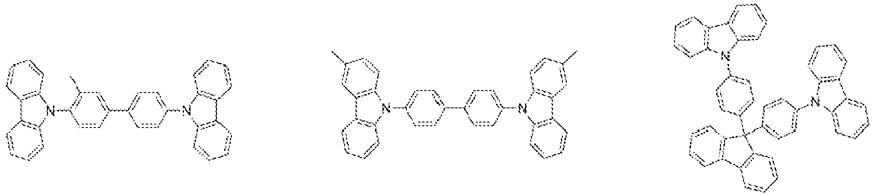

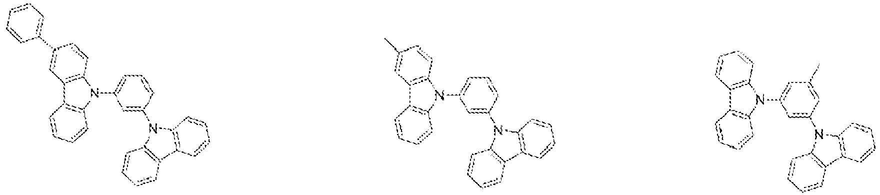

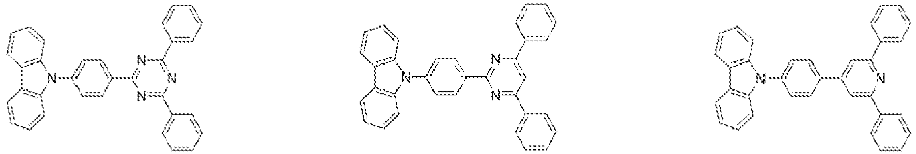

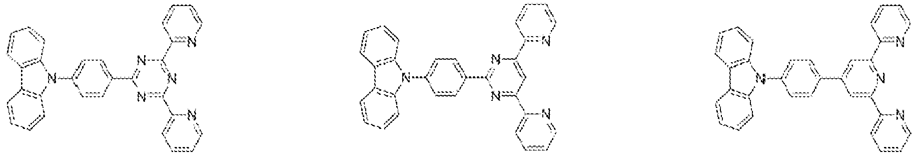

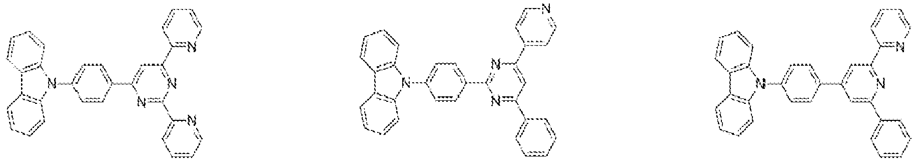

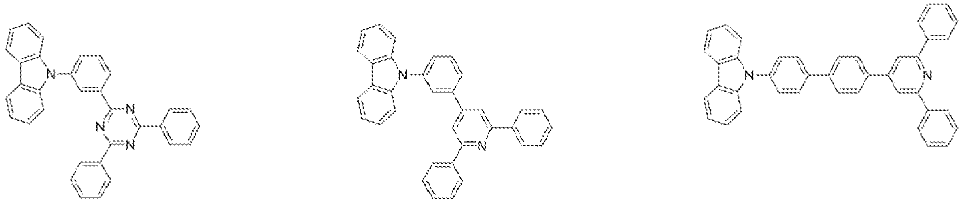

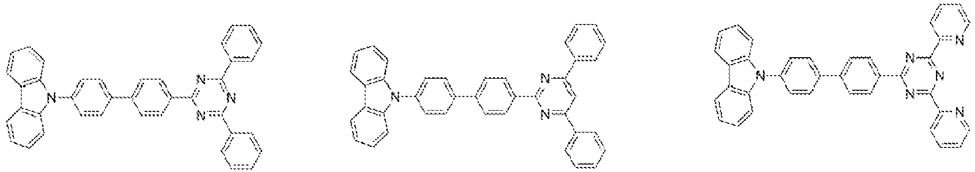

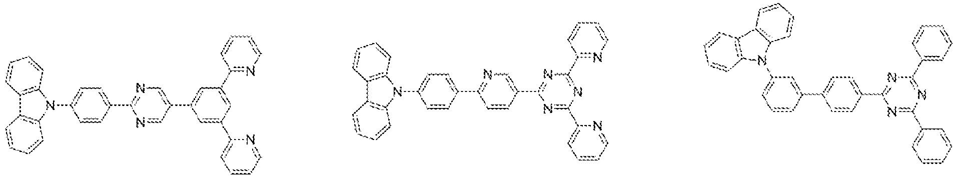

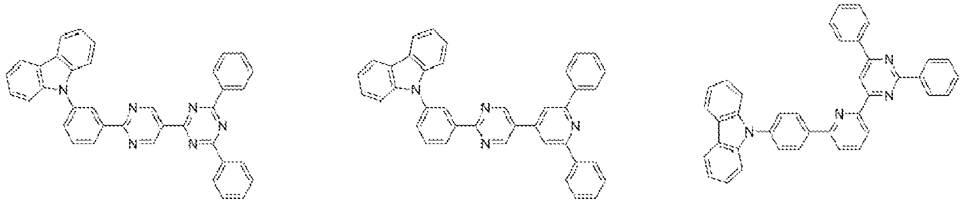

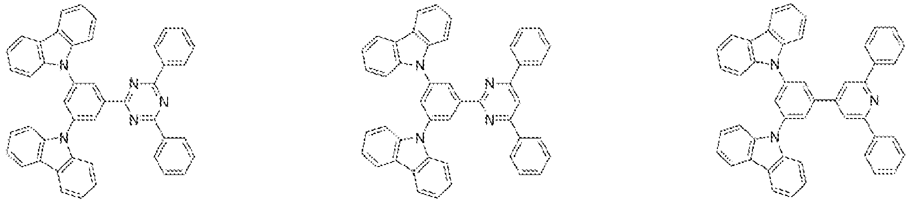

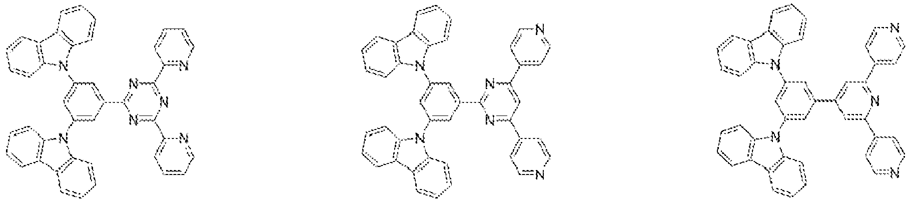

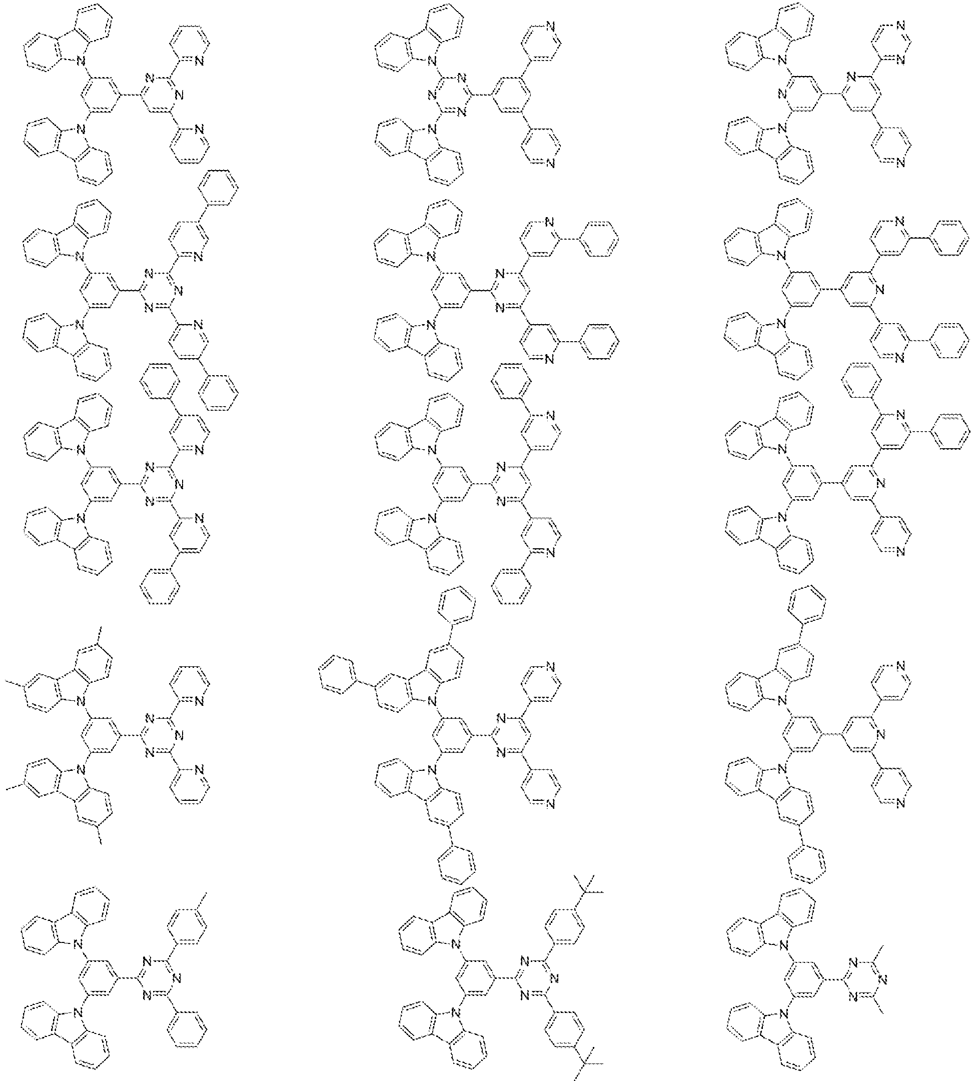

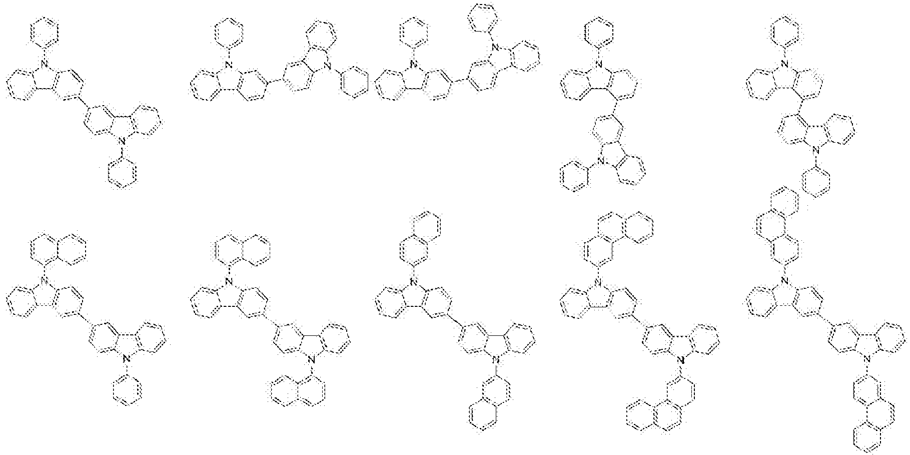

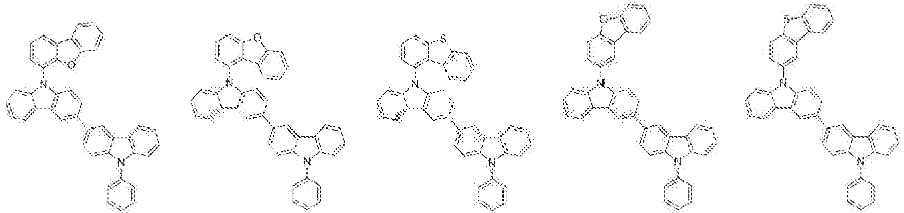

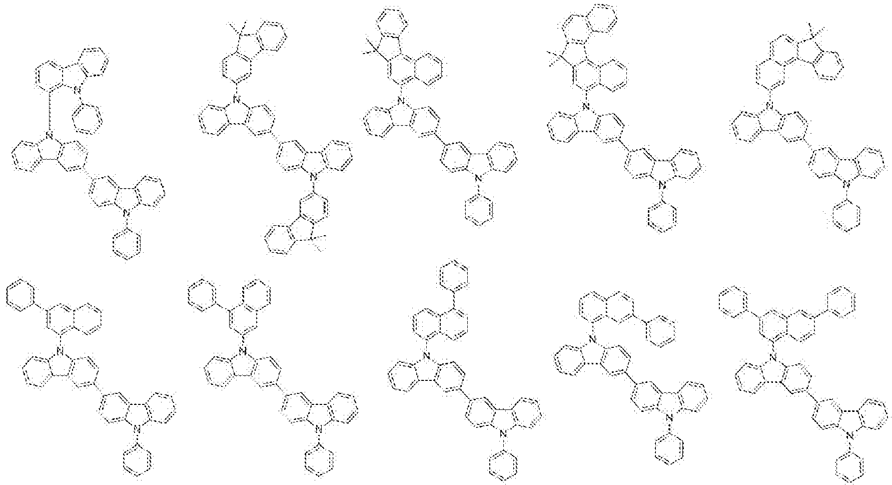

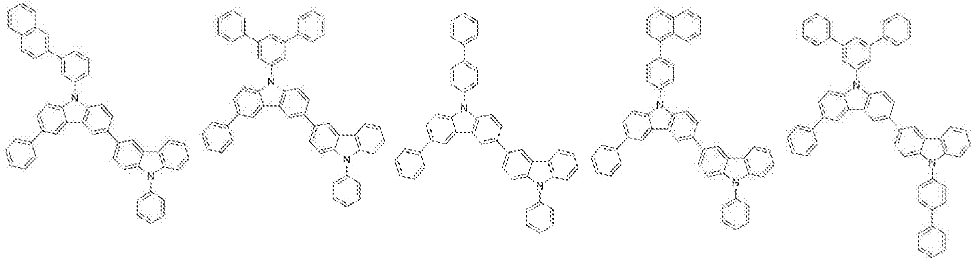

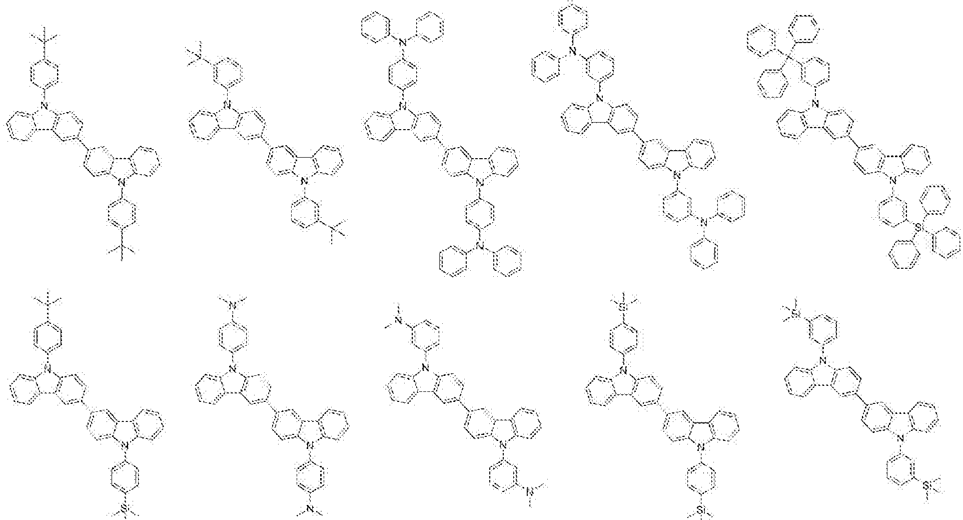

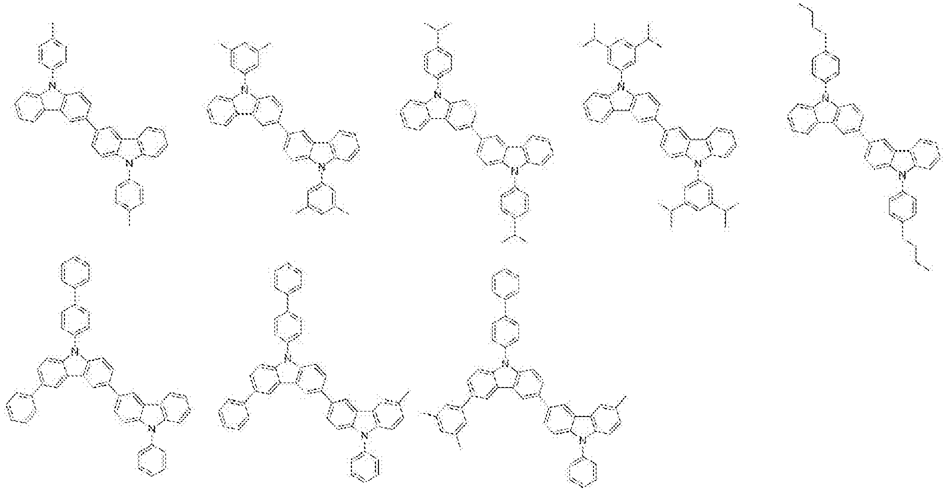

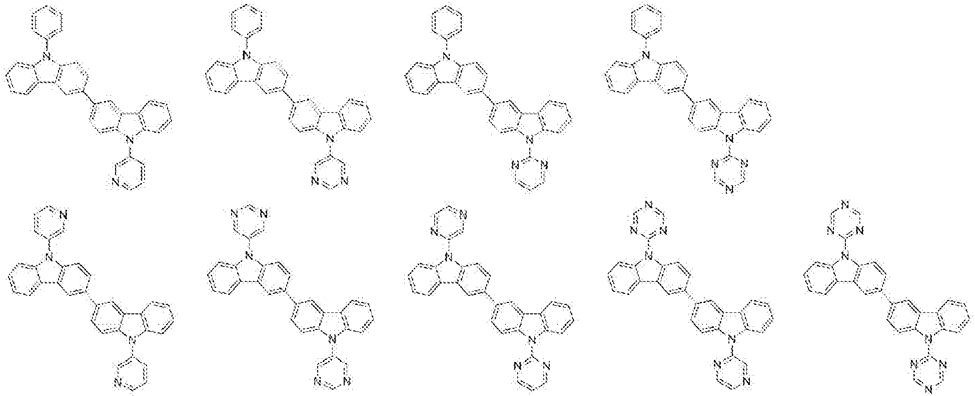

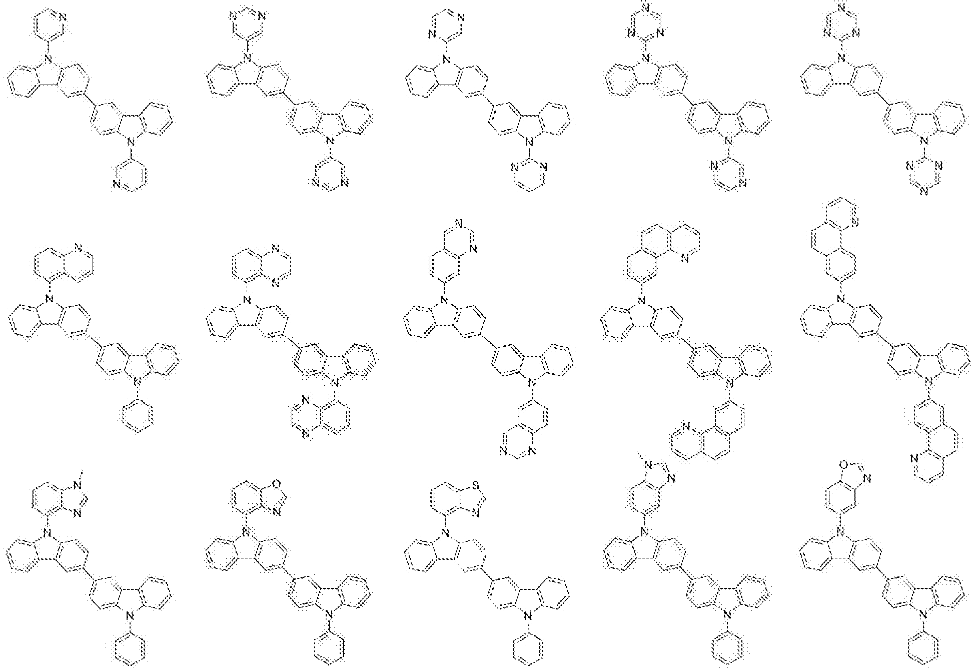

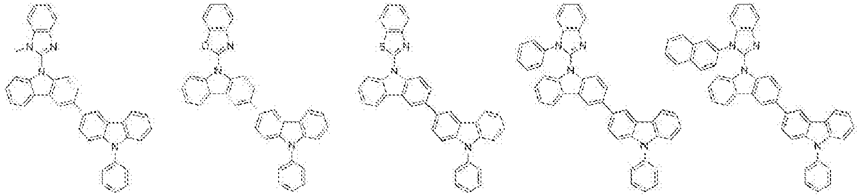

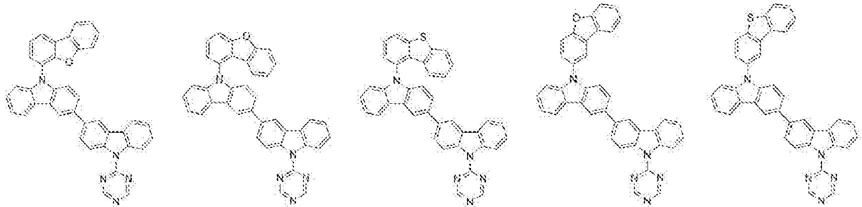

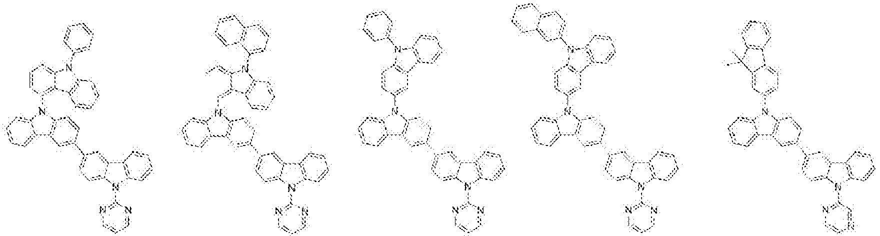

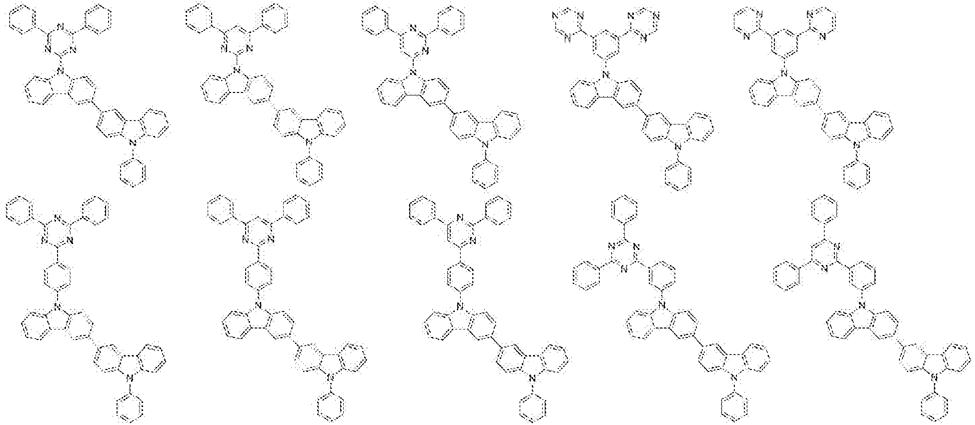

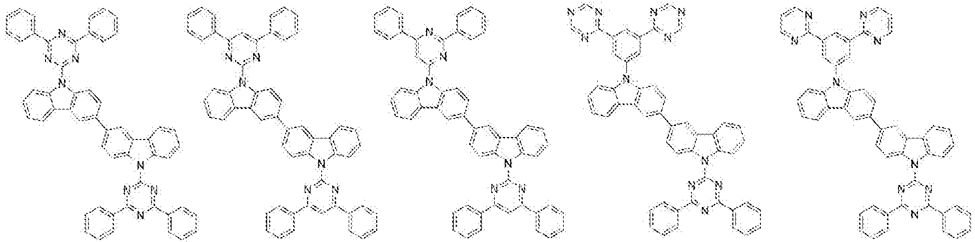

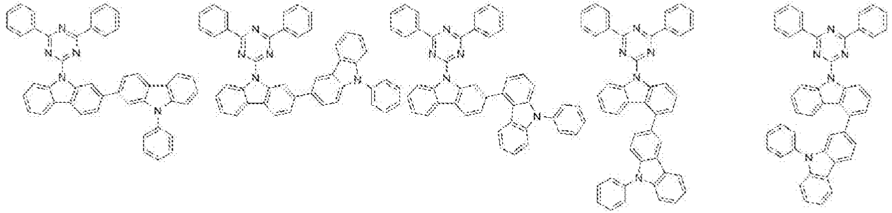

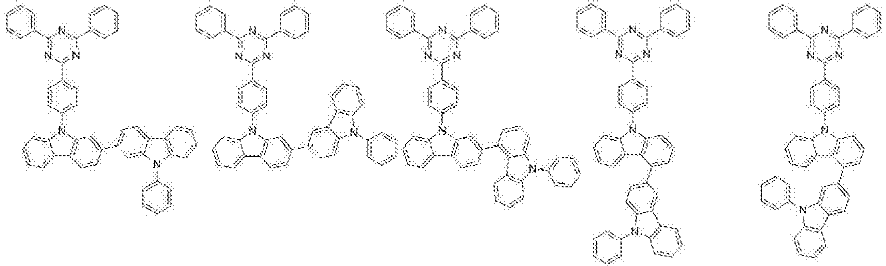

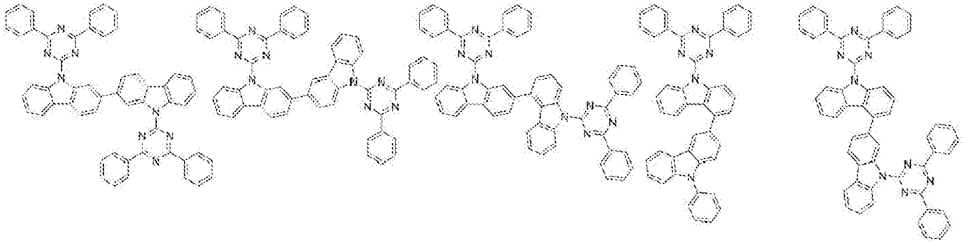

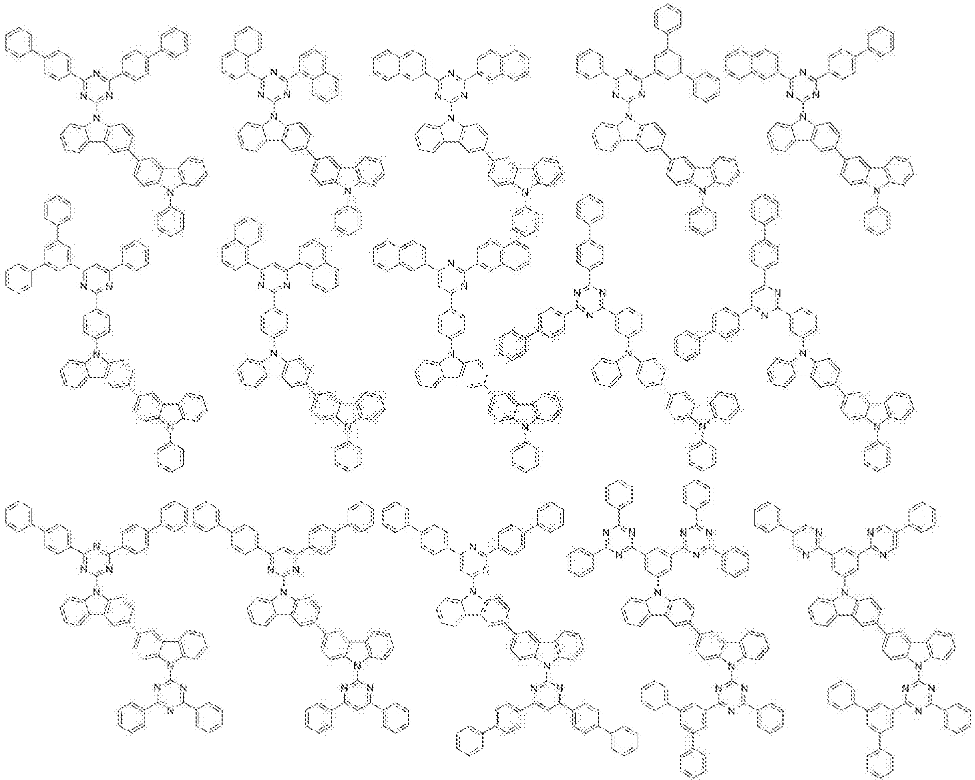

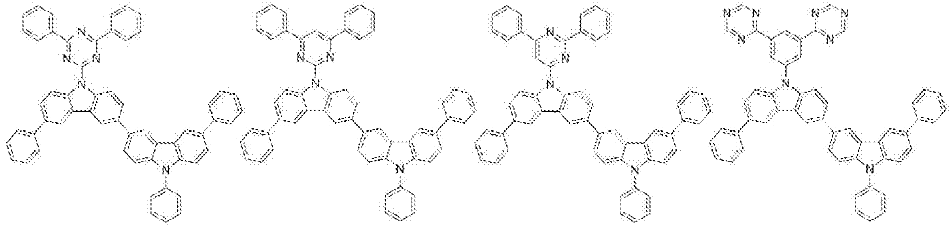

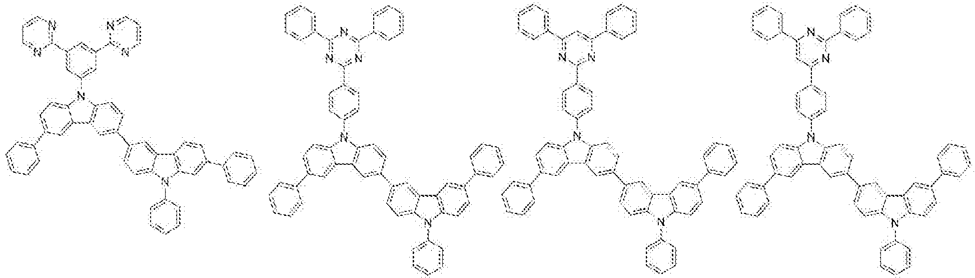

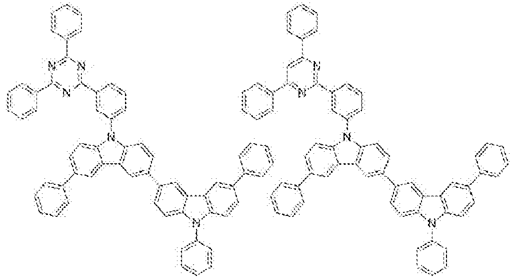

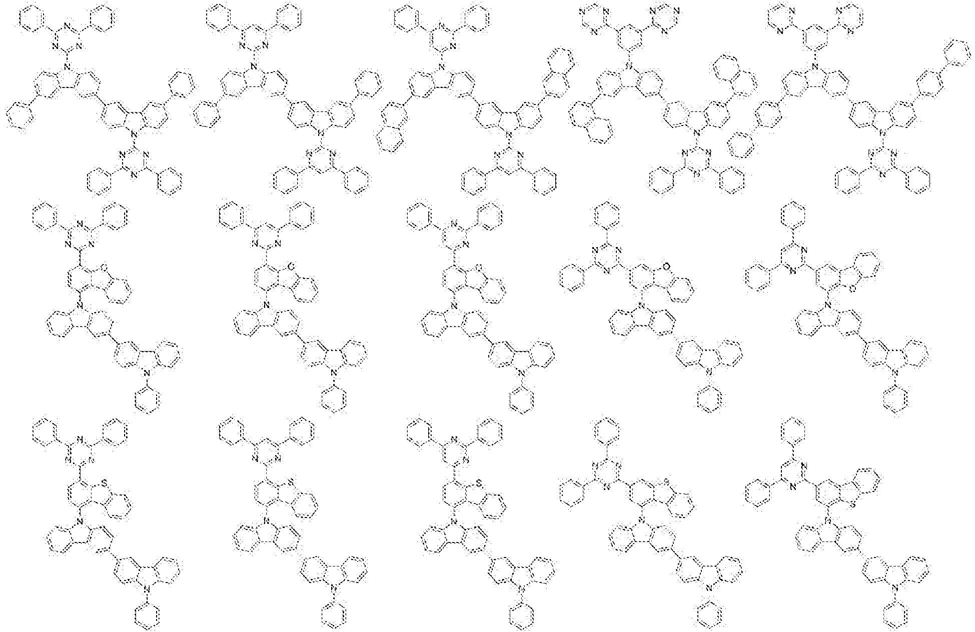

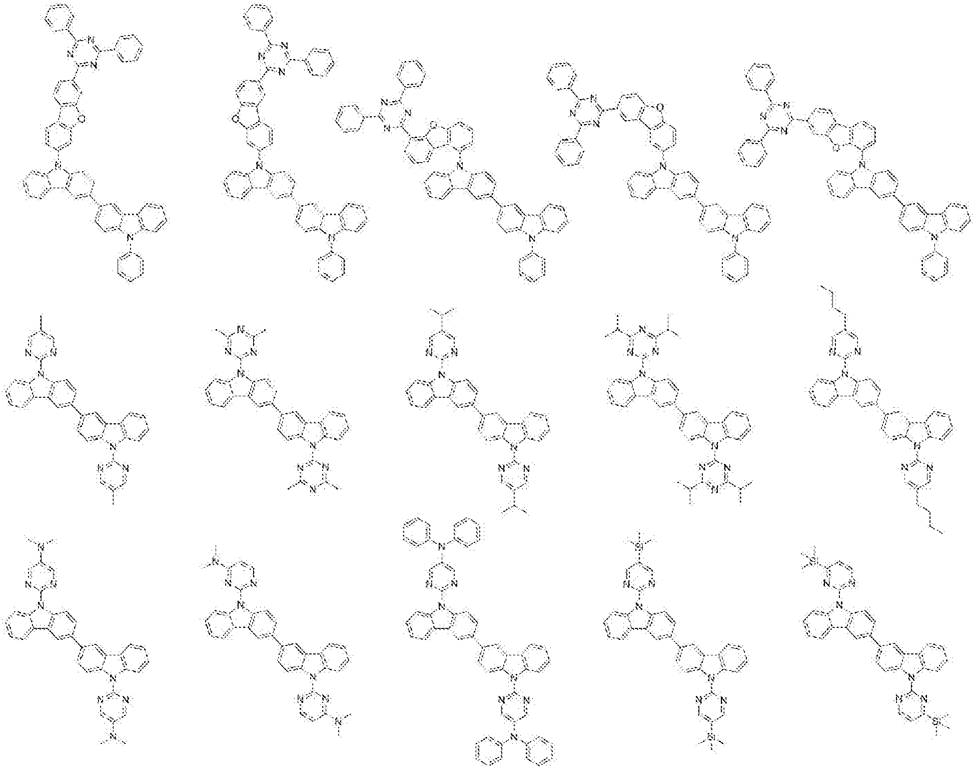

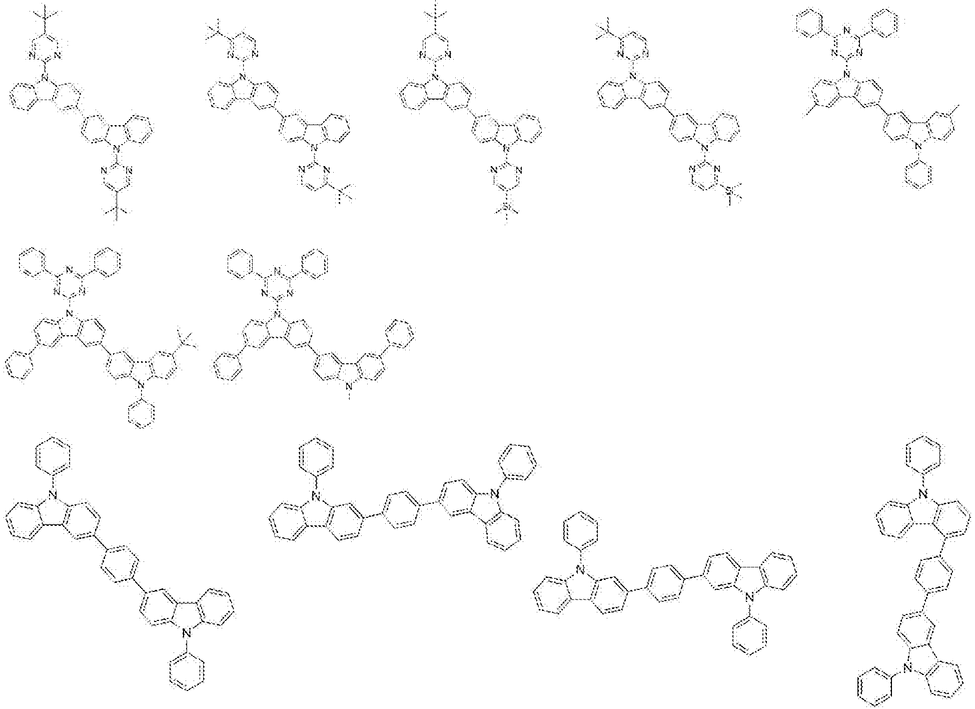

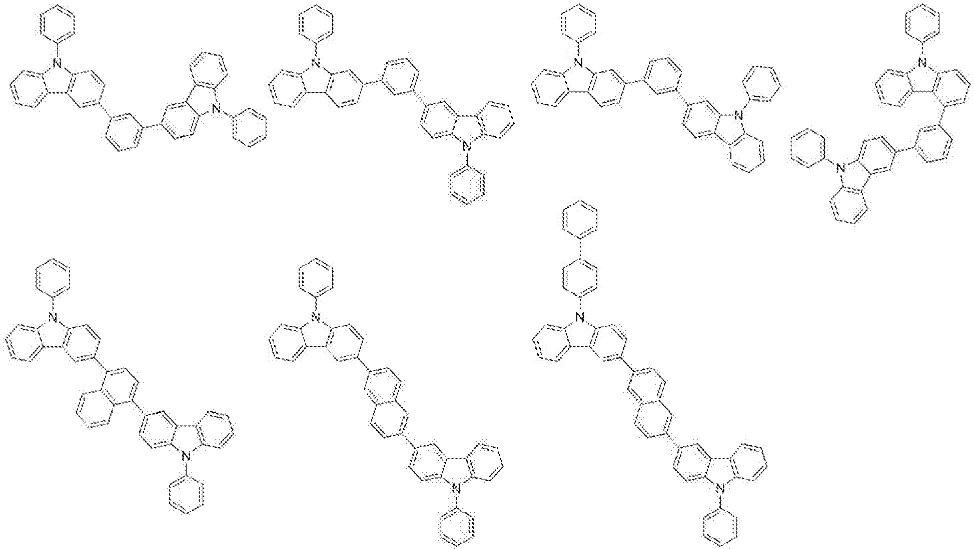

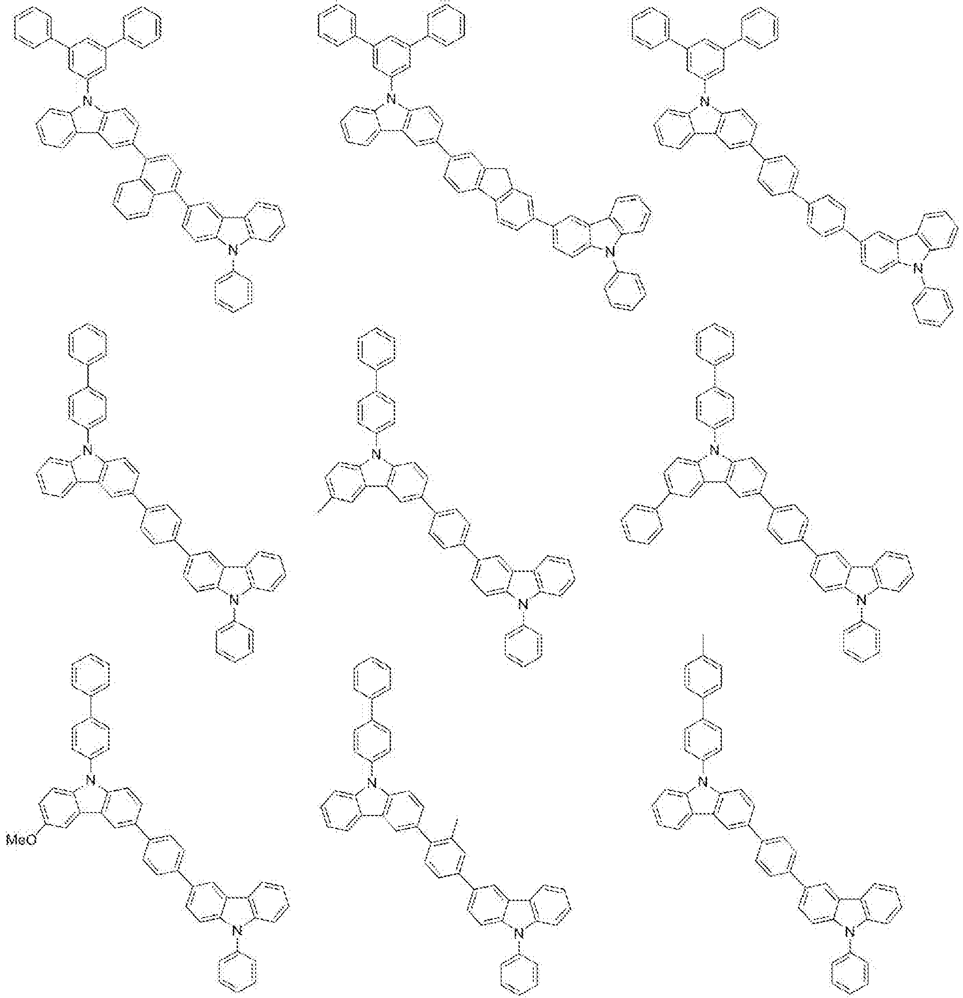

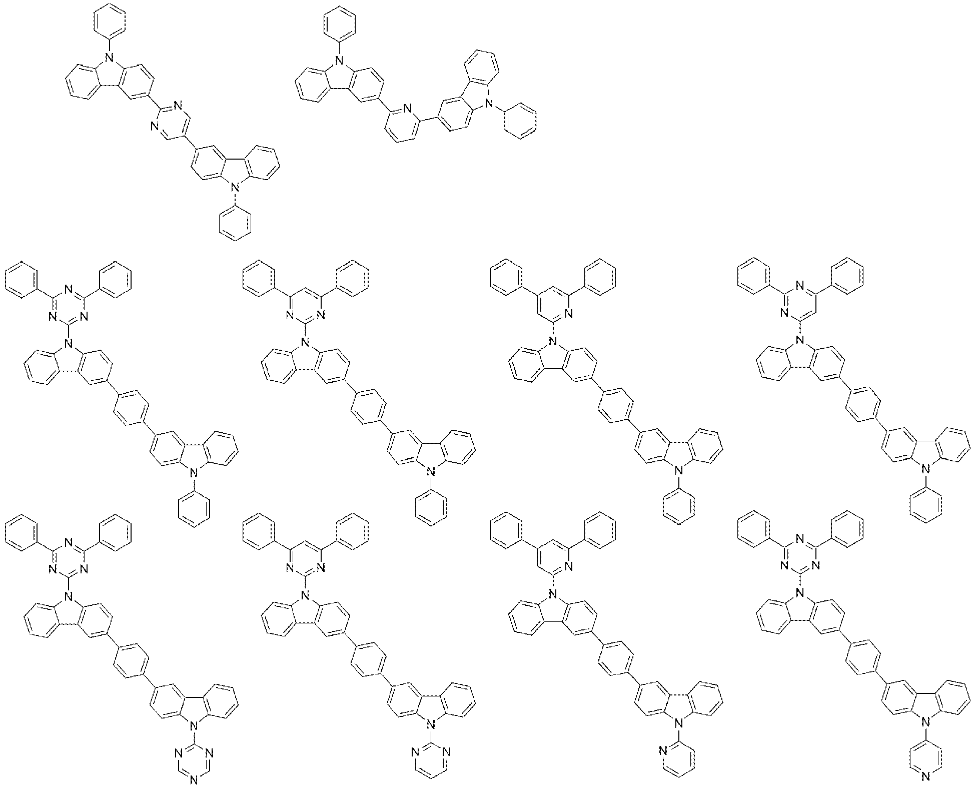

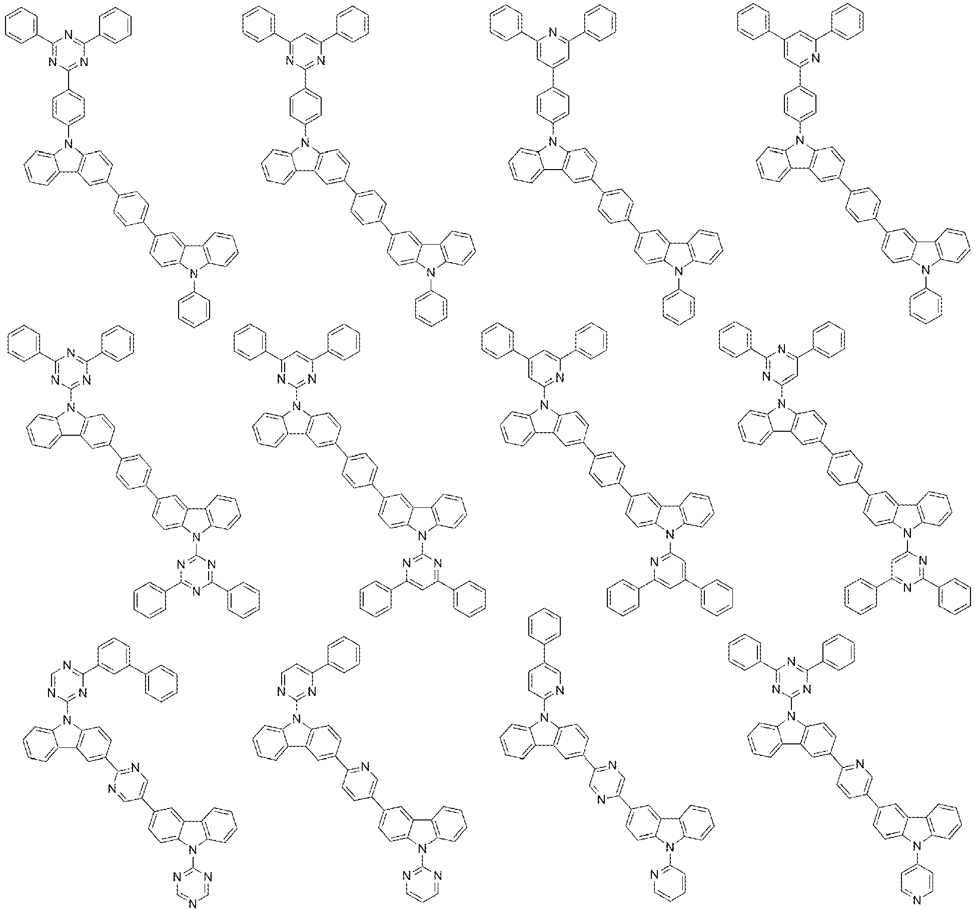

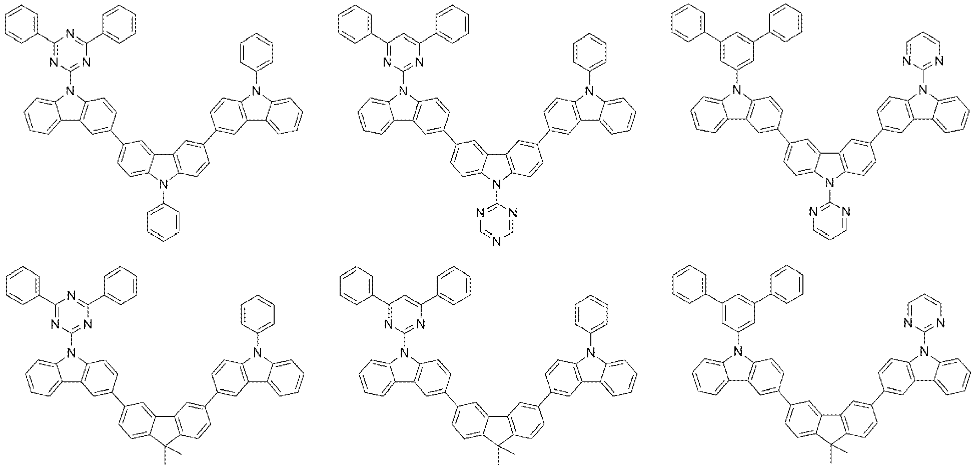

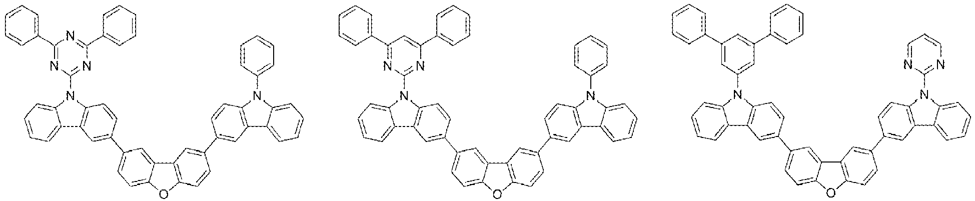

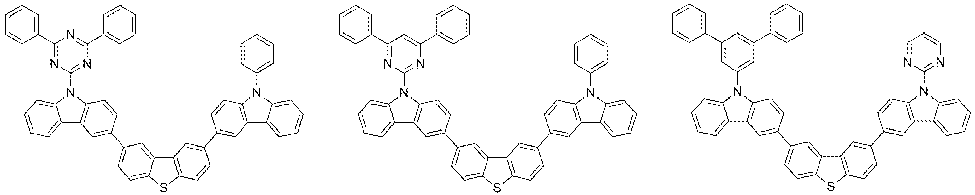

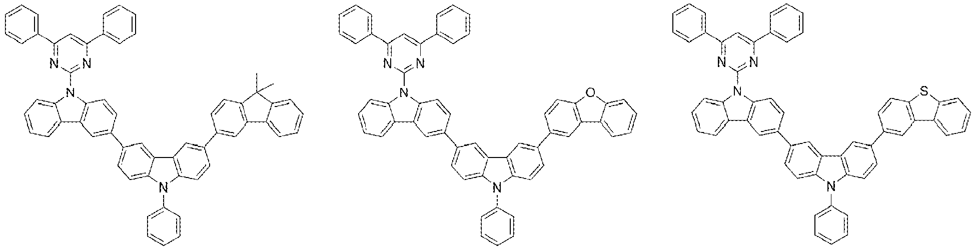

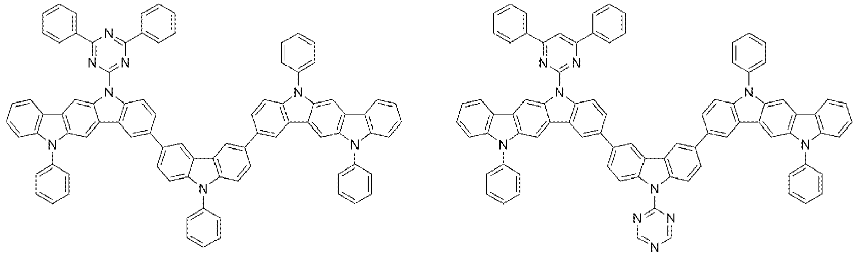

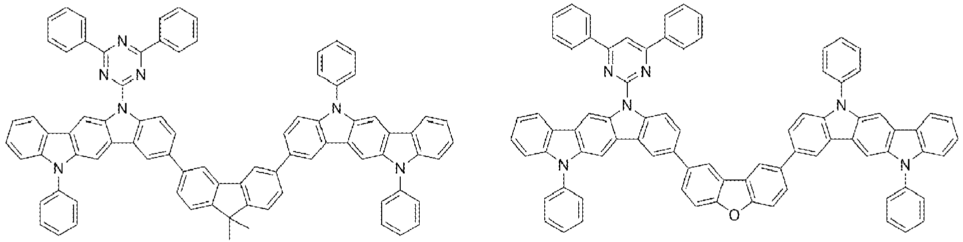

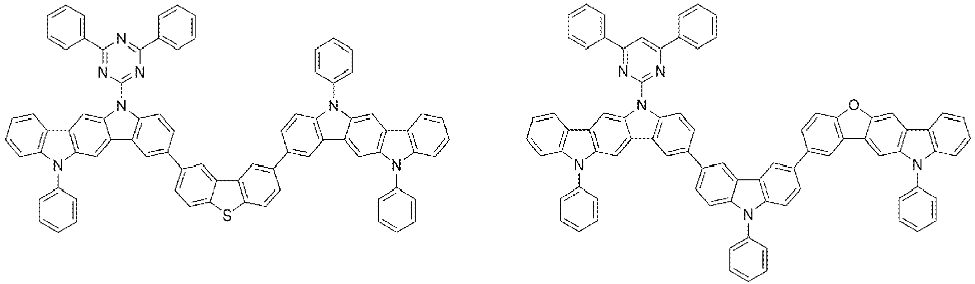

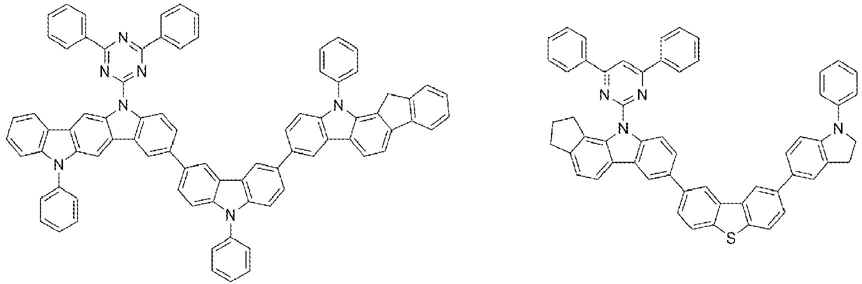

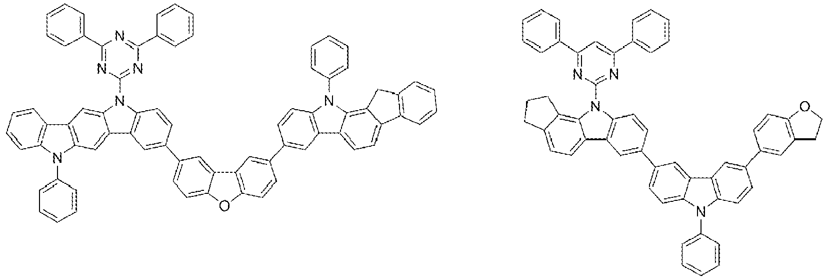

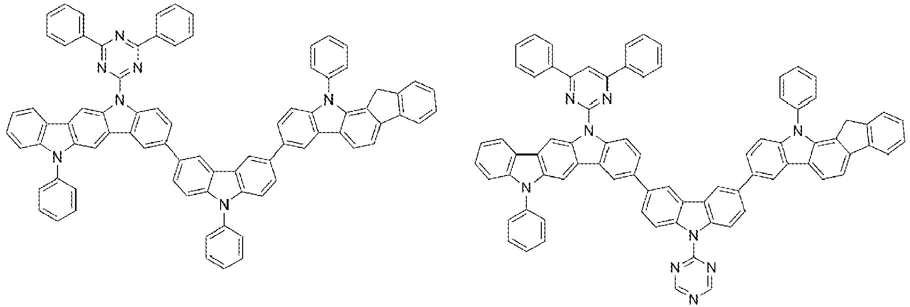

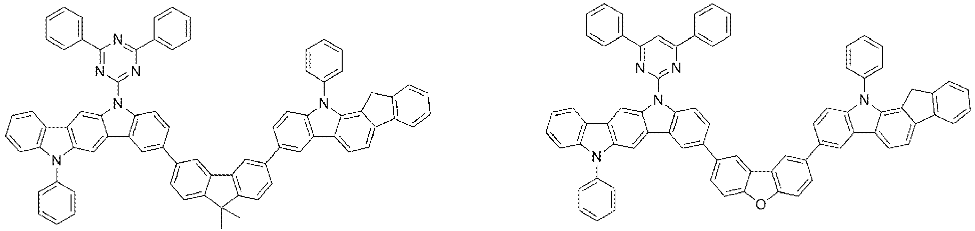

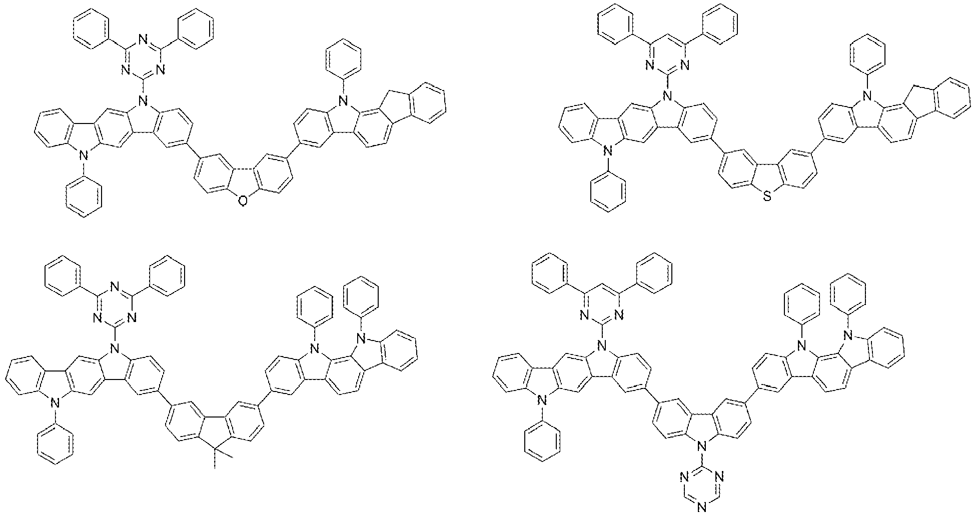

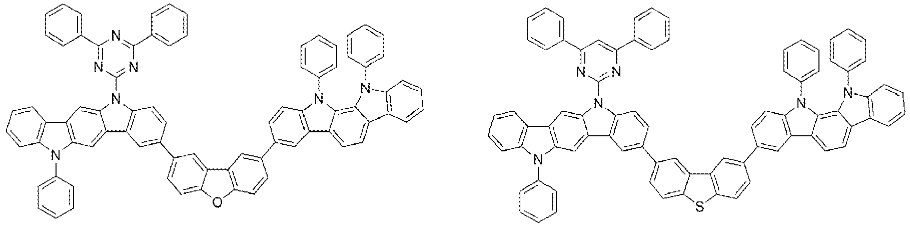

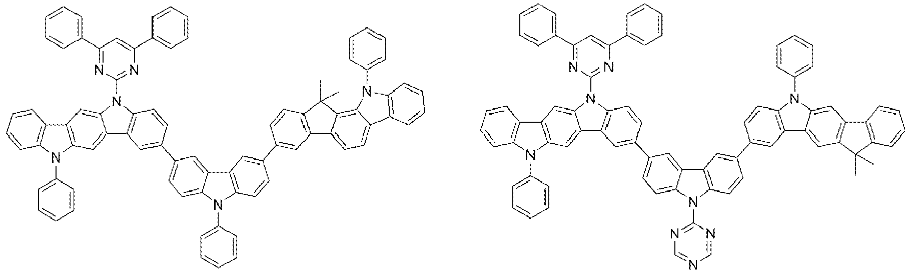

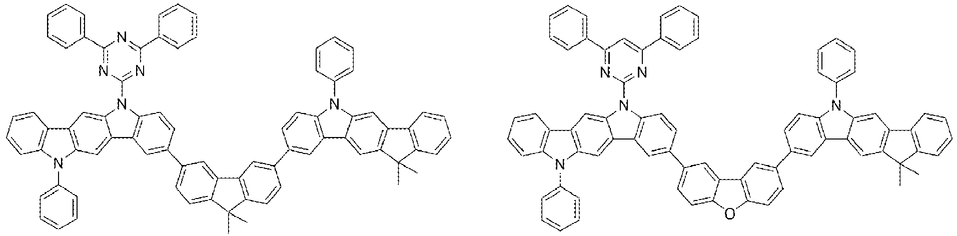

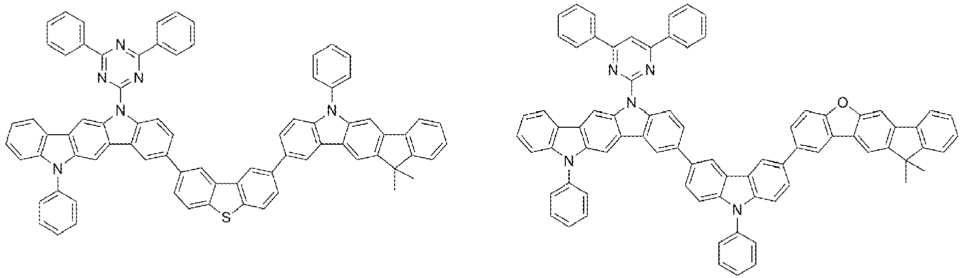

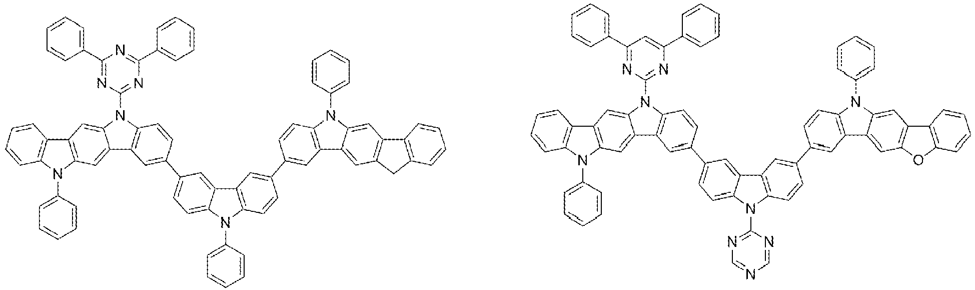

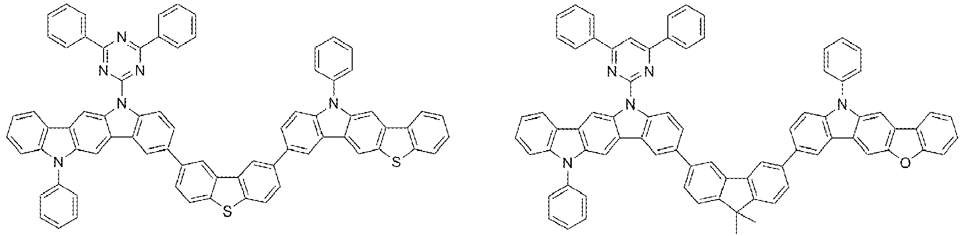

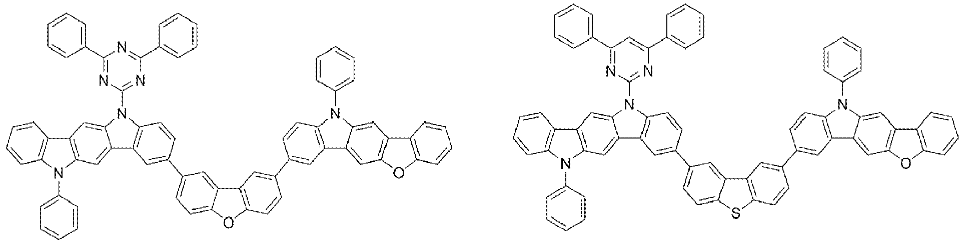

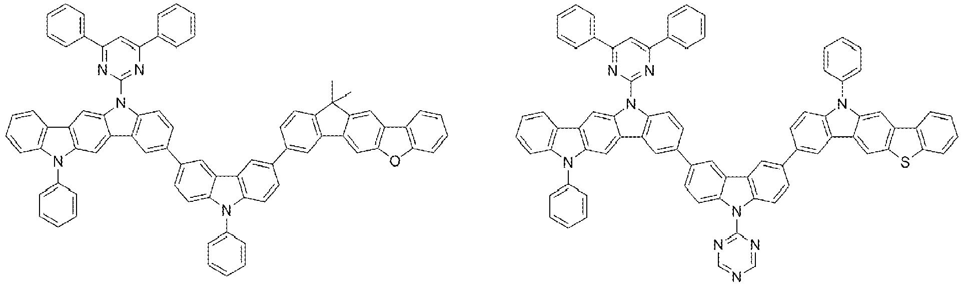

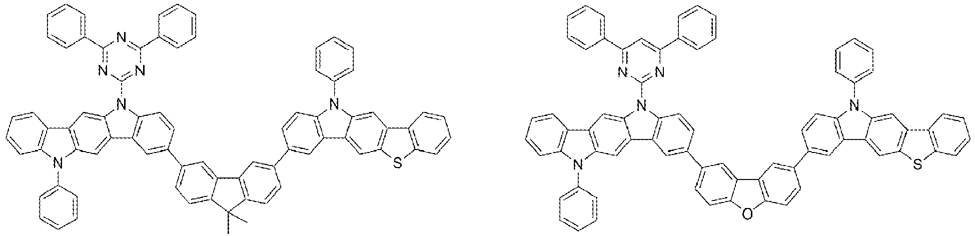

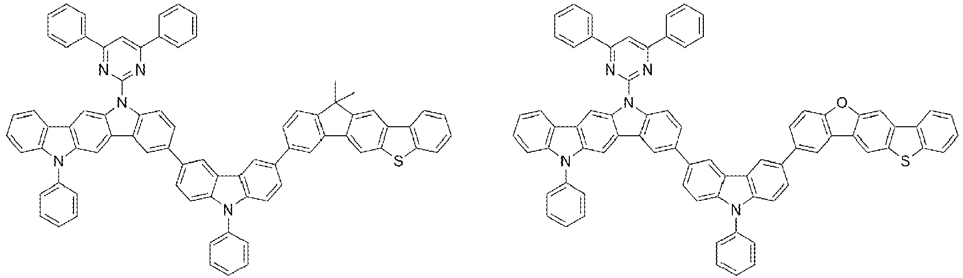

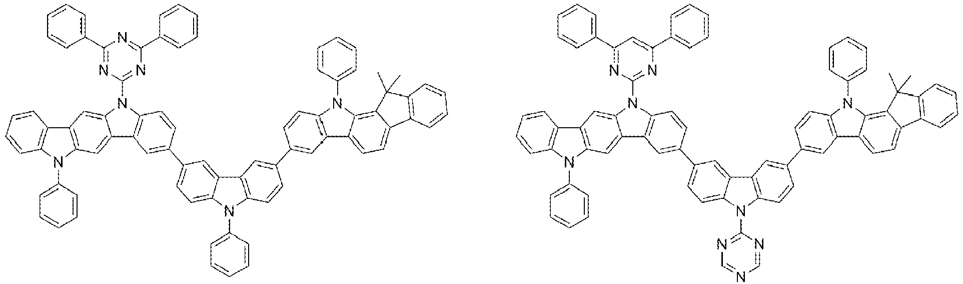

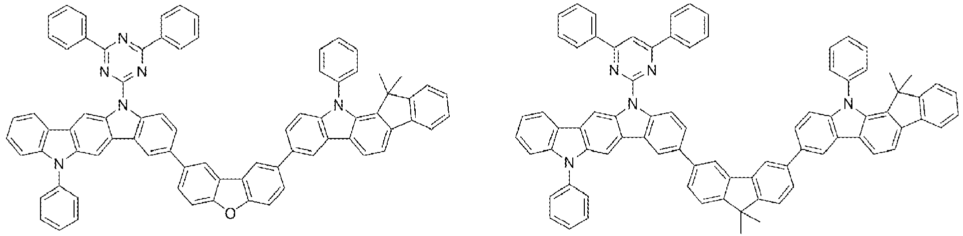

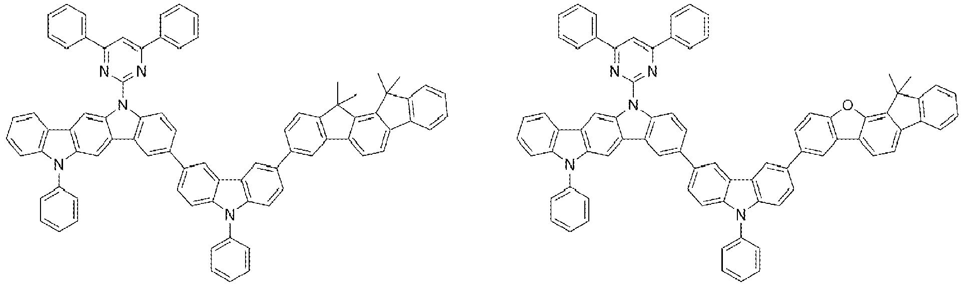

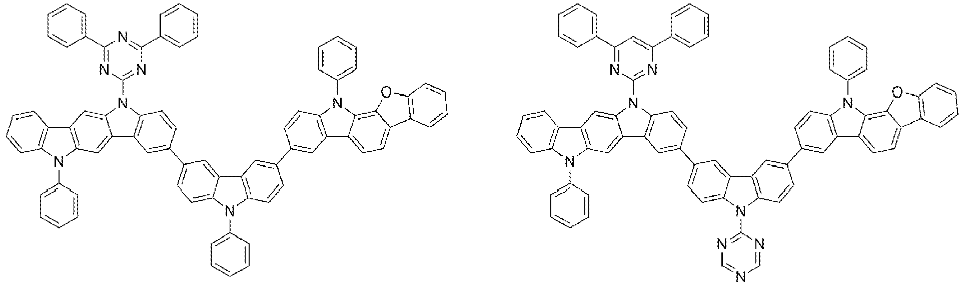

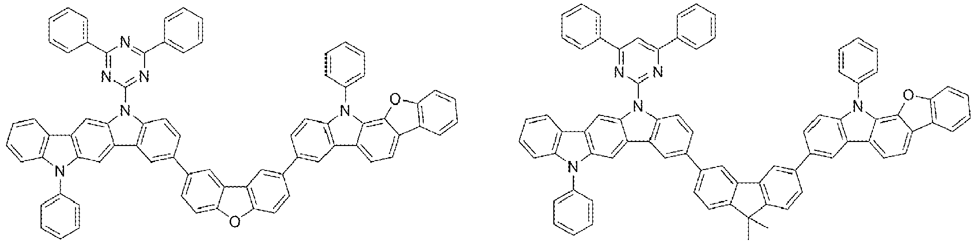

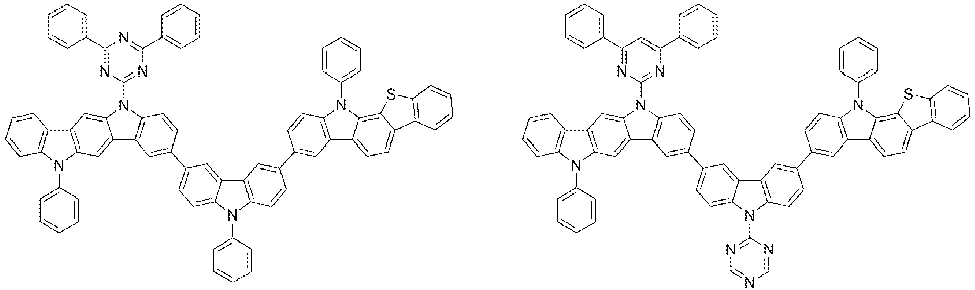

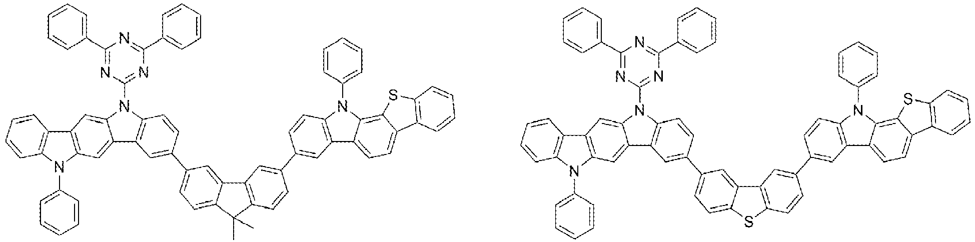

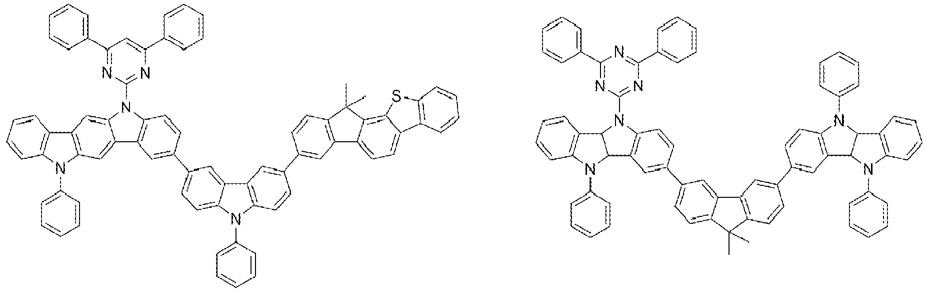

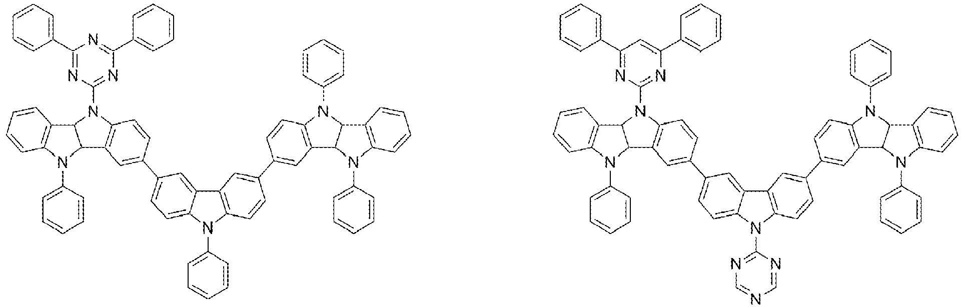

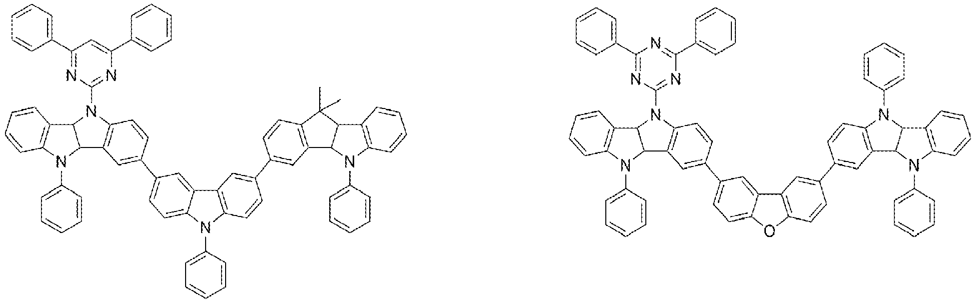

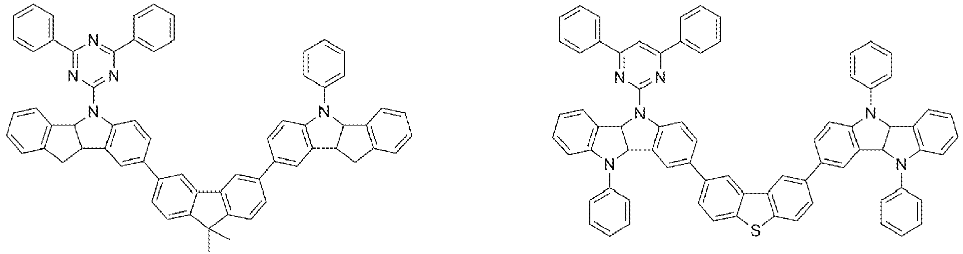

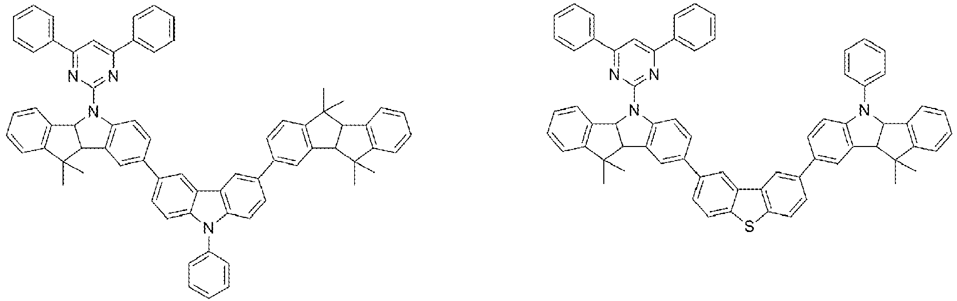

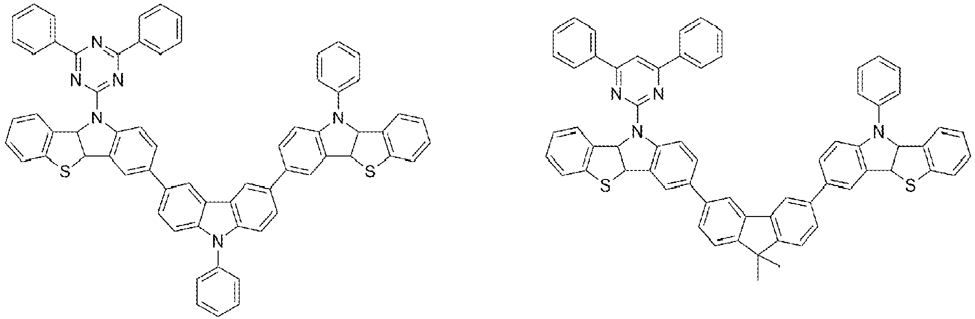

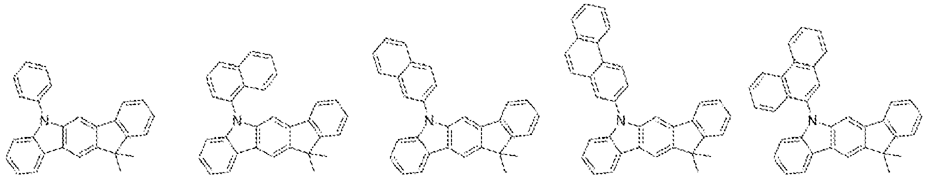

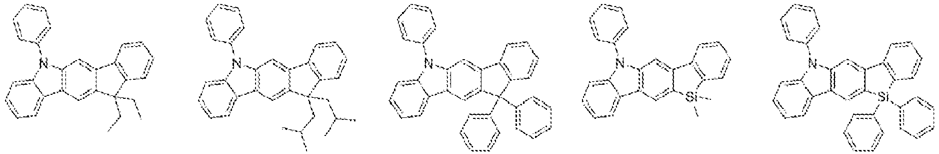

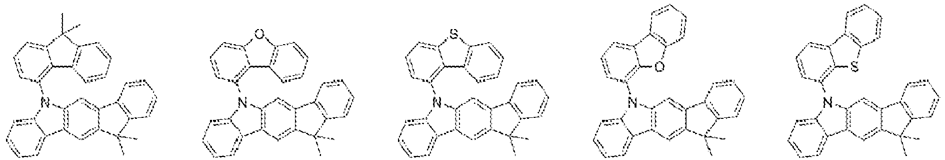

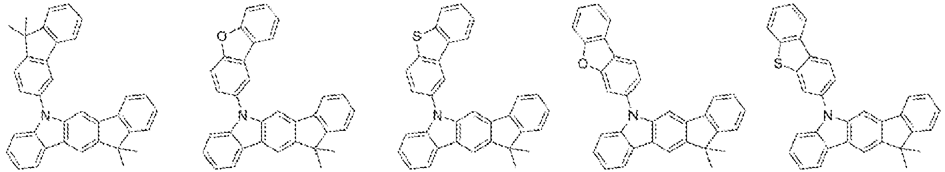

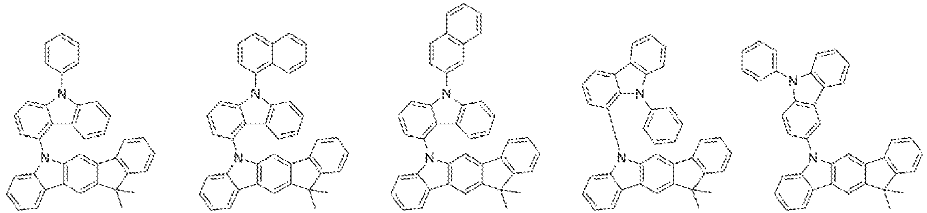

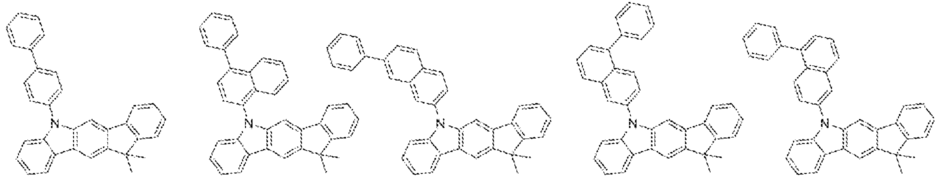

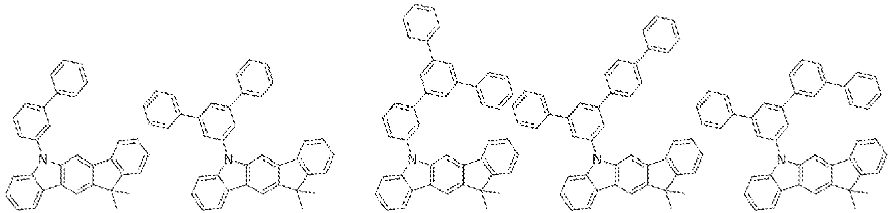

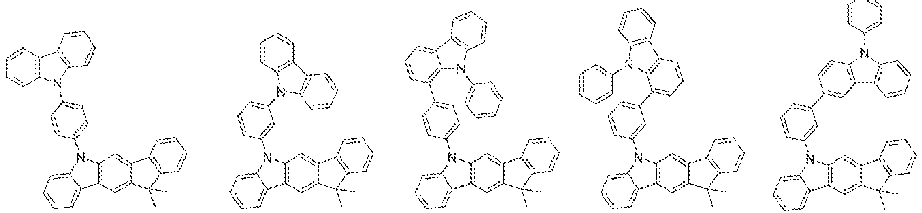

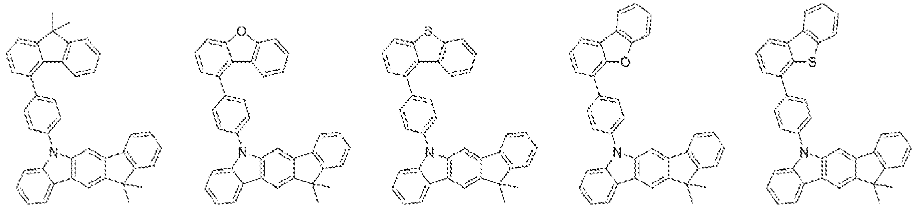

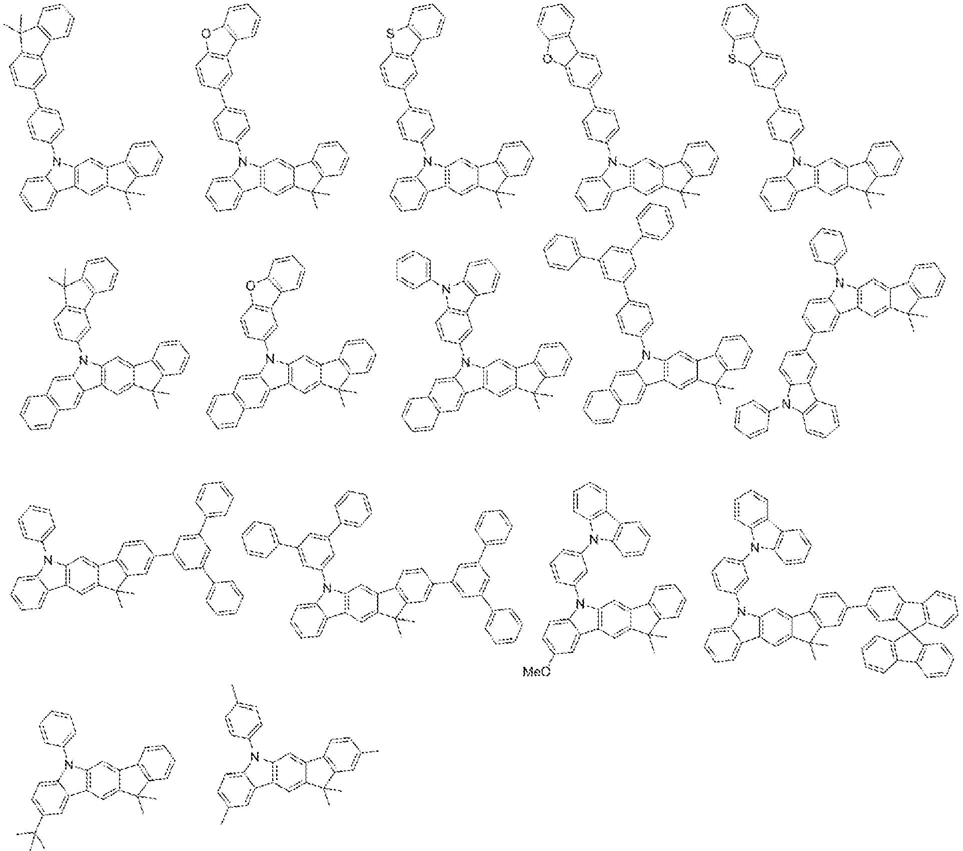

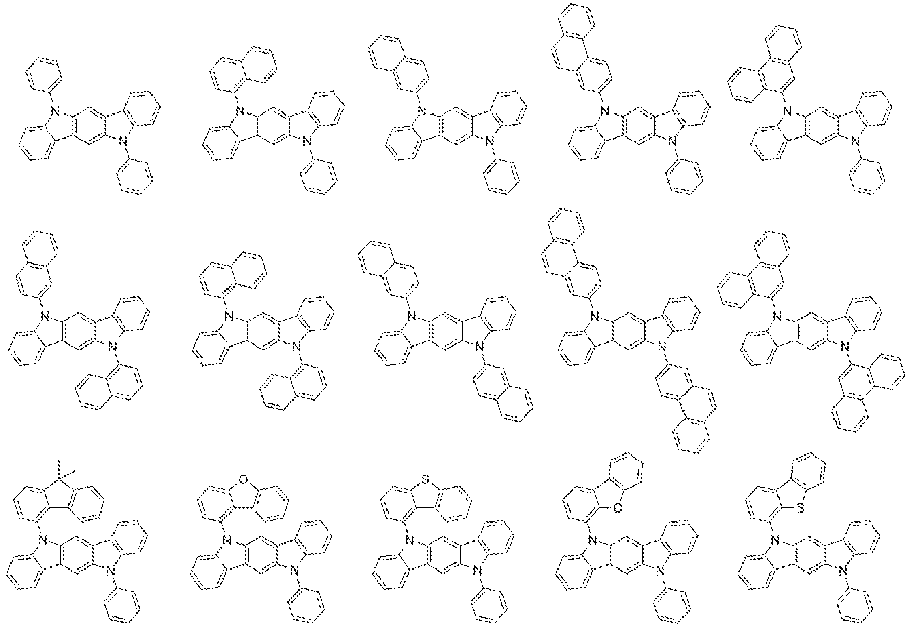

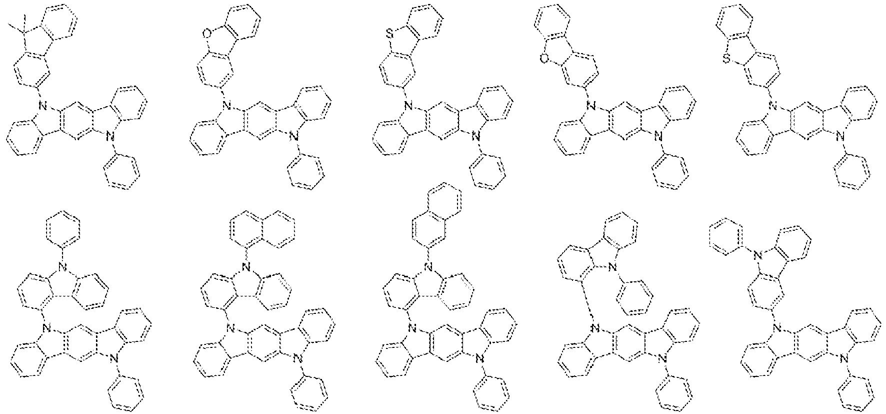

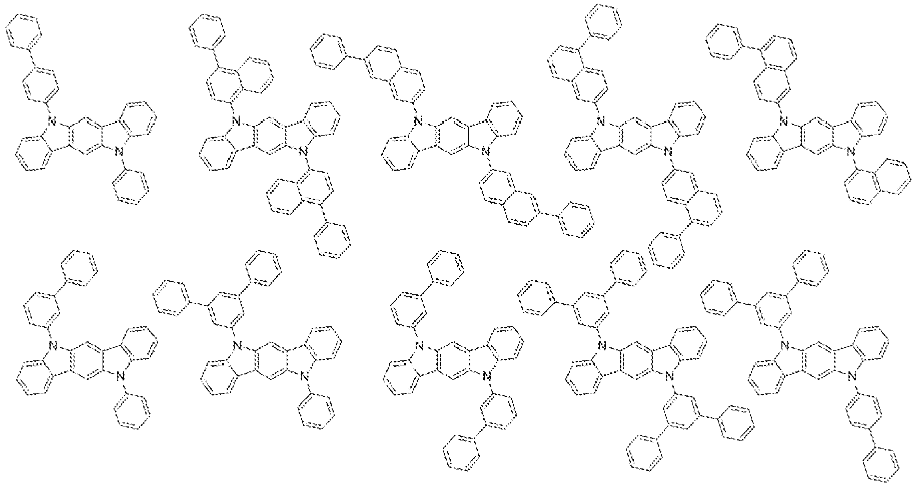

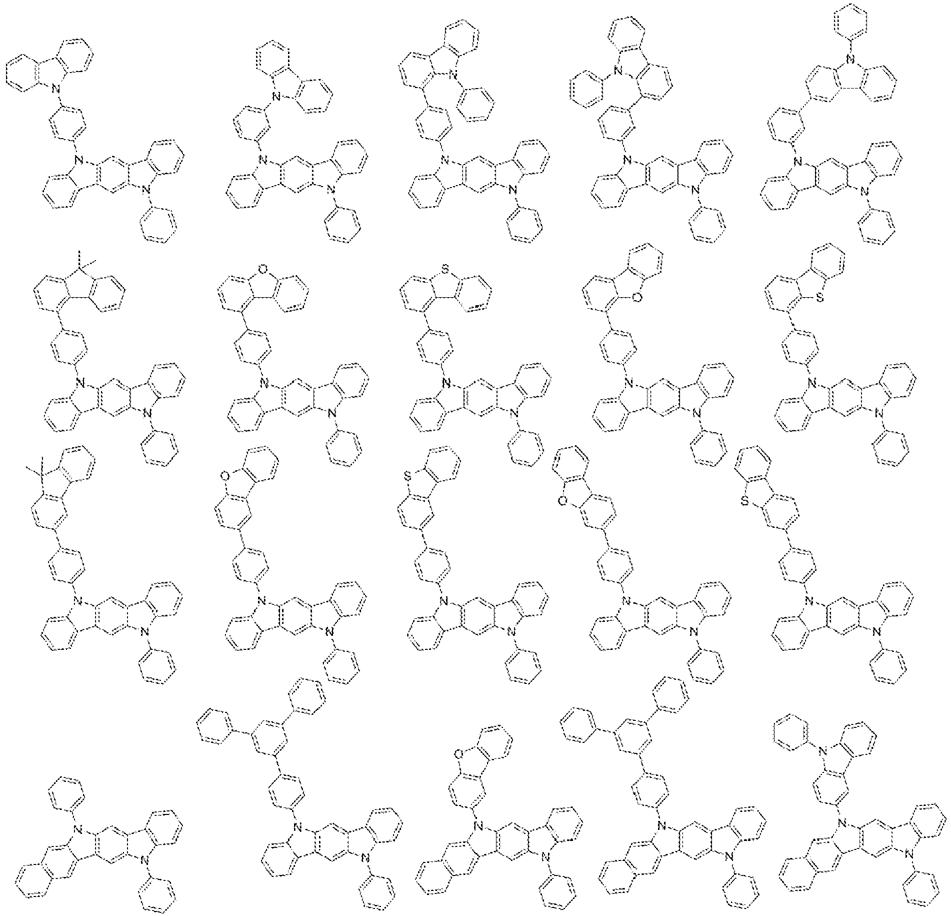

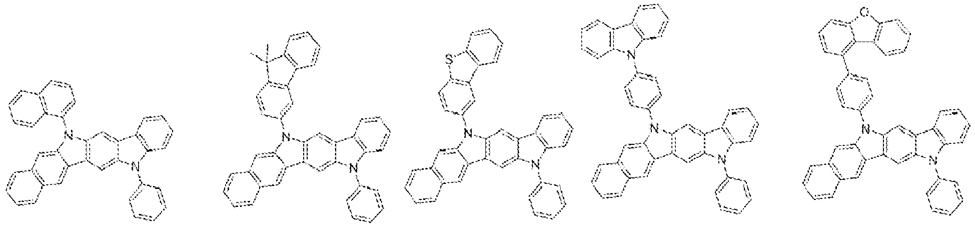

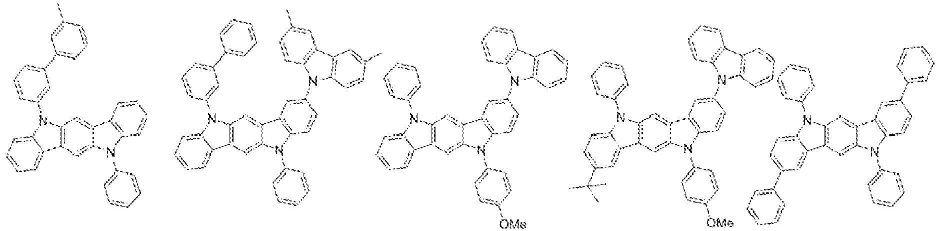

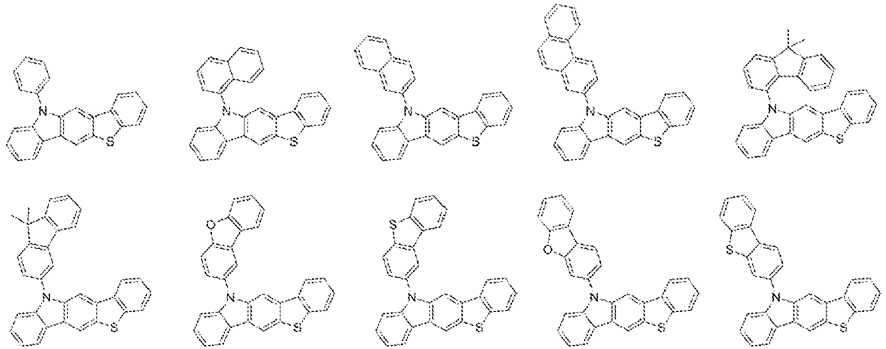

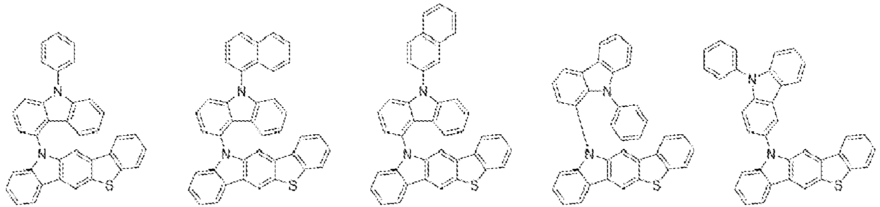

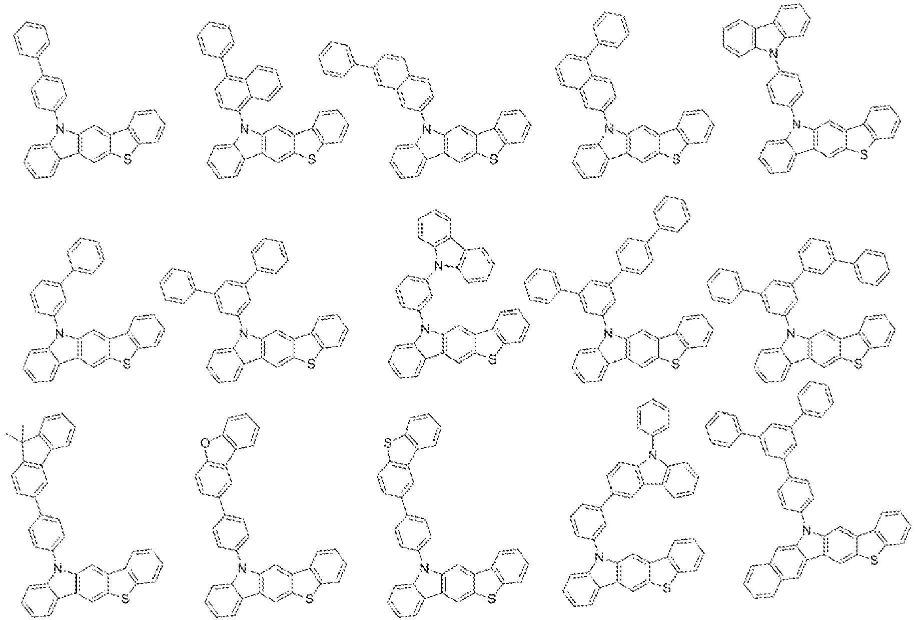

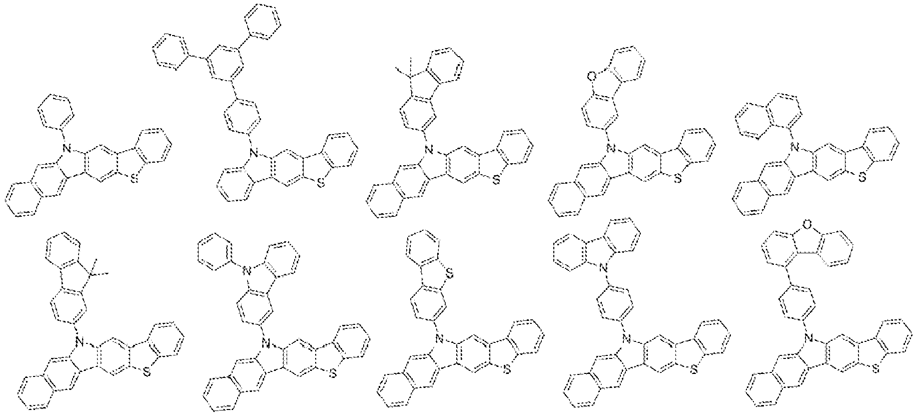

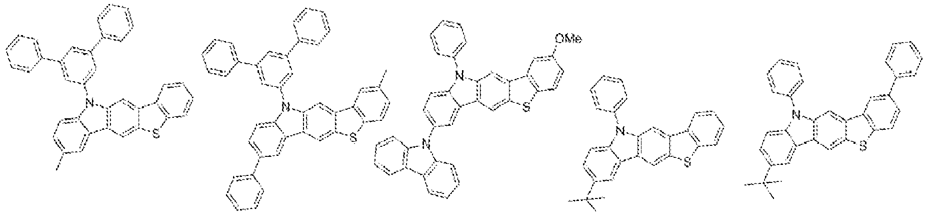

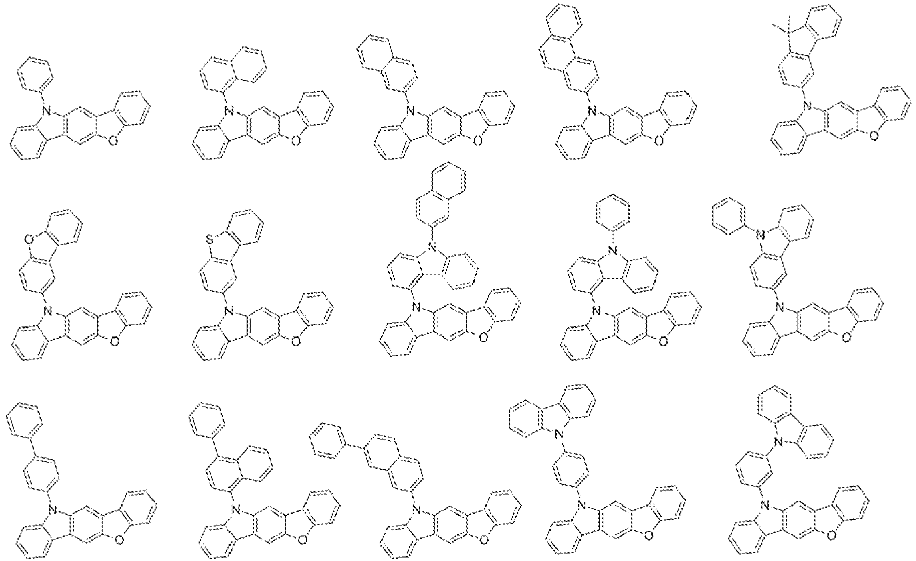

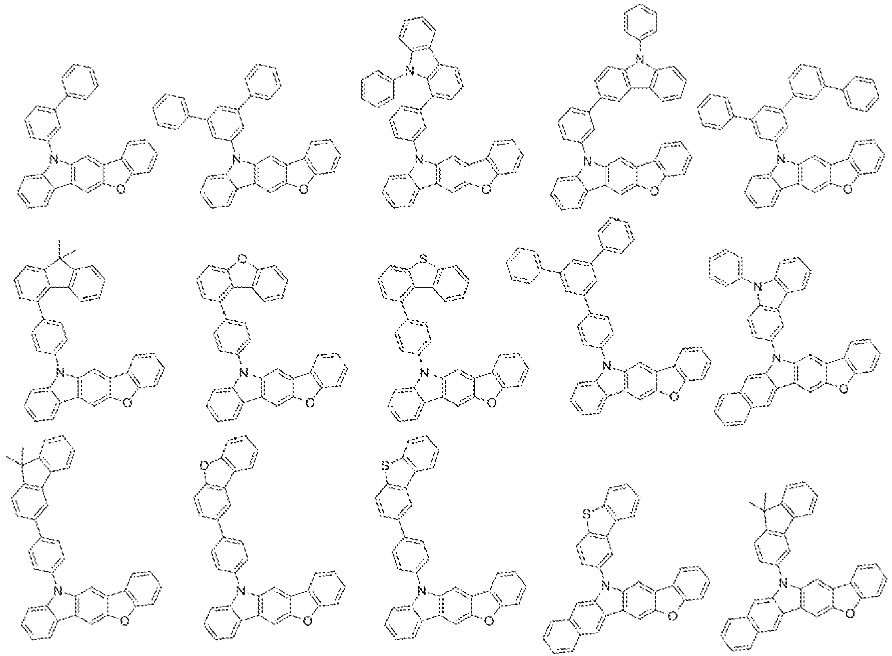

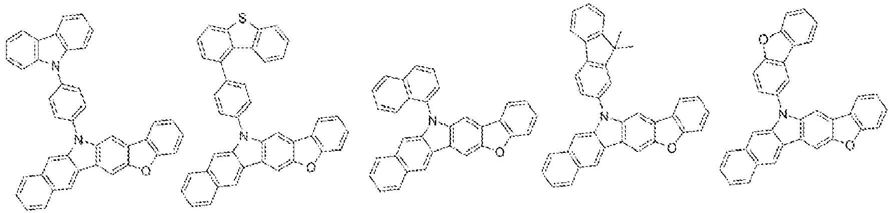

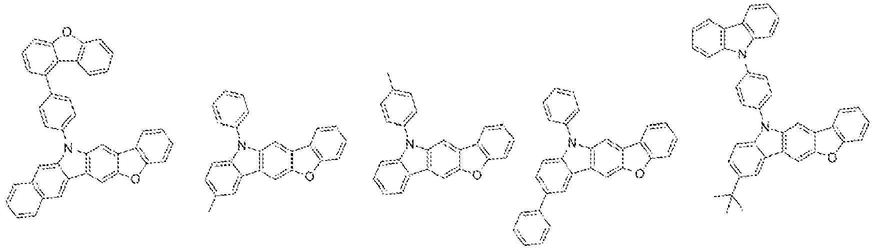

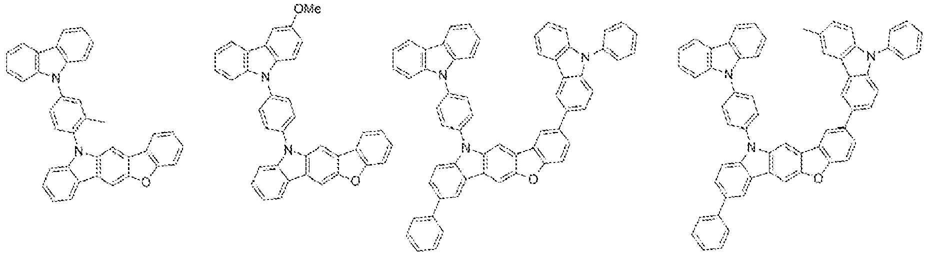

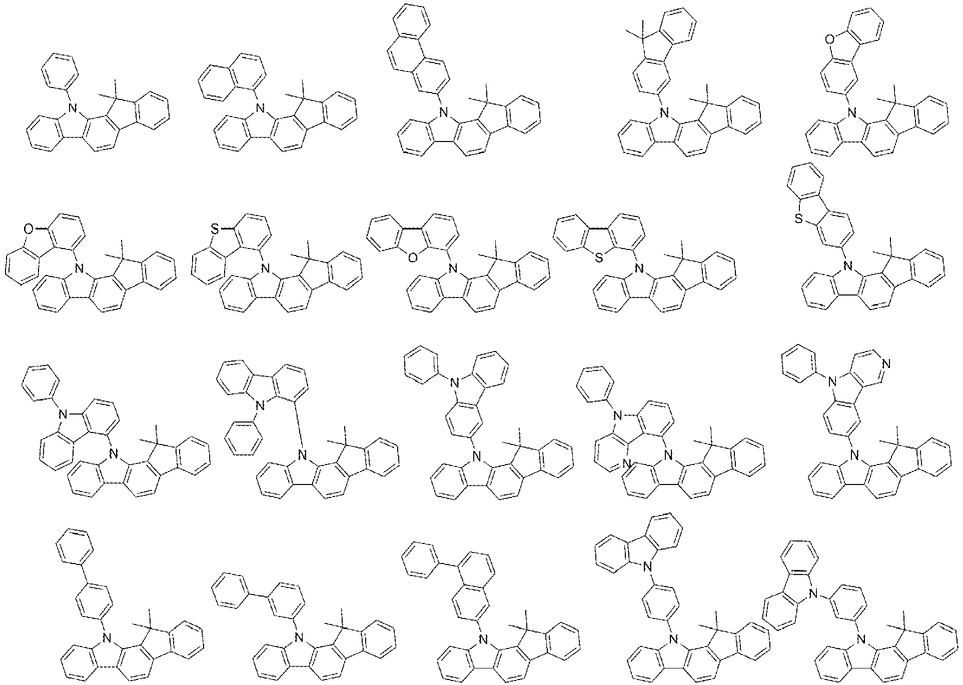

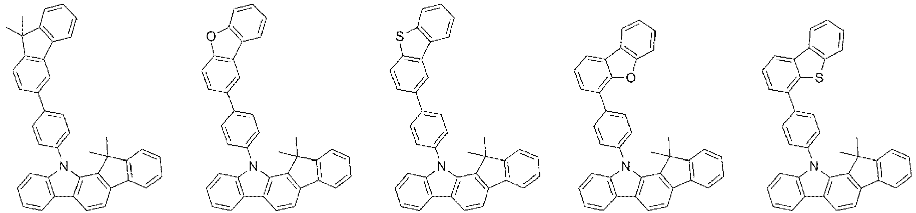

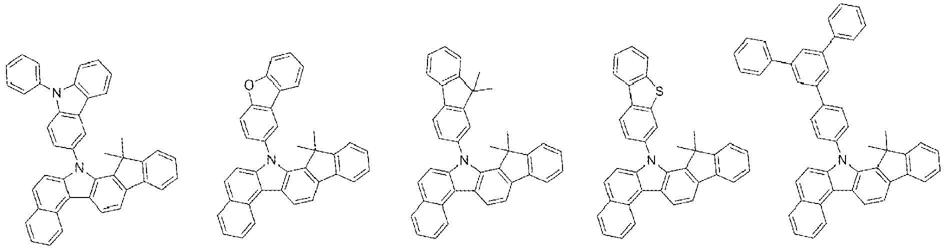

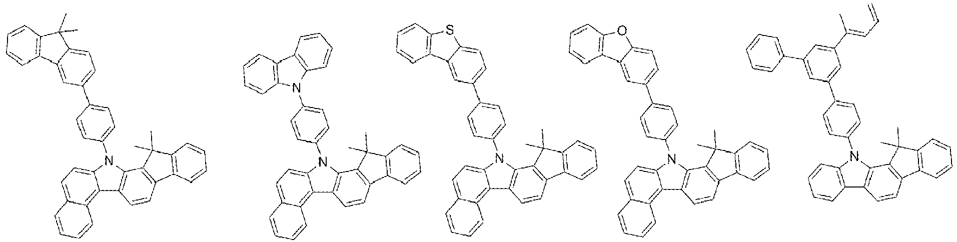

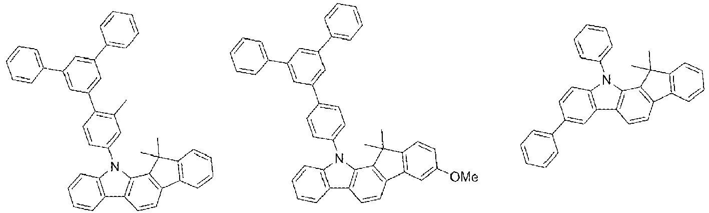

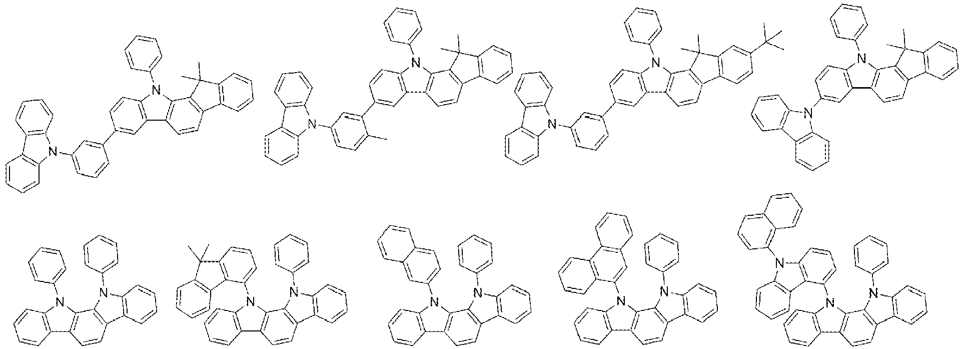

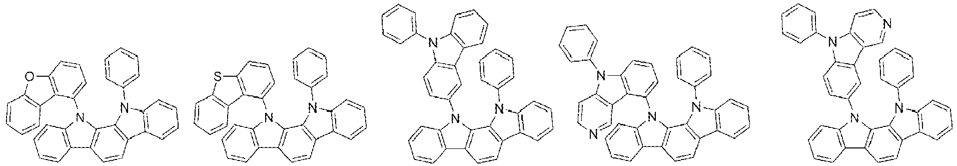

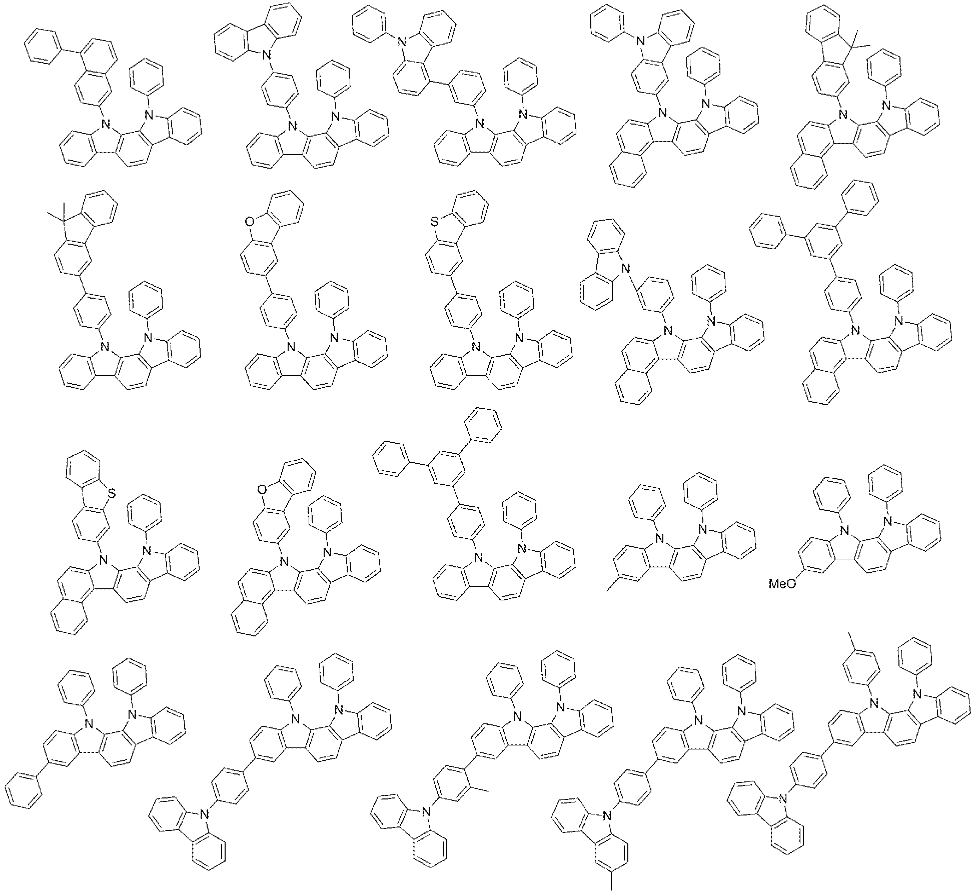

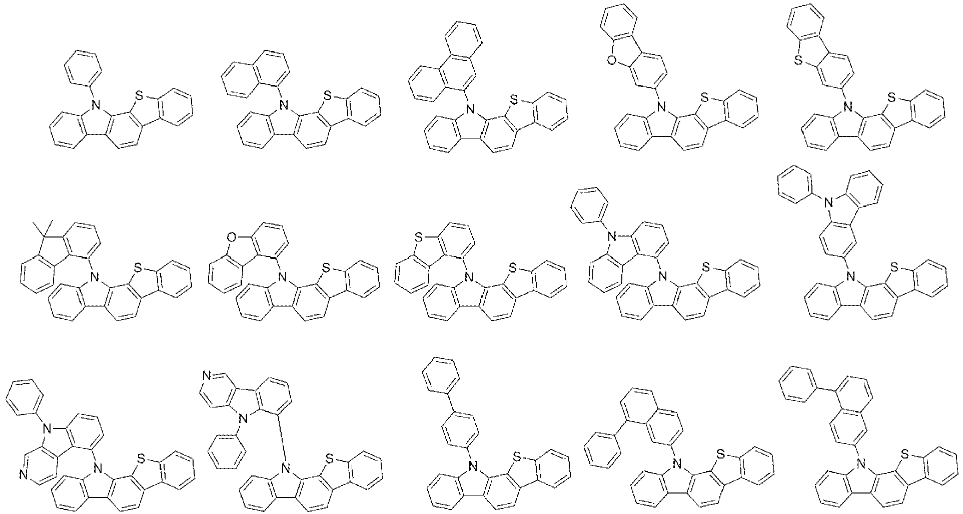

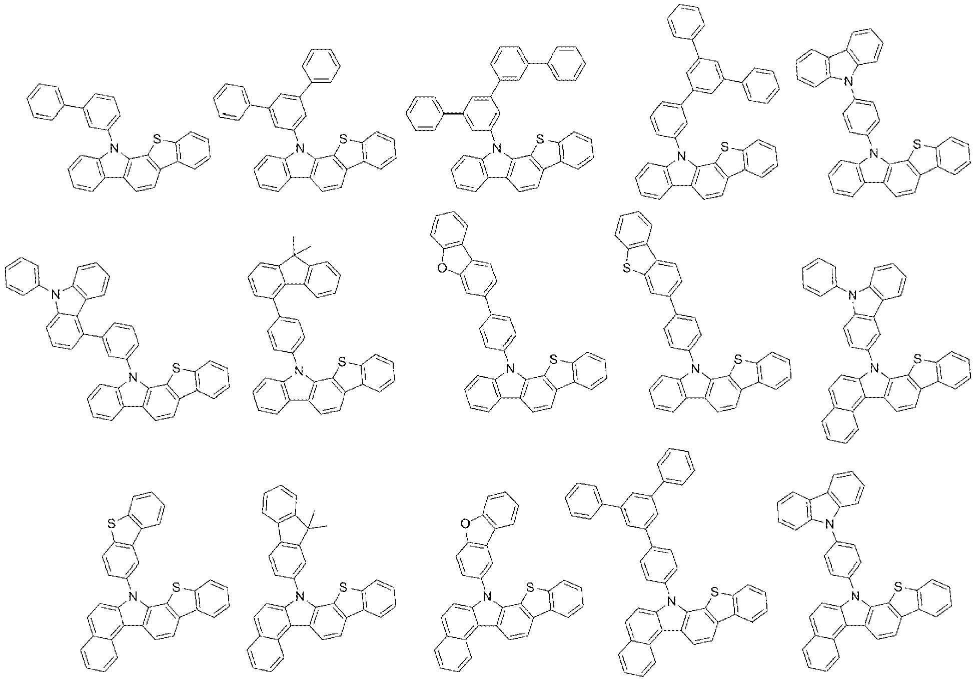

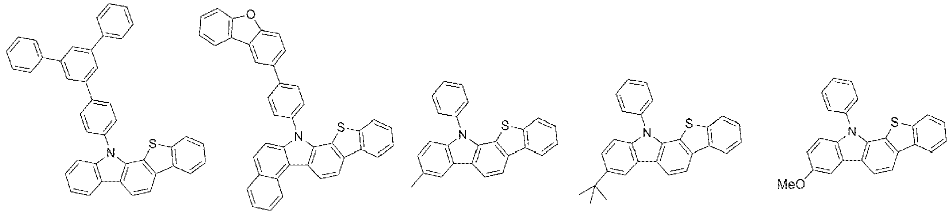

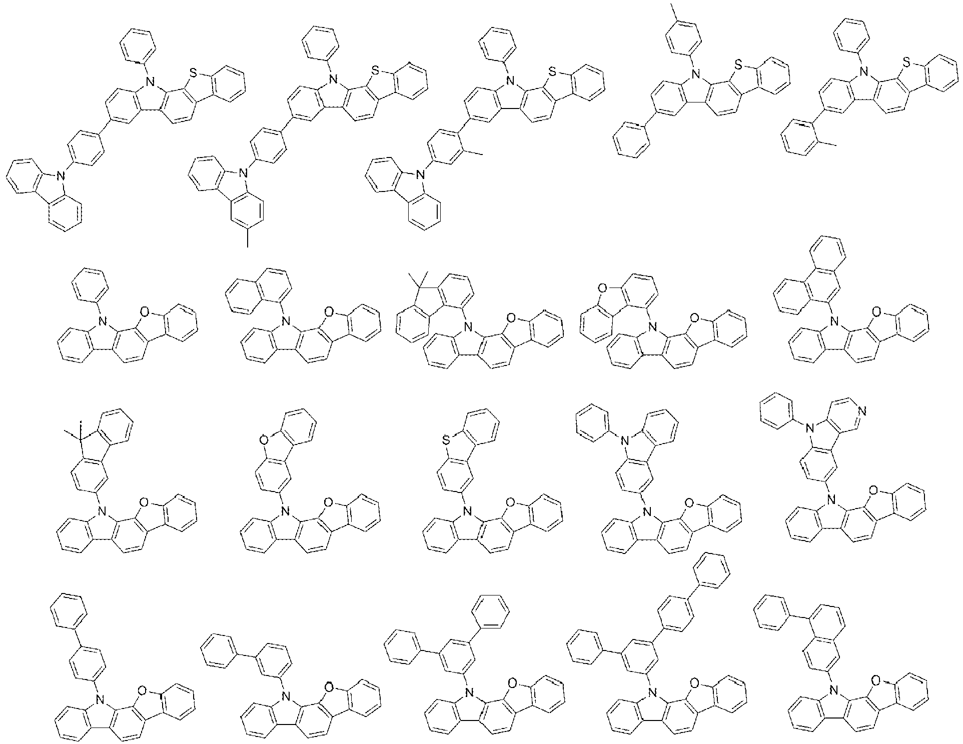

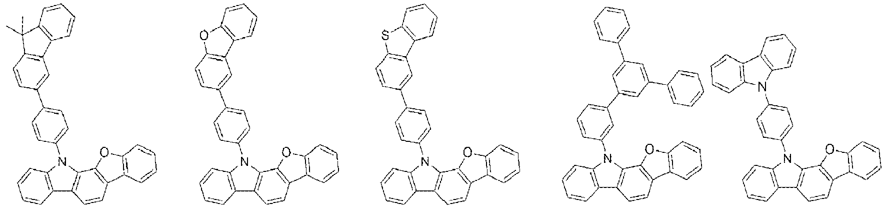

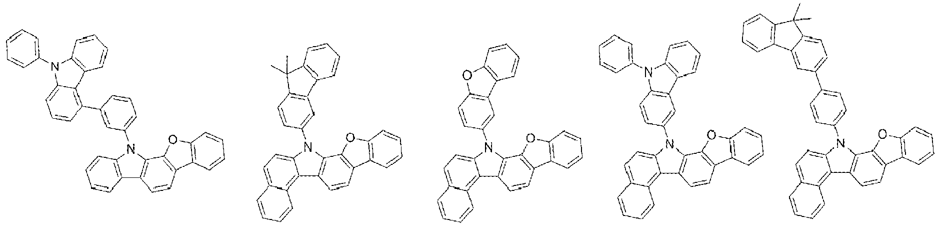

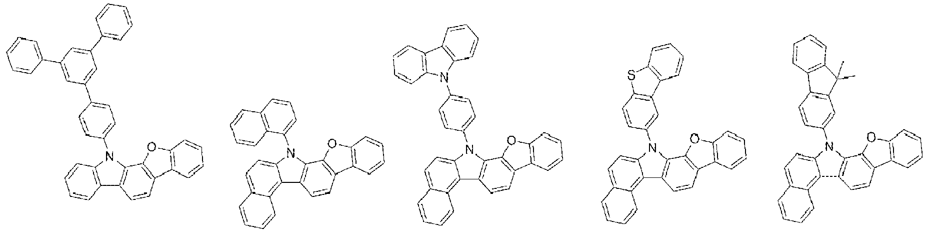

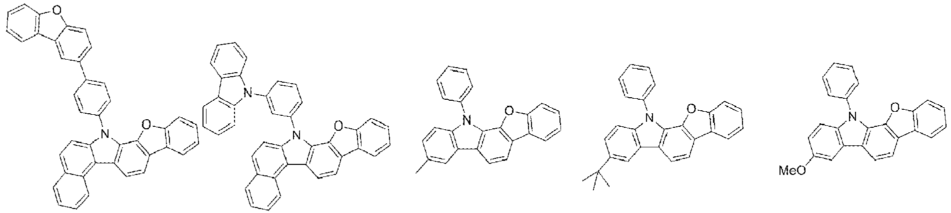

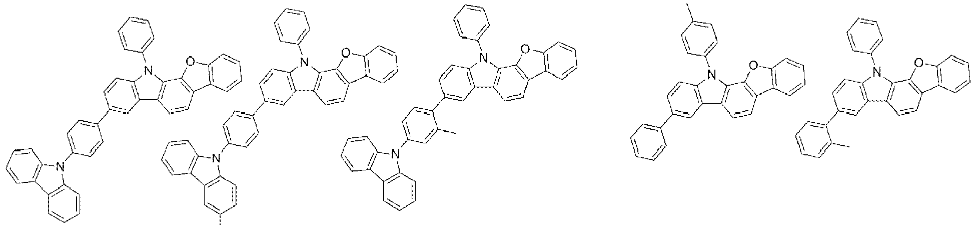

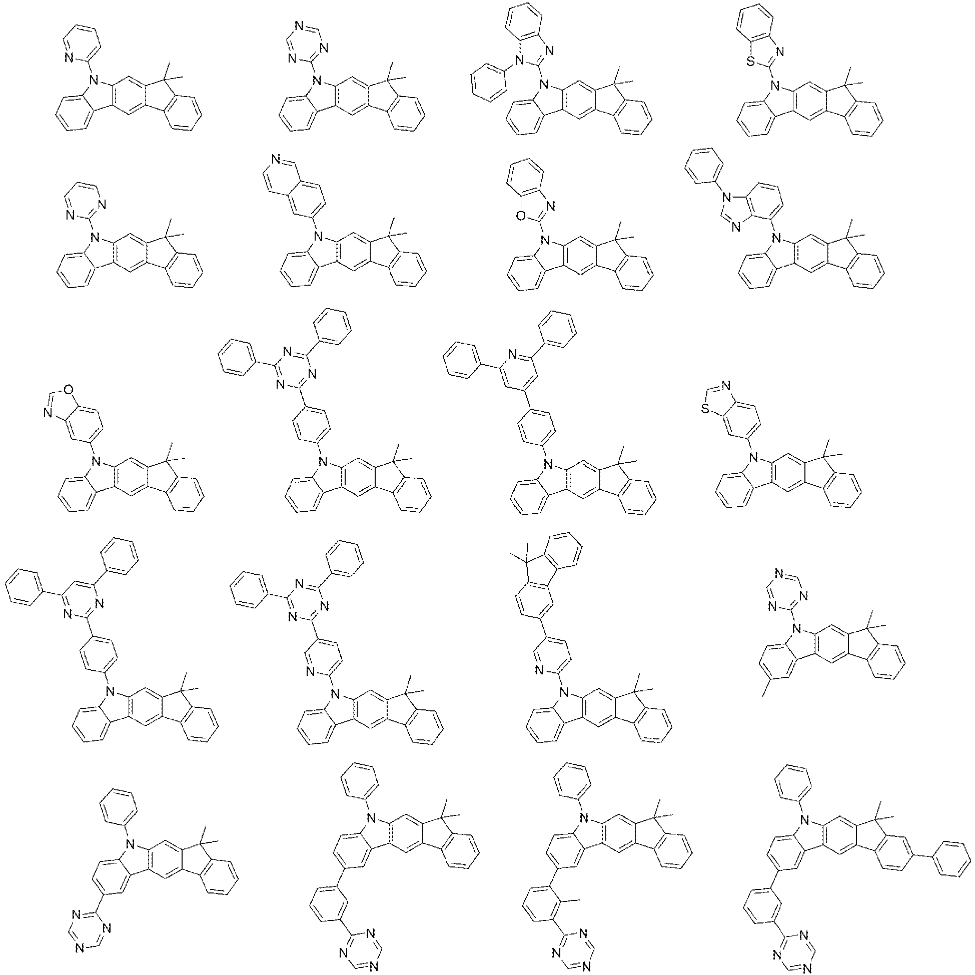

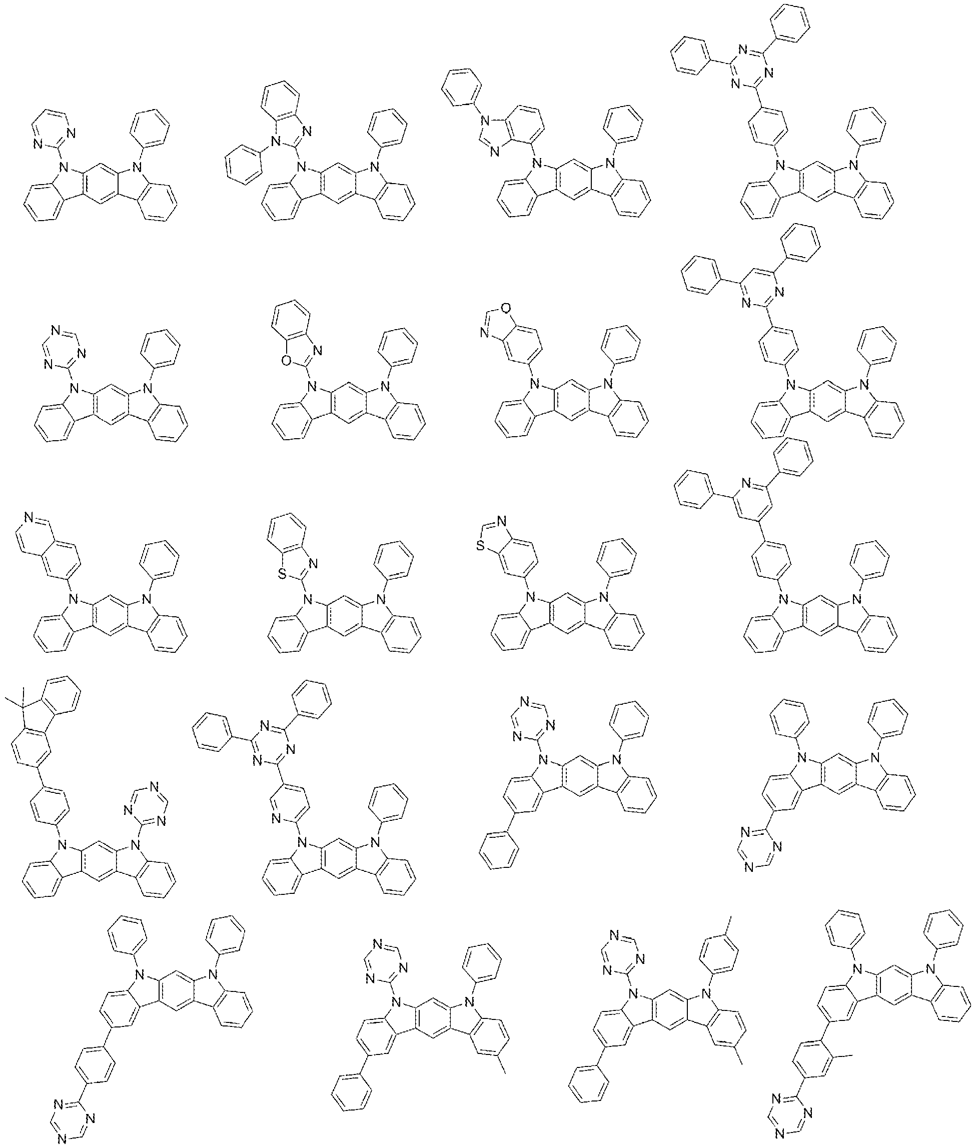

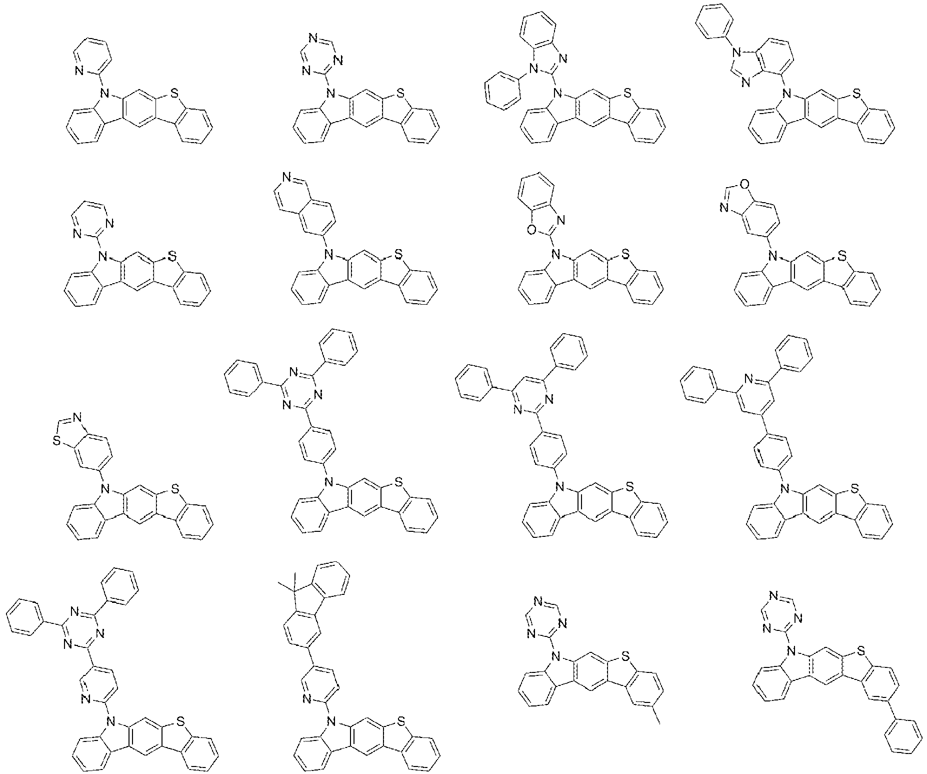

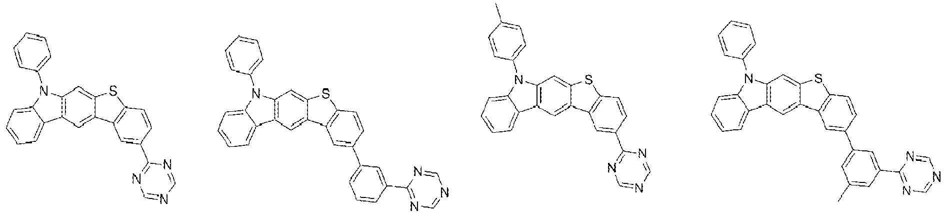

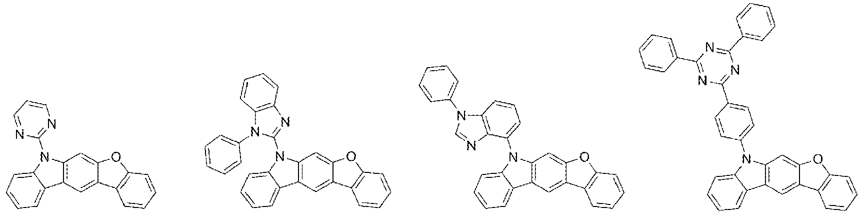

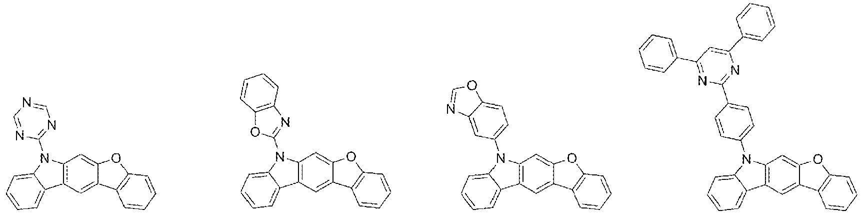

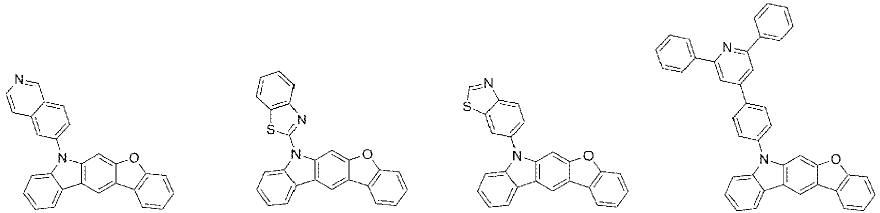

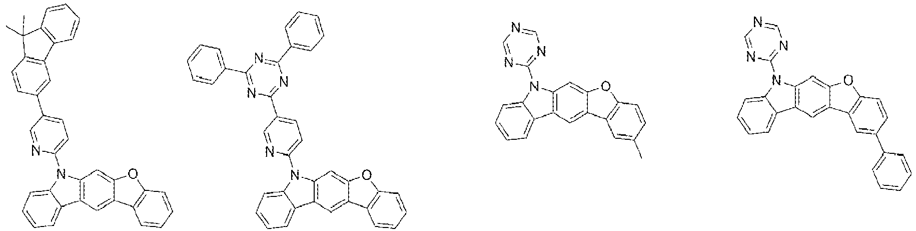

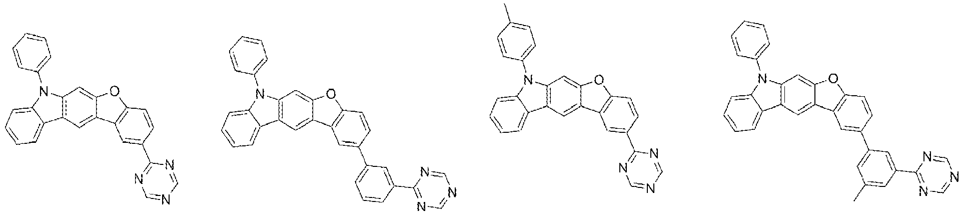

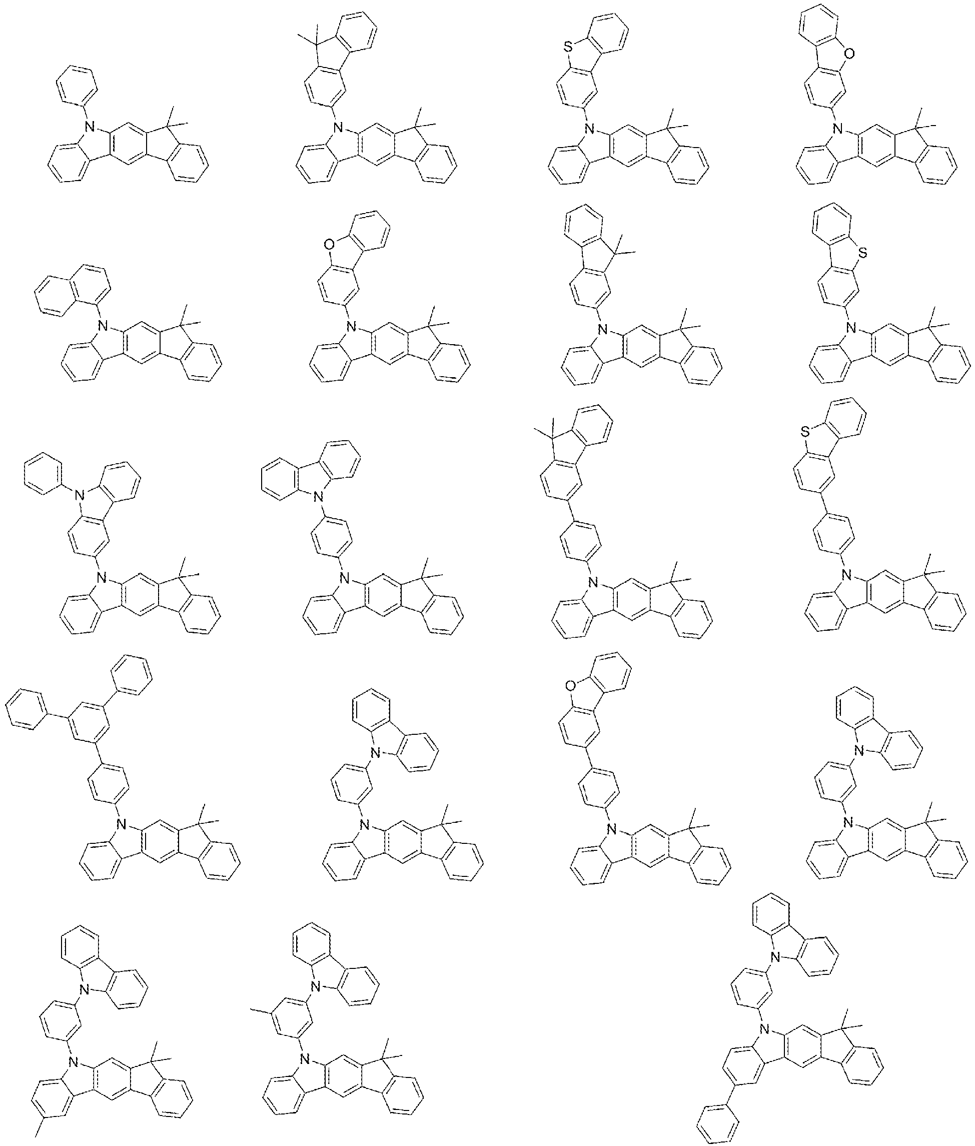

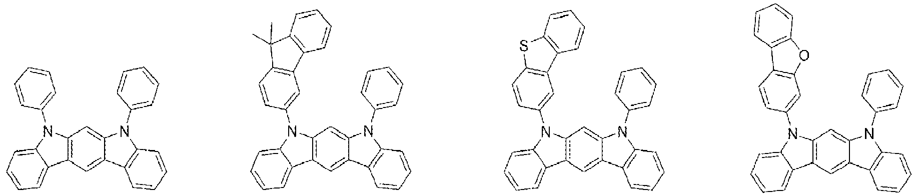

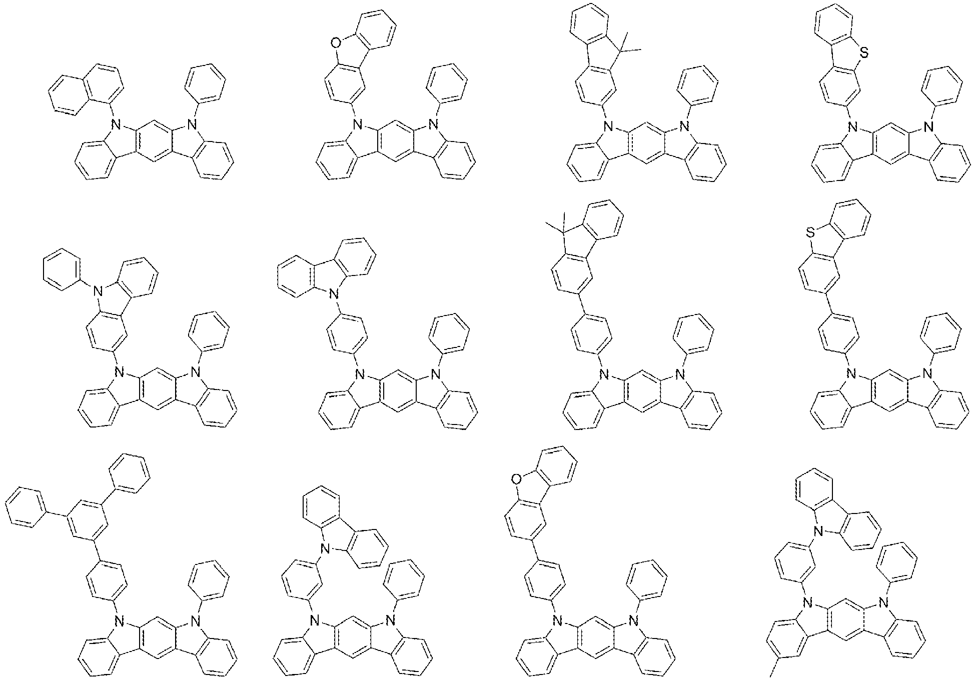

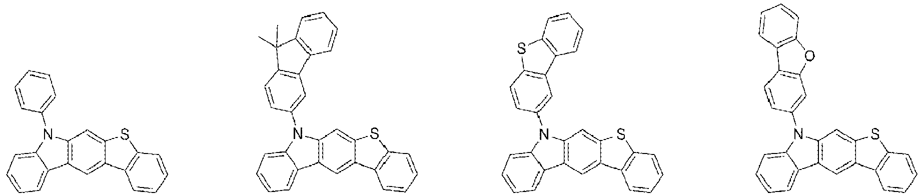

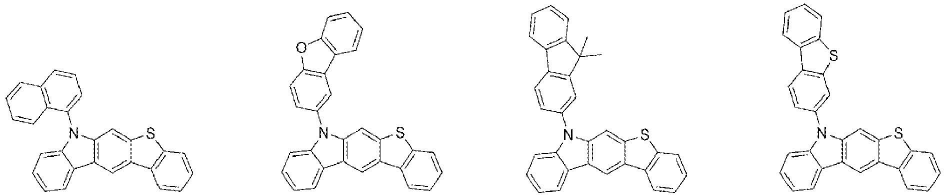

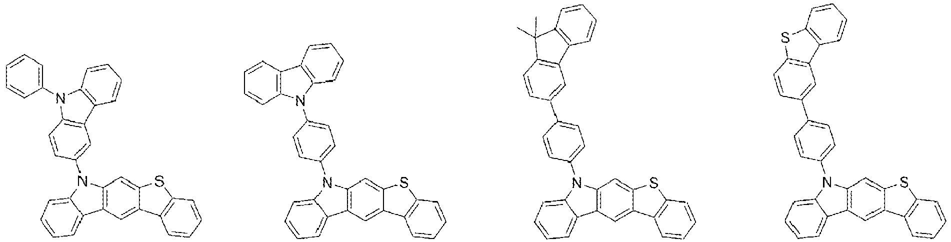

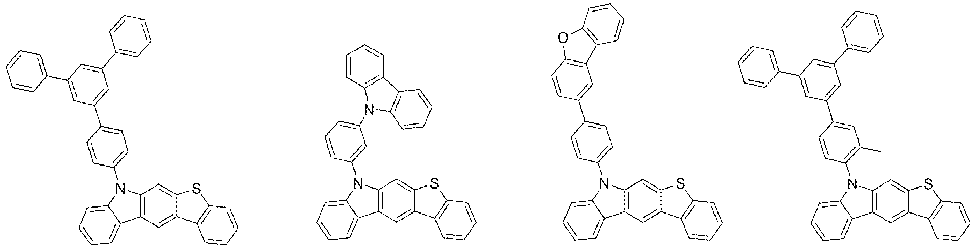

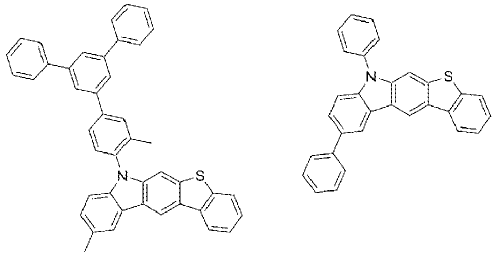

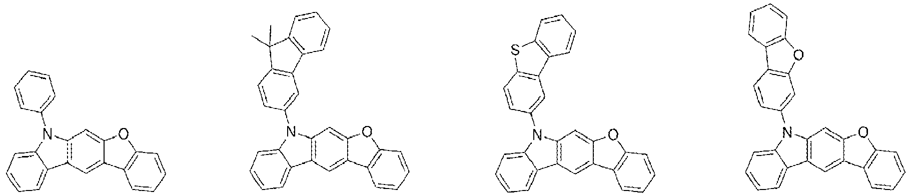

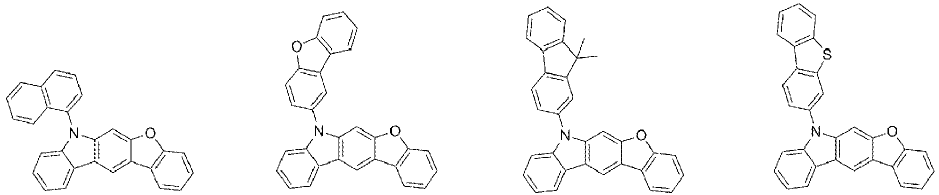

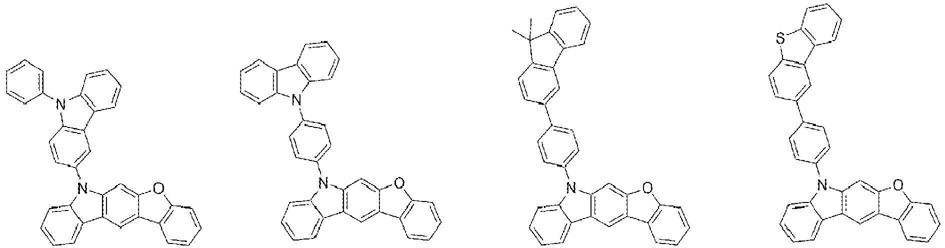

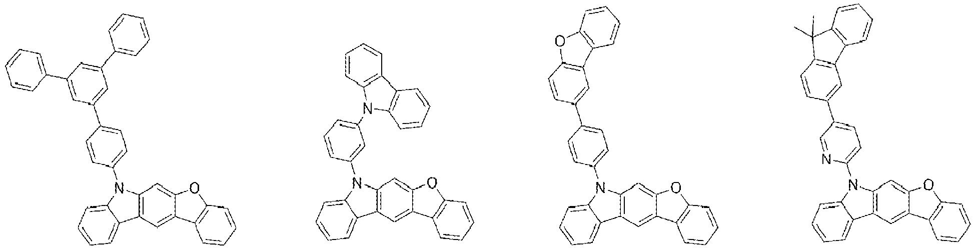

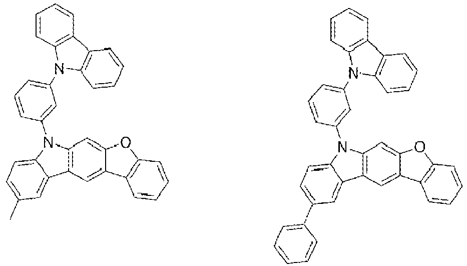

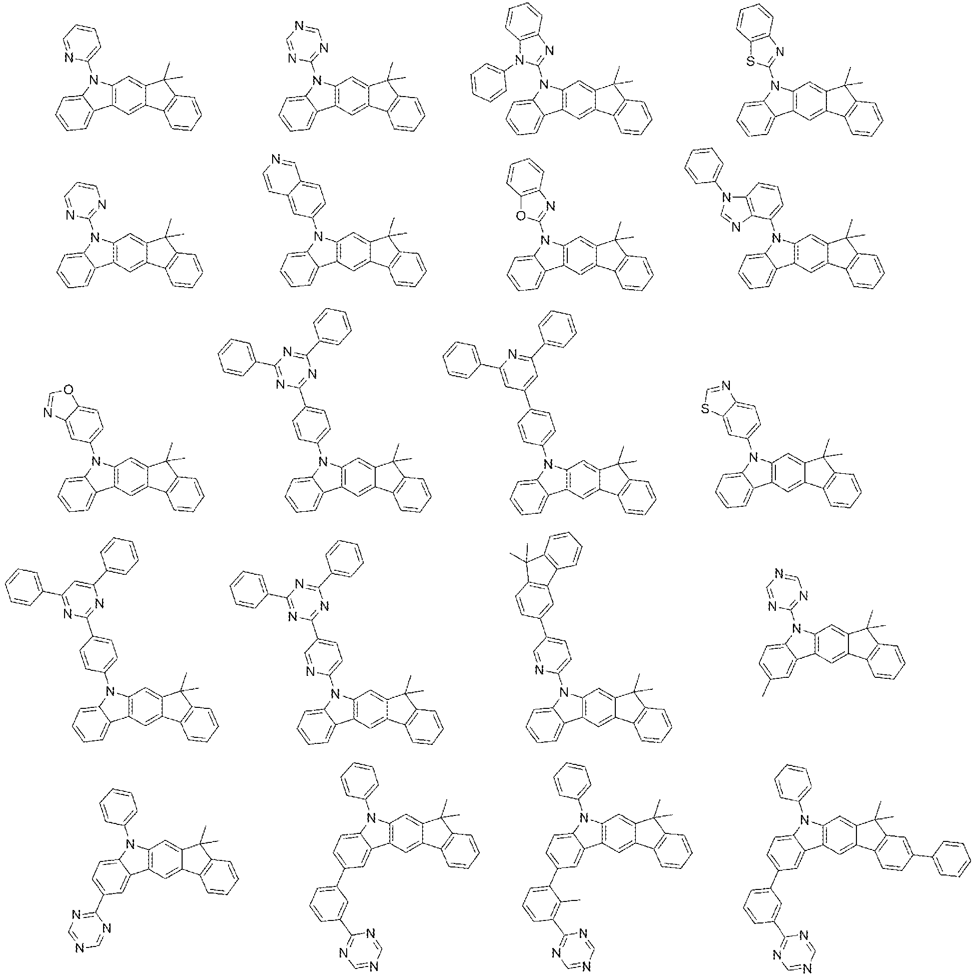

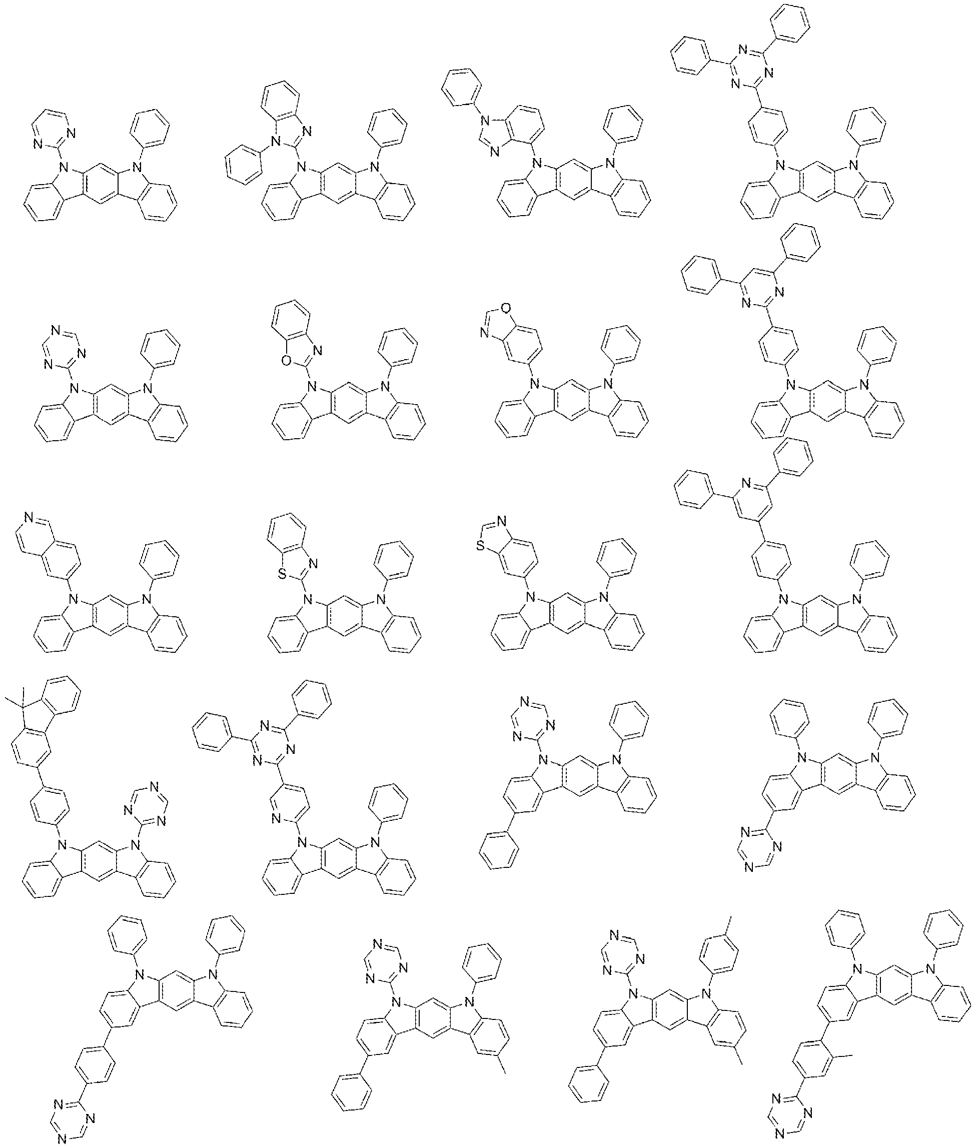

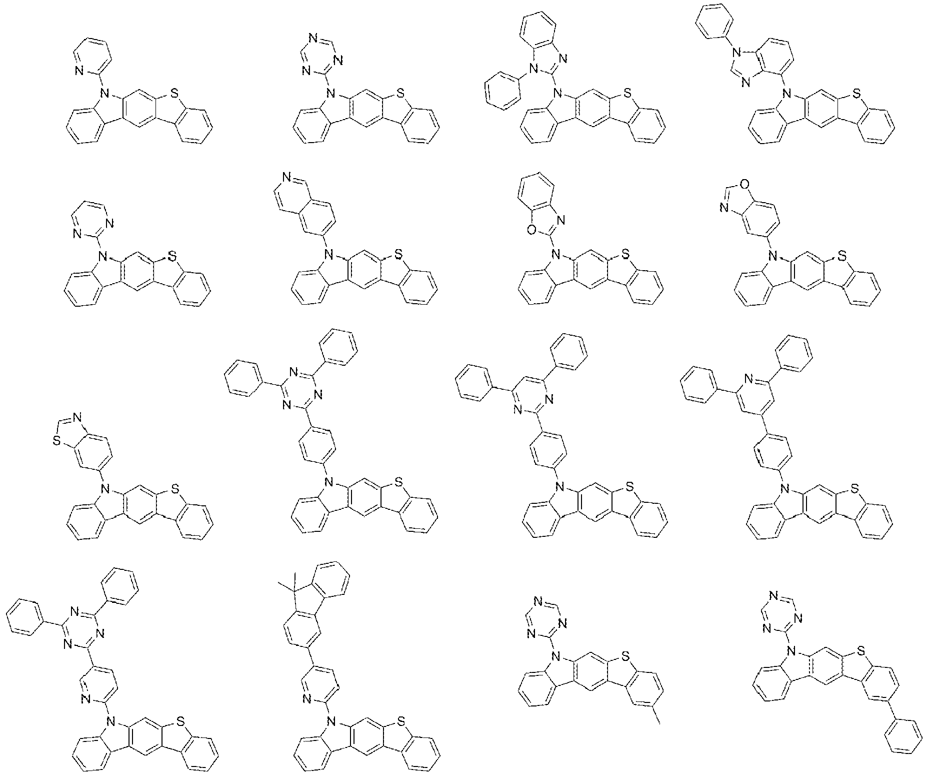

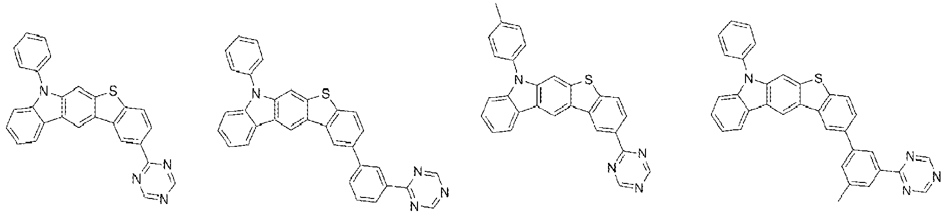

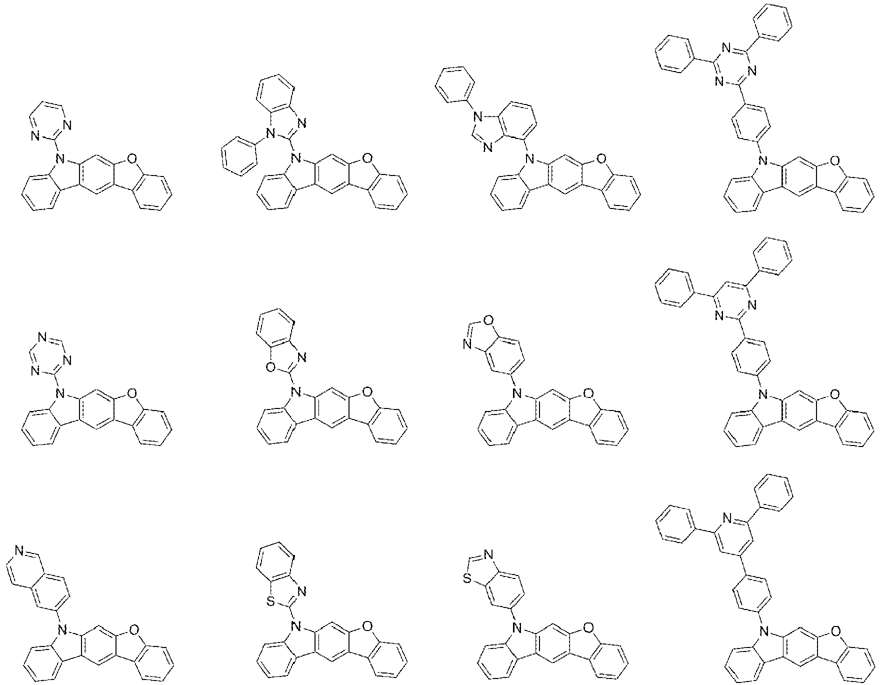

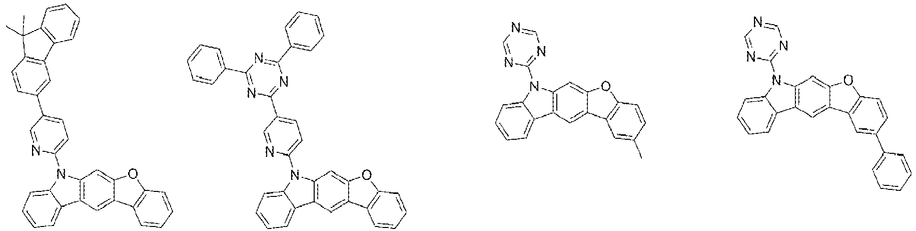

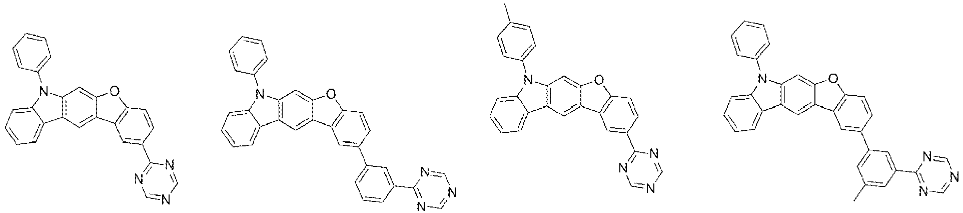

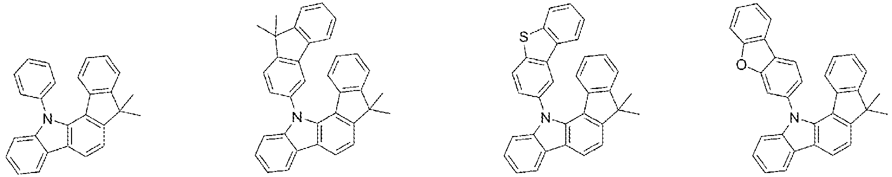

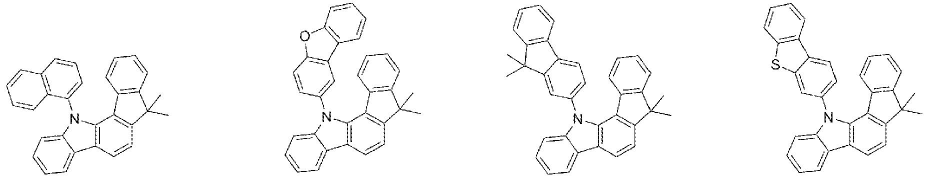

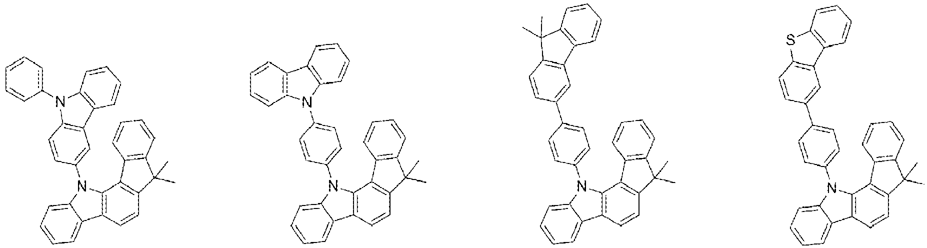

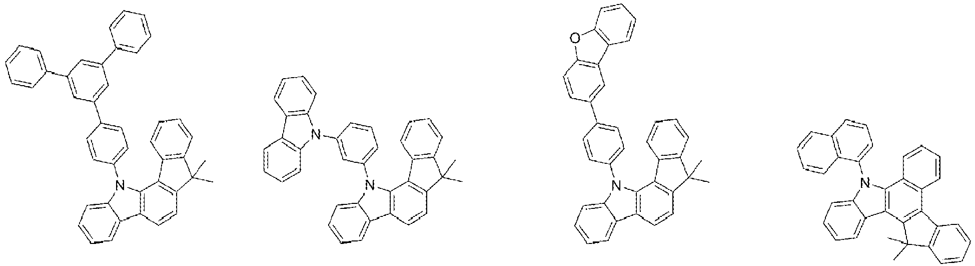

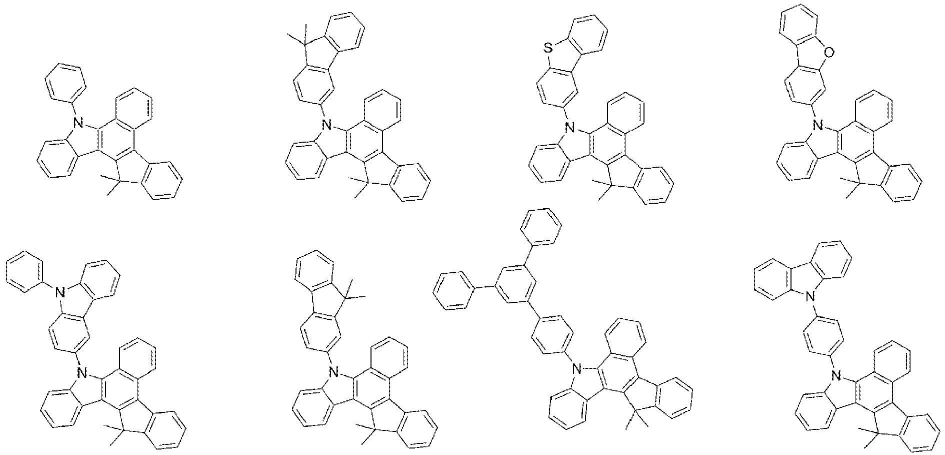

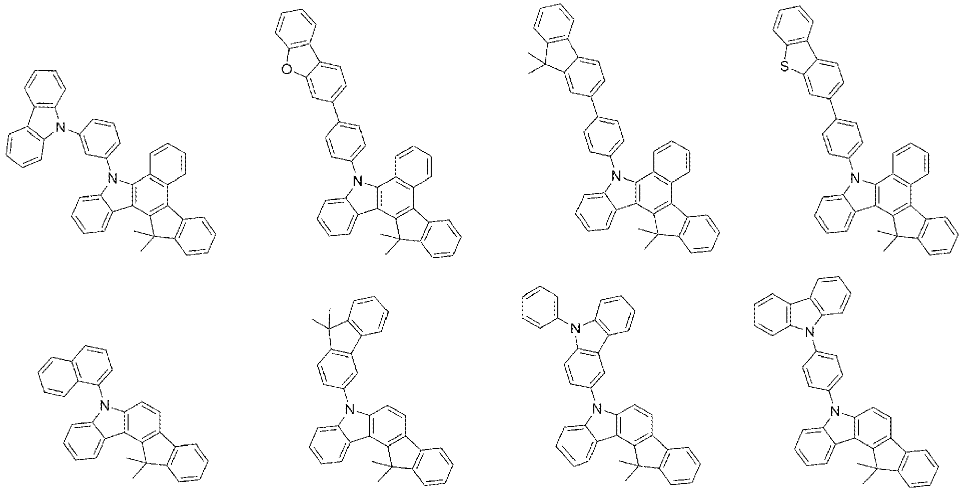

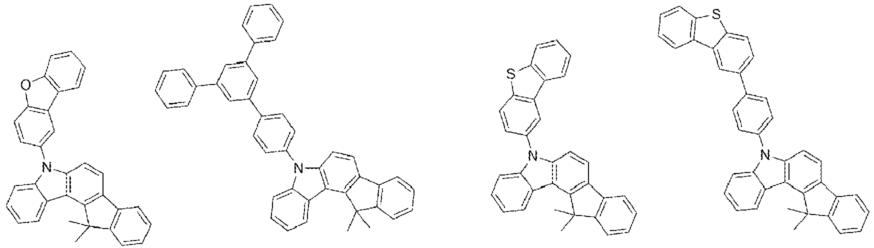

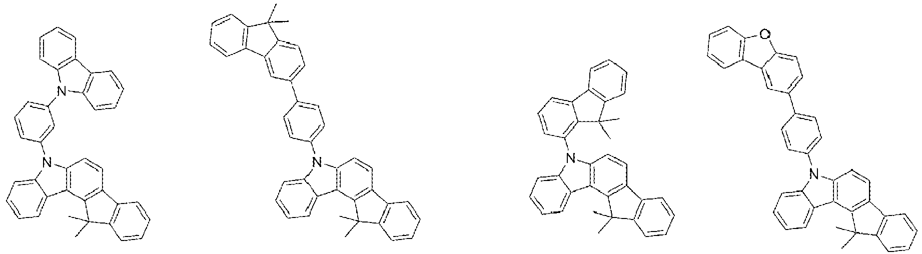

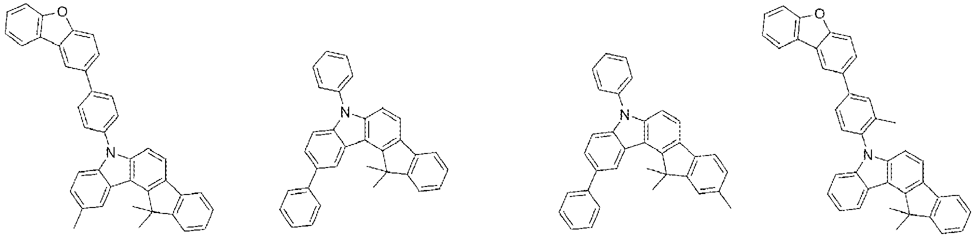

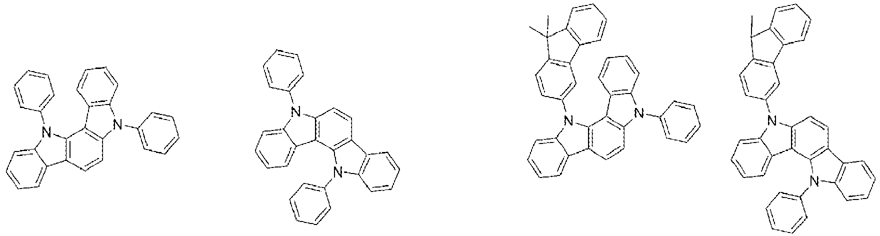

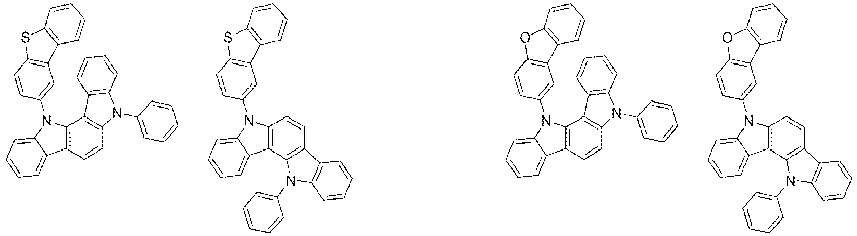

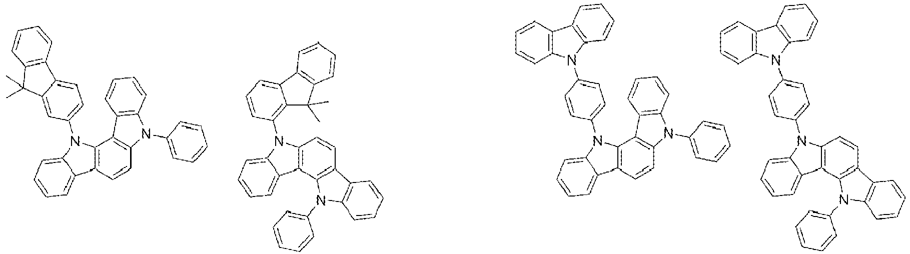

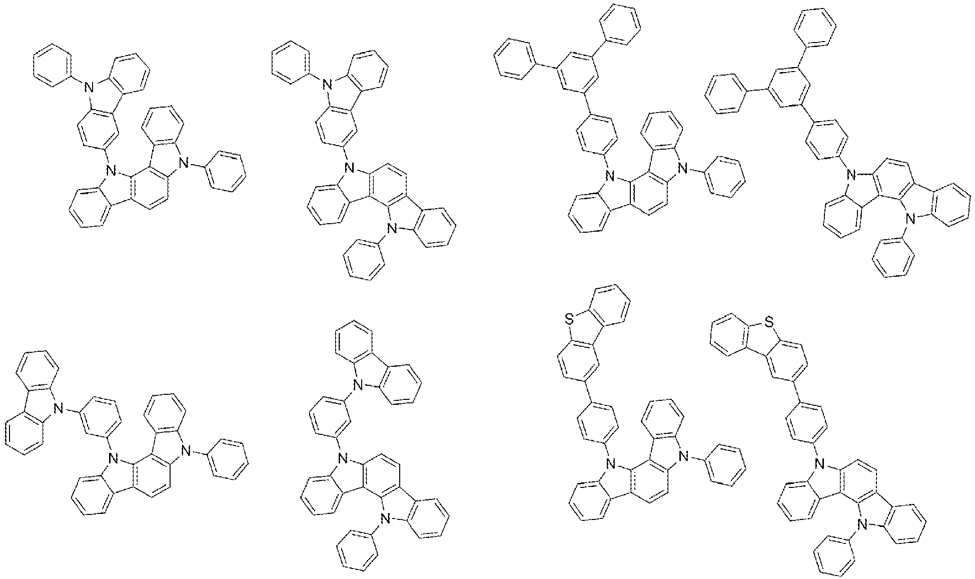

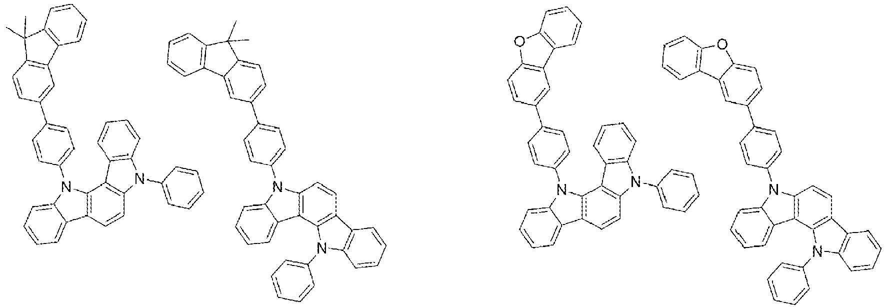

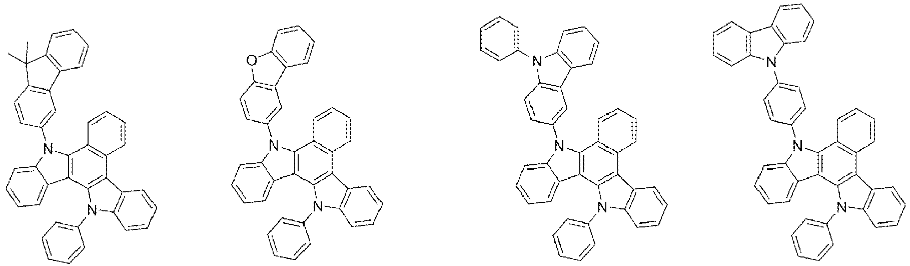

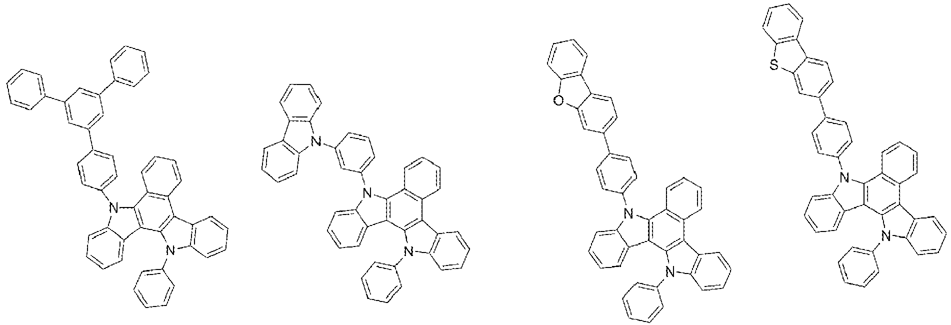

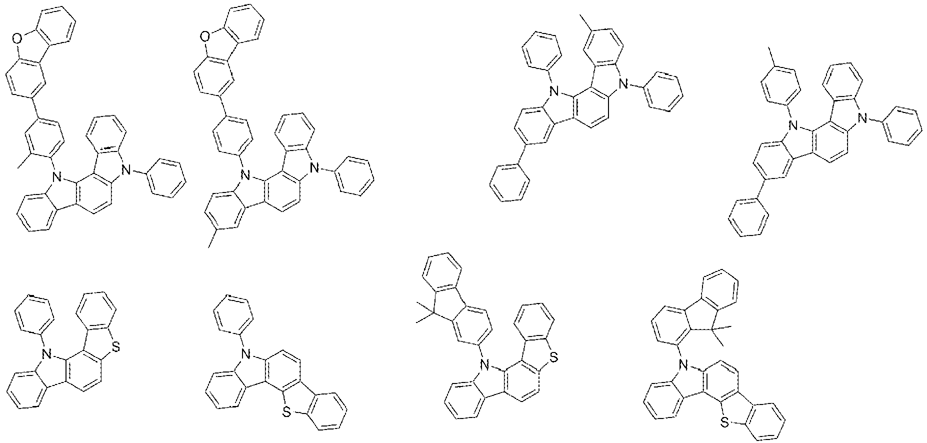

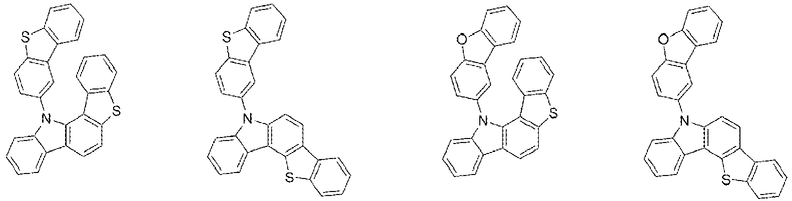

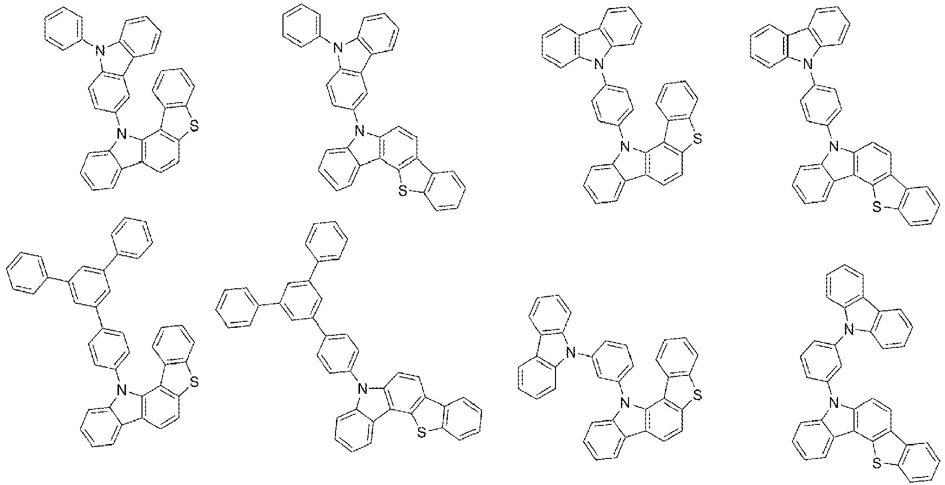

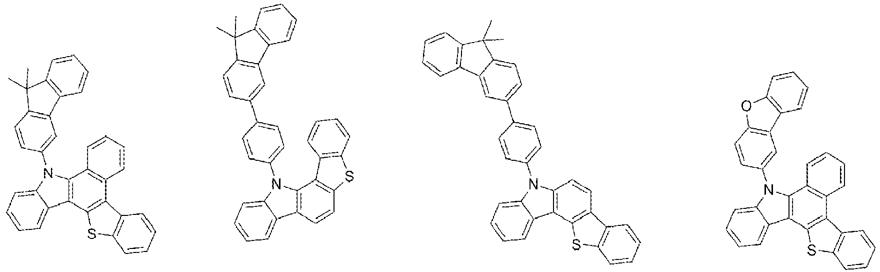

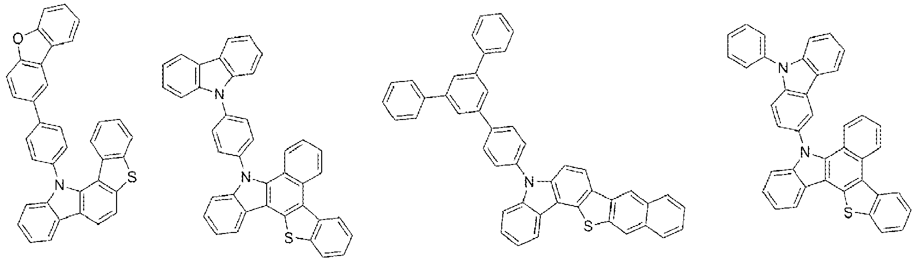

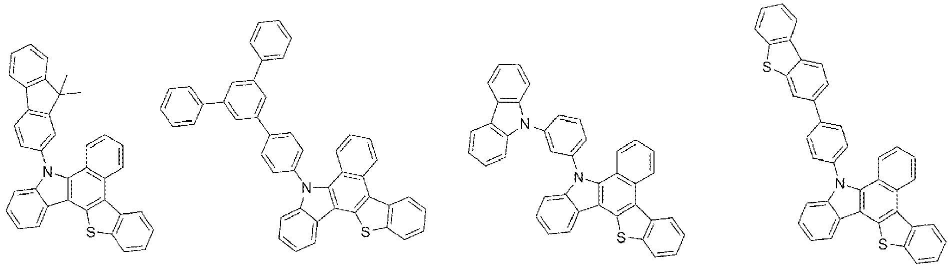

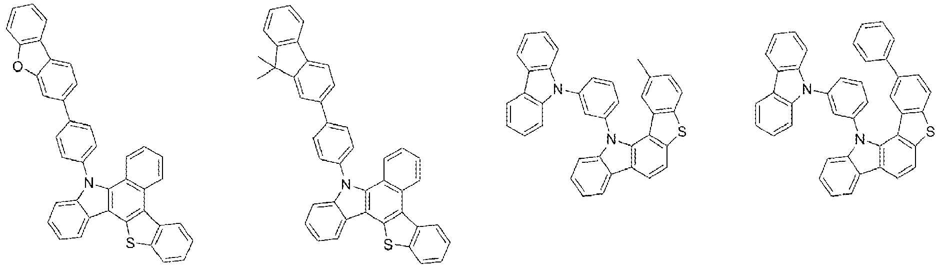

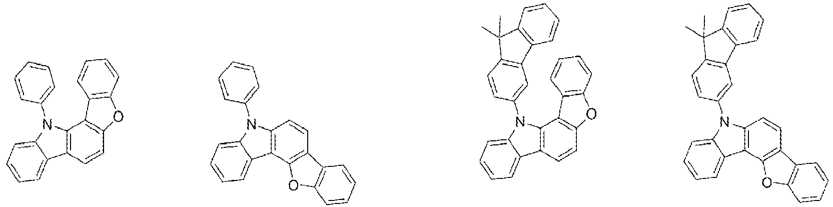

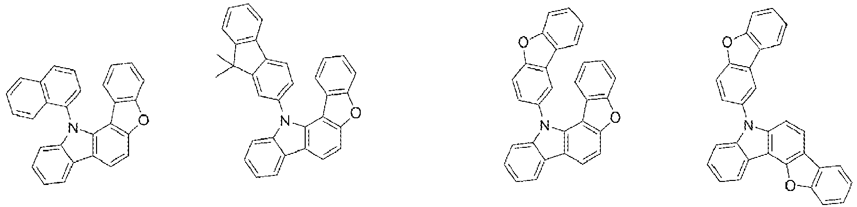

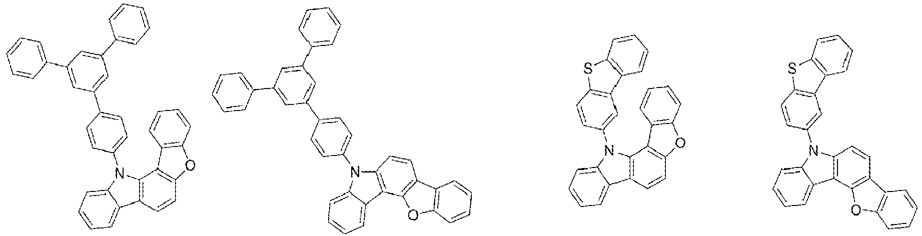

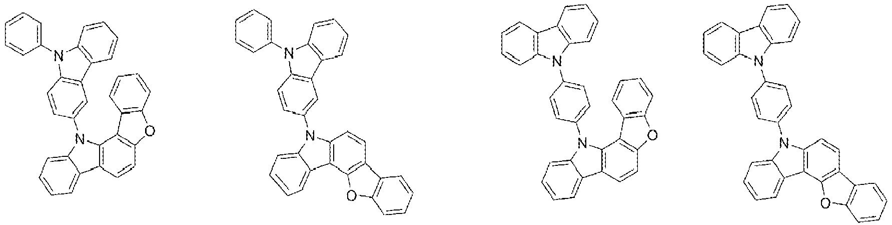

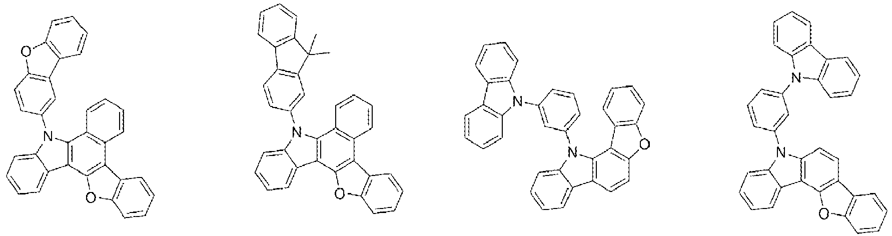

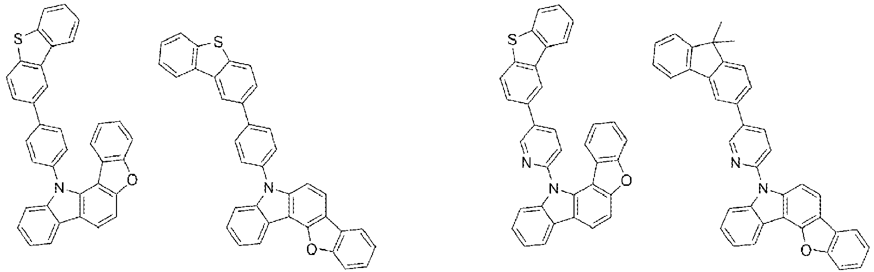

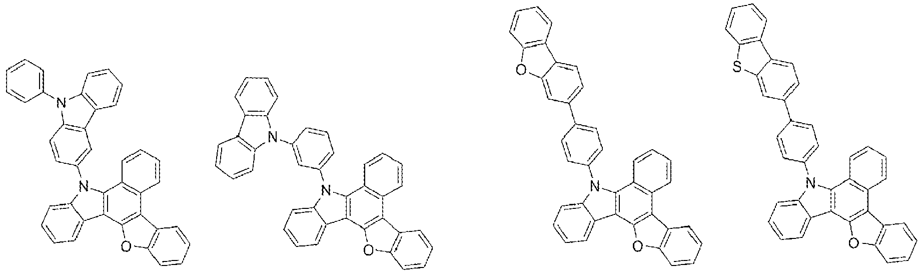

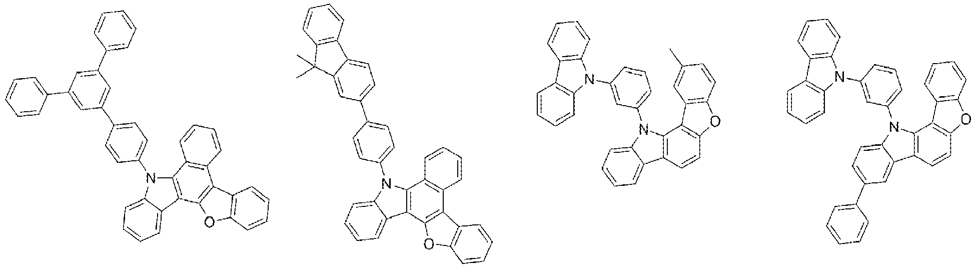

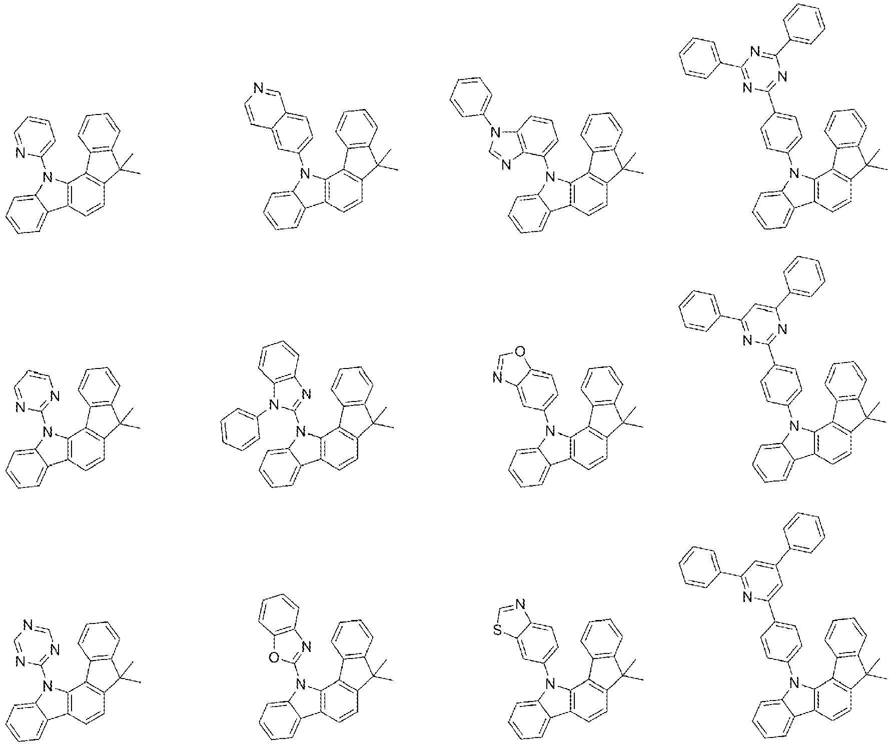

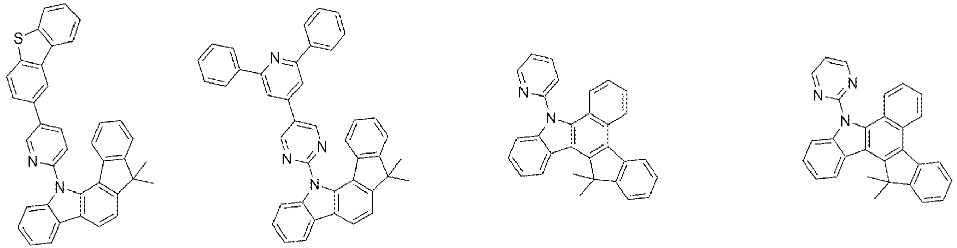

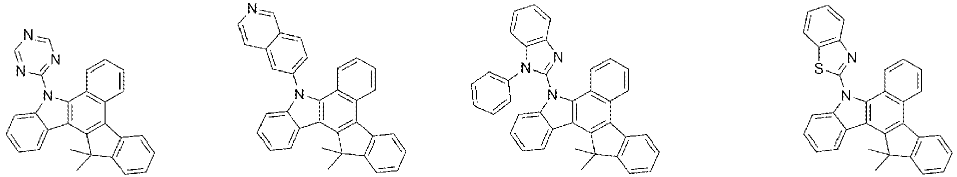

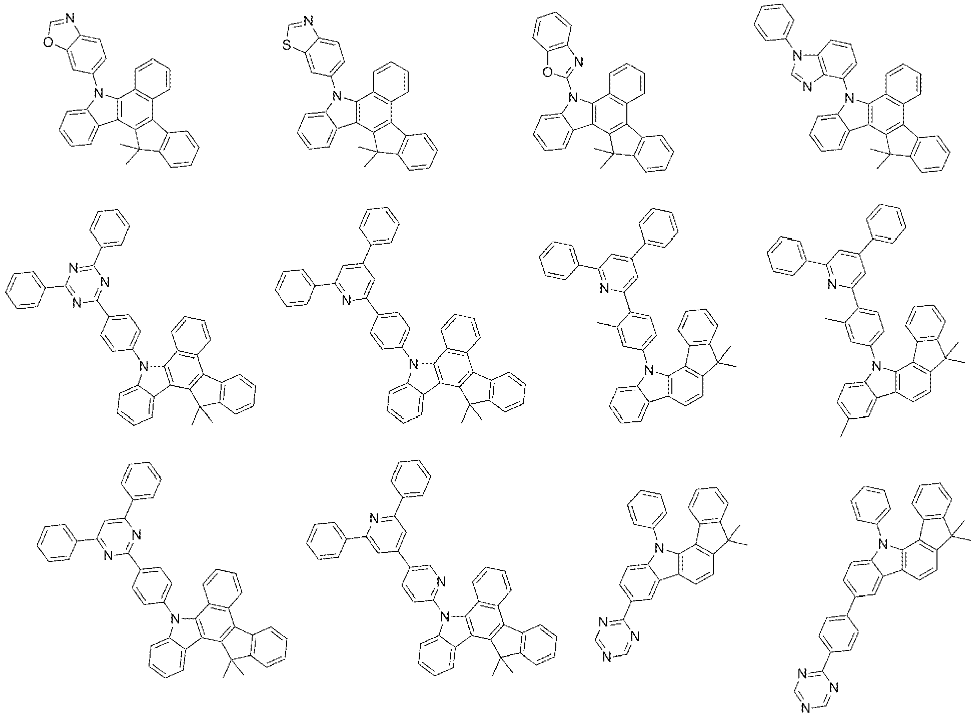

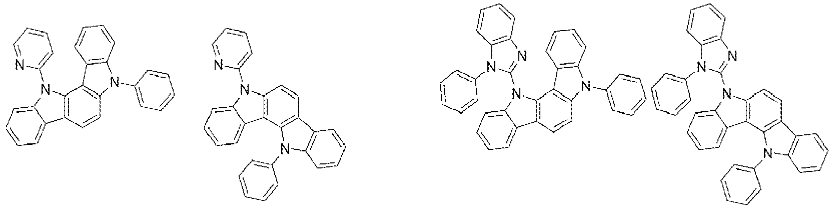

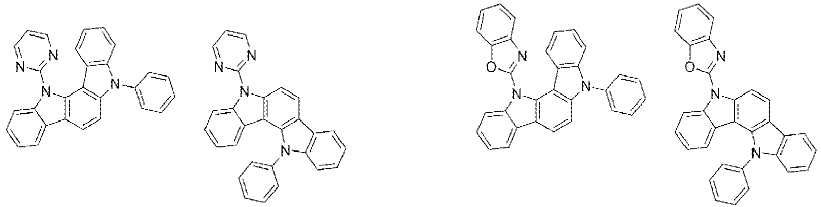

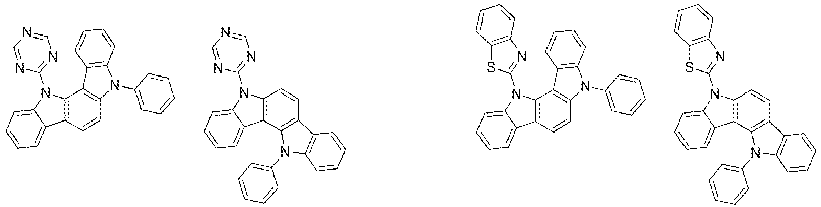

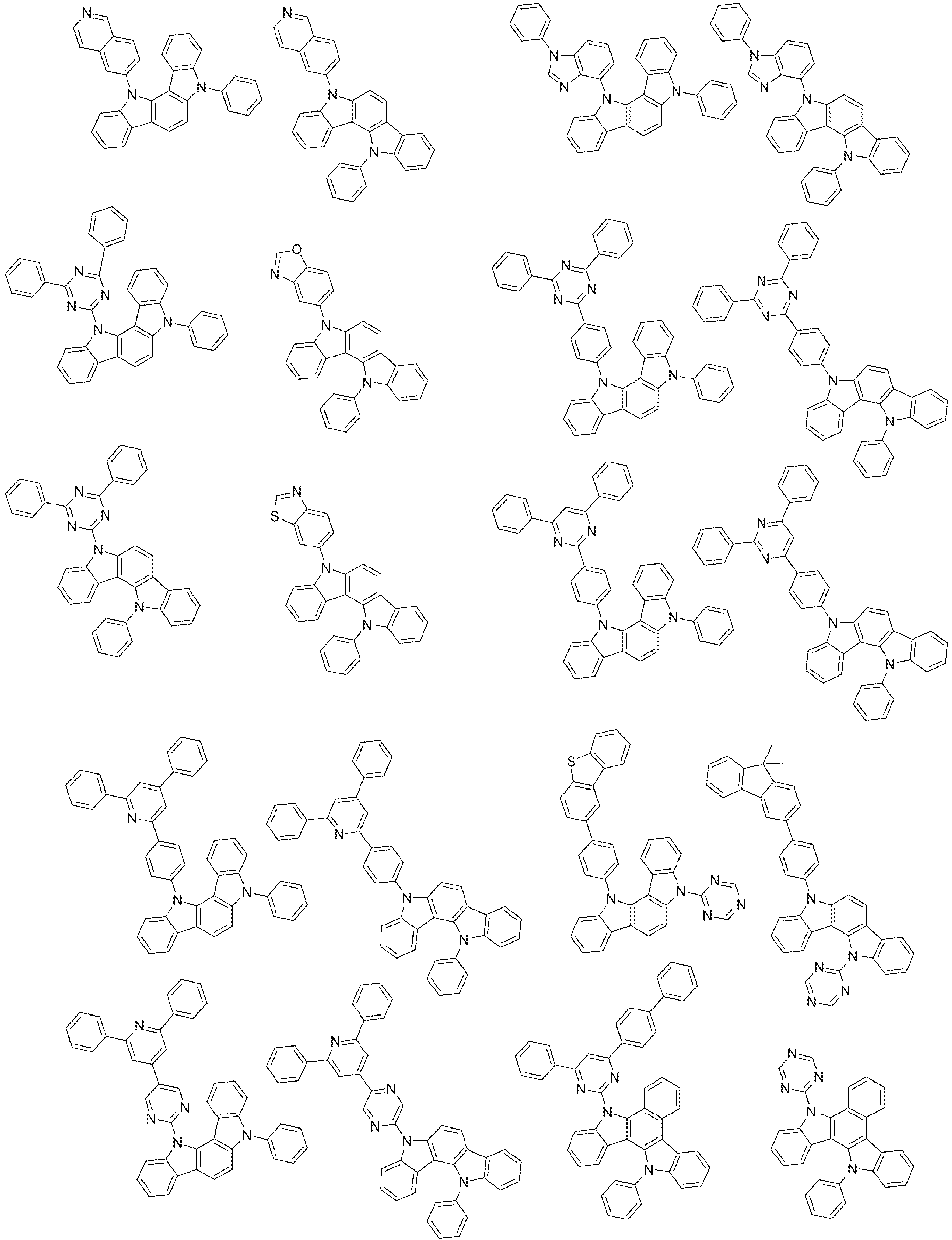

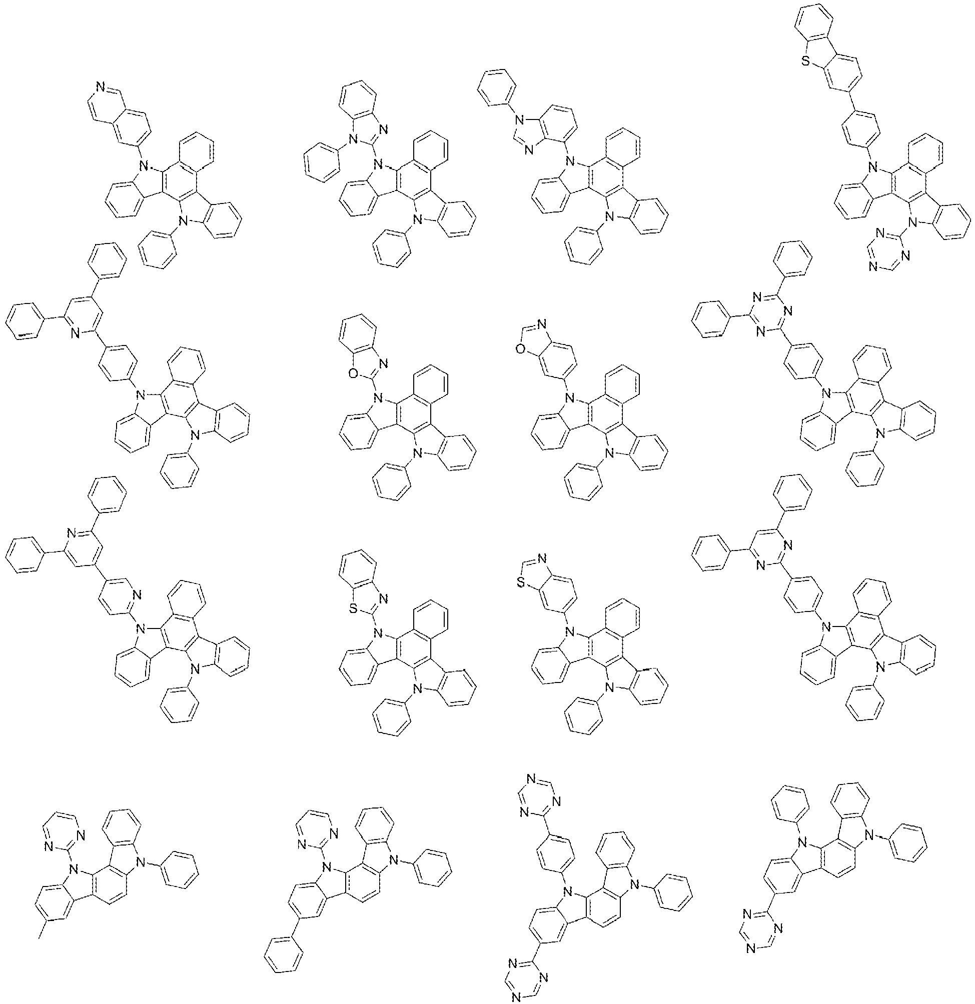

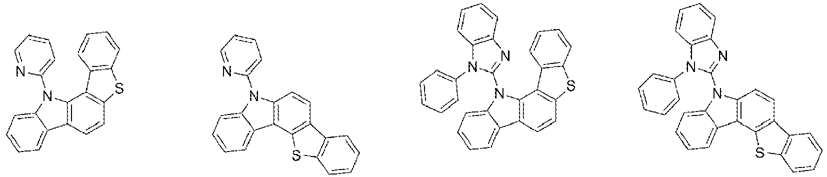

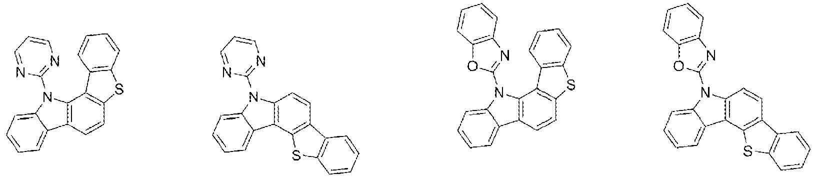

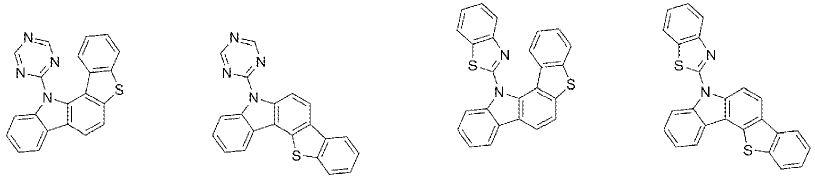

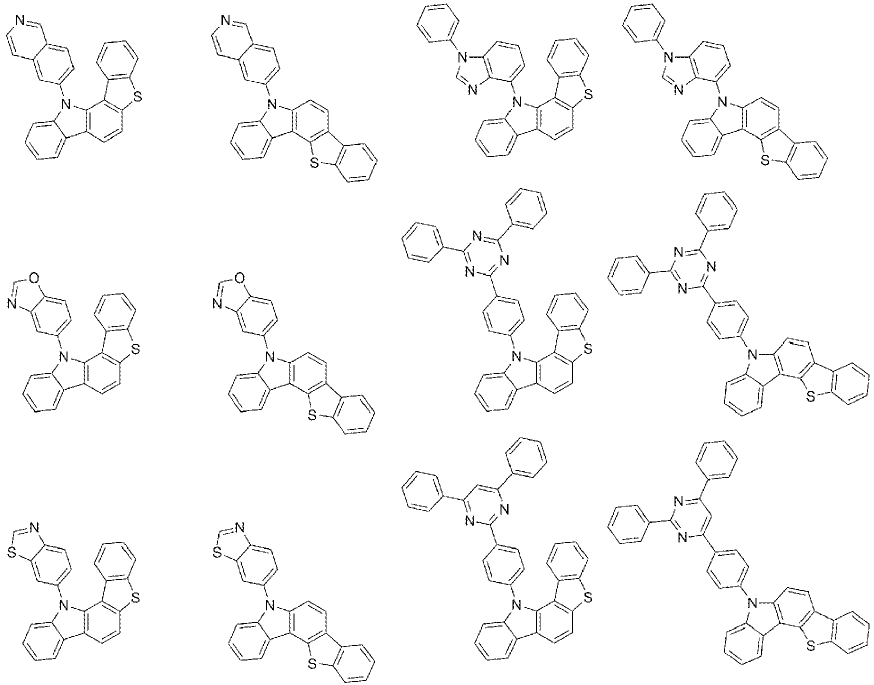

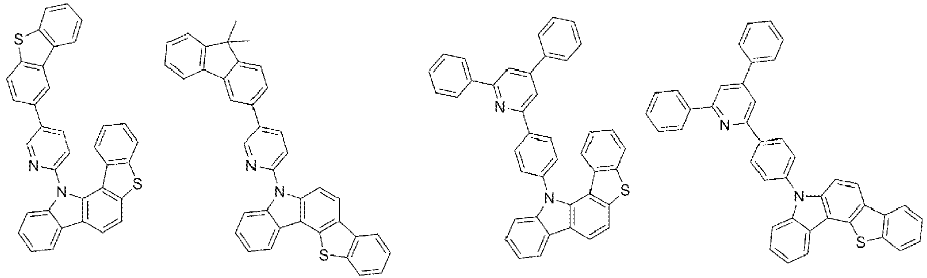

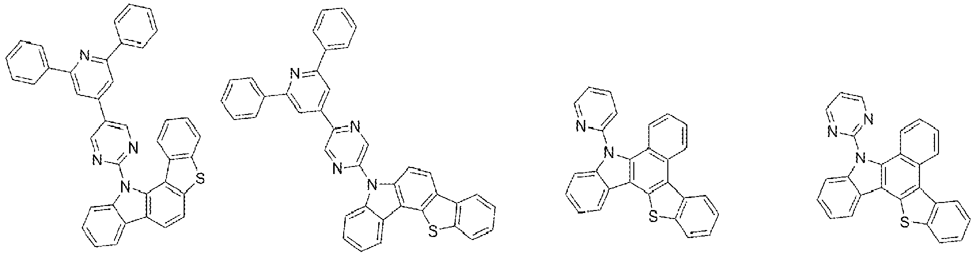

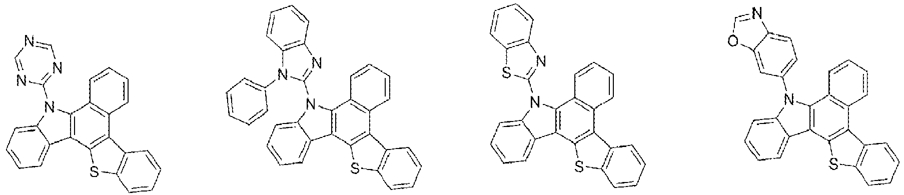

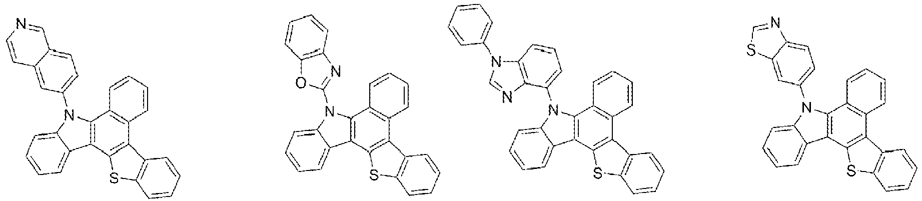

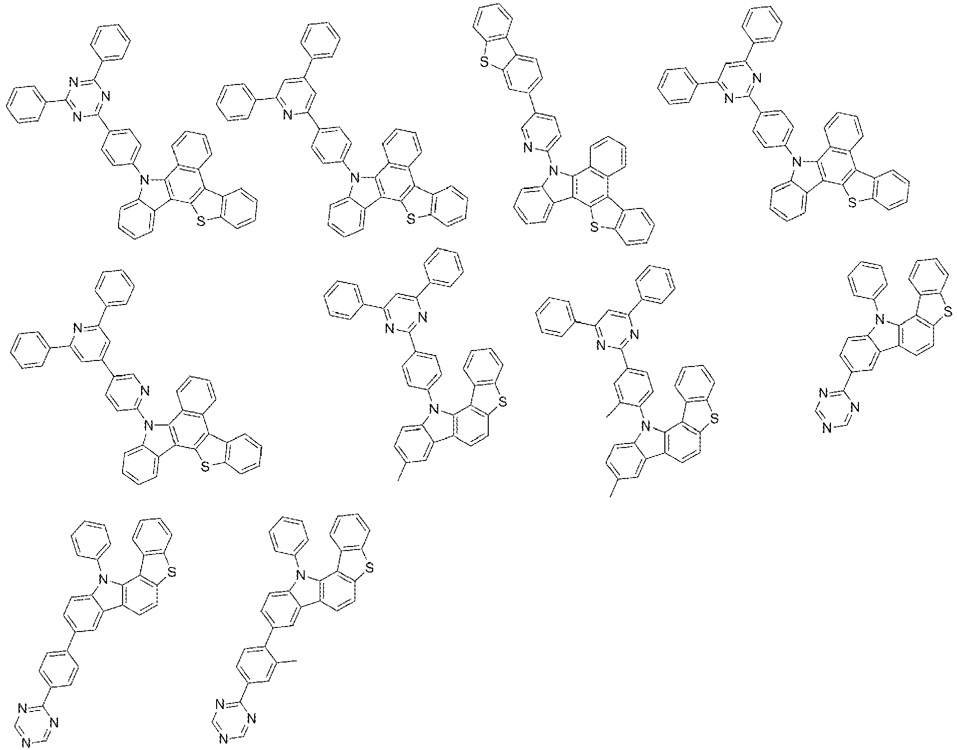

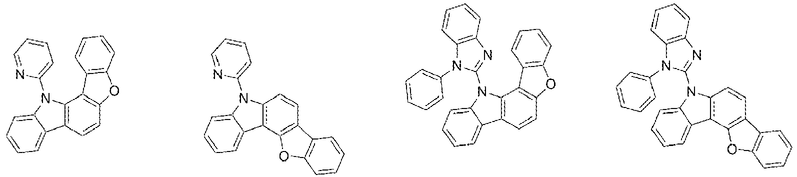

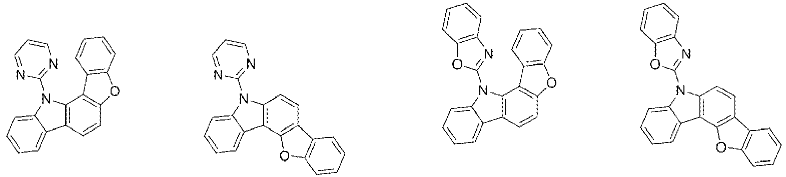

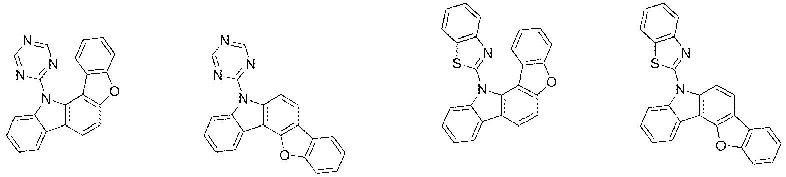

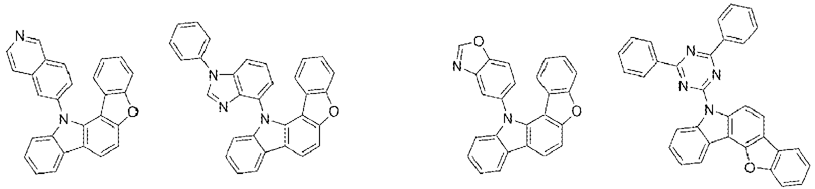

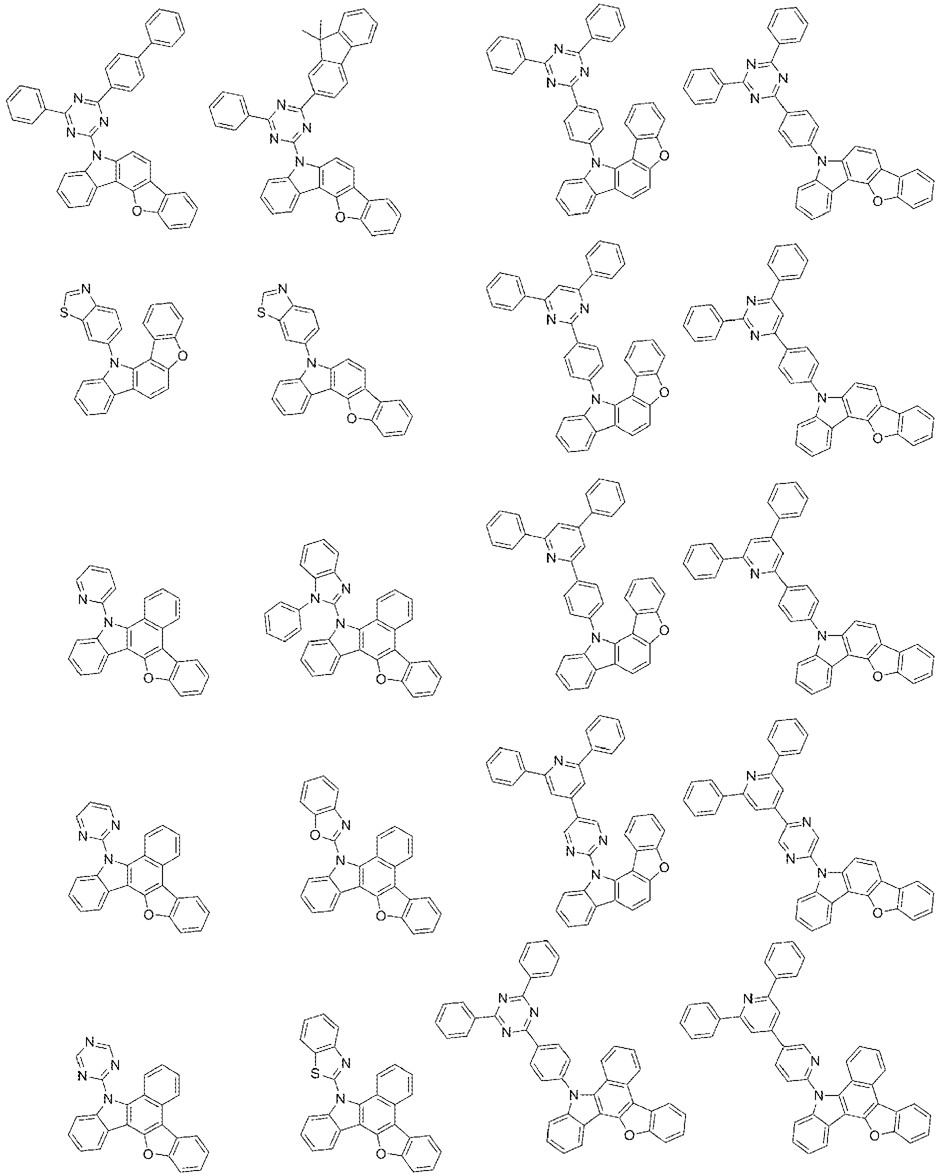

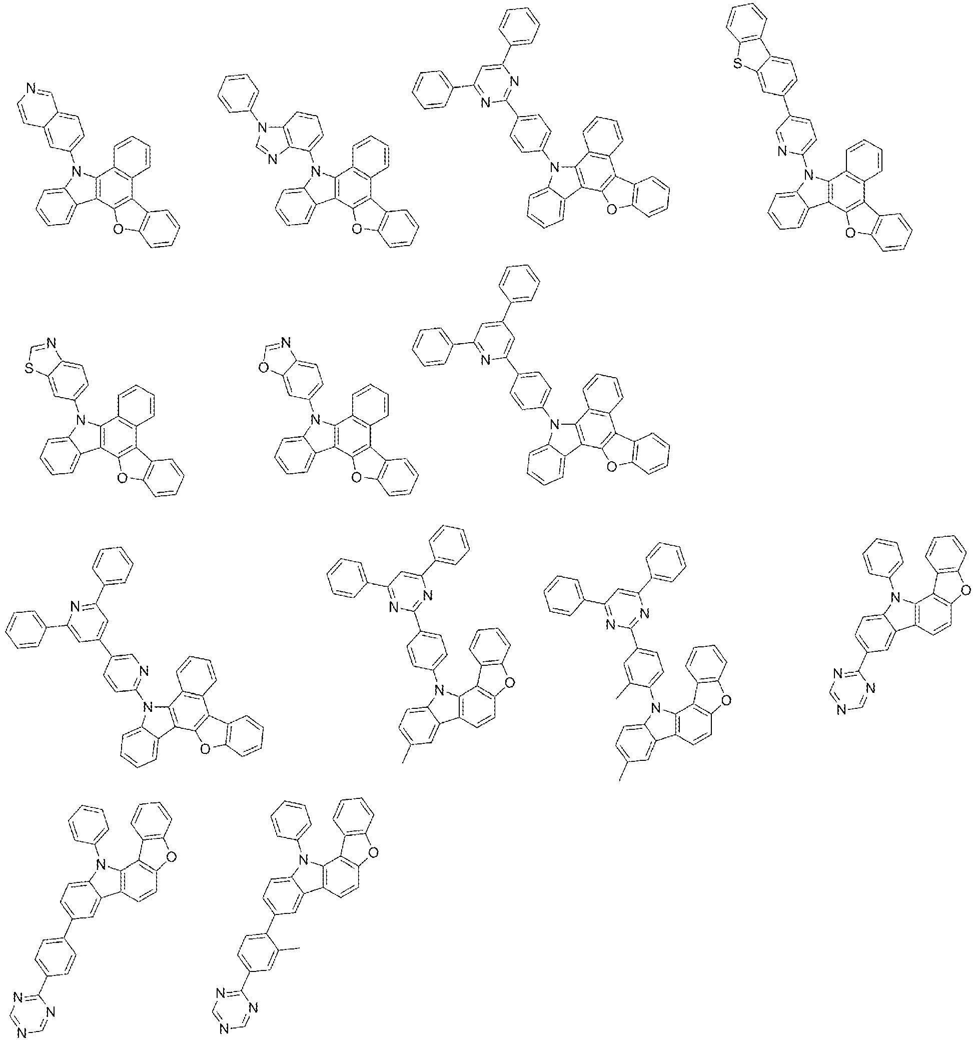

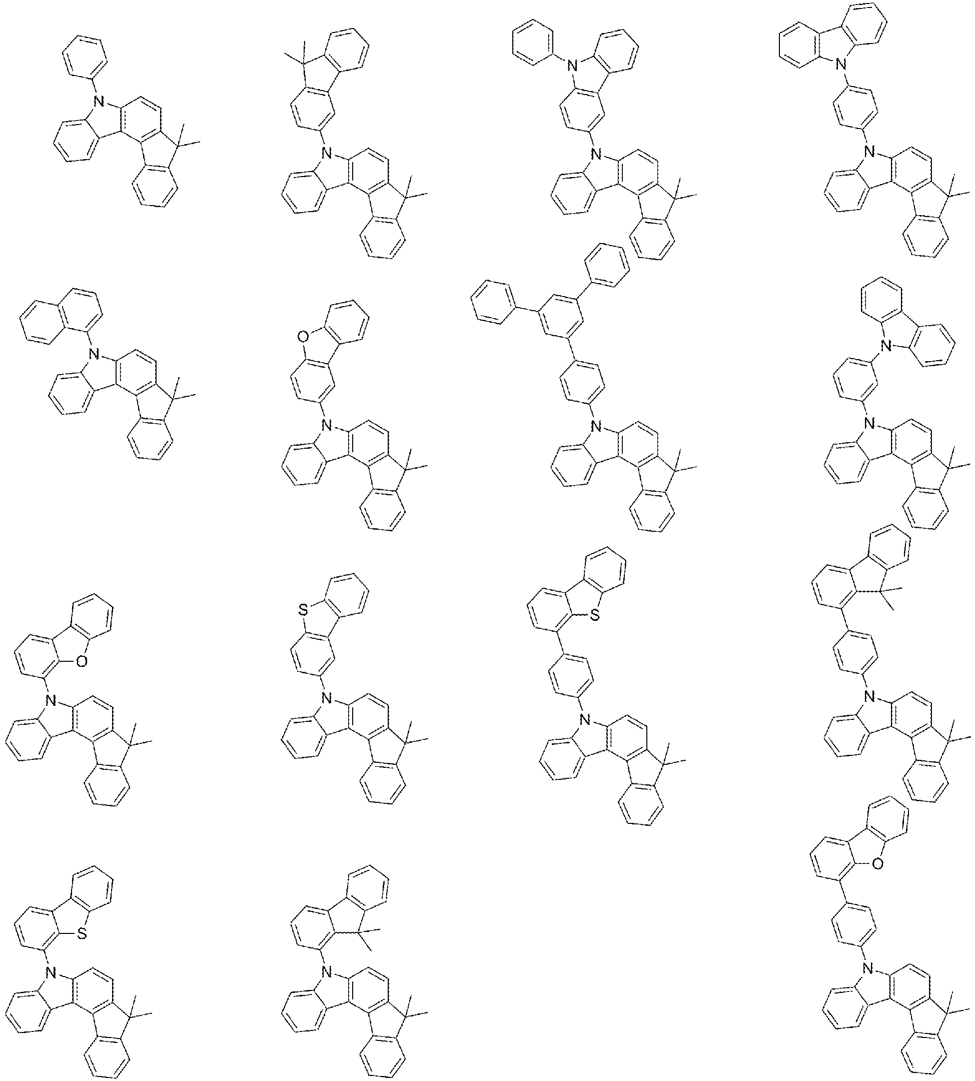

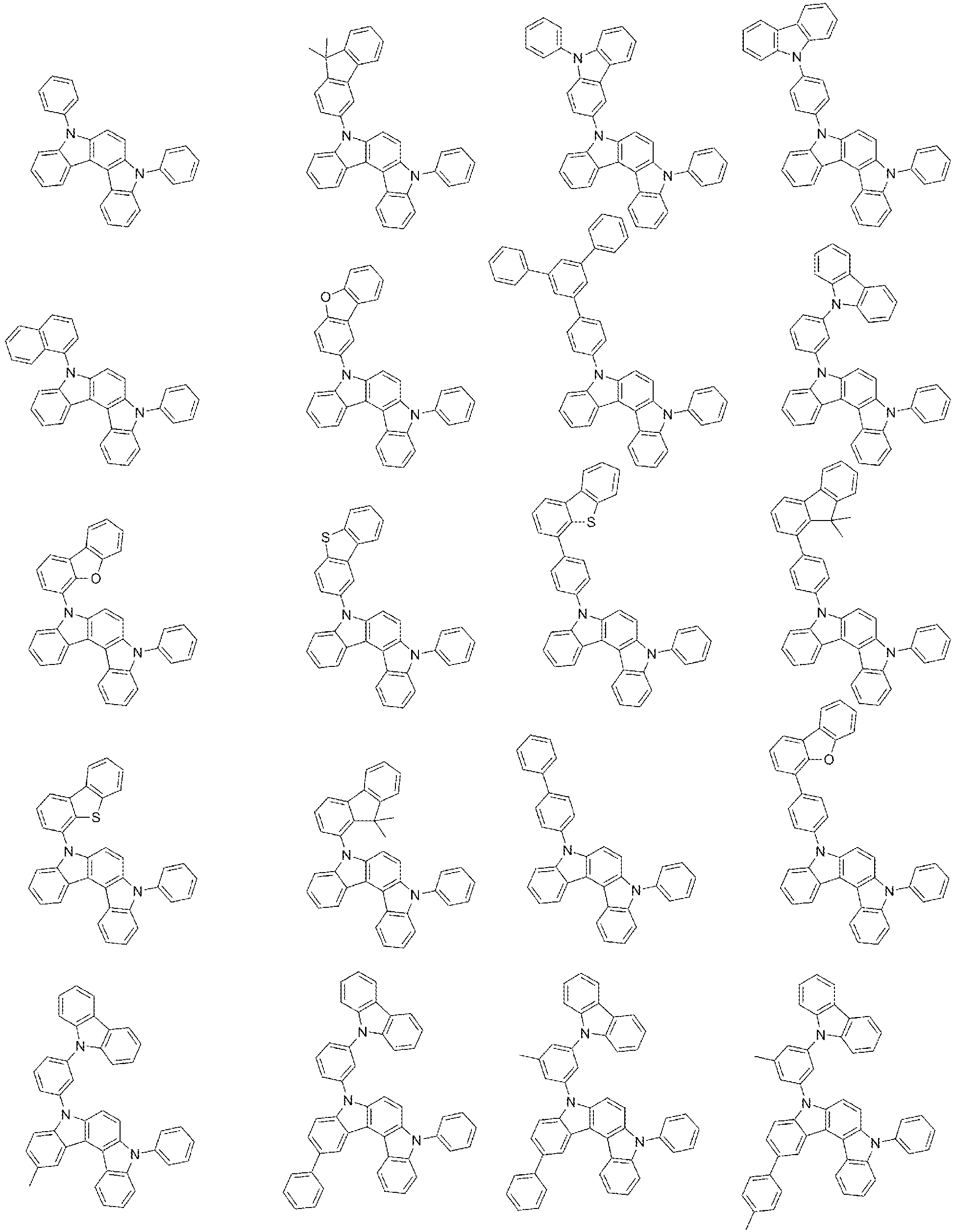

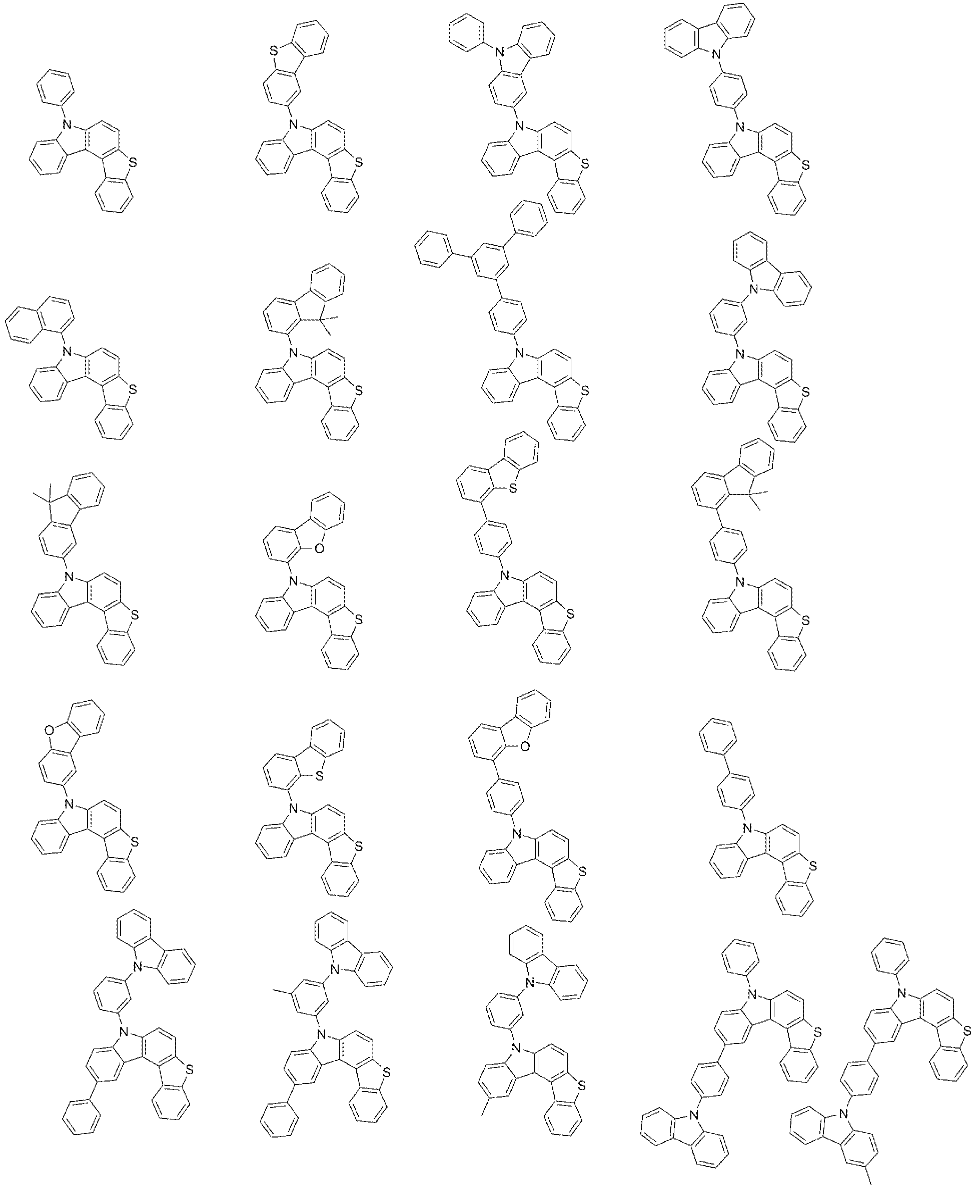

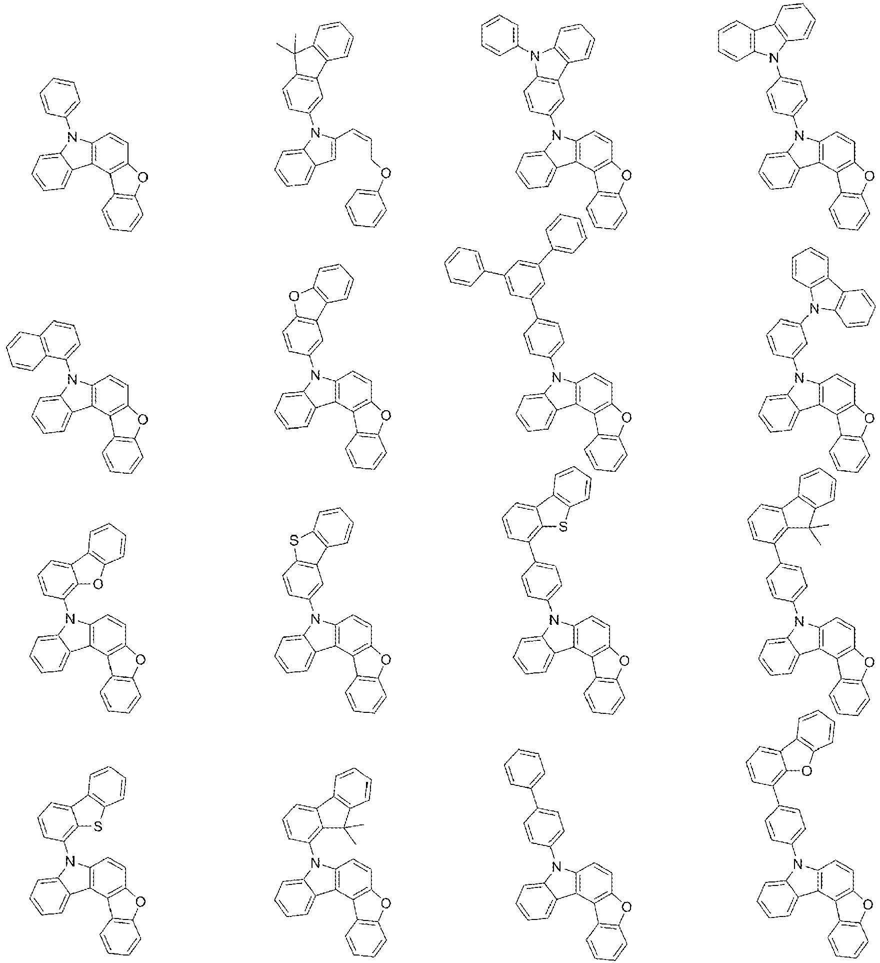

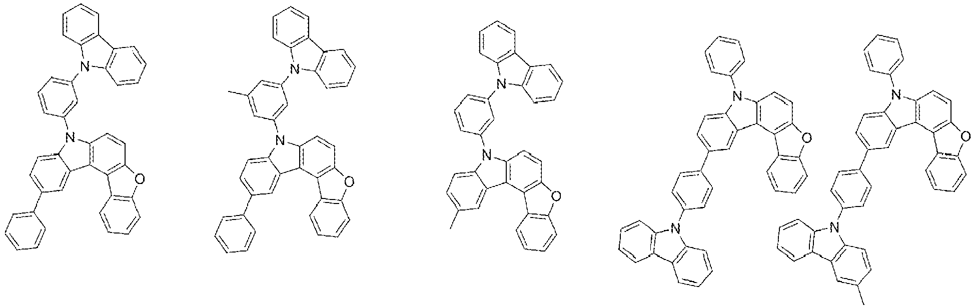

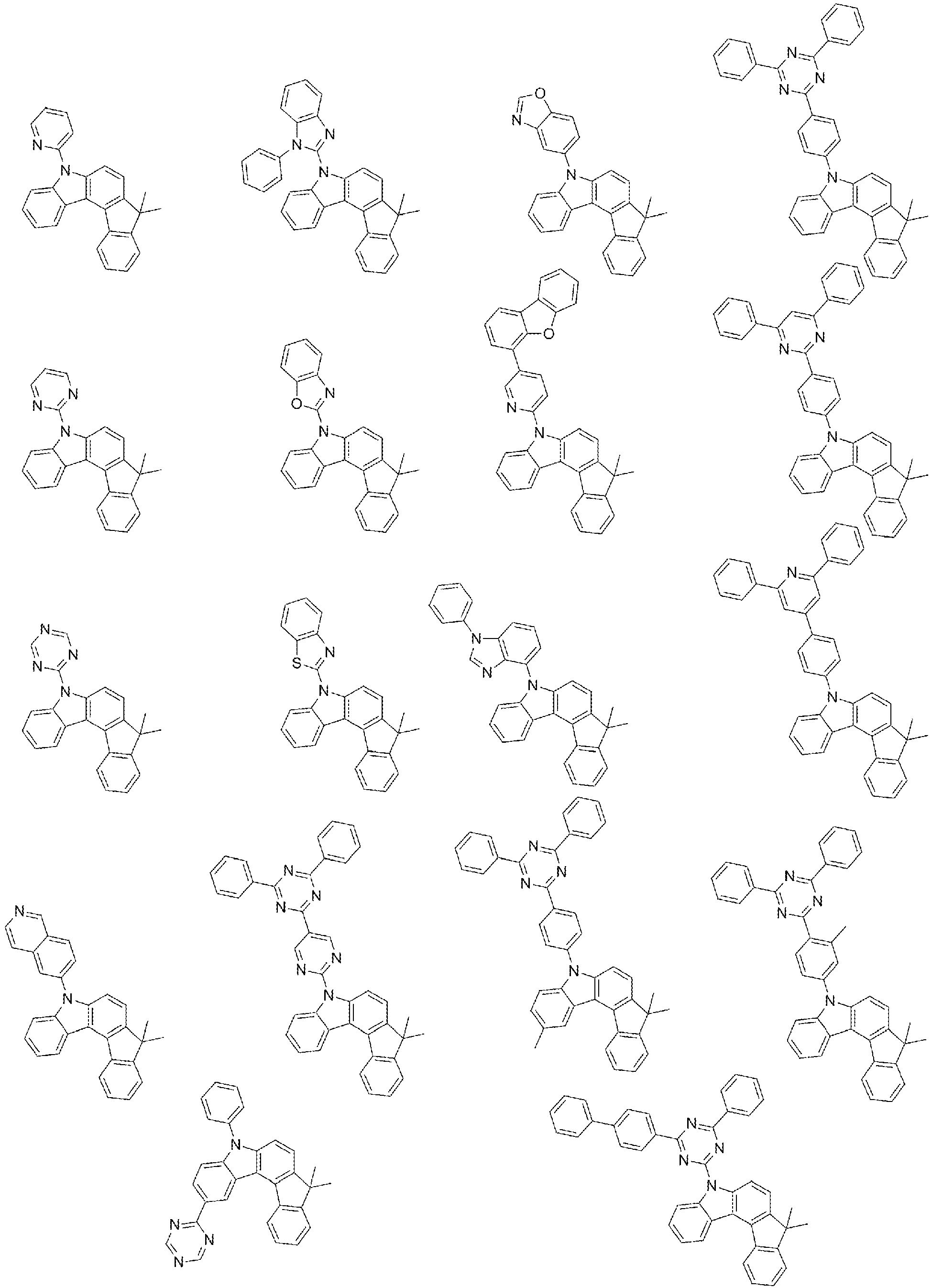

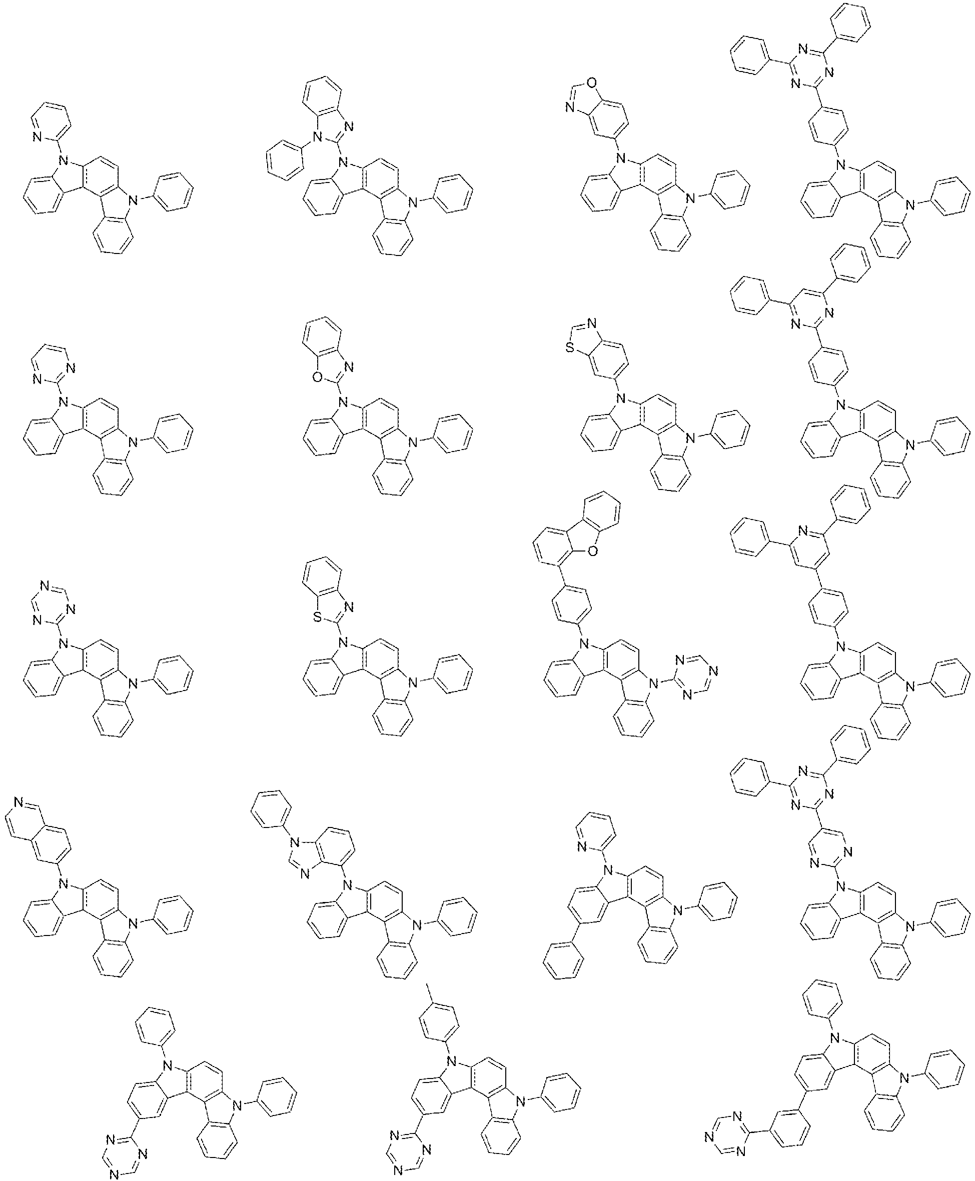

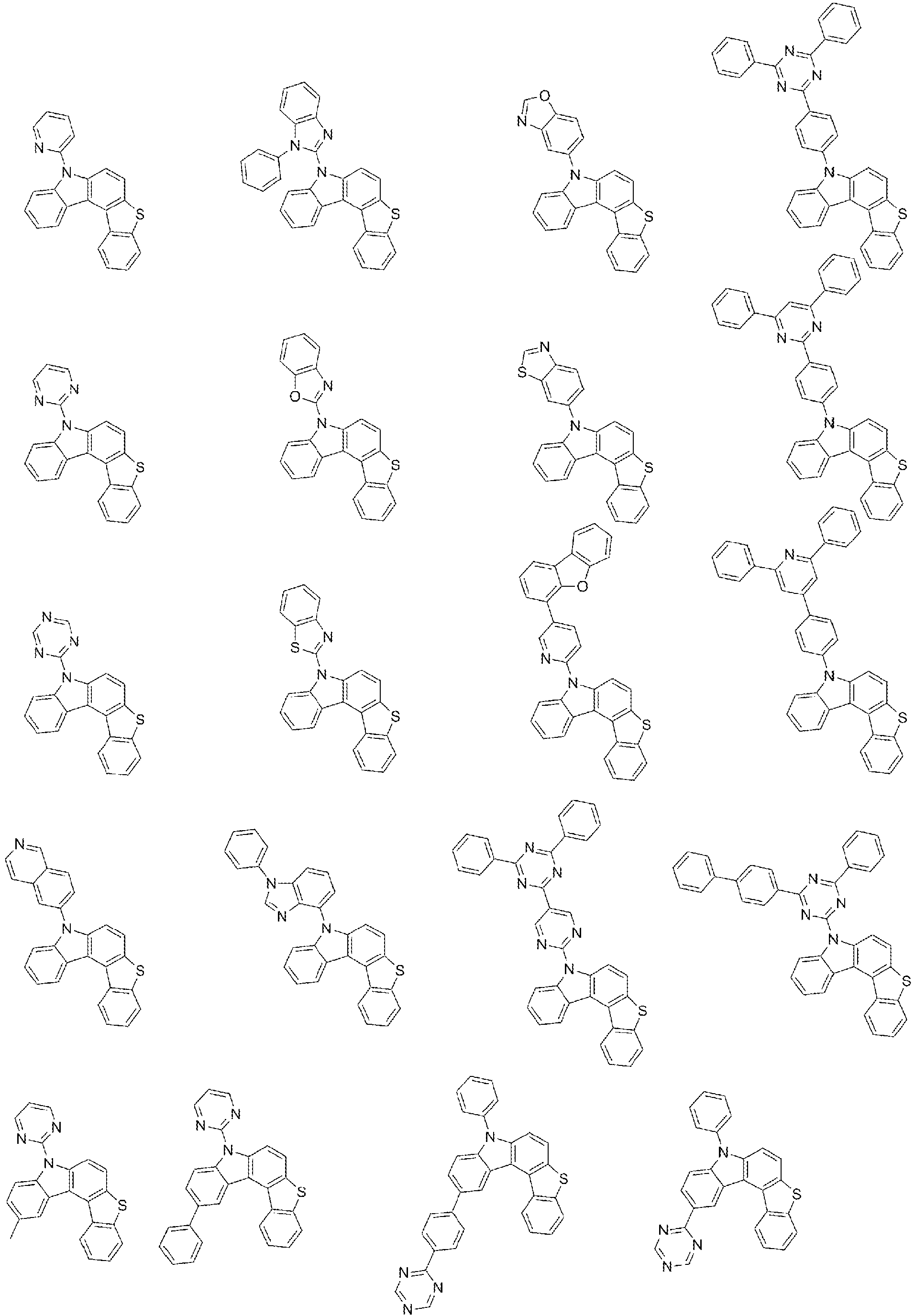

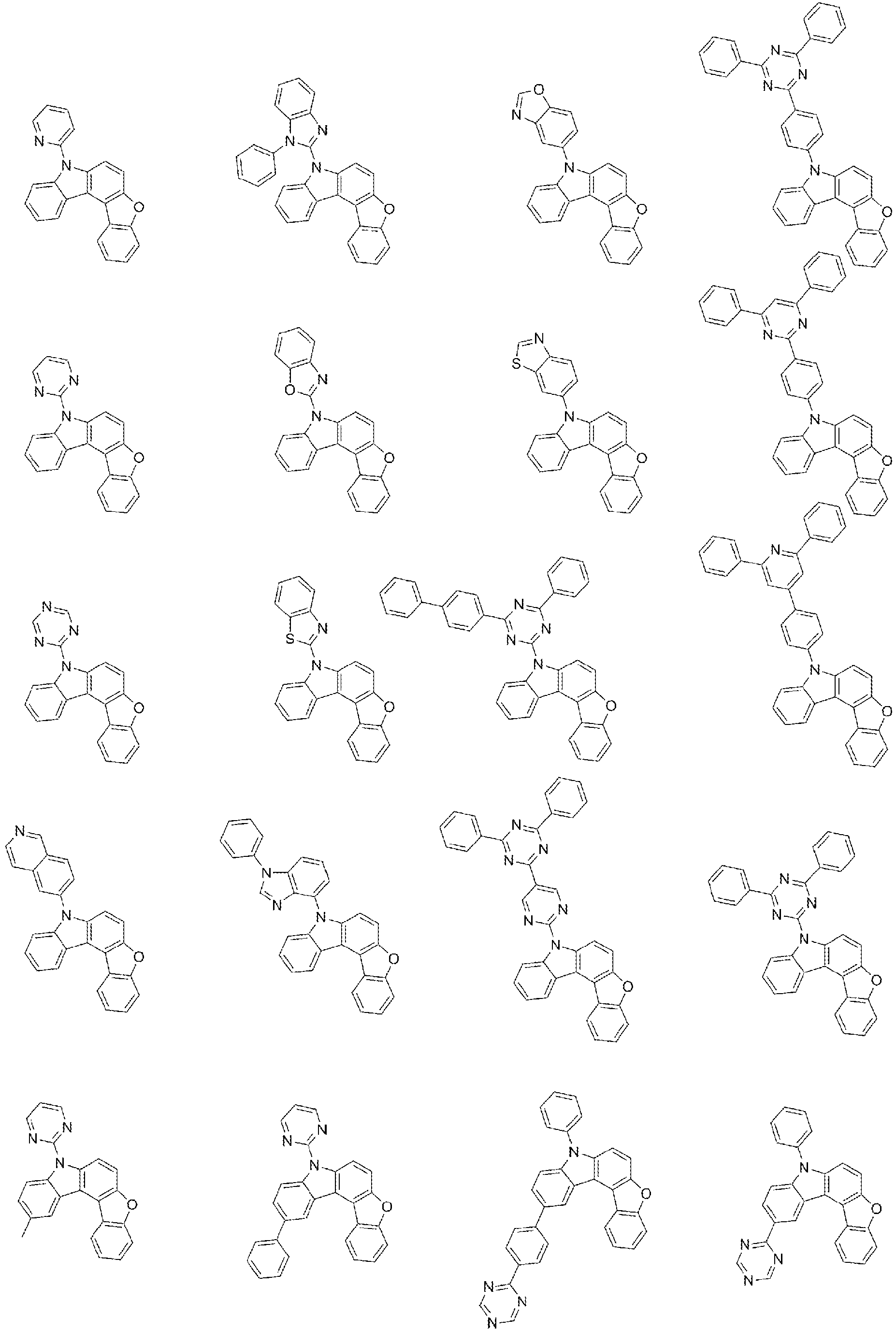

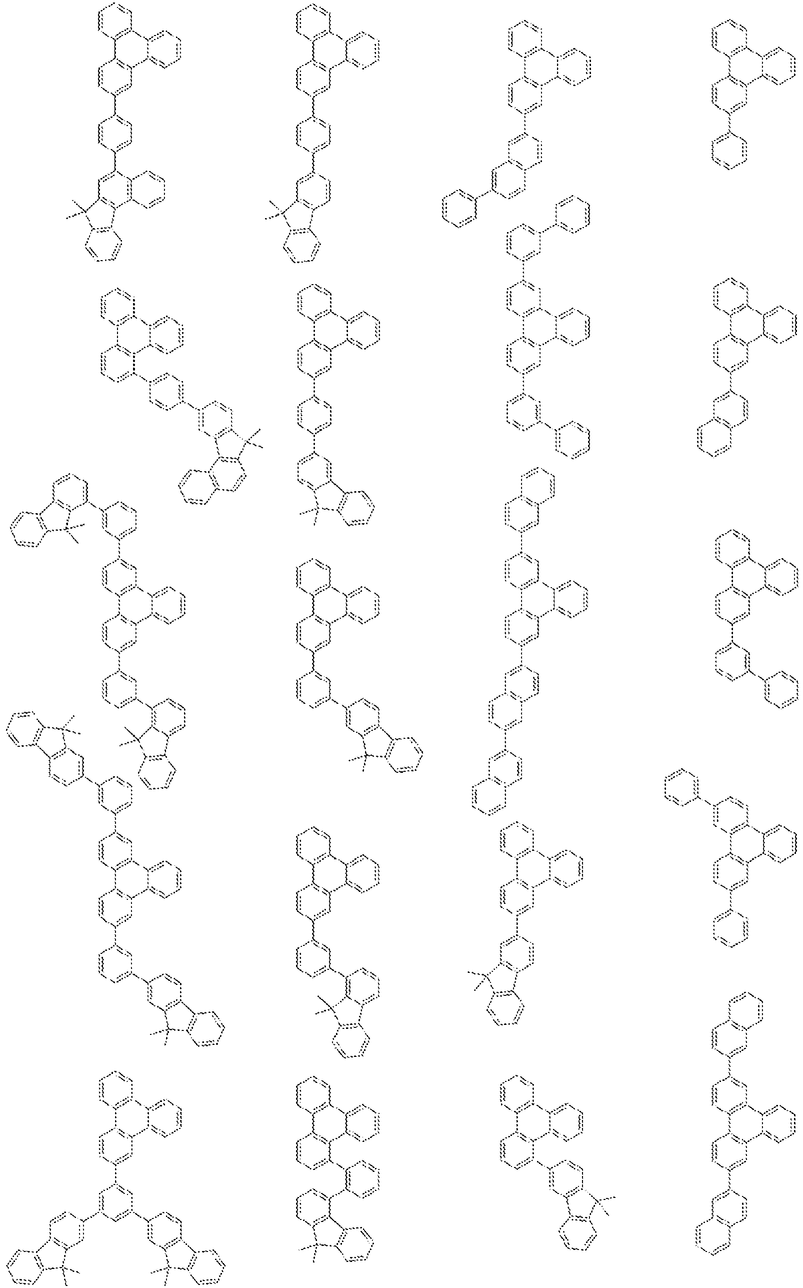

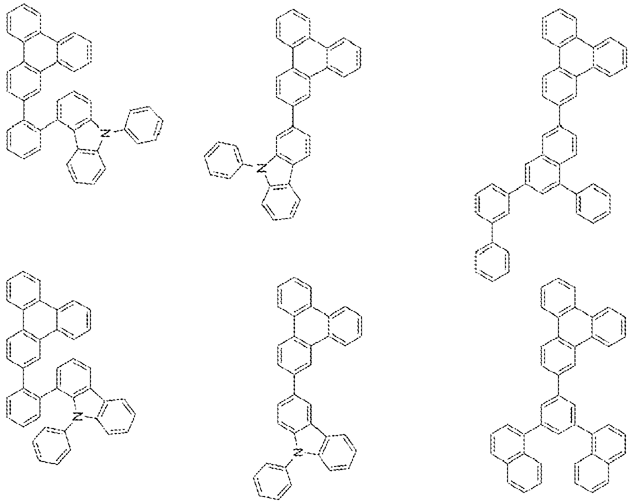

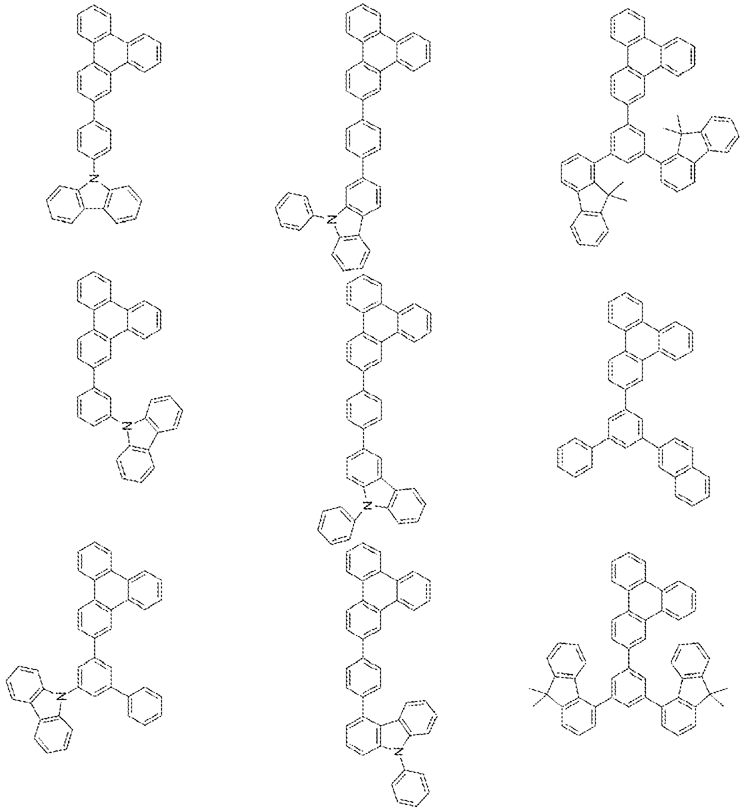

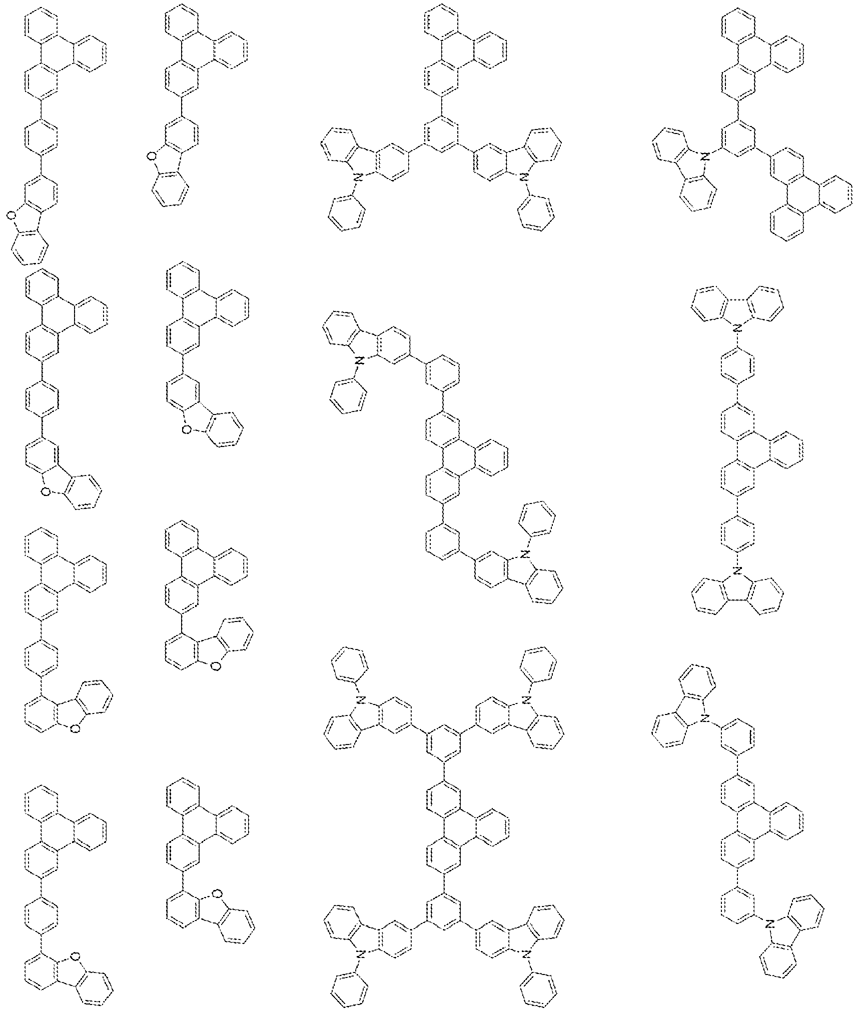

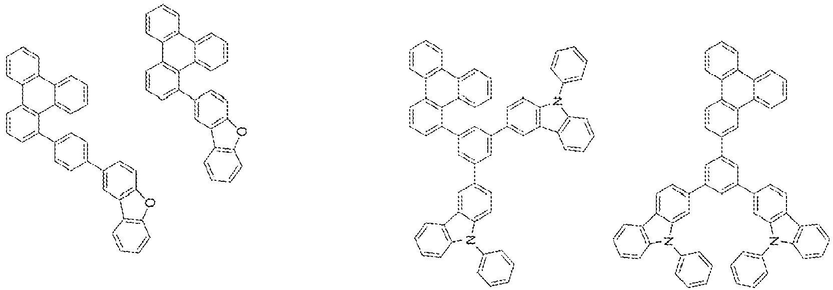

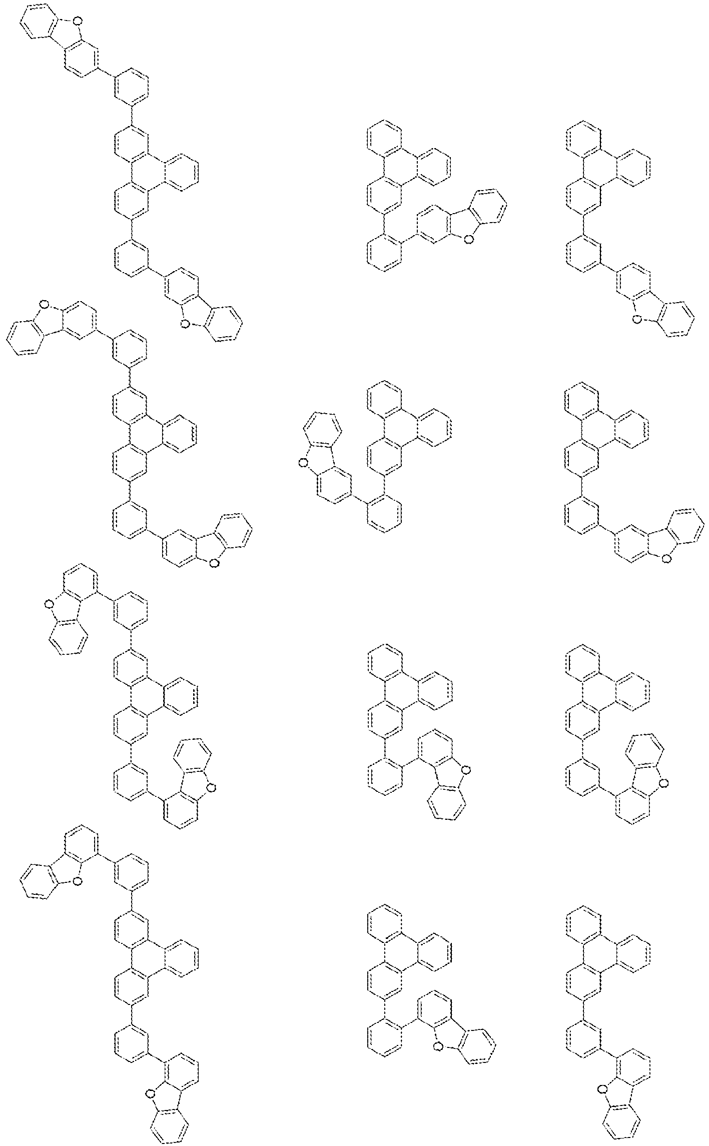

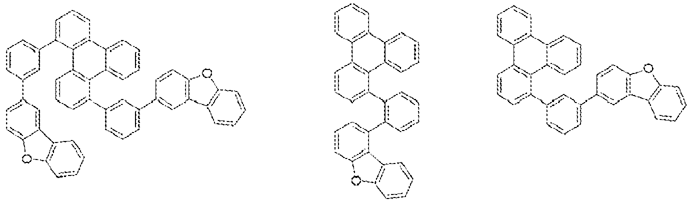

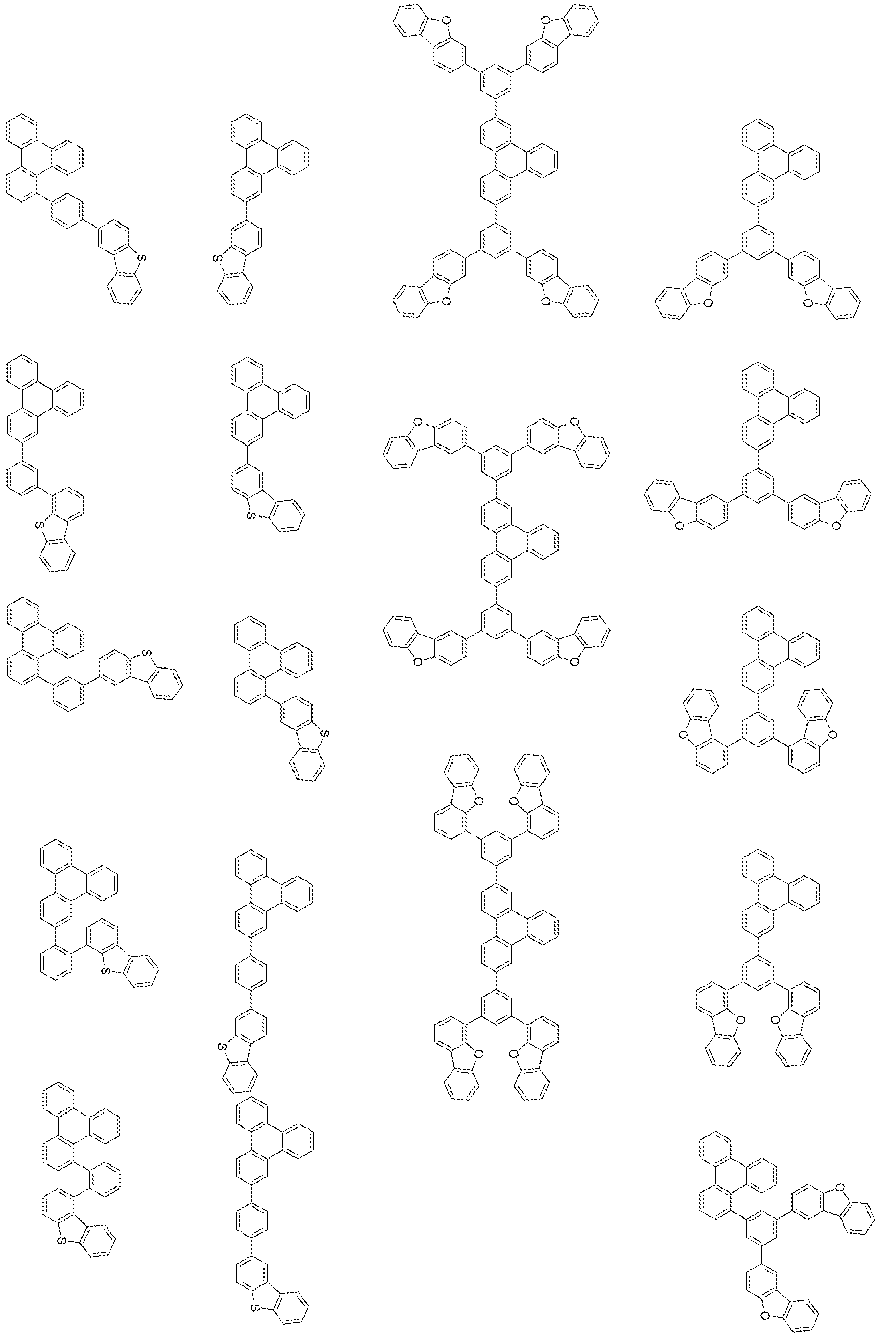

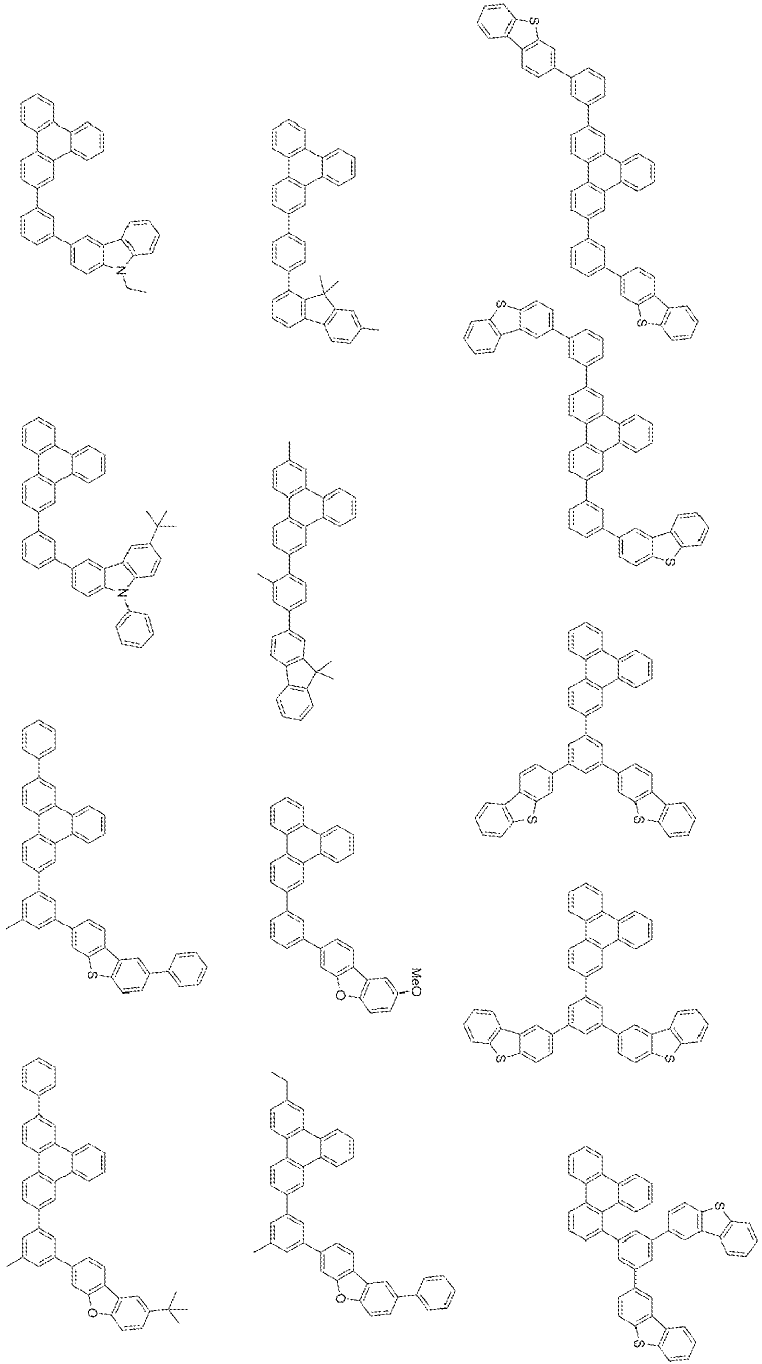

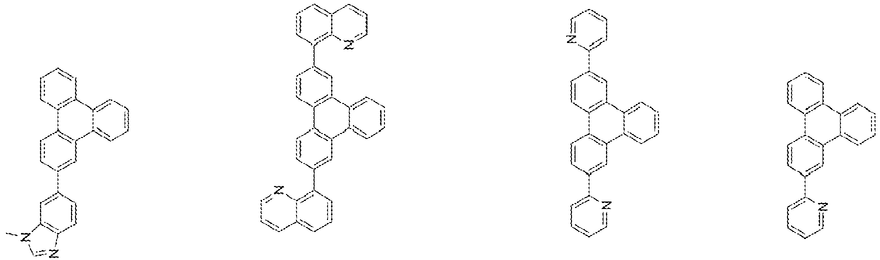

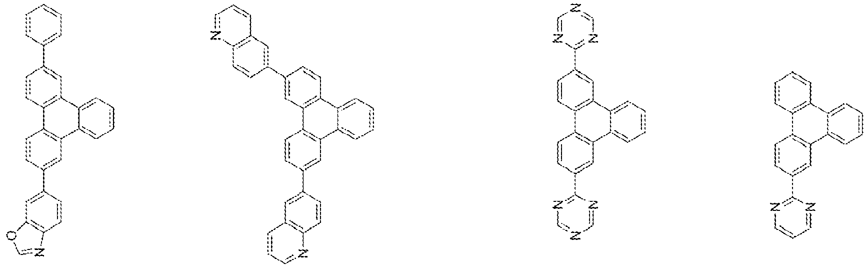

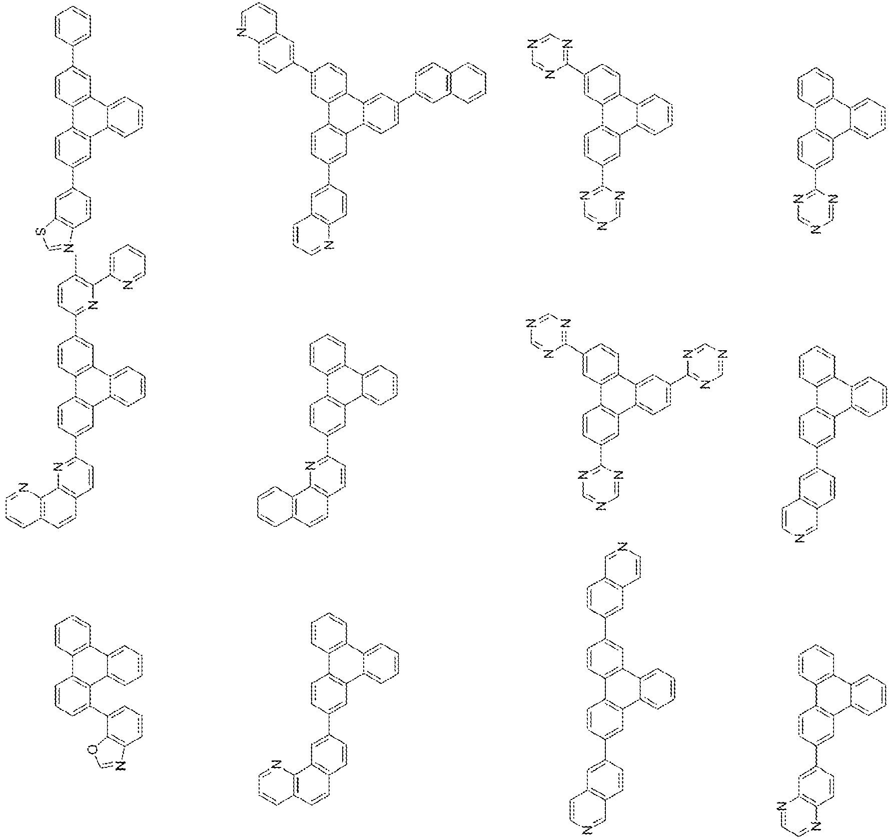

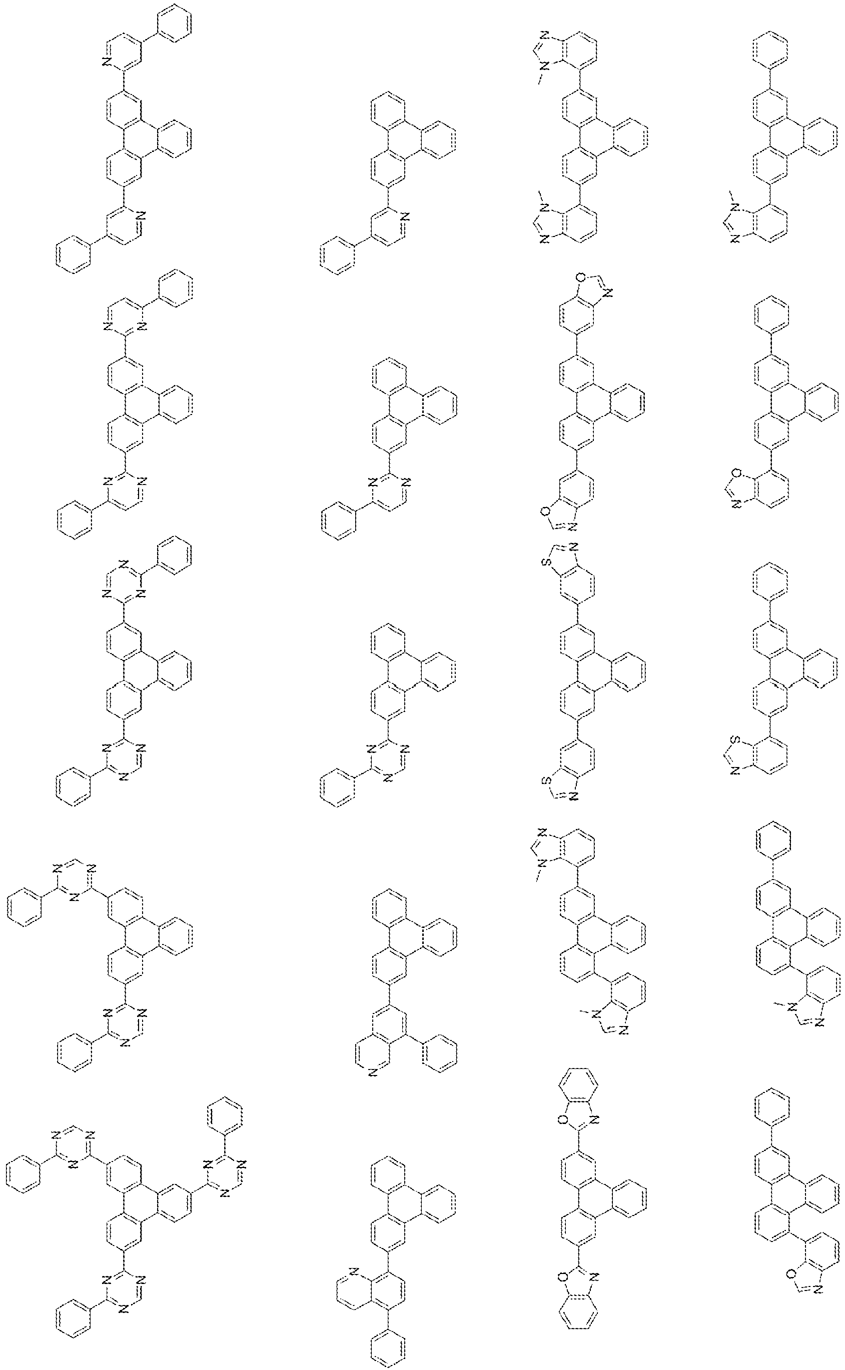

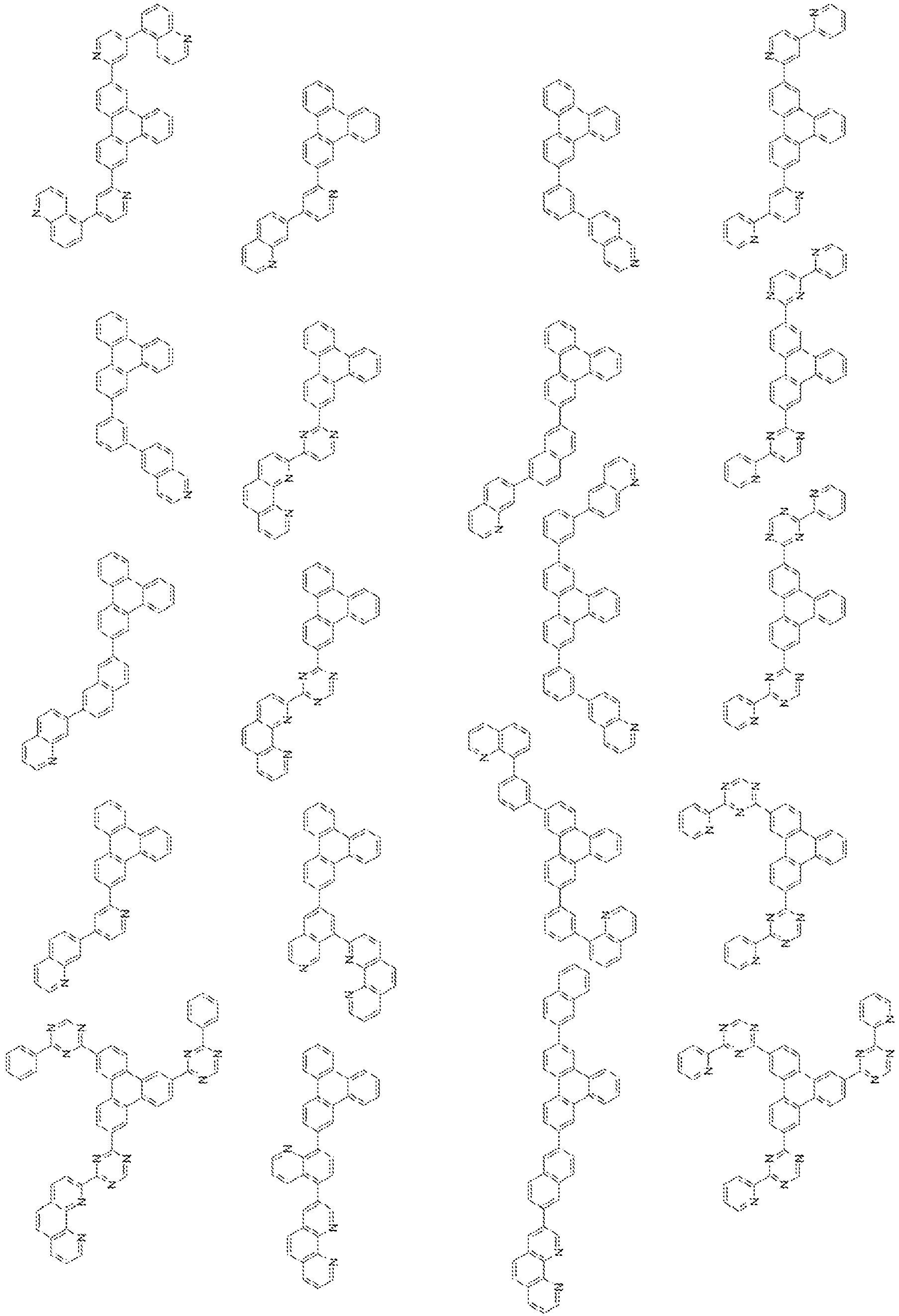

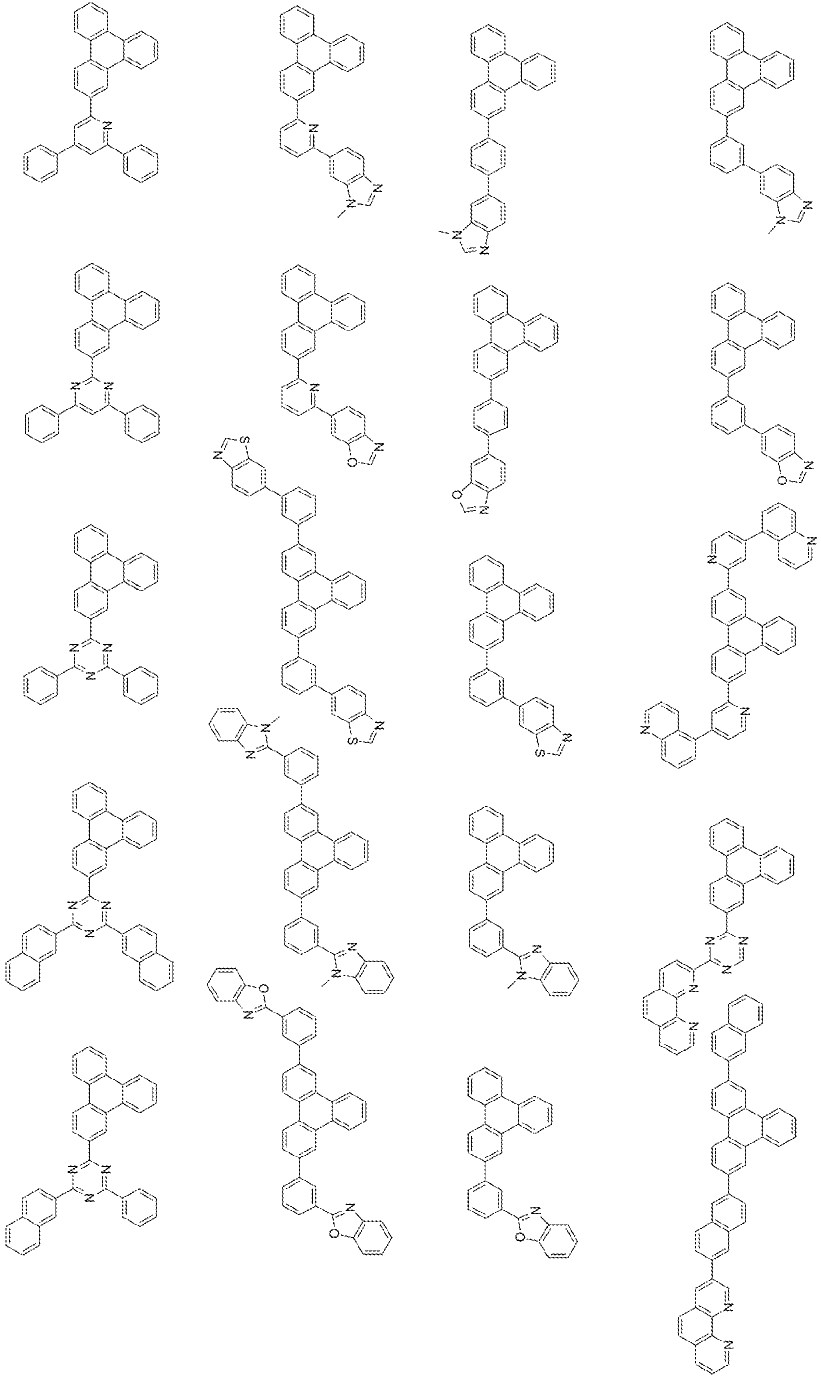

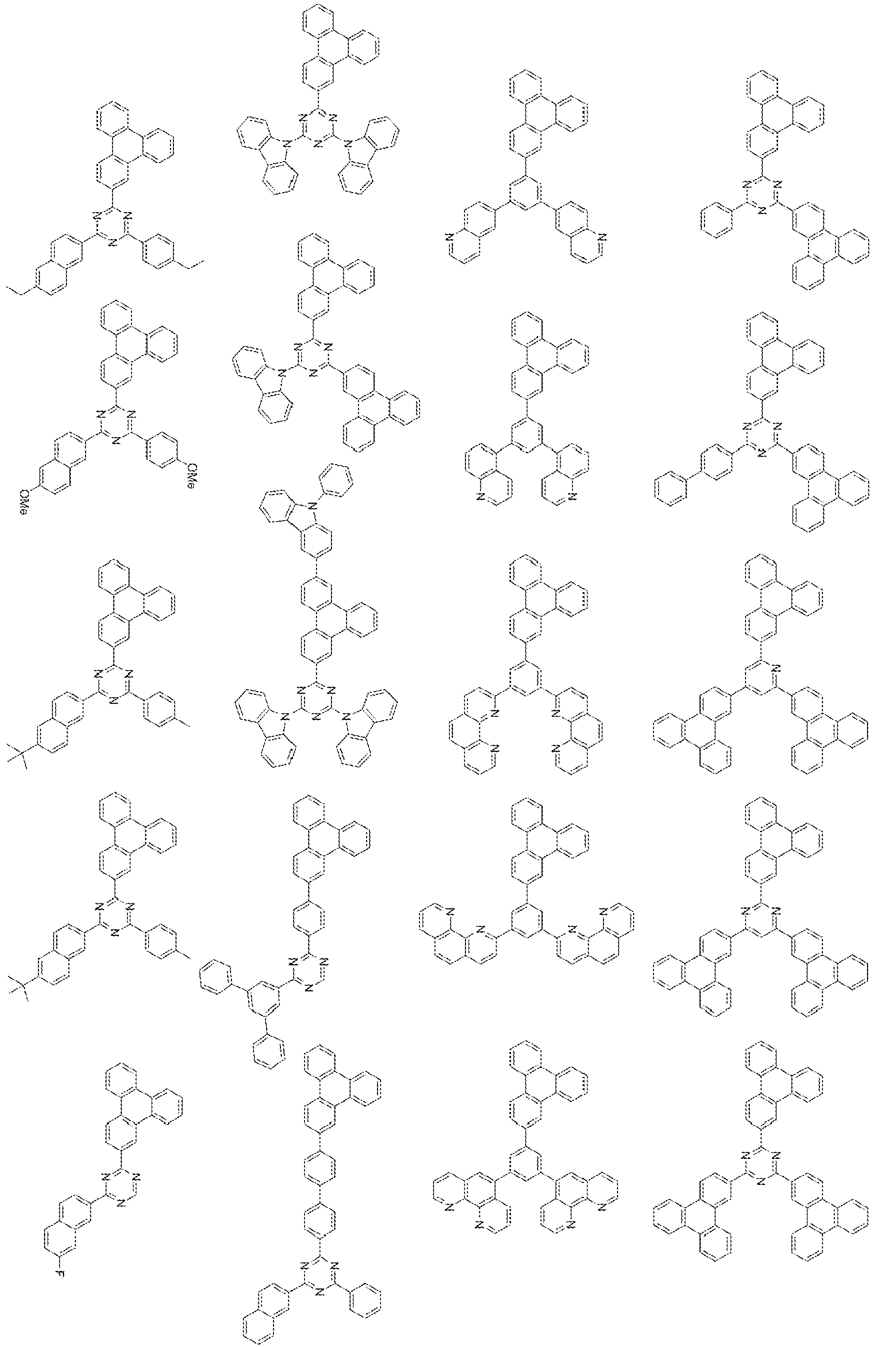

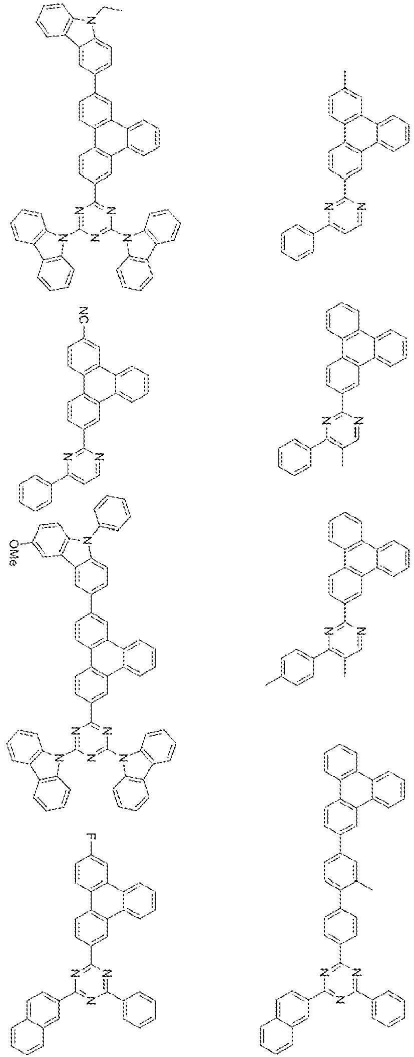

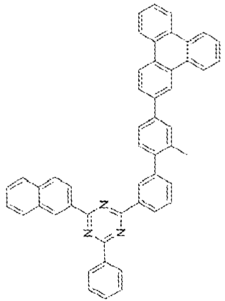

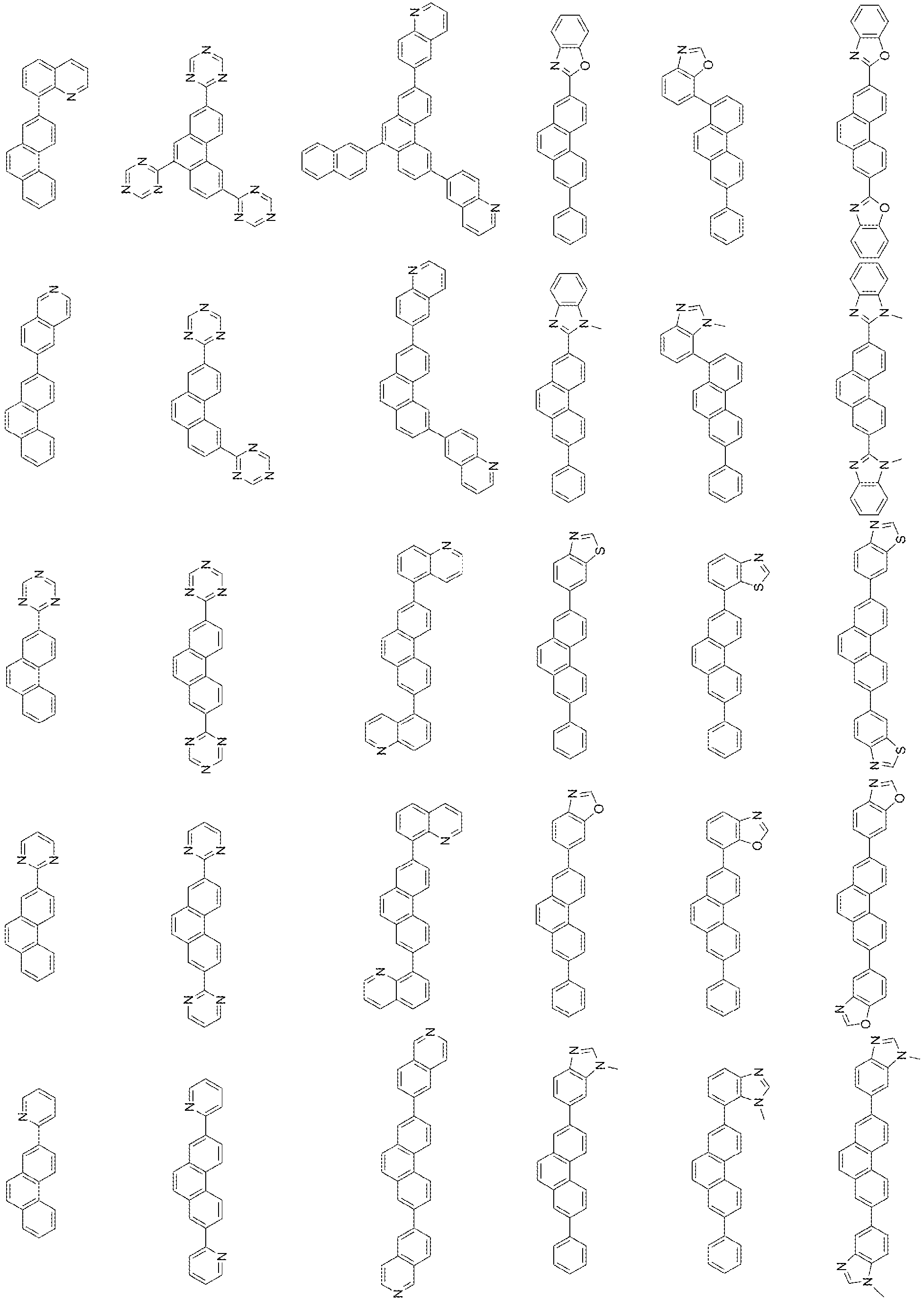

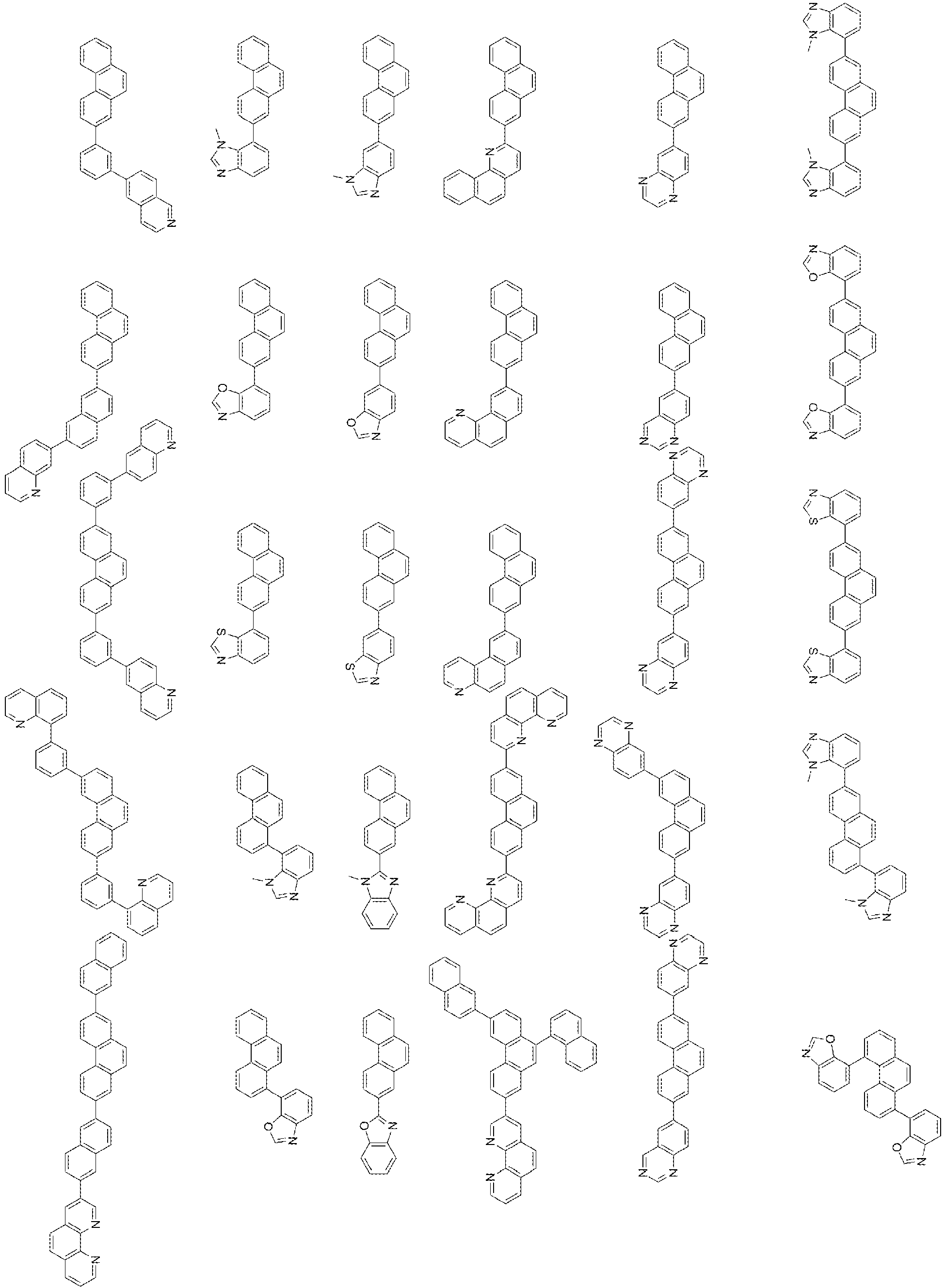

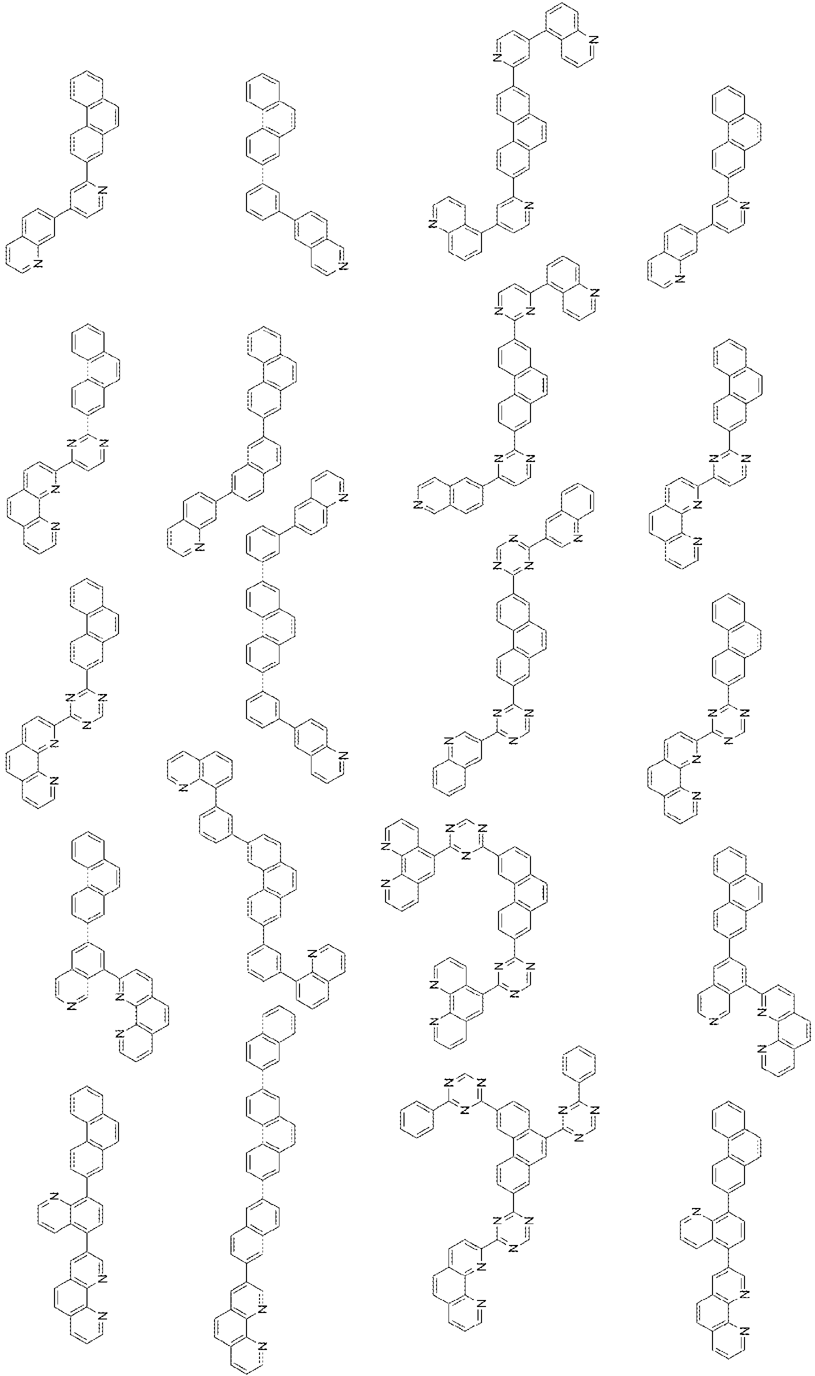

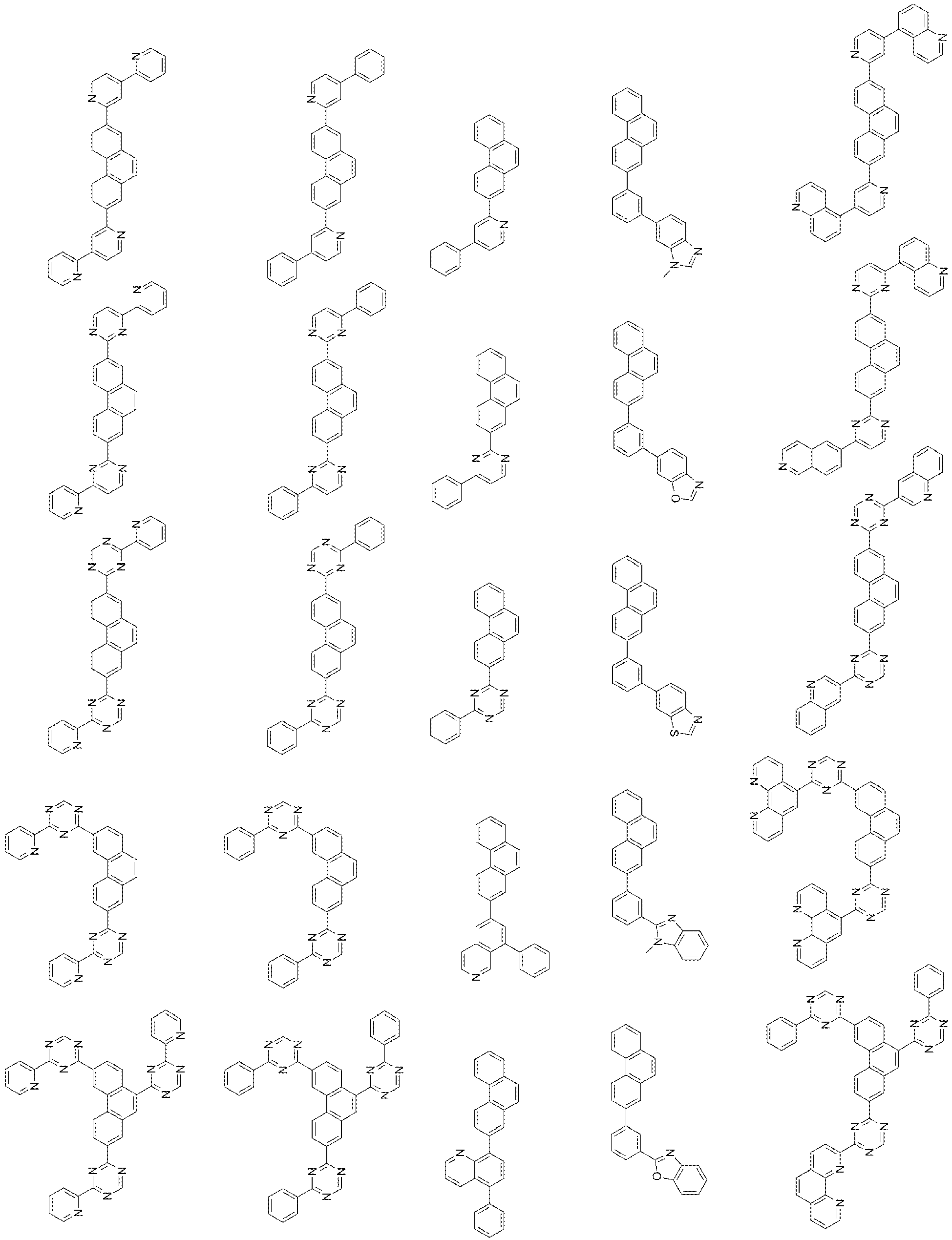

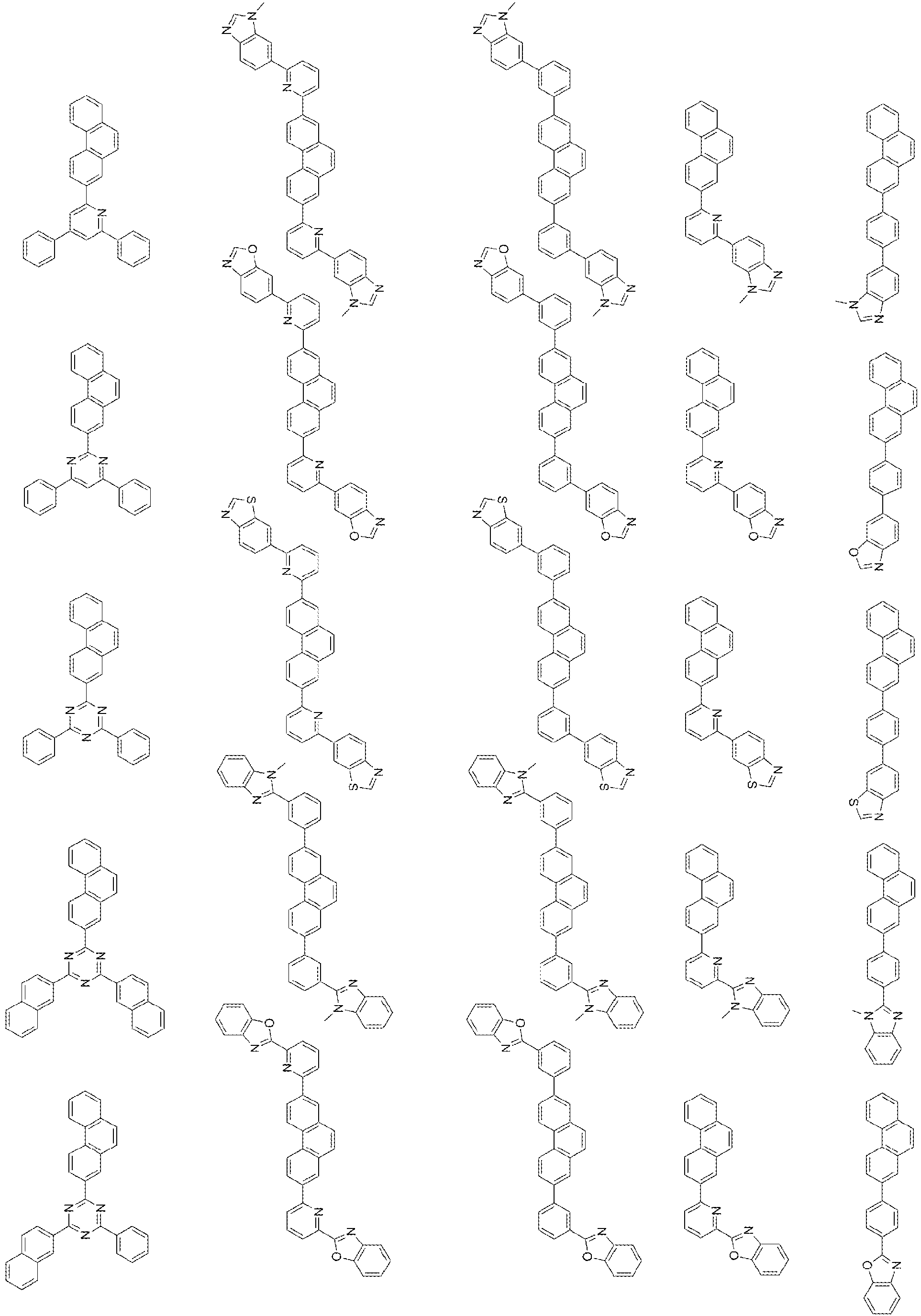

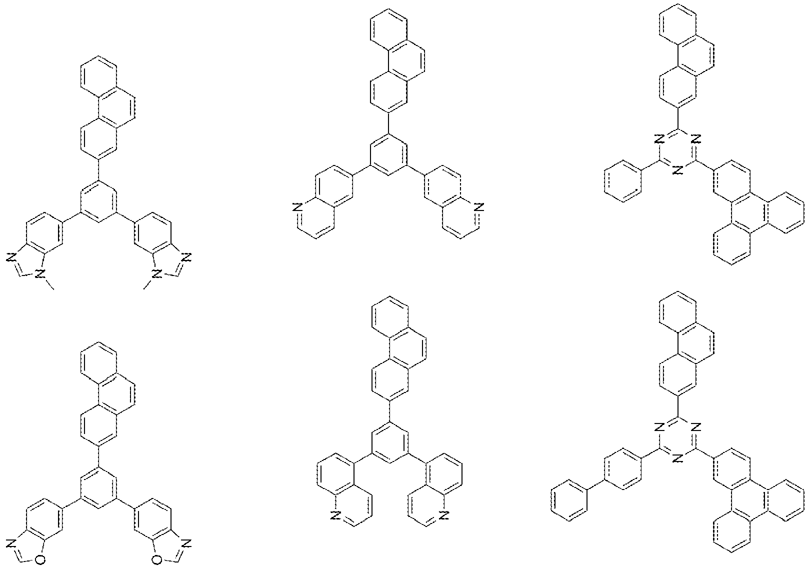

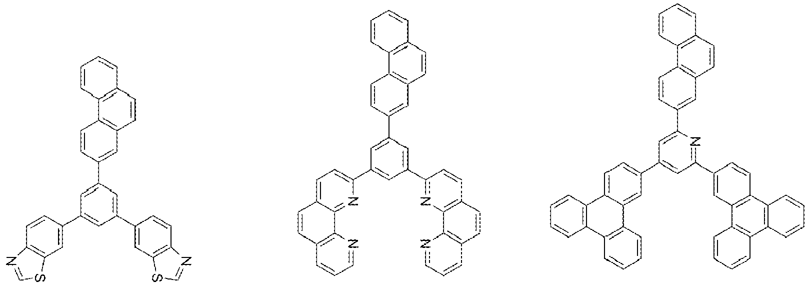

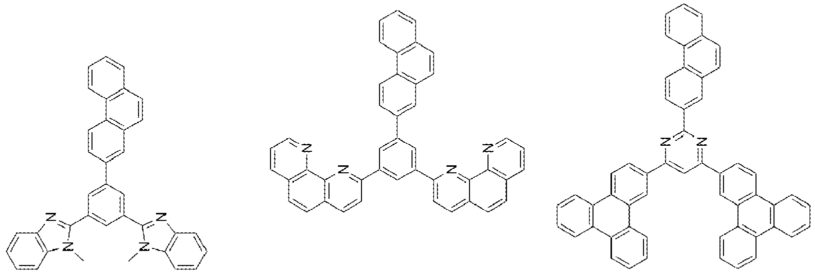

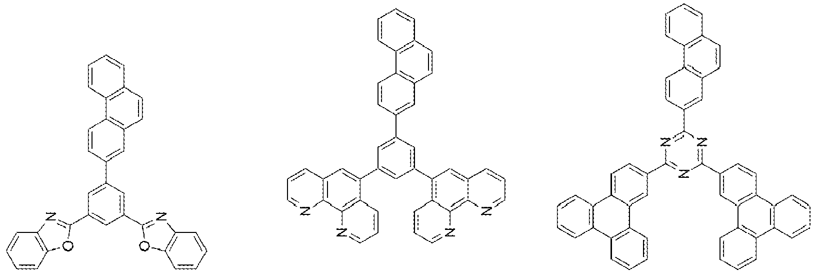

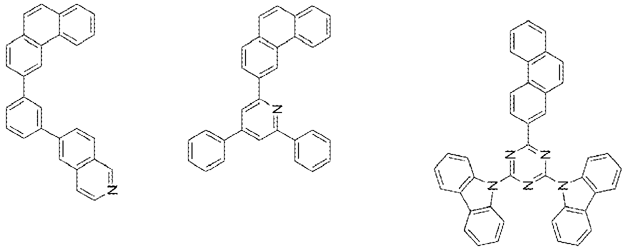

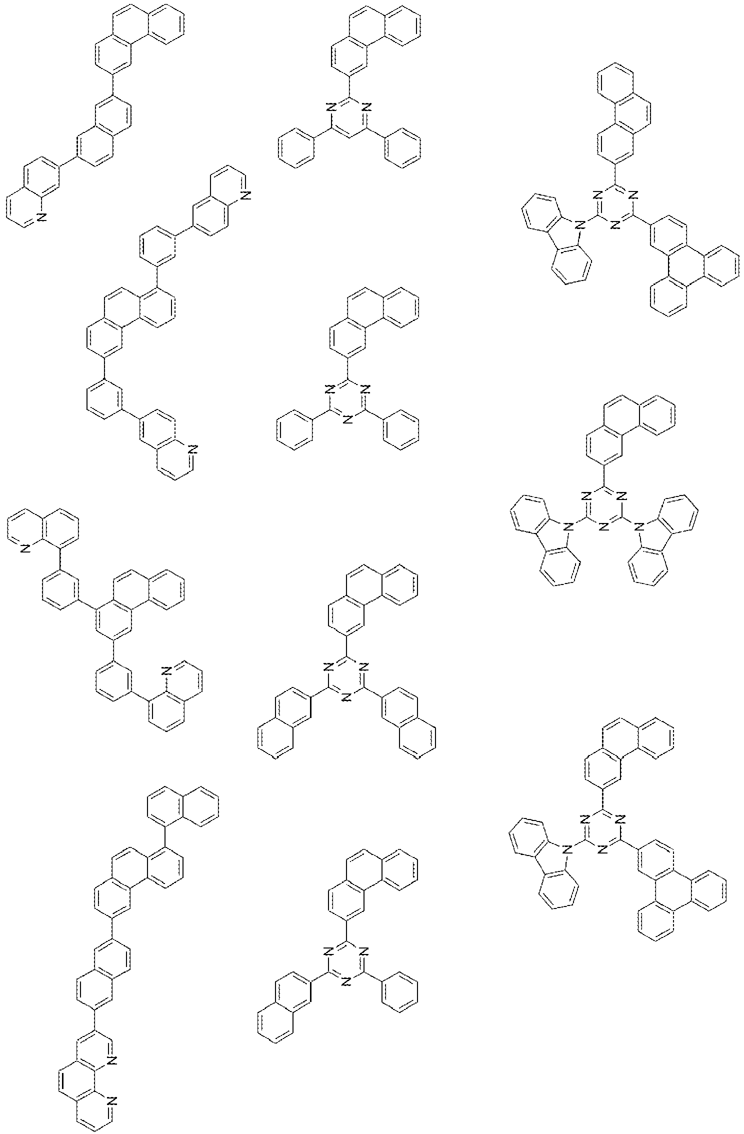

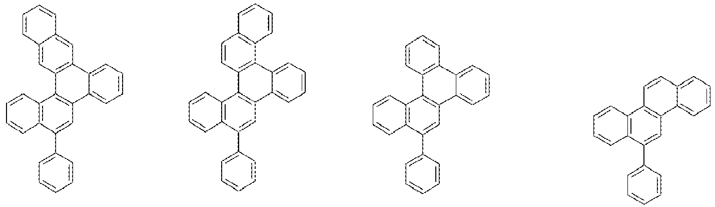

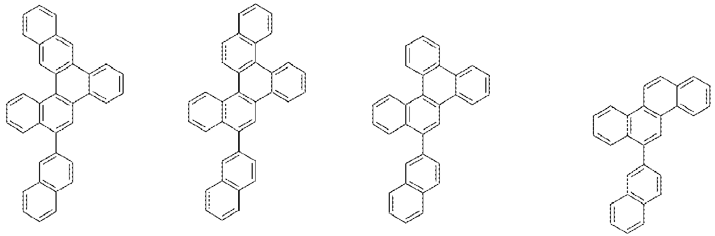

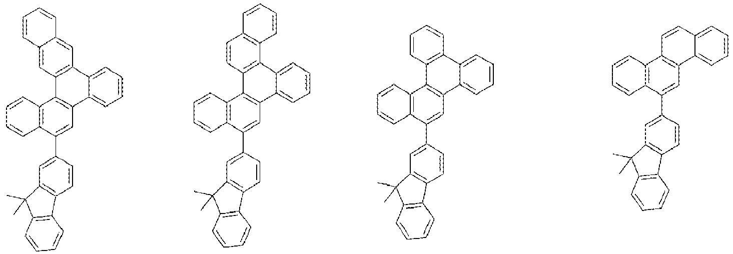

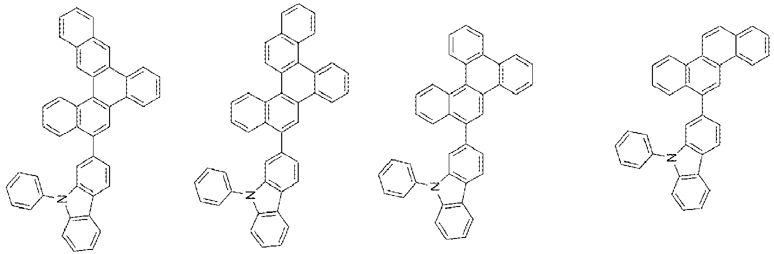

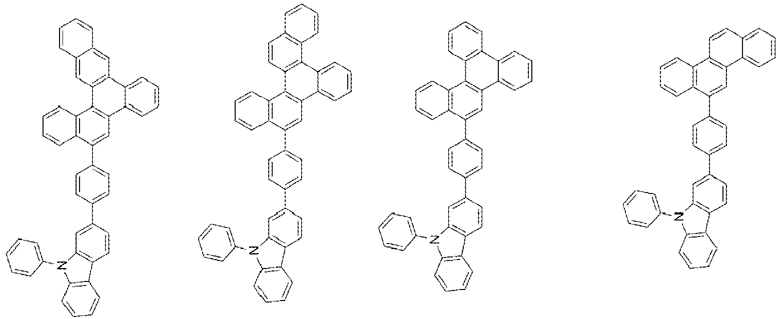

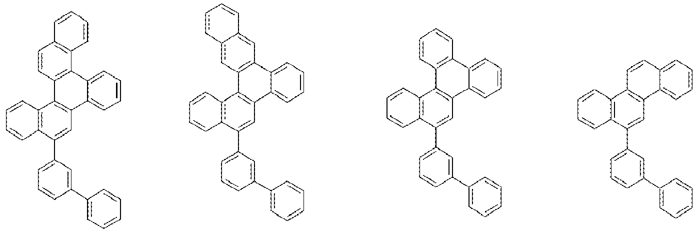

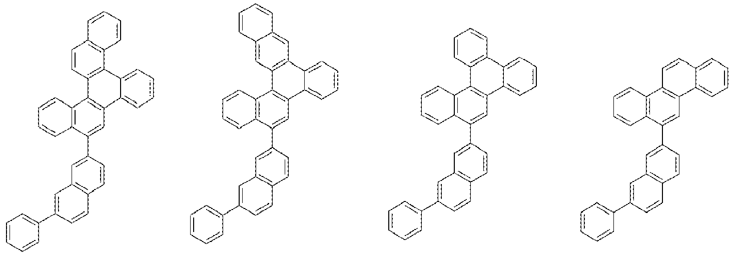

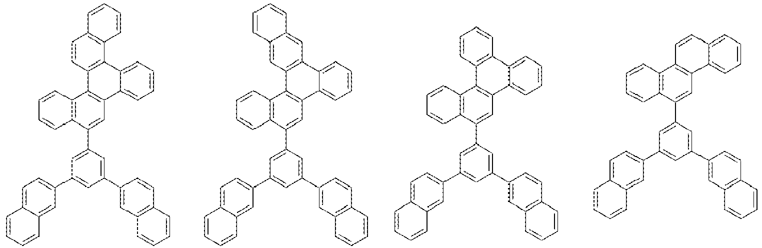

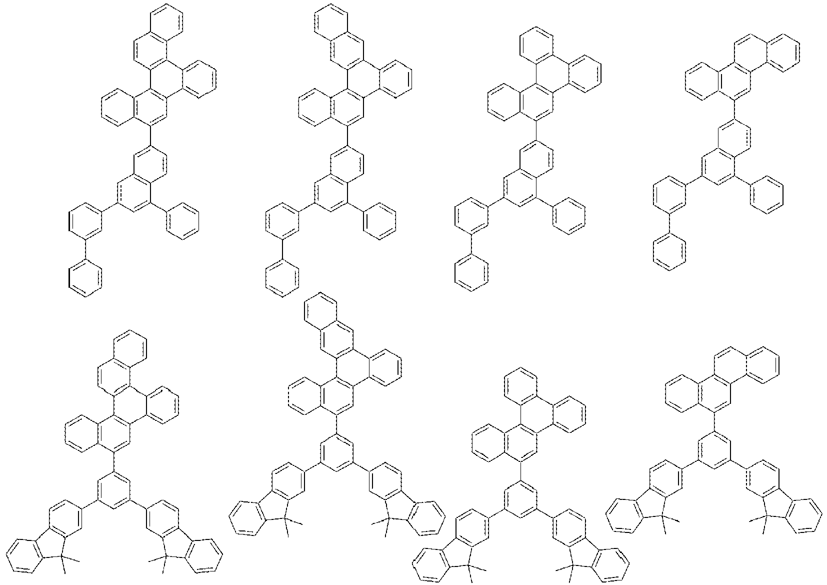

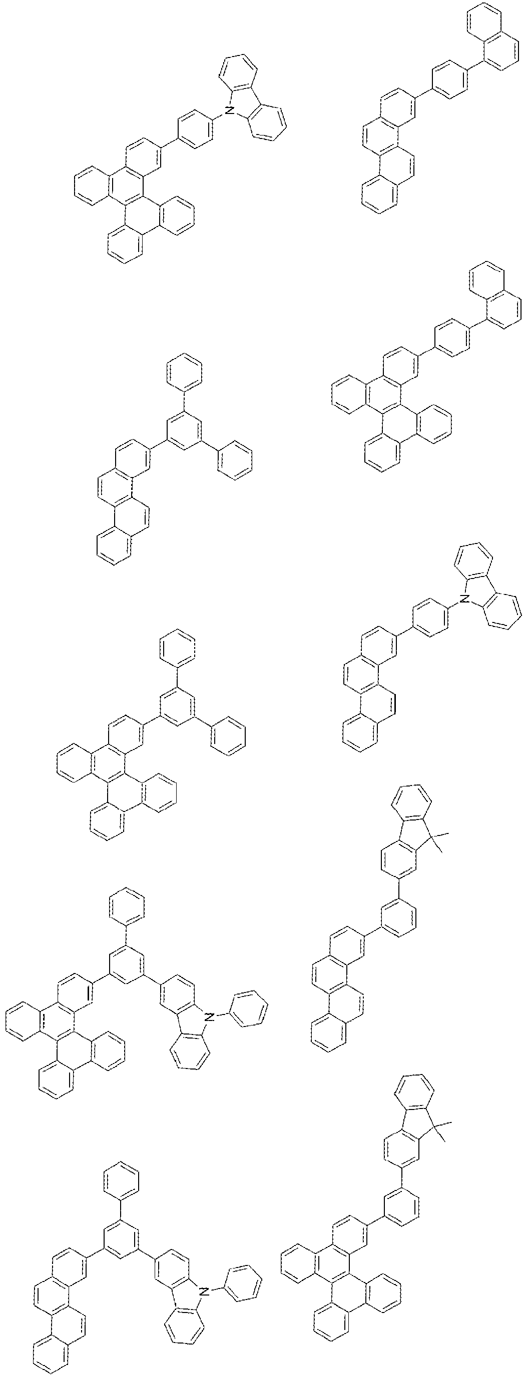

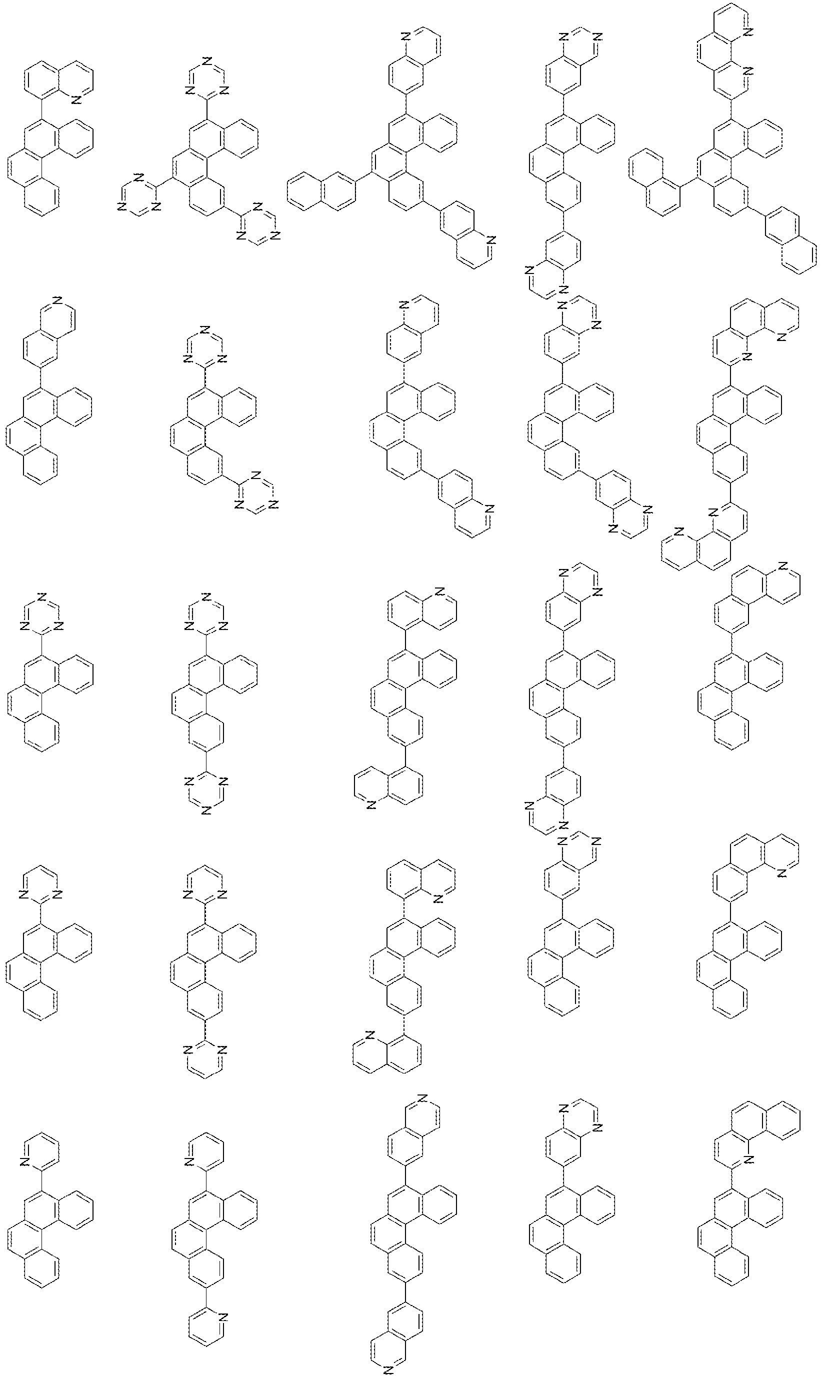

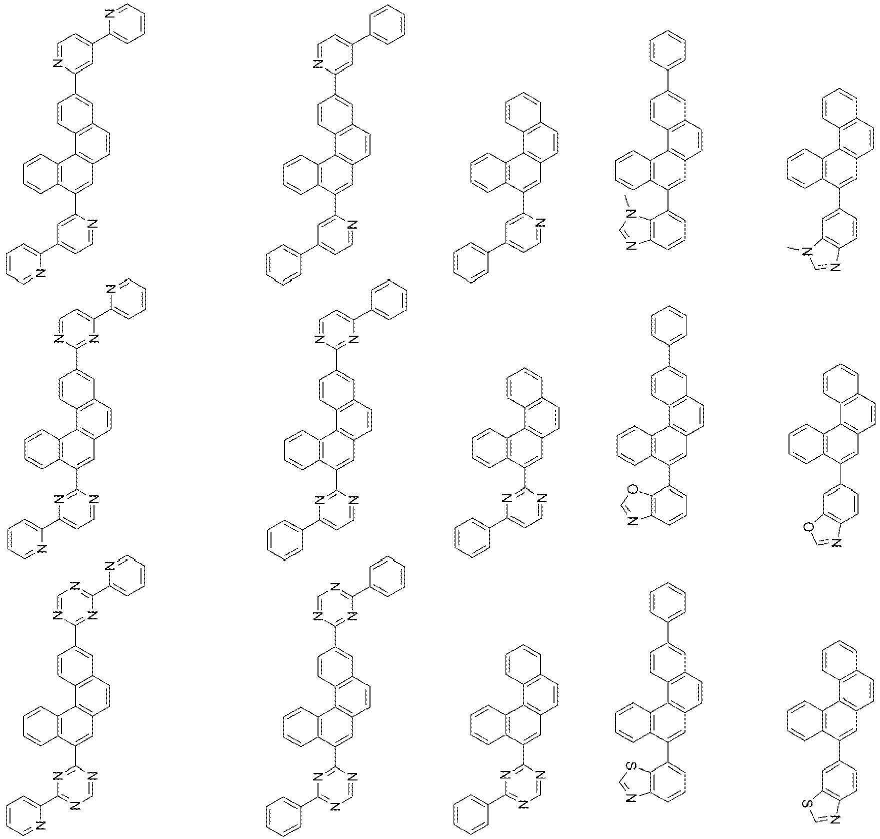

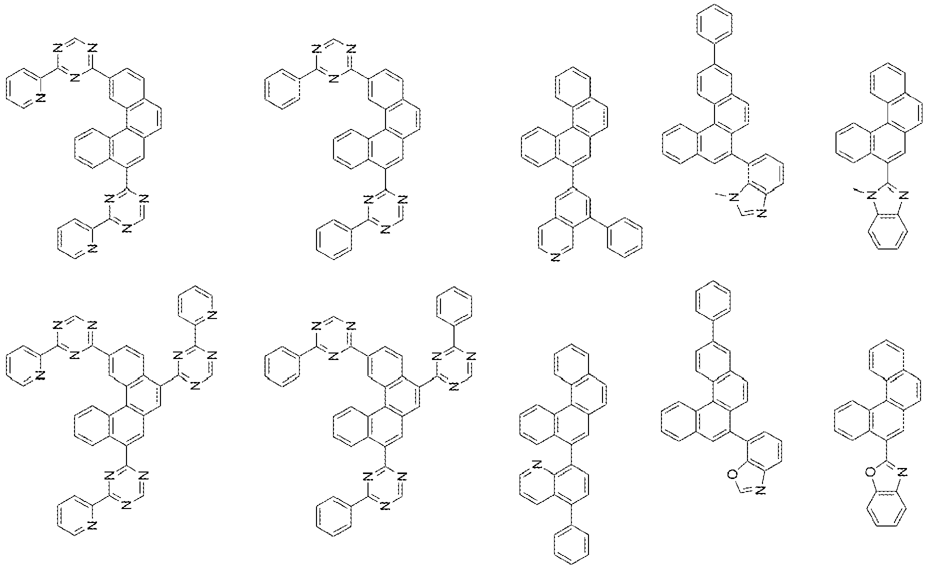

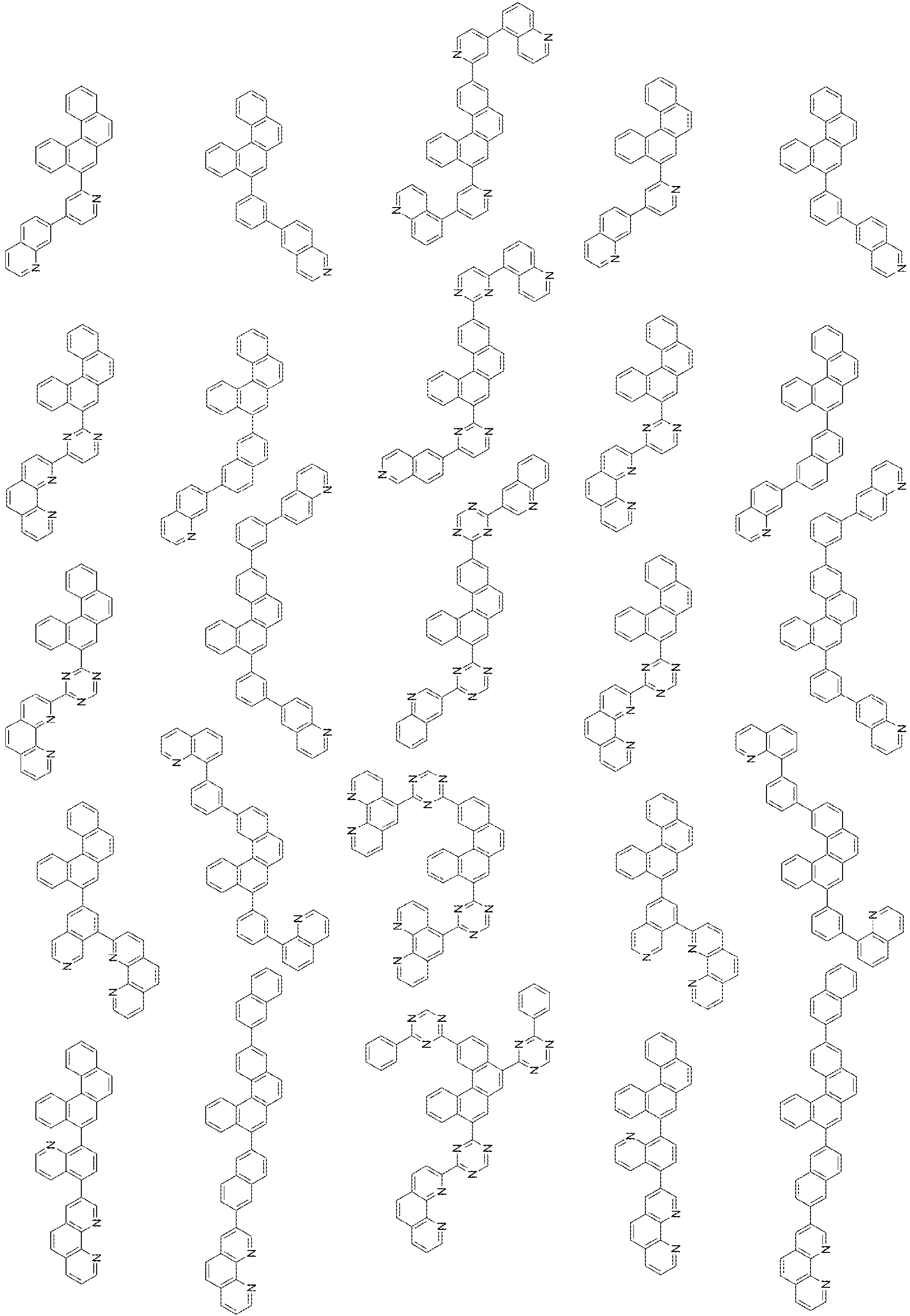

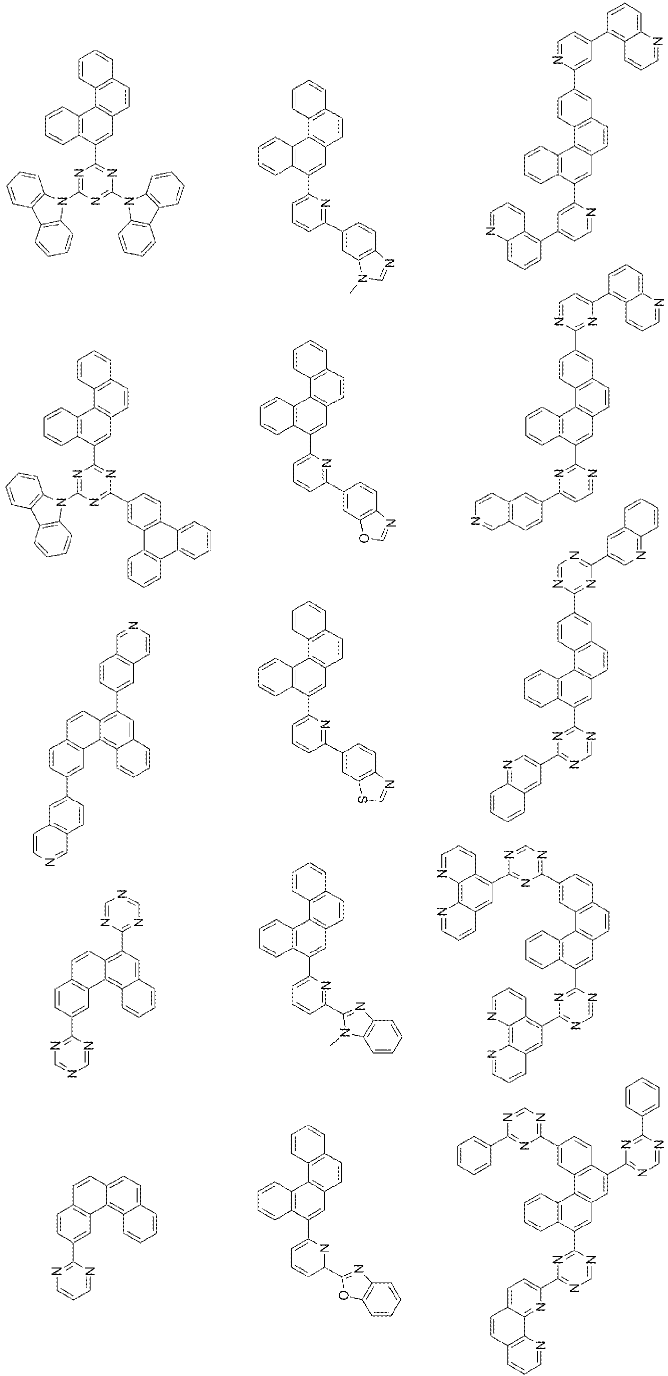

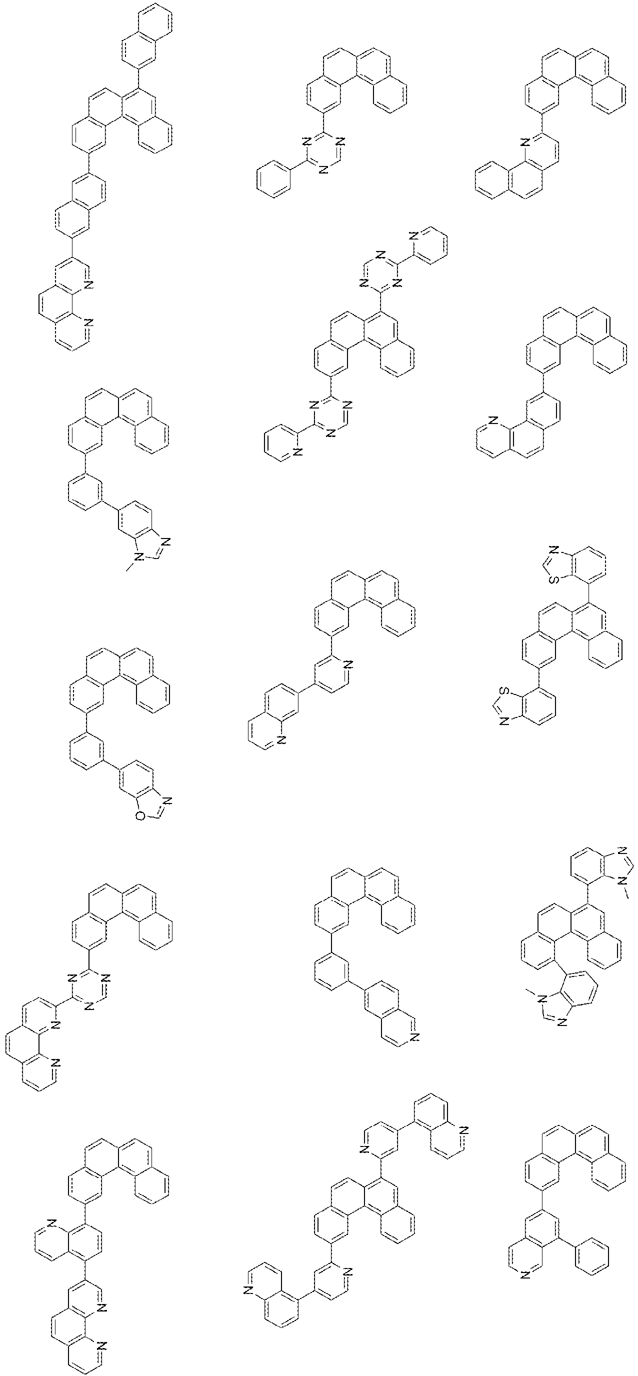

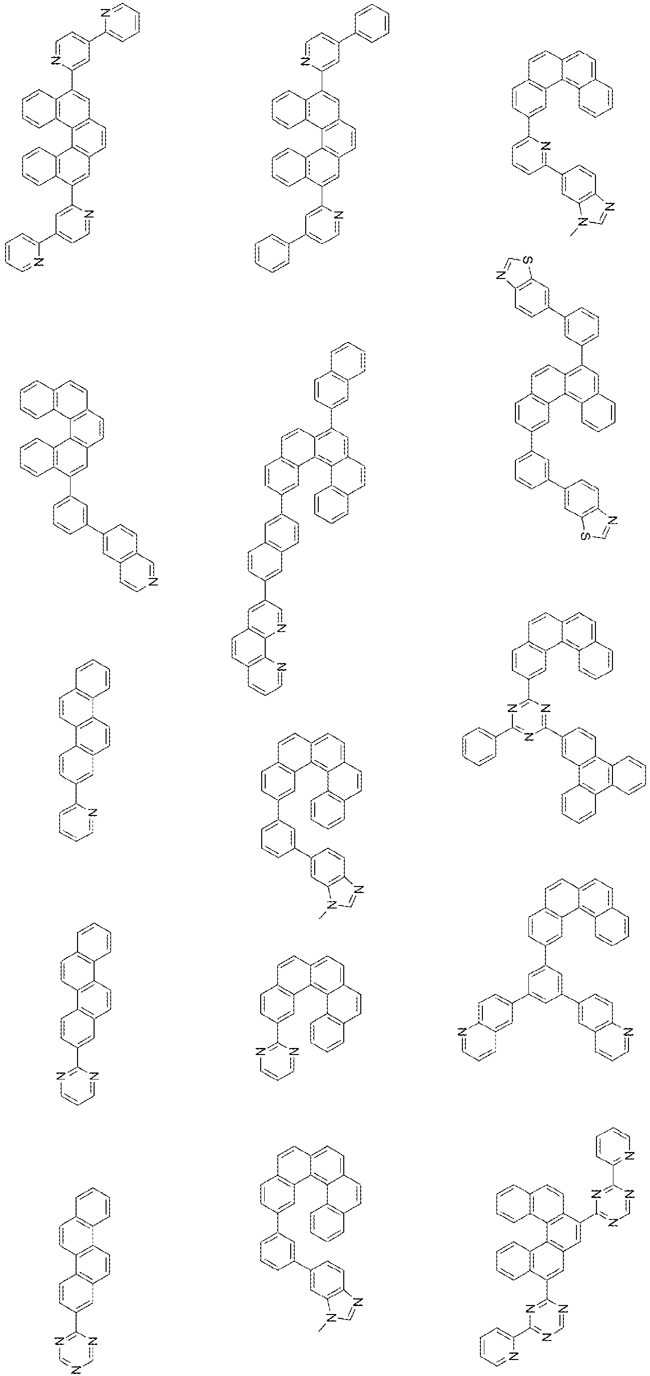

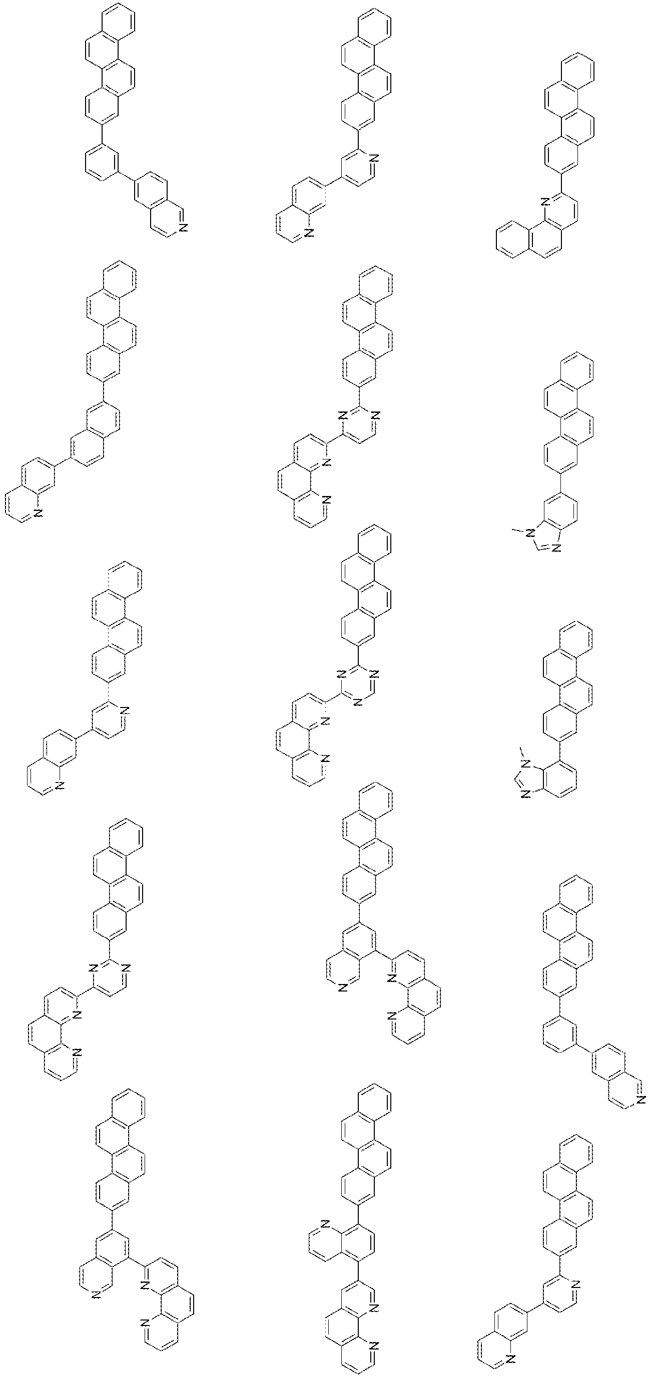

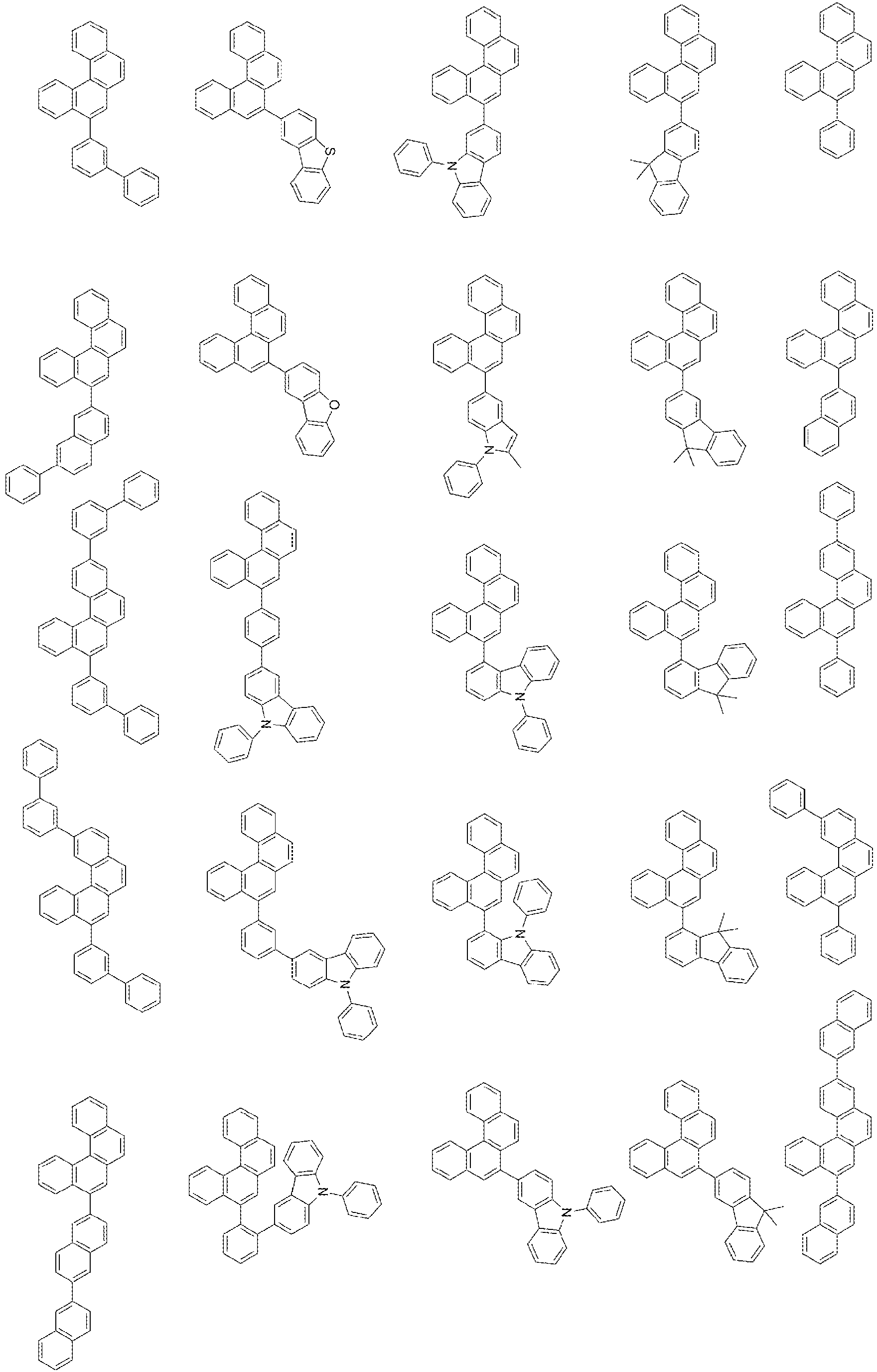

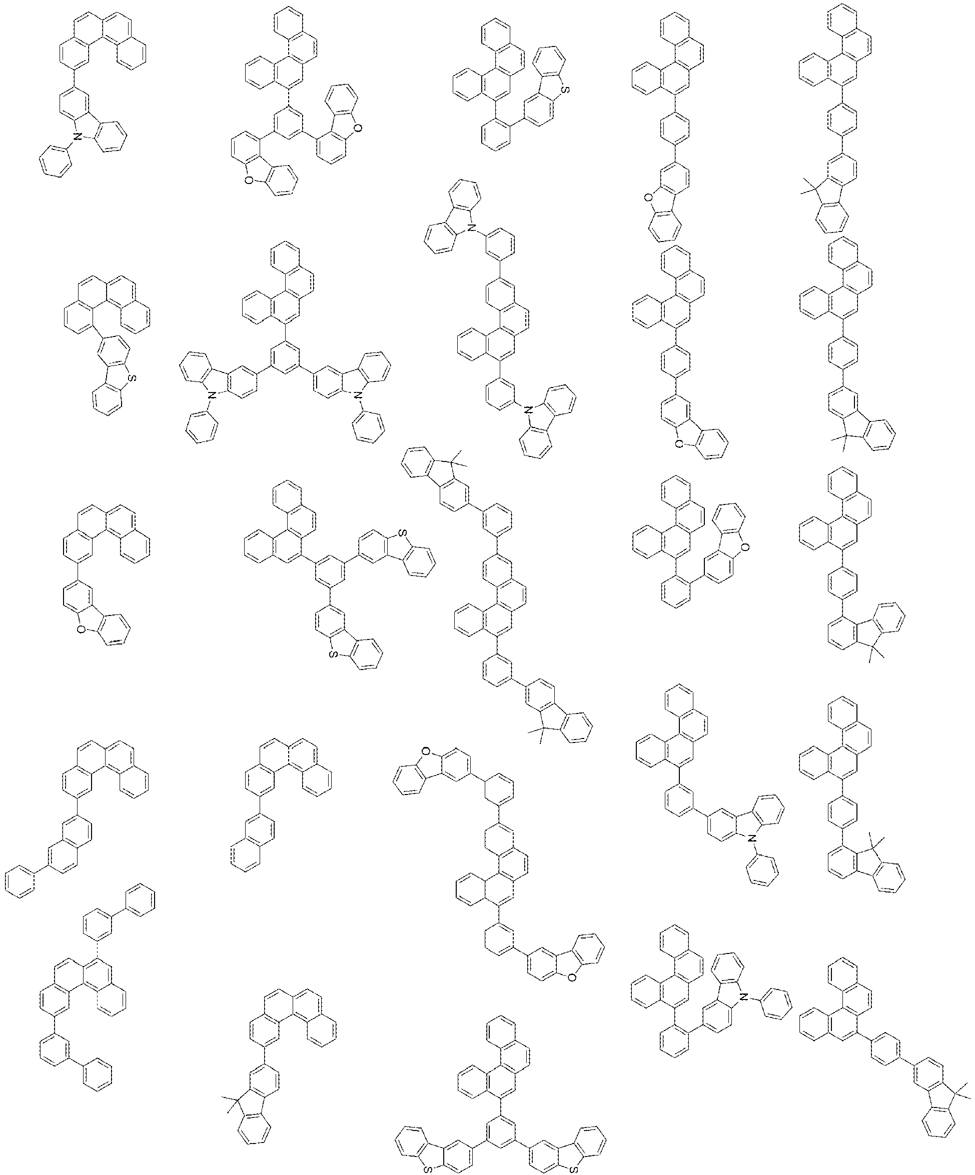

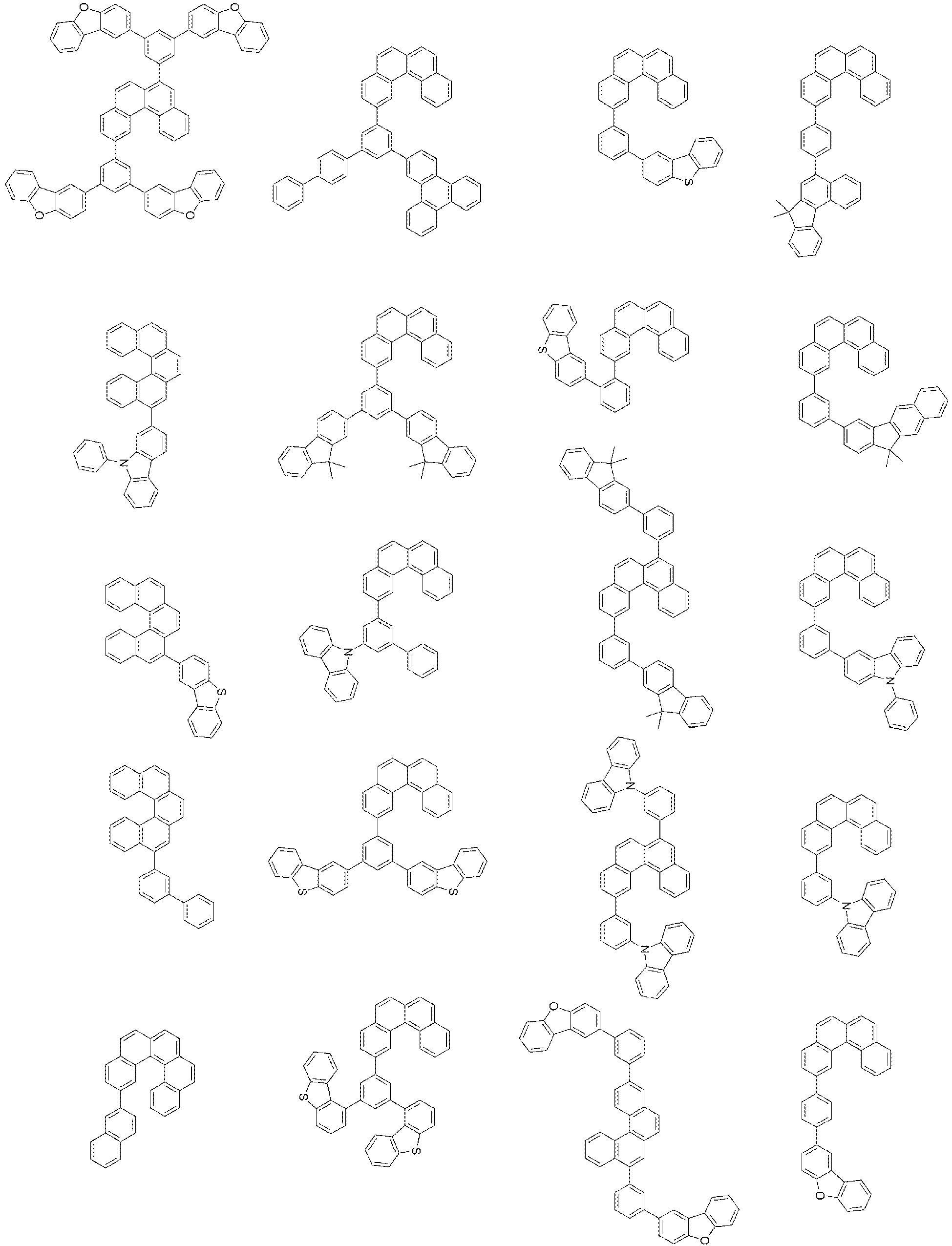

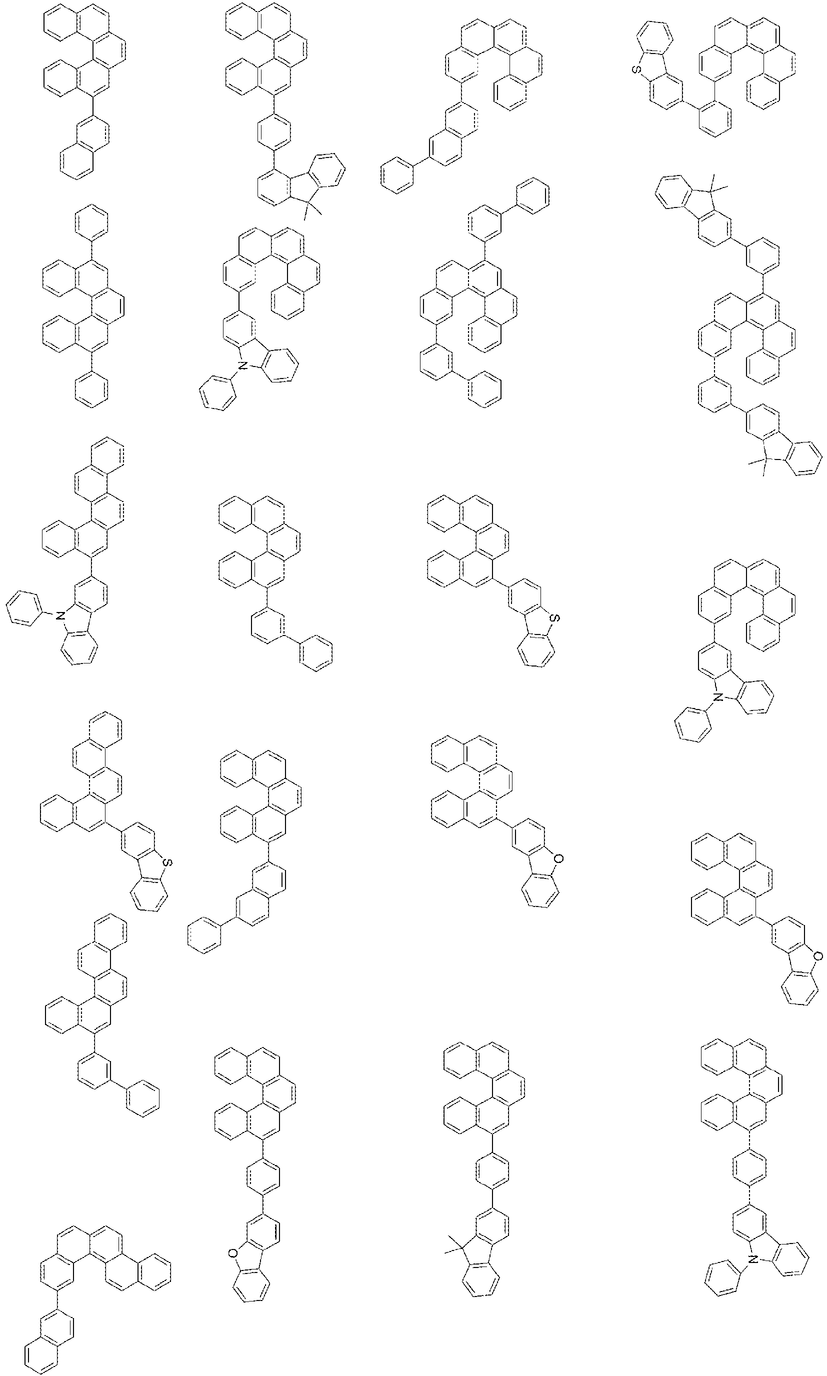

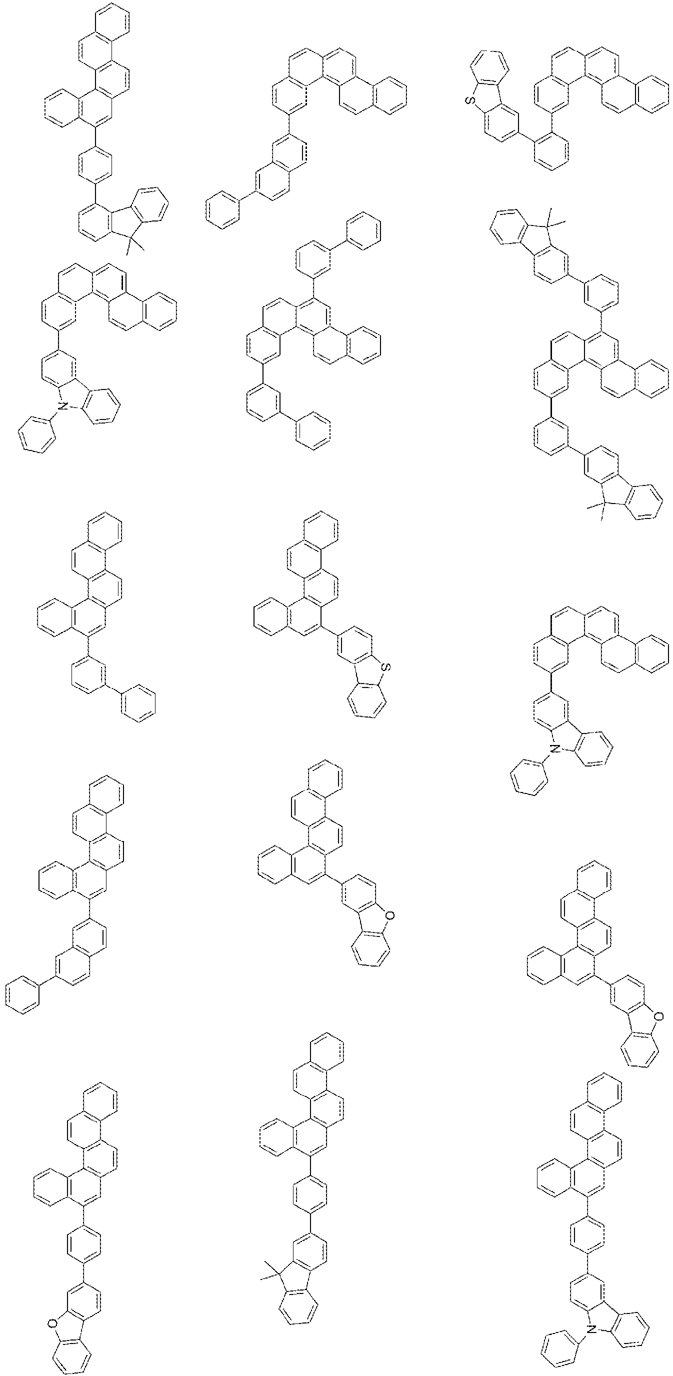

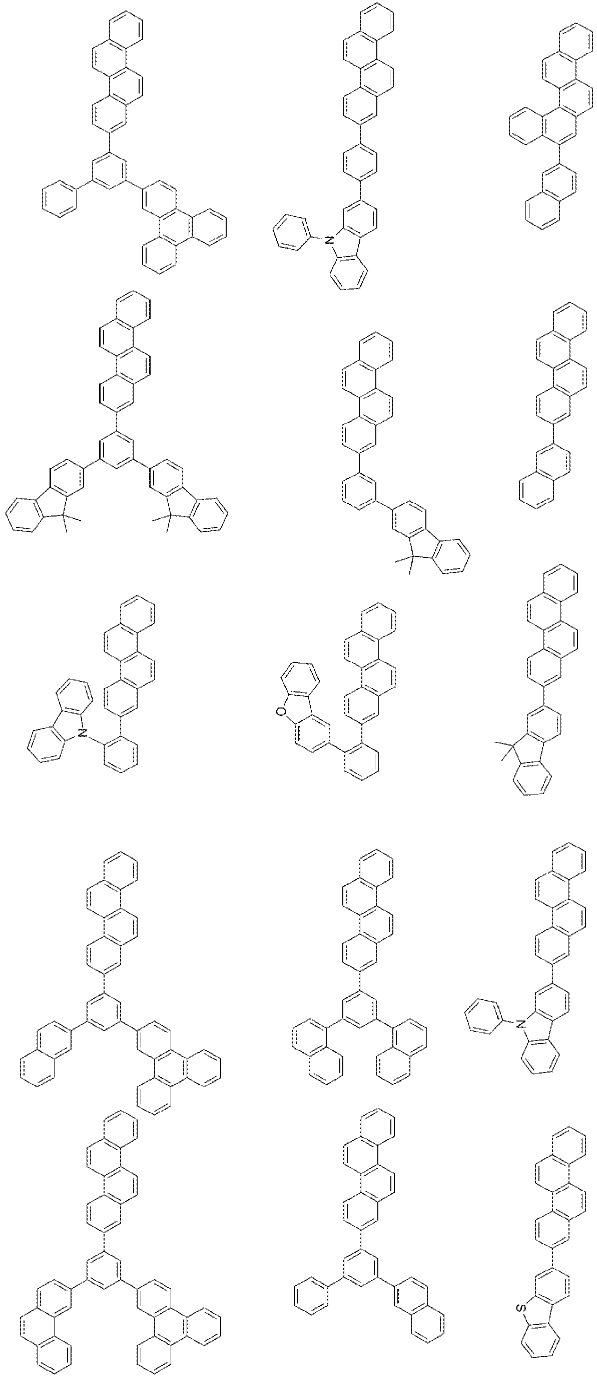

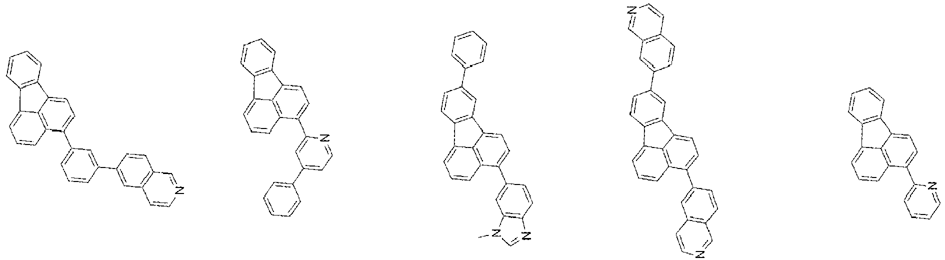

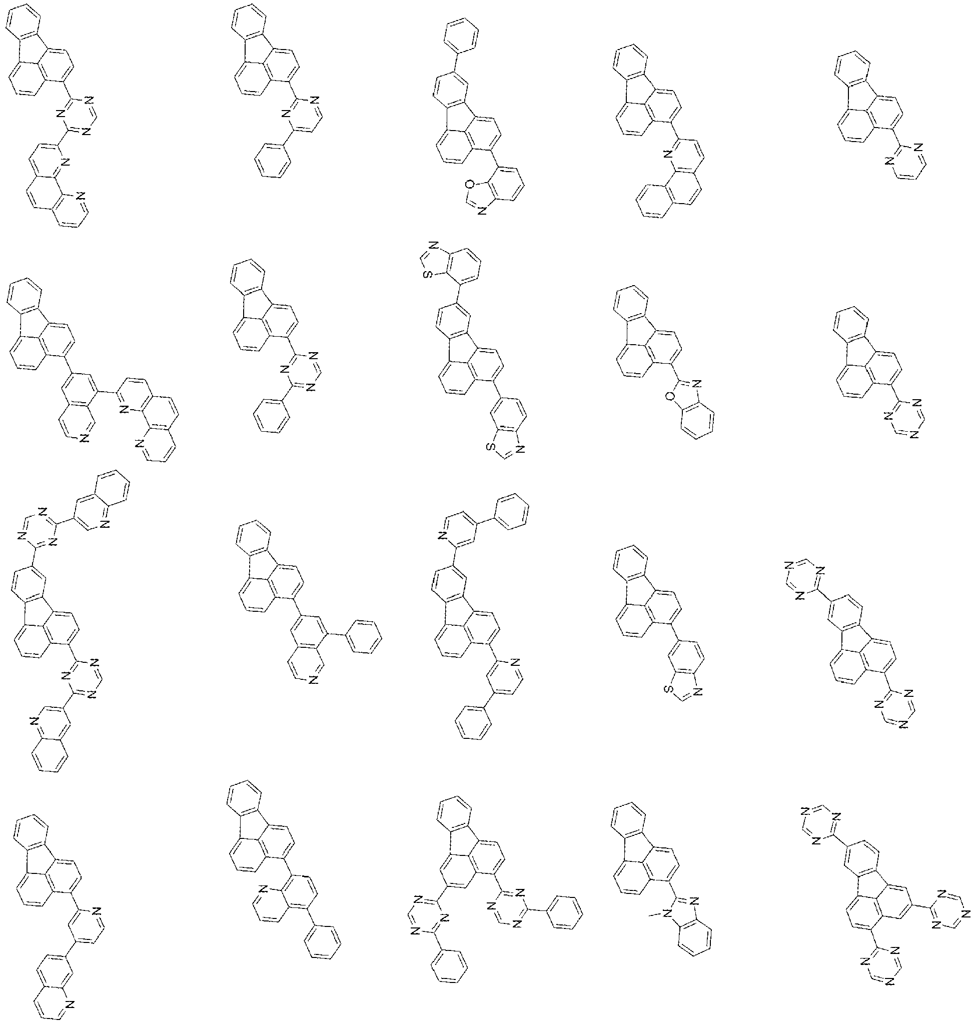

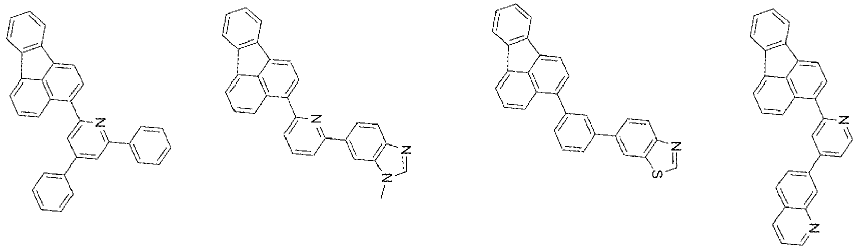

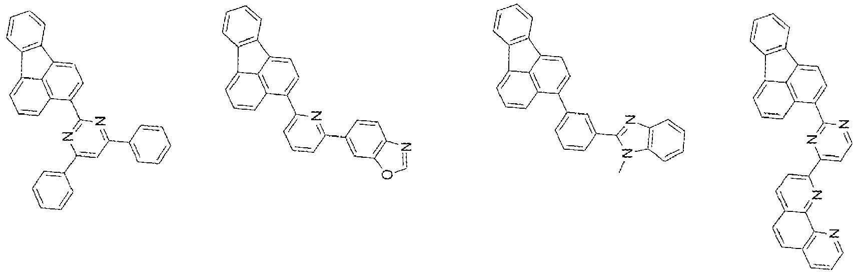

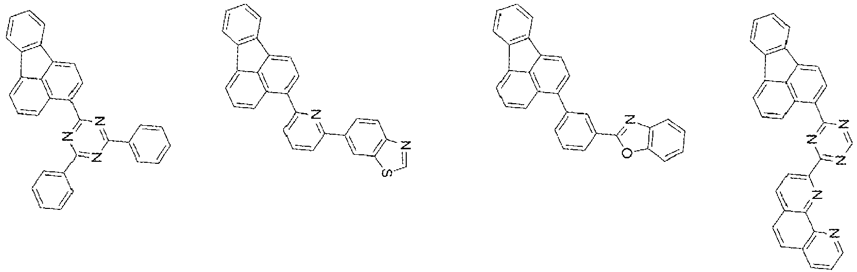

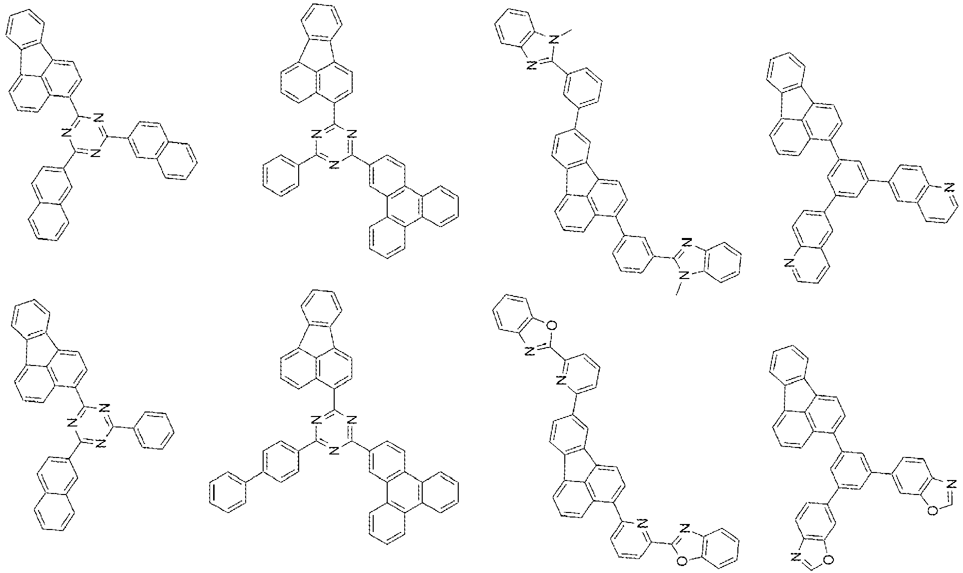

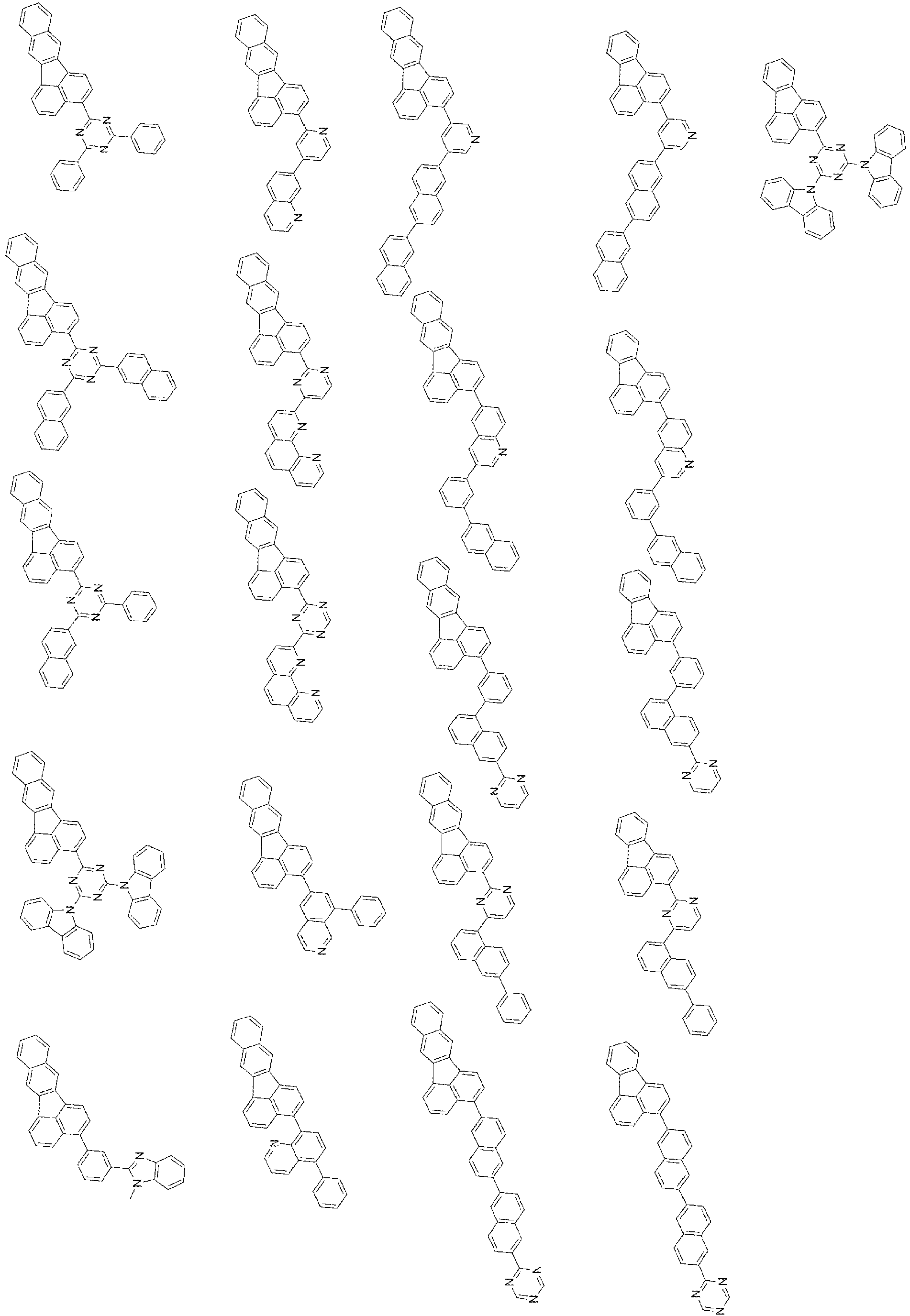

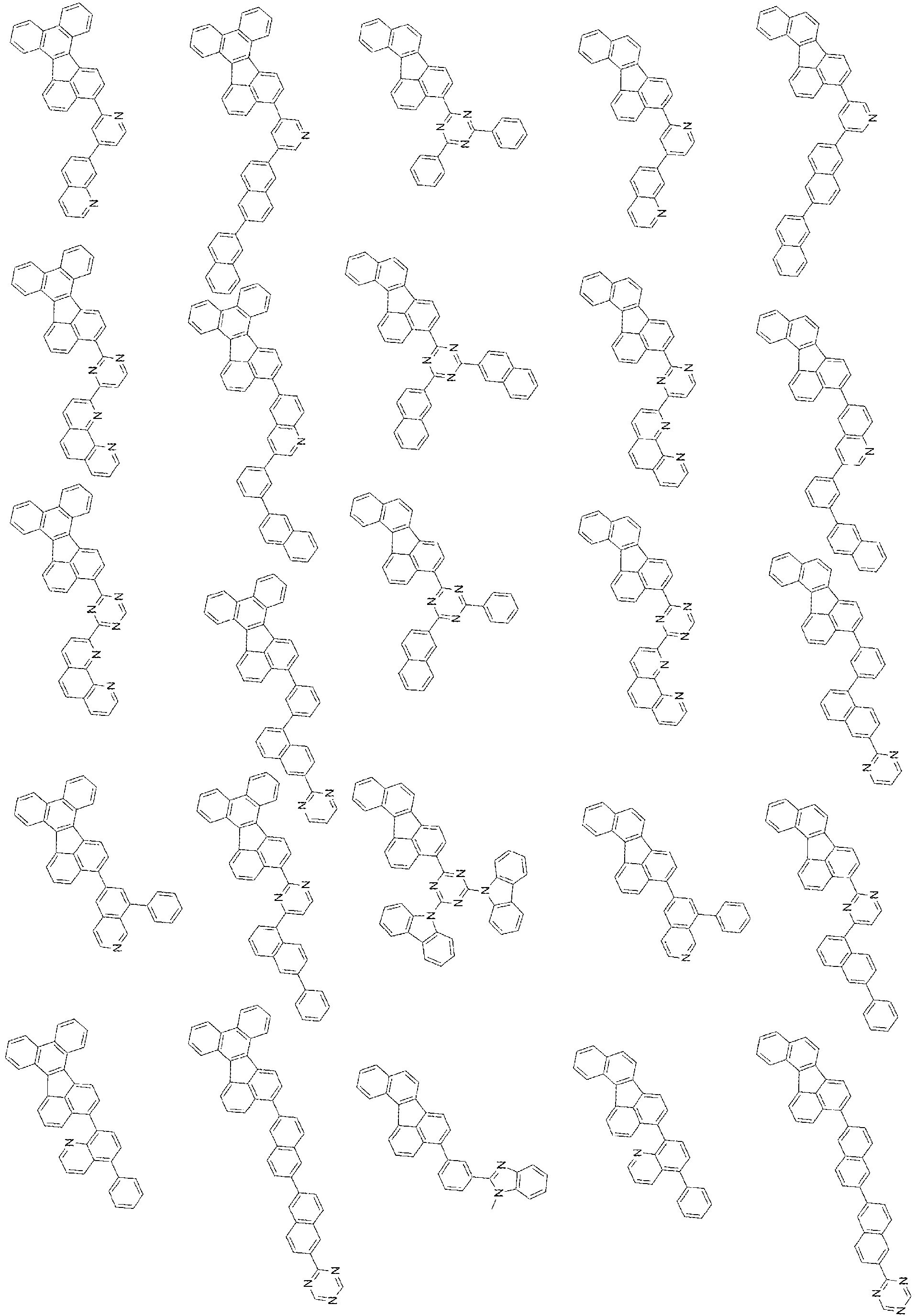

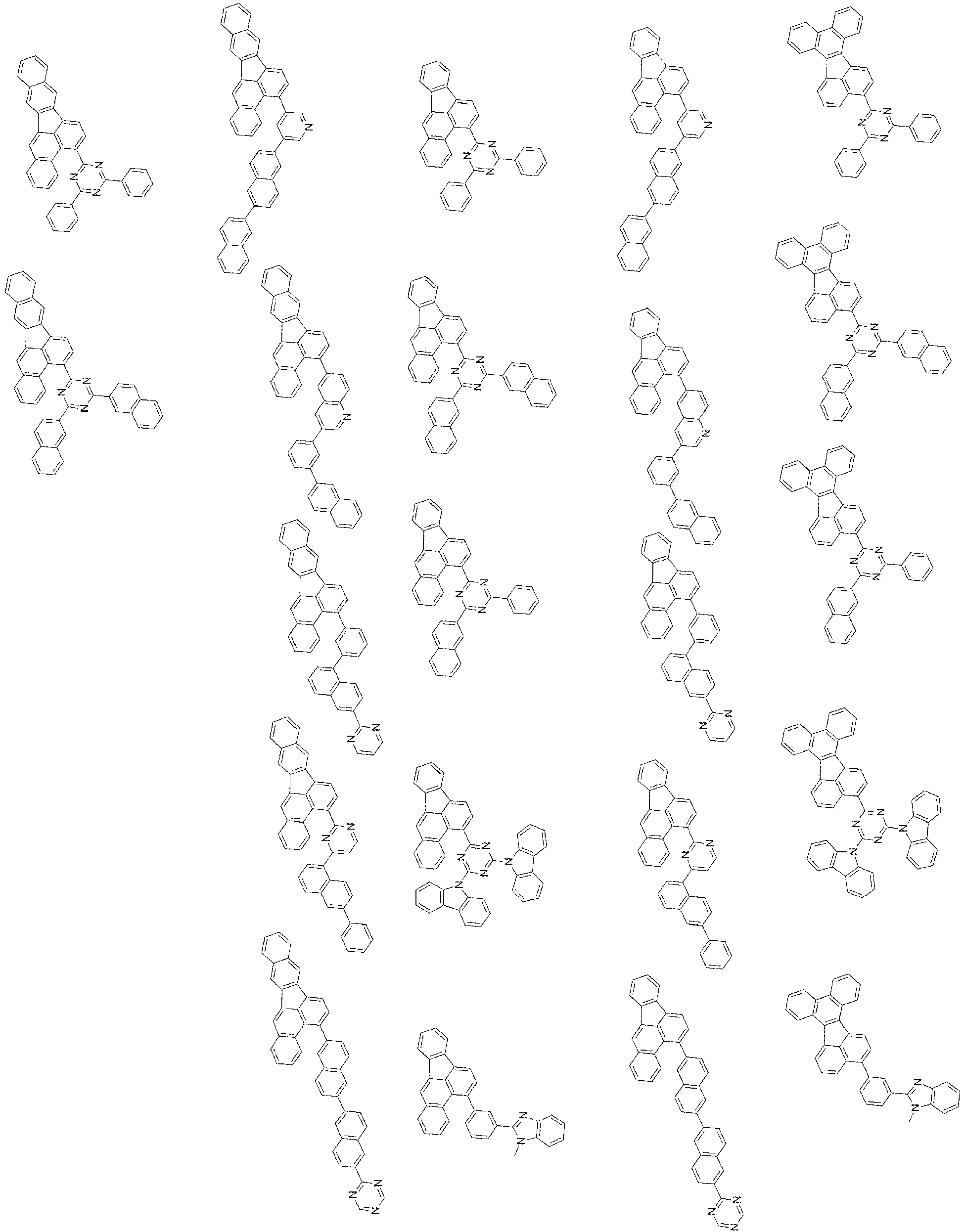

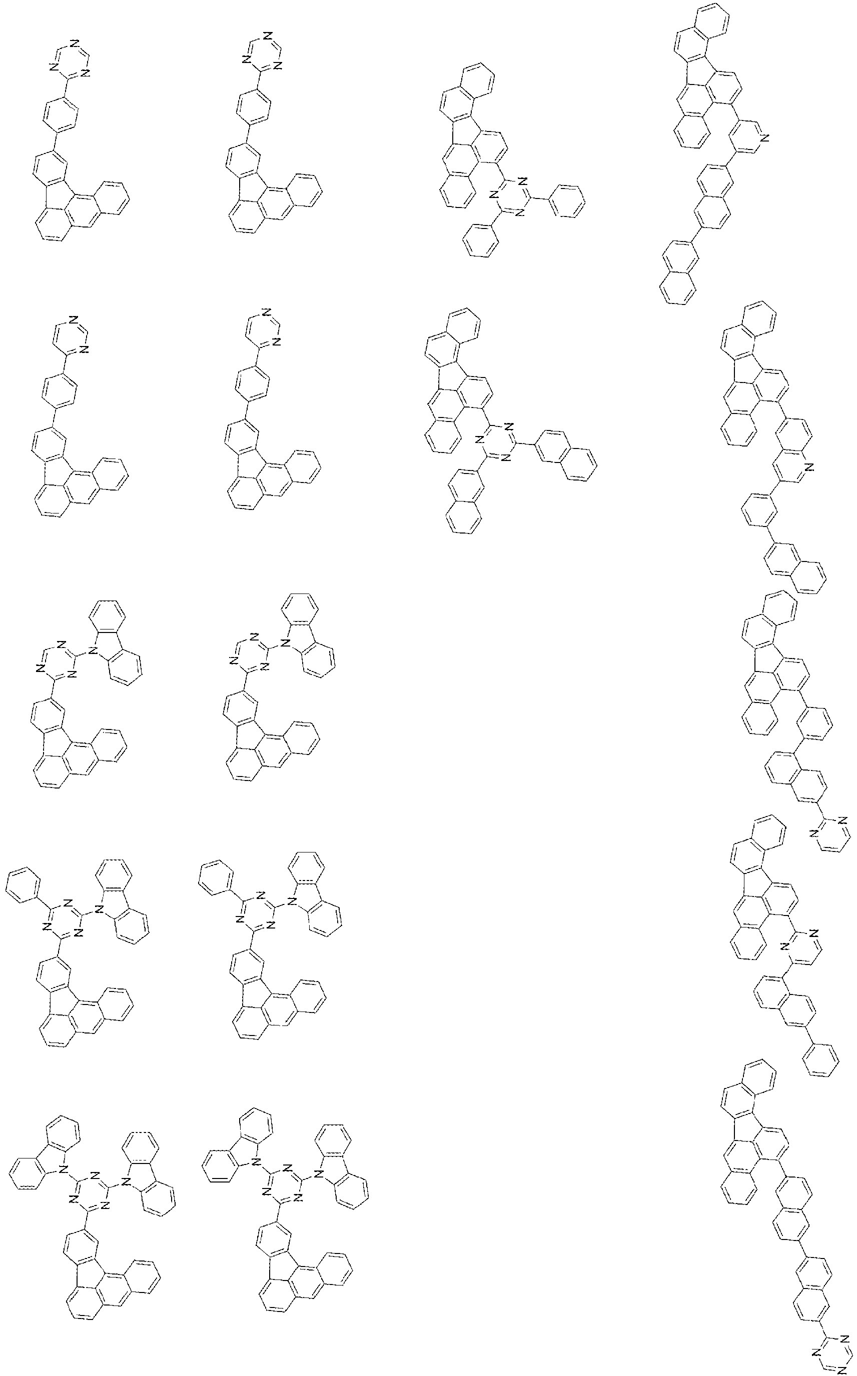

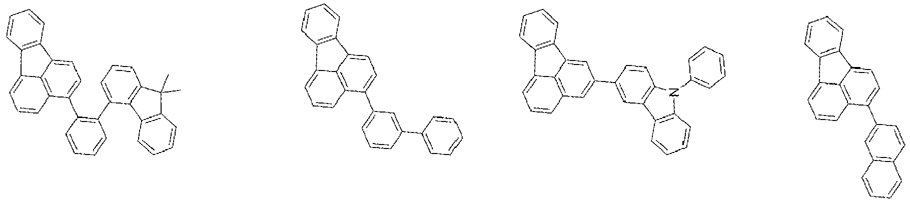

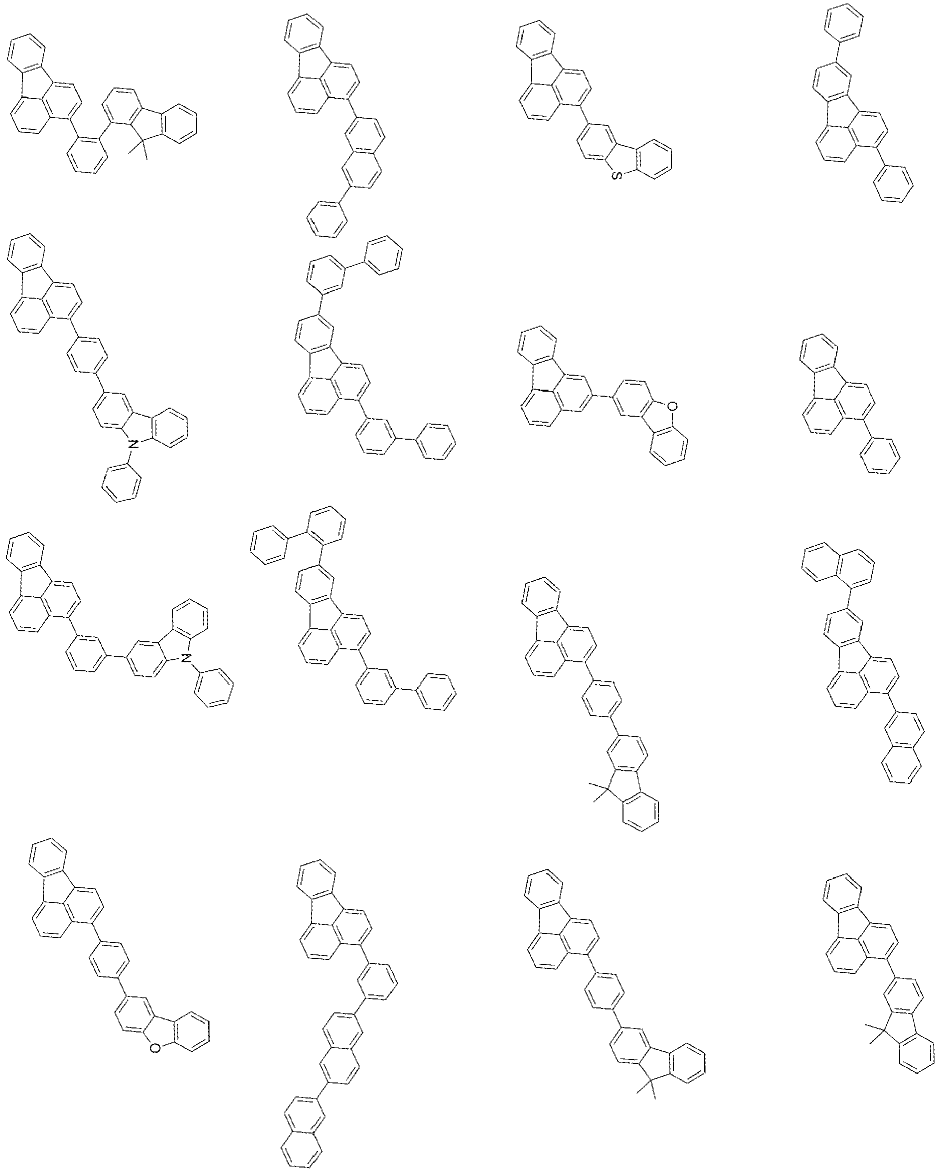

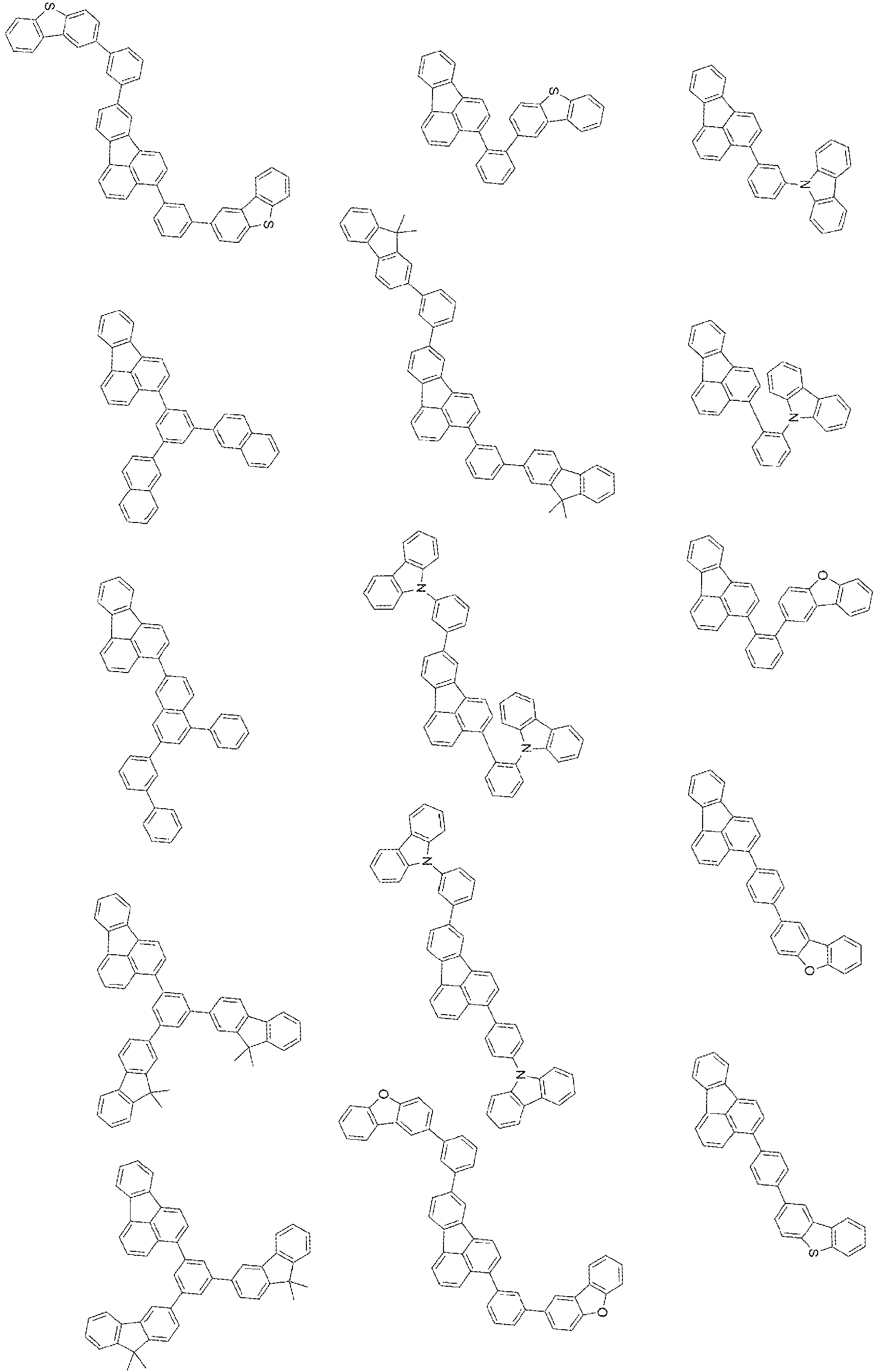

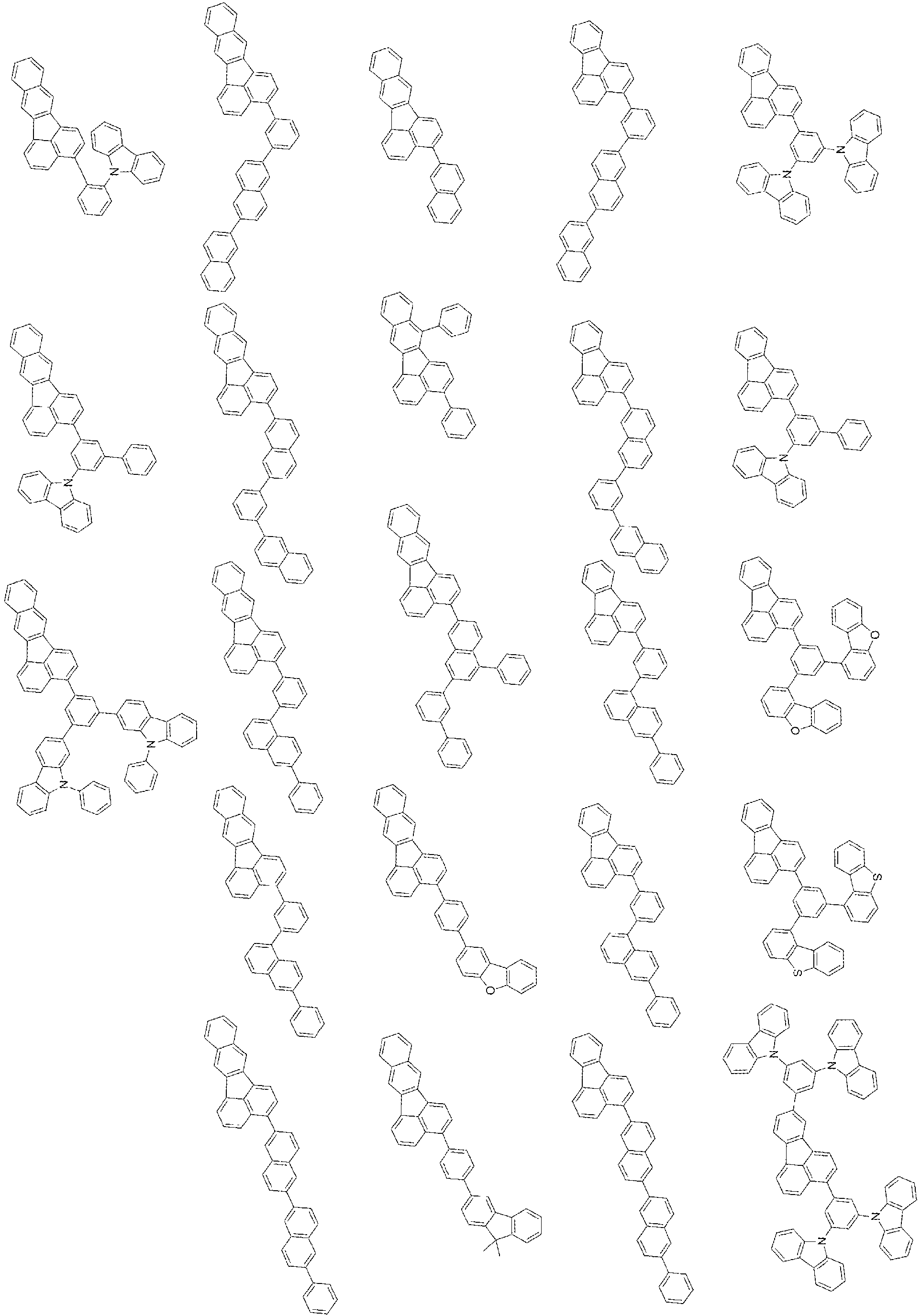

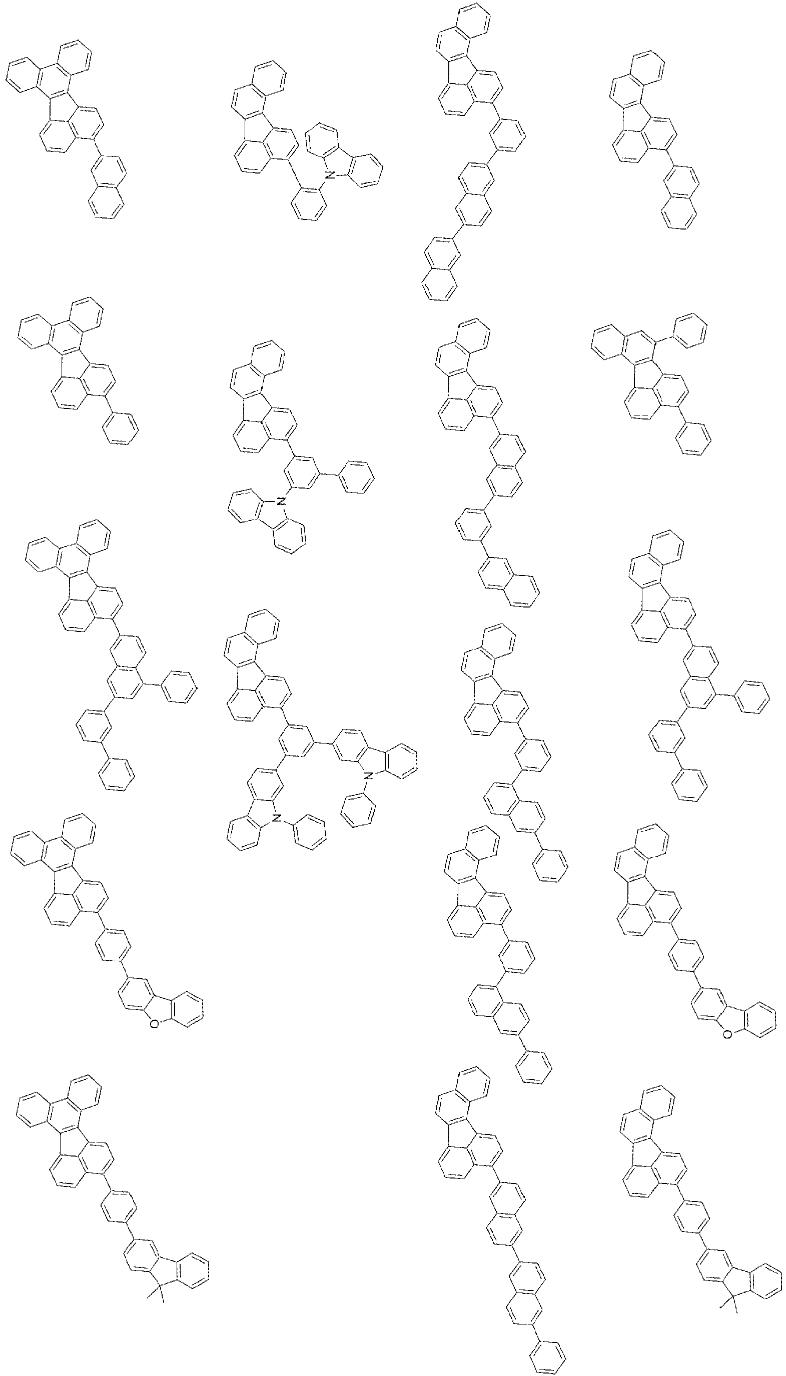

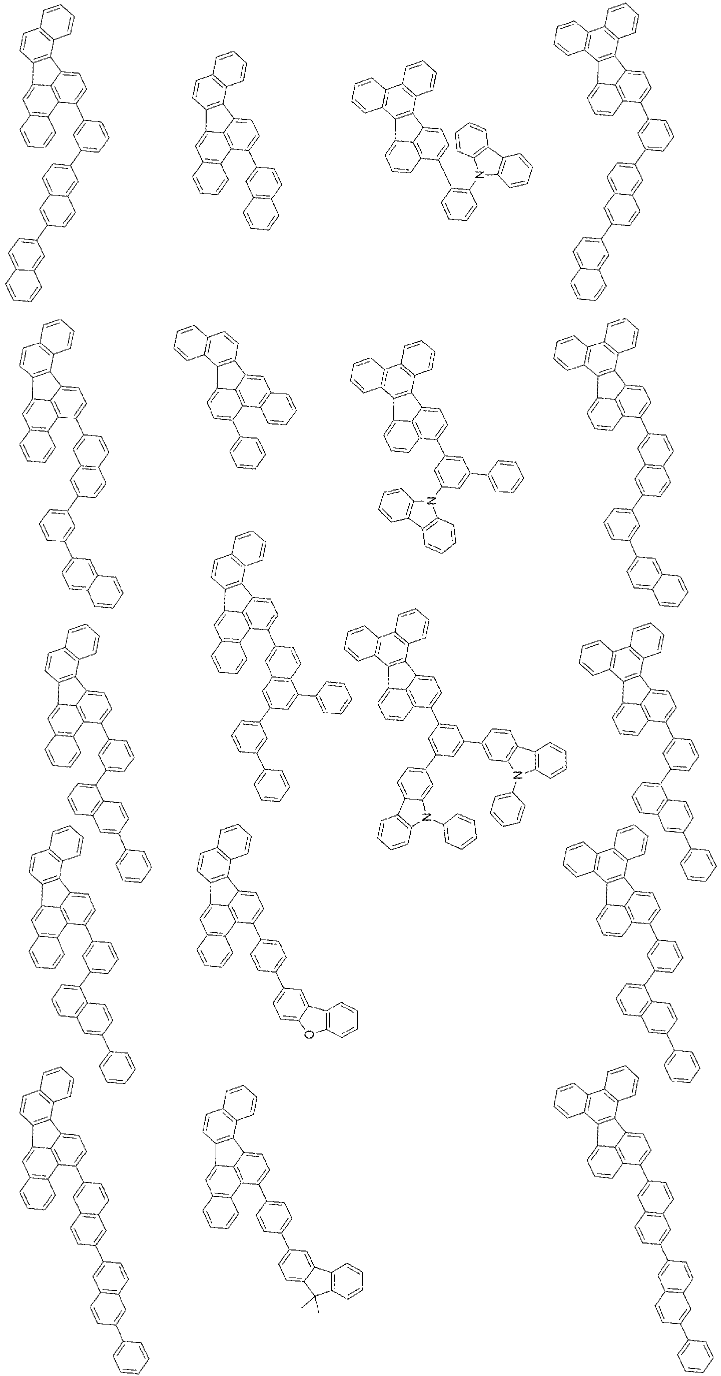

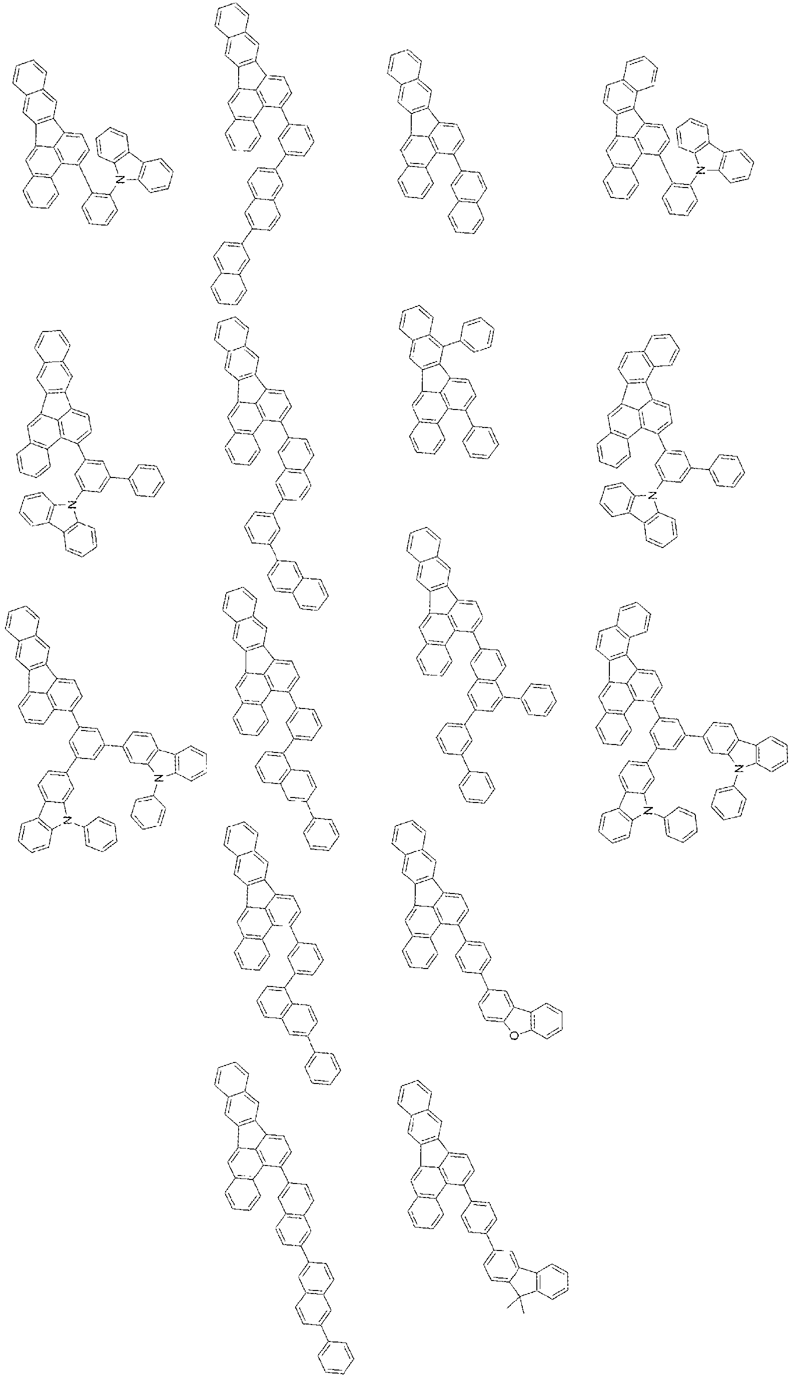

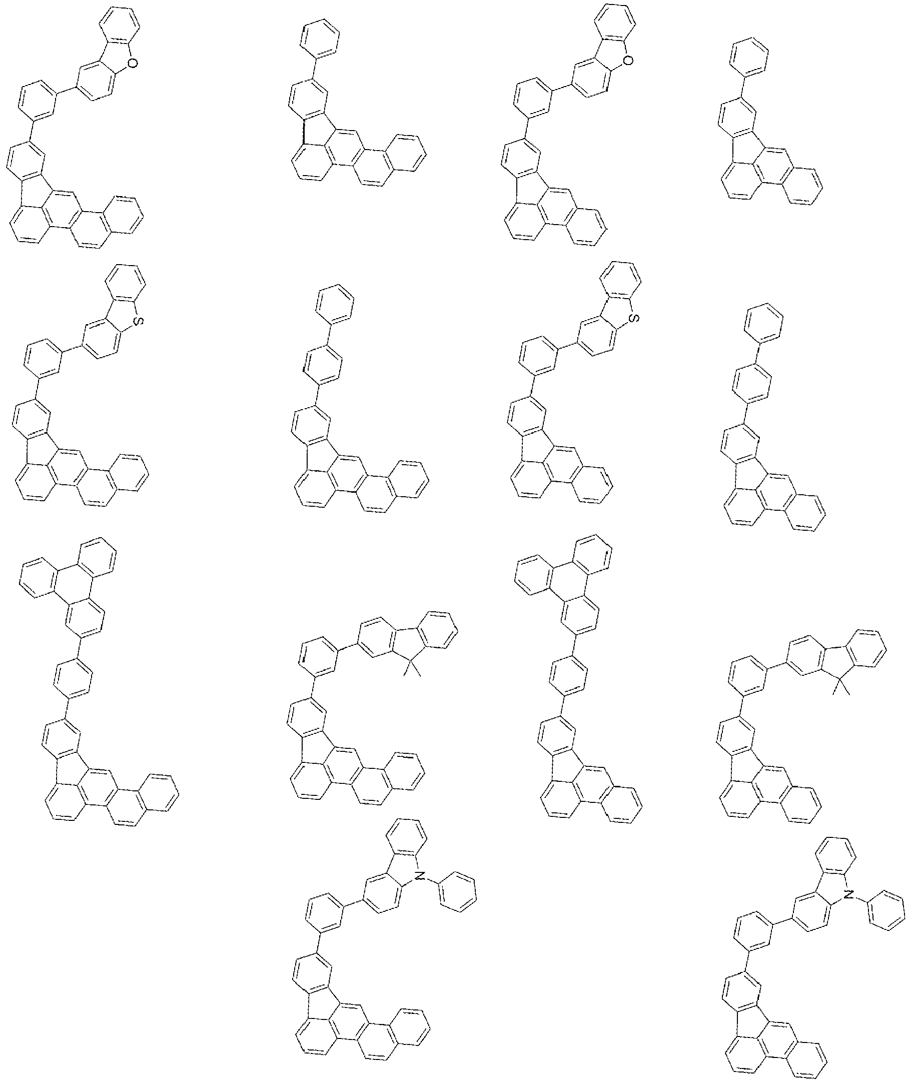

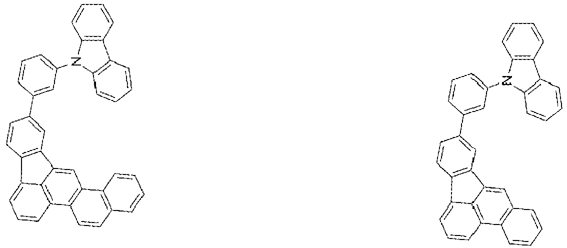

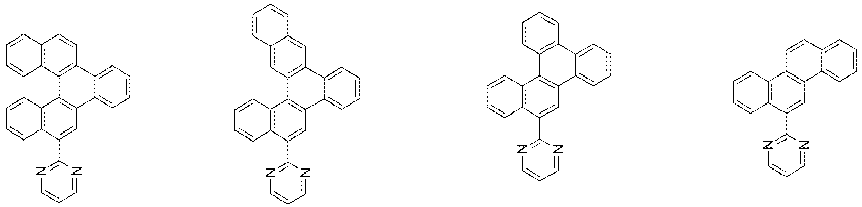

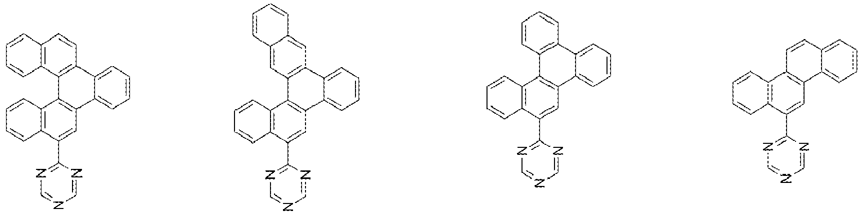

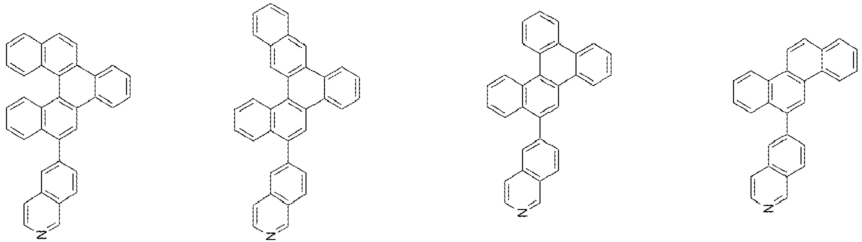

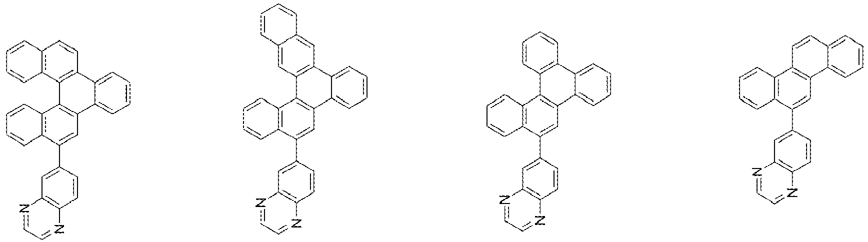

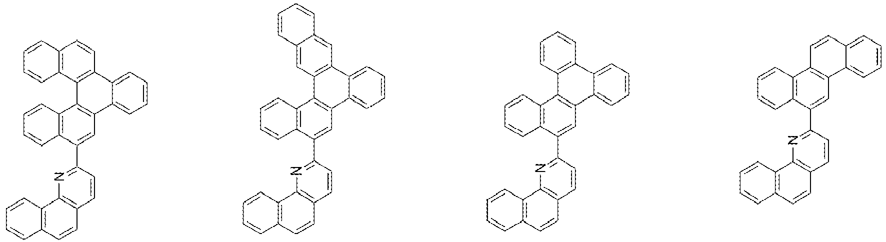

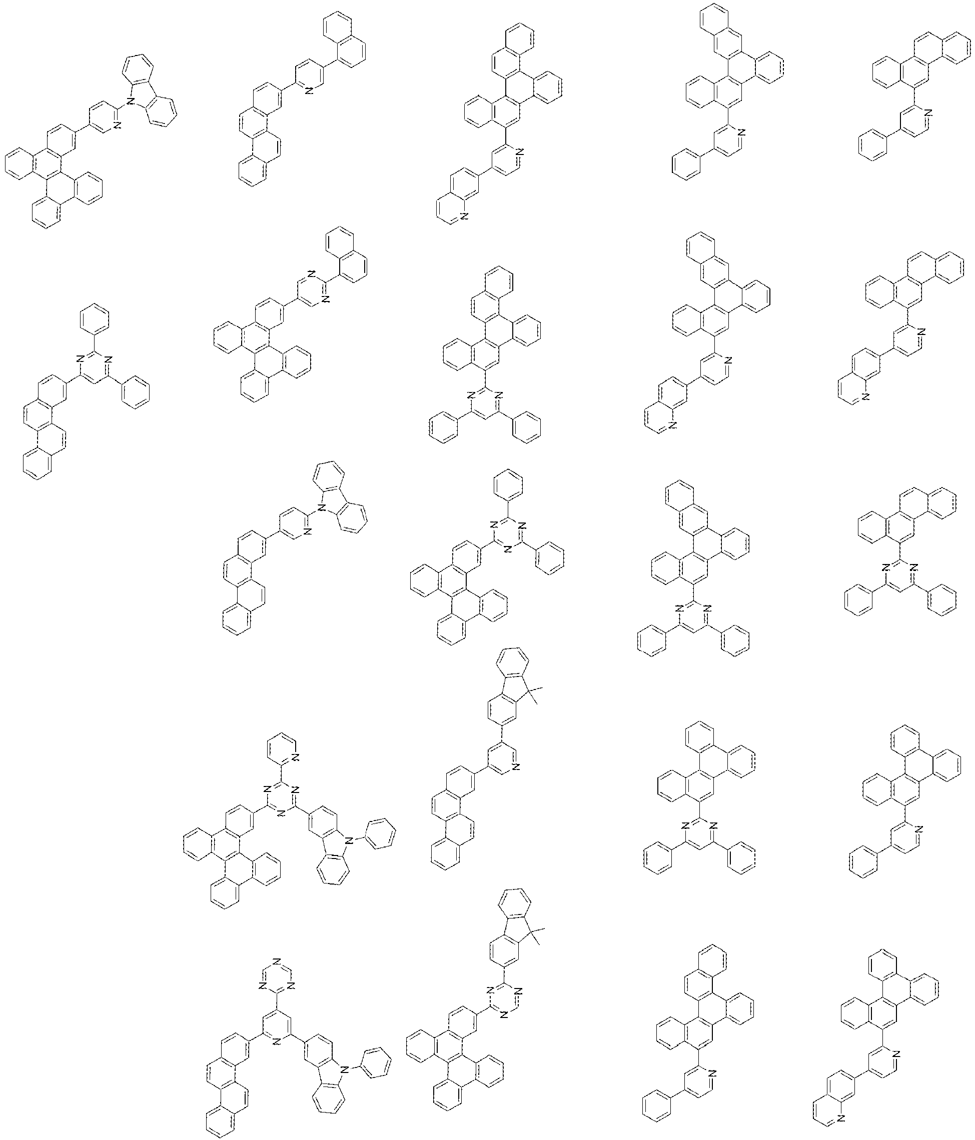

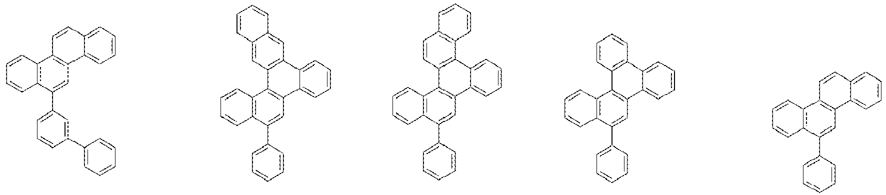

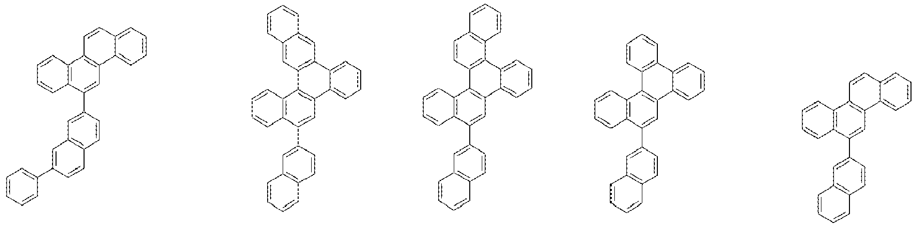

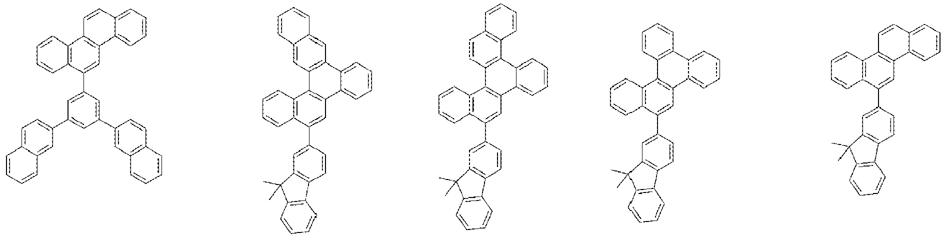

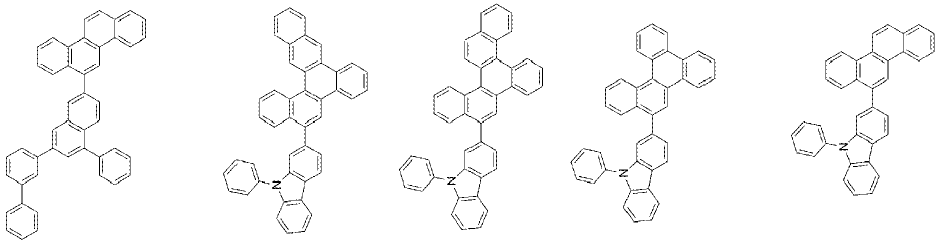

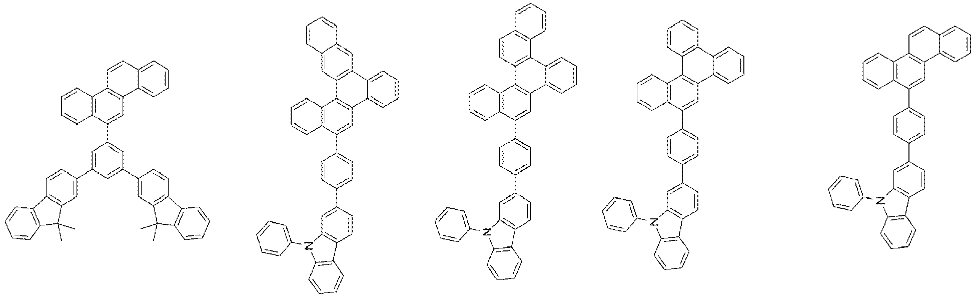

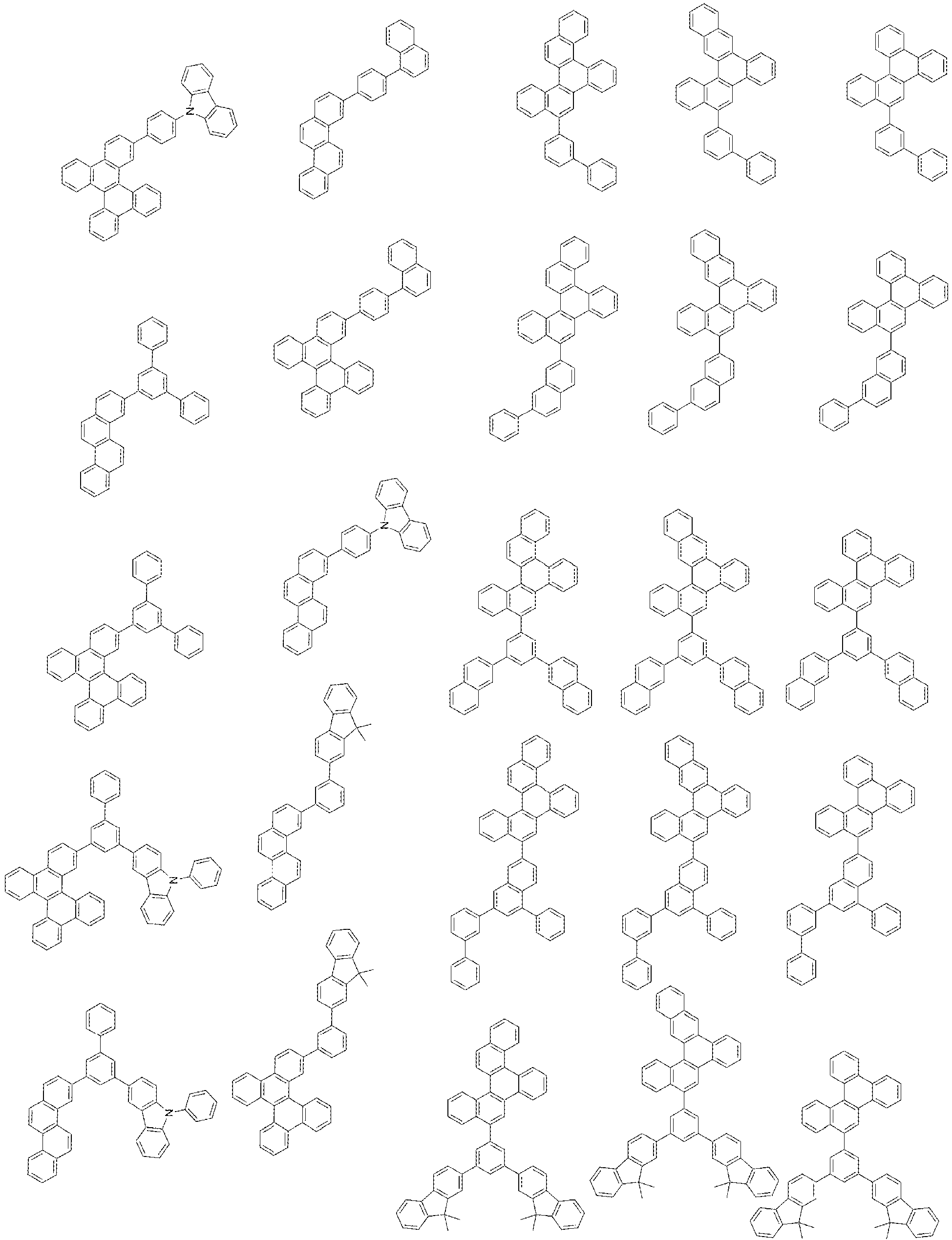

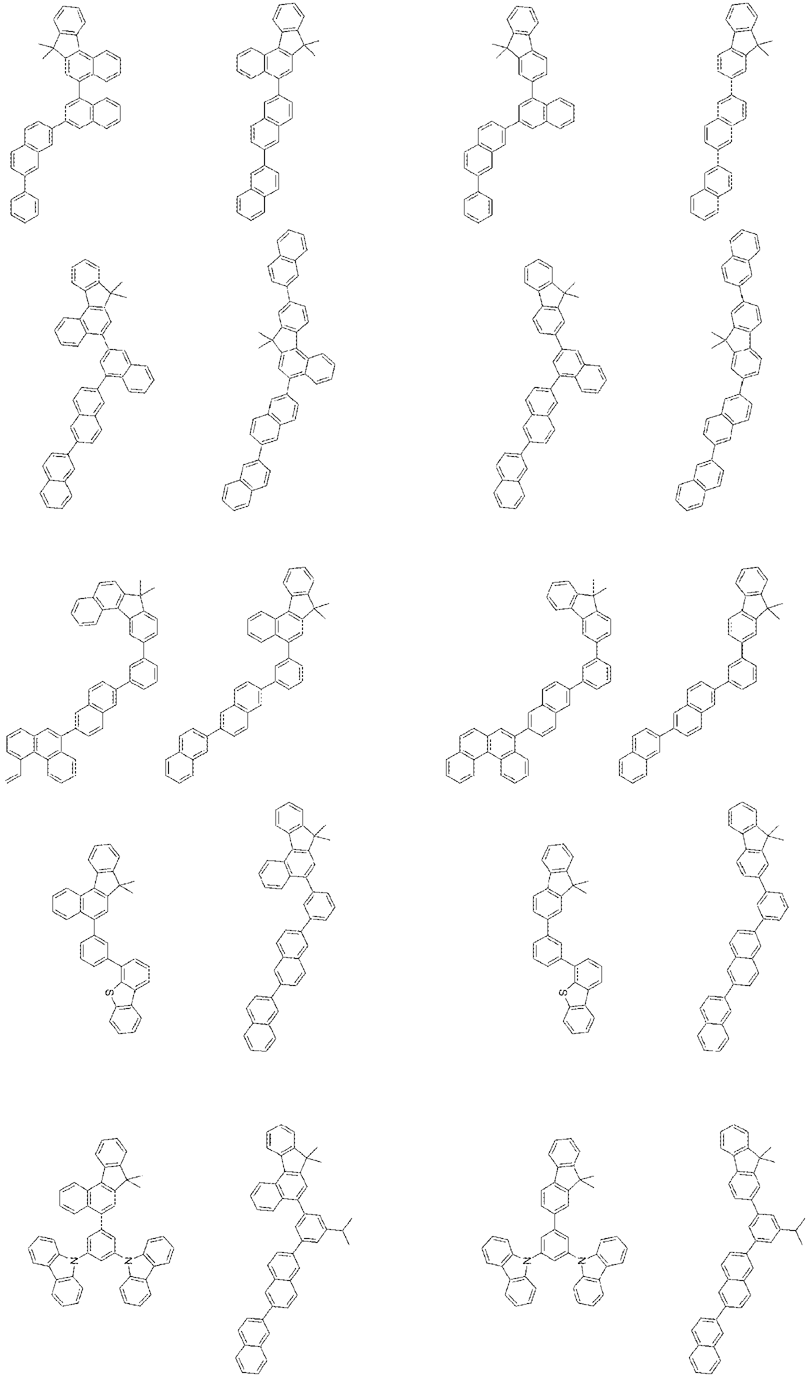

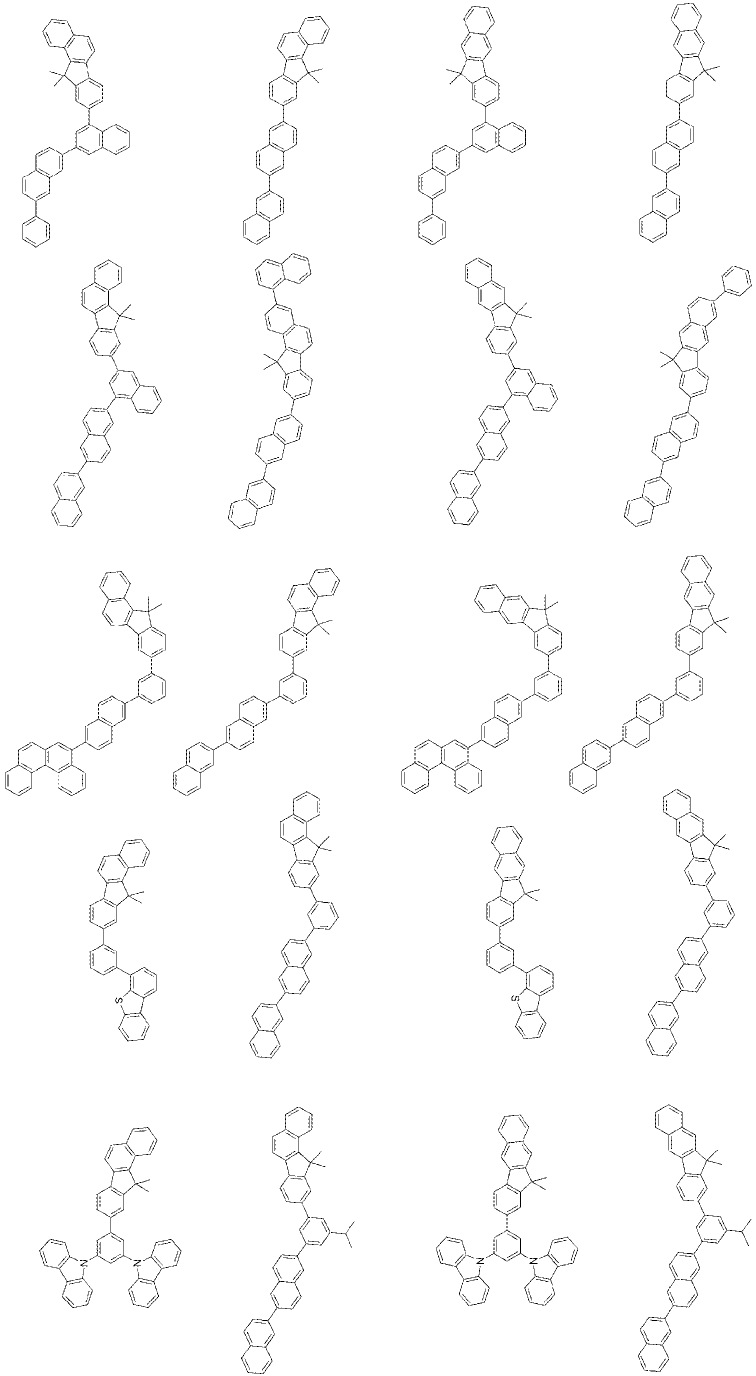

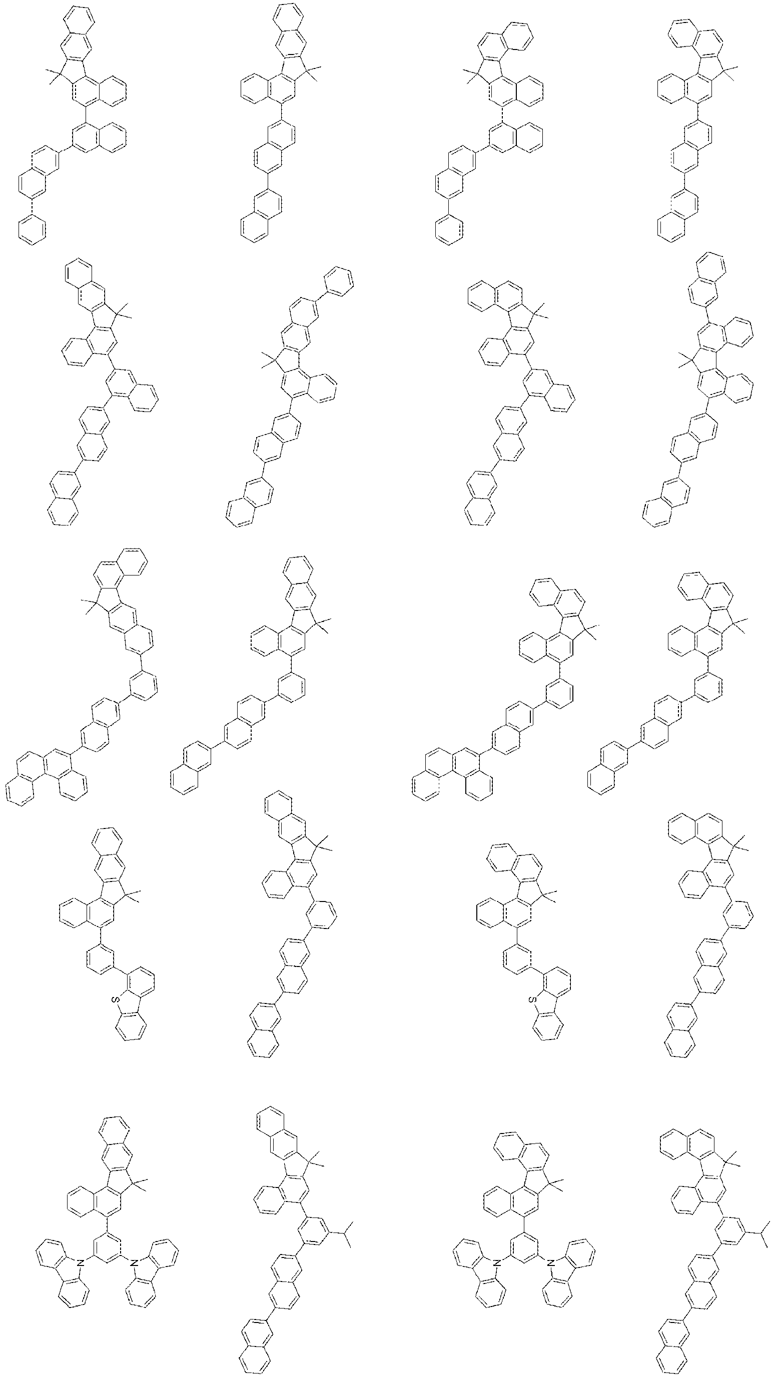

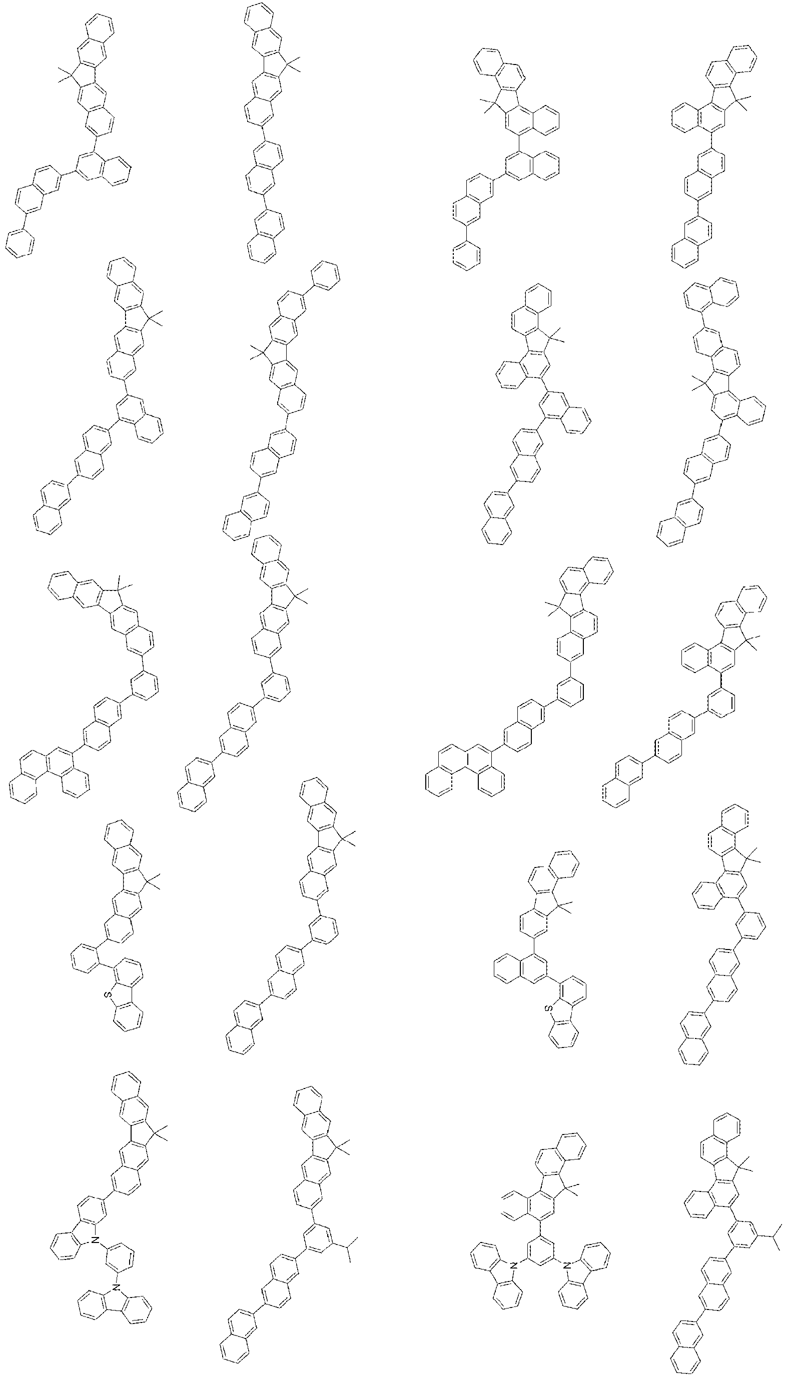

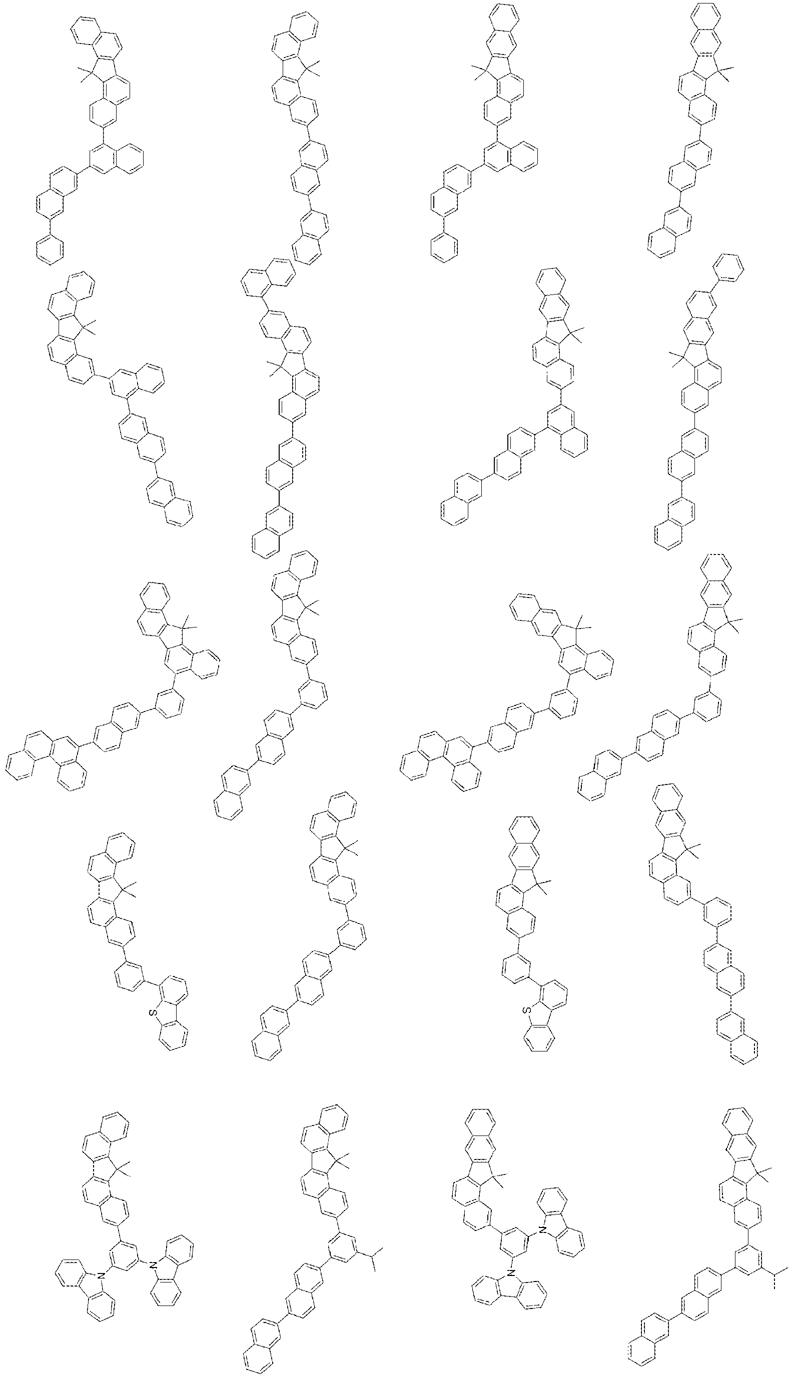

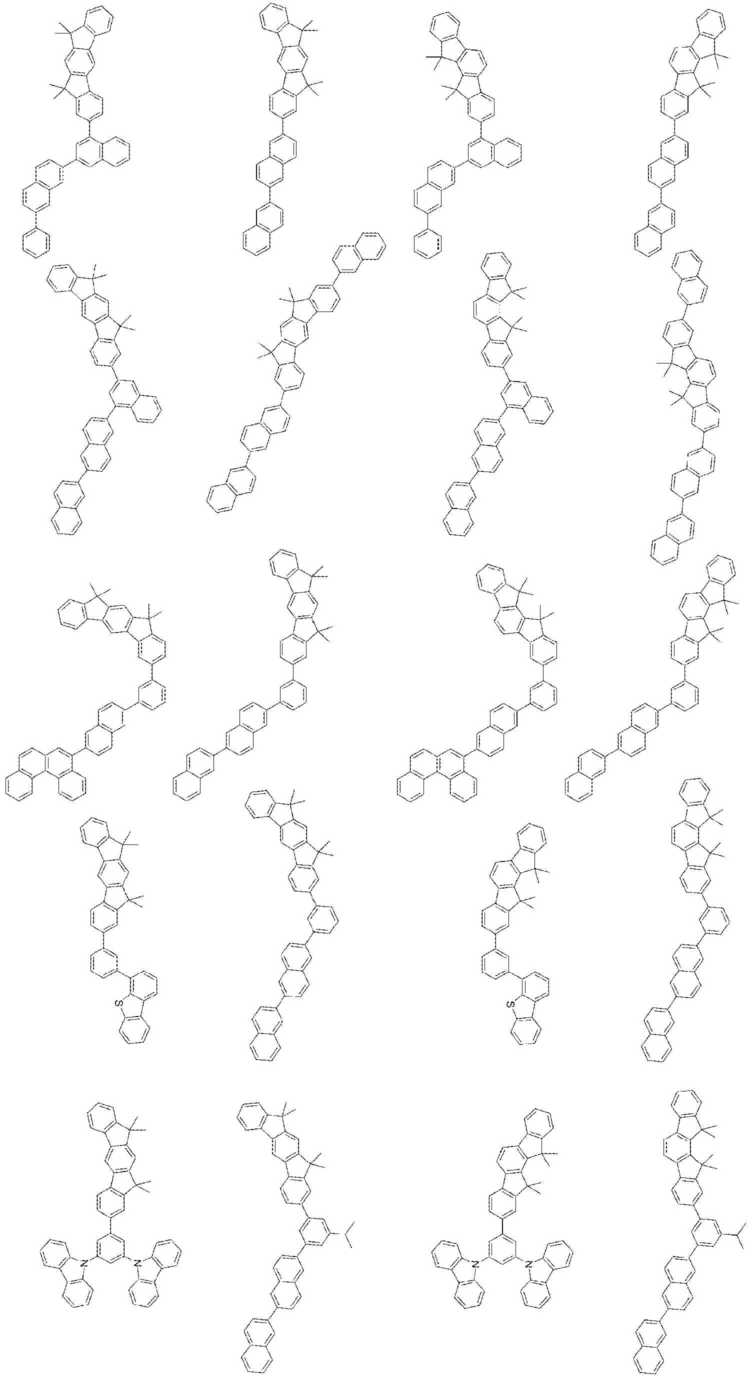

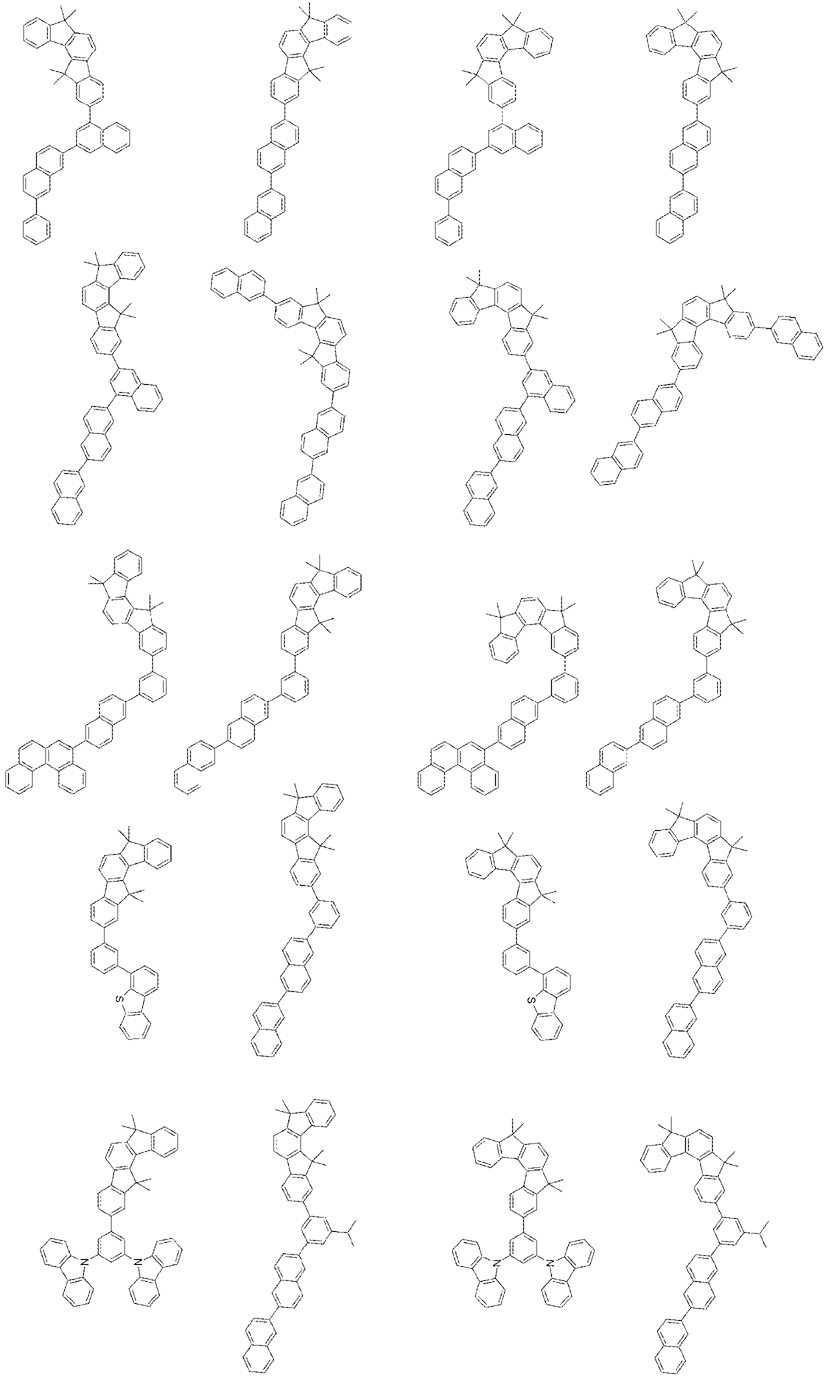

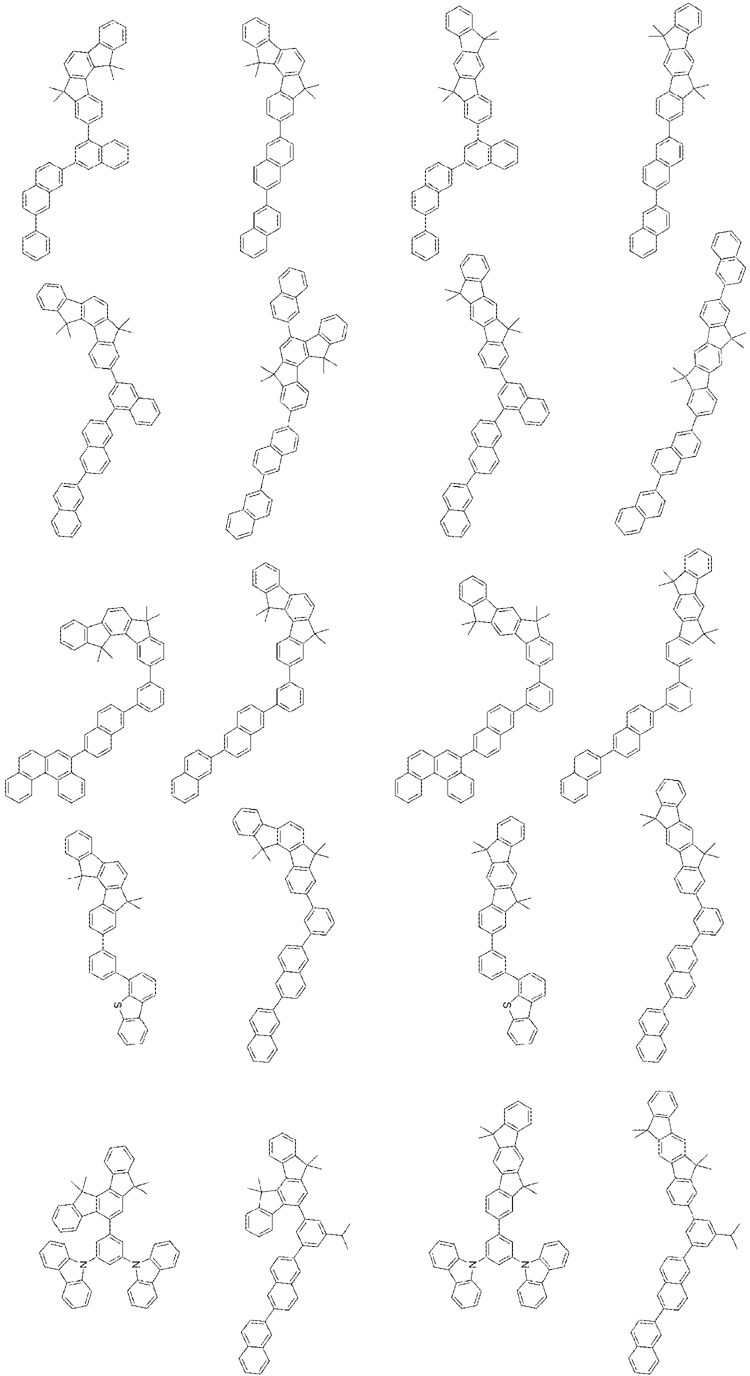

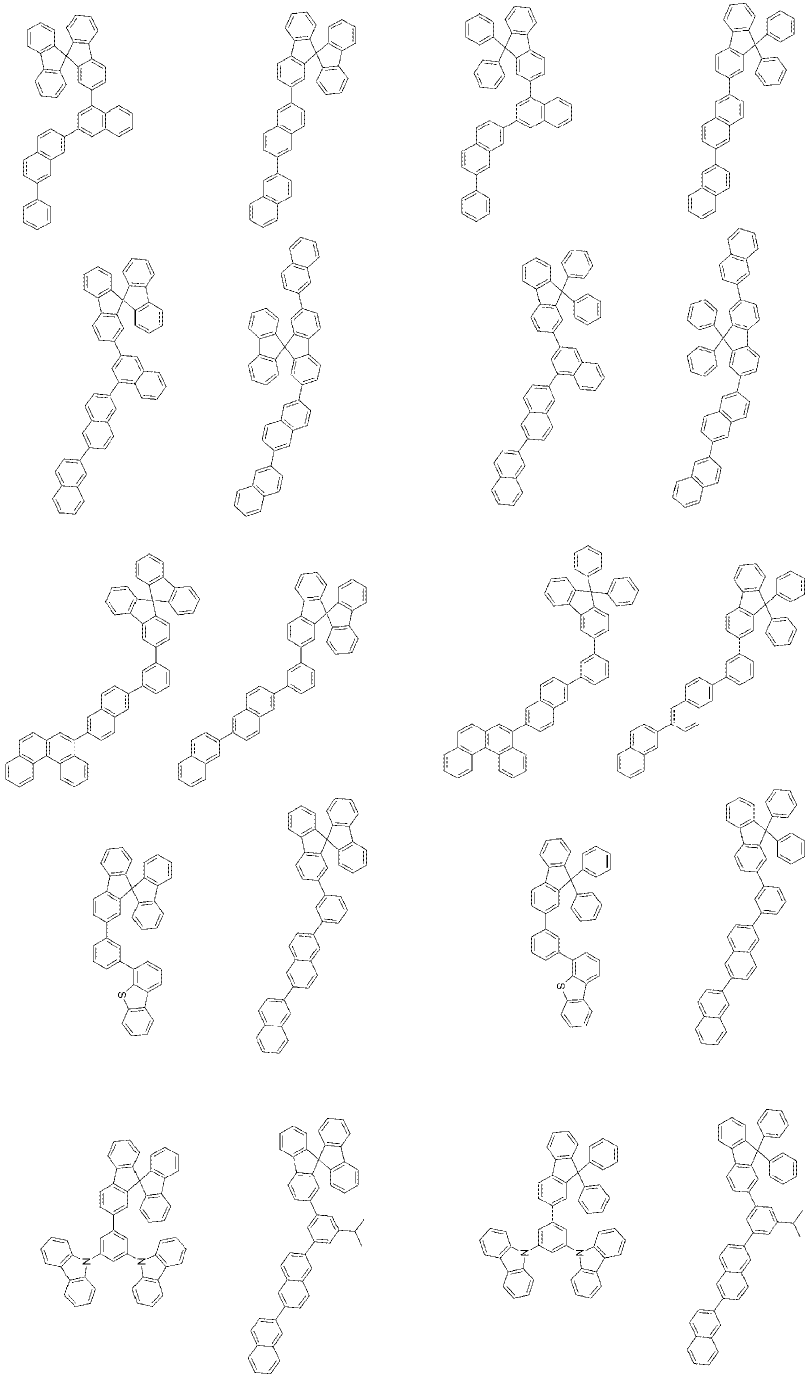

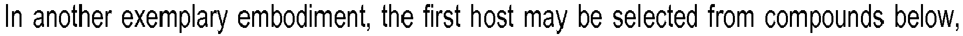

but the first host is not limited thereto:

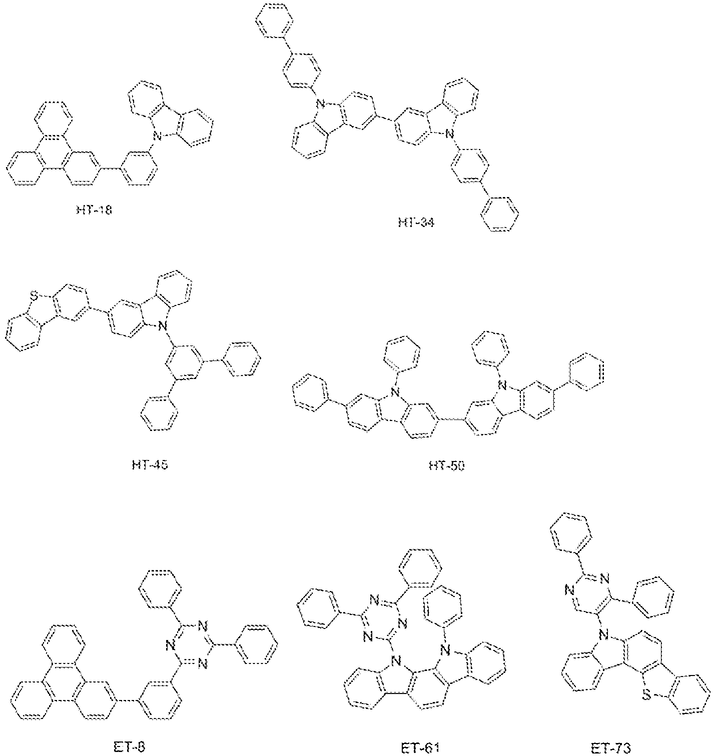

In another exemplary embodiment, the first host may be selected from compounds below, but the first host is not limited thereto:

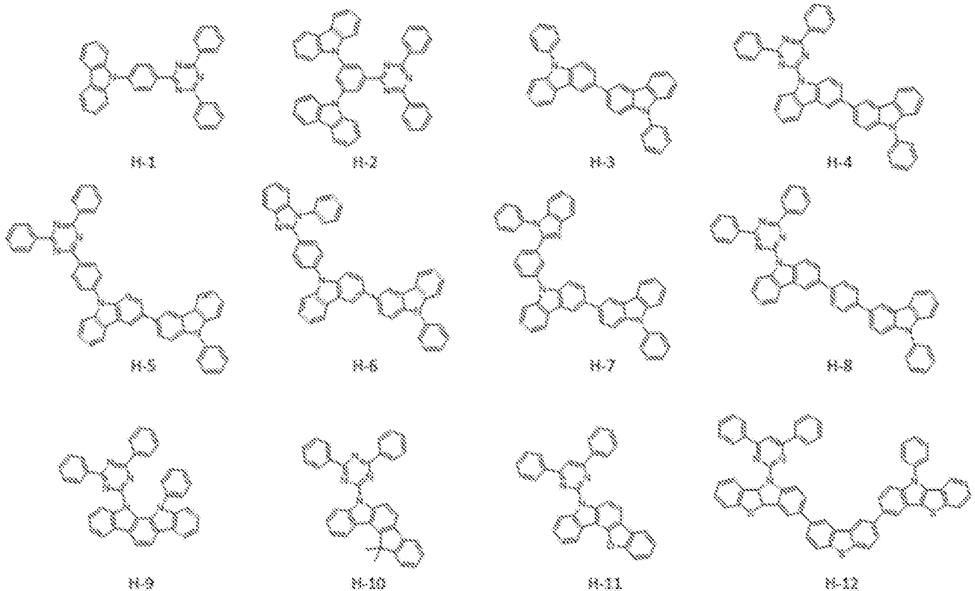

In another exemplary embodiment, the first host may be selected from compounds below, but the first host is not limited thereto:

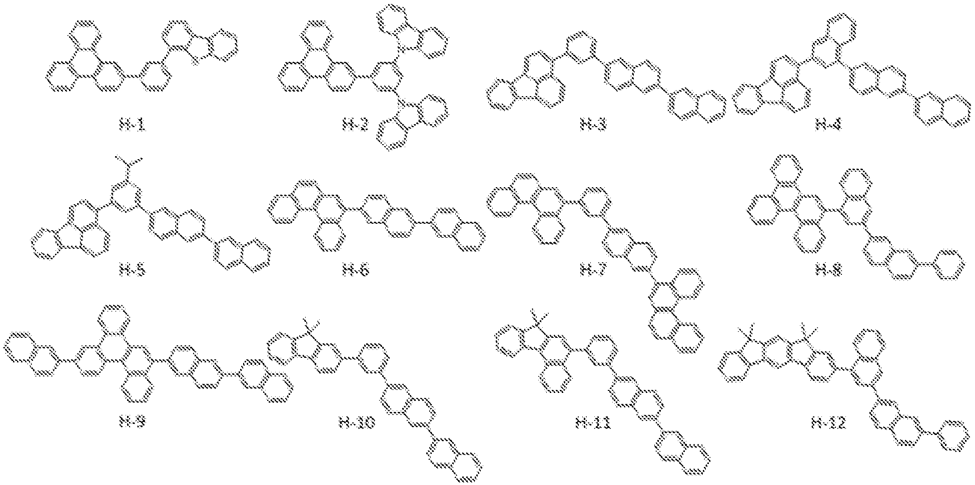

For example, the first host may have a triplet energy gap of 2.1 eV or more, but the first host is not limited thereto. When the first host has a triplet energy gap of 2.1 eV or more, the first host may have an excited state of triplet excitons of the EML in an efficient manner.

The EML may further include a second host, and the second host may be different from the first host, but the second host is not limited thereto.

For example, the second host may be selected from compounds below, but the second host is not limited thereto:

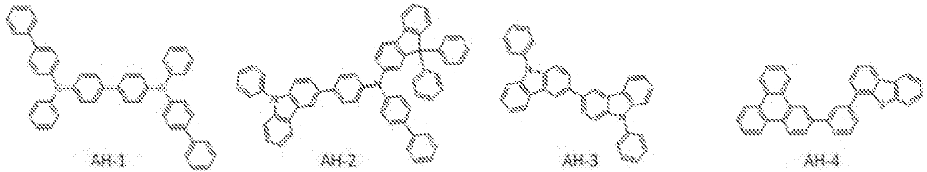

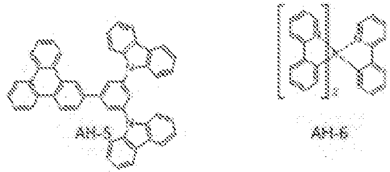

Hereinafter, an exemplary embodiment in which an EML of an organic light-emitting includes the first host and the second host will be described (Embodiment 2).

In this embodiment, the EML may include the first host and the second host, wherein the first host and the second host may each be independently represented by one of Formulae 1 and 2. The first host and the second host may be different from each other.

In another exemplary embodiment, the first host may be represented by Formula 1, and the second host may be represented by Formula 2, but the first host and the second host are not limited thereto.

In another exemplary embodiment, the first host may be represented by Formula 1, and the second host may be also represented by Formula 1, but the first host and the second host are not limited thereto.

In another exemplary embodiment, the first host may be represented Formula 2, and the second host may be also represented by Formula 2, but the first host and the second host are not limited thereto.

For example, Ar₁₁ in Formula 1 may be a group represented by one of Formulae 8A-1 to 8A-4, and Ar₂₁ in Formula 2 may be a group represented by one of Formulae 9A-1 to 9A-4, but Ar₁₁ and Ar₂₁ are not limited thereto:

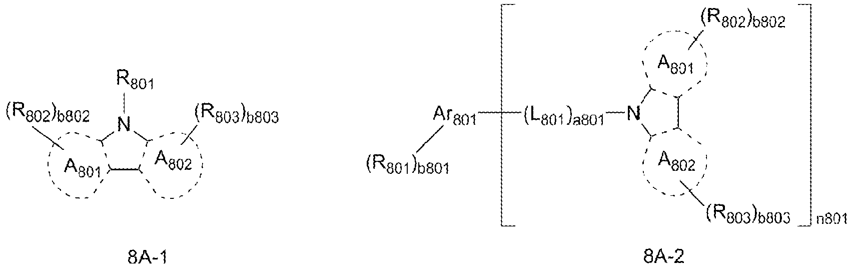

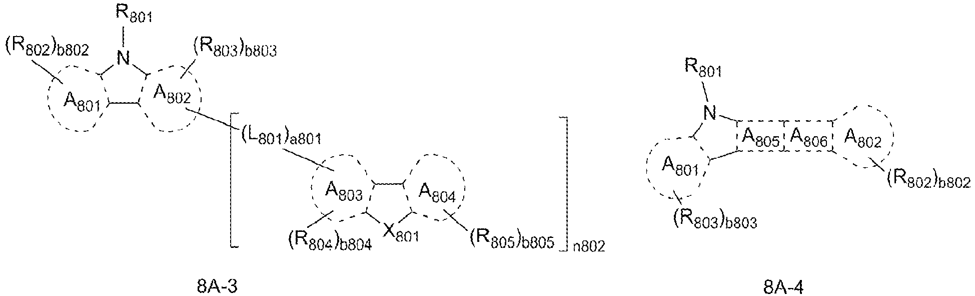

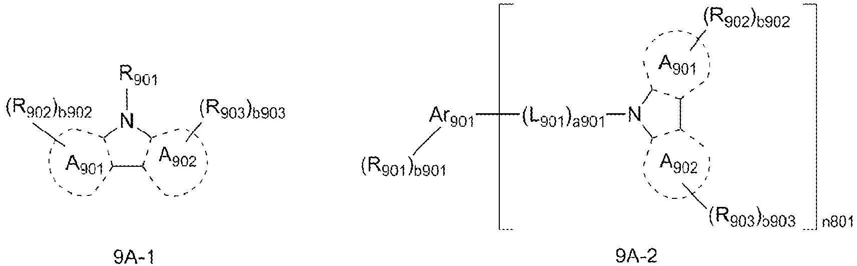

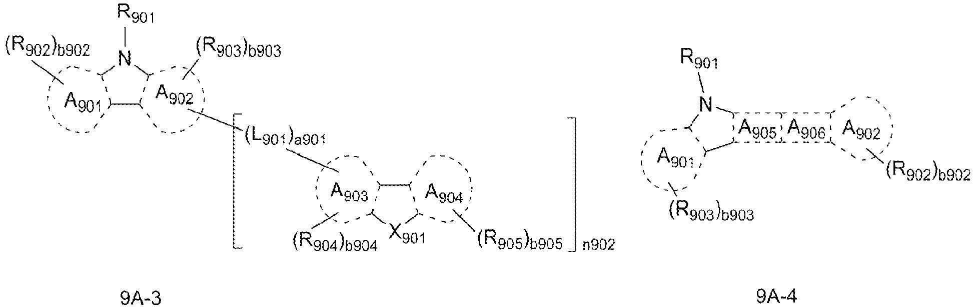

In Formulae 8A-1 to 8A-4 and 9A-1 to 9A-4,
descriptions of L₈₀₁, a801, A₈₀₁ to A₈₀₆, R₈₀₁ to R₈₀₅, b801 to b805, n801, and n802 may each be independently as referred to in the descriptions provided above, and descriptions of L₉₀₁, a901, A₉₀₁ to A₉₀₆, R₉₀₁ to R₉₀₅, b901 to b905, n901 m and n902 may each be independently as referred to in the descriptions provided above.

For example, Ar₁₁ in Formula 1 may be a group represented by one of Formulae 8B-1 to 8B-19 and 8C-1 to 8C-19, and Ar₂₁ in Formula 2 may be a group represented by one of Formulae 9B-1 to 9B-19 and 9C-1 to 9C-19, but Ar₁₁ and Ar₂₁ are not limited thereto:

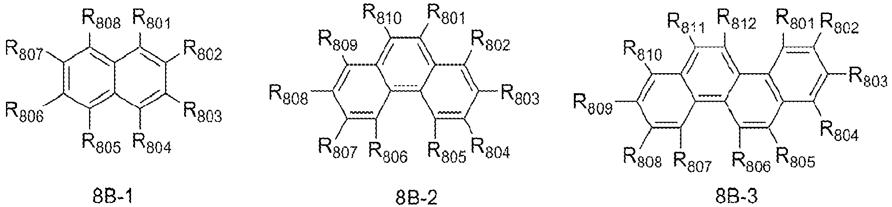

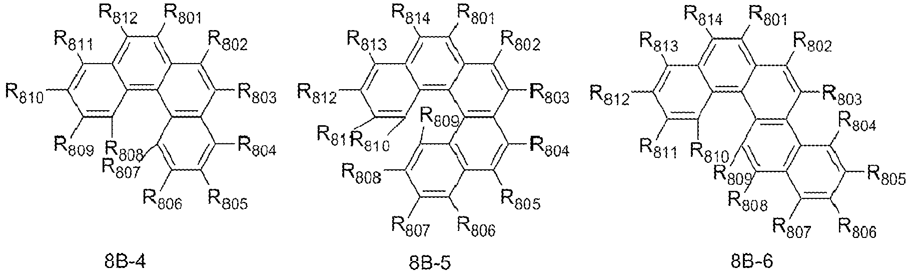

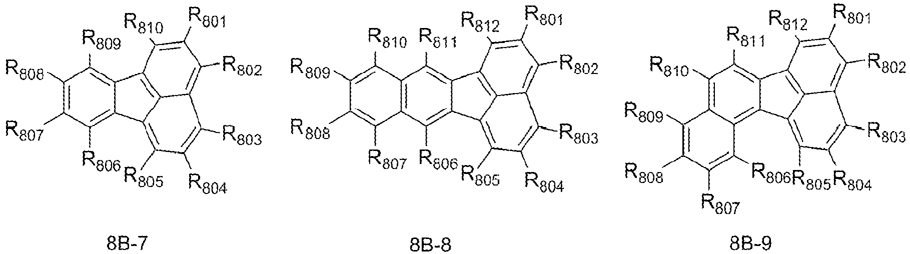

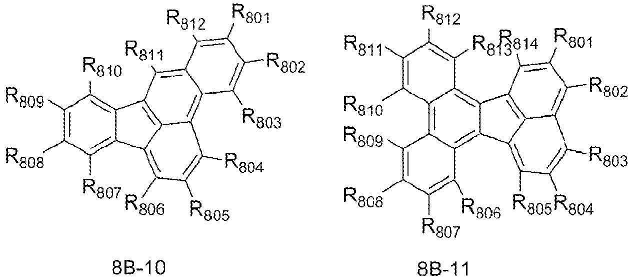

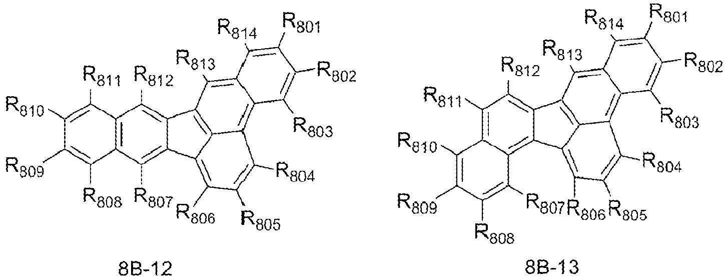

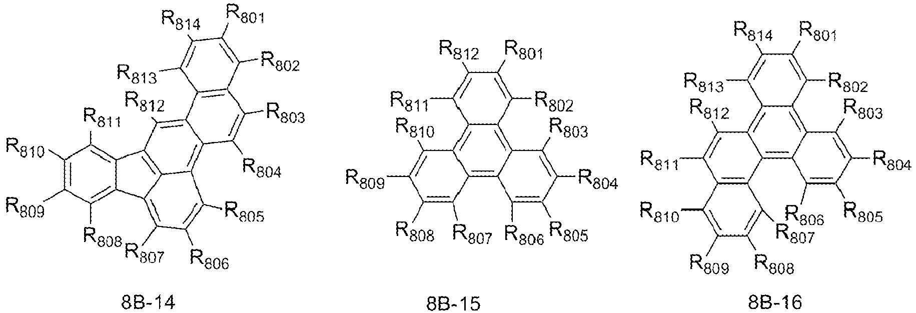

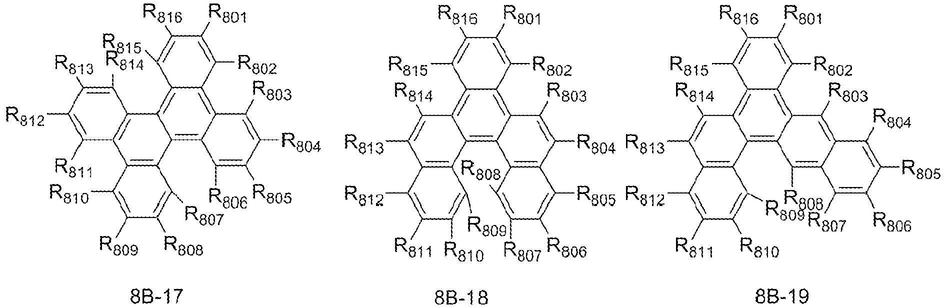

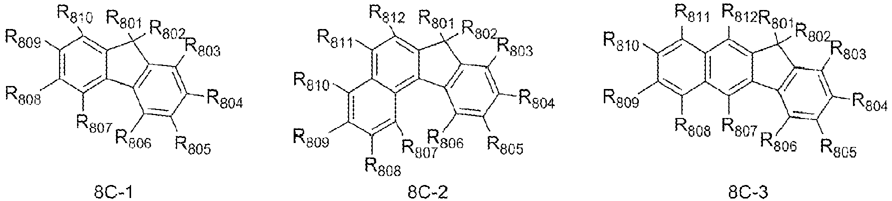

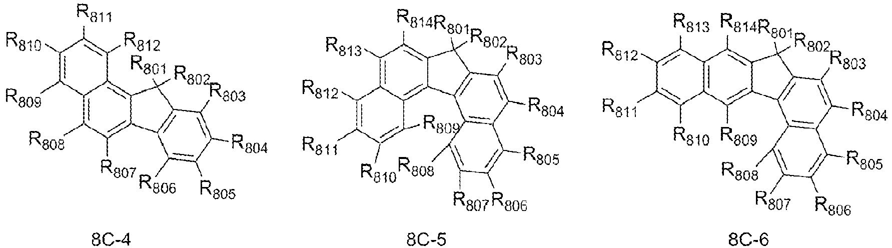

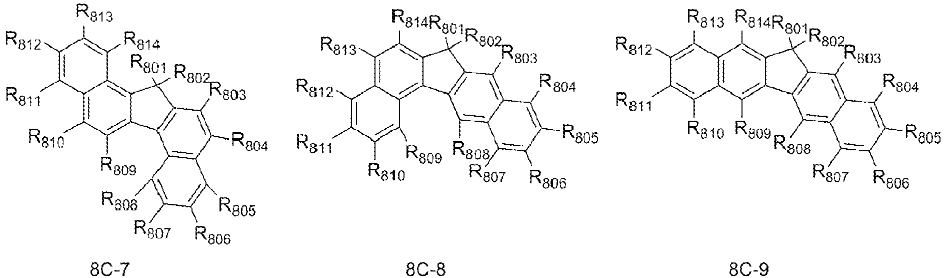

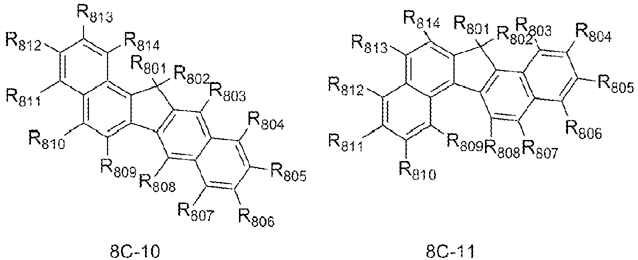

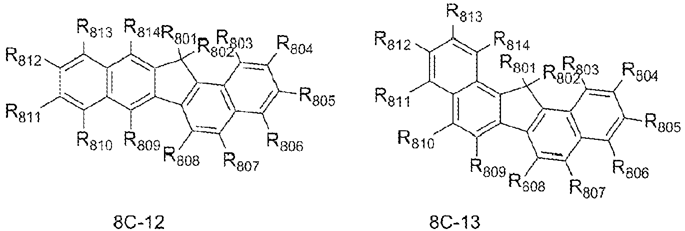

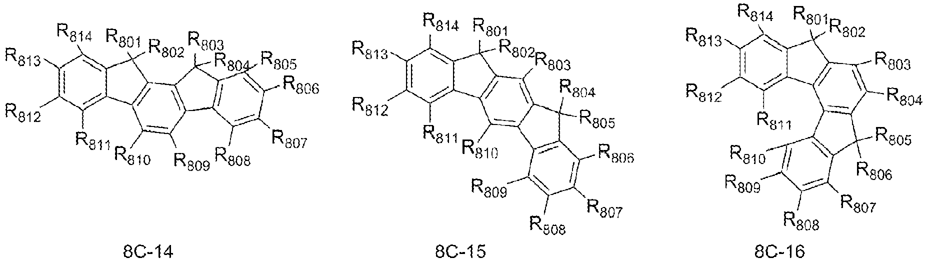

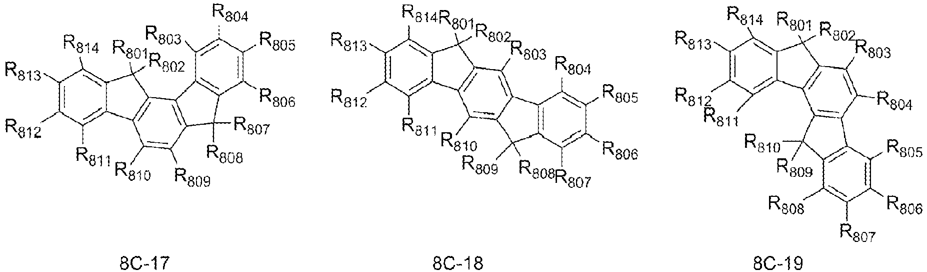

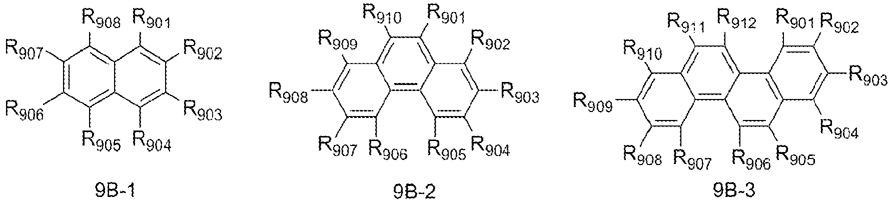

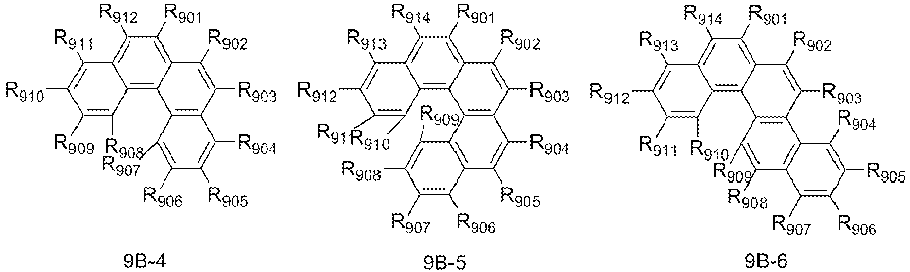

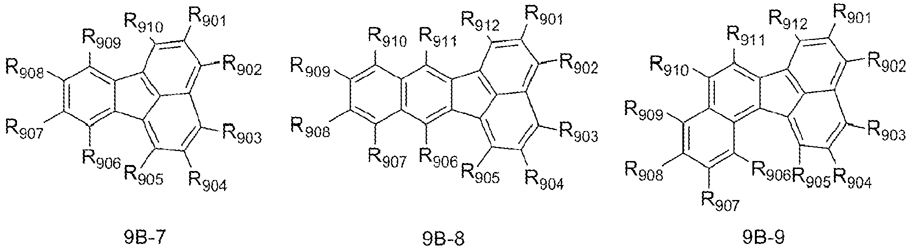

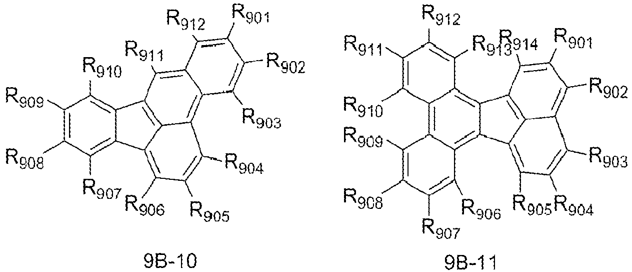

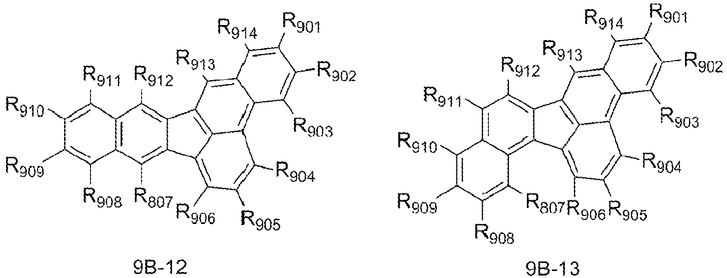

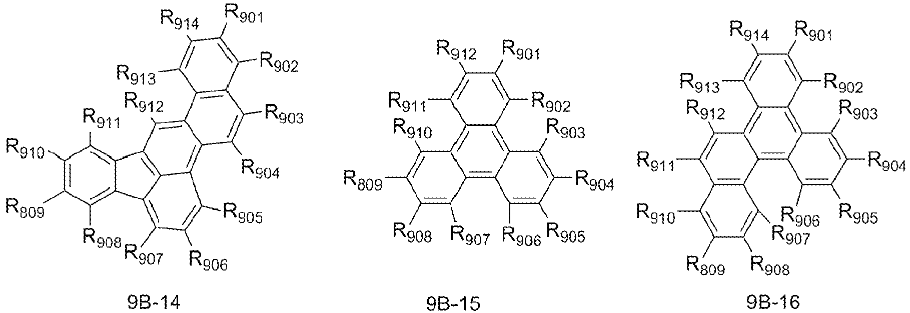

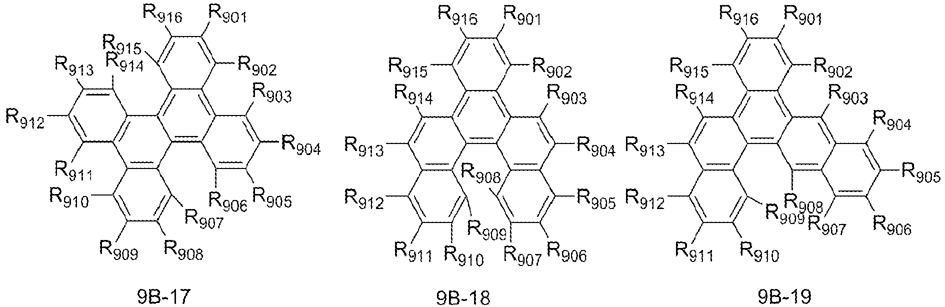

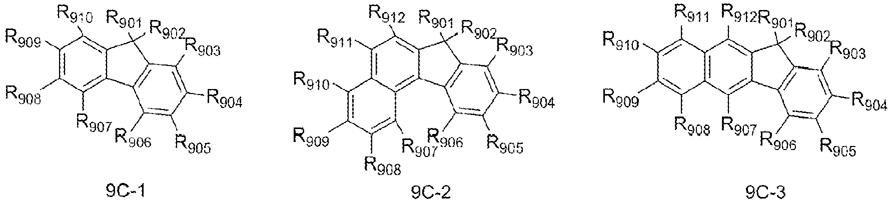

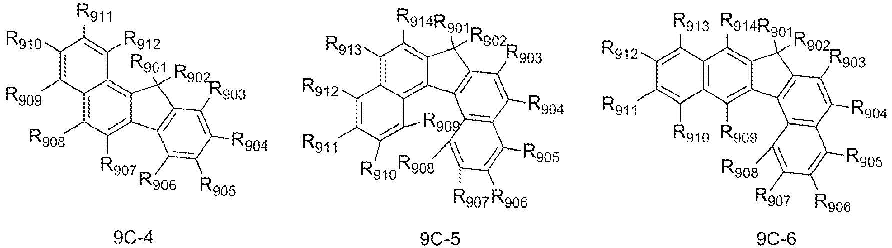

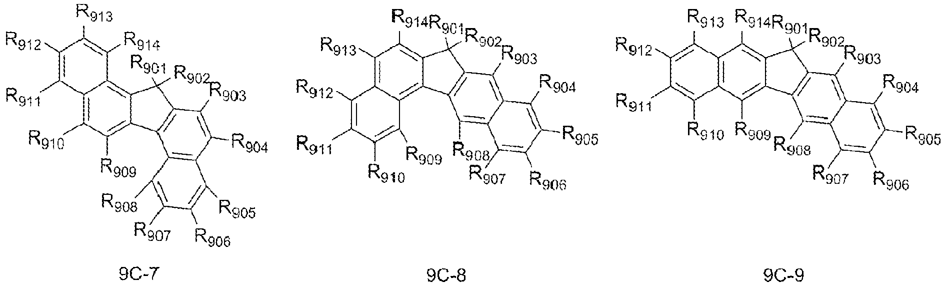

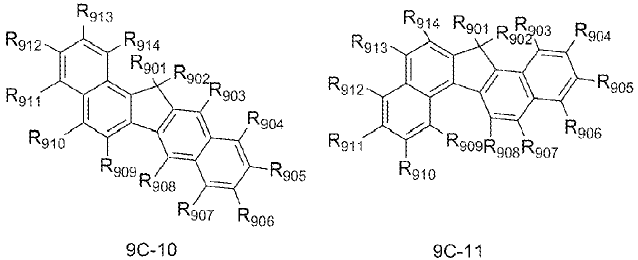

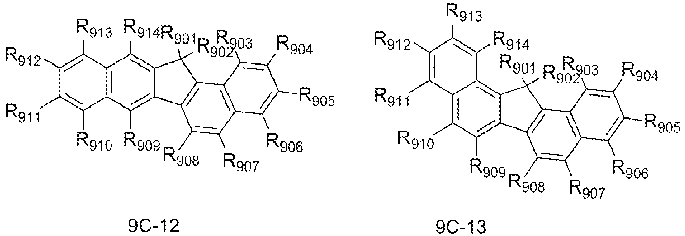

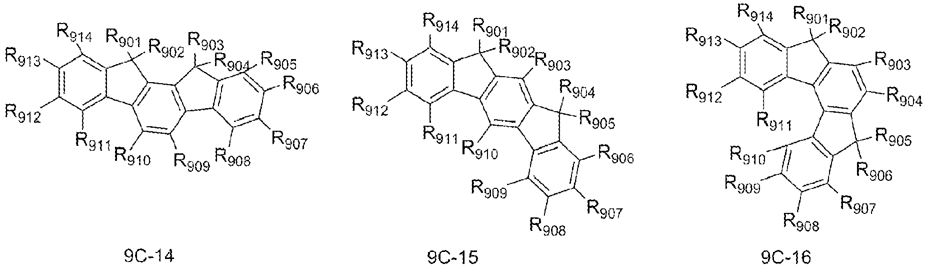

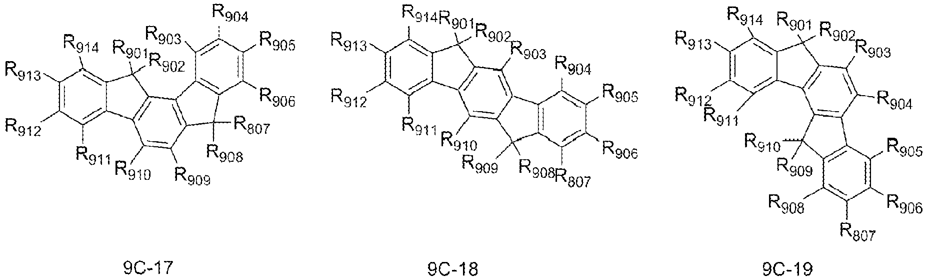

In Formulae 8B-1 to 8B-19, 8C-1 to 8C-19, 9B-1 to 9B-19, and 9C-1 to 9C-19, descriptions of R₈₀₁ to R₈₁₆ may each be independently as referred to in the descriptions provided above, and descriptions of R₉₀₁ to R₉₁₆ may each be independently as referred to in the descriptions provided above.

For example, the first host may be selected from Compounds HT-18, HT-34, HT-45, and HT-50 below, and the second host may be selected from Compounds ET-8, ET-61, and ET-73 below, but the first host and the second host are not limited thereto:

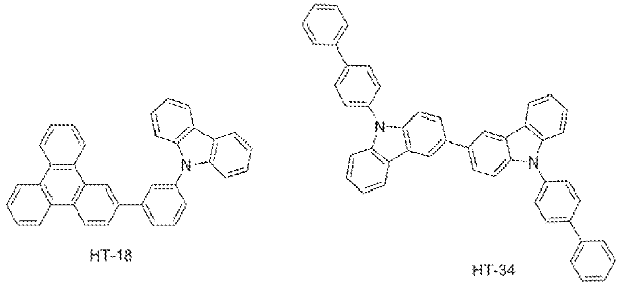

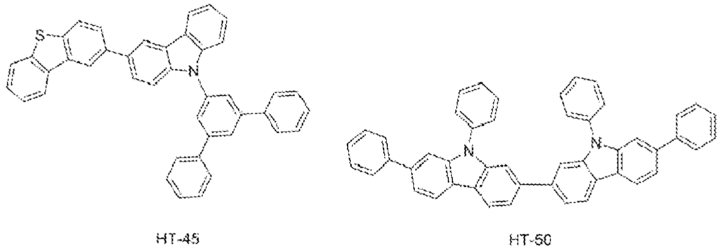

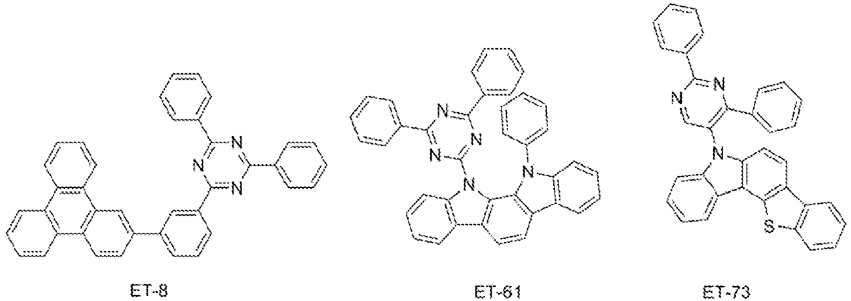

For example, the first host and the second host may each be independently selected from Compounds H-1a to H-12a bewow, but the first host and the second host are not limited thereto:

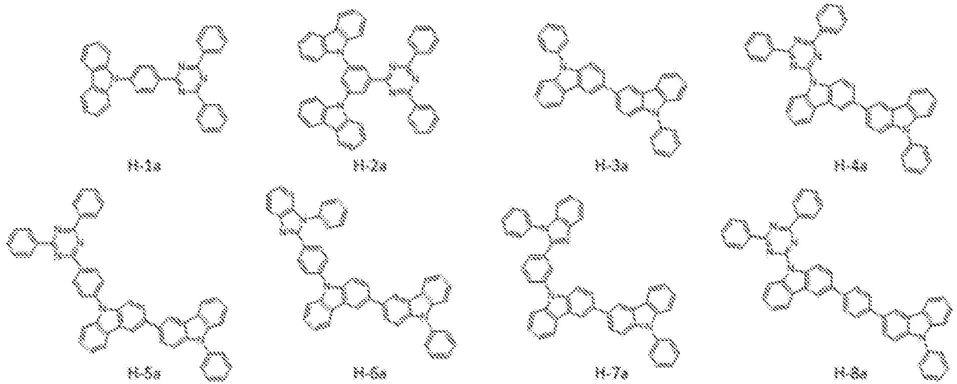

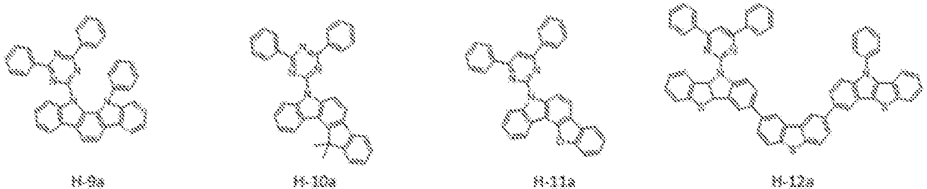

For example, the first host may be selected from Compounds H-1 a to H-12a below, and the second host may be selected from Compounds H-1 b to H-11 b below, but the first host and the second host are not limited thereto:

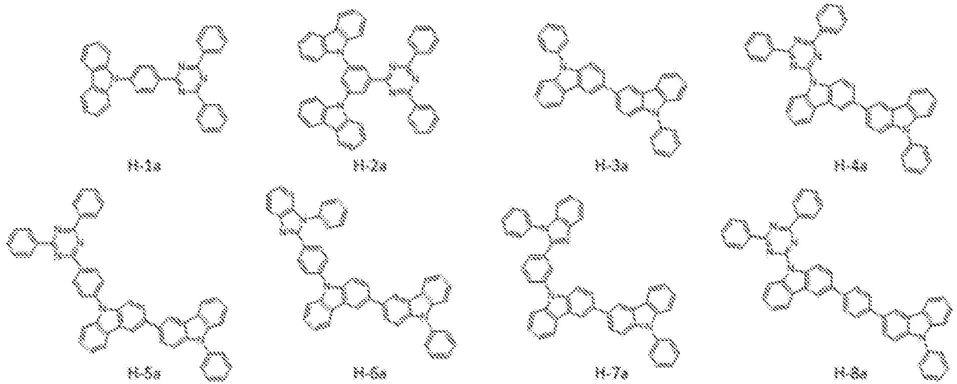

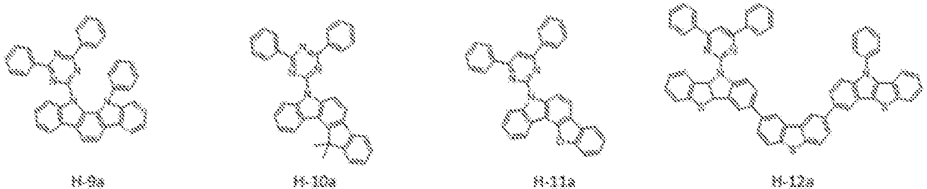

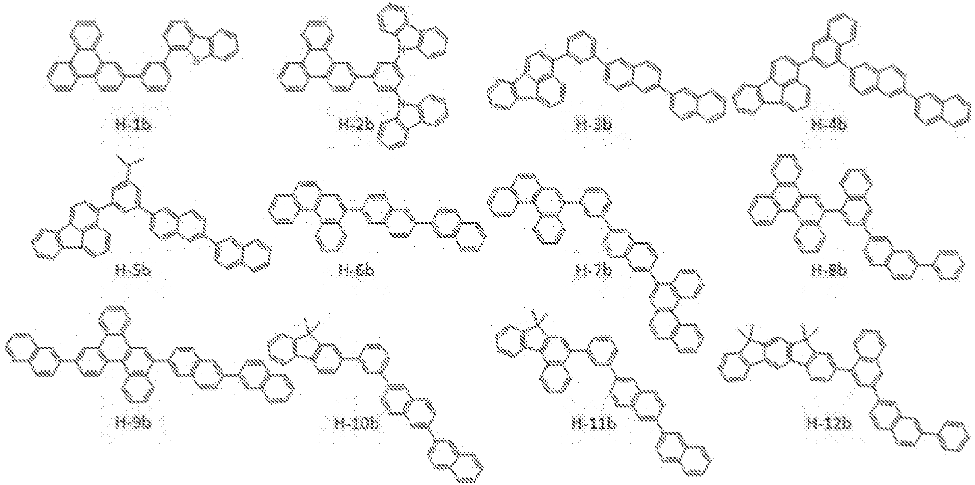

For example, the first host and and the second host may each be independently selected from Compounds H-1 b to H-11 b below, but the first host and and the second host are not limited thereto:

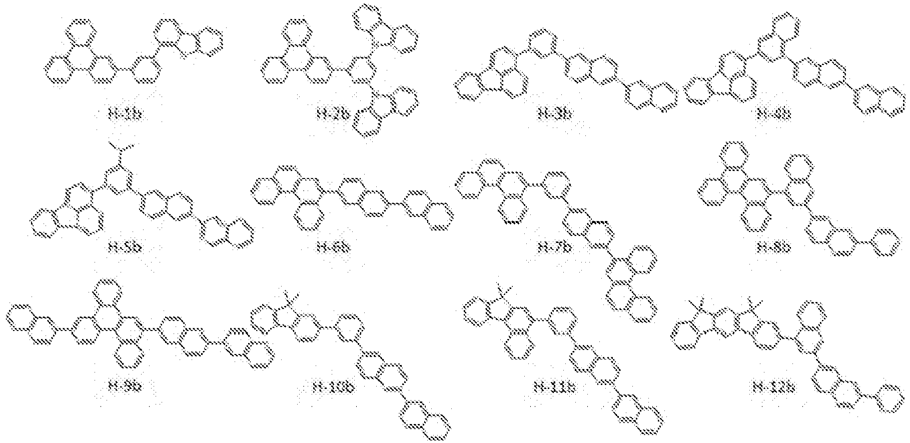

For example, one of the first host and the second host may have a triplet energy gap of 2.1 eV or more, but the first host and the second host are not limited thereto. When one of the first host and the second host has a triplet energy gap of 2.1 eV or more, one of the first host and the second host may have an excited state of triplet excitons of the EML in an efficient manner.

One of factors that influence efficiency and lifespan of organic light-emitting devices the most includes the balance between the electrons and the holes in the EML. Furthermore, it is also important to widely and evenly distribute emission regions in the EML emission layer over the HTL and the ETL. In this regard, the EML including the first host and the second host that are different from each other may be used.

In particularly, the first host may include a hole-transporting group, and the second host may include an electron-transporting group, such that the electrons and the holes in the EML may be balanced.

A weight ratio of the first host and the second host may be in a range of about 1:9 to about 9:1. For example, the weight ratio of the first host and the second host may be in a range of about 2:8 to about 8:2. For example, the weight ratio of the first host and the second host may be in a range of about 3:7 to about 7:3. For example, the weight ratio of the first host and the second host may be about 5:5.

Alternatively, a volume ratio of the first host and the second host may be in a range of about 1:9 to about 9:1. For example, the volume ratio of the first host and the second host may be in a range of about 2:8 to about 8:2. For example, the volume ratio of the first host and the second host may be in a range of about 3:7 to about 7:3. For example, the volume ratio of the first host and the second host may be about 5:5.

When the first host includes a hole-transporting group and an amount of the first host is 5 parts by weight or greater, the organic light-emitting device including the first host may have improve lifespan, but increased driving voltage. Thus, in consideration of the balance of the carriers in the organic light-emitting device, an optimal weight ratio of the carriers needs to be selected.

For example, when the organic light-emitting device includes the second host having a relatively strong electron-transporting group (e.g., a triazine) and a large amount of the first host, which does not include an electron-transporting group, the organic light-emitting device may have excellent efficiency and lifespan.

Alternatively, when when the organic light-emitting device includes the second host having a relatively weak electron-transporting group (e.g., a pyridine or a pyrimidine) and a small amount of the first host, which does not include an electron-transporting group, the organic light-emitting device may also have excellent efficiency and lifespan.

As such, the weight ratio of the first host and the second host may vary depending upon the electric characteristics and the balance thereof in the organic light-emitting device.

Hereinafter, an exemplary embodiment in which an EML of an organic light-emitting includes the Host I will be described (Embodiment 3).

In this embodiment, the emission layer may include a Host I, and the Host I may be represented by Formula 11:

Ar₁₁₁⁅(L₁₁₁)ₐ₁₁₁-(R₁₁₁)_{b111}]ₙ₁₁₁

In Formula 11, Ar₁₁₁ may be selected from a substituted or unsubstituted C₄-C₃₀ pyrrolidine-based core and a substituted or unsubstituted C₇-C₃₀ condensed polycyclic-based core,
at least one substituent of the substituted C₄-C₃₀ pyrrolidine-based core and the substituted C₇-C₃₀ condensed polycyclic-based core may be selected from:
a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), and -B(Q₁₆)(Q₁₇);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group,
and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), and -B(Q₂₆)(Q₂₇); and
-N(Q₃₁)(Q₃₂), -Si(Q₃₃)(Q₃₄)(Q₃₅), and -B(Q₃₆)(Q₃₇),
wherein Q₁ to Q₇, Q₁₁ to Q₁₇, Q₂₁ to Q₂₇, and Q₃₁ to Q₃₇ may each be independently selected from a hydrogen, a C₁-C₆₀ alkyl group, a C₁-C₆₀ alkoxy group, a C₆-C₆₀ aryl group, a C₁-C₆₀ heteroaryl group, a monovalent non-aromatic condensed polycyclic group, and a monovalent non-aromatic condensed heteropolycyclic group.

For example, in Formula 11, Ar₁₁₁ may be a group represented by one of Formulae 10-1 to 10-3, but Ar₁₁₁ is not limited thereto:

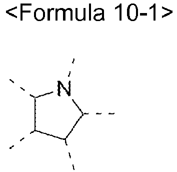

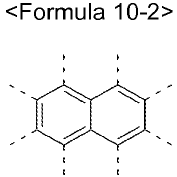

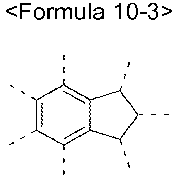

In Formulae 10-1 to 10-3, the line '- - -' indicates a covalent bond with an adjacent atom.

In another exemplary embodiment, in Formula 11, Ar₁₁₁ may be a group represented by one of Formulae 12A-1 to 12A-4, 12B-1 to 12B-19, and 12C-1 to 12C-19, but Ar₁₁₁ is not limited thereto:

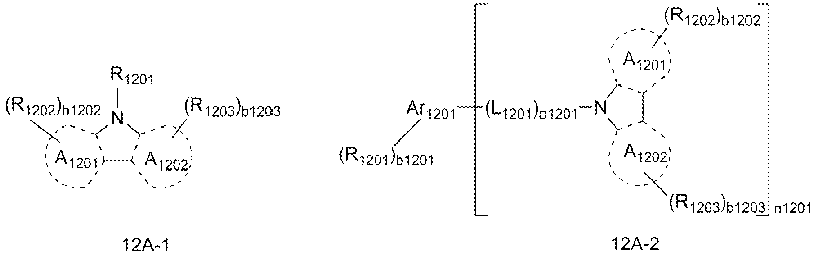

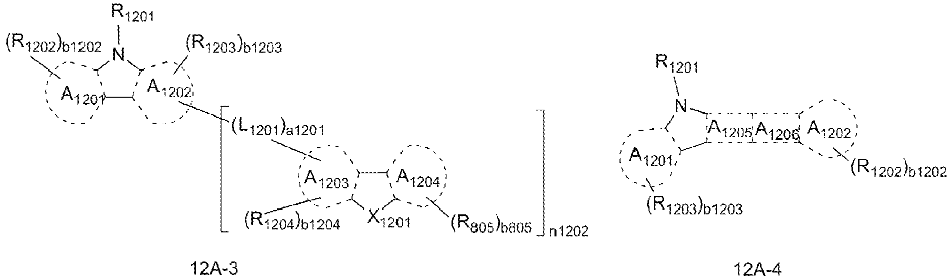

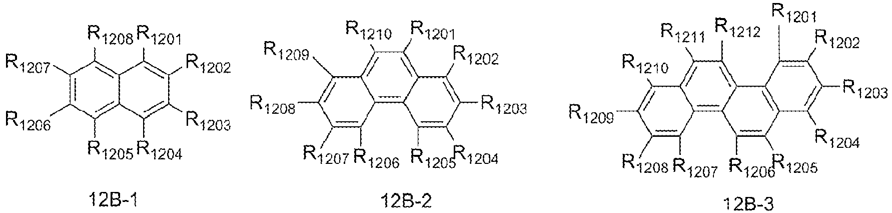

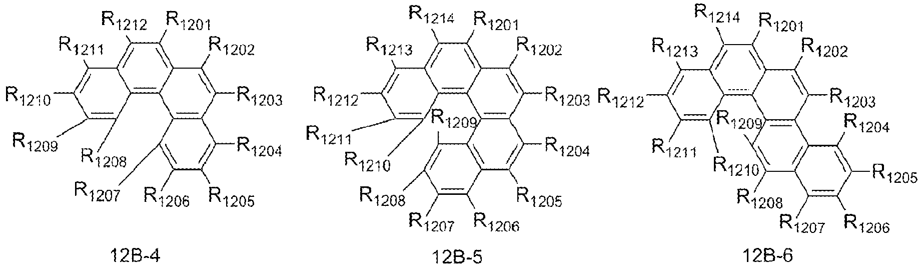

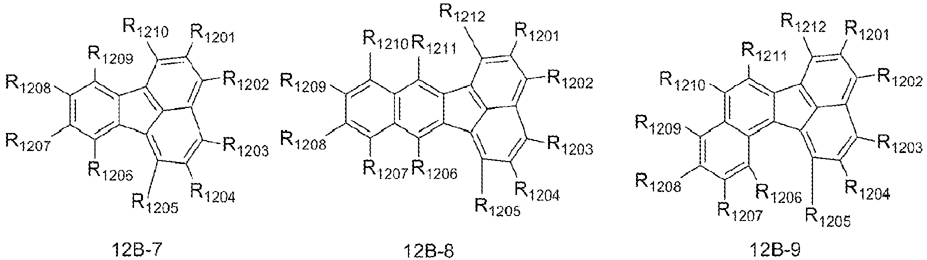

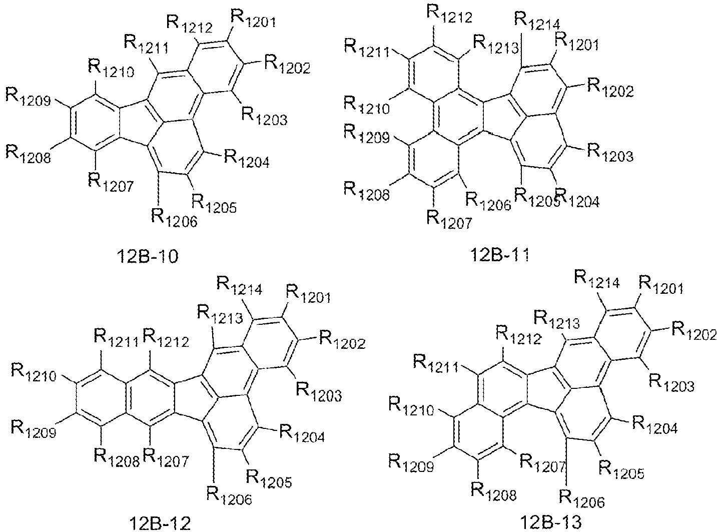

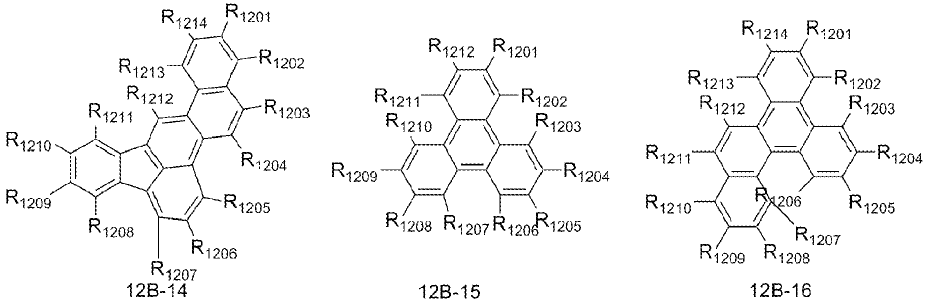

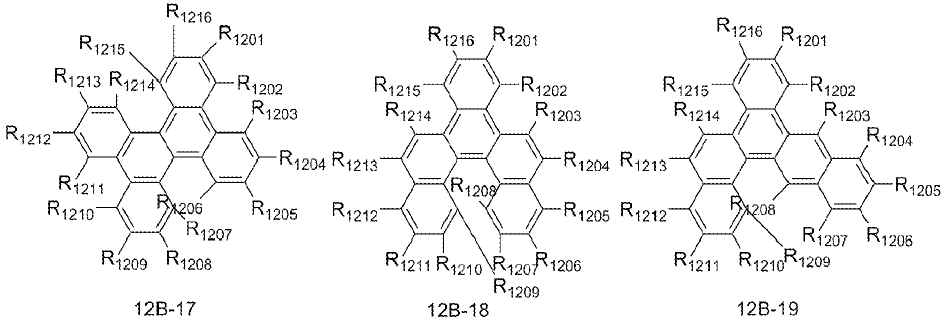

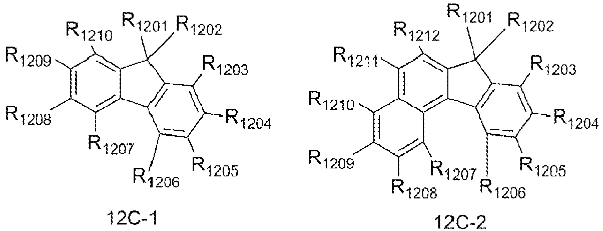

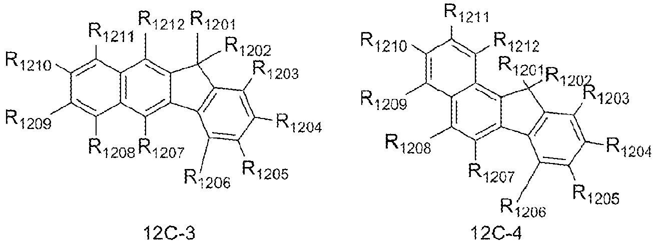

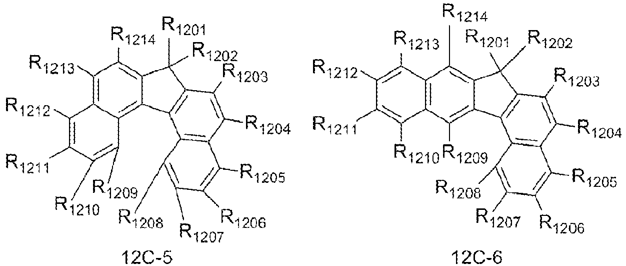

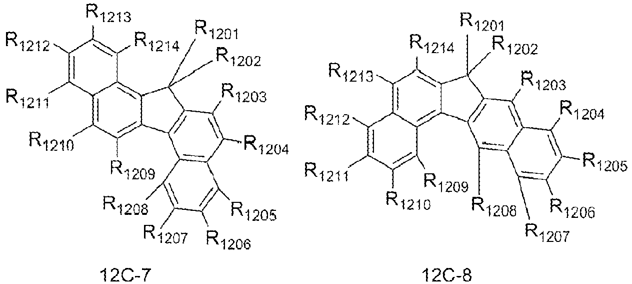

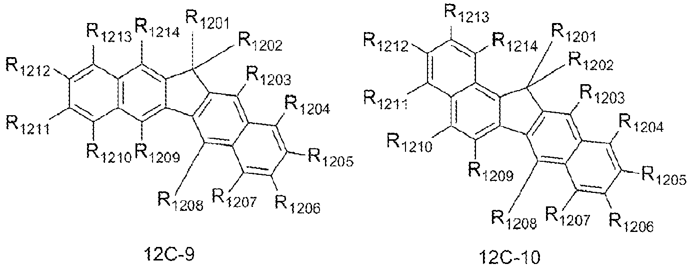

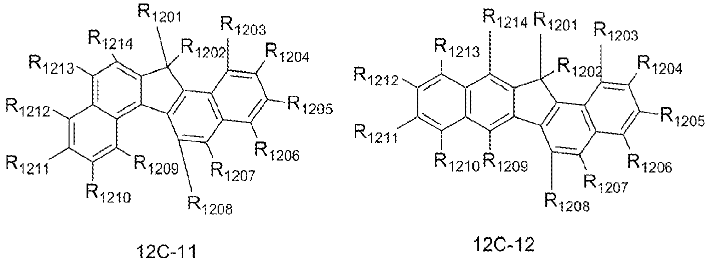

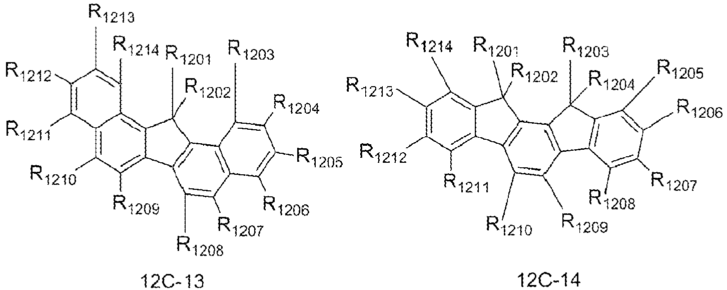

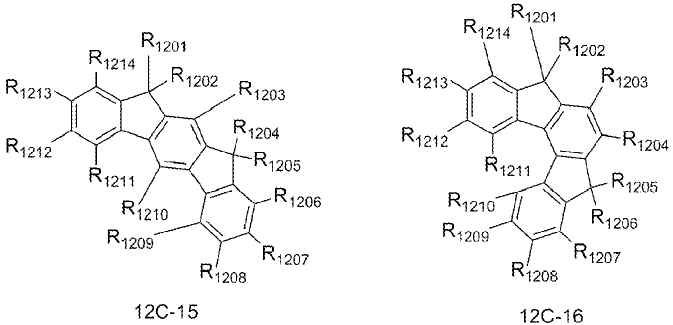

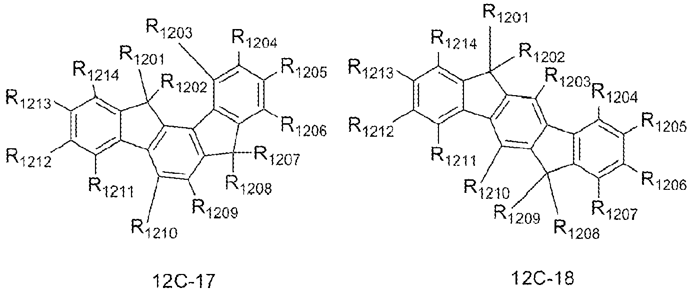

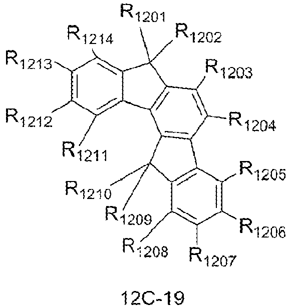

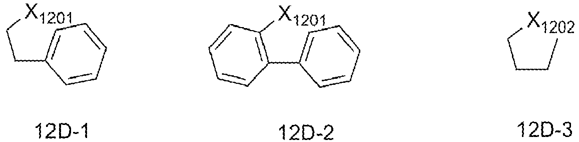

In Formulae above,
Ar₁₂₀₁ may be selected from a substituted or unsubstituted C₃-C₁₀ cycloalkane group, a substituted or unsubstituted C₁-C₁₀ heterocycloalkane group, a substituted or unsubstituted C₃-C₁₀ cycloalkene group, a substituted or unsubstituted C₁-C₁₀ heterocycloalkene group, a substituted or unsubstituted C₆-C₆₀ arene group, a substituted or unsubstituted C₁-C₆₀ heteroarene group, a substituted or unsubstituted non-aromatic condensed polycyclic group, and a substituted or unsubstituted non-aromatic condensed heteropolycyclic group,
L₁₂₀₁ may be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
a1201 may be selected from 0, 1, 2, and 3,
A₁₂₀₁ to A₁₂₀₄ may each be independently selected from a benzene, a naphthalene, a pyridine, a pyrimidine, a pyrazine, a quinoline, an isoquinoline, a 2,6-naphthyridine, a 1,8-naphthyridine, a 1,5-naphthyridine, a 1,6-naphthyridine, a 1,7-naphthyridine, a 2,7-naphthyridine, a quinoxaline, a phthalazine, a quinazoline, a group represented by Formula 12D-1 above, and a group represented by Formula 12D-2 above,
A₁₂₀₅ and A₁₂₀₅ may each be independently selected from a benzene and a naphthalene,
A₁₂₀₆ may be a group represented by Formula 12D-3 above,
X₁₂₀₁ and X₁₂₀₂ may each be independently selected from N(R₁₂₀₆), O, S, C(R₁₂₀₆)(R₁₂₀₇), Si(R₁₂₀₆)(R₁₂₀₇), B(R₁₂₀₆), P(R₁₂₀₆), and P(=O)(R₁₂₀₆),
R₁₂₀₁ to R₁₂₁₆ may each be independently selected from *-[(L₁₁₁)ₐ₁₁₁-(R₁₁₁)_{b111}], a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, wherein at least one of R₁₂₀₁ to R₁₂₁₆ is selected from *-[(L₁₁₁)ₐ₁₁₁-(R₁₁₁)_{b111}],
b1201 to b1205 may each be independently selected from 1, 2, 3, and 4,
n1201 may be selected from 2, 3, and 4,
n1202 may be selected from 1, 2, and 3,
at least one substituent of the substituted C₃-C₁₀ cycloalkane group, the substituted C₁-C₁₀ heterocycloalkane group, the substituted C₃-C₁₀ cycloalkene group, the substituted C₁-C₁₀ heterocycloalkene group, the substituted C₆-C₆₀ arene group, the substituted C₁-C₆₀ heteroarene group, the substituted non-aromatic condensed polycyclic group, the substituted non-aromatic condensed heteropolycyclic group, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the subtstituted divalent non-aromatic condensed polycyclic group, the subtstituted divalent non-aromatic condensed heteropolycyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted or unsubstituted monovalent non-aromatic condensed heteropolycyclic group may be selected from:
a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), and -B(Q₁₆)(Q₁₇);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), and -B(Q₂₆)(Q₂₇); and
-N(Q₃₁)(Q₃₂), -Si(Q₃₃)(Q₃₄)(Q₃₅), and -B(Q₃₆)(Q₃₇),
wherein Q₁₁ to Q₁₇, Q₂₁ to Q₂₇, and Q₃₁ to Q₃₇ may each be independently selected from a hydrogen, a C₁-C₆₀ alkyl group, a C₁-C₆₀ alkoxy group, a C₆-C₆₀ aryl group, a C₁-C₆₀ heteroaryl group, a monovalent non-aromatic condensed polycyclic group, and a monovalent non-aromatic condensed heteropolycyclic group.

In Formula 11, L₁₁₁ may be selected from:
a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group, and
at least one substituent of the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the subtstituted divalent non-aromatic condensed polycyclic group, and the subtstituted divalent non-aromatic condensed heteropolycyclic group may be selected from:
   a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
   a C₁-C₆₀ alkyl group, a C₂-C₆₀ alkenyl group, a C₂-C₆₀ alkynyl group, and a C₁-C₆₀ alkoxy group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), and -B(Q₁₆)(Q₁₇);
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), and -B(Q₂₆)(Q₂₇); and
   -N(Q₃₁)(Q₃₂), -Si(Q₃₃)(Q₃₄)(Q₃₅), and -B(Q₃₆)(Q₃₇),
   wherein Q₁ to Q₇, Q₁₁ to Q₁₇, Q₂₁ to Q₂₇, and Q₃₁ to Q₃₇ may each be independently selected from a hydrogen, a C₁-C₆₀ alkyl group, a C₁-C₆₀ alkoxy group, a C₆-C₆₀ aryl group, a C₁-C₆₀ heteroaryl group, a monovalent non-aromatic condensed polycyclic group, and a monovalent non-aromatic condensed heteropolycyclic group.

For example, in Formula 11, L₁₁₁ may be selected from a phenylene group, a pentalenylene group, an indenylene group, a naphthylene group, an azulenylene group, a heptalenylene group, an indacenylene group, an acenaphthylene group, a fluorenylene group, a spiro-fluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a pyrrolylene group, a thiophenylene group, a furanylene group, an imidazolylene group, a pyrazolylene group, a thiazolylene group, an isothiazolylene group, an oxazolylene group, an isoxazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzimidazolylene group, a benzofuranylene group, a benzothiophenylene group, an isobenzothiazolylene group, a benzoxazolylene group, an isobenzoxazolylene group, a triazolylene group, a tetrazolylene group, an oxadiazolylene group, a triazinylene group, a dibenzofuranylene group, a dibenzothiophenylene group, a benzocarbazolylene group, and a dibenzocarbazolylene group; and
a phenylene group, a pentalenylene group, an indenylene group, a naphthylene group, an azulenylene group, a heptalenylene group, an indacenylene group, an acenaphthylene group, a fluorenylene group, a spiro-fluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a pyrrolylene group, a thiophenylene group, a furanylene group, an imidazolylene group, a pyrazolylene group, a thiazolylene group, an isothiazolylene group, an oxazolylene group, an isoxazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzimidazolylene group, a benzofuranylene group, a benzothiophenylene group, an isobenzothiazolylene group, a benzoxazolylene group, an isobenzoxazolylene group, a triazolylene group, a tetrazolylene group, an oxadiazolylene group, a triazinylene group, a dibenzofuranylene group, a dibenzothiophenylene group, a benzocarbazolylene group, and a dibenzocarbazolylene group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, a thiadiazolyl group, and an imidazopyridinyl group.

In another exemplary embodiment, in Formula 11, L₁₁₁ may be a group represented by one of Formulae 3-1 to 3-18, but L₁₁₁ is ot limited thereto:

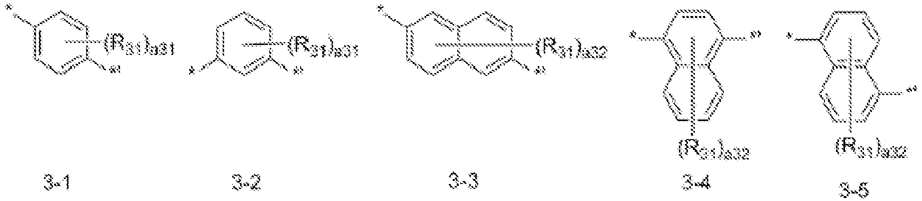

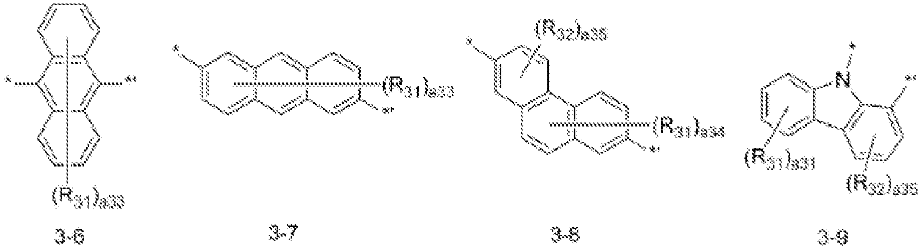

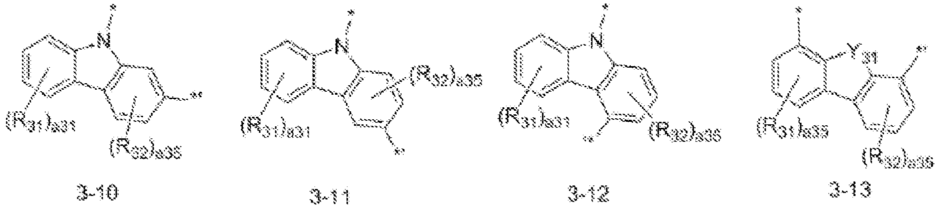

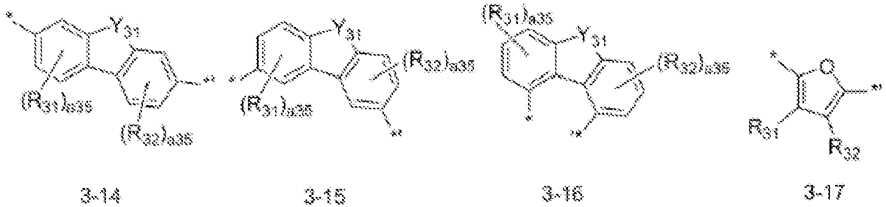

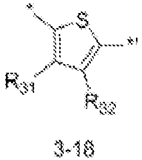

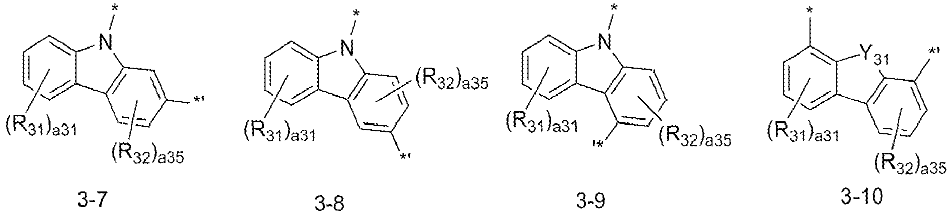

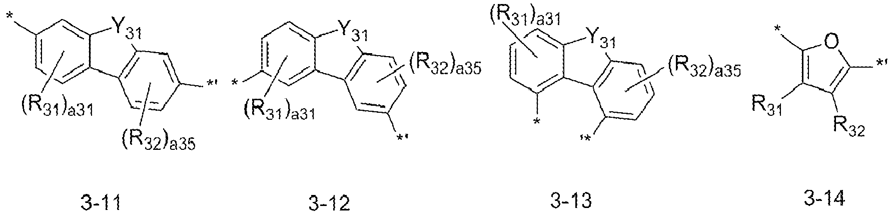

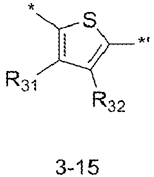

In Formulae 3-1 to 3-18,
Y₃₁ may be selected from C(R₃₃)(R₃₄), N(R₃₃), O, S, and Si(R₃₃)(R₃₄),
R₃₁ to R₃₄ may each be independently selected from a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group,
a31 may be selected from 1, 2, 3, and 4,
a32 may be selected from 1, 2, 3, 4, 5, and 6,
a33 may be selected from 1, 2, 3, 4, 5, 6, 7, and 8,
a34 may be selected from 1, 2, 3, 4, and 5,
a35 may be selected from selected from 1, 2, and 3, and
* and *' may each independently indicate a binding site to an adjacent atom.

In Formula 11, a111 may be selected from 0, 1, 2, and 3.

In Formula 11, R₁₁₁ may be selected from:
a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, wherein at least one R₁₁₁ may be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, and
at least one substituent of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group may be selected from:
a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), and -B(Q₁₆)(Q₁₇);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), and -B(Q₂₆)(Q₂₇); and
-N(Q₃₁)(Q₃₂), -Si(Q₃₃)(Q₃₄)(Q₃₅), and -B(Q₃₆)(Q₃₇),
wherein Q₁ to Q₇, Q₁₁ to Q₁₇, Q₂₁ to Q₂₇, and Q₃₁ to Q₃₇ may each be independently selected from a hydrogen, a C₁-C₆₀ alkyl group, a C₁-C₆₀ alkoxy group, a C₆-C₆₀ aryl group, a C₁-C₆₀ heteroaryl group, a monovalent non-aromatic condensed polycyclic group, and a monovalent non-aromatic condensed heteropolycyclic group.

For example, in Formula 11, R₁₁₁ may be selected from a phenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a carbazolyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, a benzothiazoly group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a dibenzosilolyl group, a benzocarbazolyl group, a dibenzocarbazolyl group, an imidazopyridinyl group, an imidazopyrimidinyl group, a pyridobenzofuranyl group, a pyrimidobenzofuranyl group, a pyridobenzothiophenyl group, a pyrimidobenzothiopheny group, and -N(R₅₆)(R₅₇); and
a phenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a carbazolyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, a benzothiazolyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a dibenzosilolyl group, a benzocarbazolyl group, a dibenzocarbazolyl group, an imidazopyridinyl group, an imidazopyrimidinyl group, a pyridobenzofuranyl group, a pyrimidobenzofuranyl group, a pyridobenzothiophenyl group, and a pyrimidobenzothiophenyl group, each substituted with at least one of a deuterium, -F, - CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a carbazolyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, a benzothiazolyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, -N(Q₃₁)(Q₃₂), -Si(Q₃₃)(Q₃₄)(Q₃₅), and -B(Q₃₆)(Q₃₇),
R₅₆ and R₅₇ may each be independently selected from:
a C₁-C₂₀ alkyl group, a C₆-C₂₀ aryl group, a C₁-C₂₀ heteroaryl group, a monovalent non-aromatic condensed polycyclic group, and a monovalent non-aromatic condensed heteropolycyclic group; and
a C₁-C₂₀ alkyl group, a C₆-C₂₀ aryl group, a C₁-C₂₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₆-C₂₀ aryl group, a C₁-C₂₀ heteroaryl group, a monovalent non-aromatic condensed polycyclic group, and a monovalent non-aromatic condensed heteropolycyclic group,
wherein Q₃₁ to Q₃₇ may each be independently selected from a C₁-C₆₀ alkyl group, a C₆-C₆₀ aryl group, a C₁-C₆₀ heteroaryl group, a monovalent non-aromatic condensed polycyclic group, and a monovalent non-aromatic condensed heteropolycyclic group, but embodiments are not limited thereto.

In another exemplary embodiment, in Formula 11, R₁₁₁ may be a group represented by one of Formulae 5-1 to 5-13 and 5-21 to 5-79, but R₁₁₁ is not limited thereto:

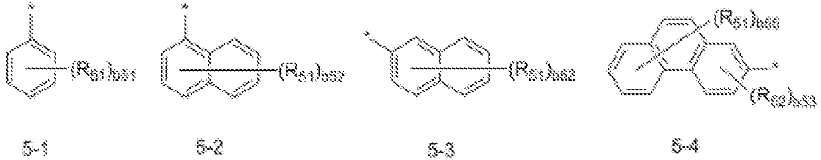

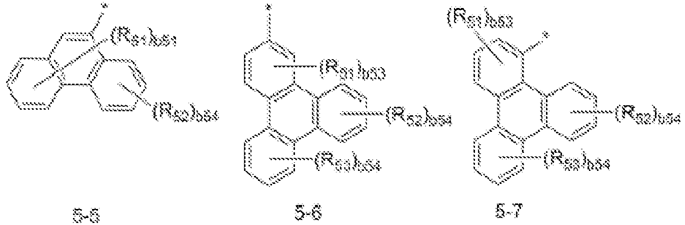

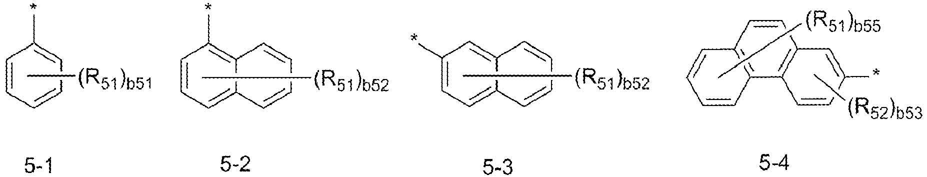

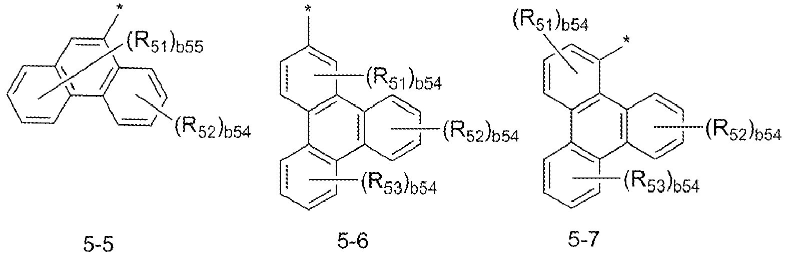

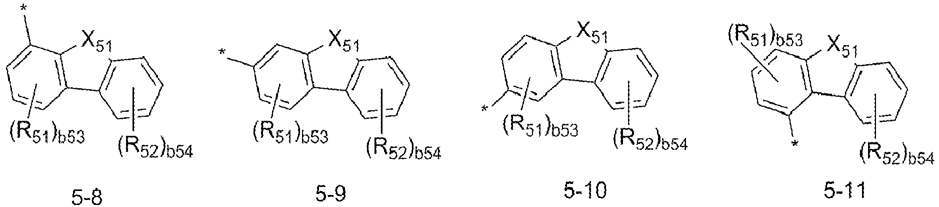

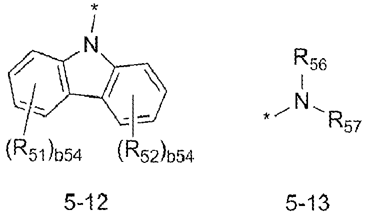

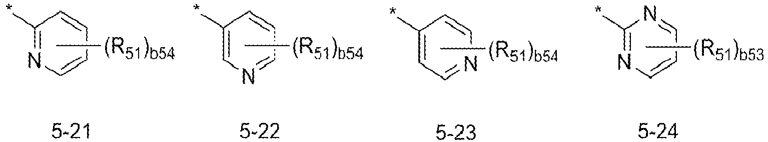

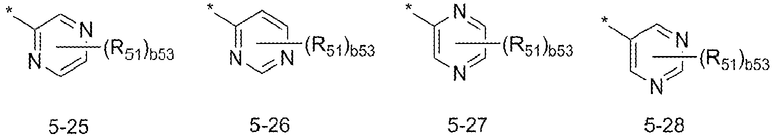

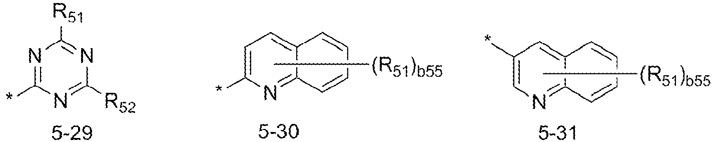

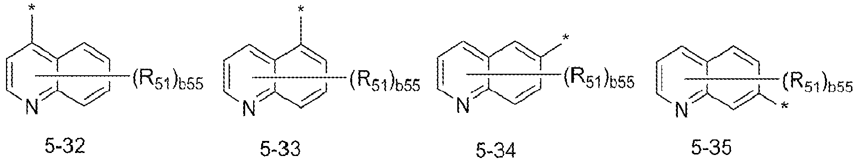

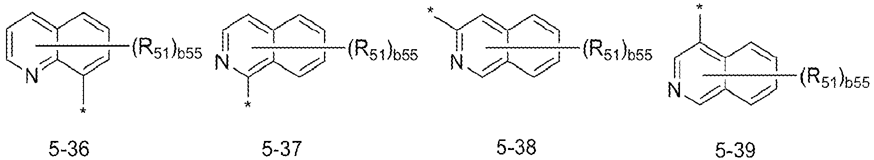

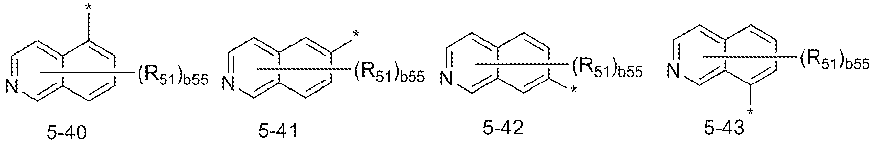

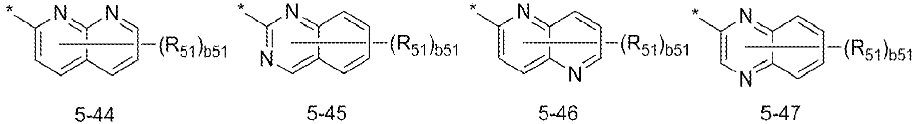

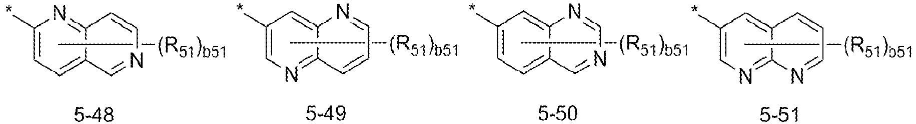

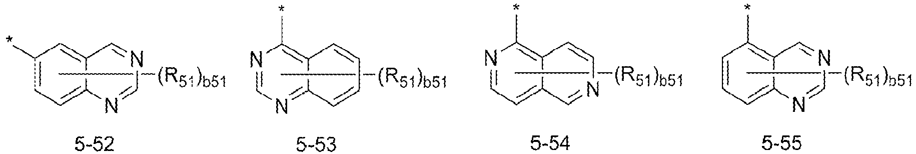

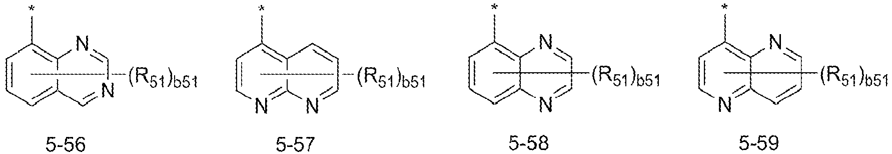

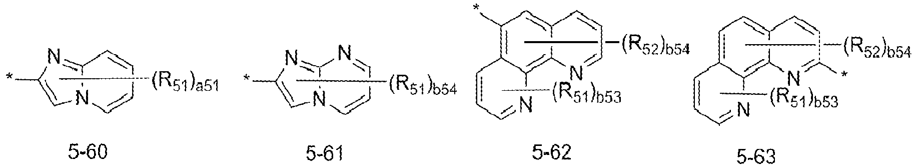

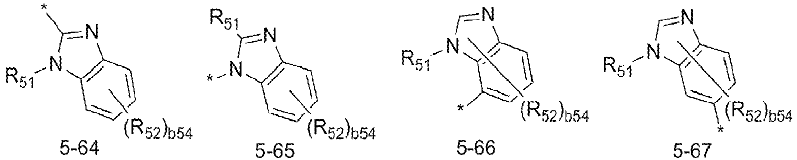

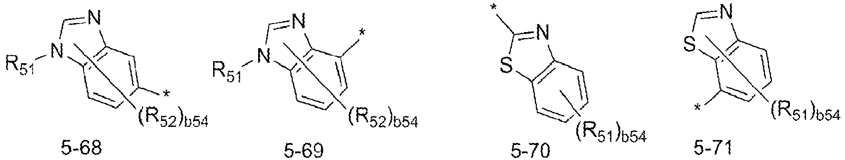

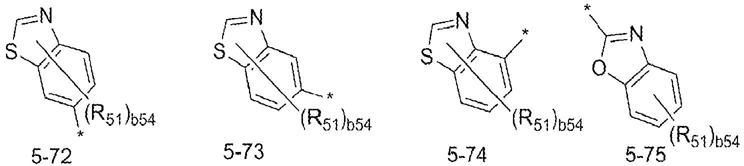

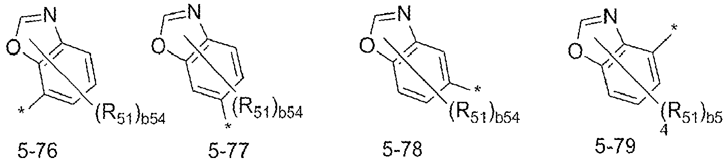

In Formulae 5-1 to 5-13 and 5-21 to 5-57,
X₅₁ may be selected from O, S, N(R₅₄), and C(R₅₄)(R₅₅),
R₅₁ to R₅₅ may each be independently selected from:
a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₆-C₂₀ aryl group, a C₁-C₂₀ heteroaryl group, a monovalent non-aromatic condensed polycyclic group, a monovalent non-aromatic condensed heteropolycyclic group, -N(Q₄₁)(Q₄₂), -Si(Q₄₃)(Q₄₄)(Q₄₅), and - B(Q₄₆)(Q₄₇); and
a C₆-C₂₀ aryl group, a C₁-C₂₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, and a C₁-C₂₀ alkoxy group,
R₅₆ and R₅₇ may each be independently selected from:
   a C₁-C₂₀ alkyl group, a C₆-C₂₀ aryl group, a C₁-C₂₀ heteroaryl group, a monovalent non-aromatic condensed polycyclic group, and a monovalent non-aromatic condensed heteropolycyclic group; and
   a C₁-C₂₀ alkyl group, a C₆-C₂₀ aryl group, a C₁-C₂₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₆-C₂₀ aryl group, a C₁-C₂₀ heteroaryl group, a monovalent non-aromatic condensed polycyclic group, and a monovalent non-aromatic condensed heteropolycyclic group,
   wherein Q₄₁ to Q₄₇ may each be independently selected from a C₁-C₂₀ alkyl group, a C₆-C₂₀ aryl group, a C₁-C₂₀ heteroaryl group, a monovalent non-aromatic condensed polycyclic group, and a monovalent non-aromatic condensed heteropolycyclic group,
   b51 may be selected from 1, 2, 3, 4, and 5,
   b52 may be selected from 1, 2, 3, 4, 5, 6, and 7,
   b53 may be selected from 1, 2, and 3,
   b54 may be selected from 1, 2, 3, and 4,
   b55 may be selected from 1, 2, 3, 4, 5, and 6, and
   * may indicate a binding site to an adjacent atom.

In another exemplary embodiment, in Formula 11, R₁₁₁ may be a group represented by one of Formulae 6-1 to 6-59 and 6-61 to 6-219, but R₁₁₁ is not limited thereto:

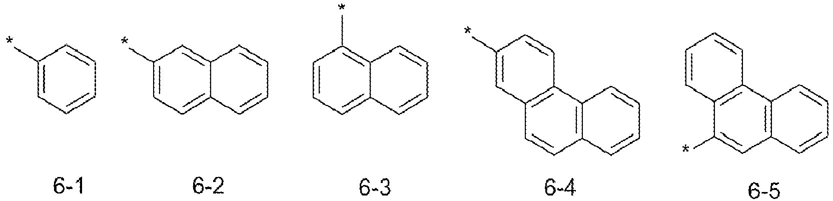

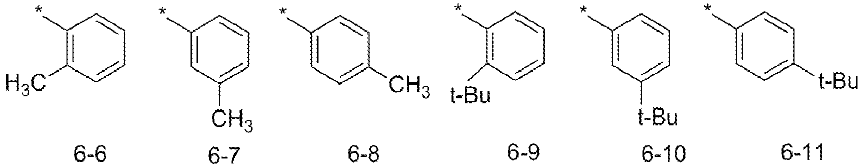

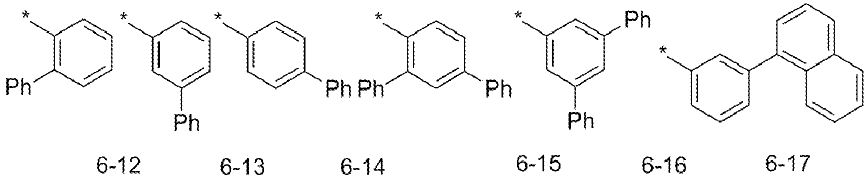

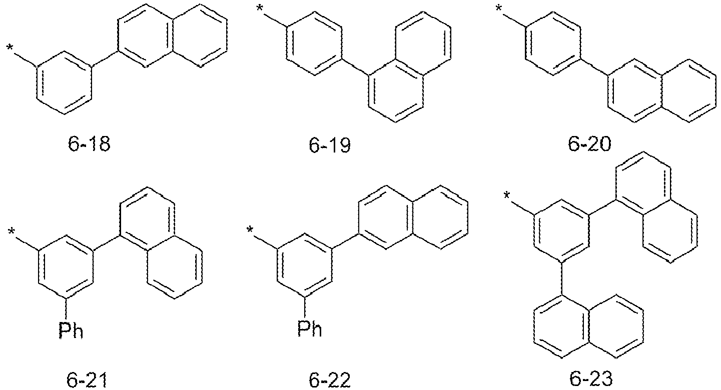

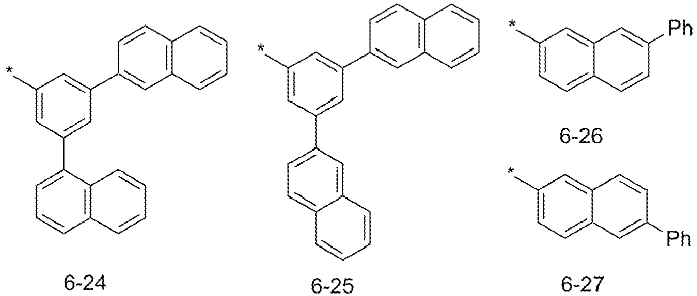

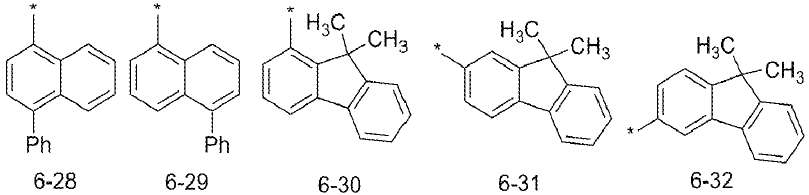

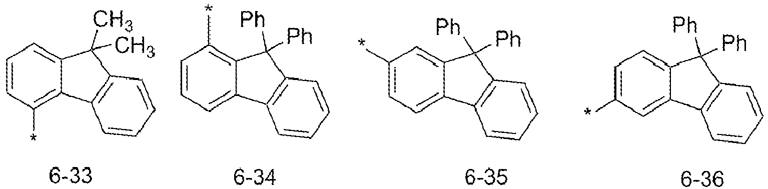

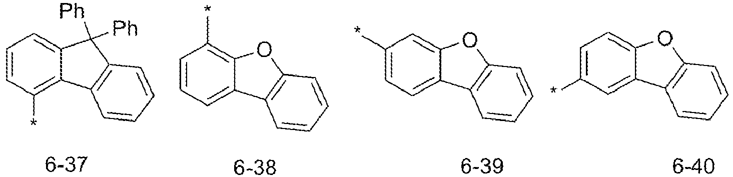

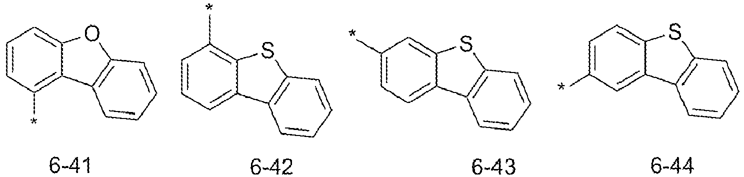

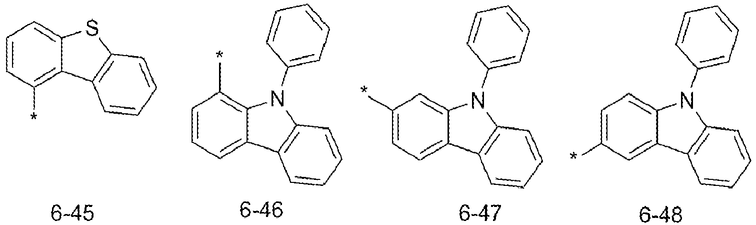

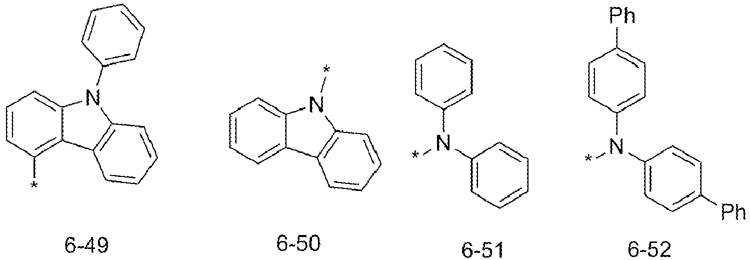

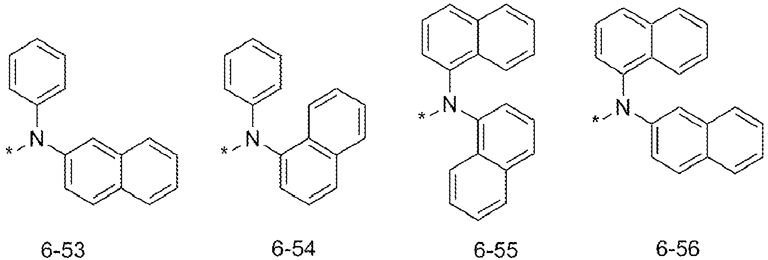

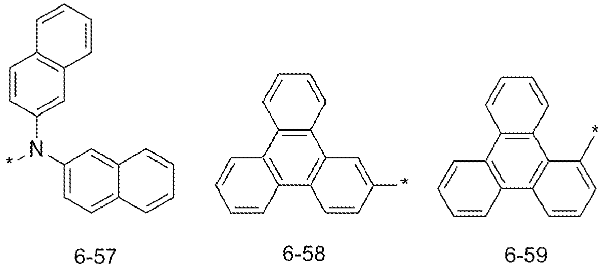

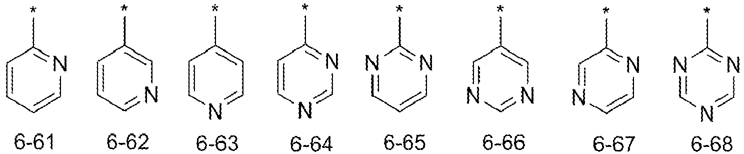

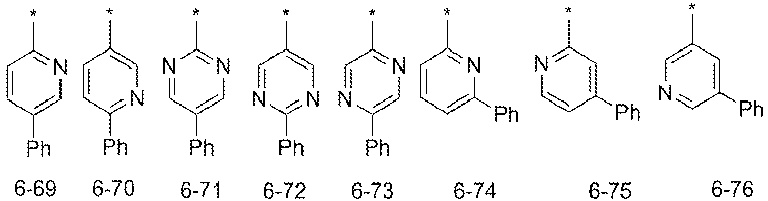

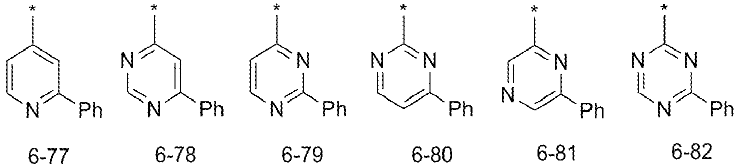

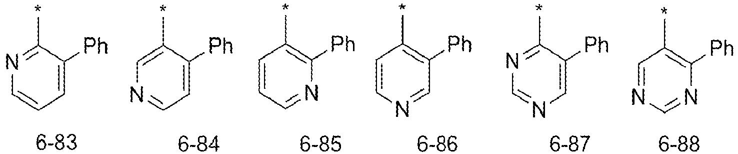

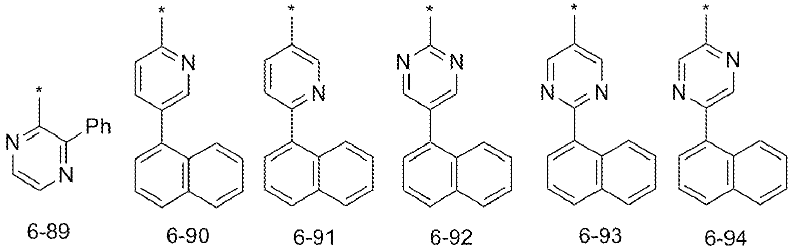

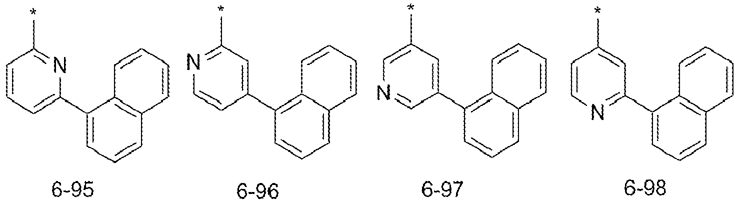

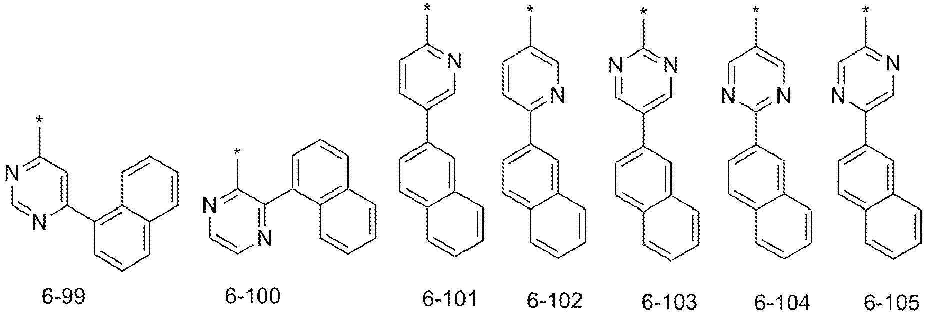

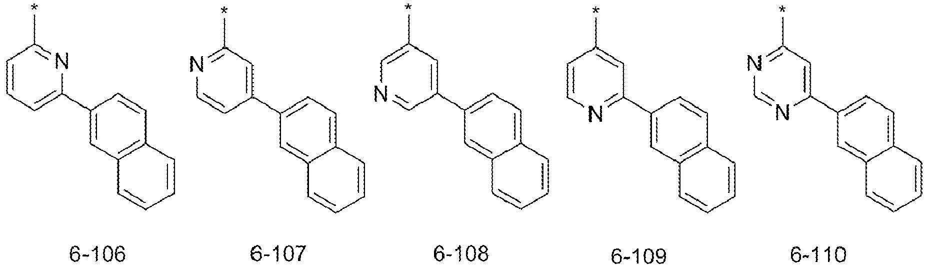

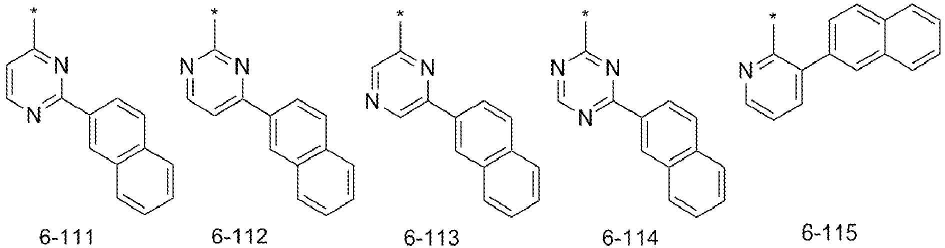

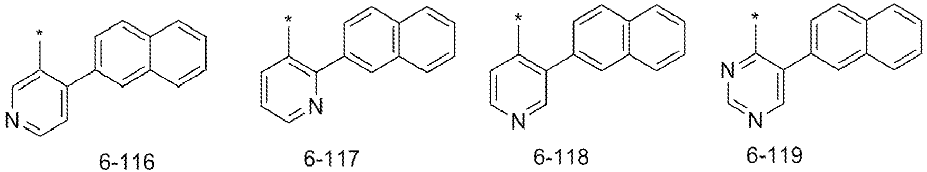

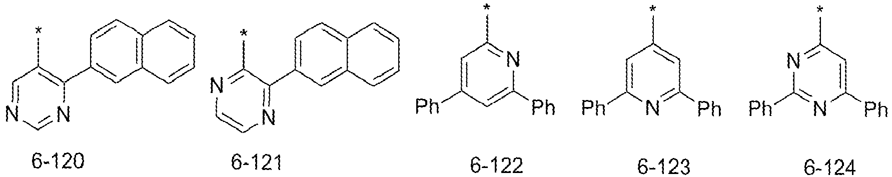

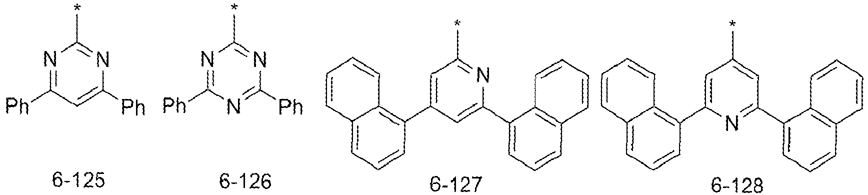

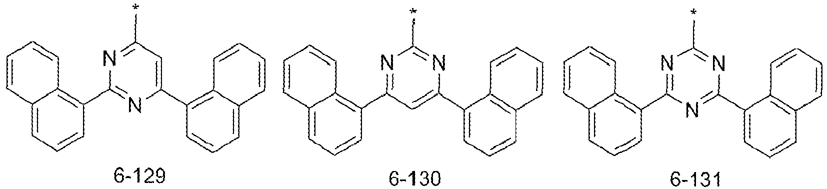

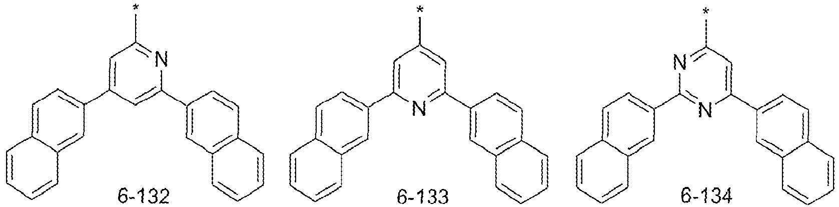

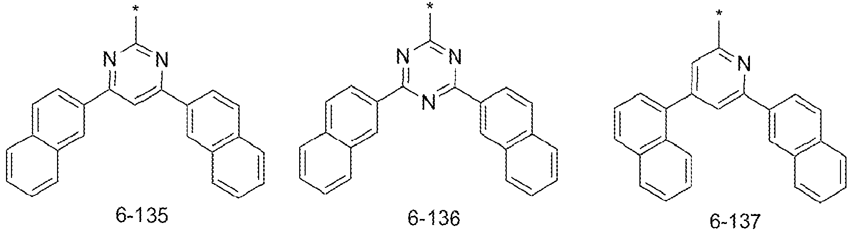

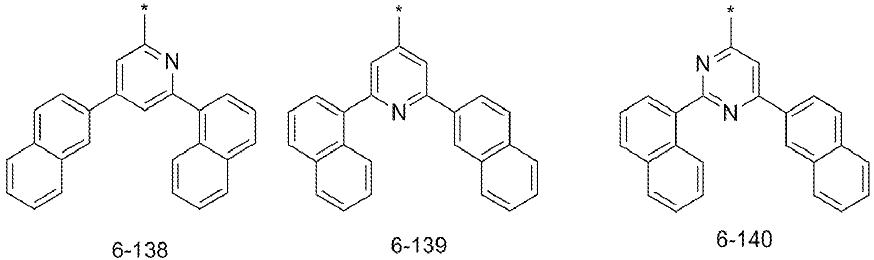

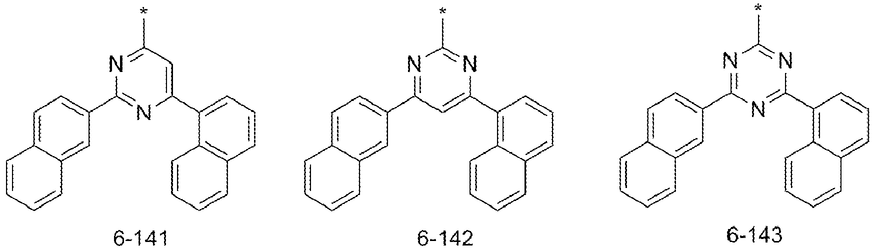

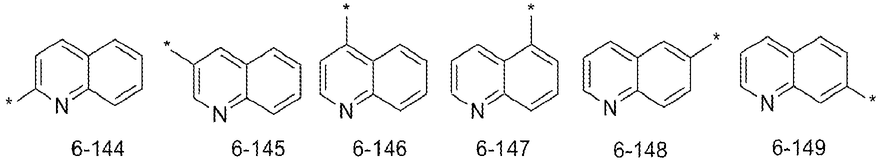

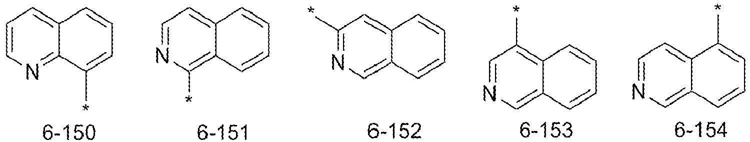

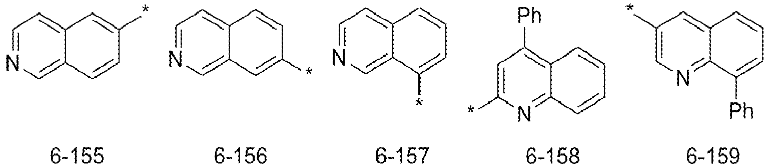

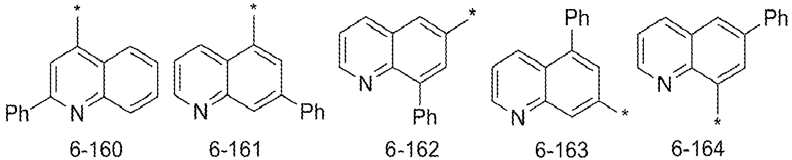

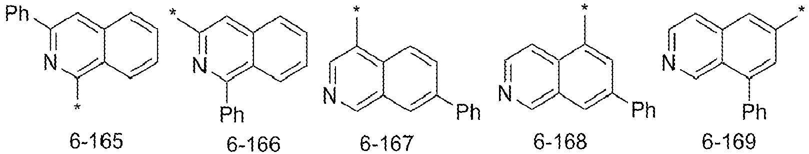

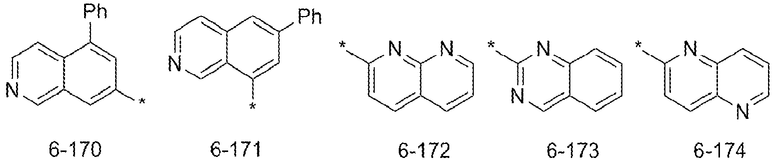

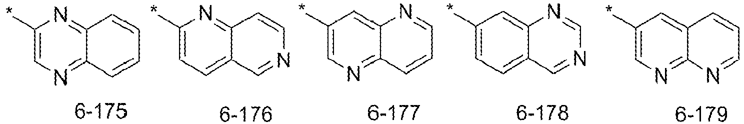

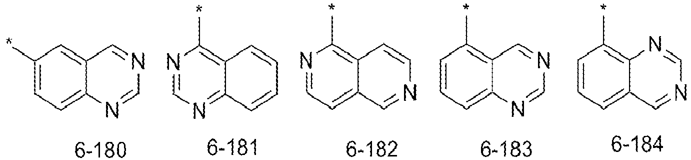

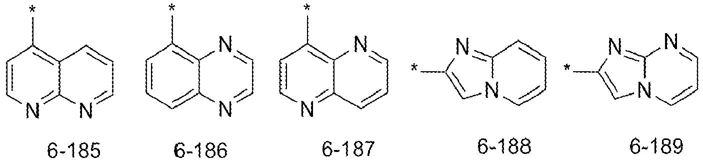

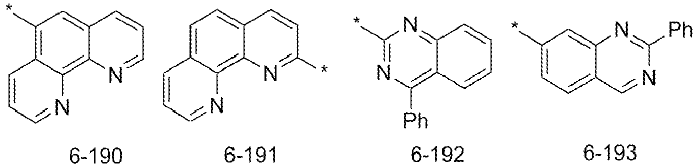

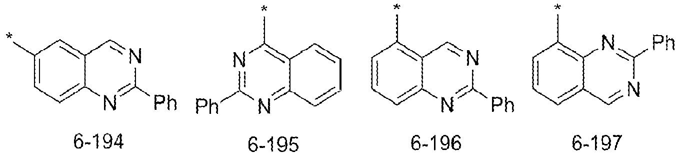

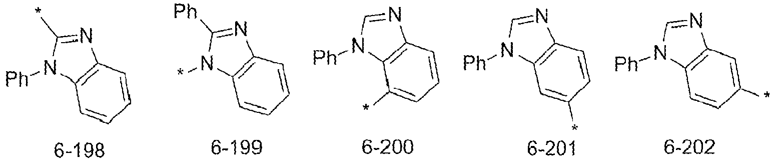

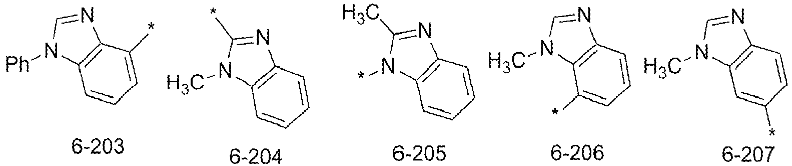

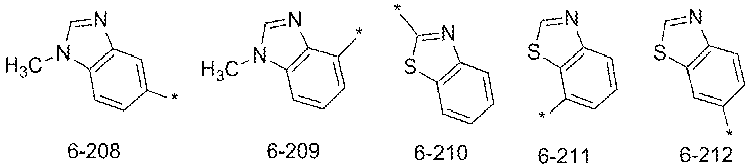

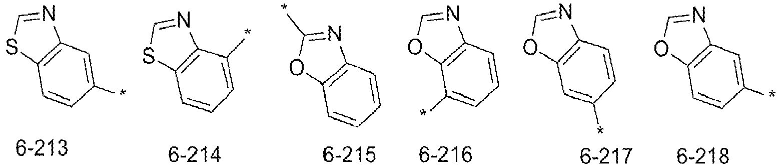

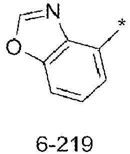

In Formulae 6-1 to 6-59 and 6-61 to 6-219,
t-Bu indicates a tert-butyl group,
Ph indicates a phenyl group, and
* indicates a binding site to an adjacent atom.

In Formula 11, b111 may be selected from 1, 2, and 3.

In Formula 11, n111 may be selected from 1, 2, 3, and 4.

For example, the Host I may be selected from compounds below, but the Host I is not limited thereto:

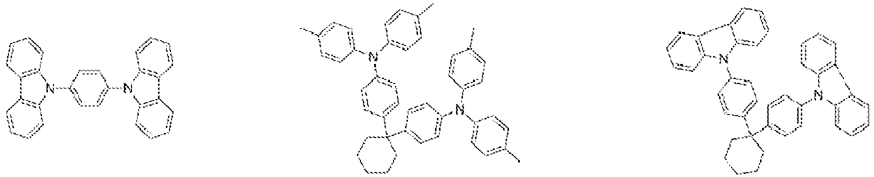

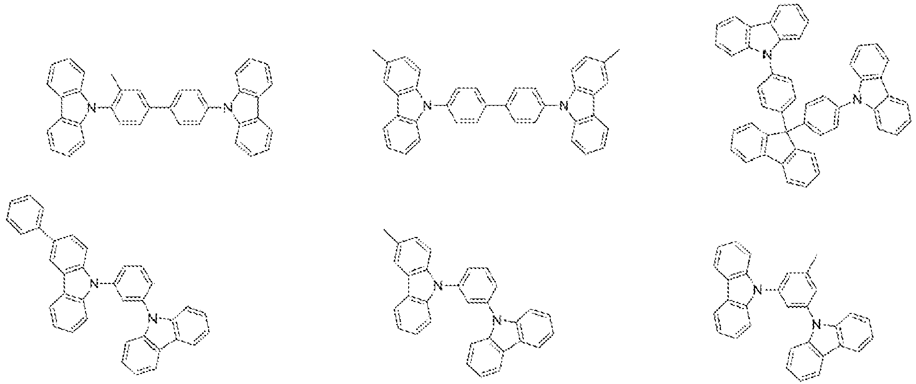

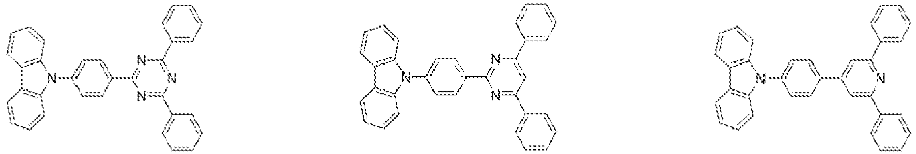

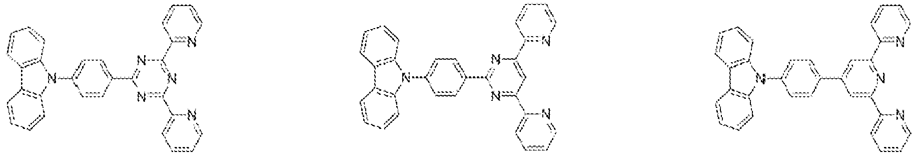

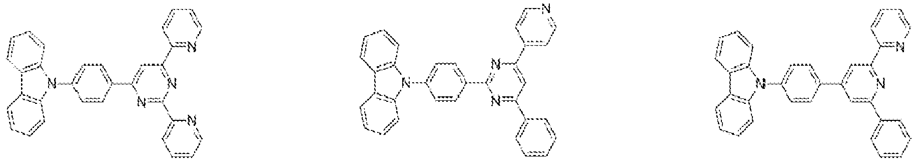

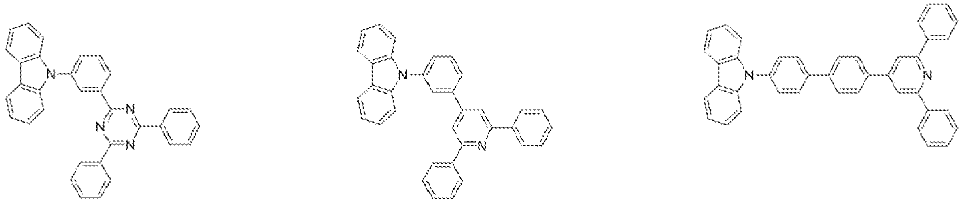

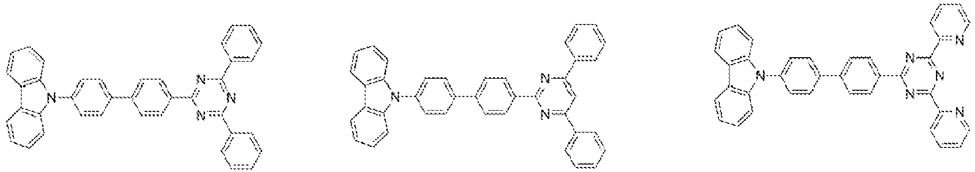

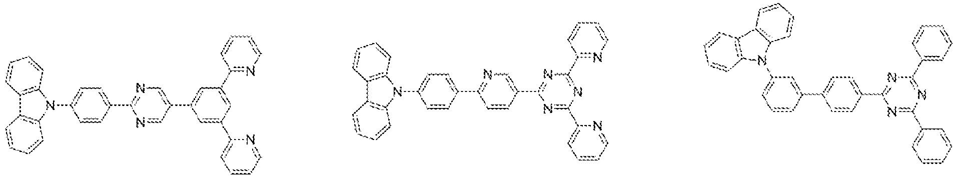

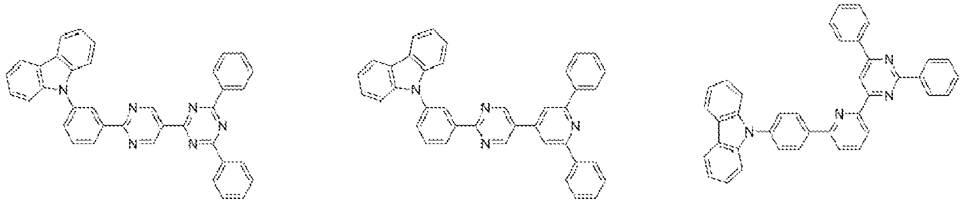

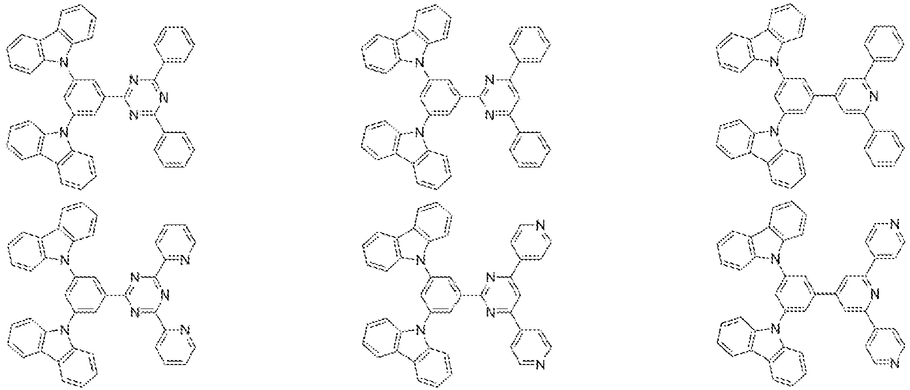

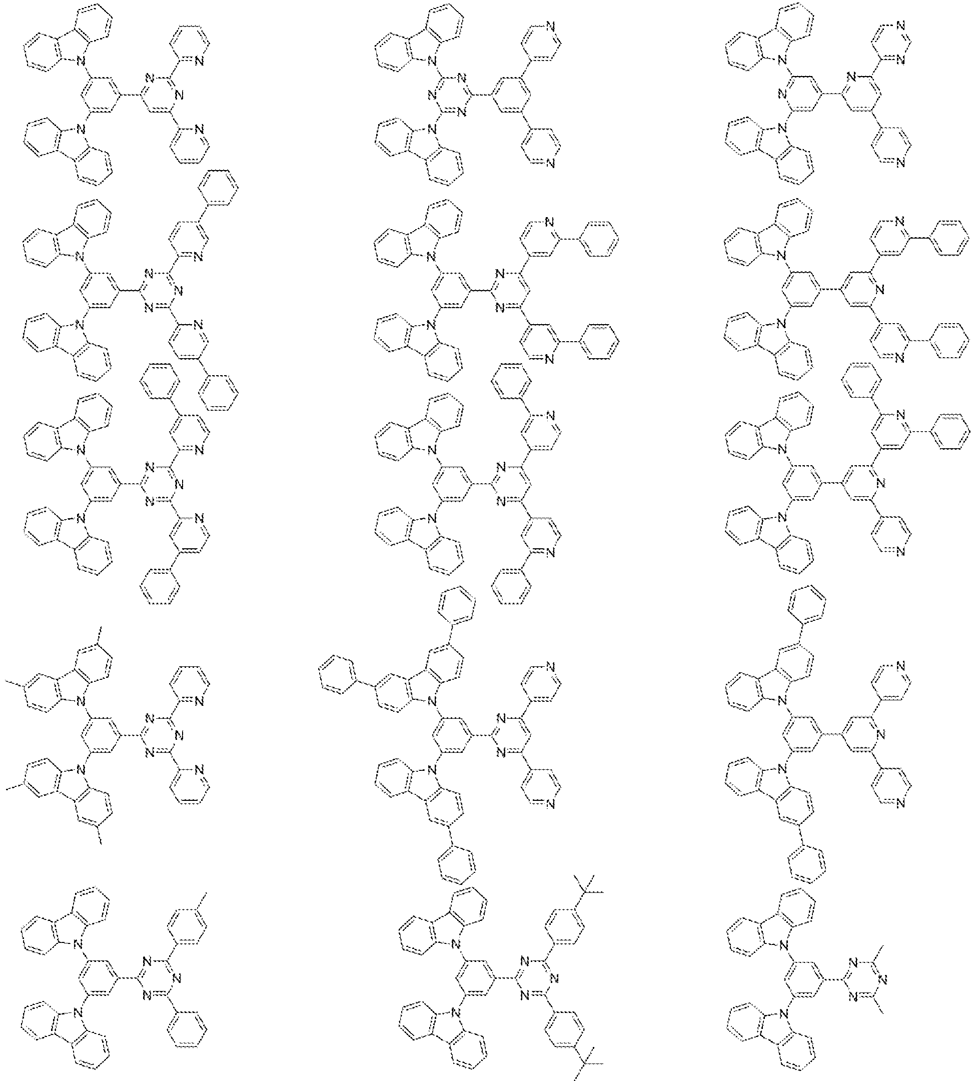

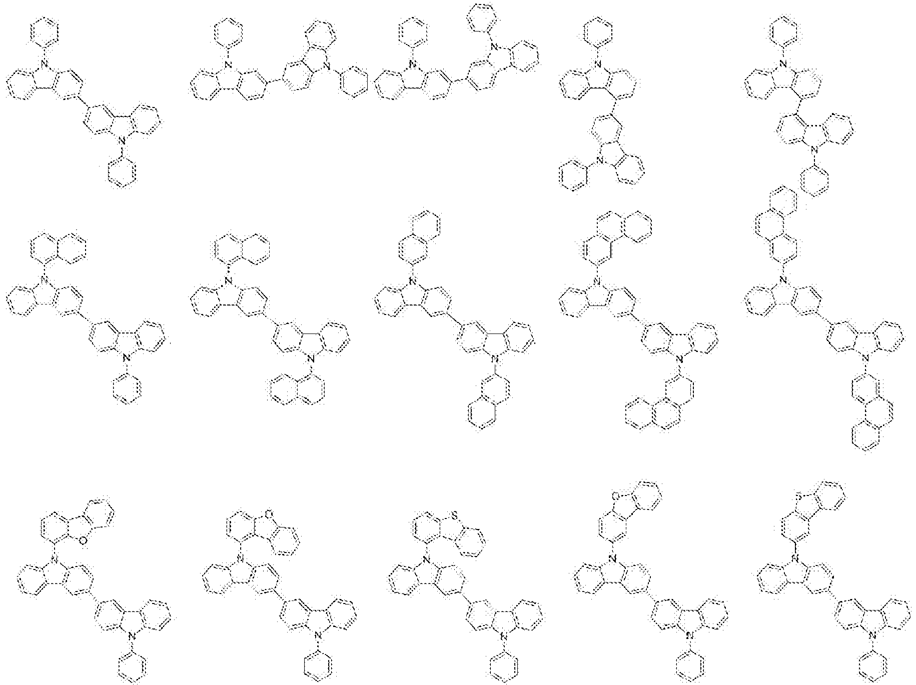

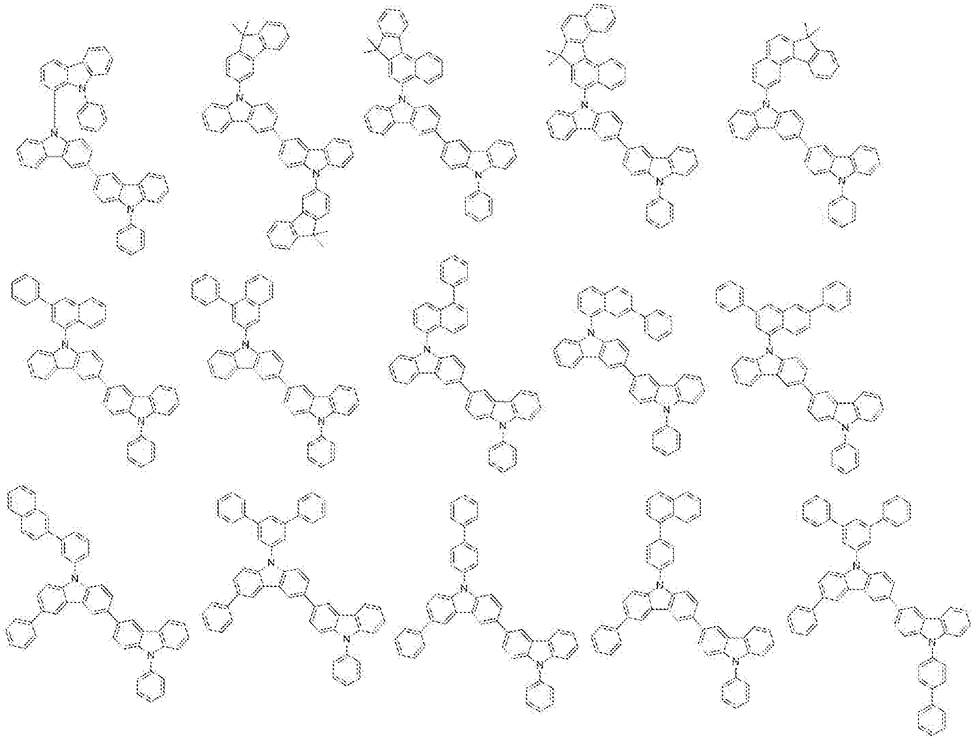

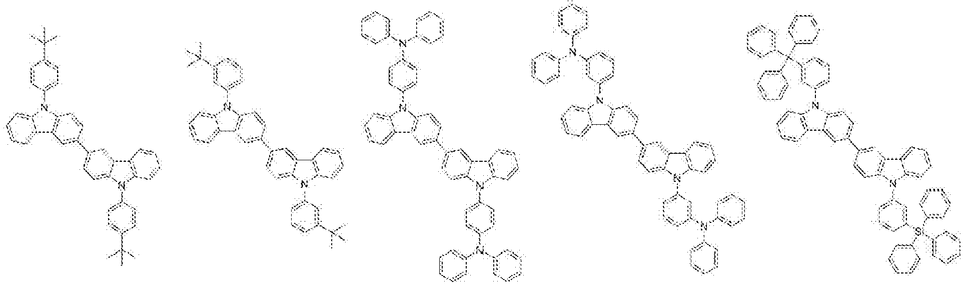

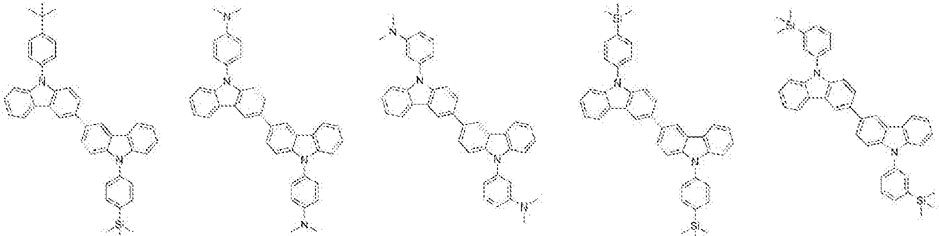

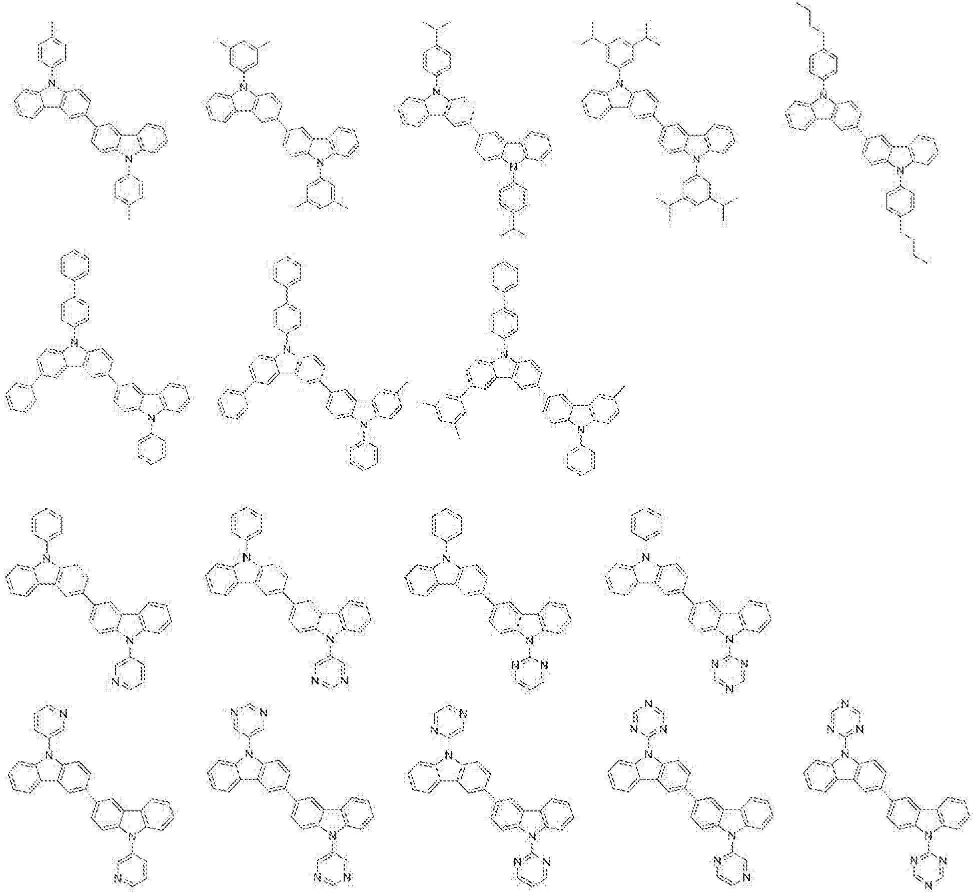

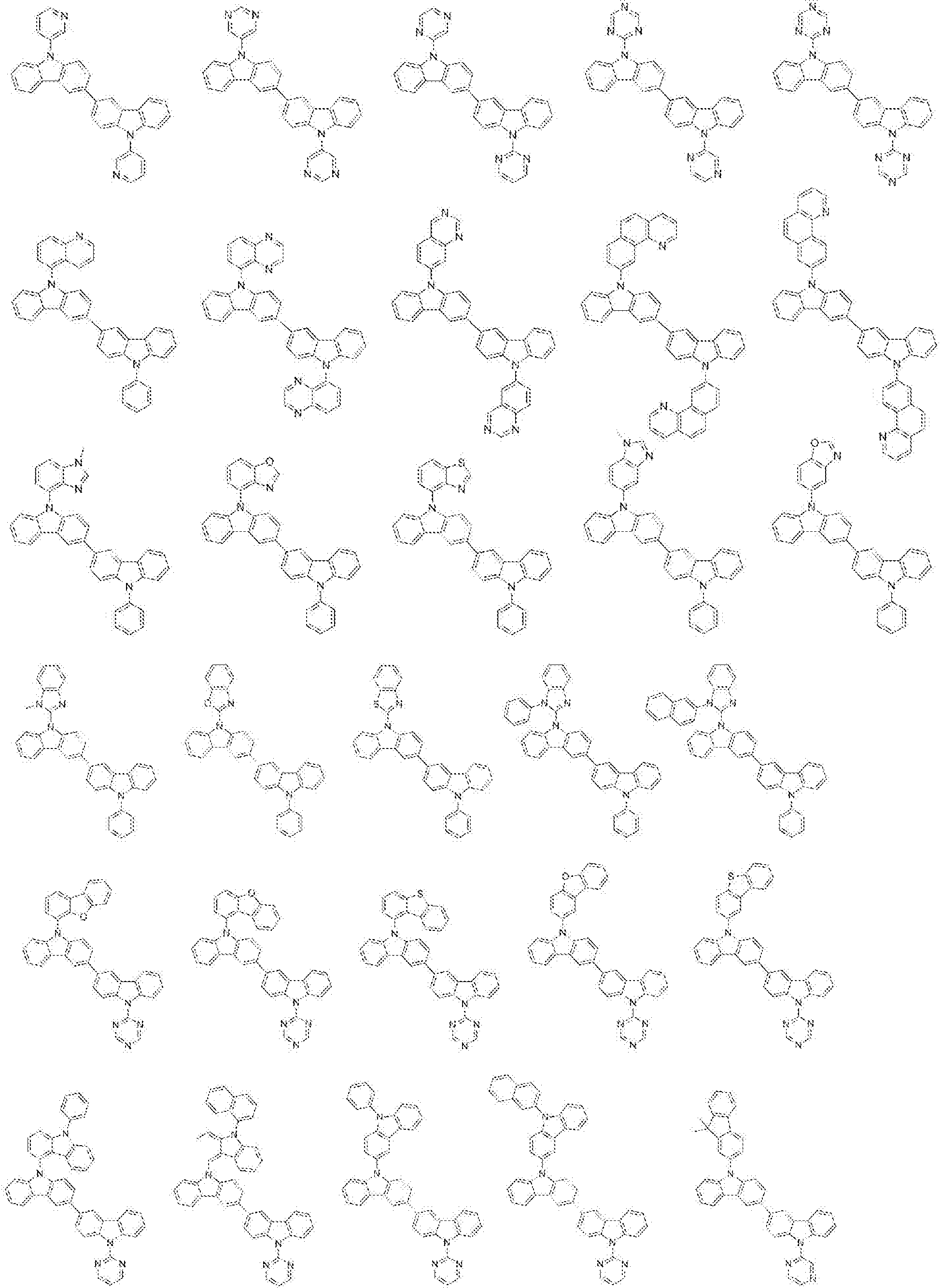

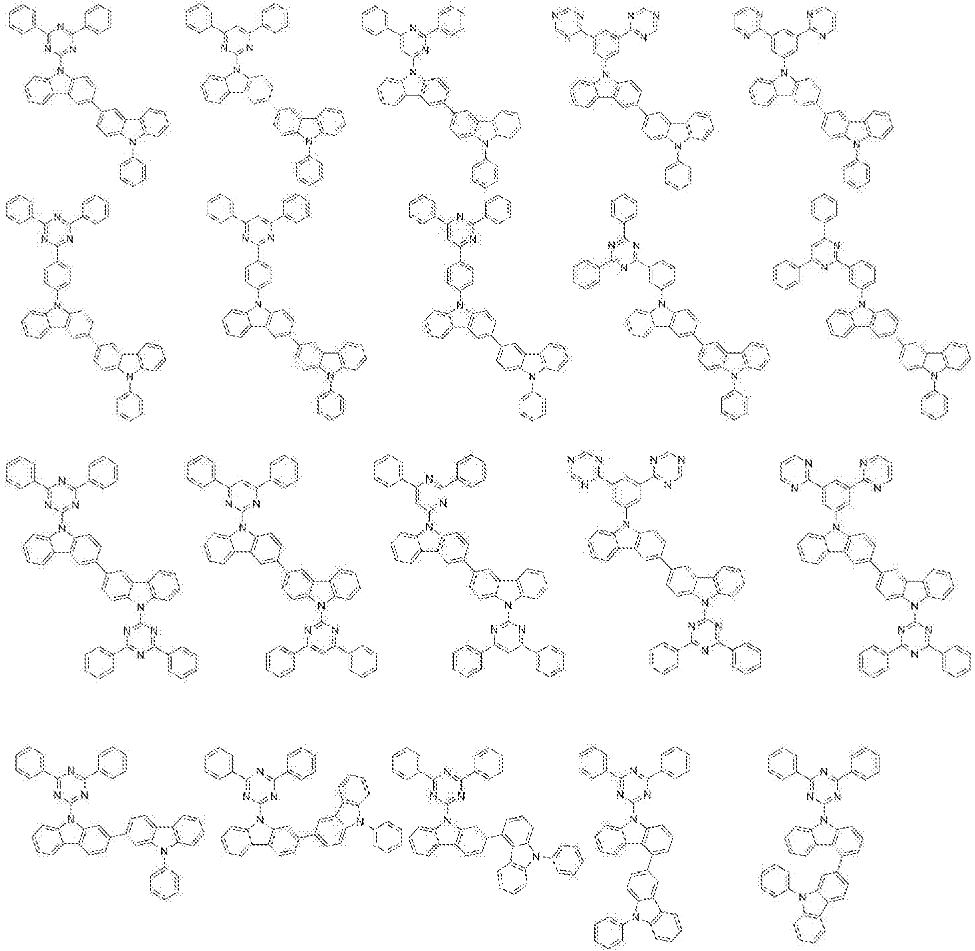

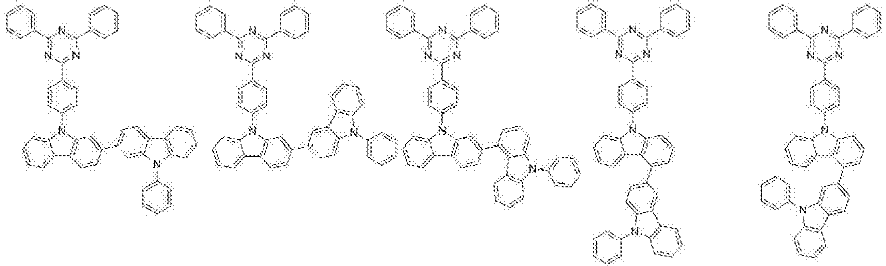

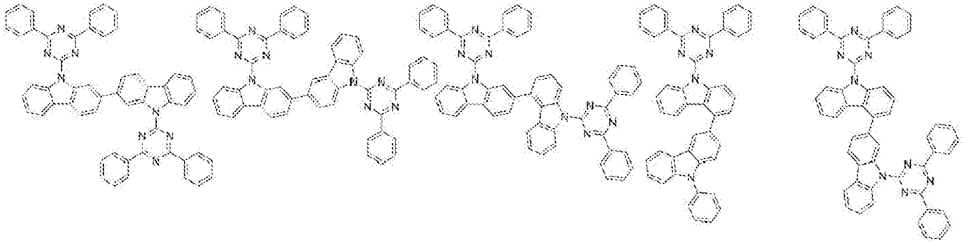

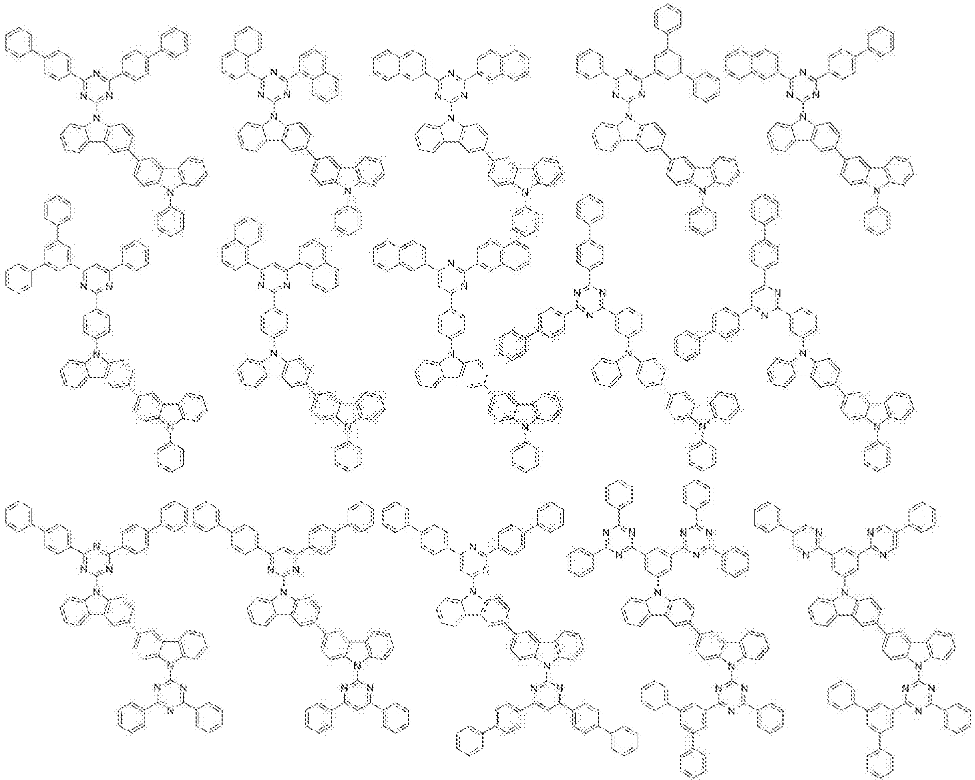

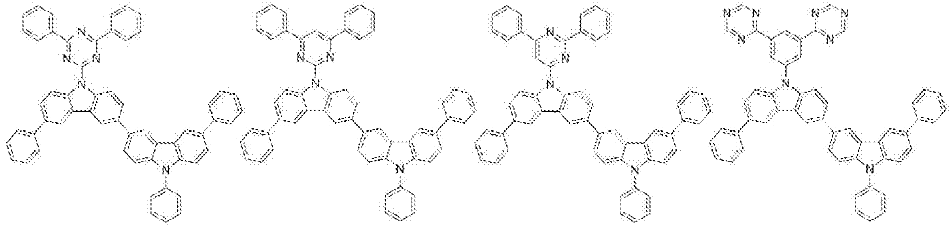

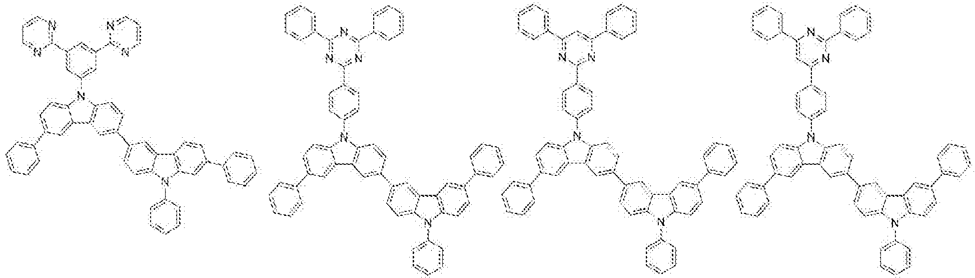

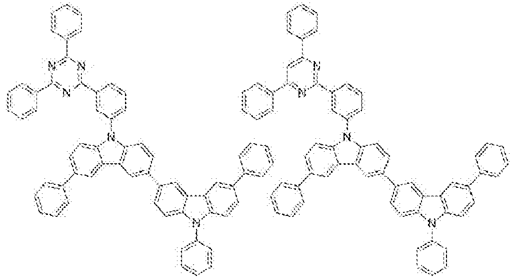

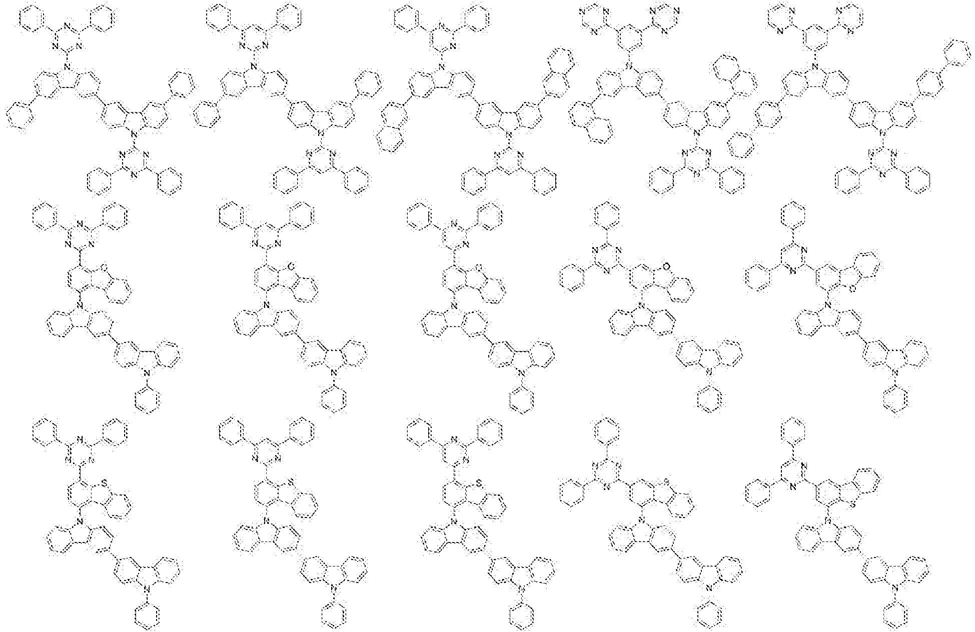

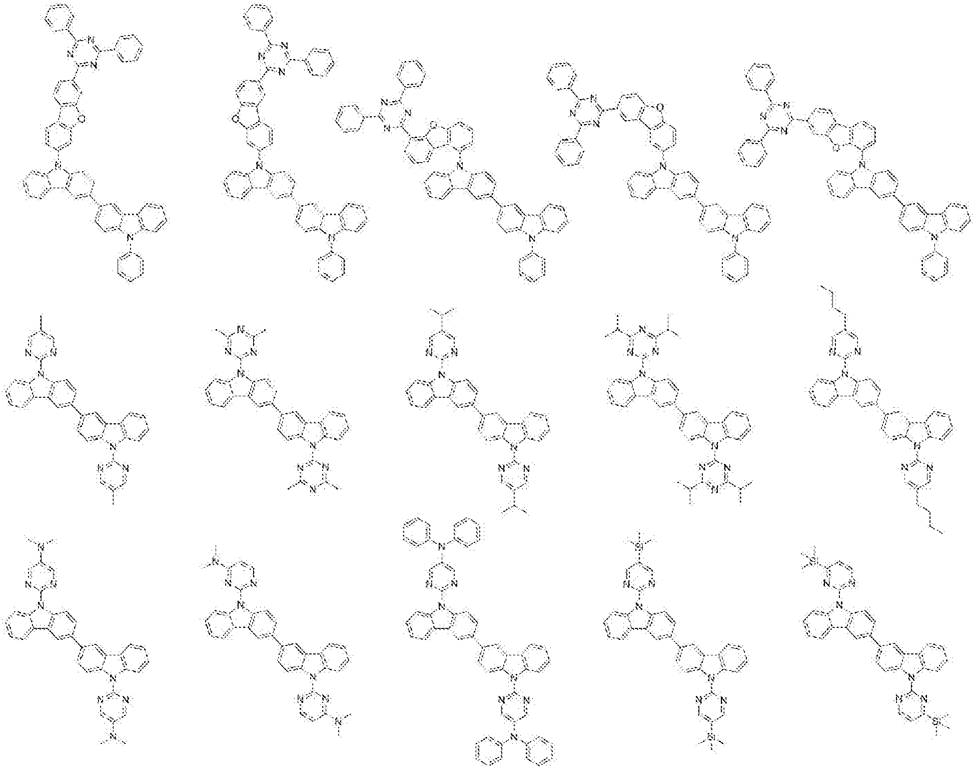

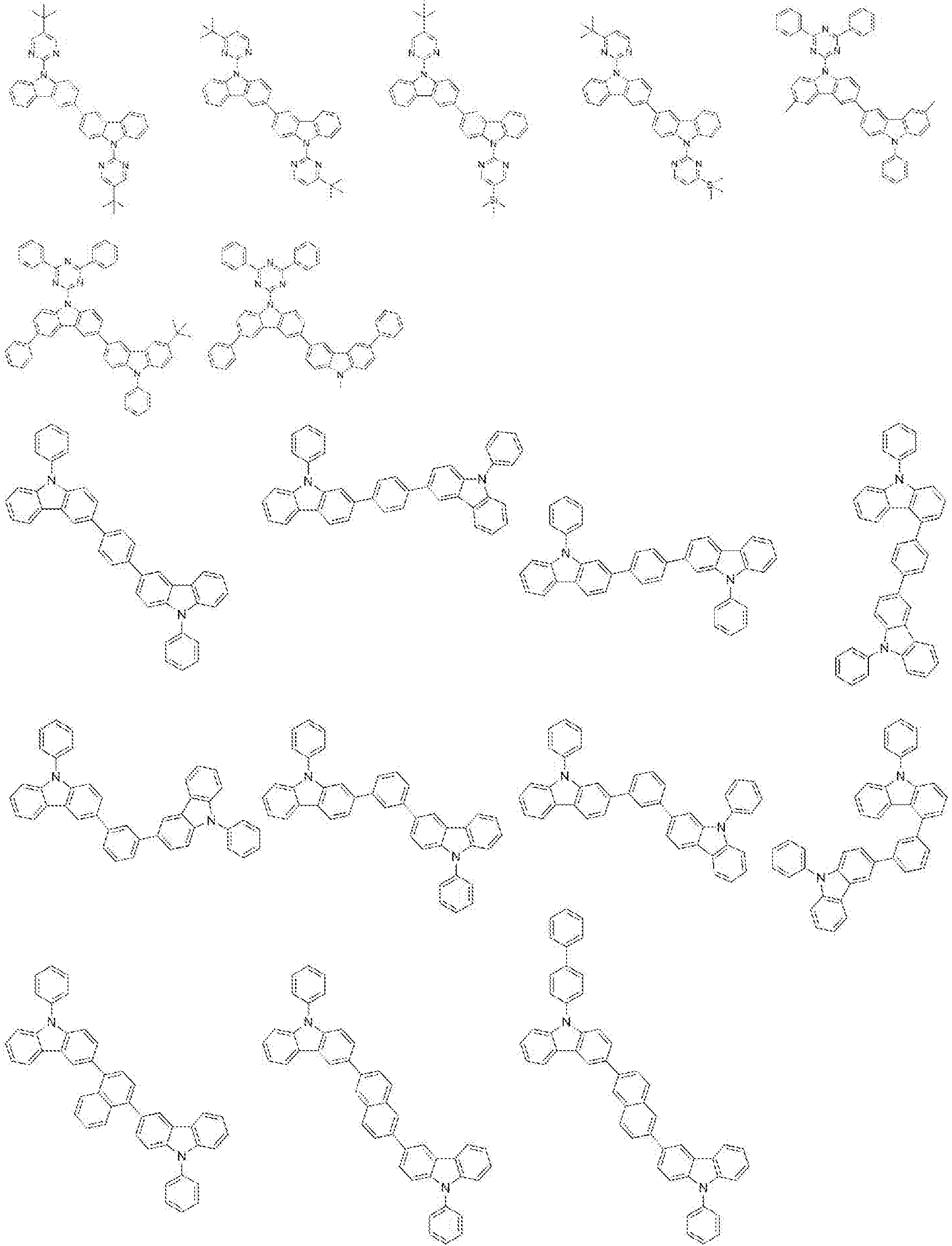

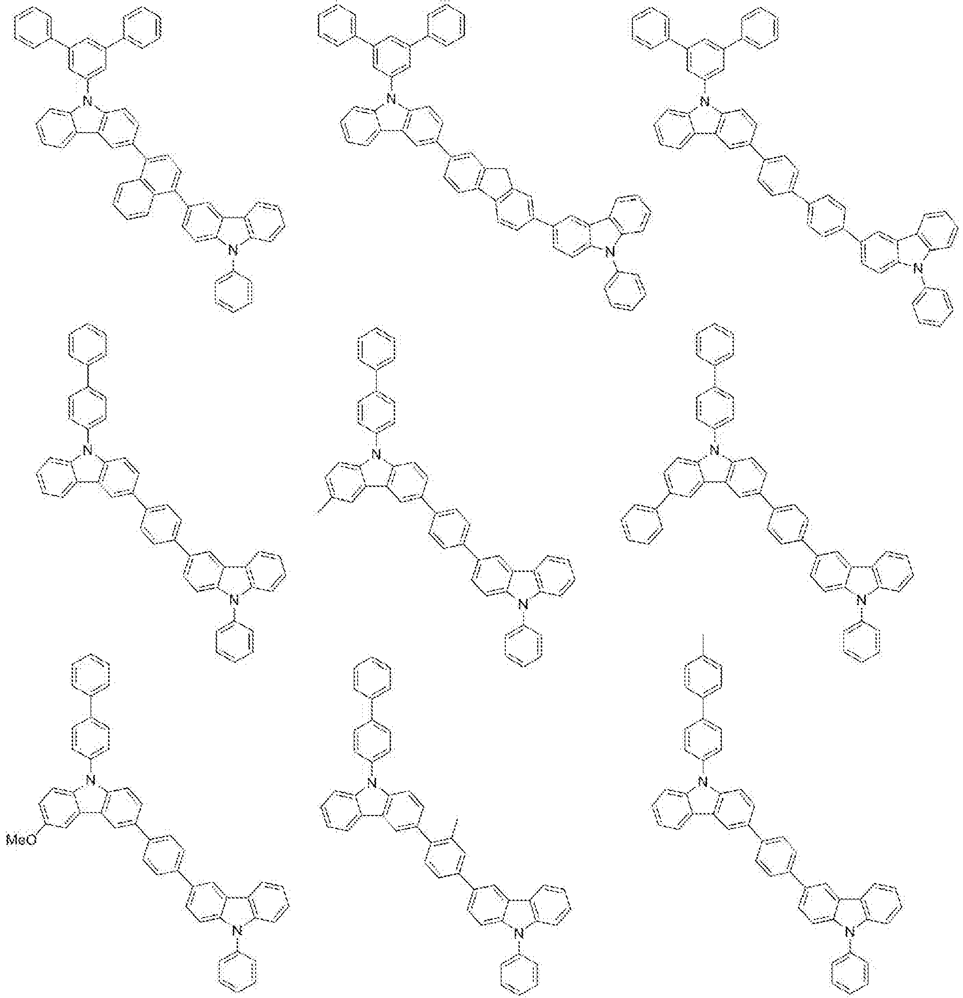

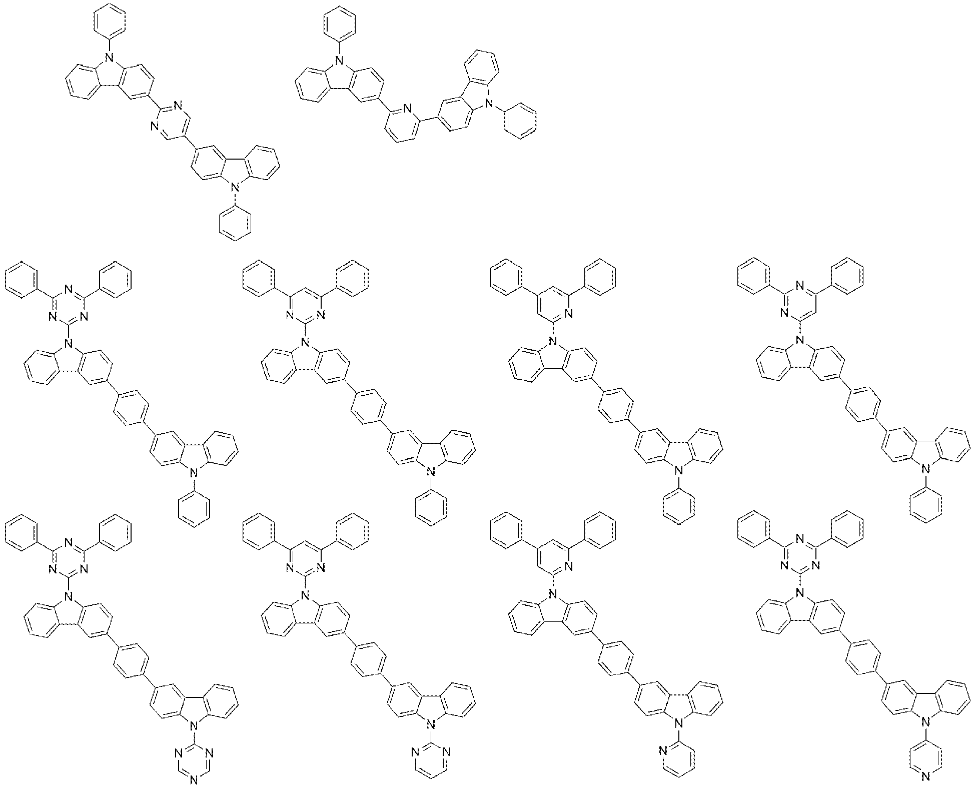

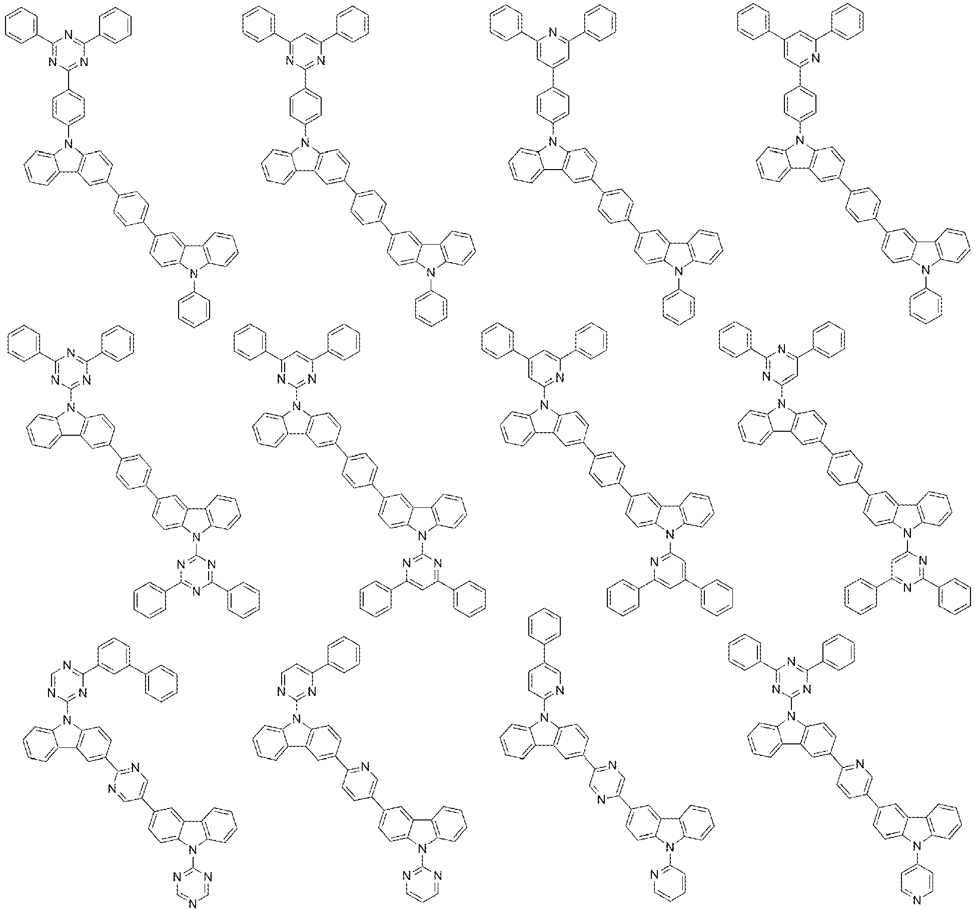

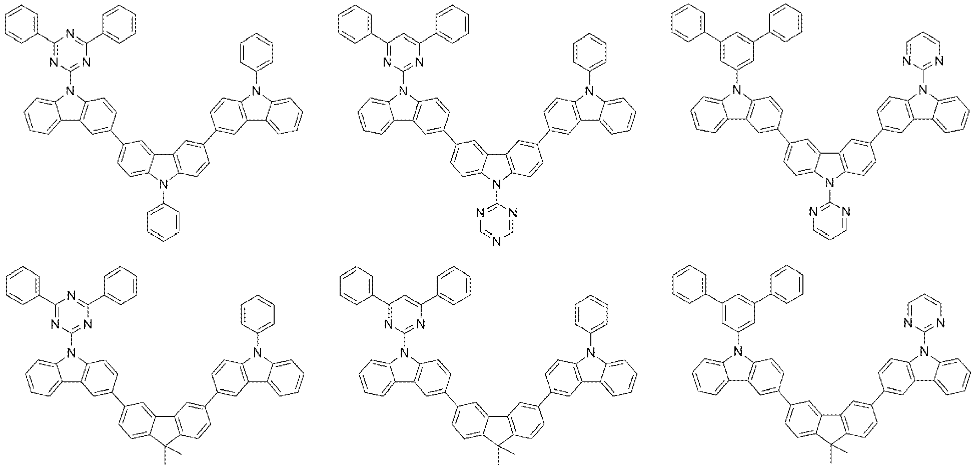

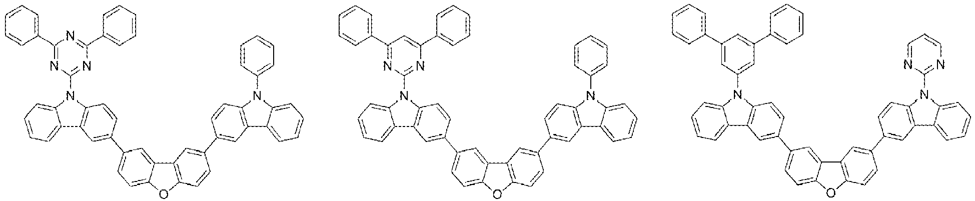

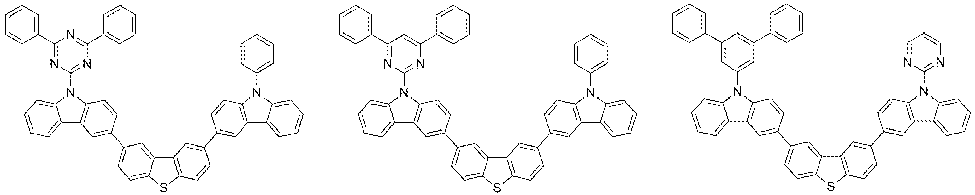

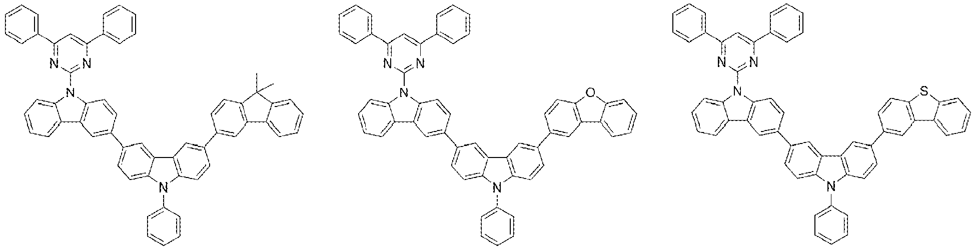

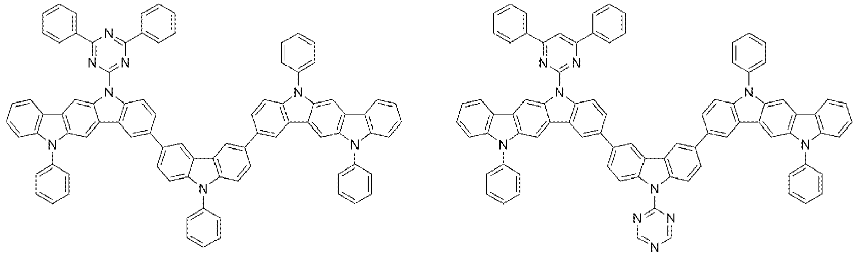

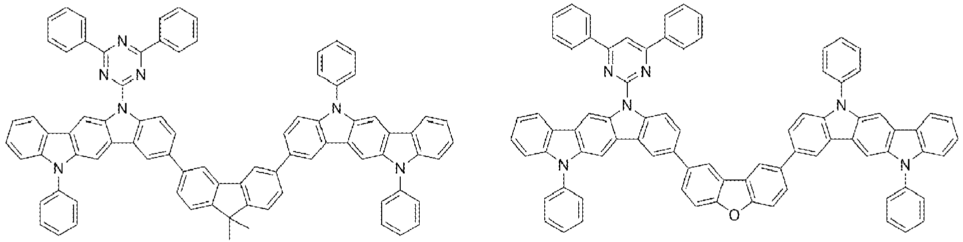

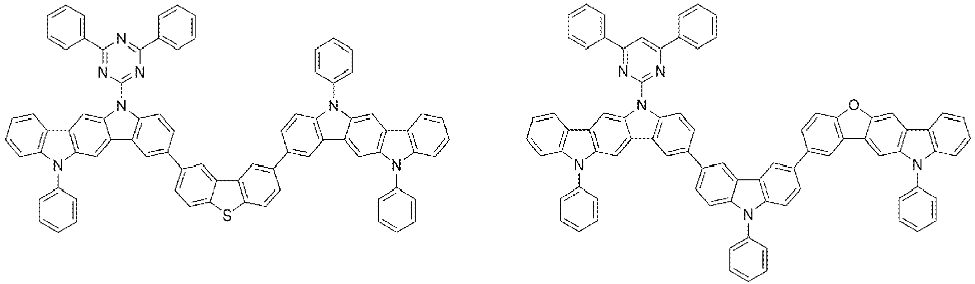

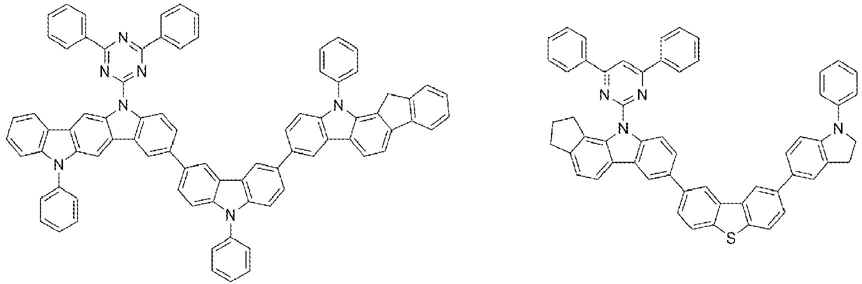

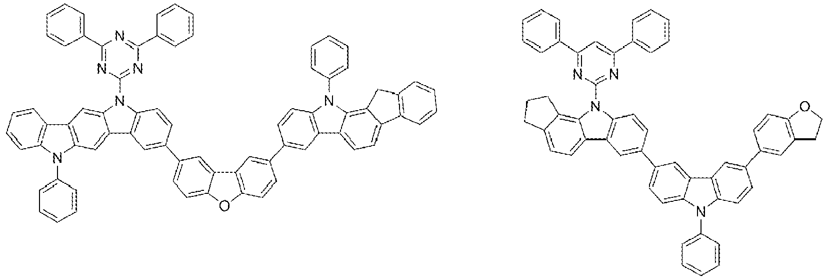

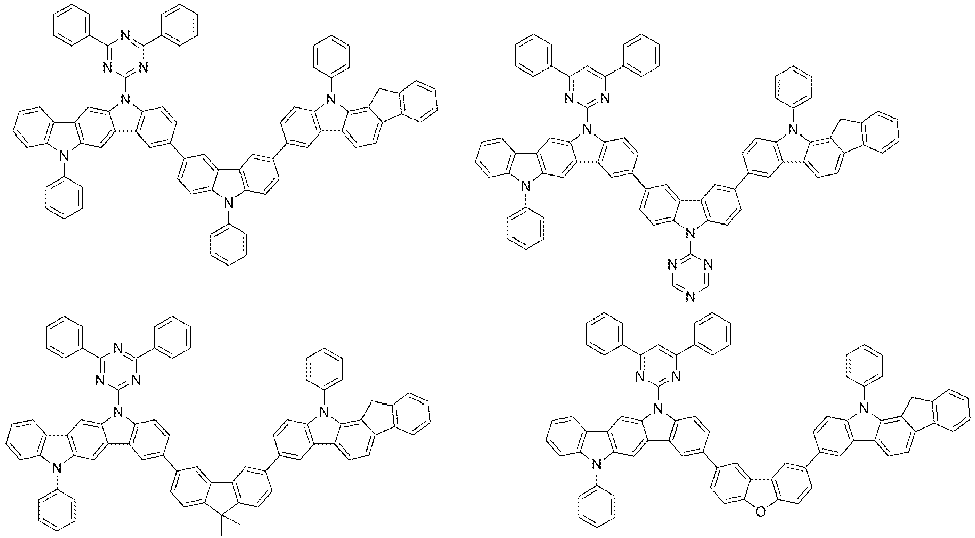

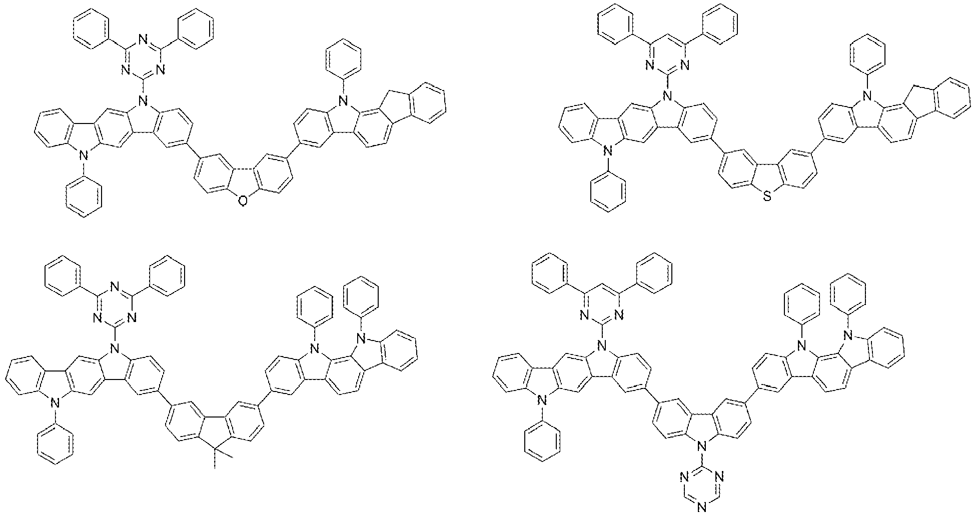

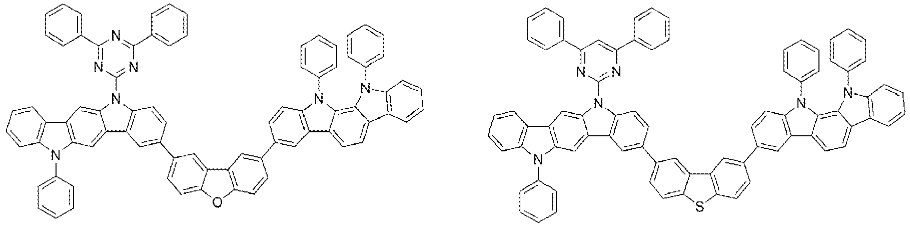

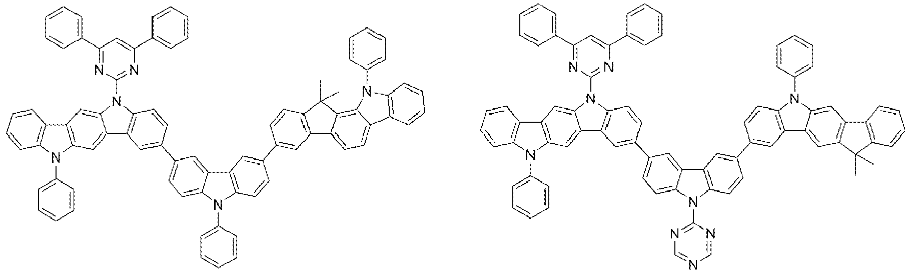

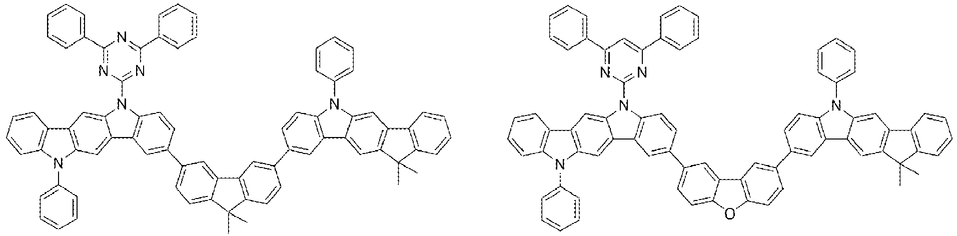

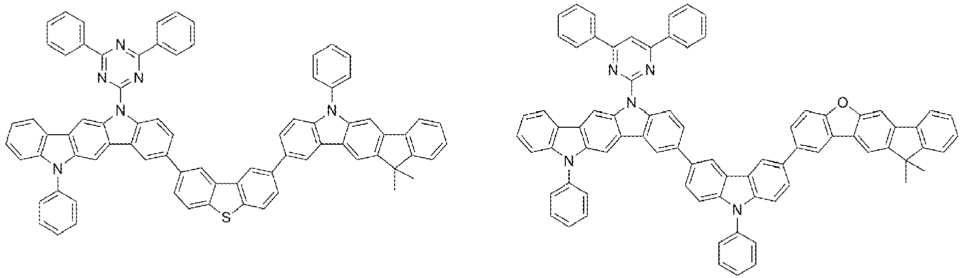

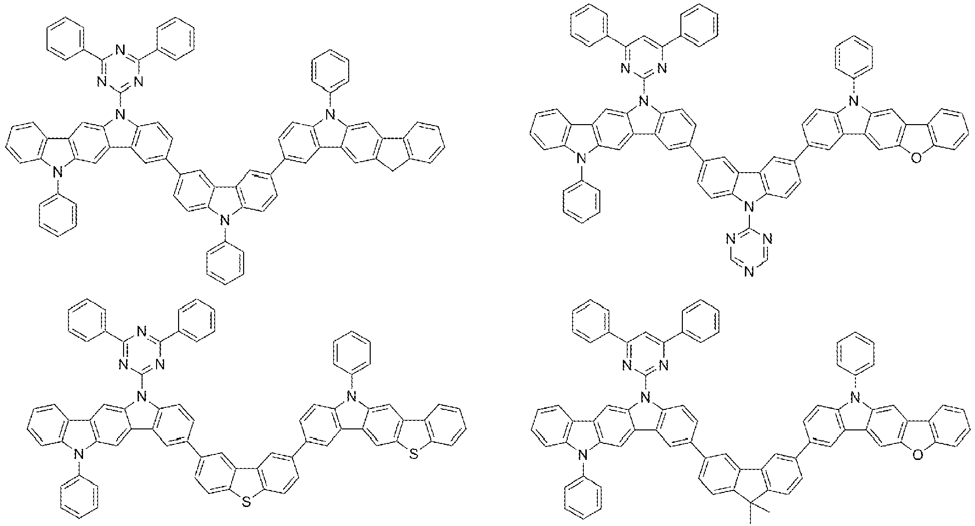

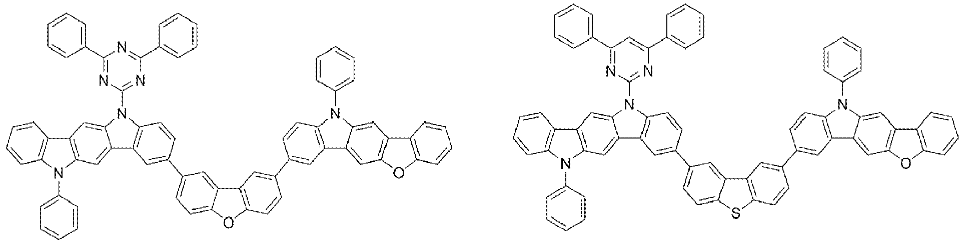

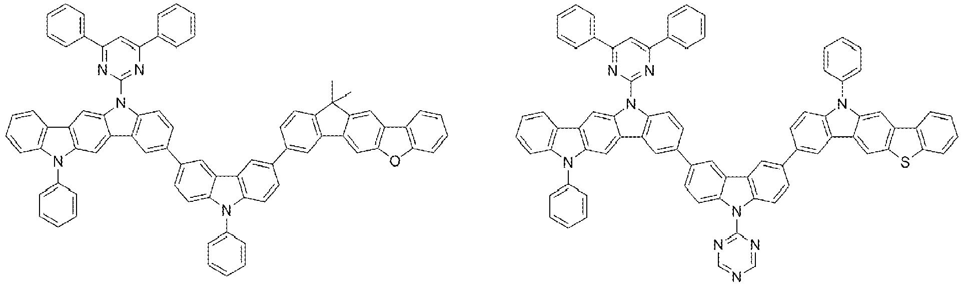

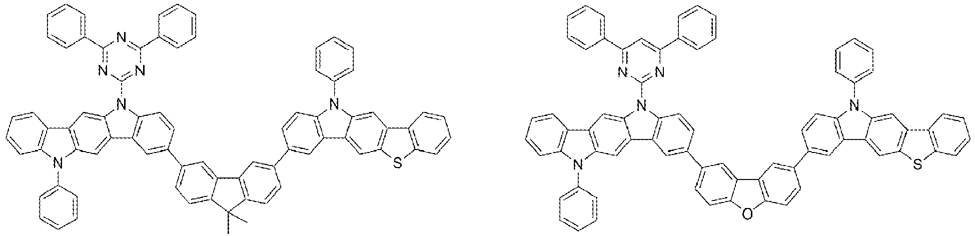

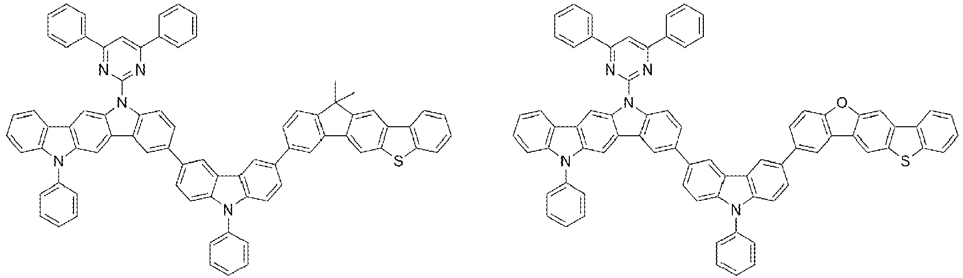

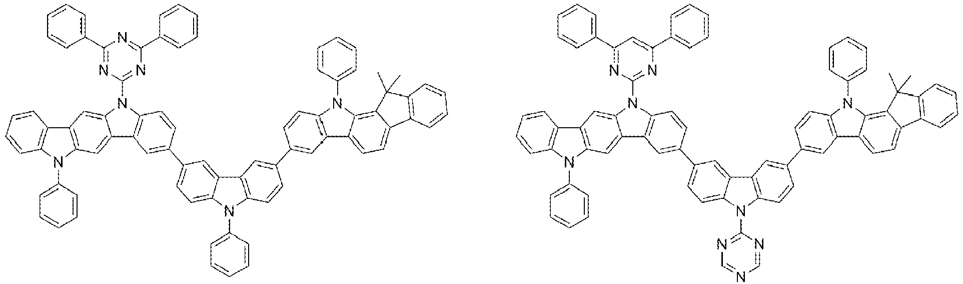

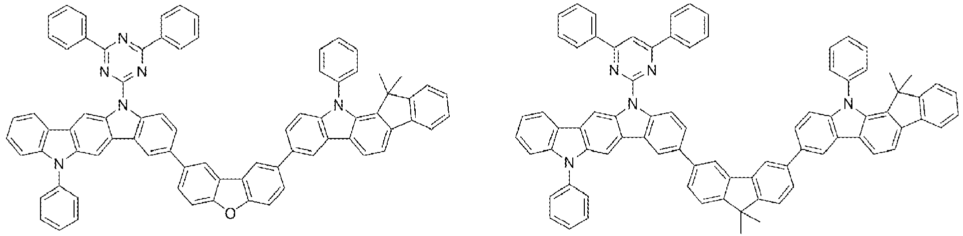

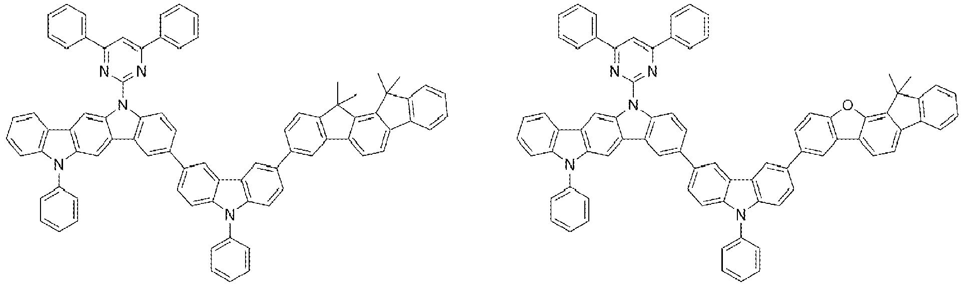

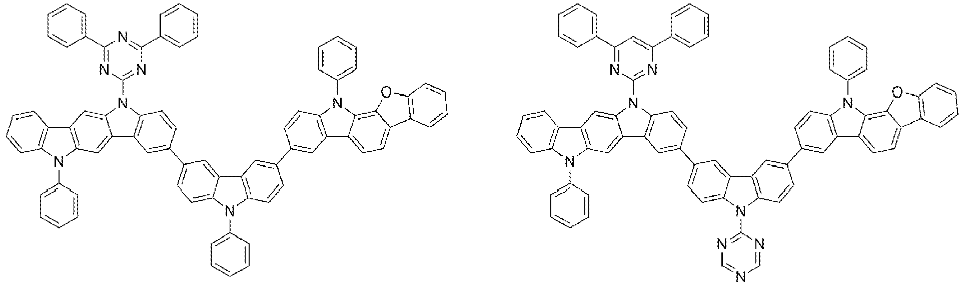

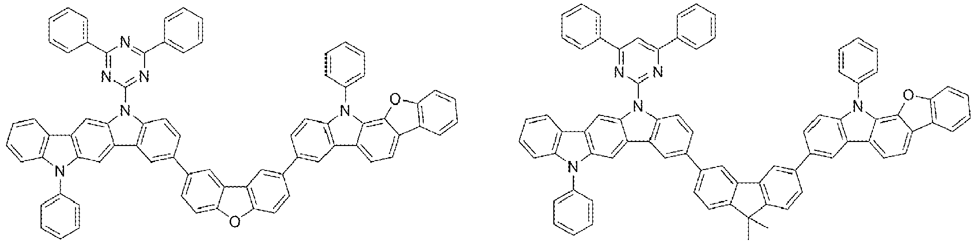

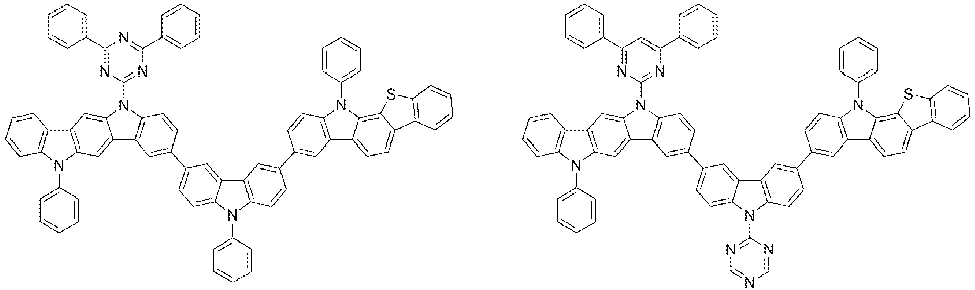

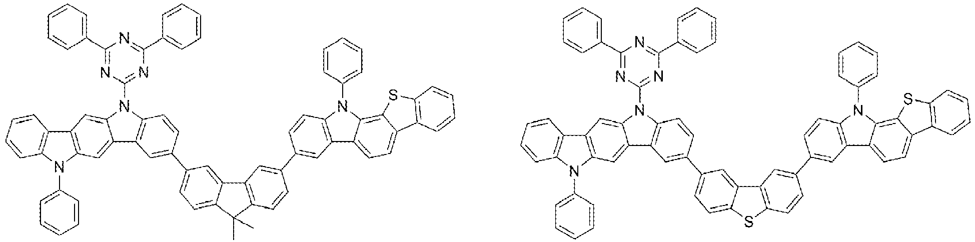

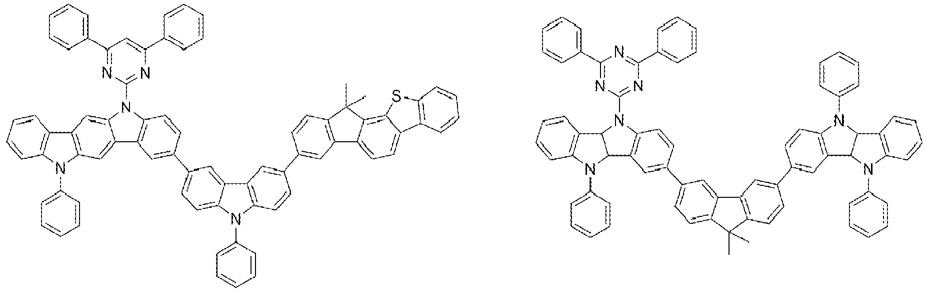

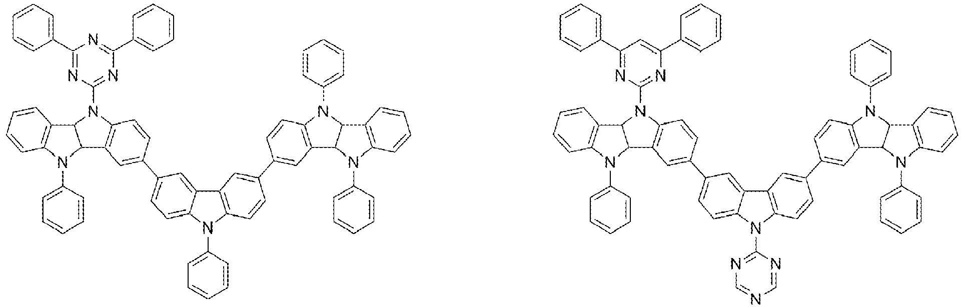

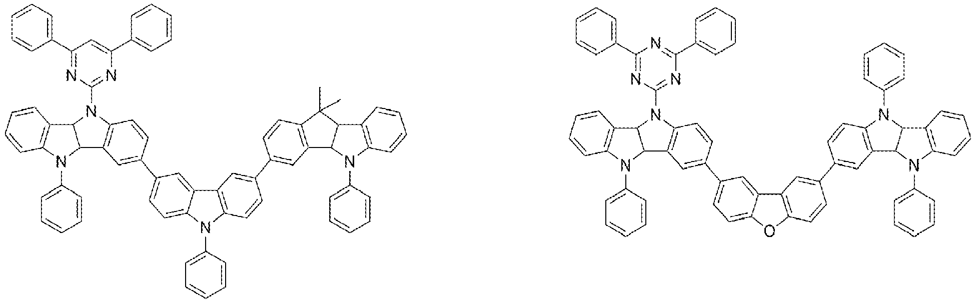

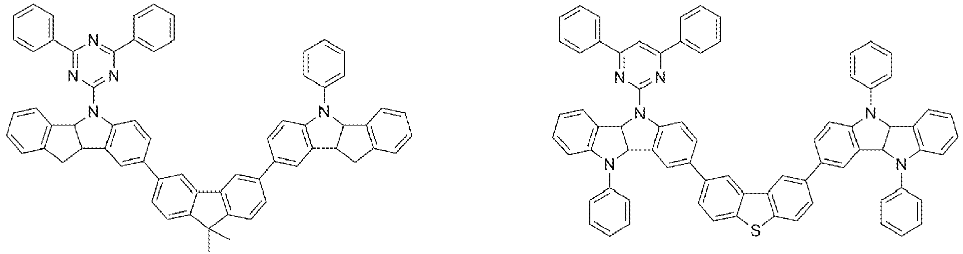

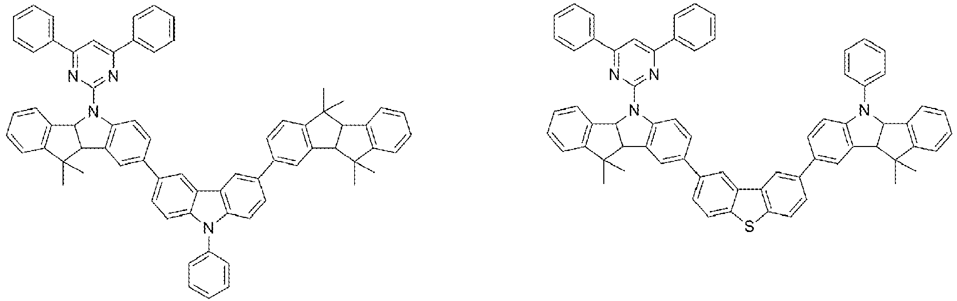

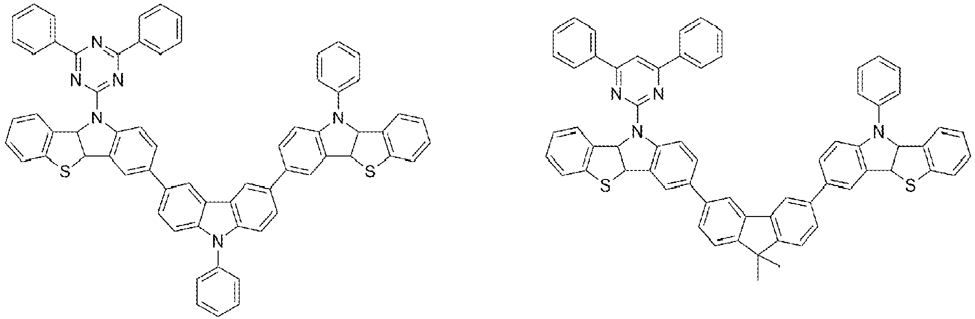

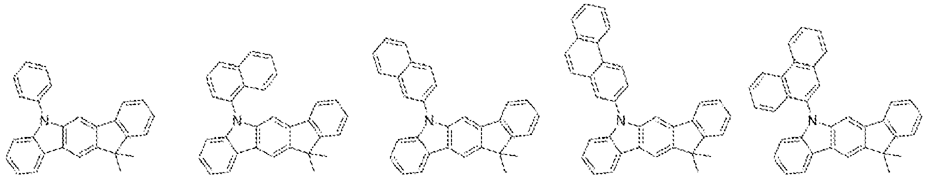

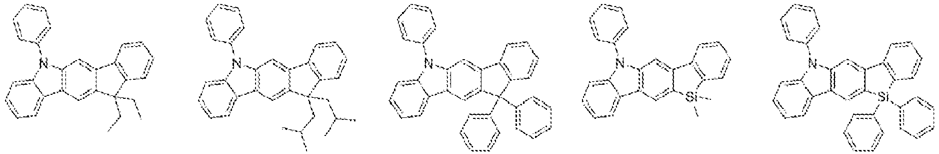

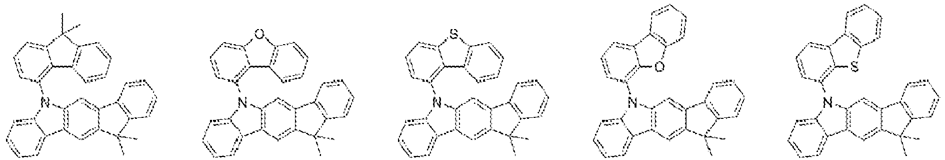

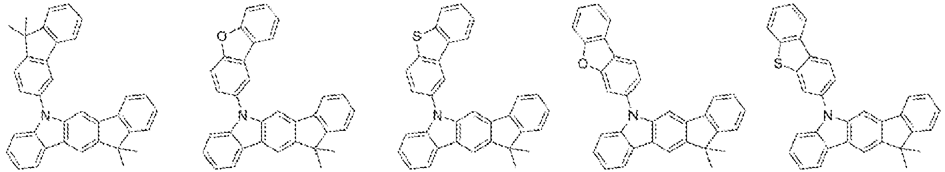

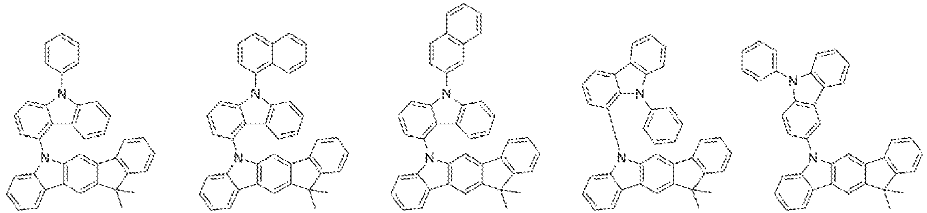

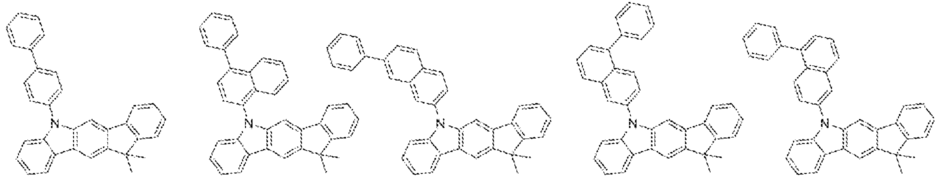

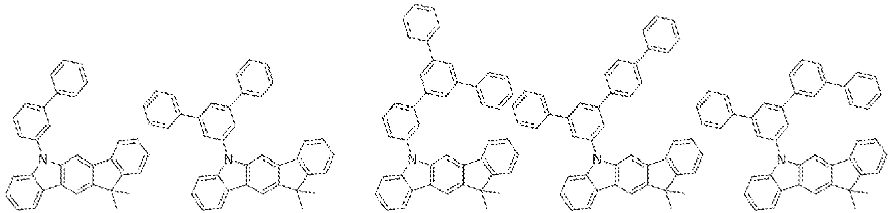

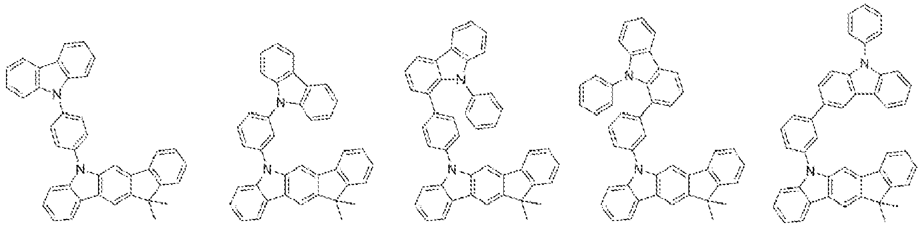

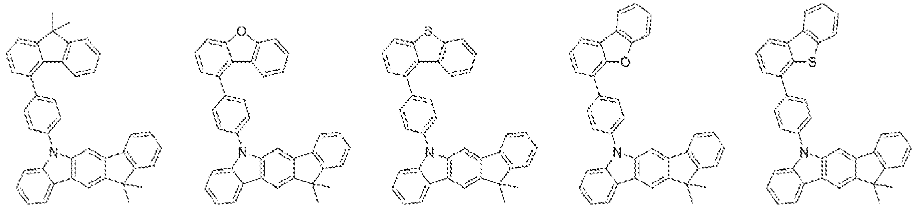

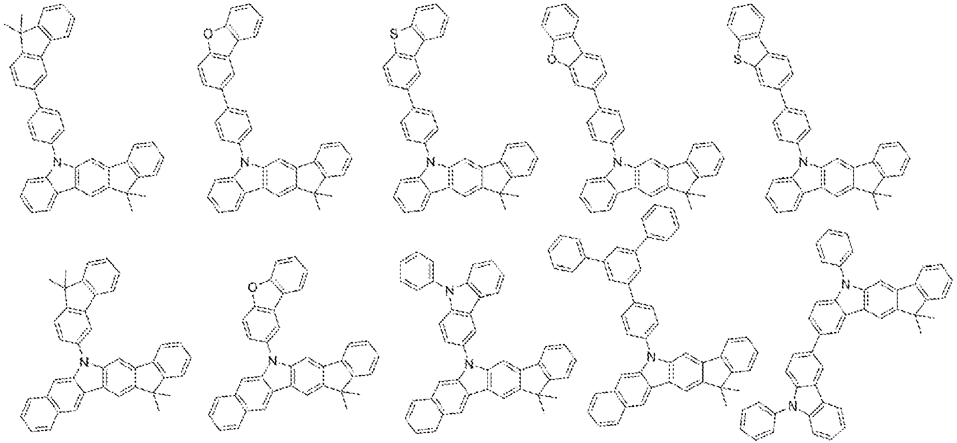

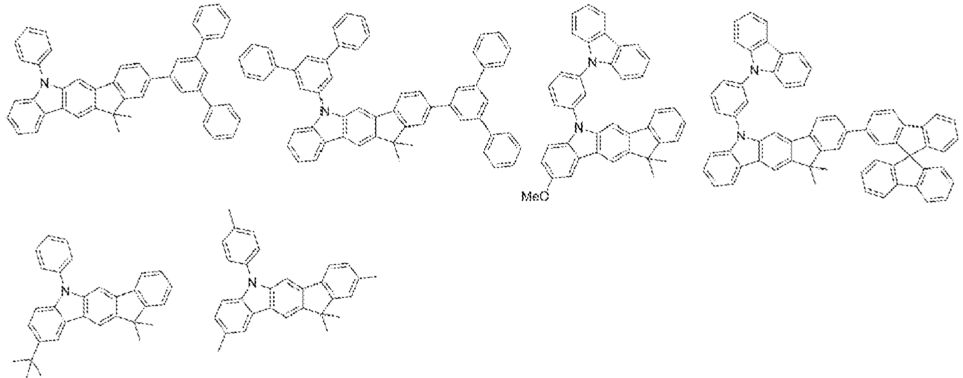

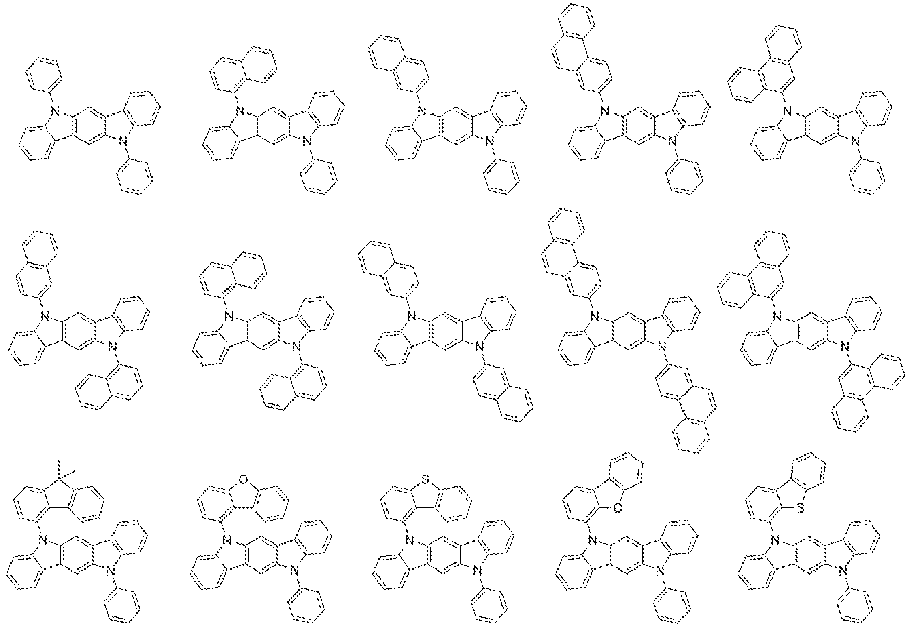

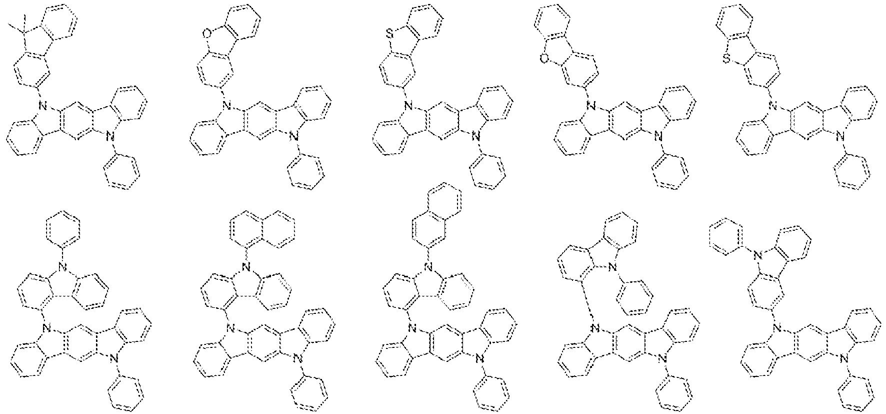

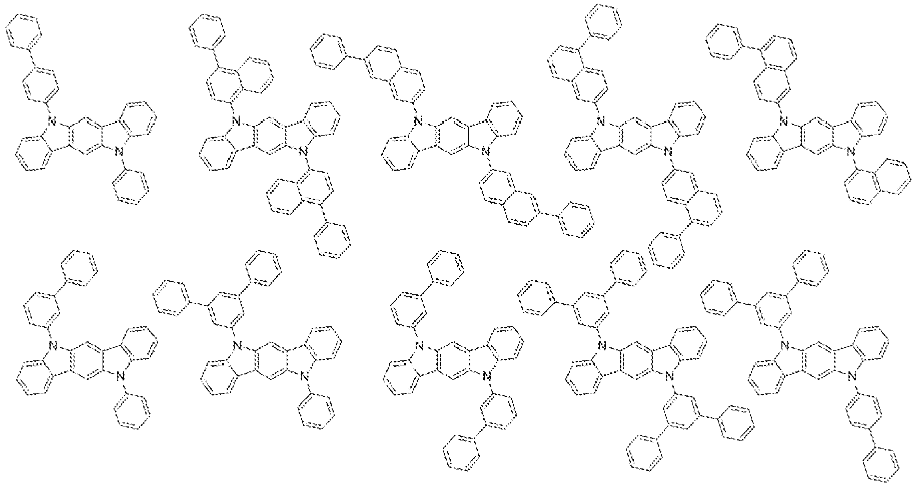

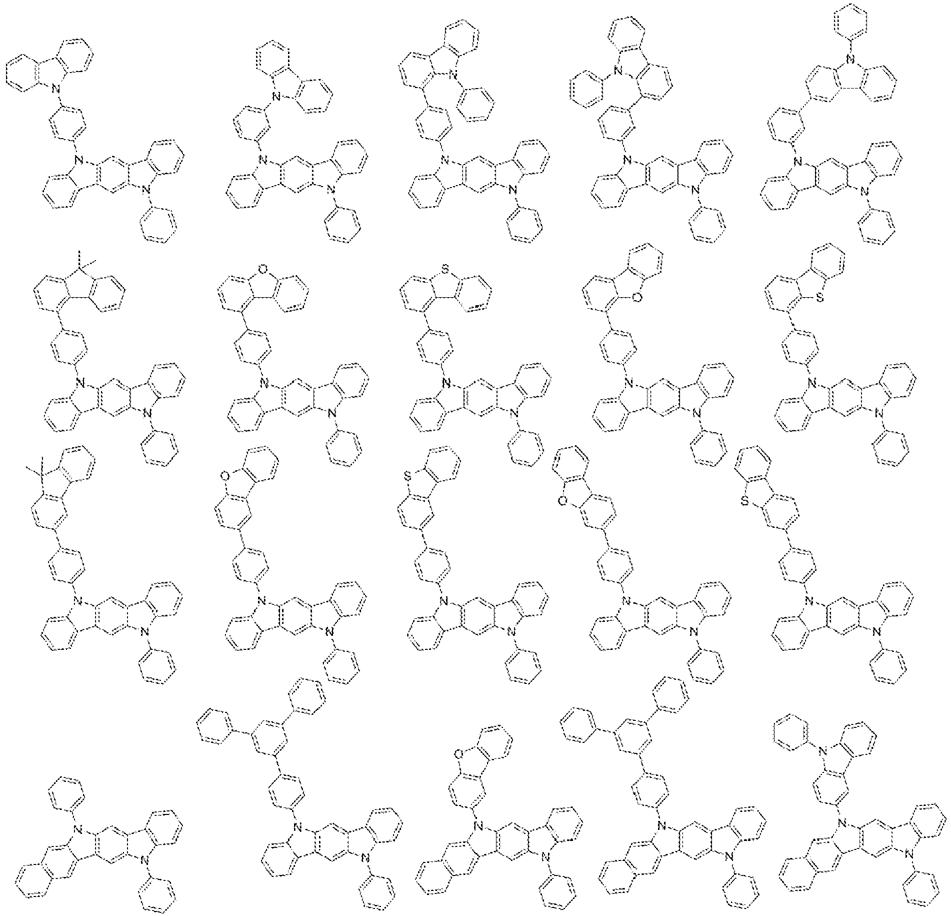

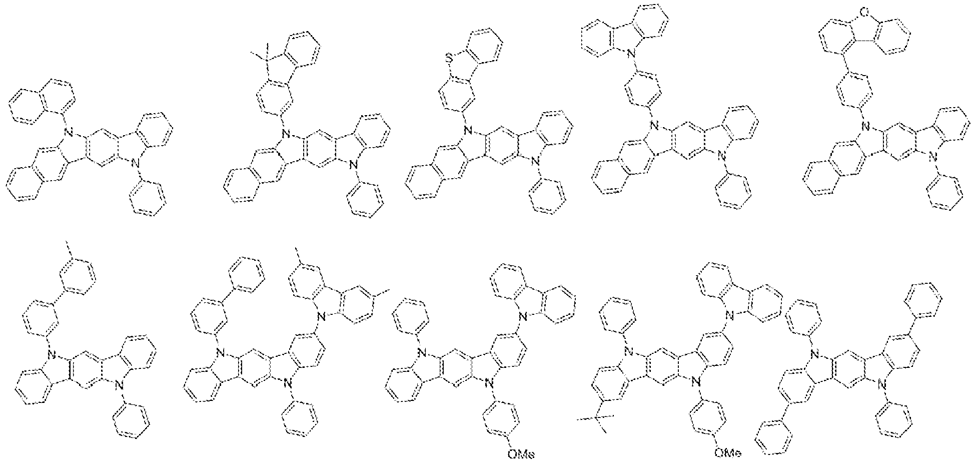

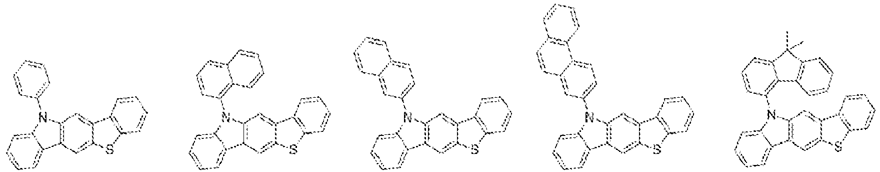

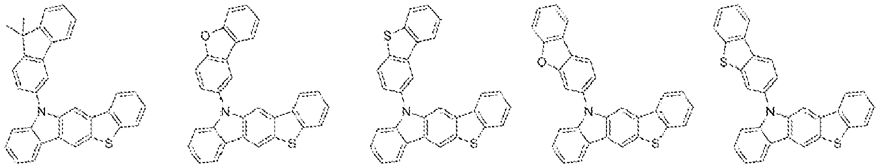

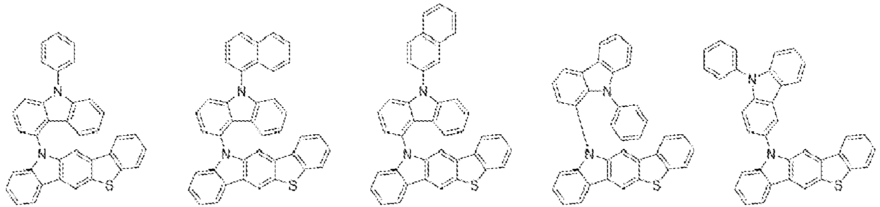

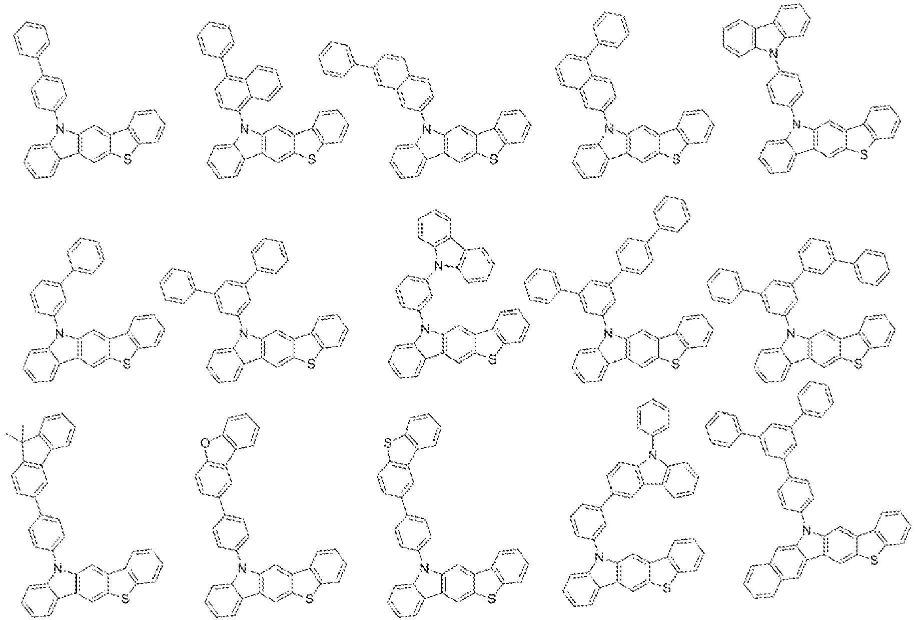

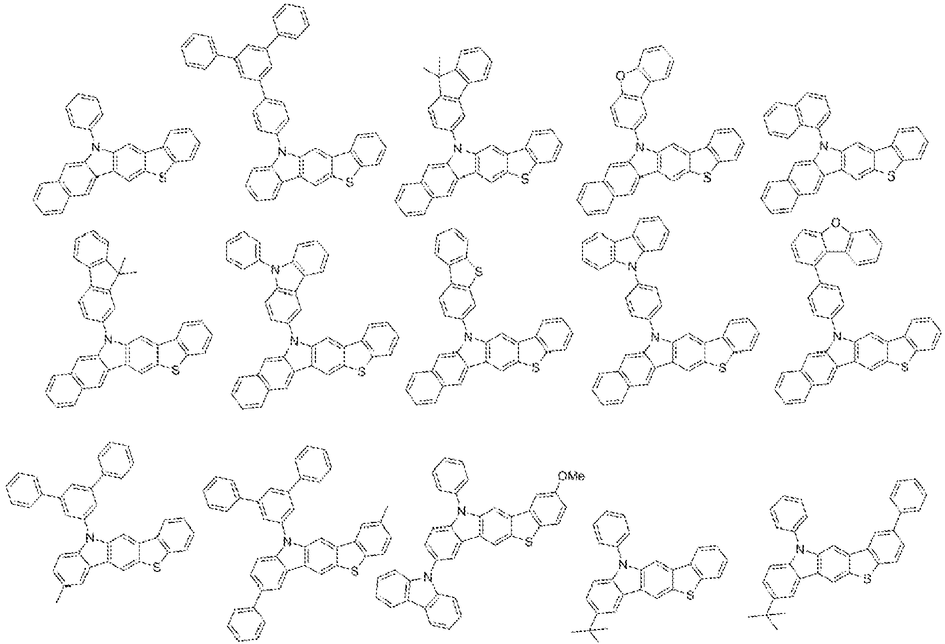

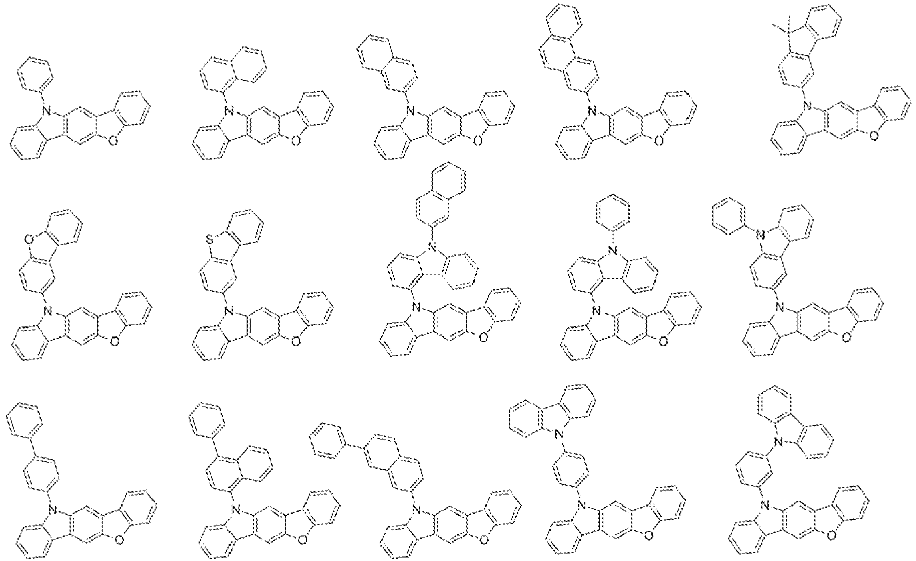

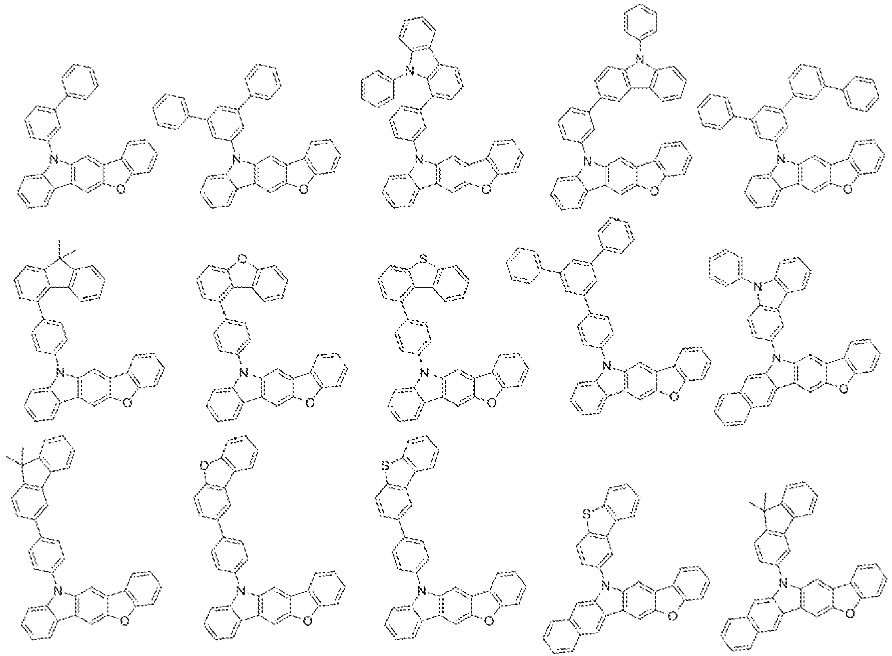

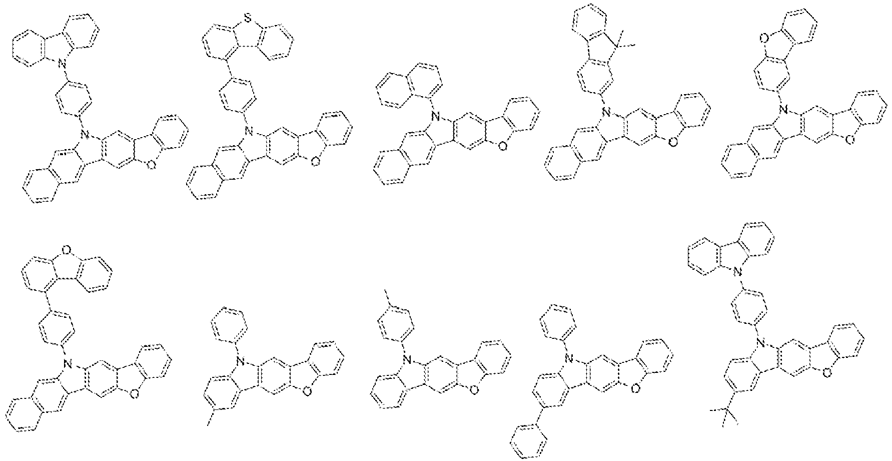

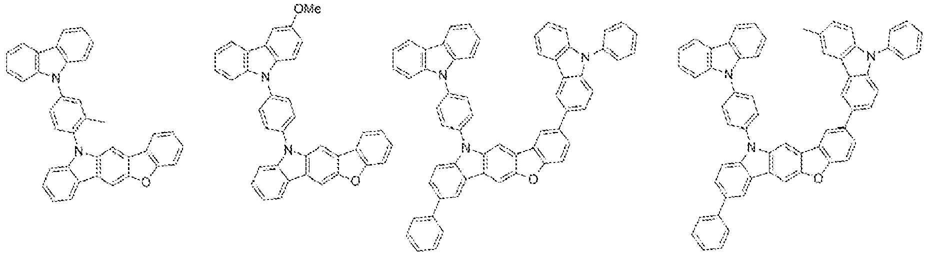

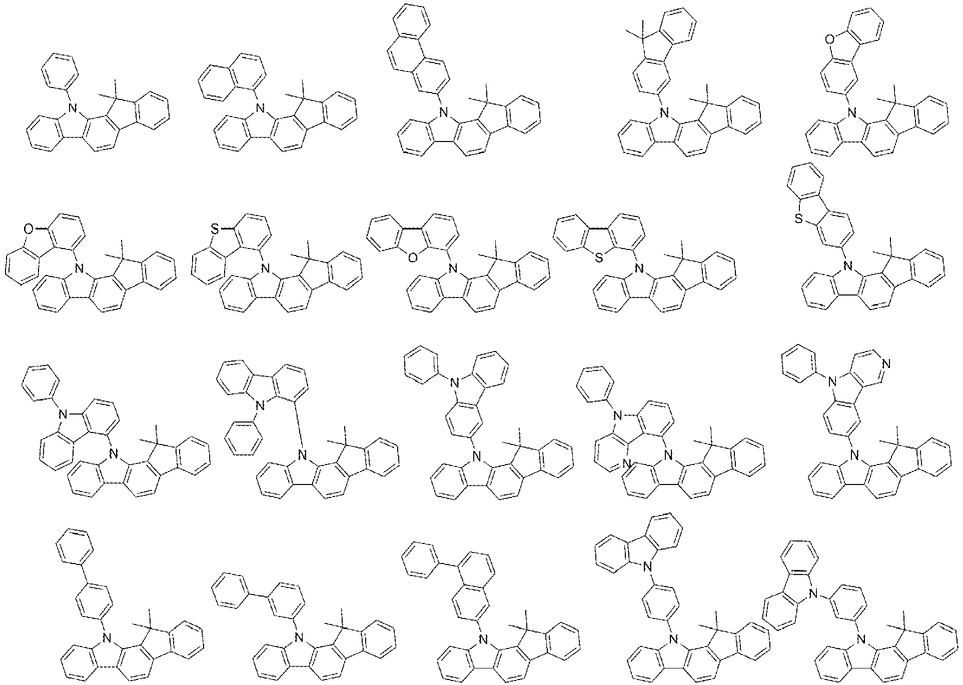

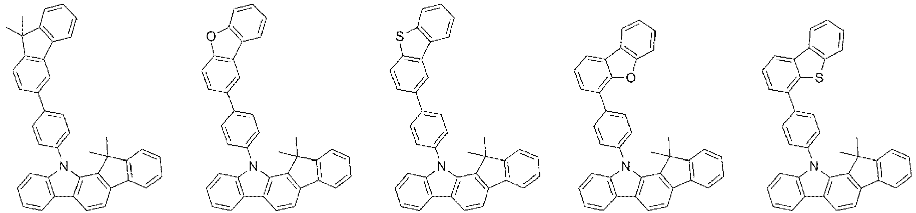

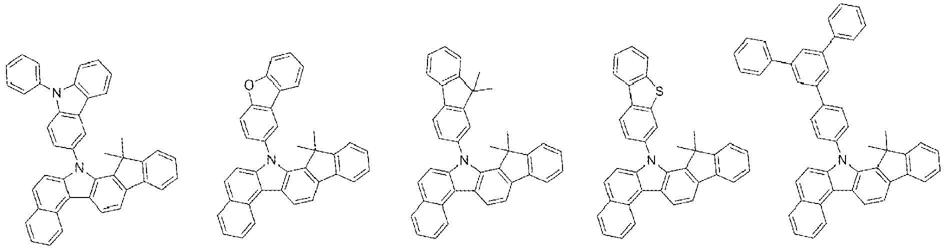

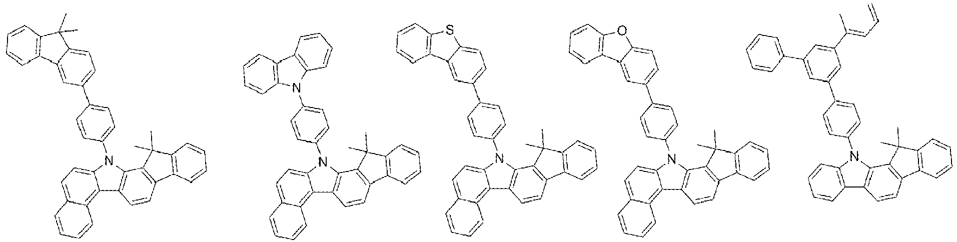

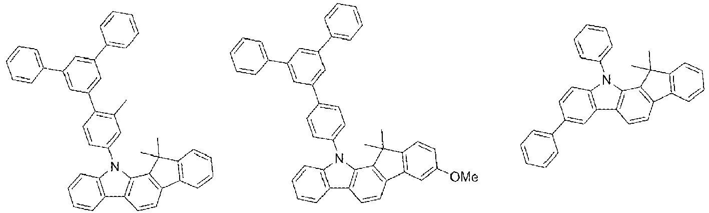

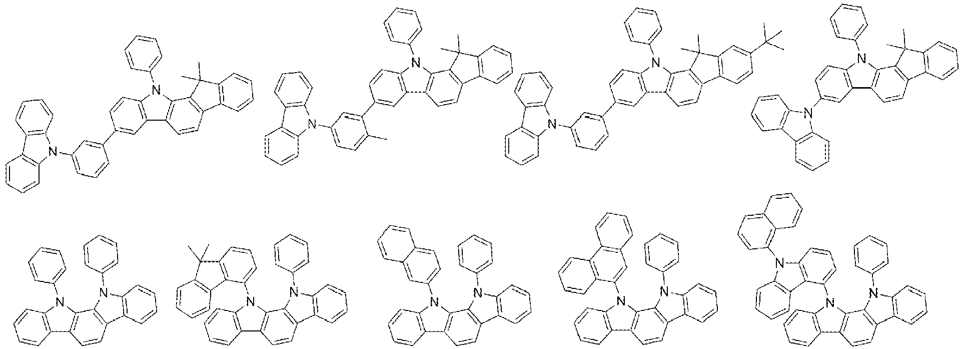

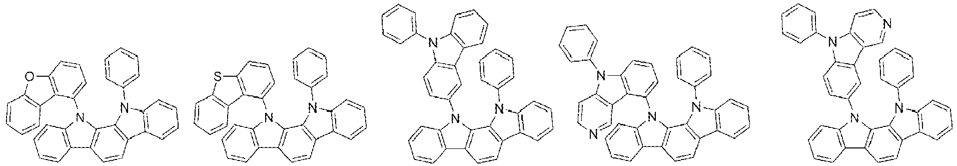

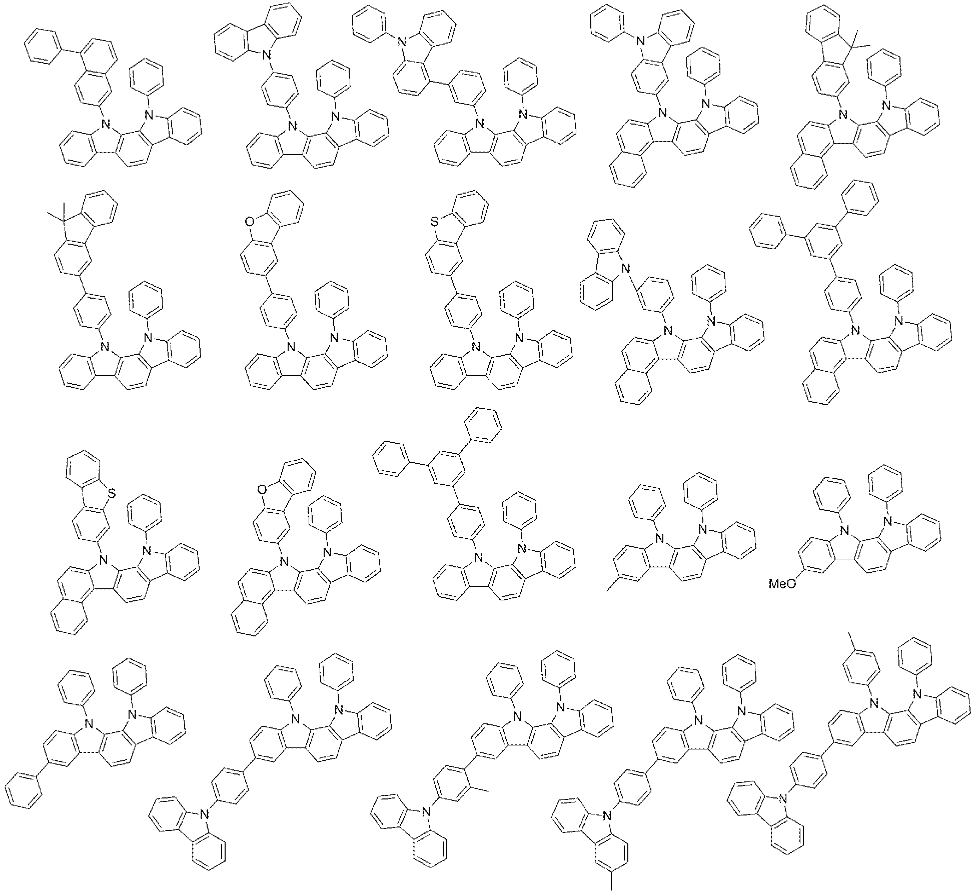

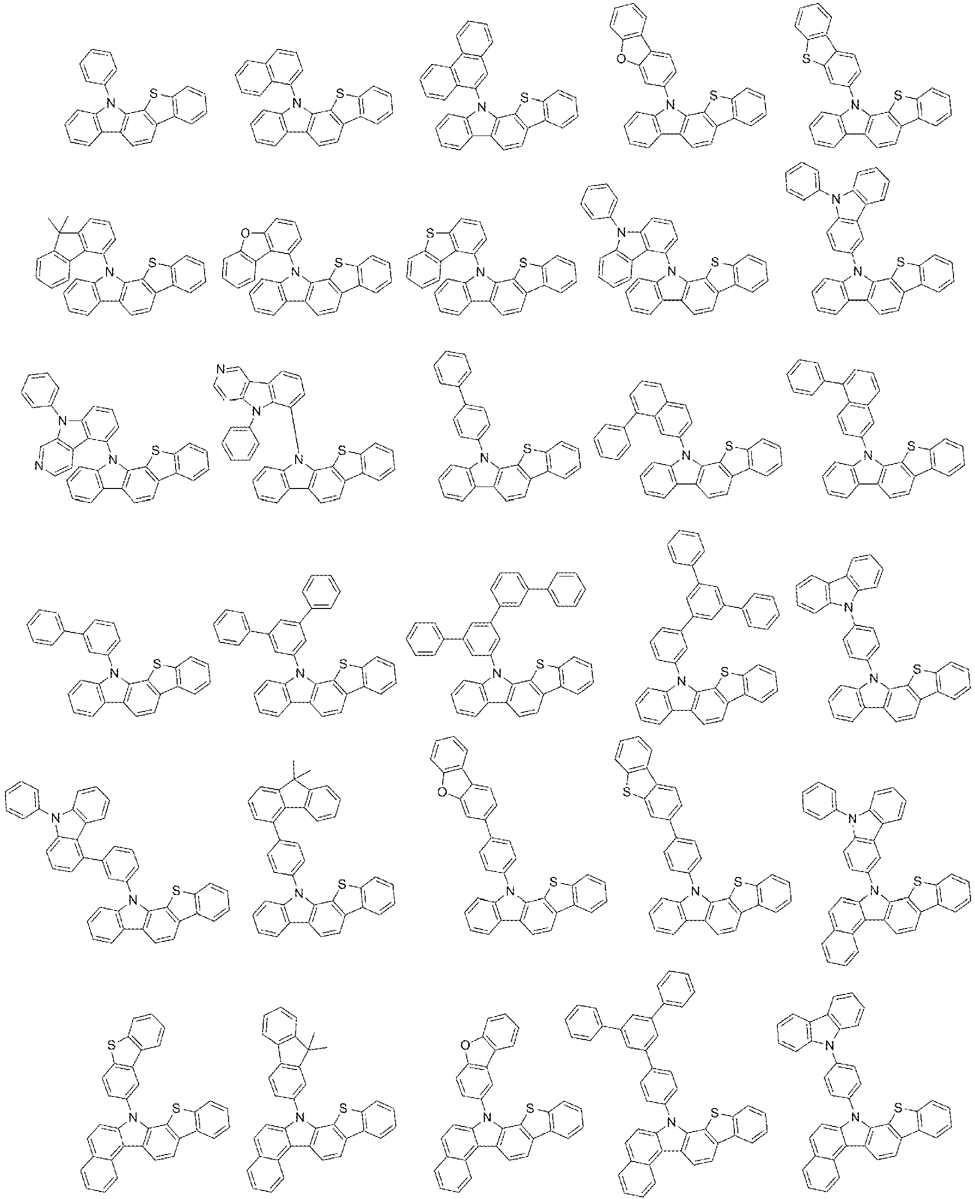

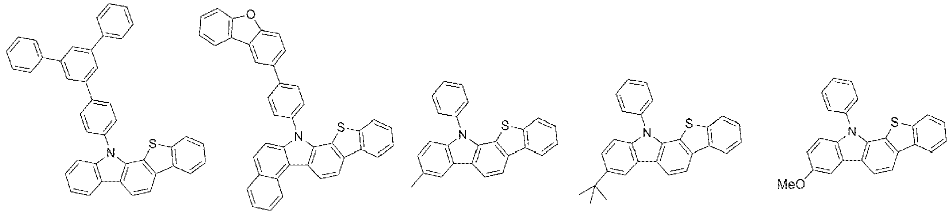

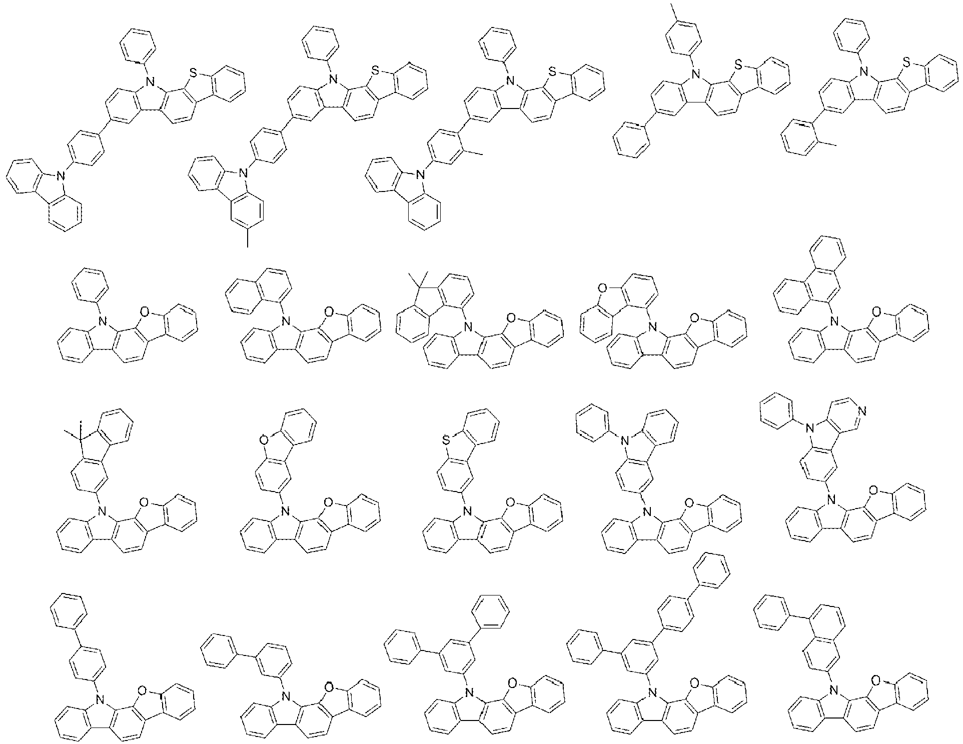

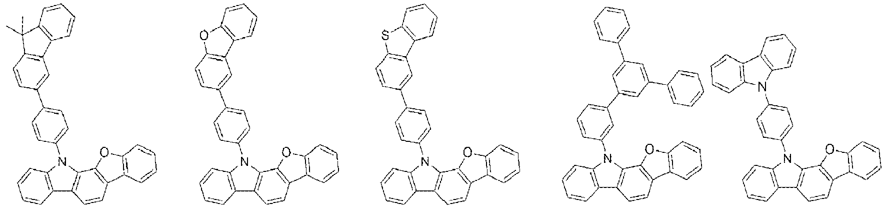

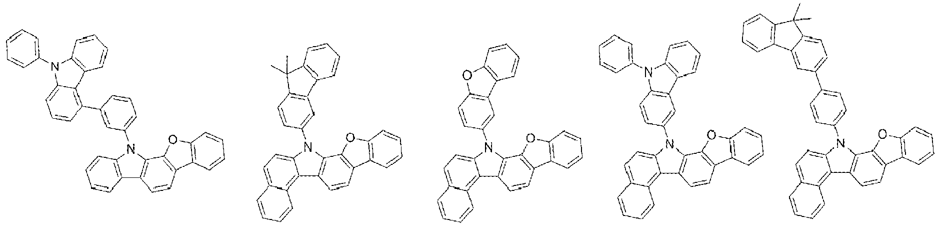

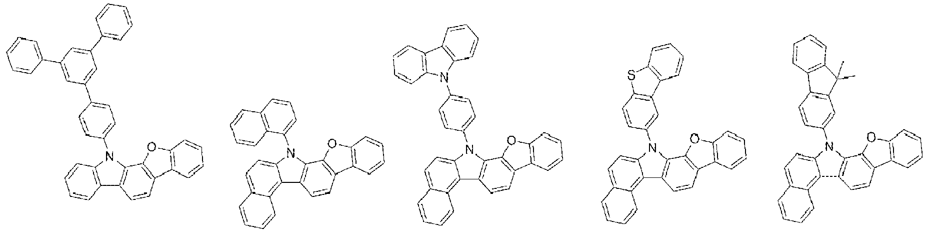

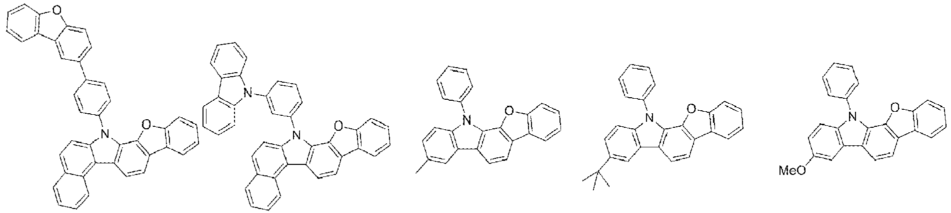

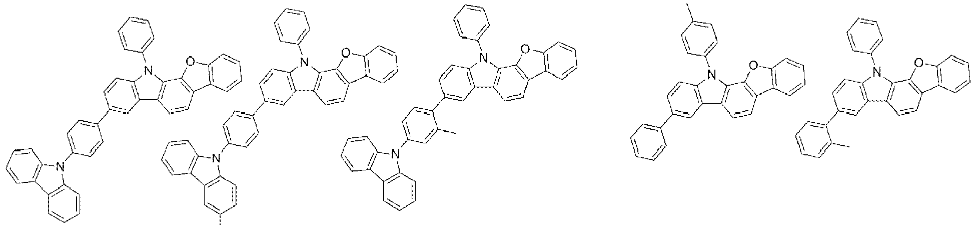

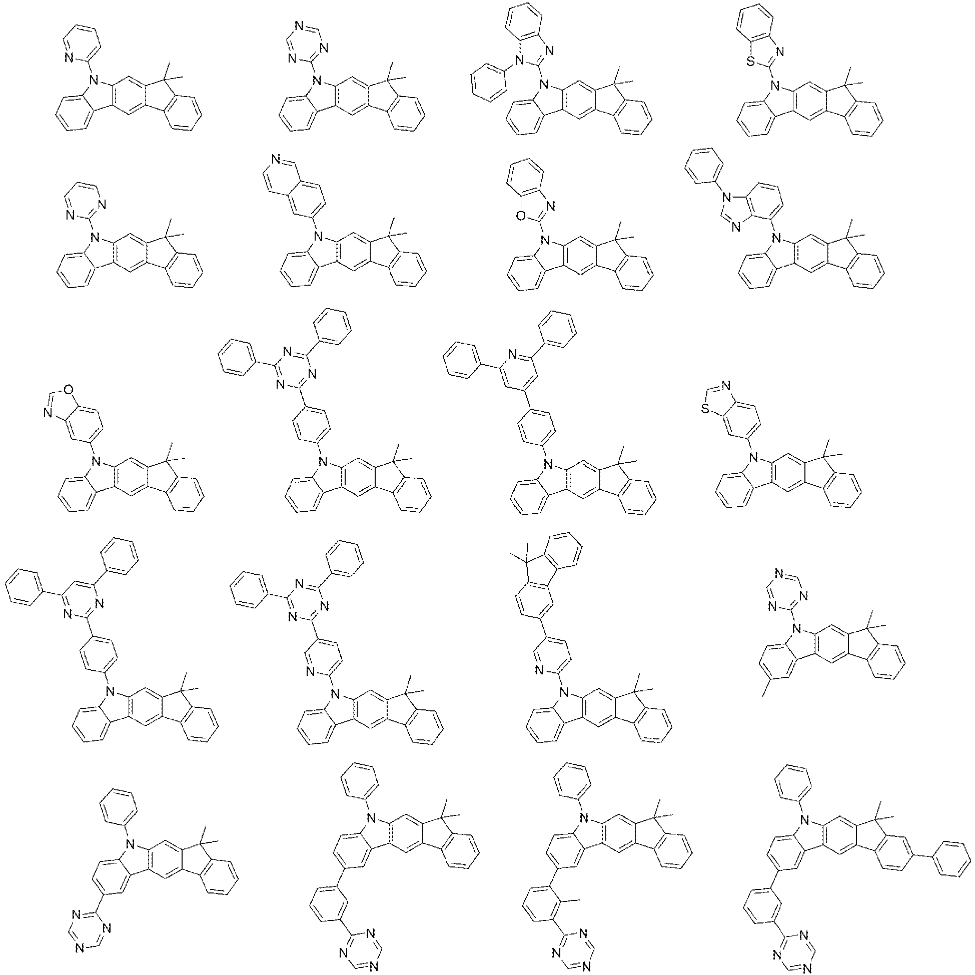

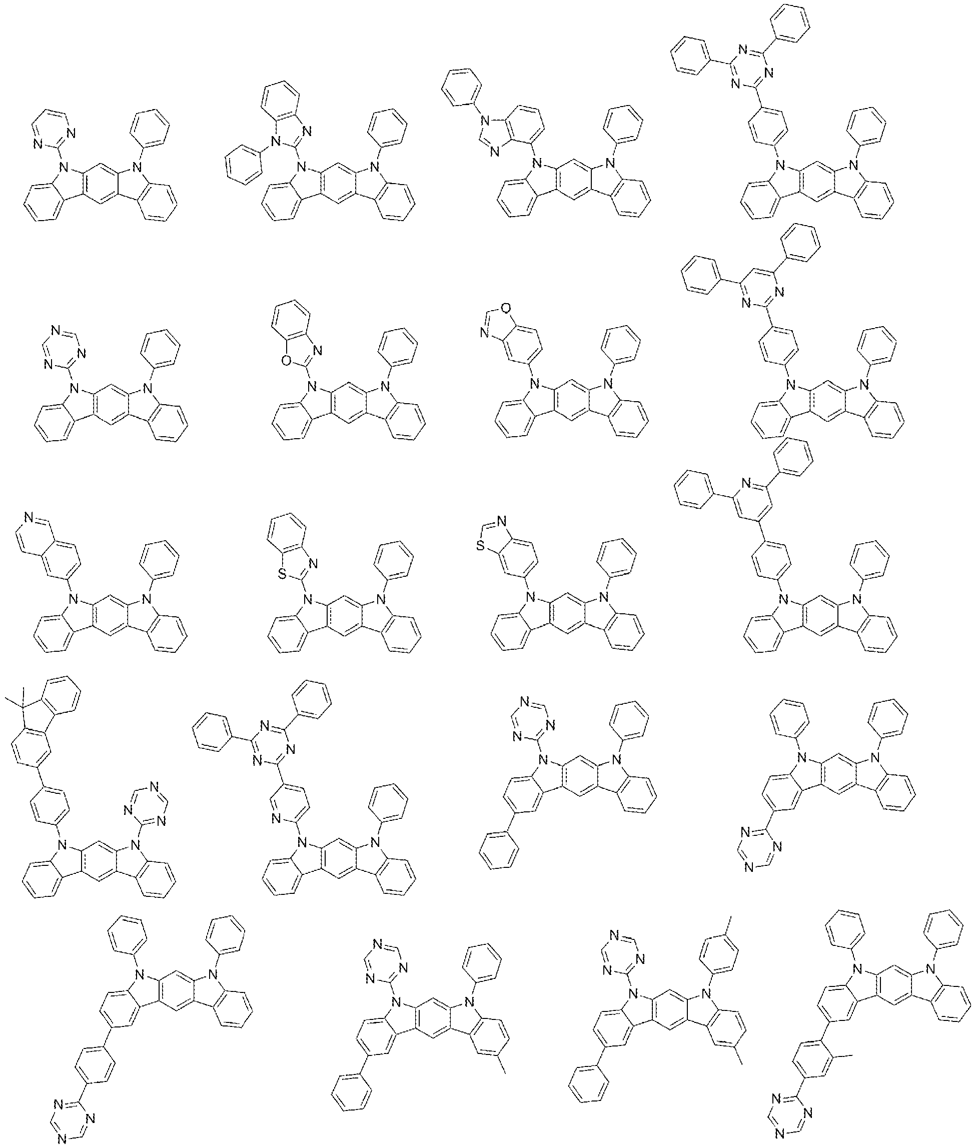

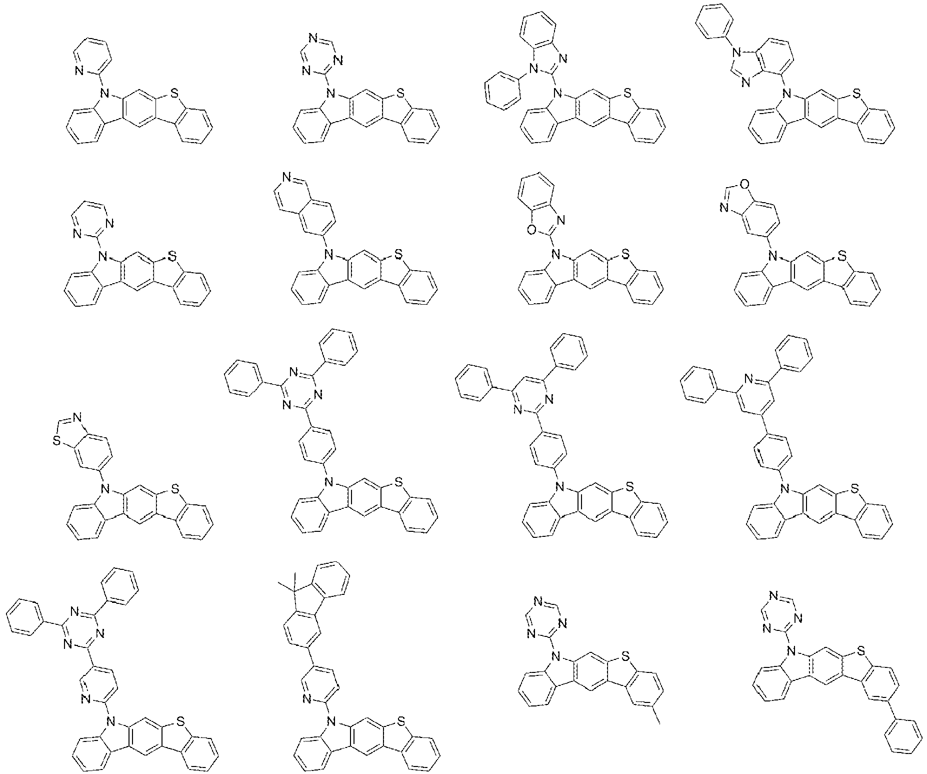

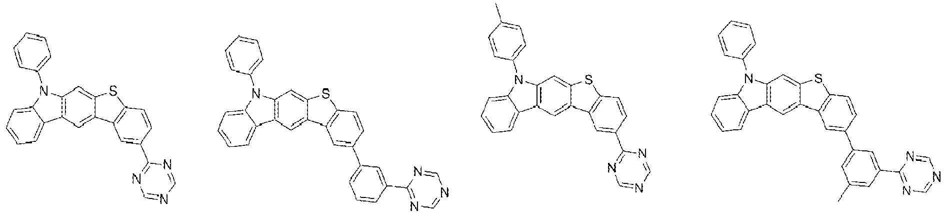

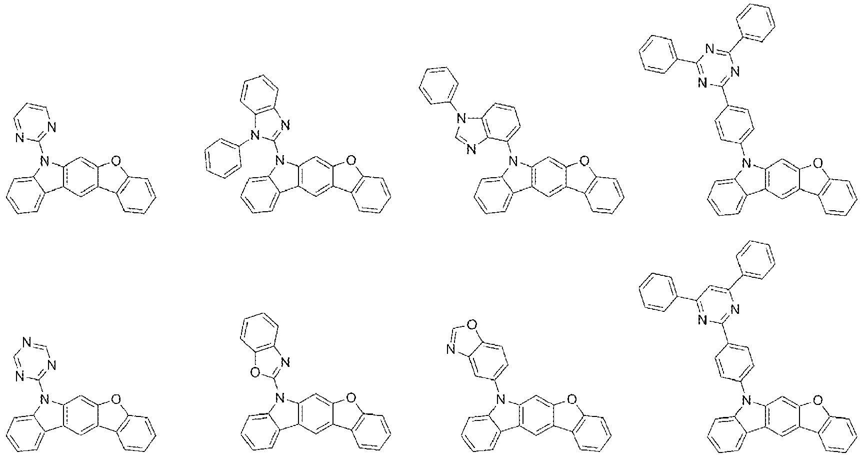

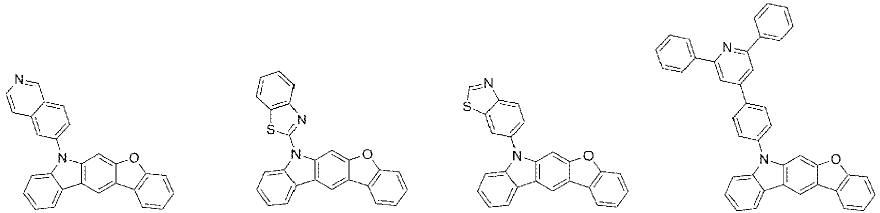

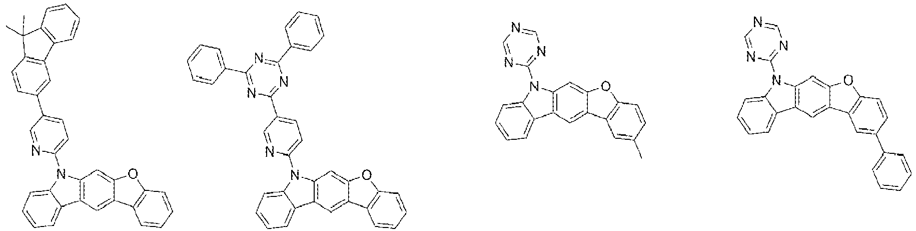

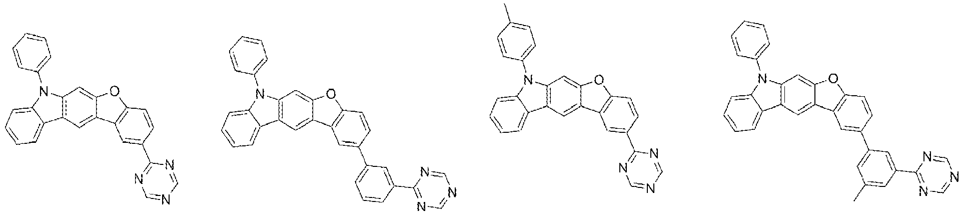

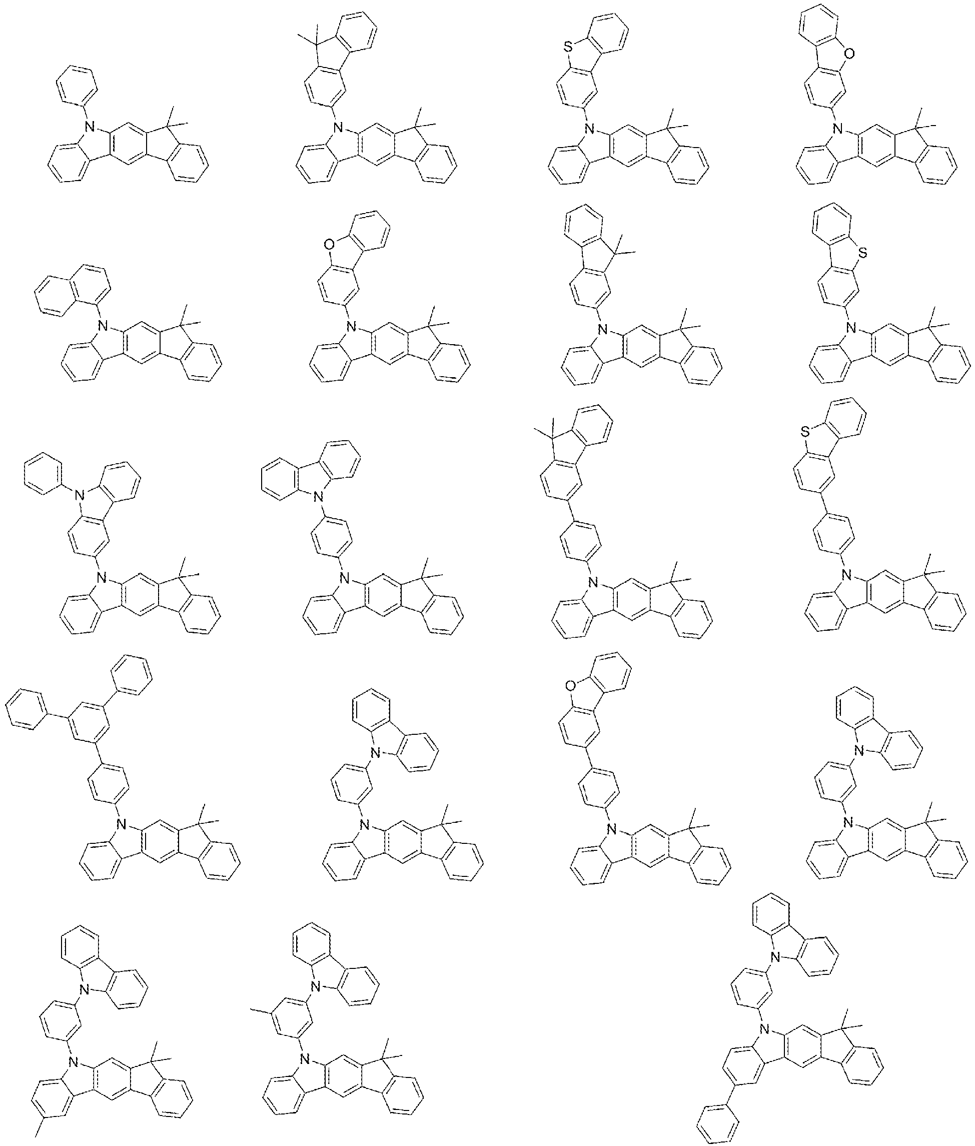

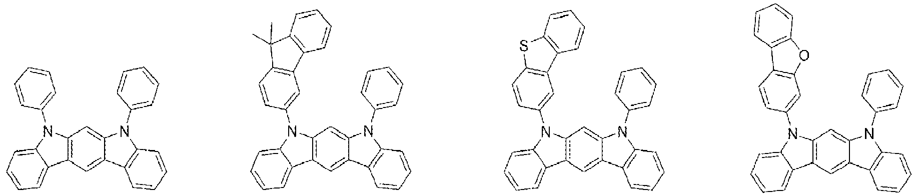

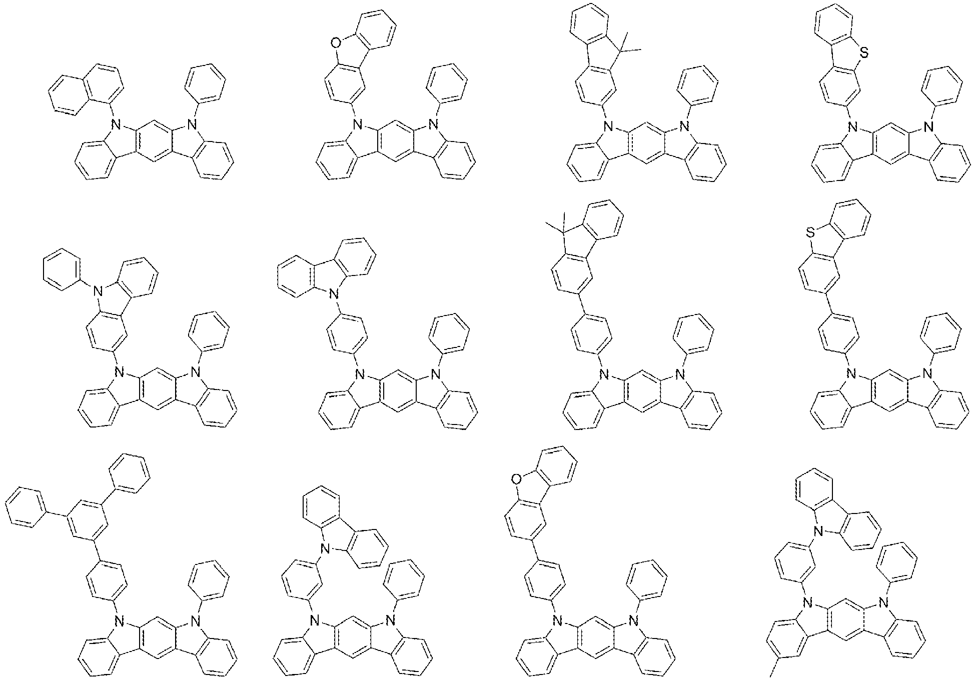

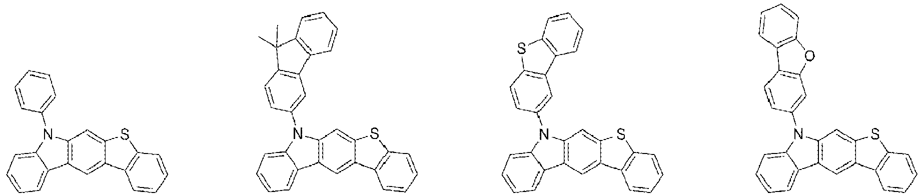

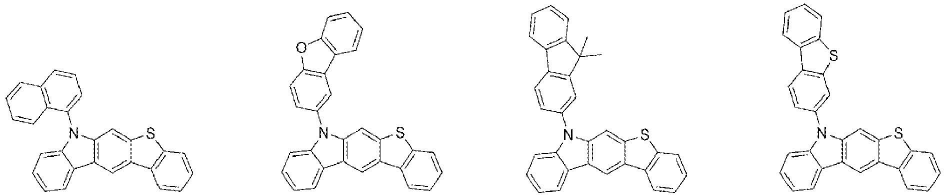

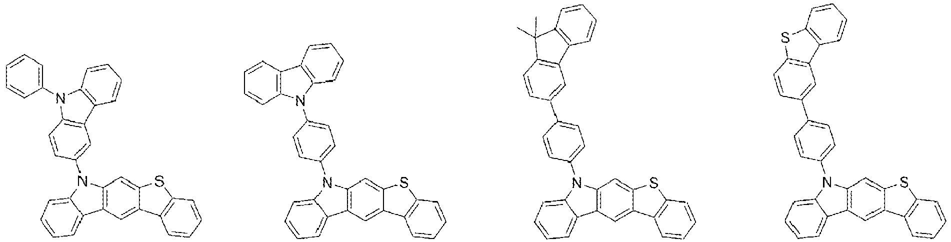

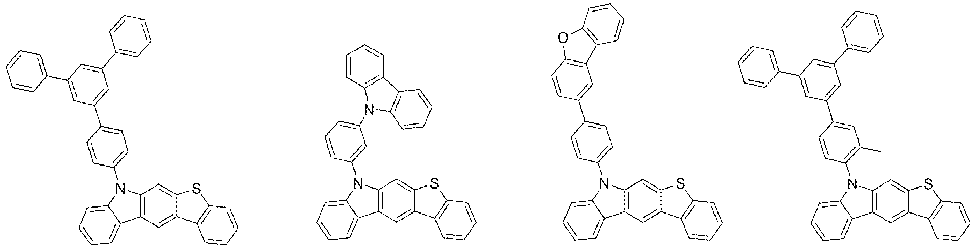

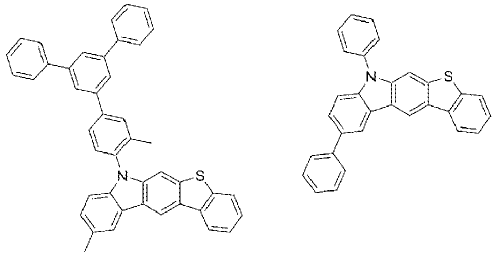

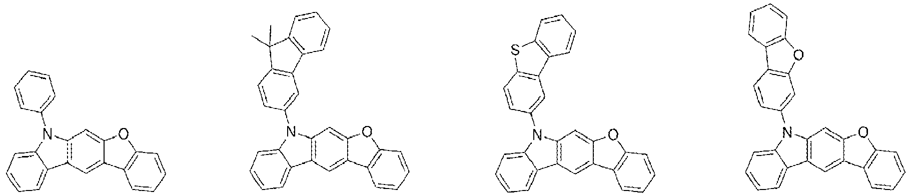

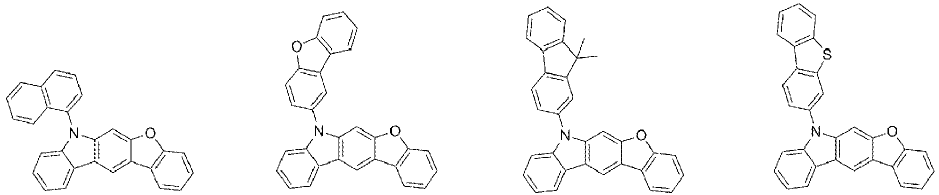

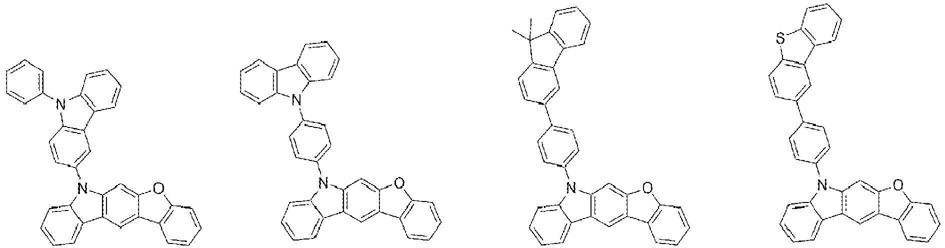

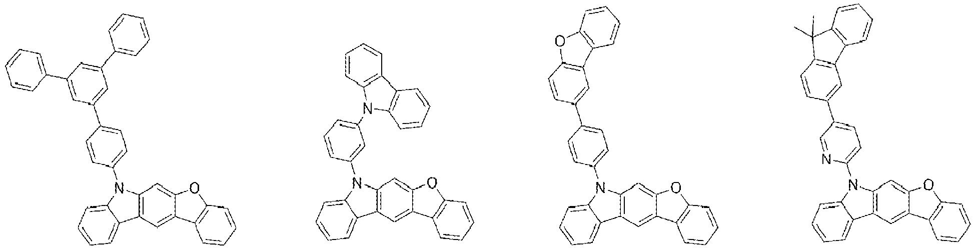

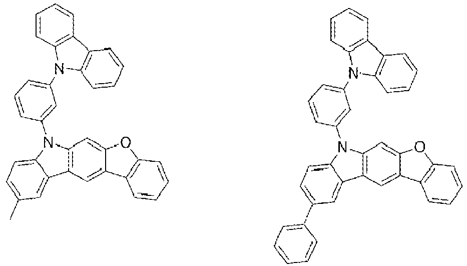

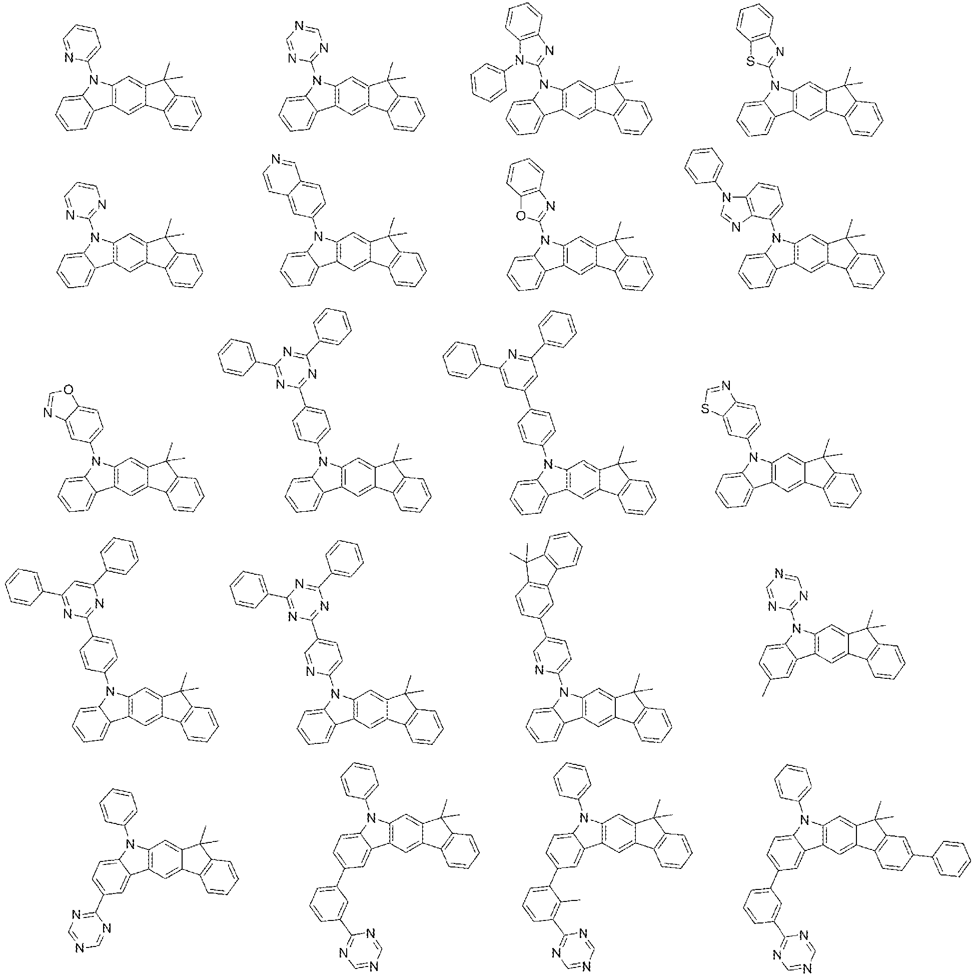

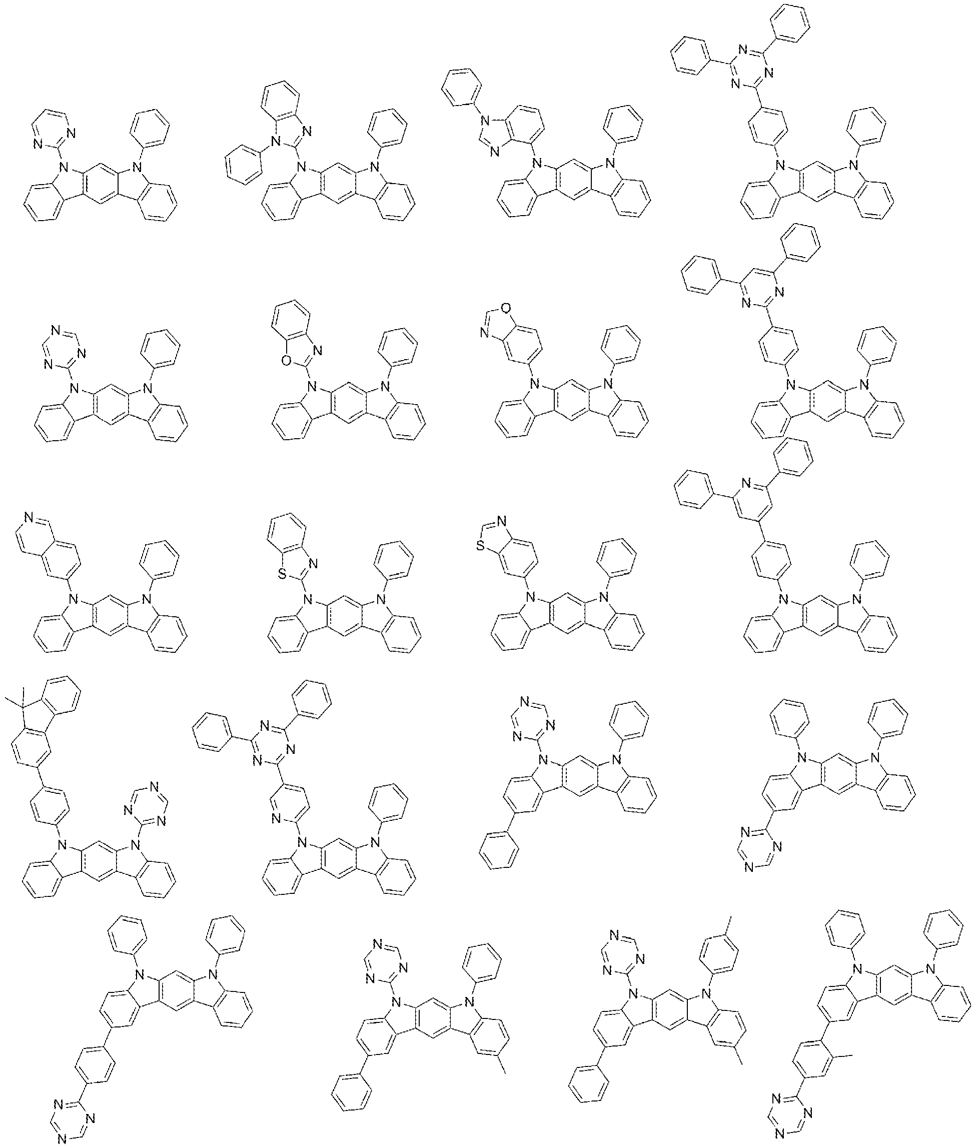

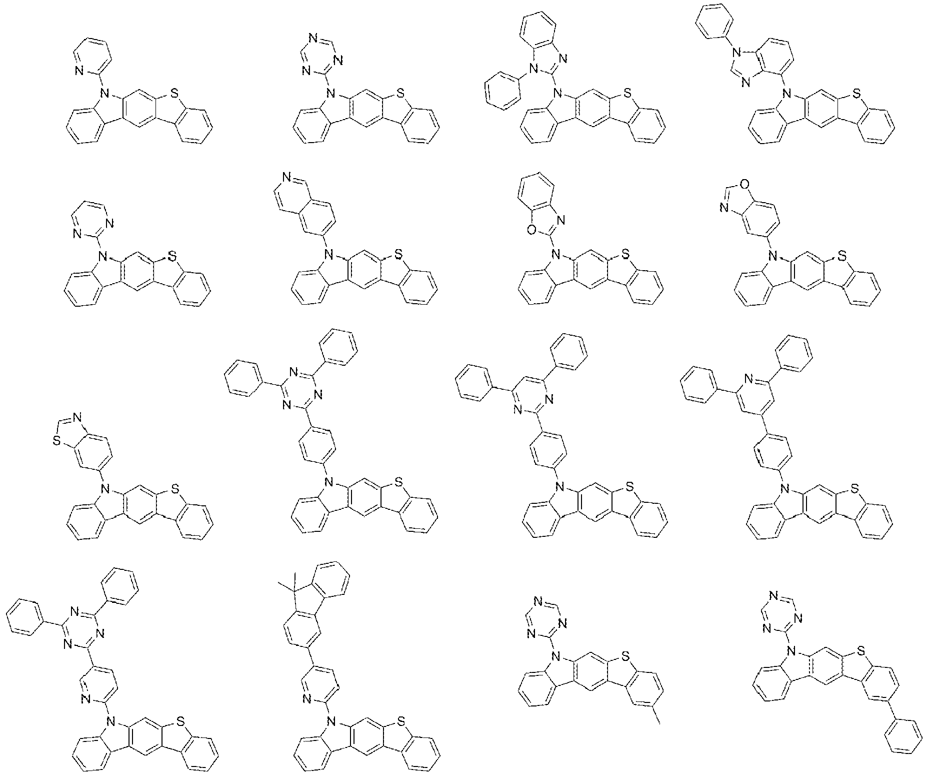

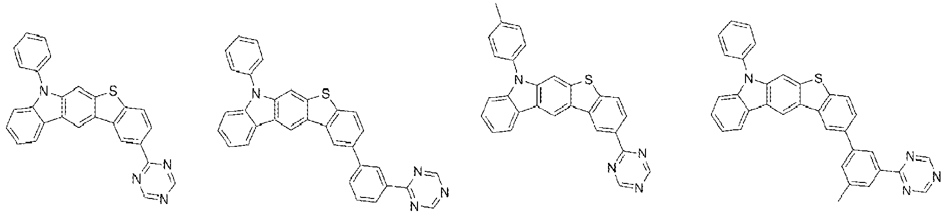

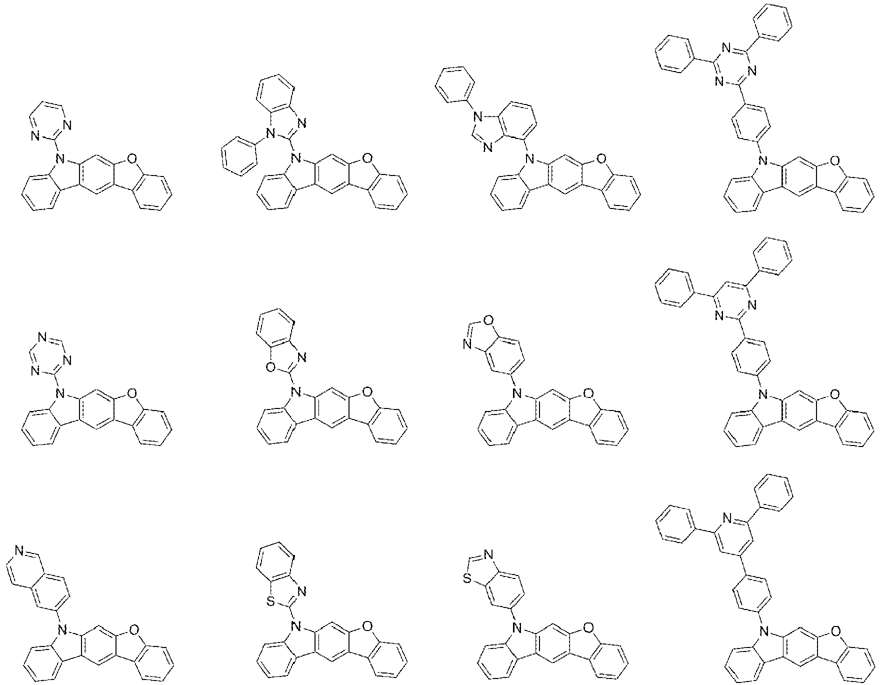

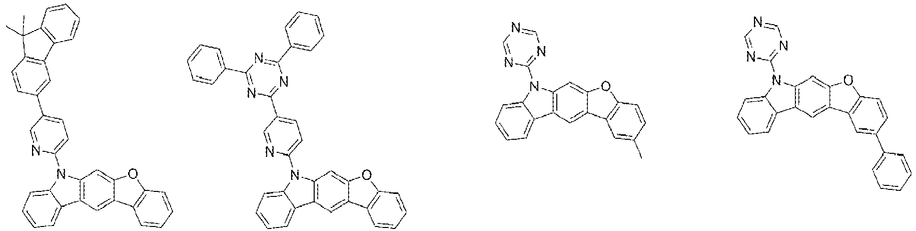

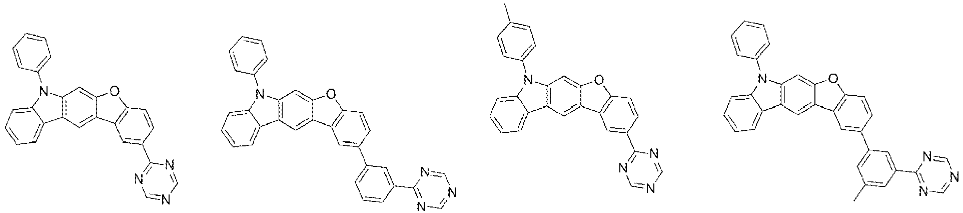

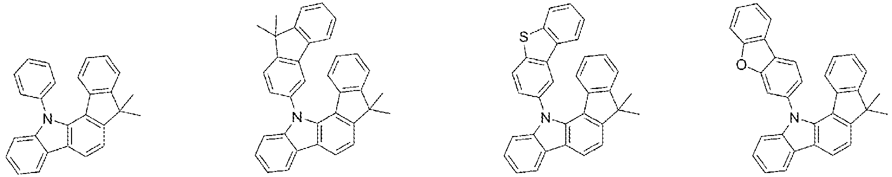

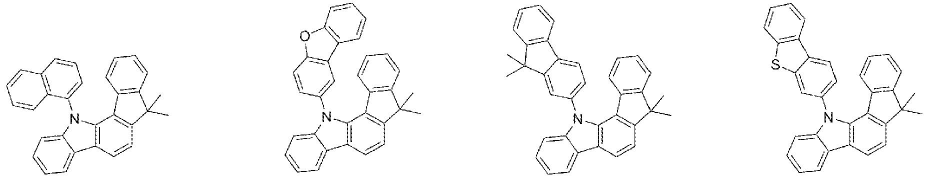

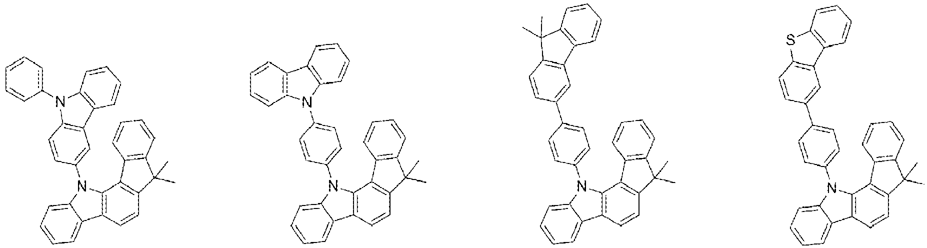

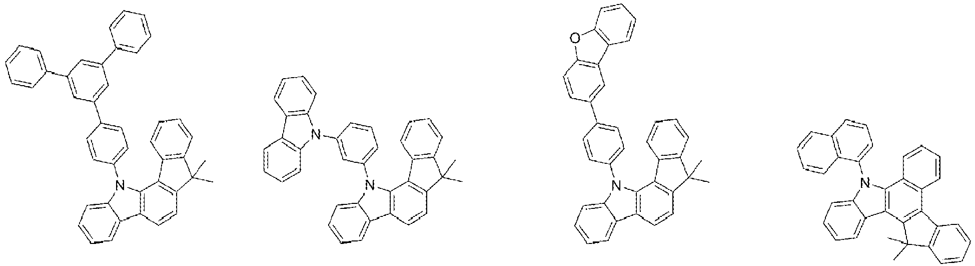

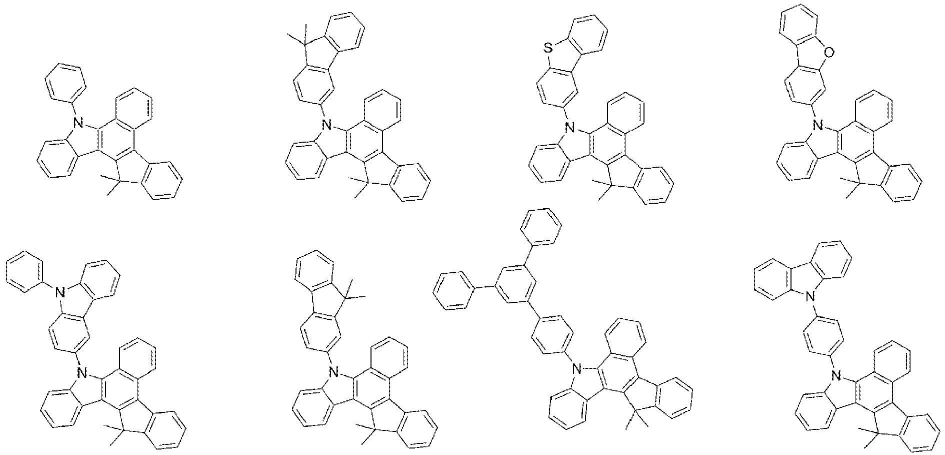

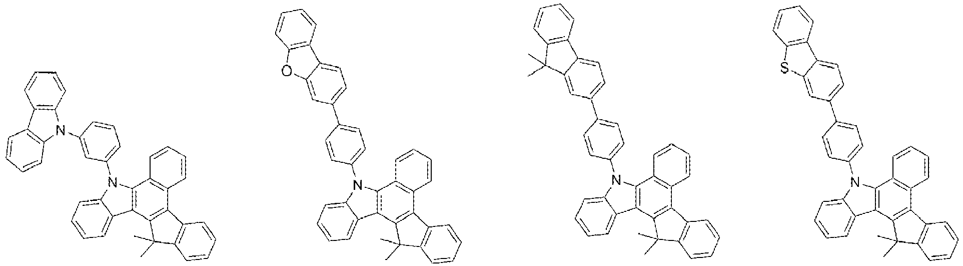

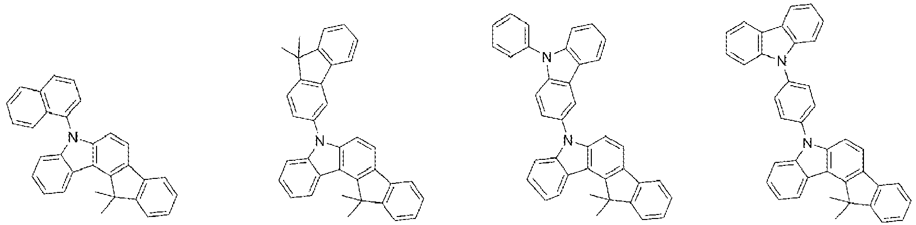

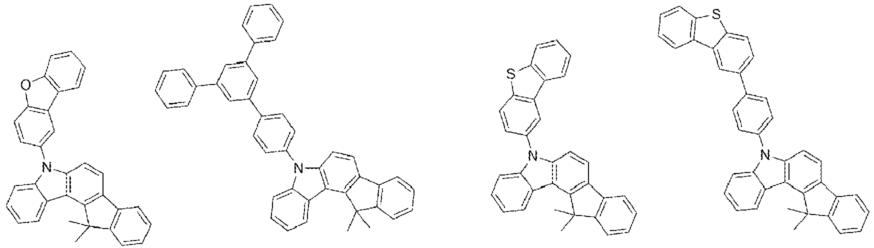

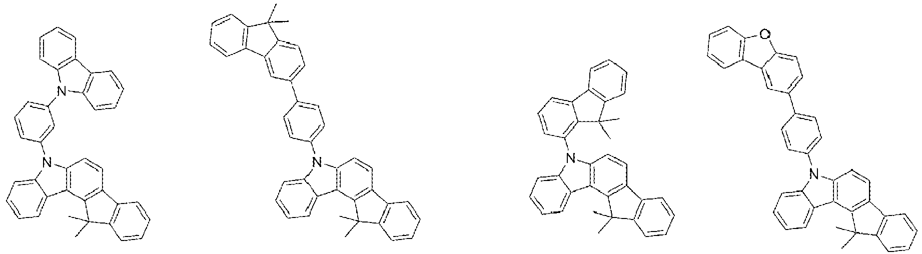

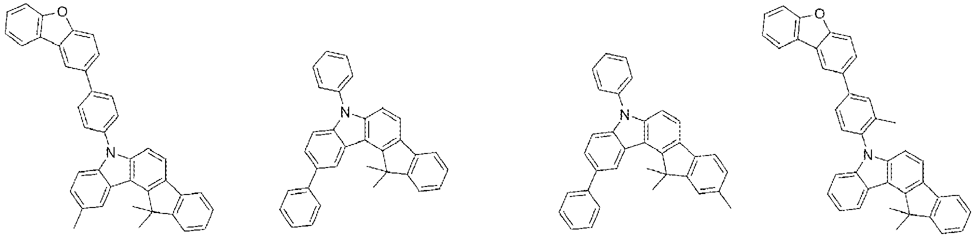

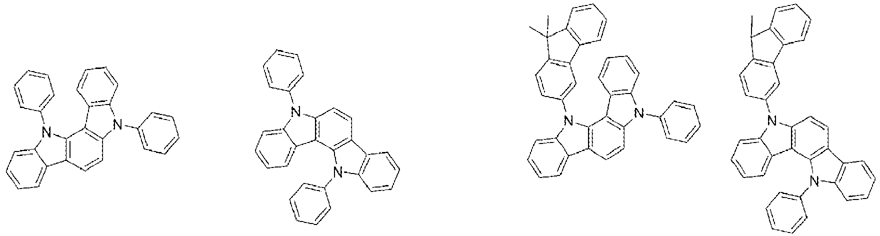

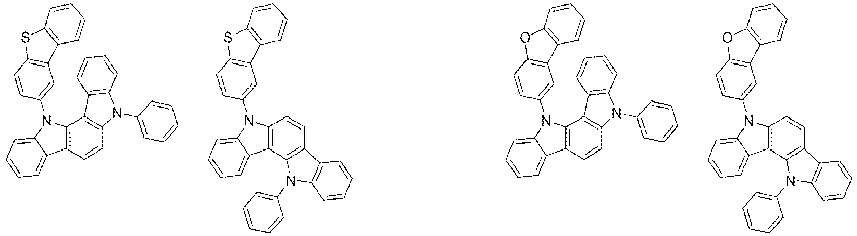

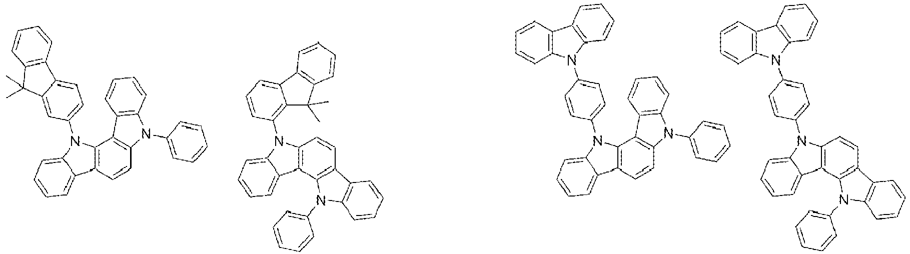

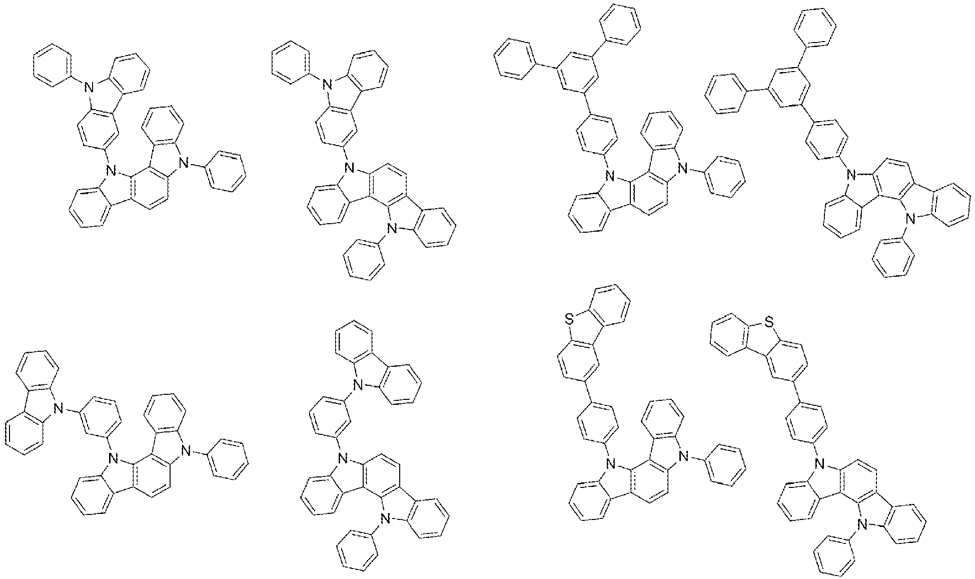

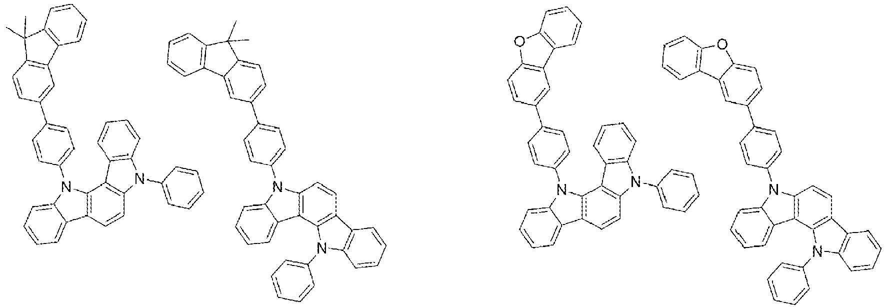

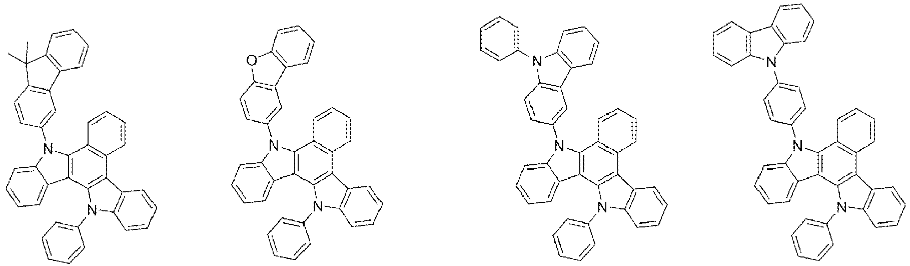

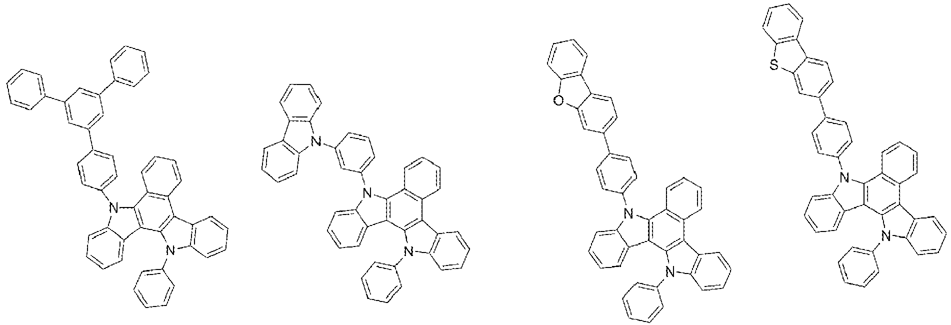

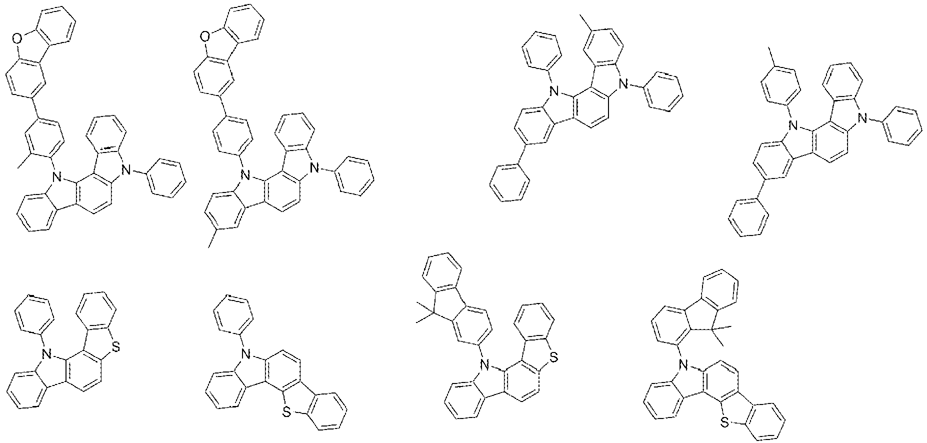

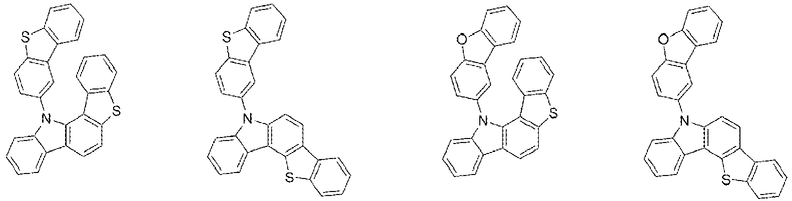

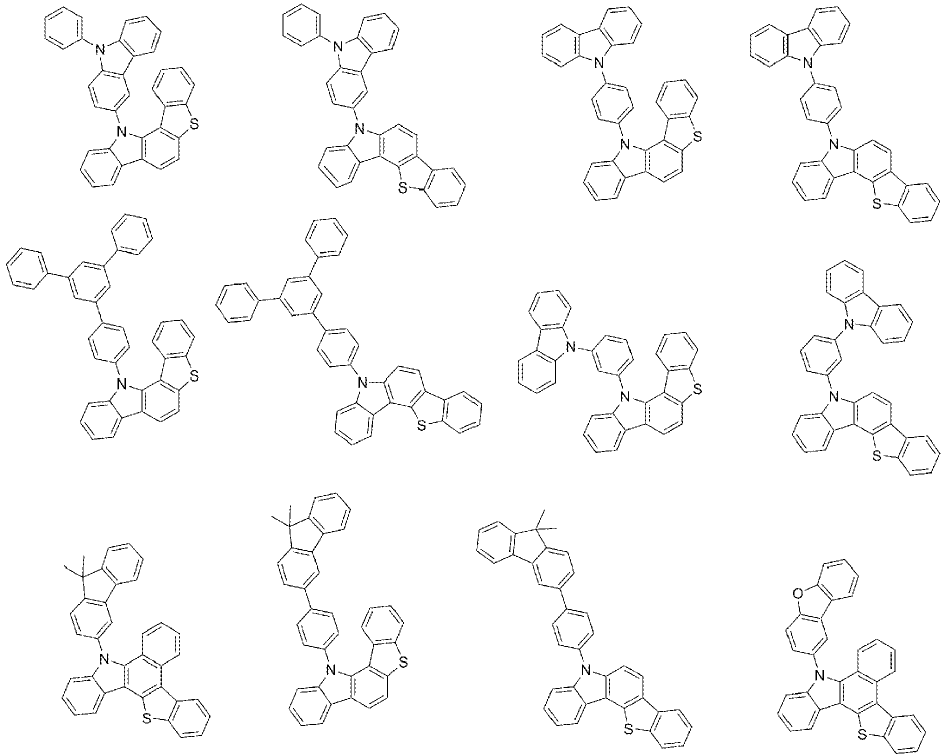

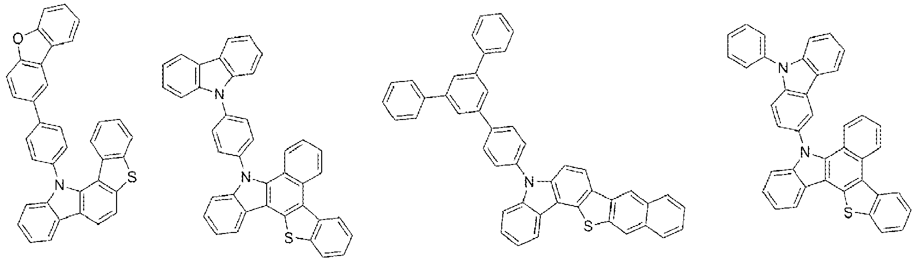

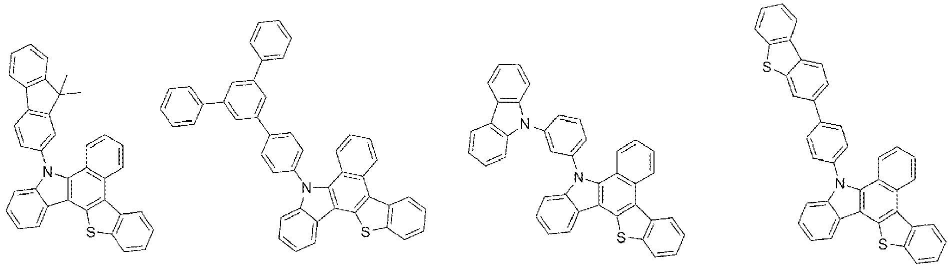

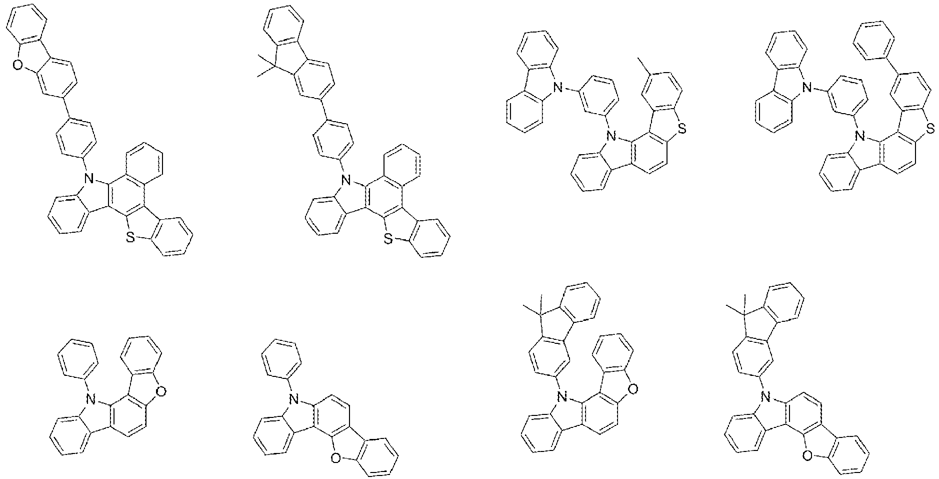

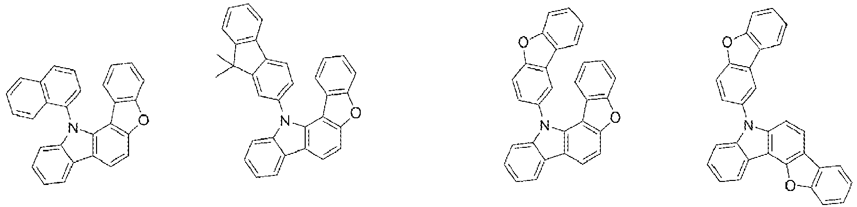

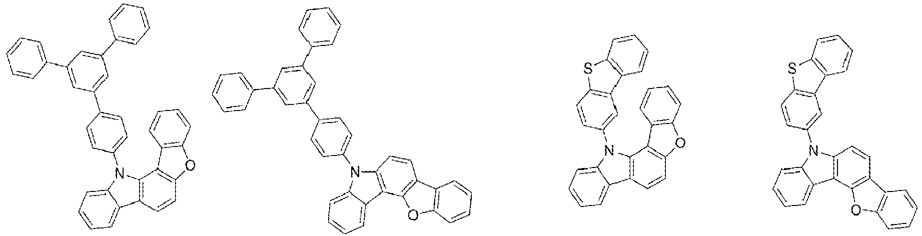

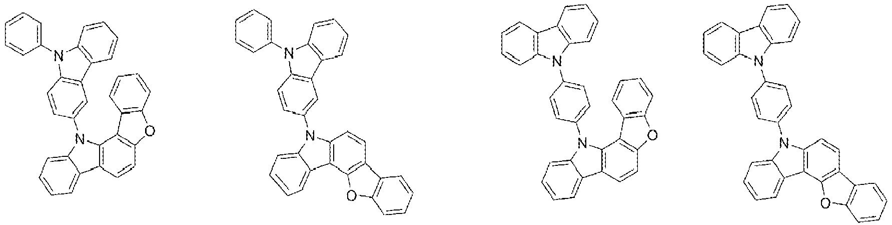

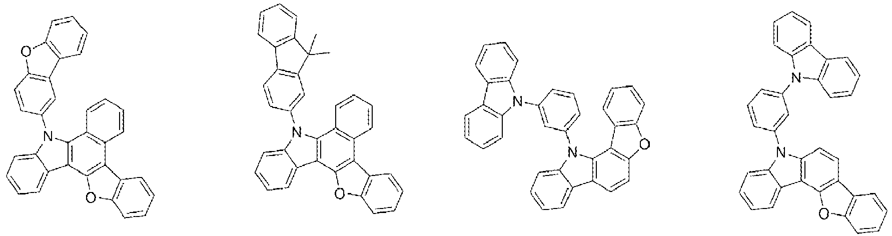

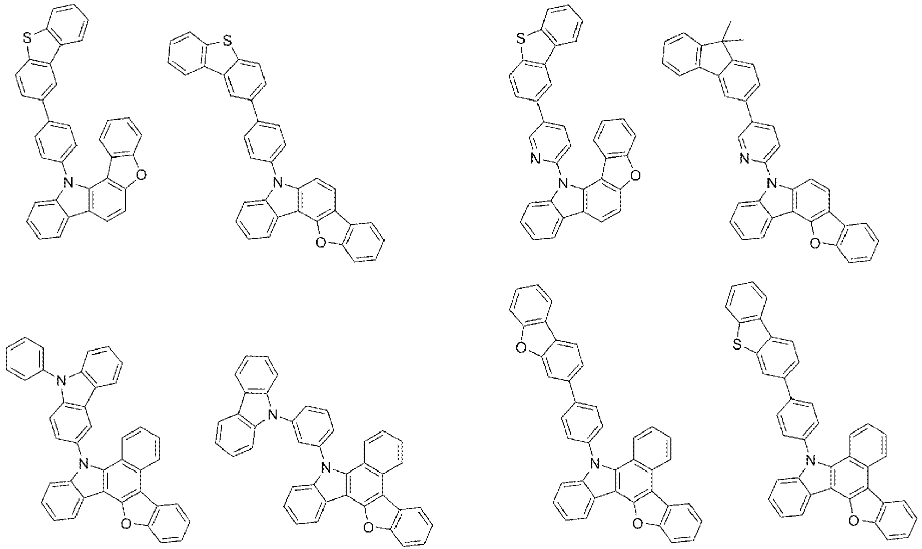

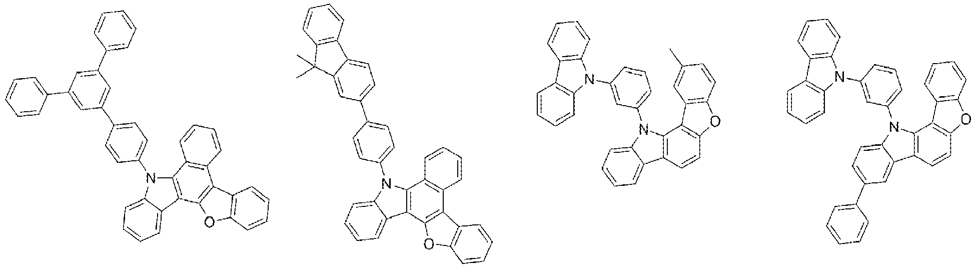

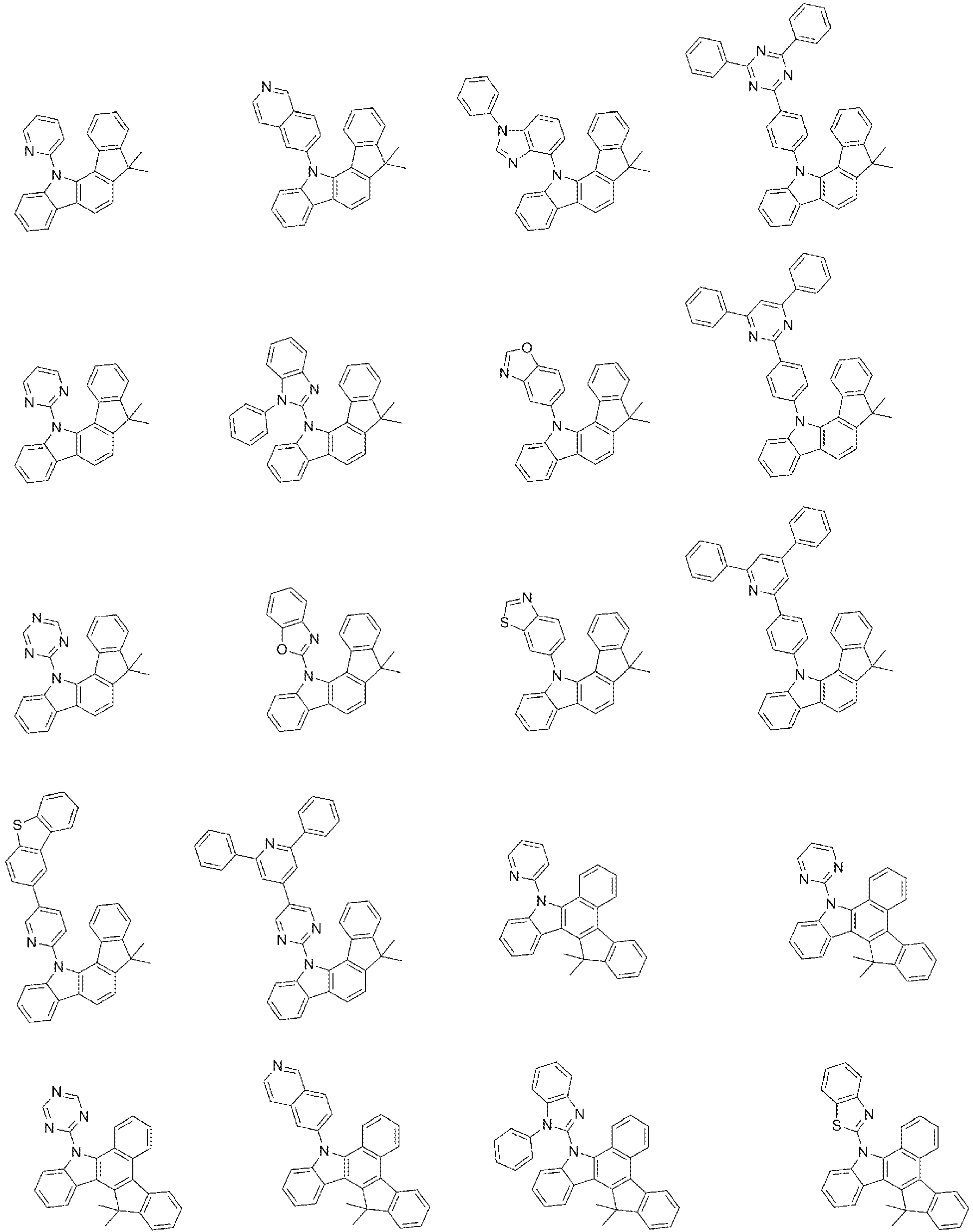

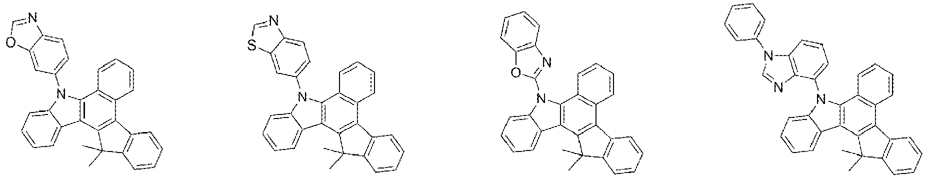

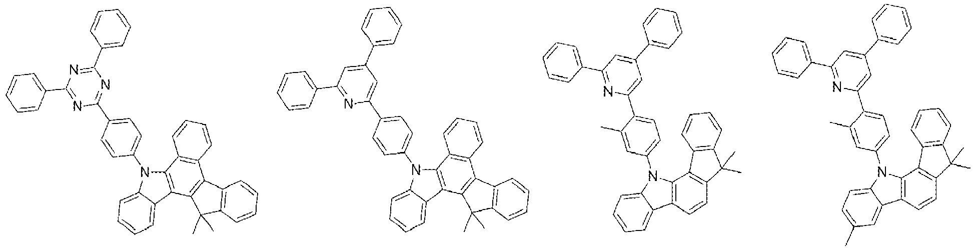

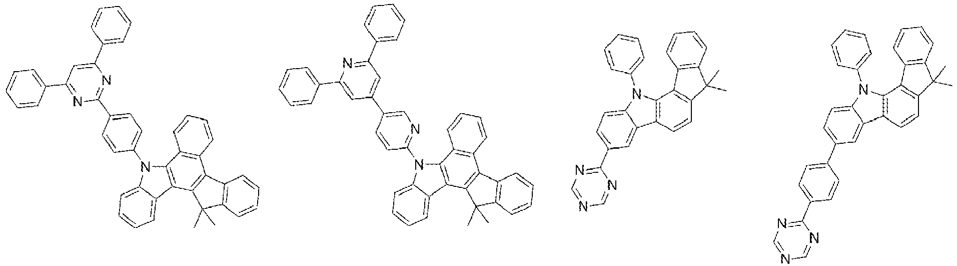

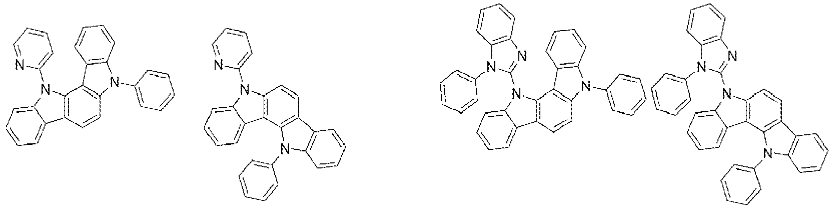

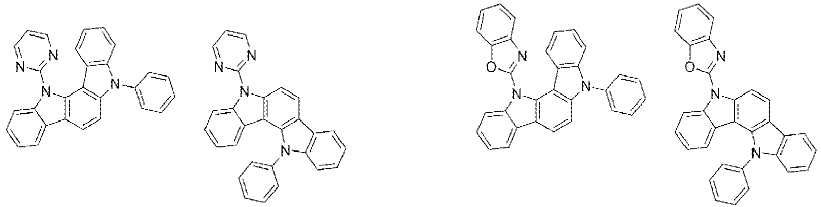

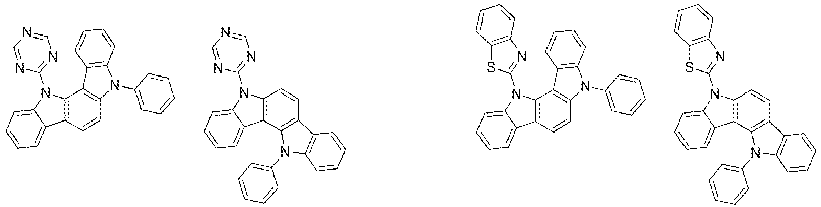

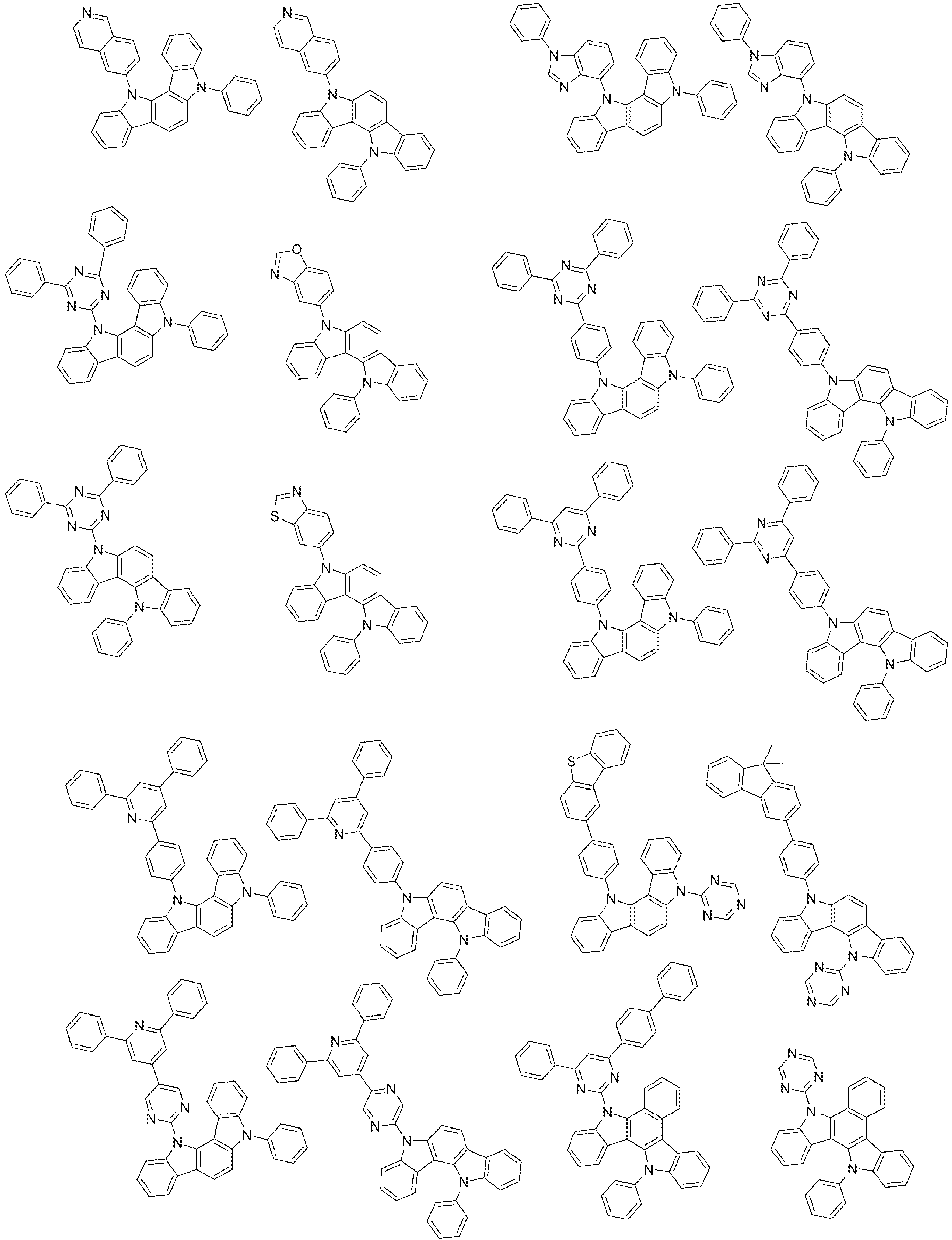

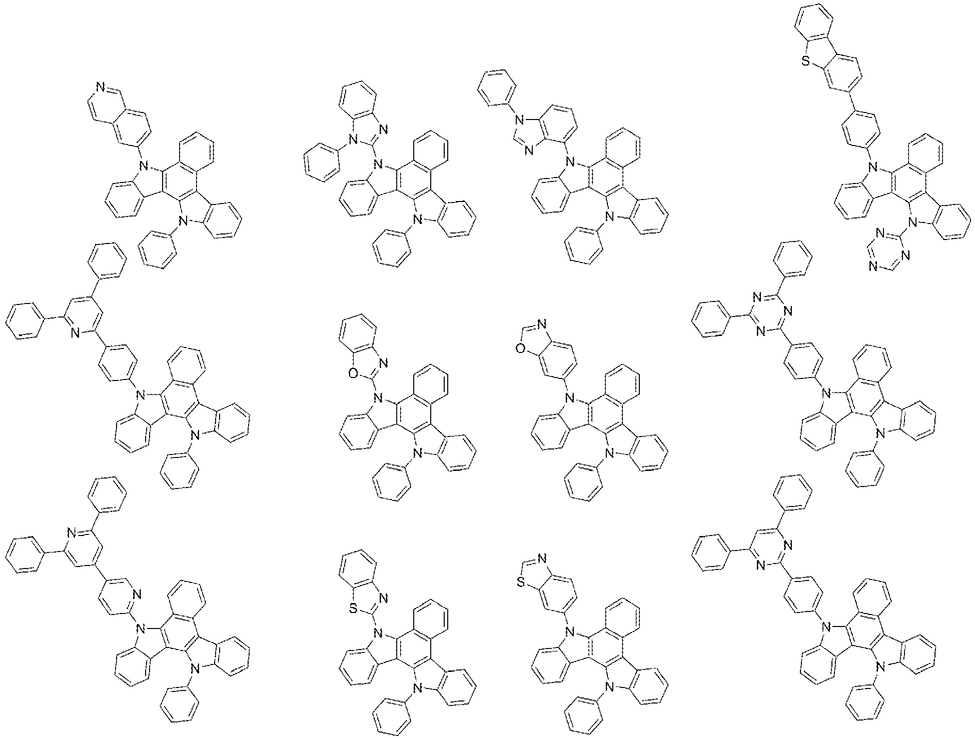

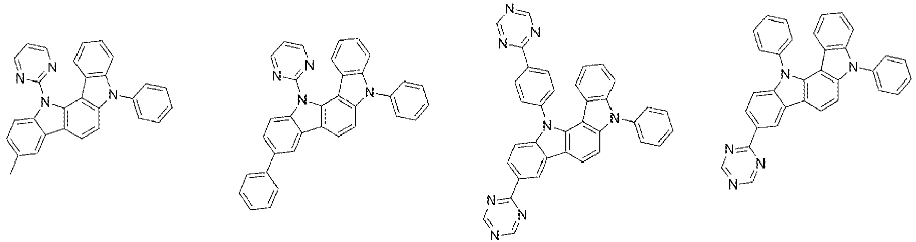

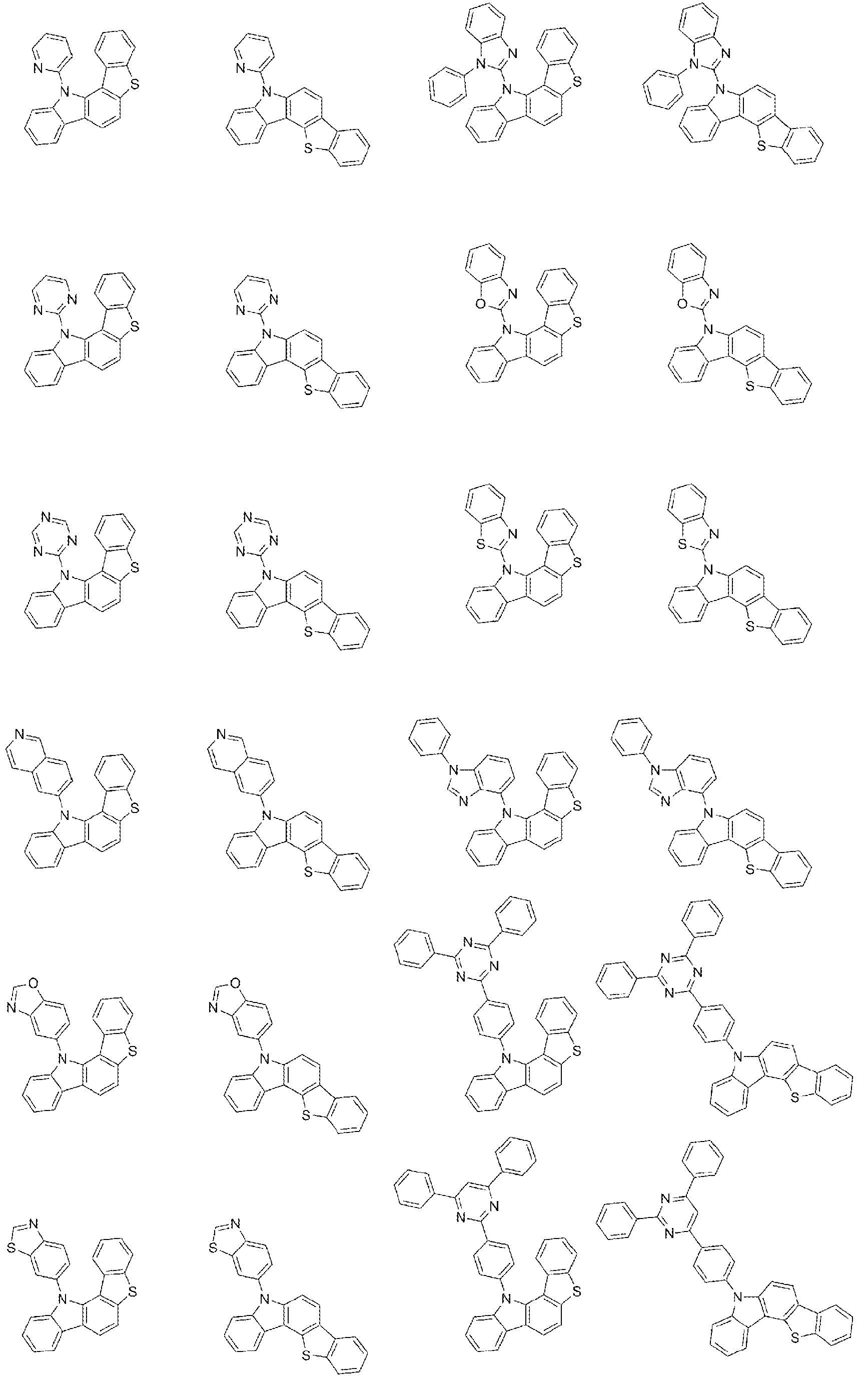

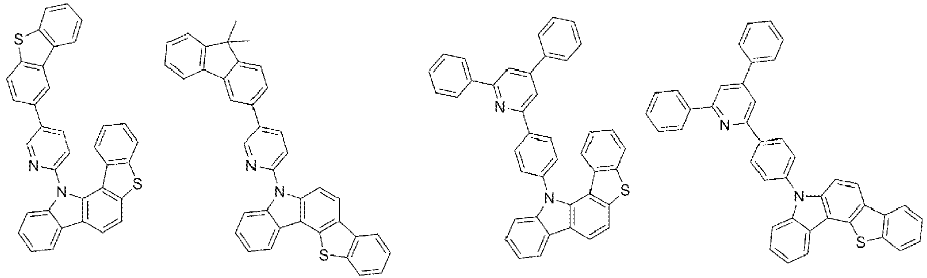

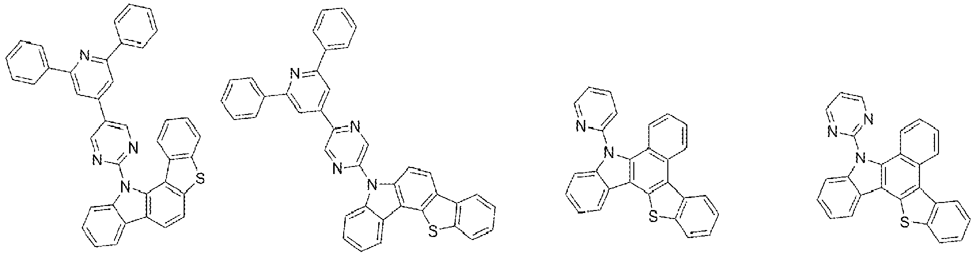

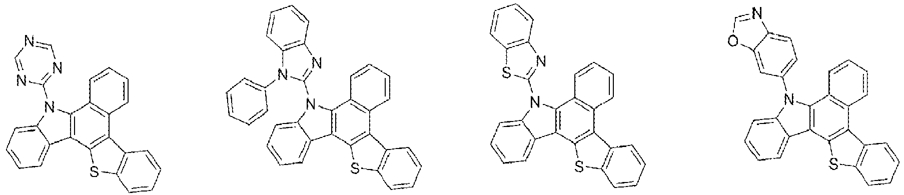

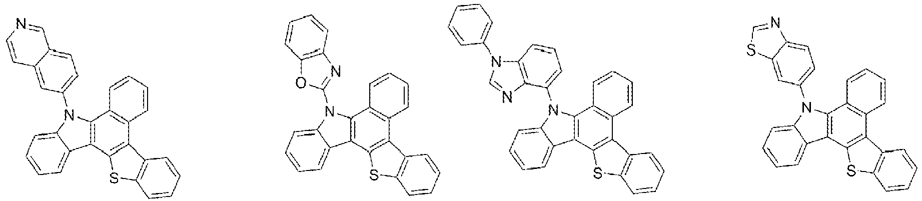

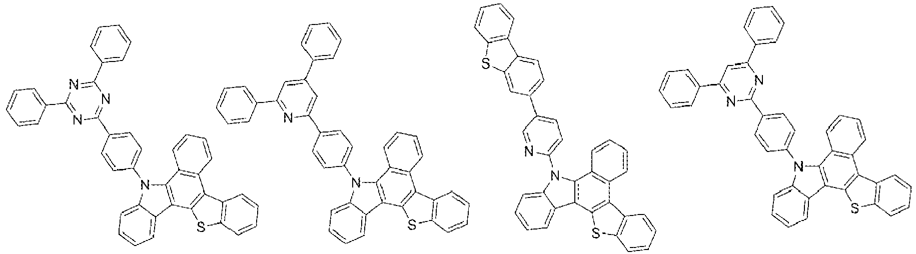

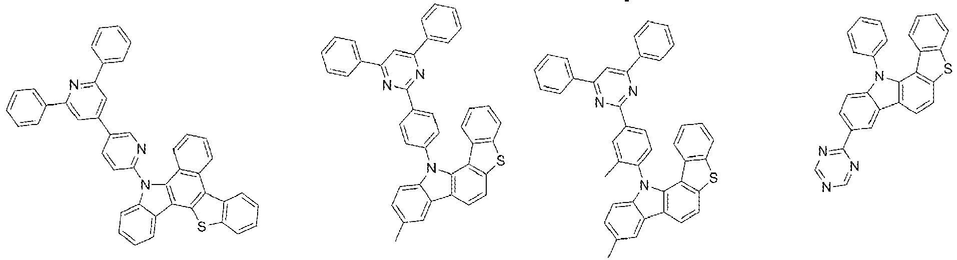

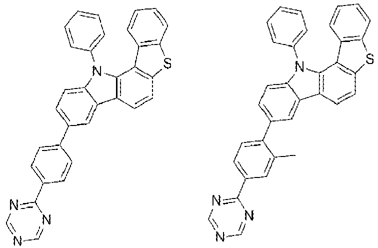

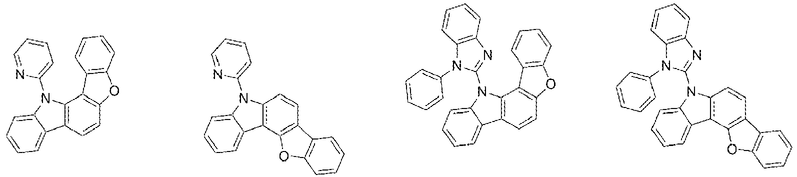

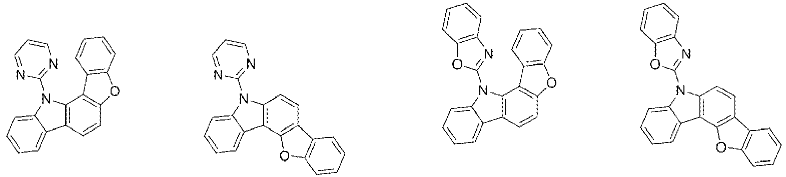

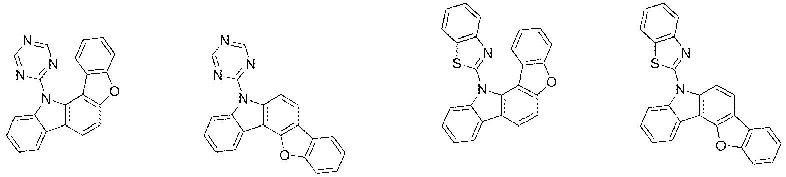

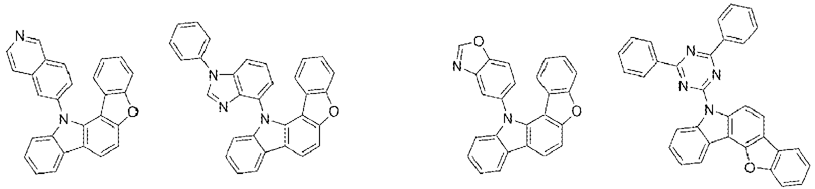

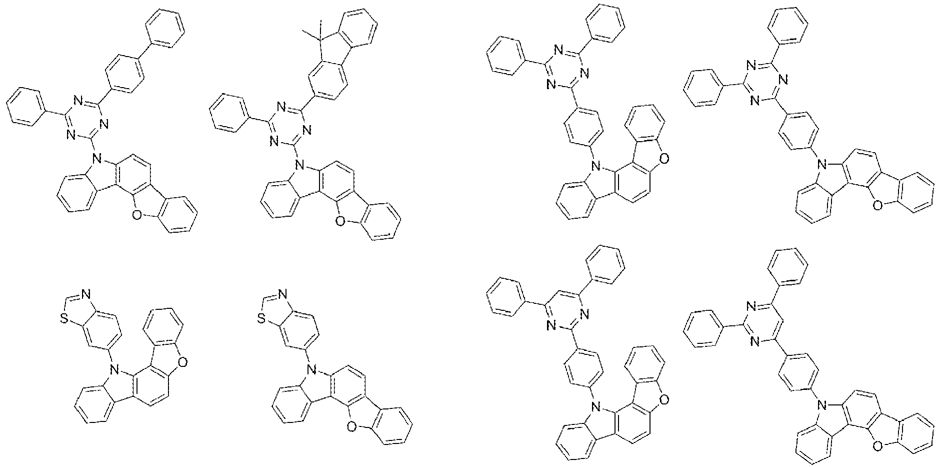

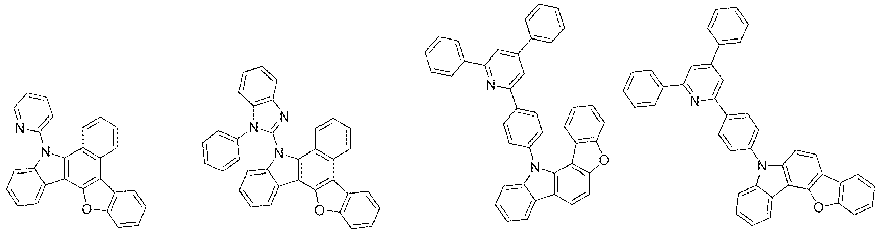

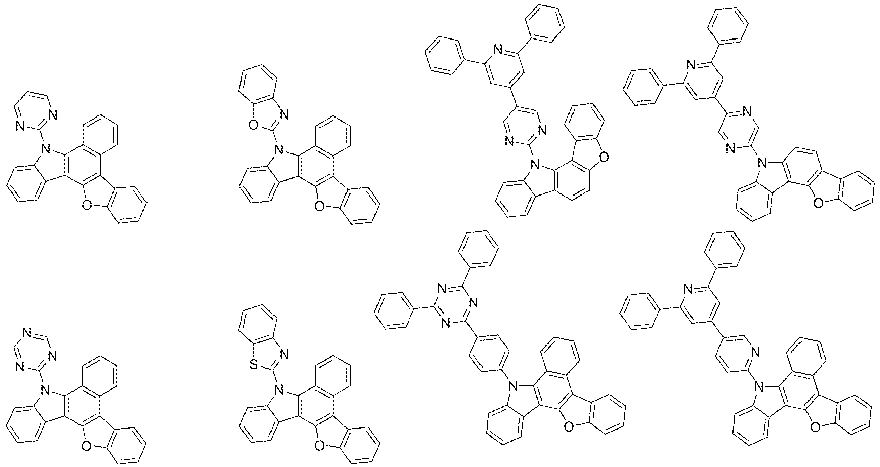

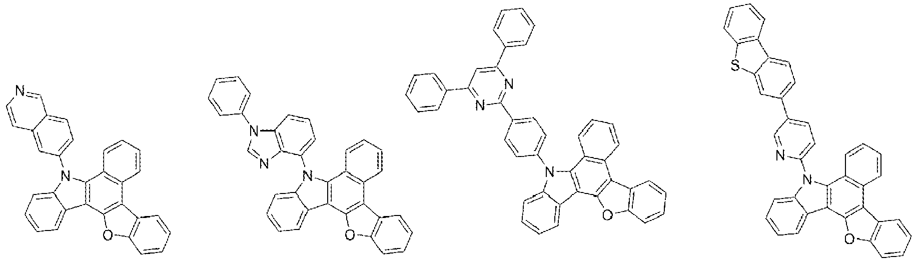

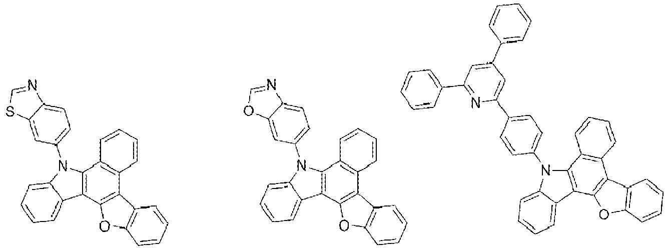

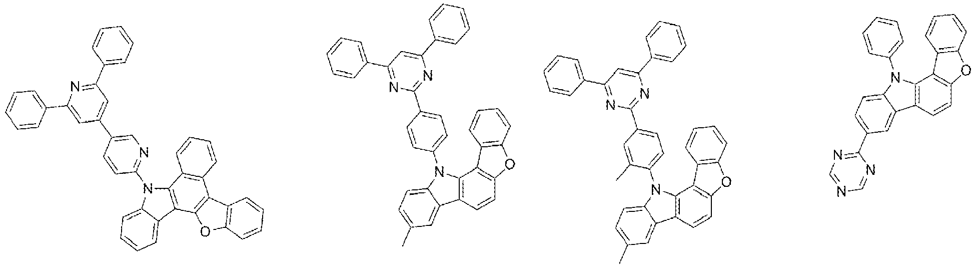

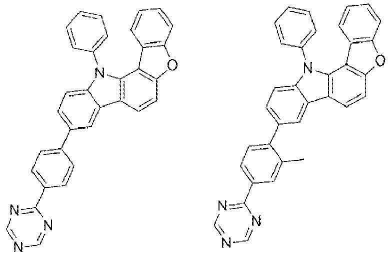

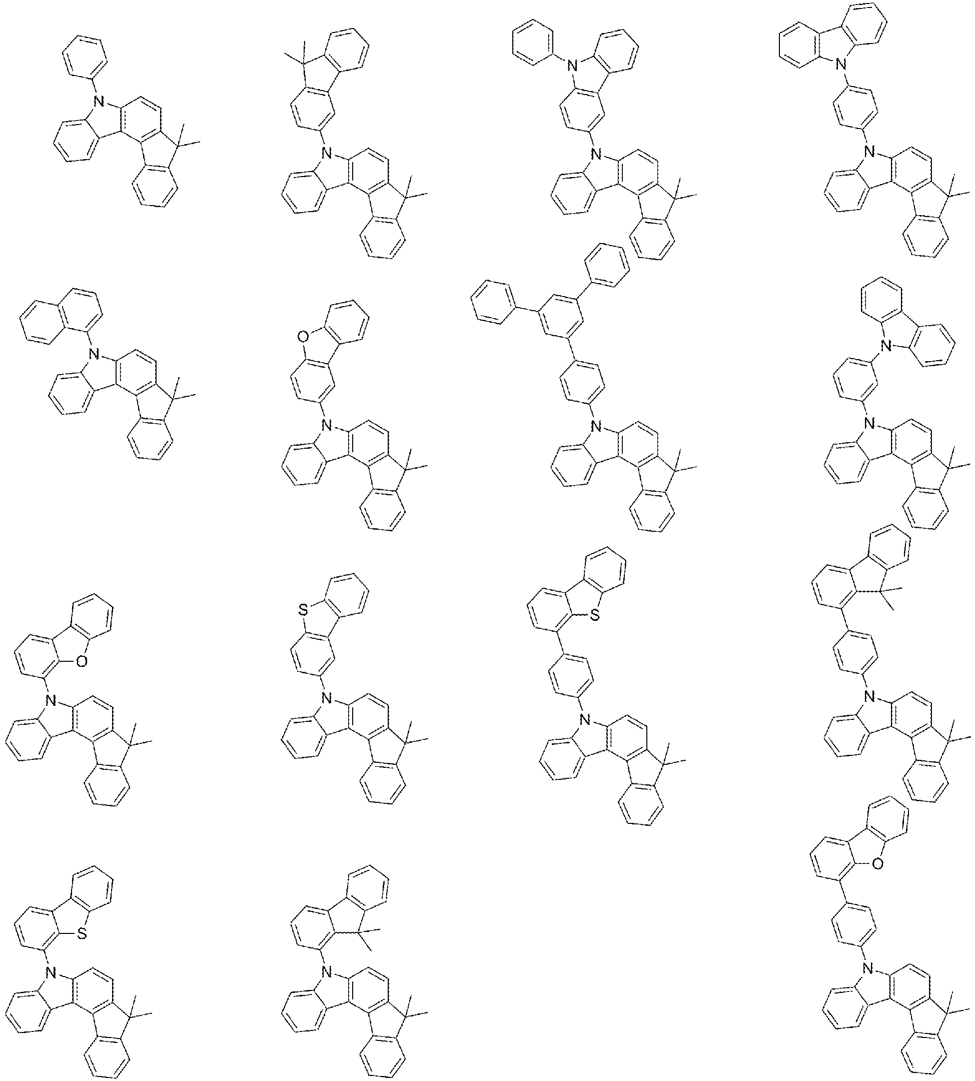

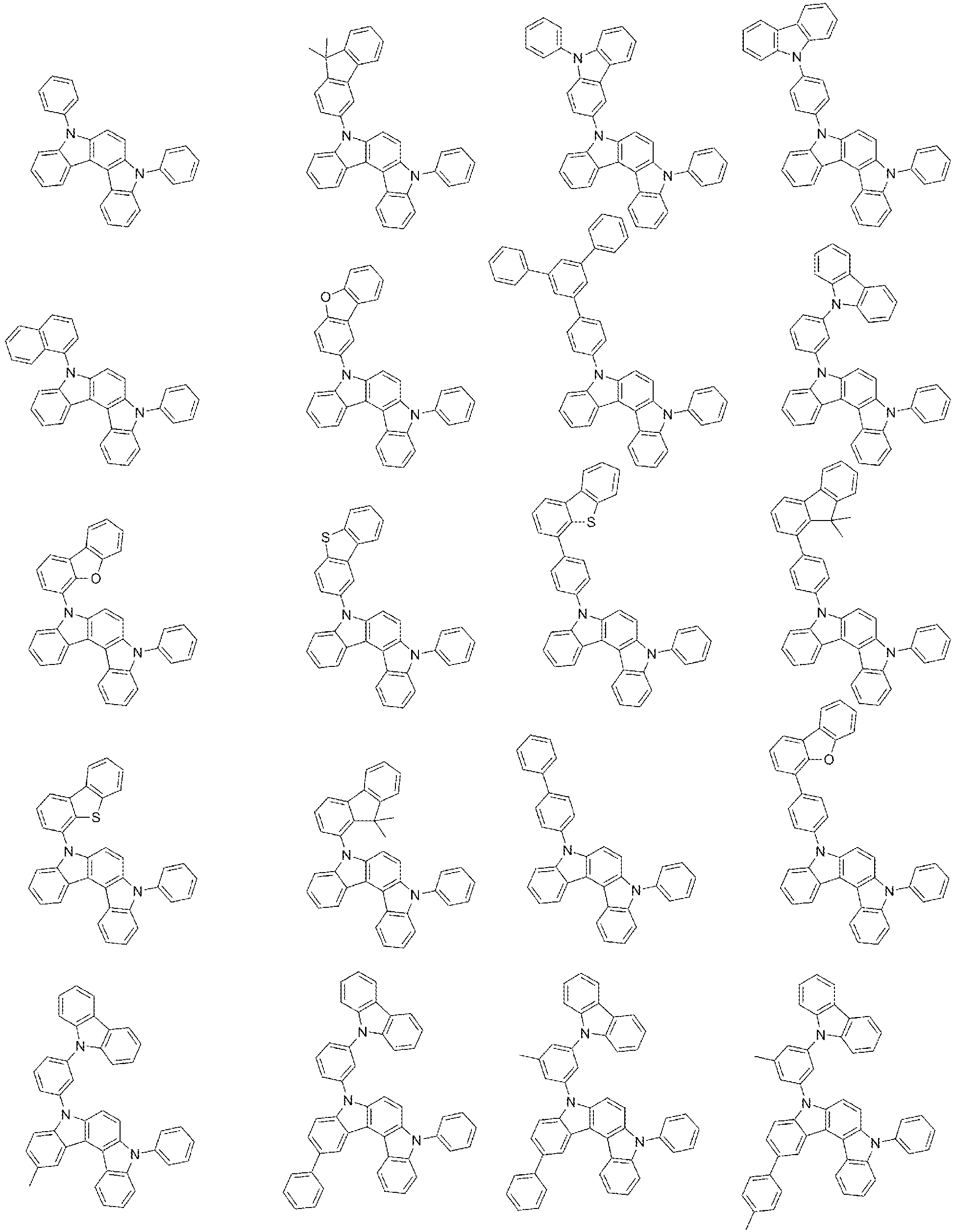

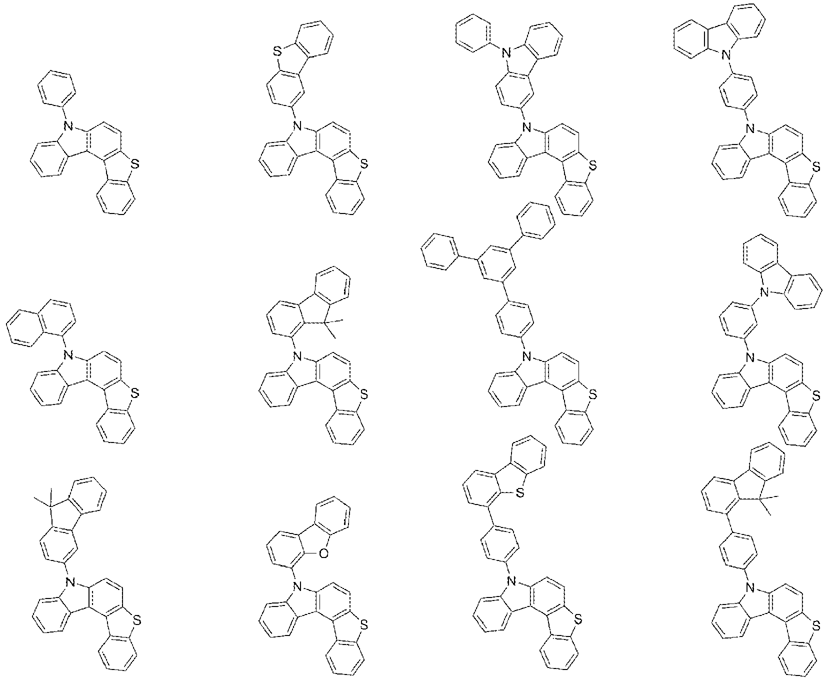

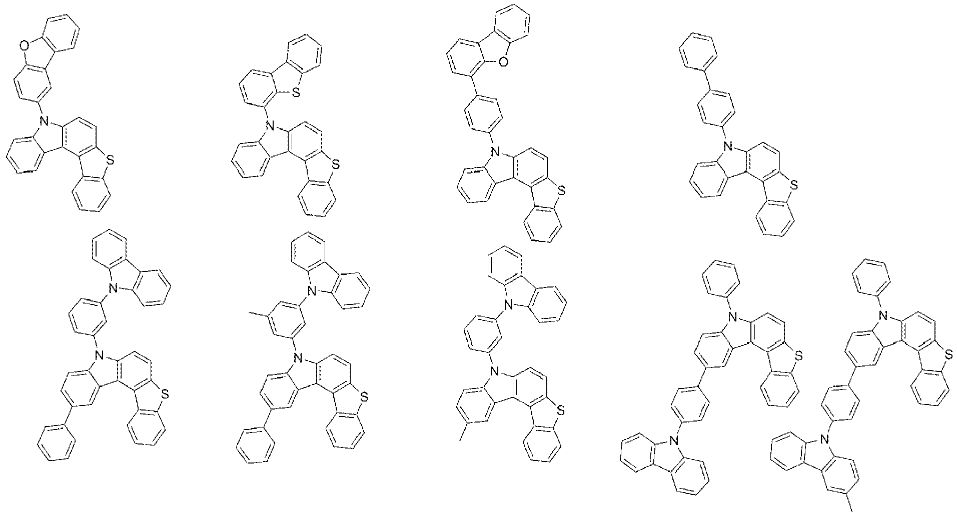

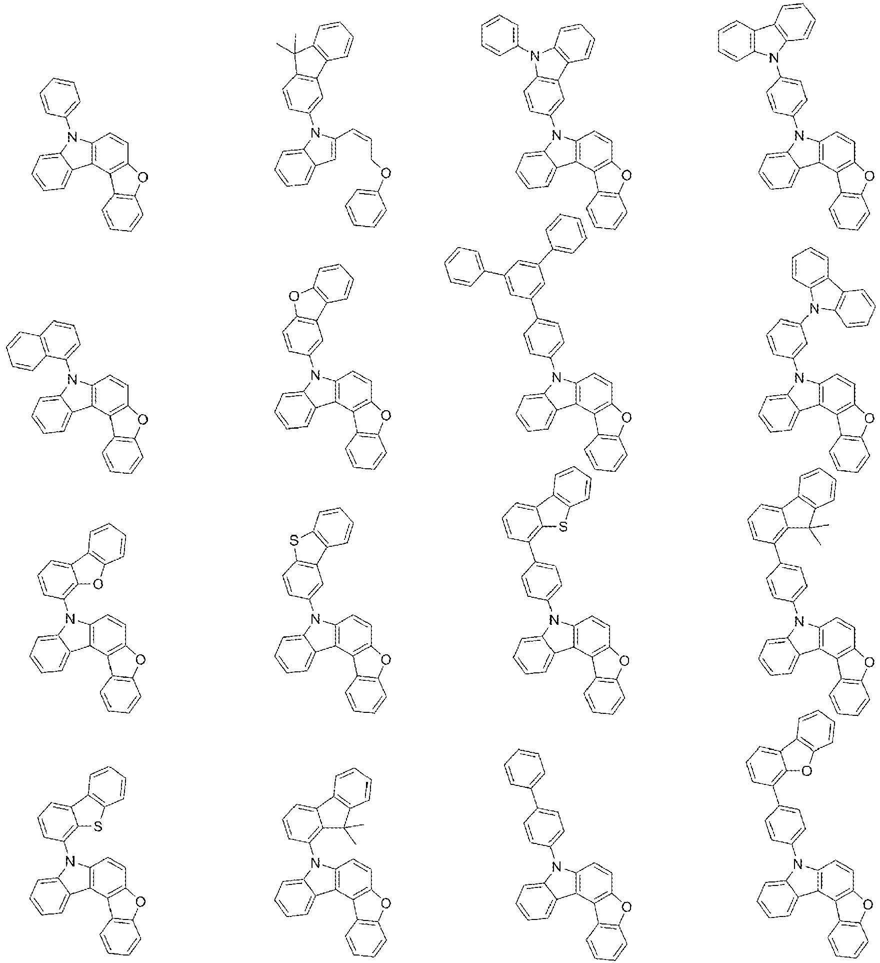

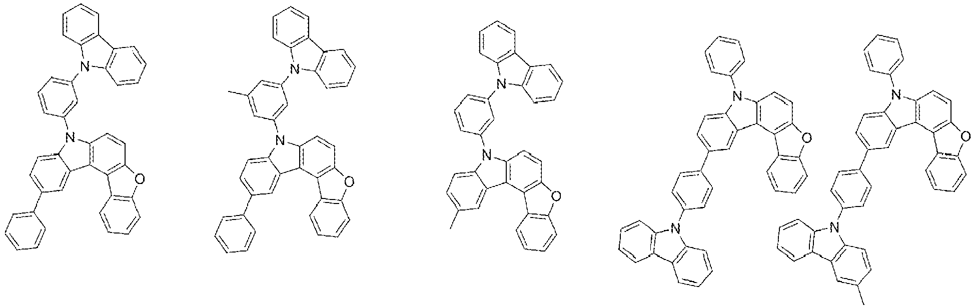

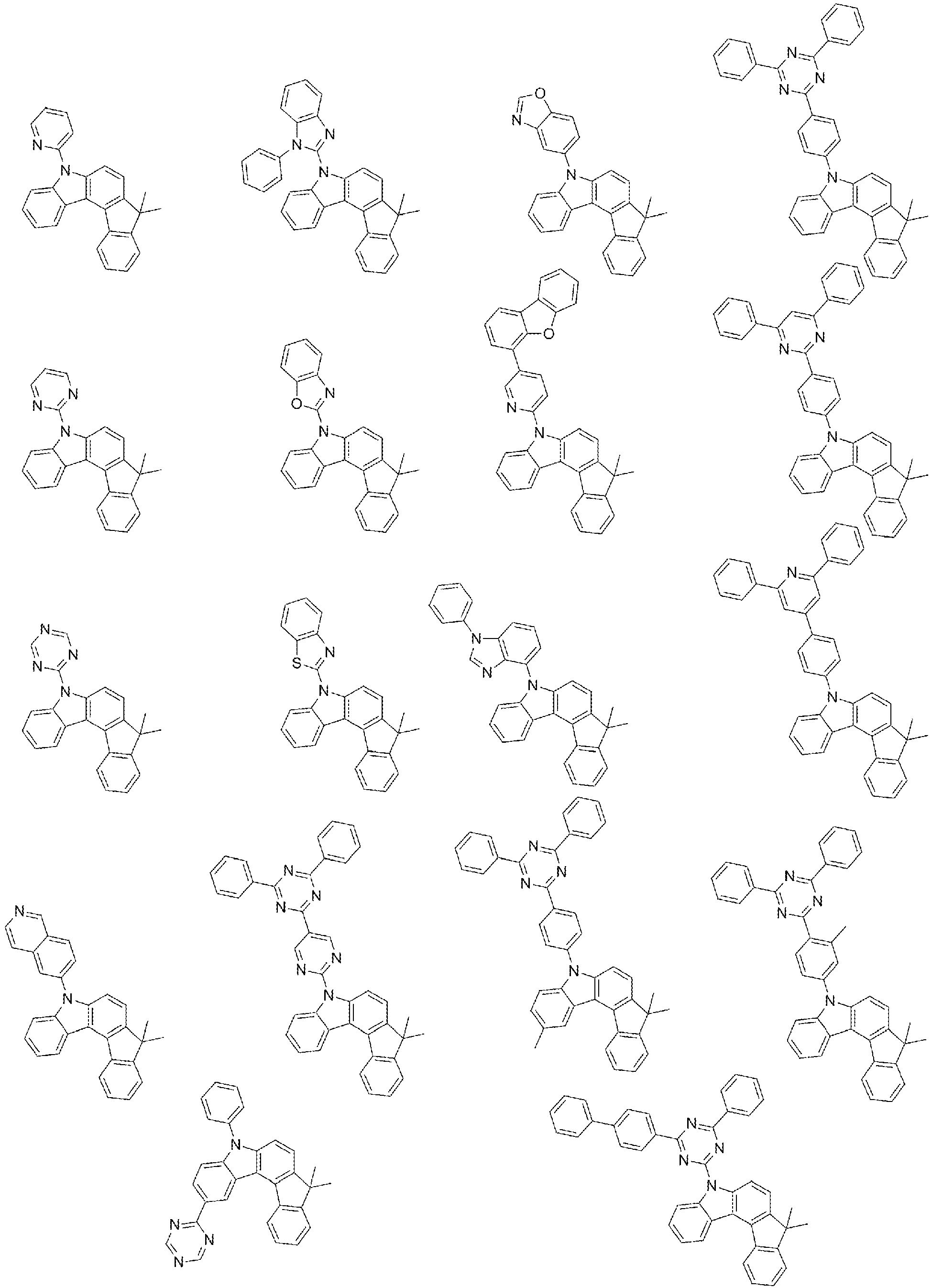

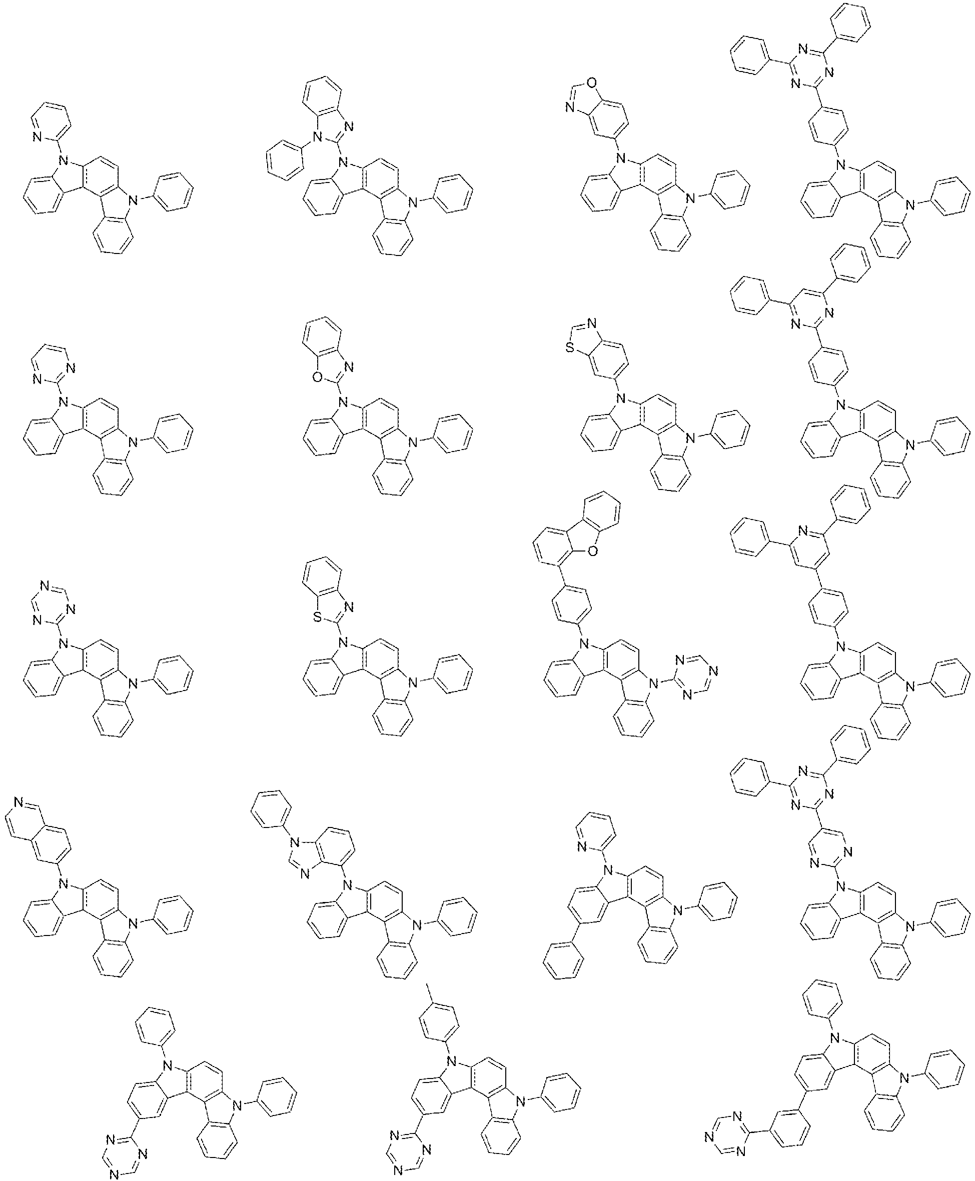

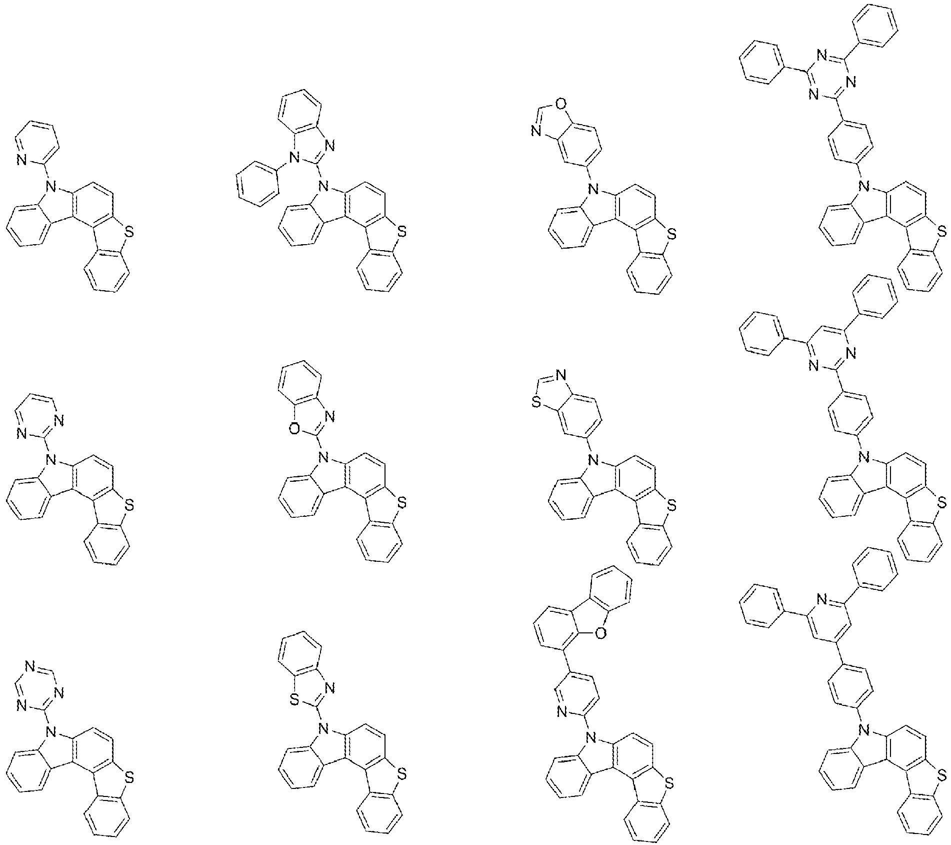

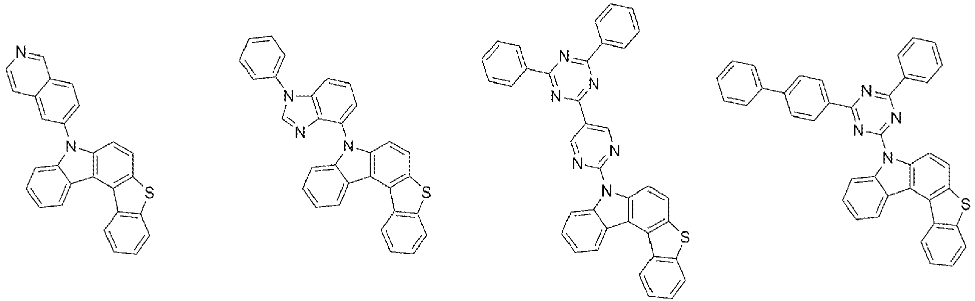

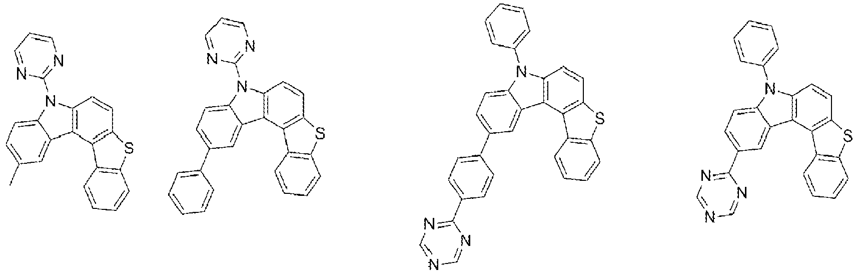

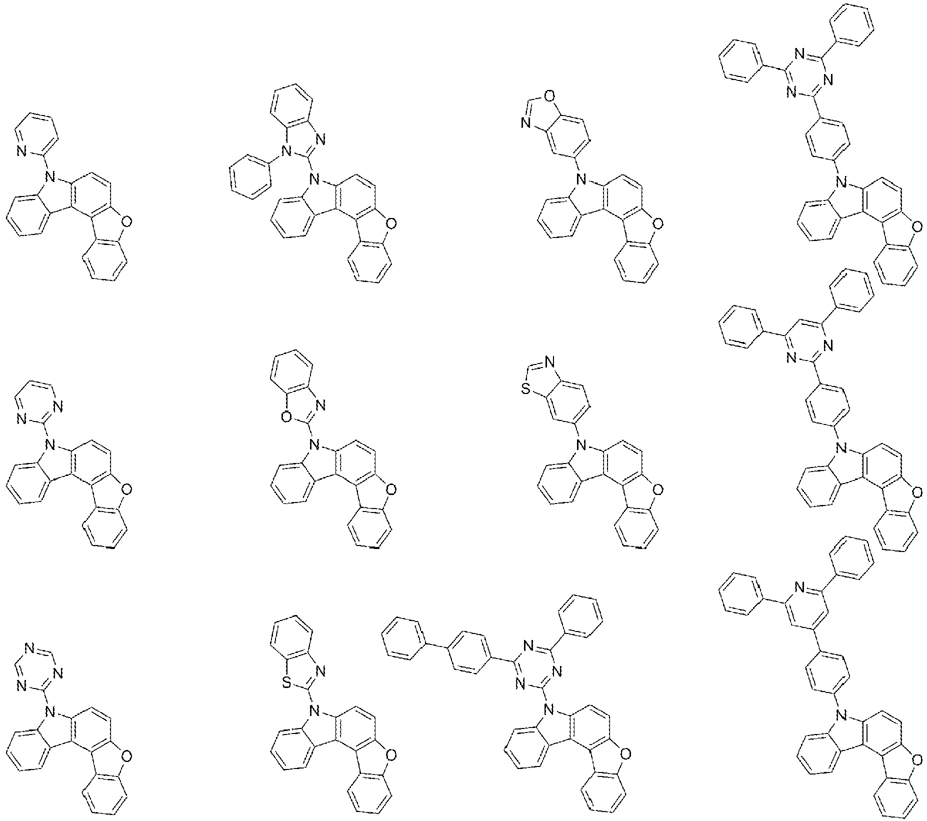

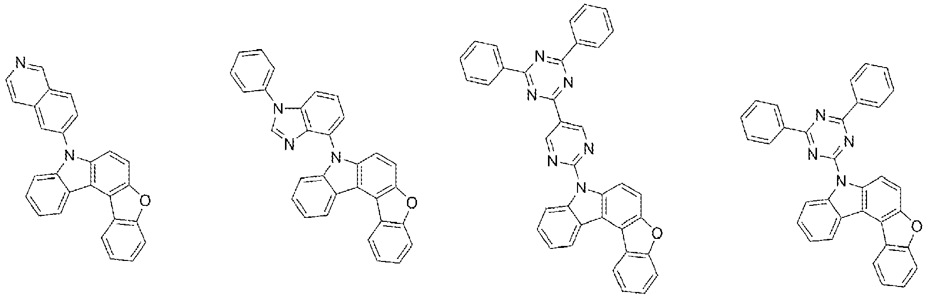

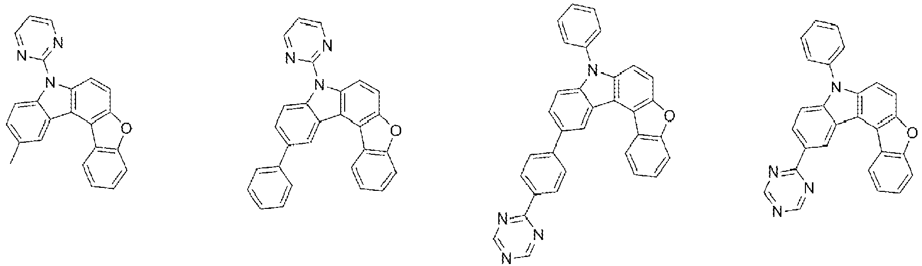

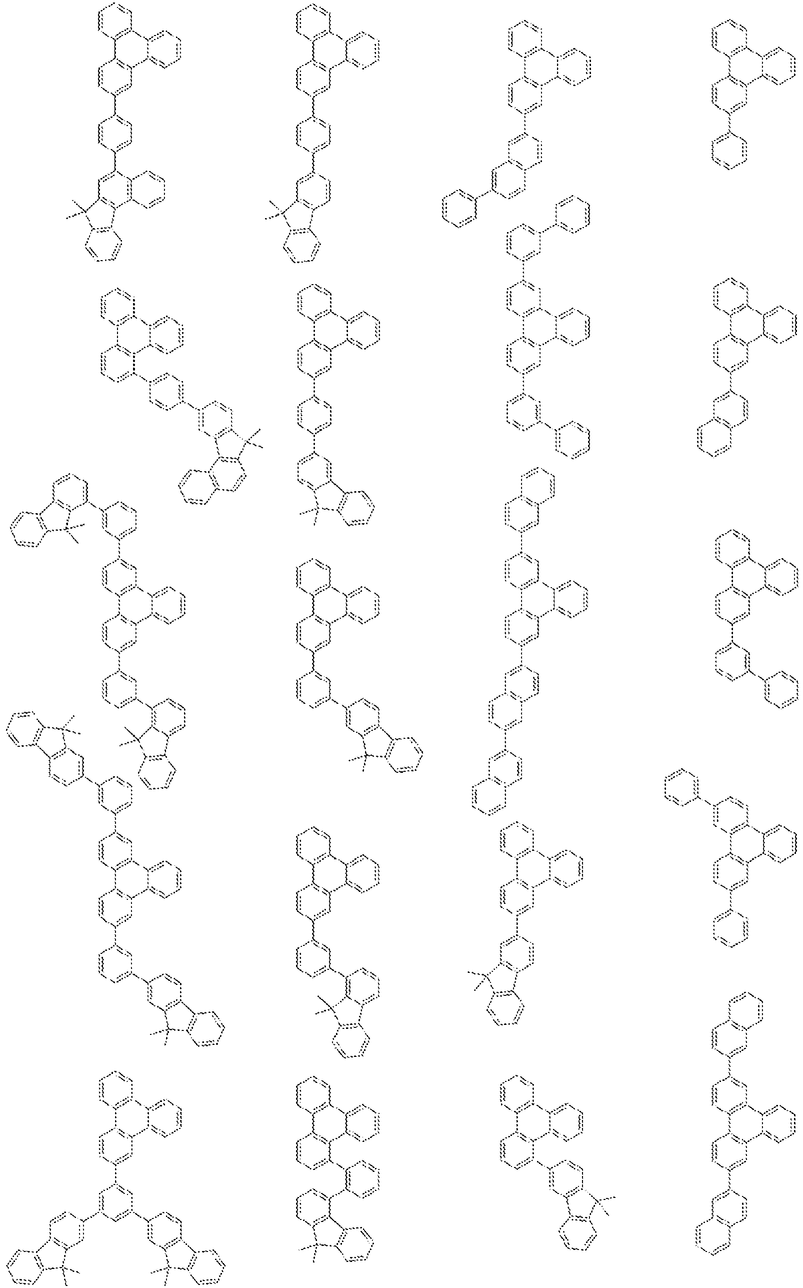

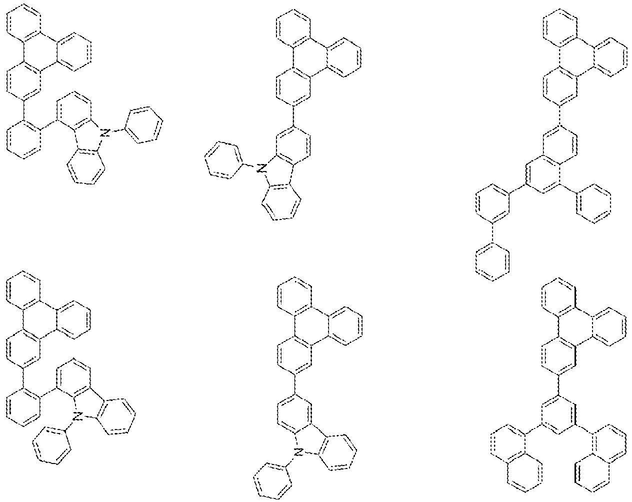

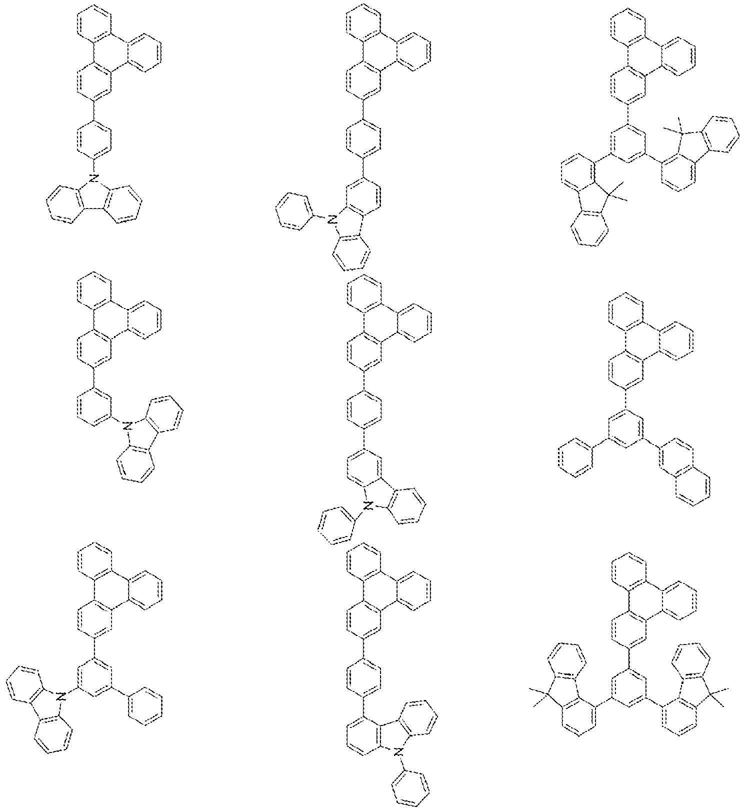

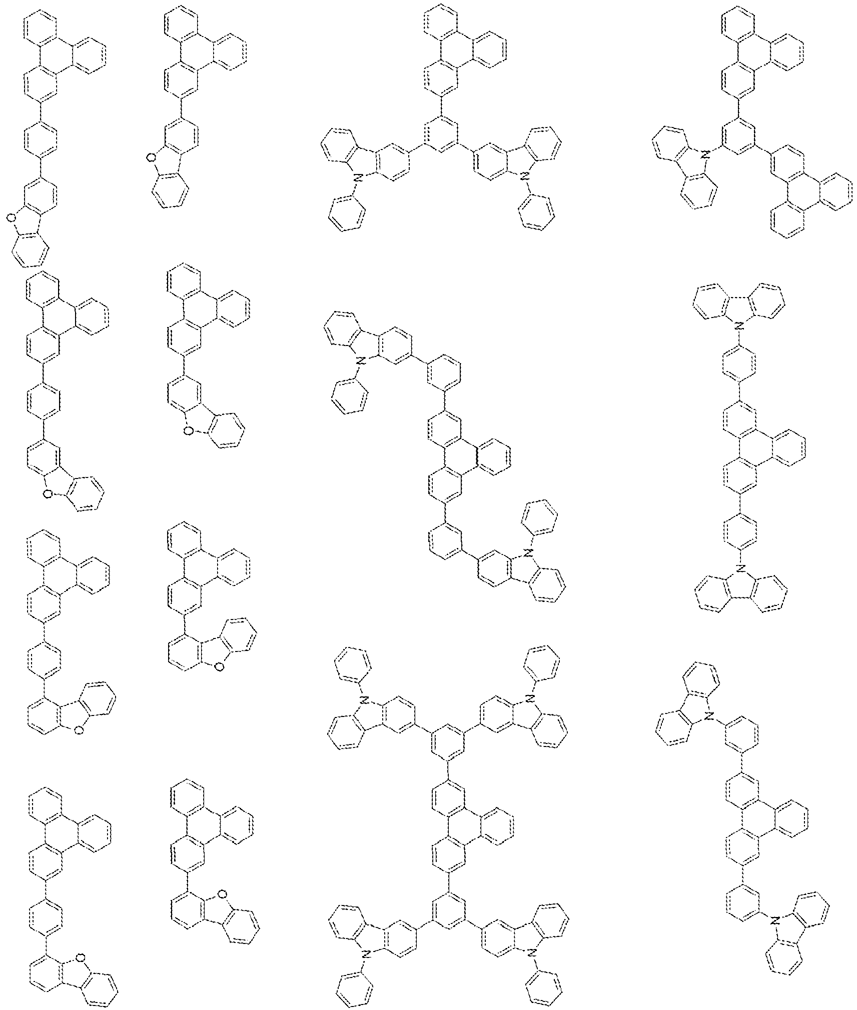

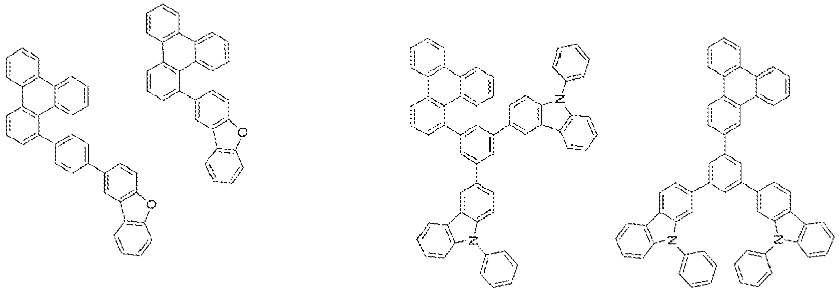

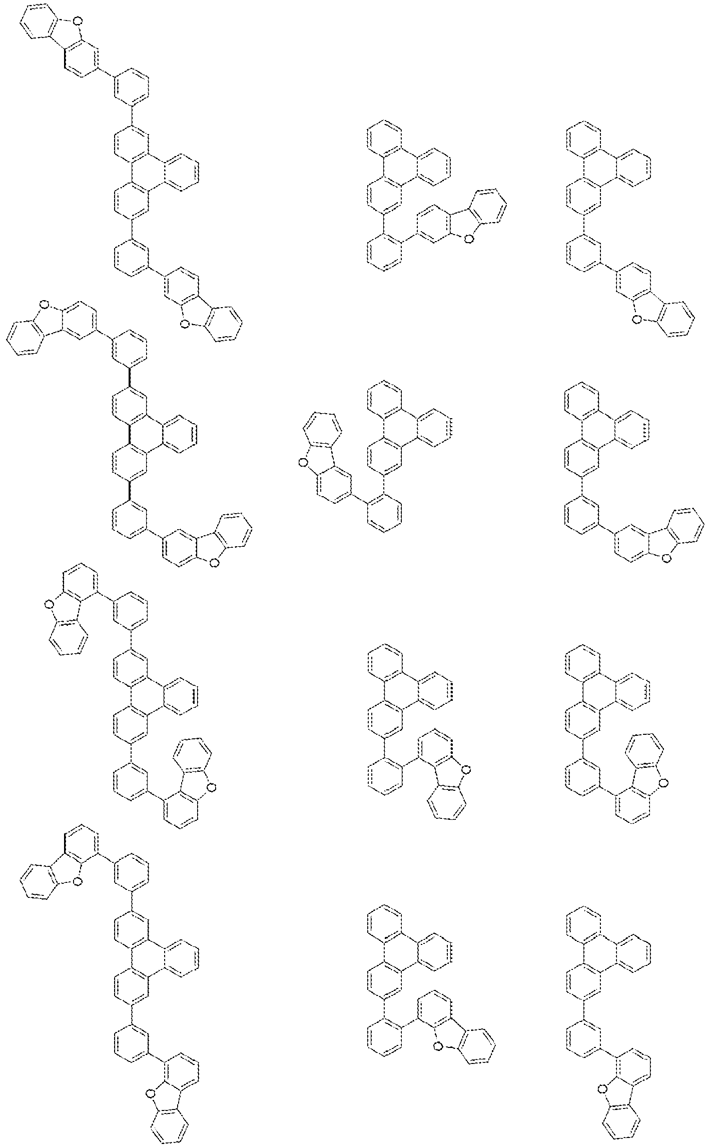

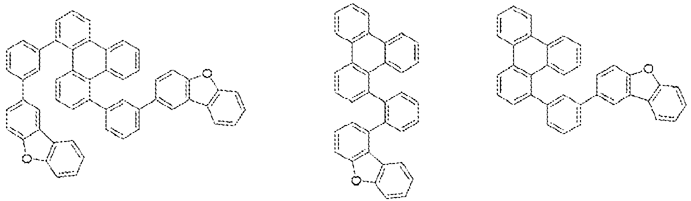

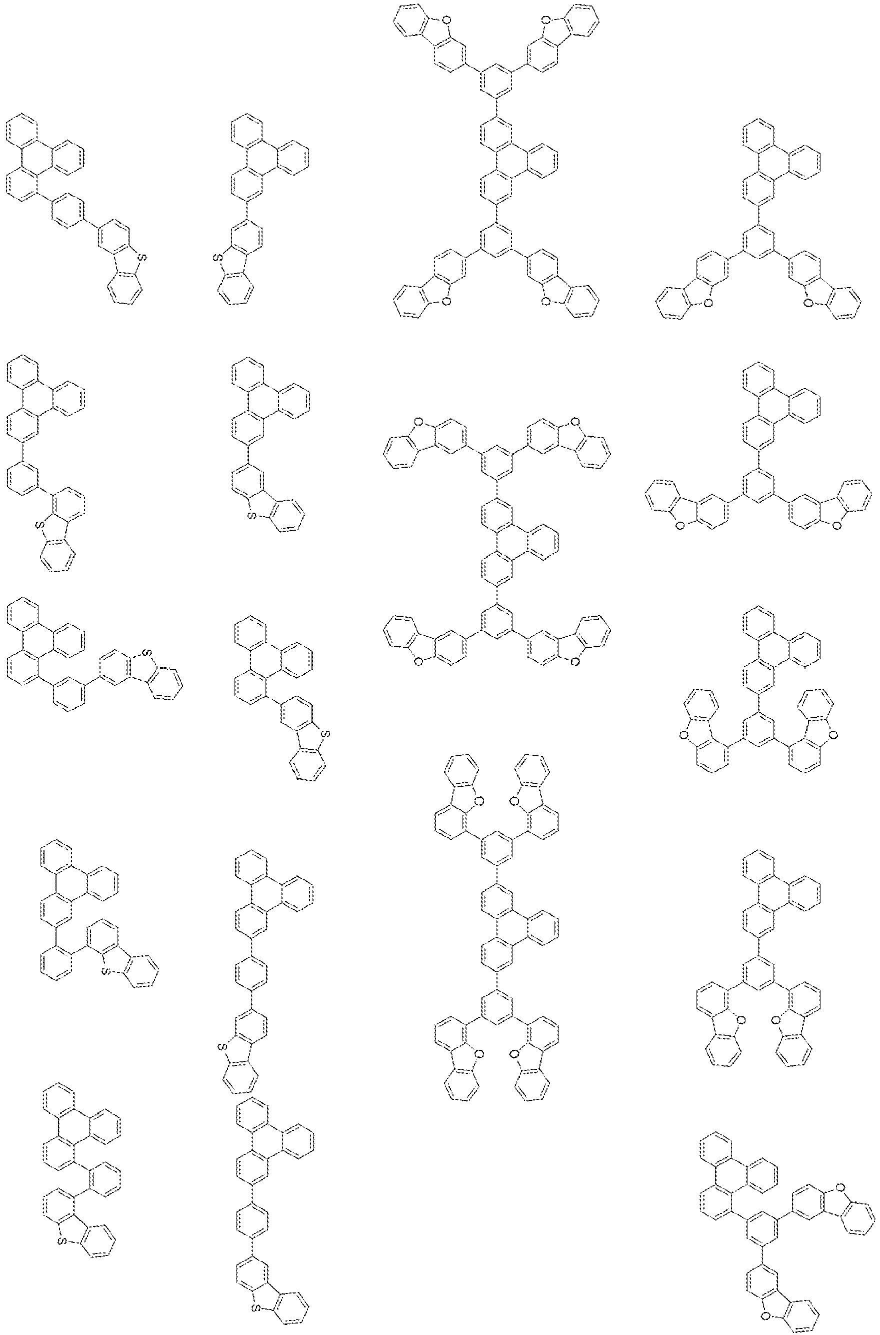

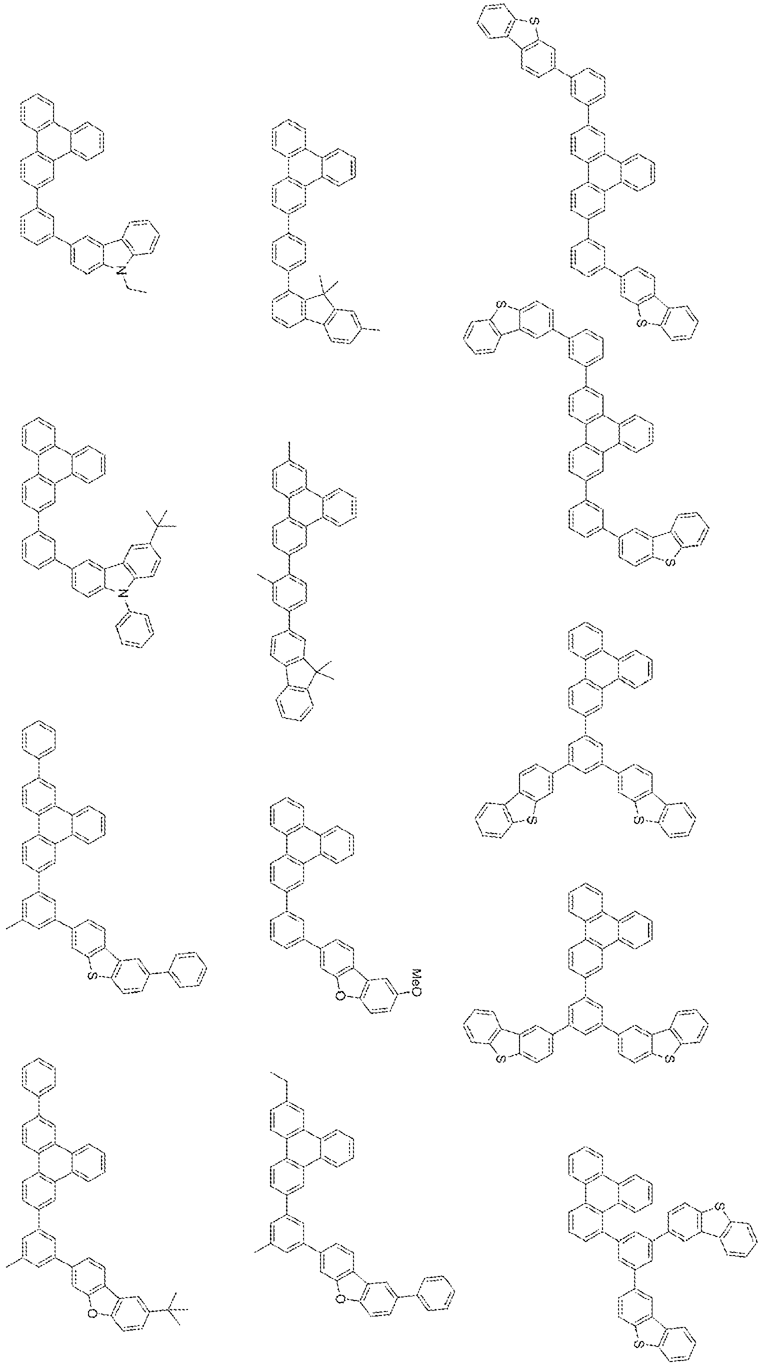

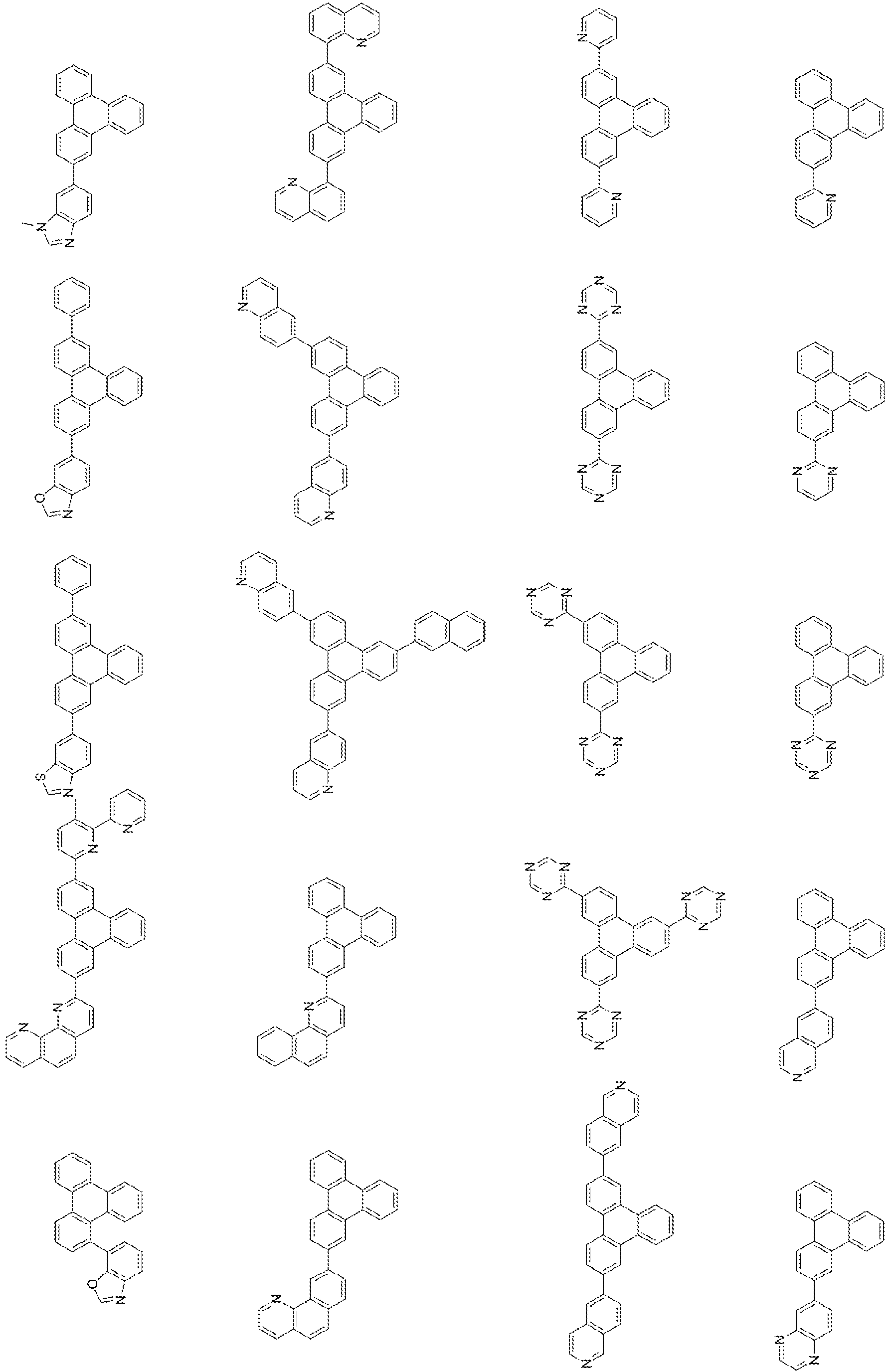

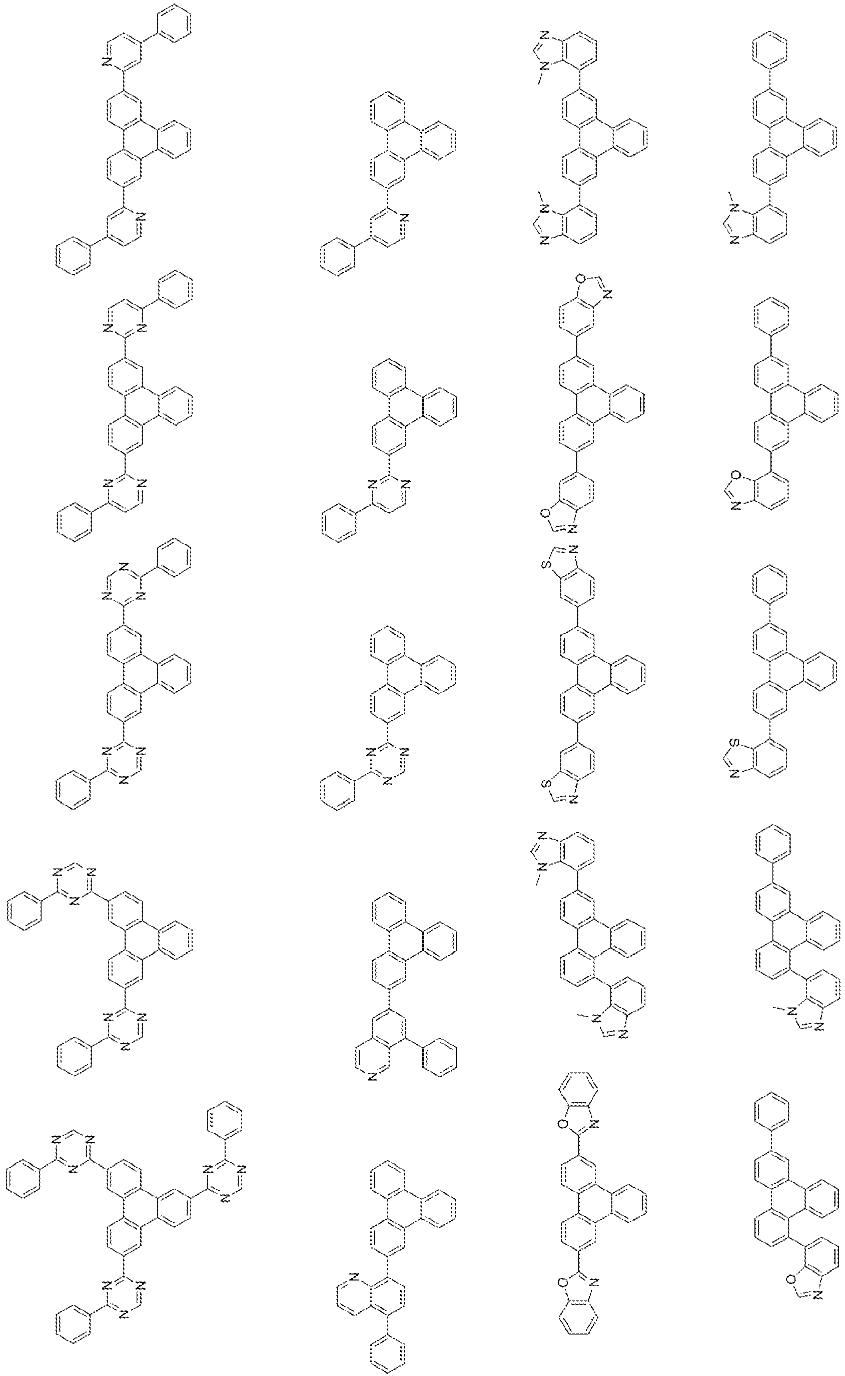

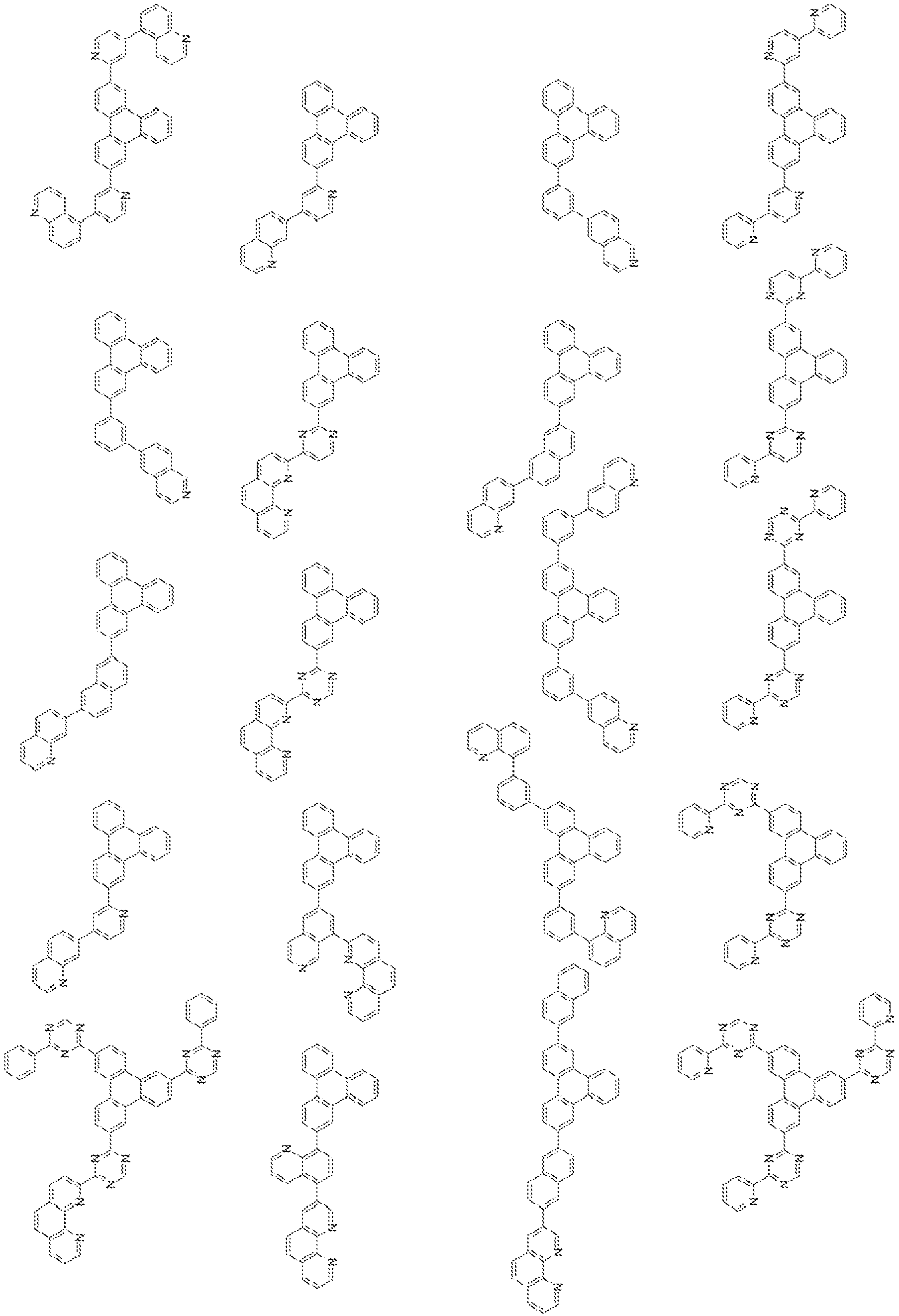

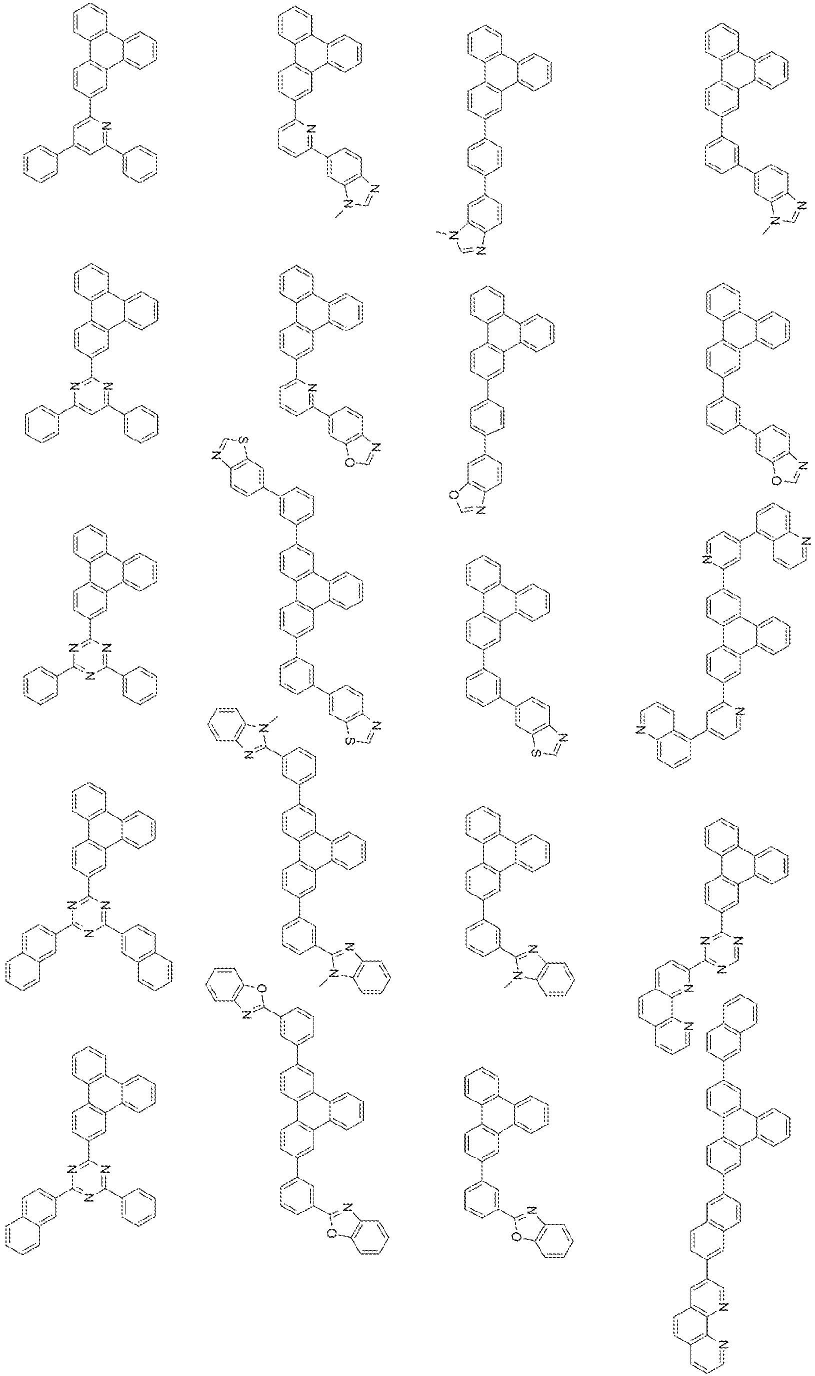

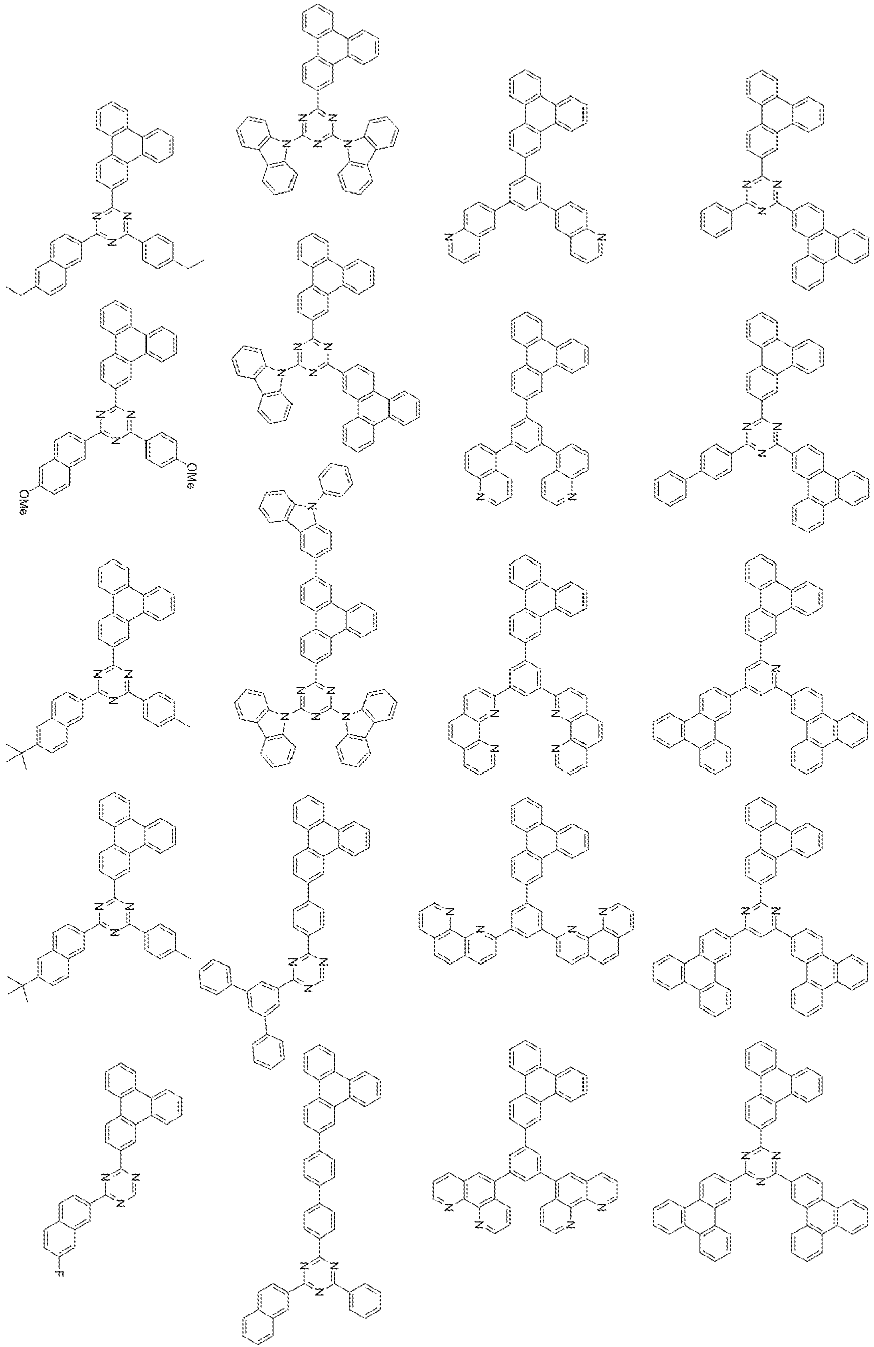

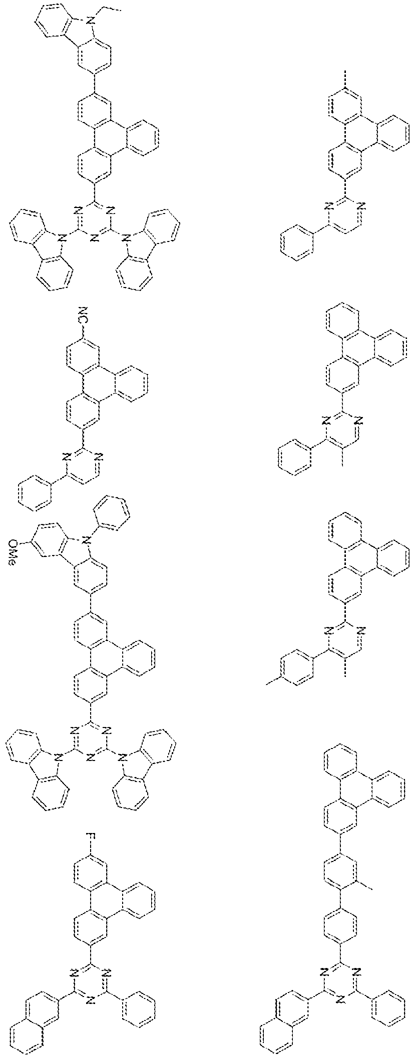

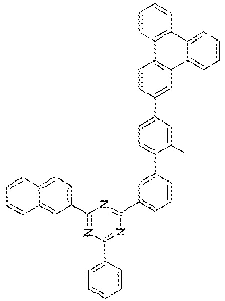

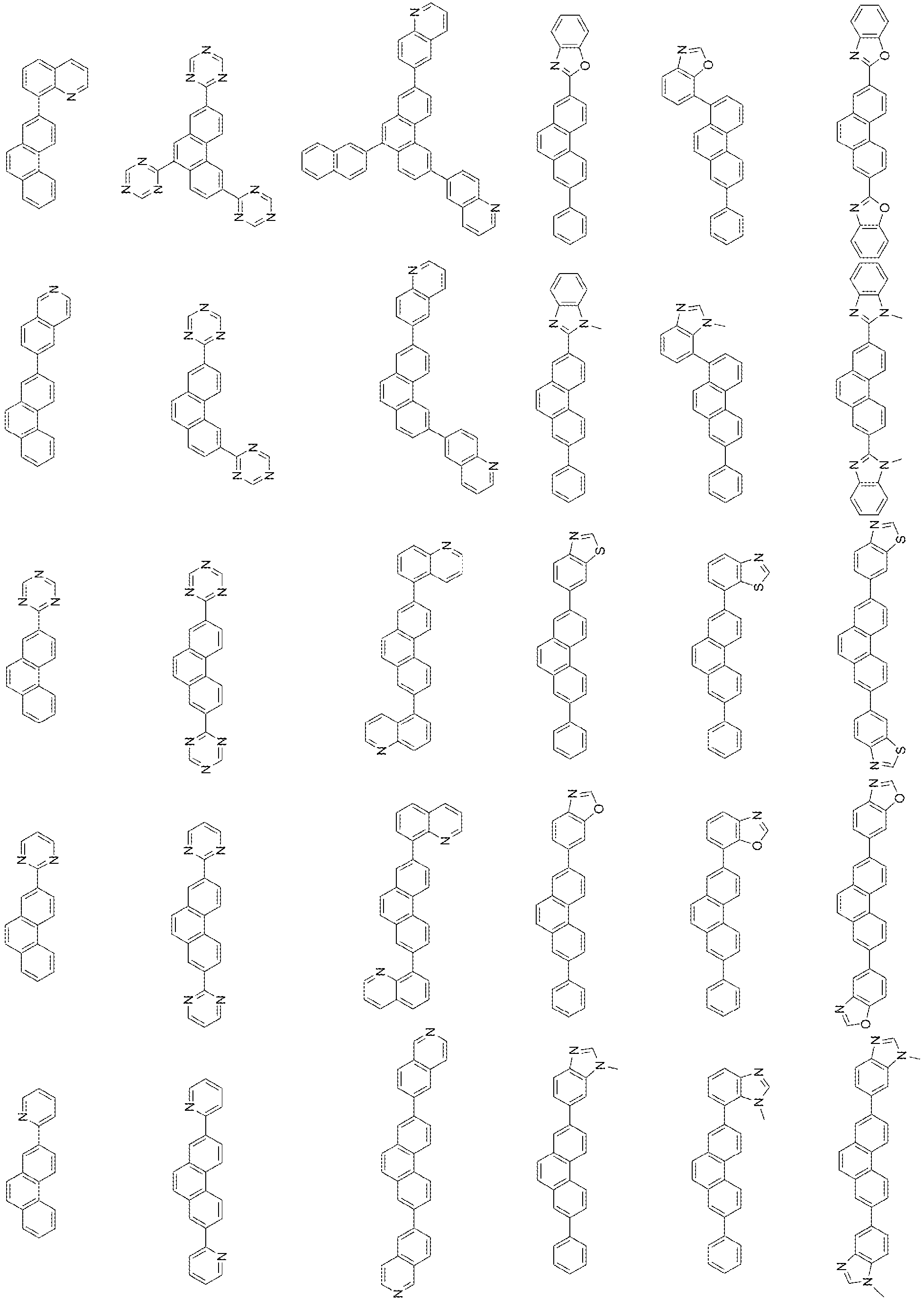

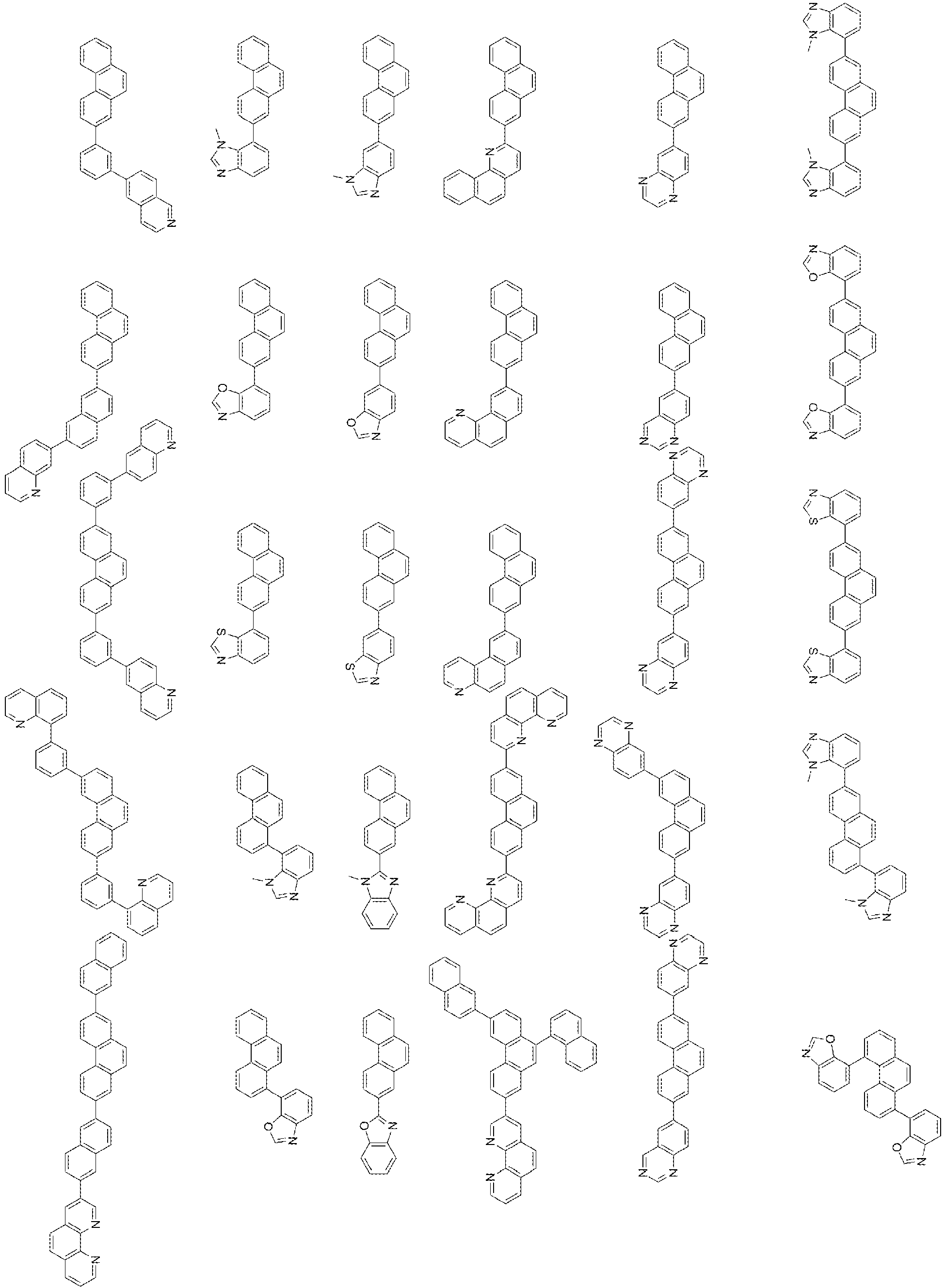

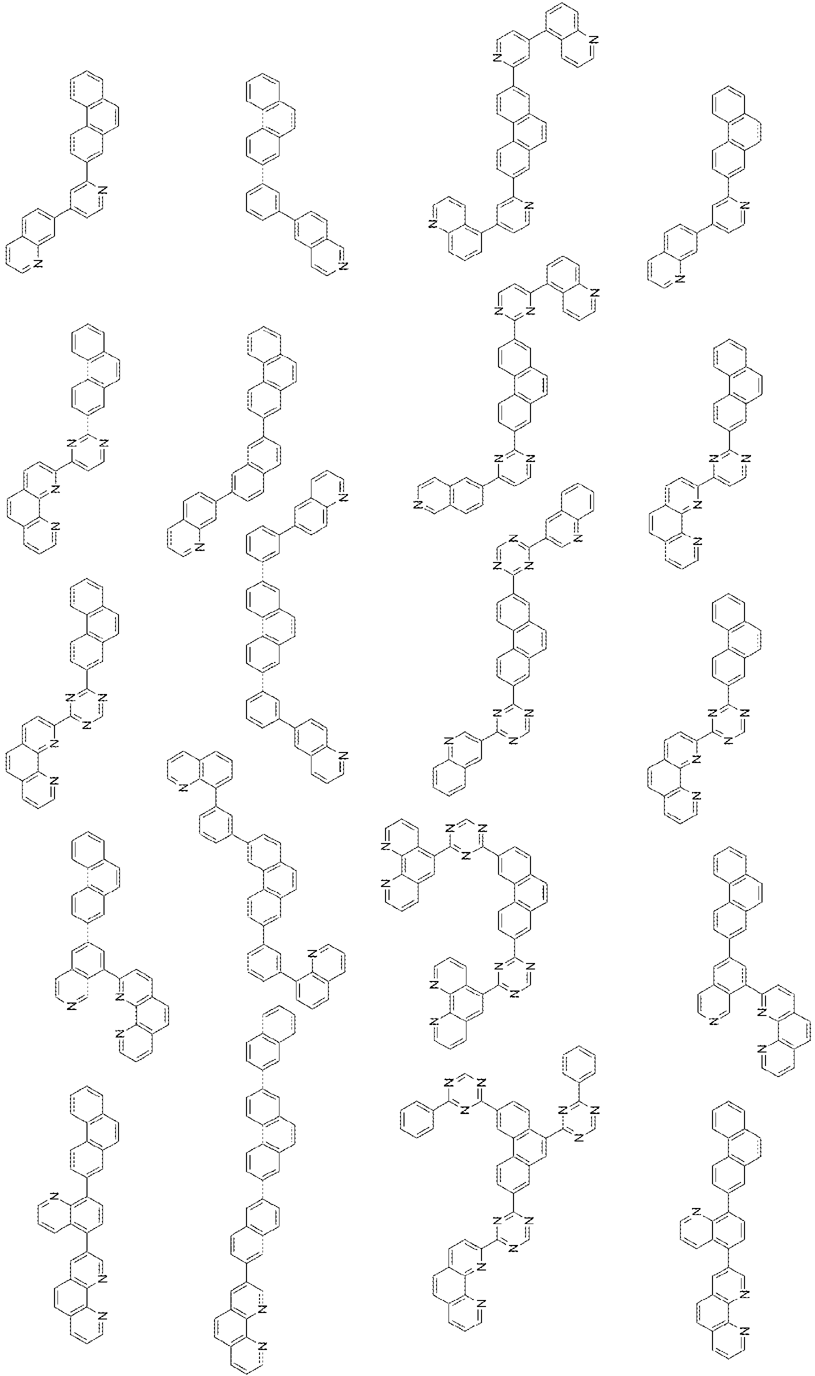

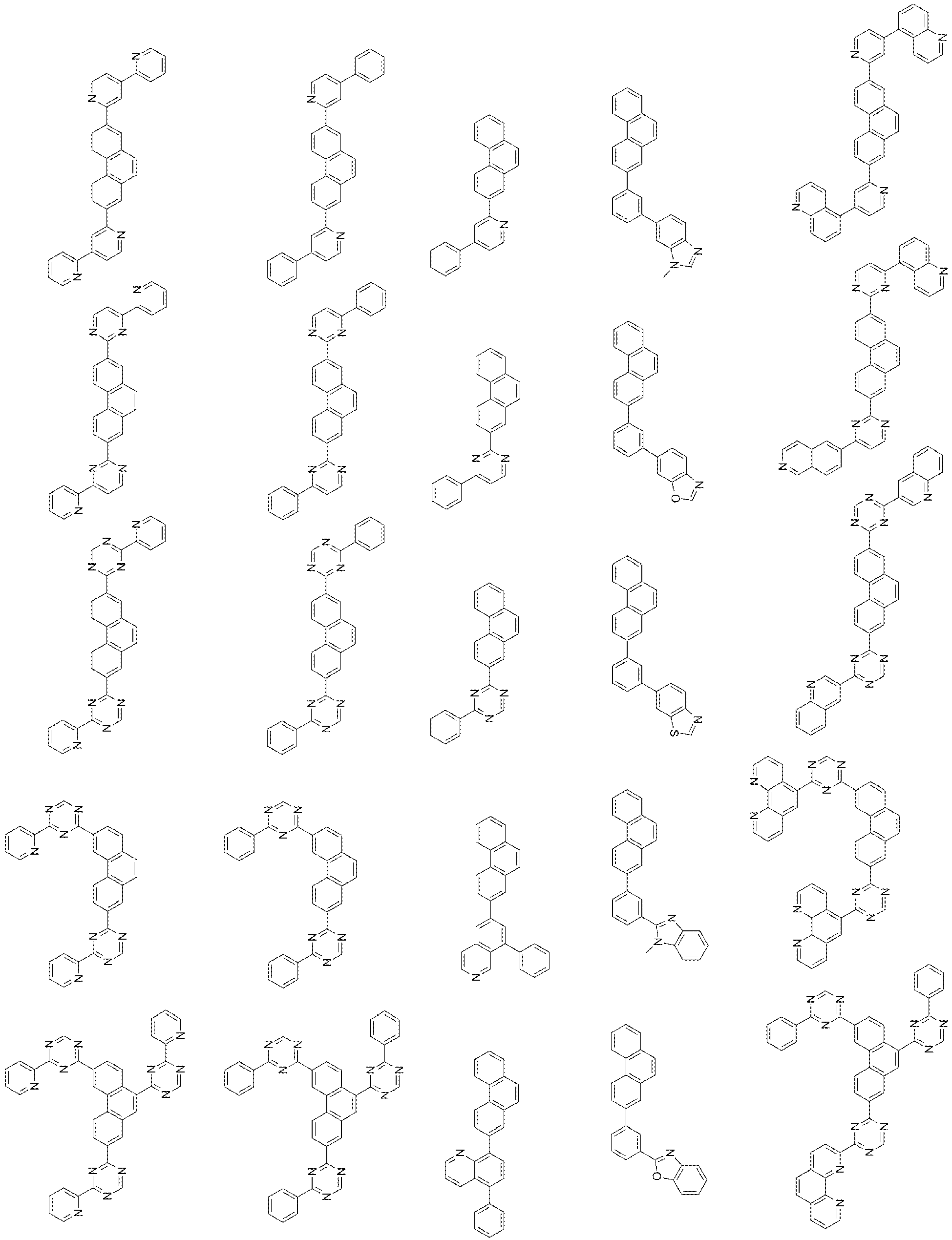

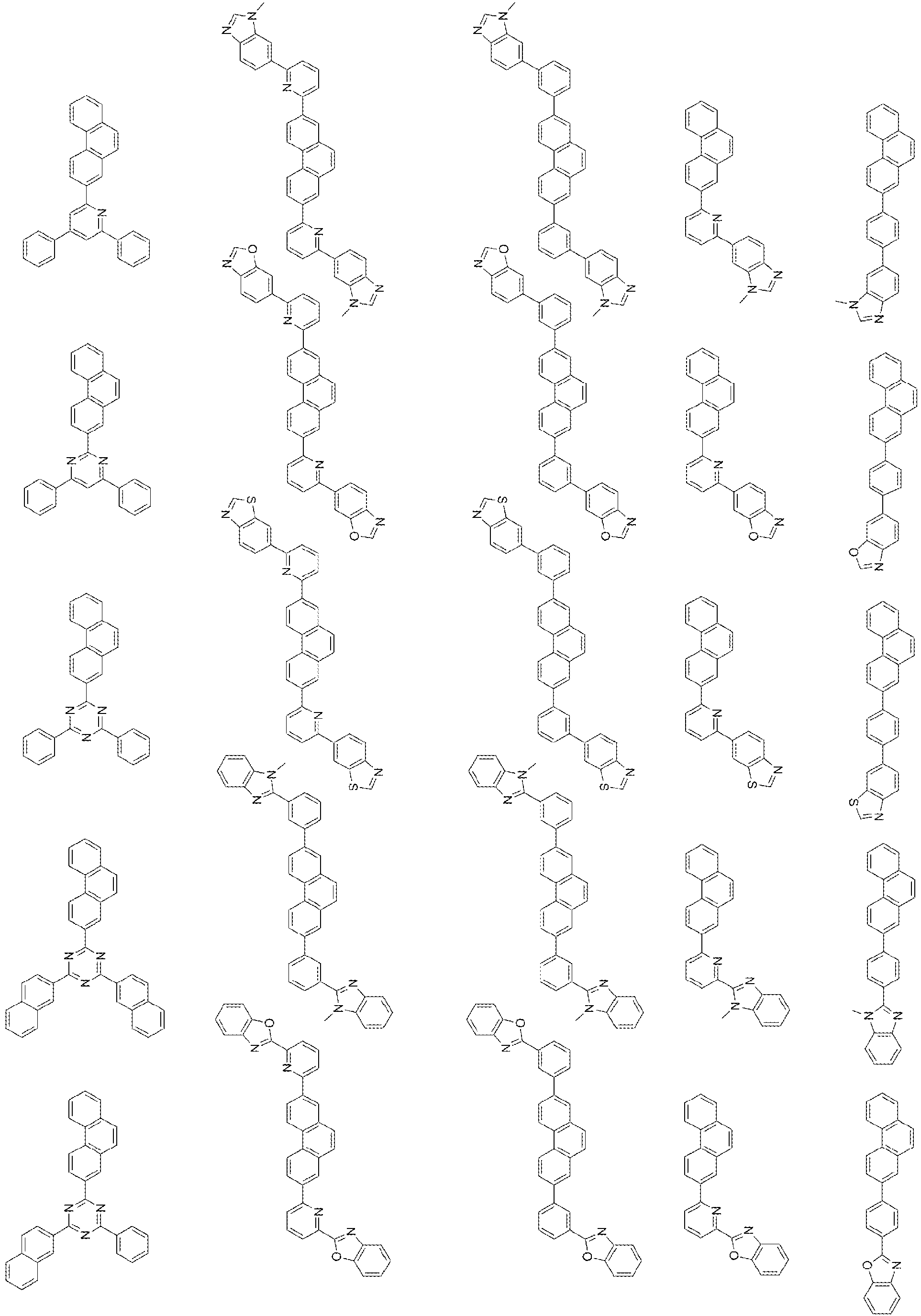

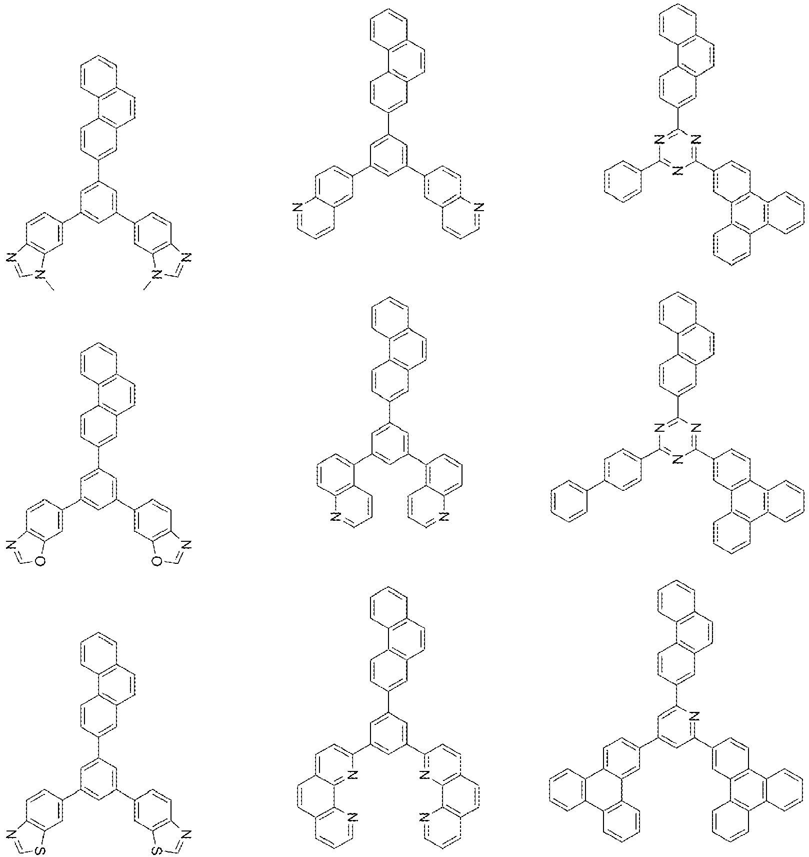

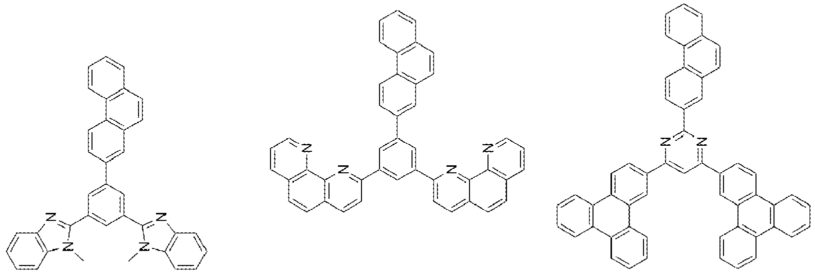

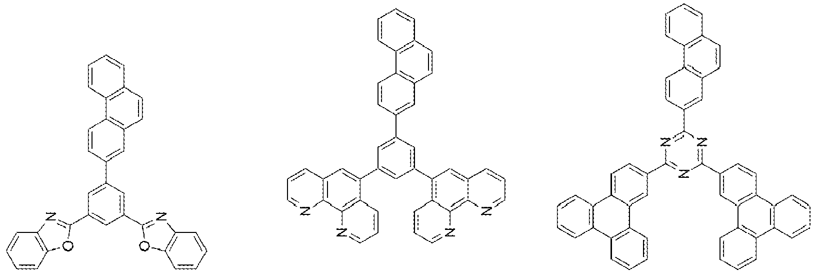

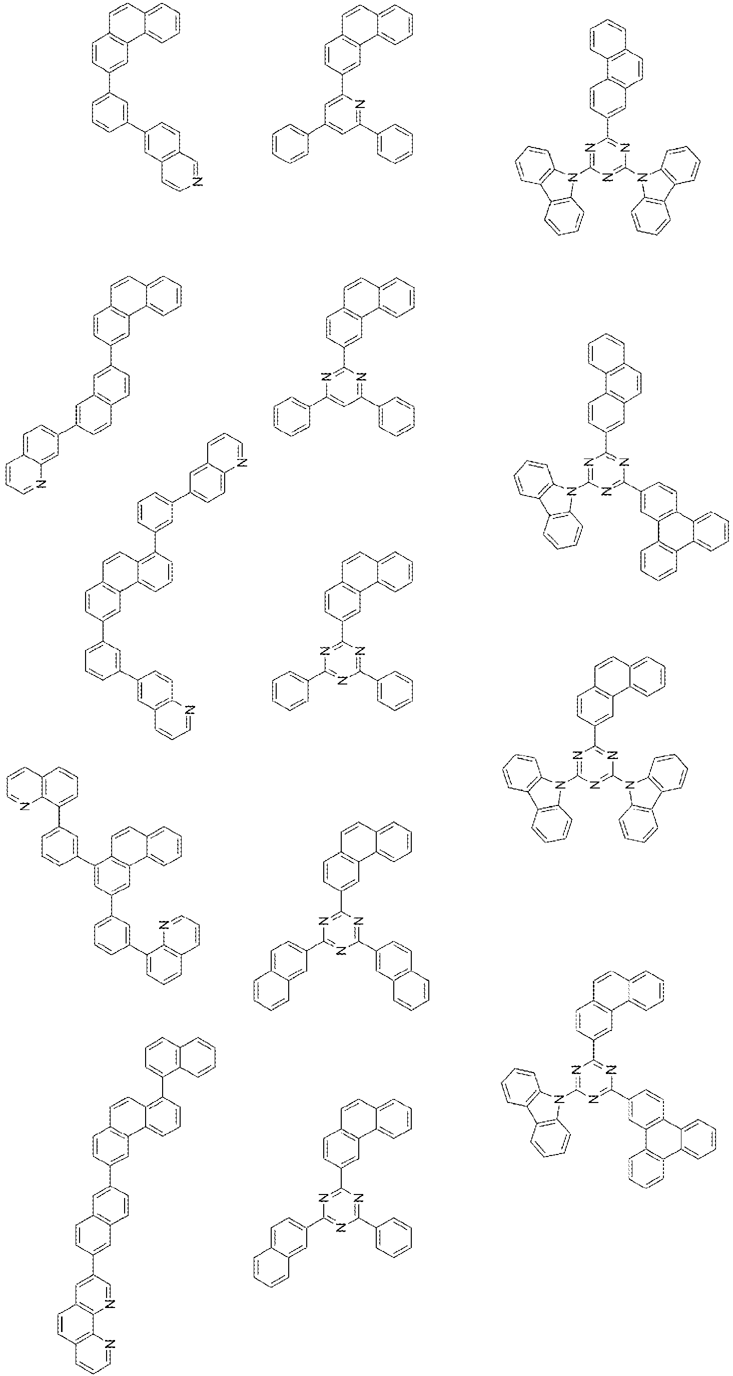

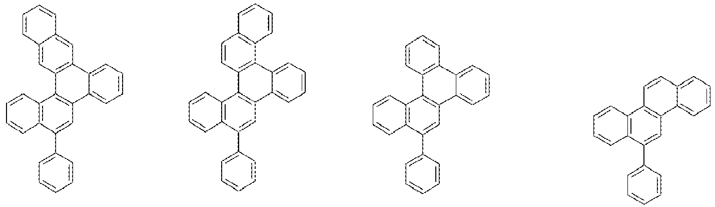

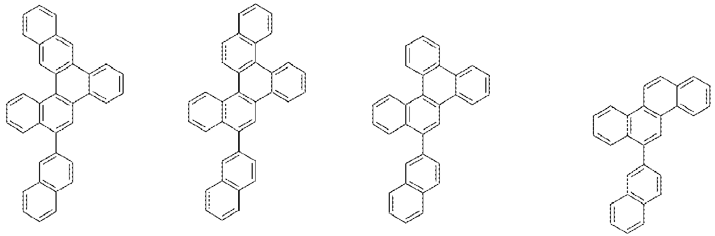

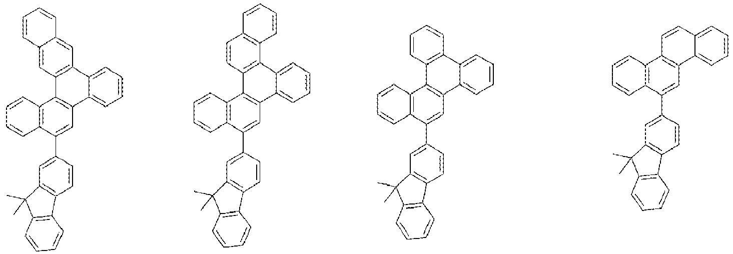

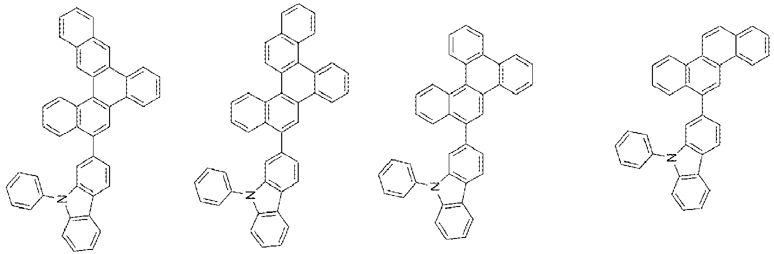

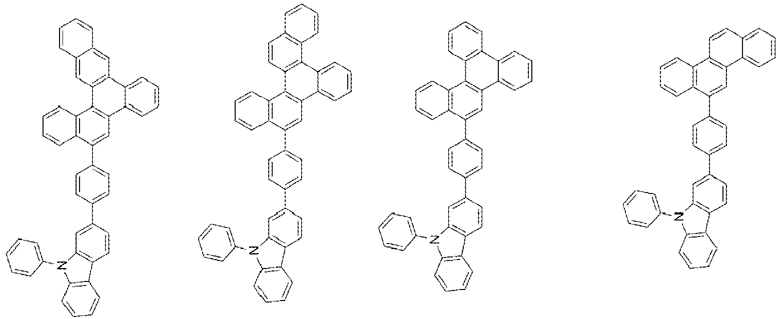

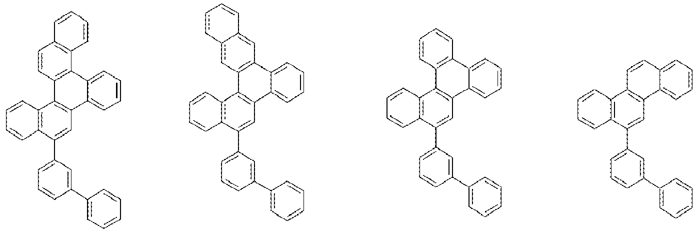

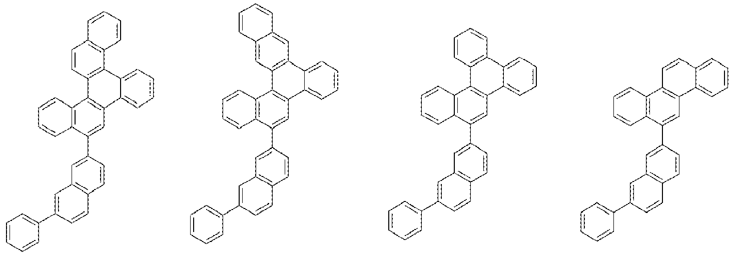

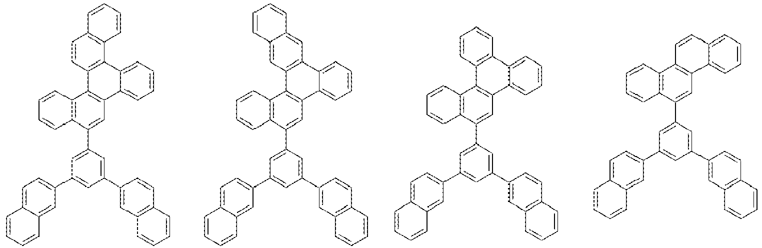

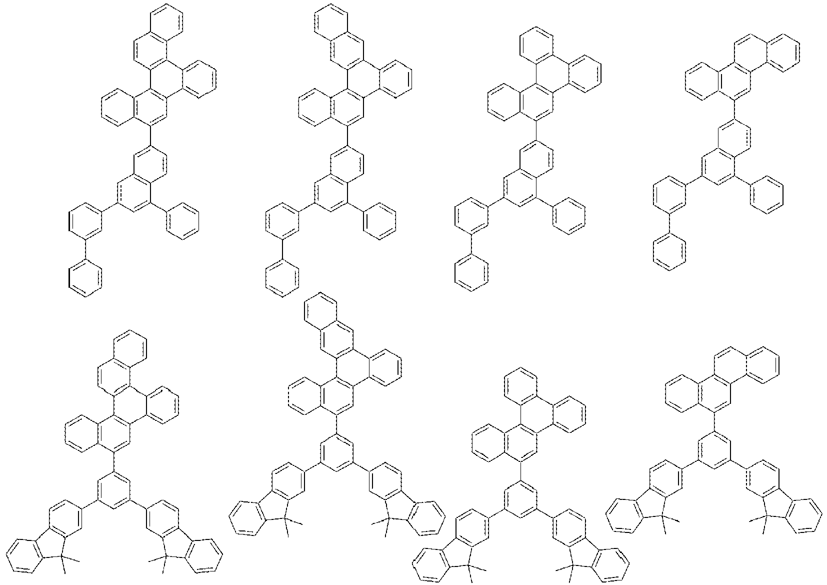

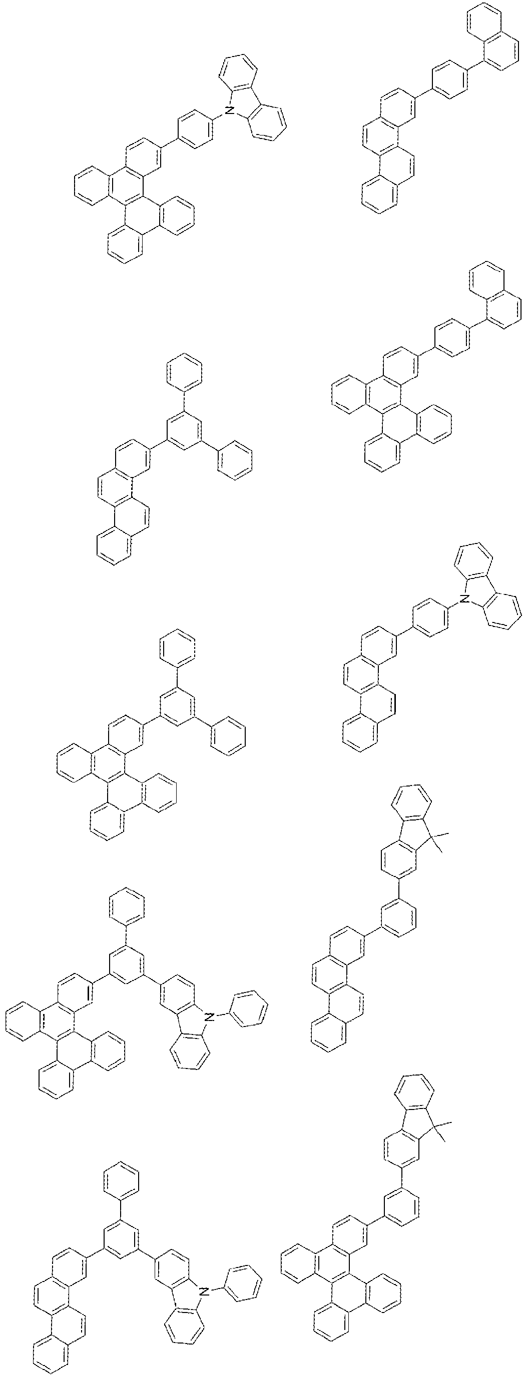

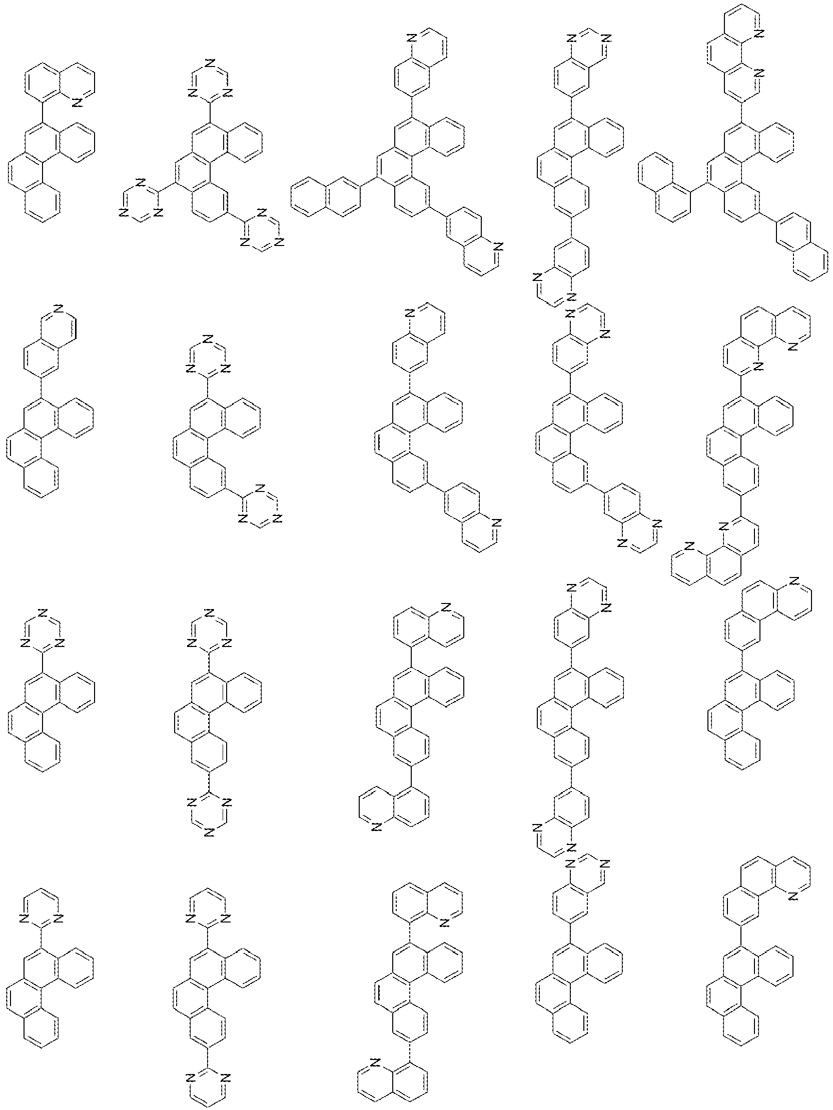

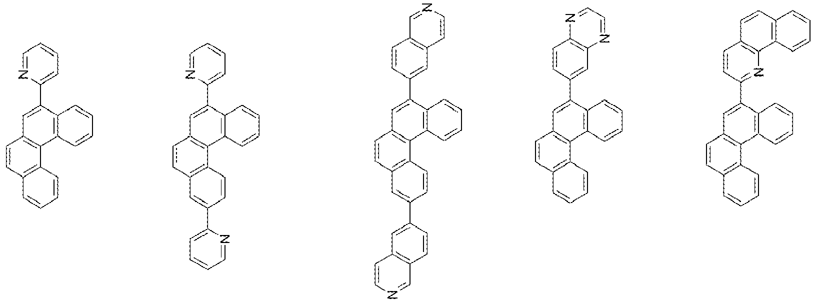

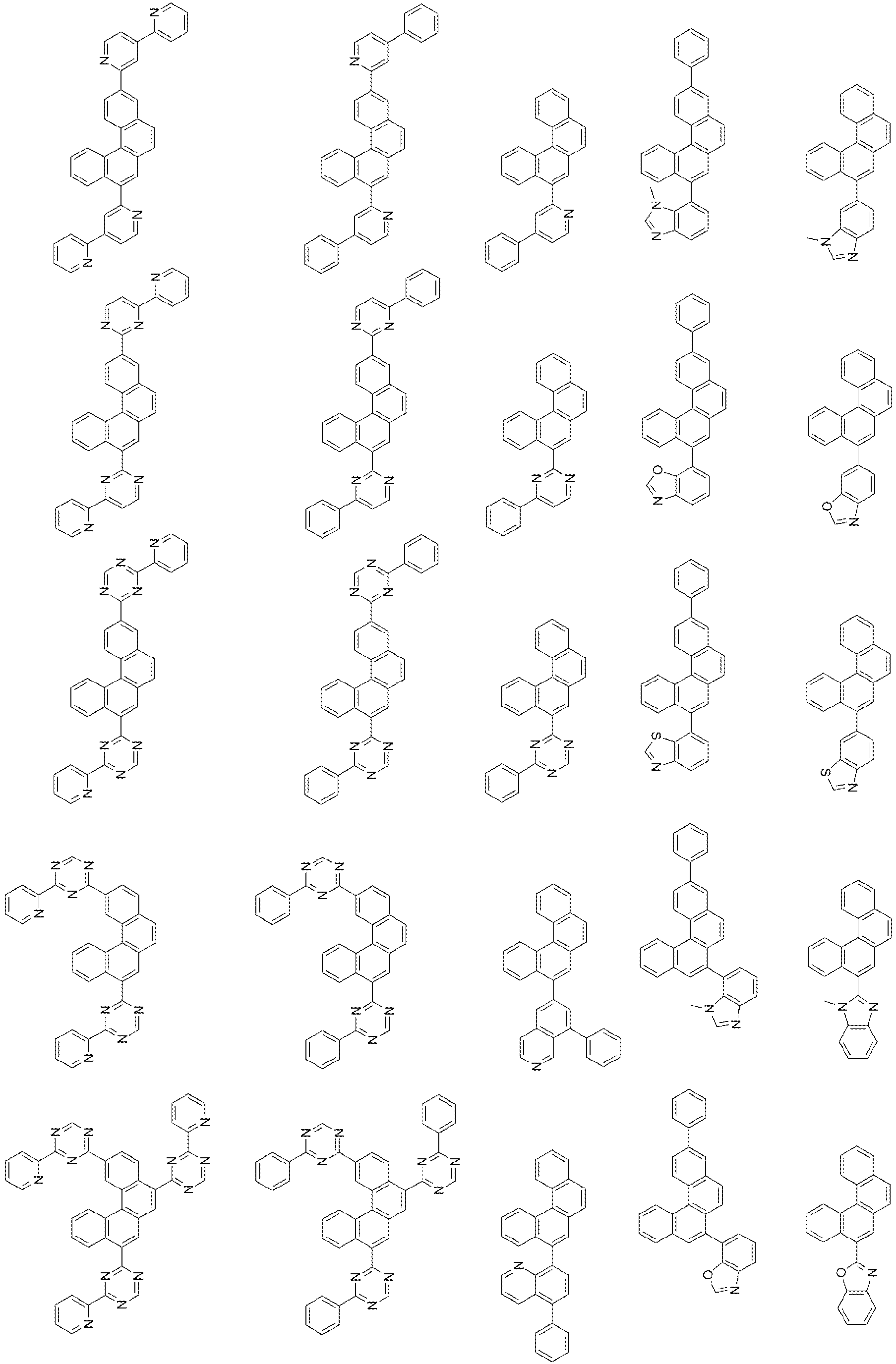

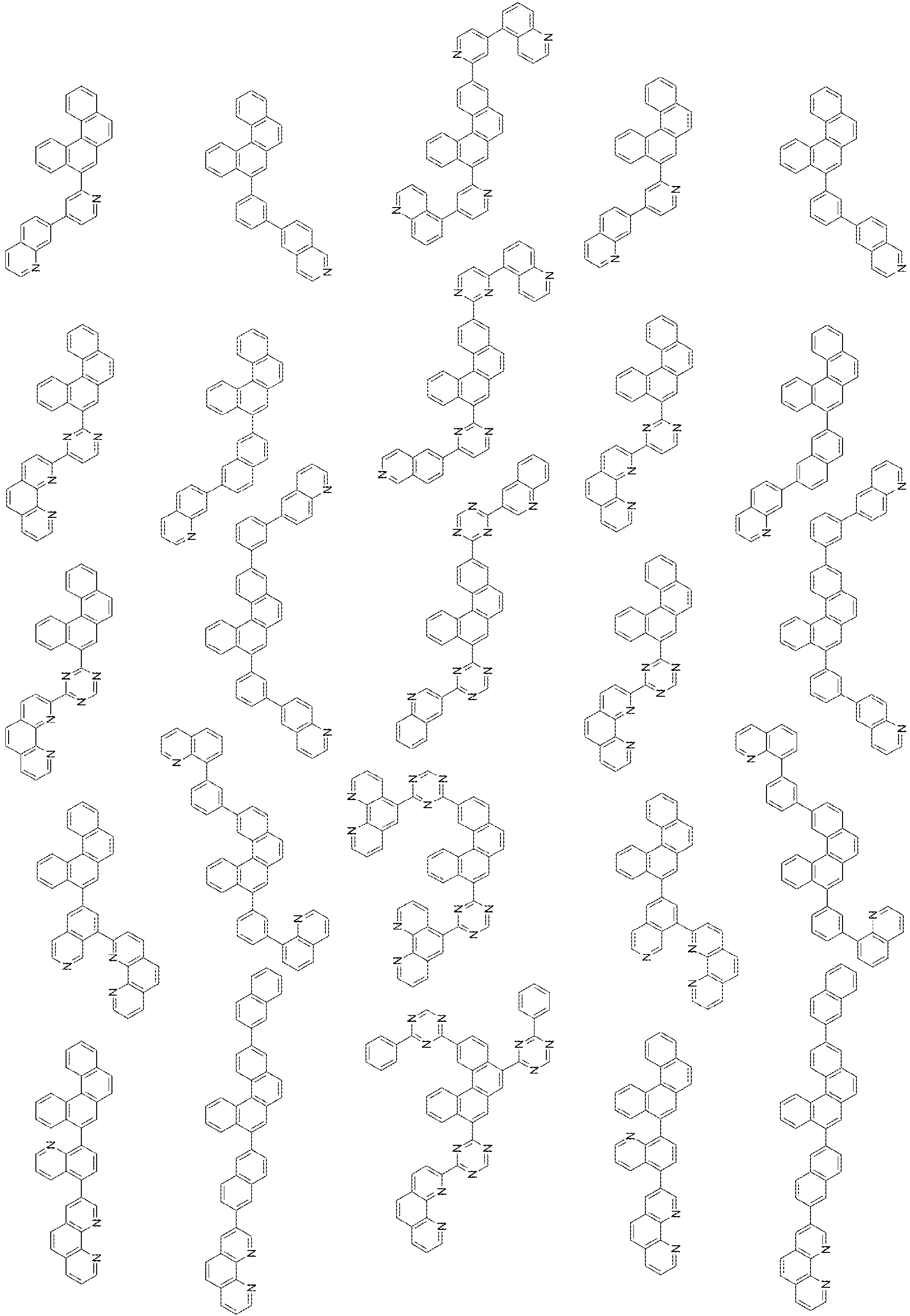

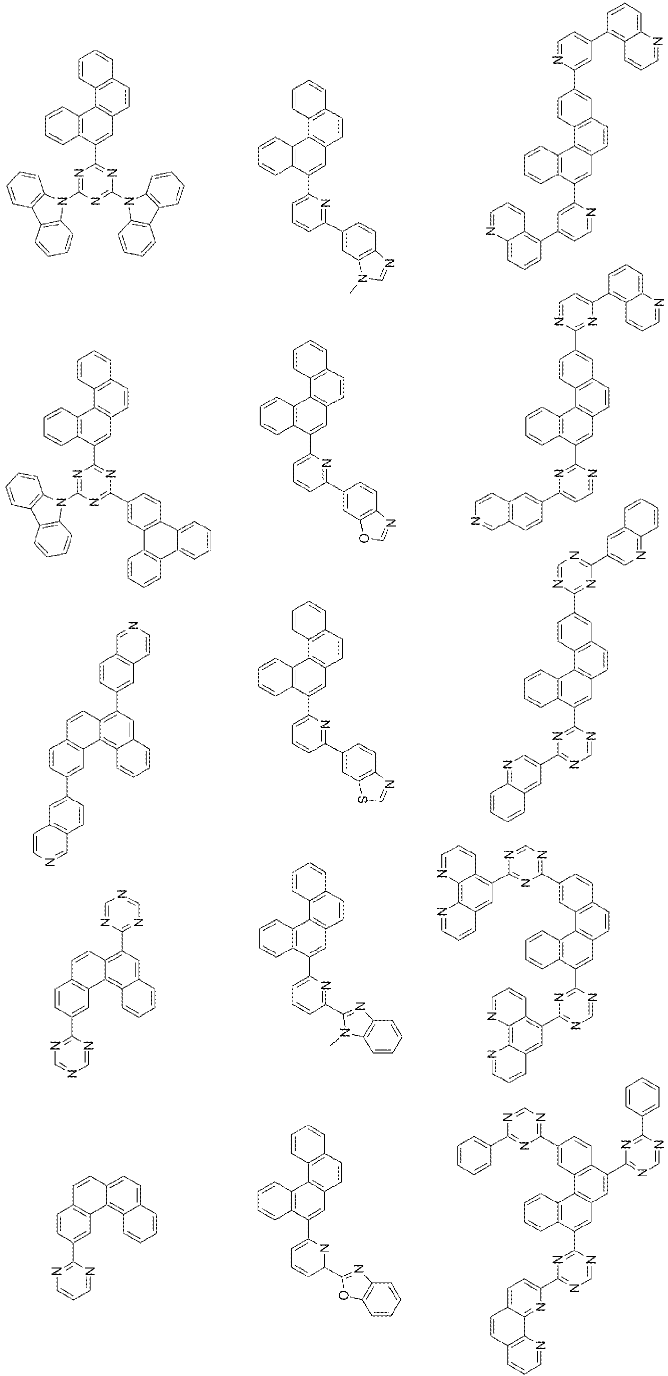

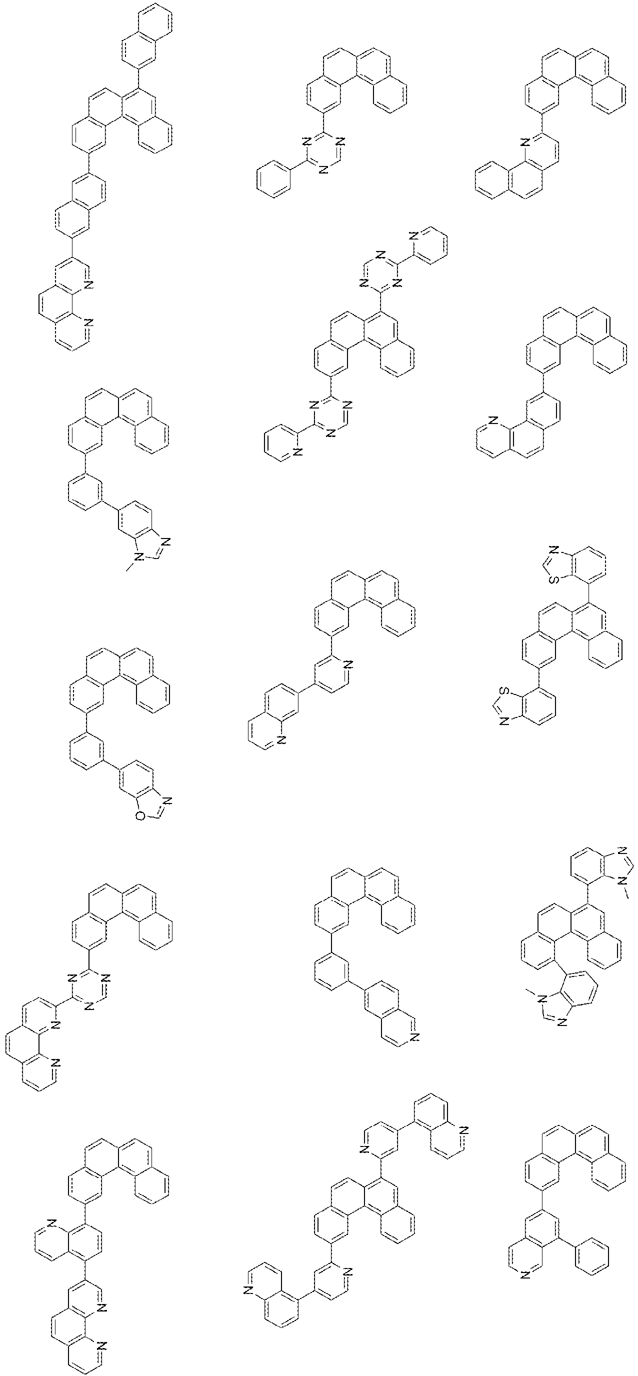

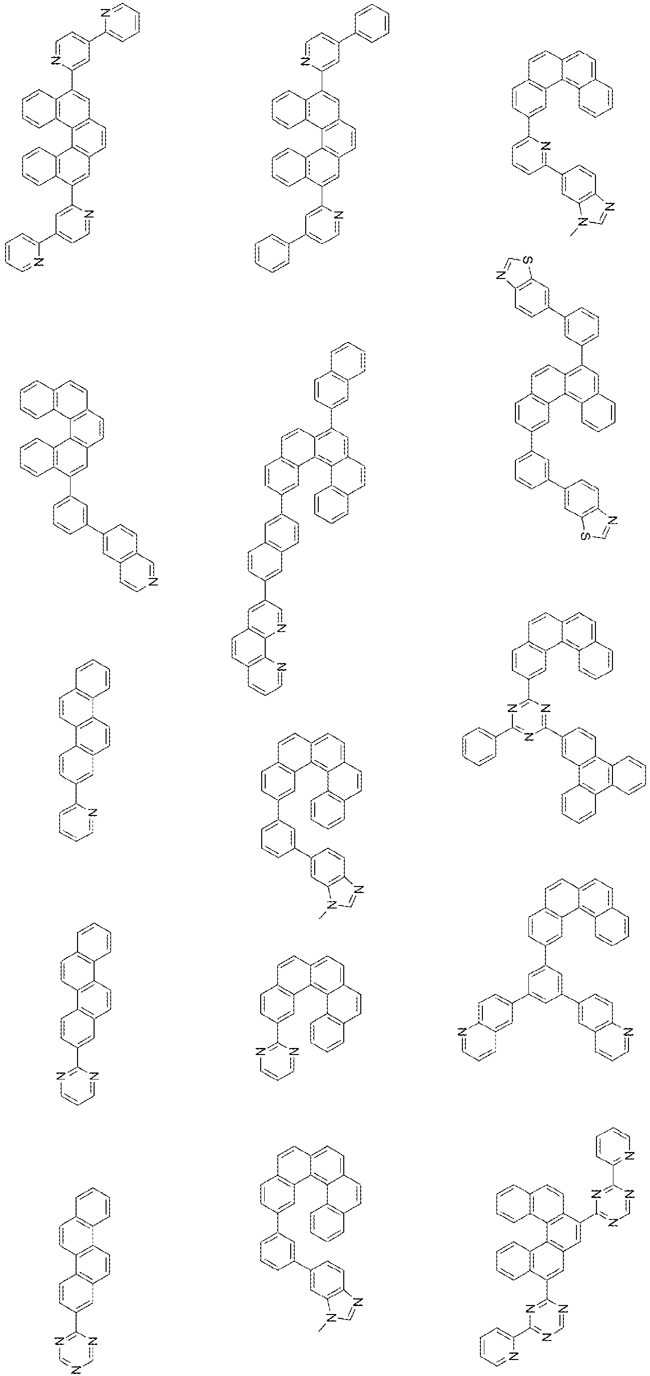

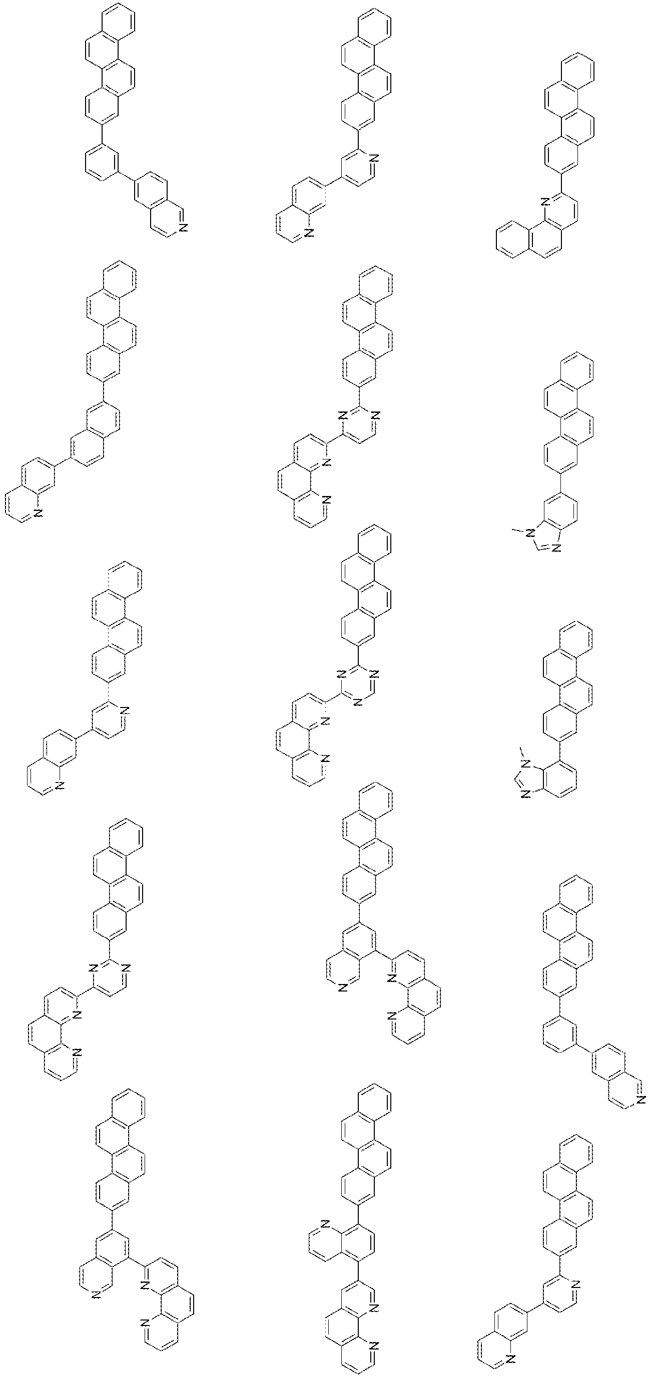

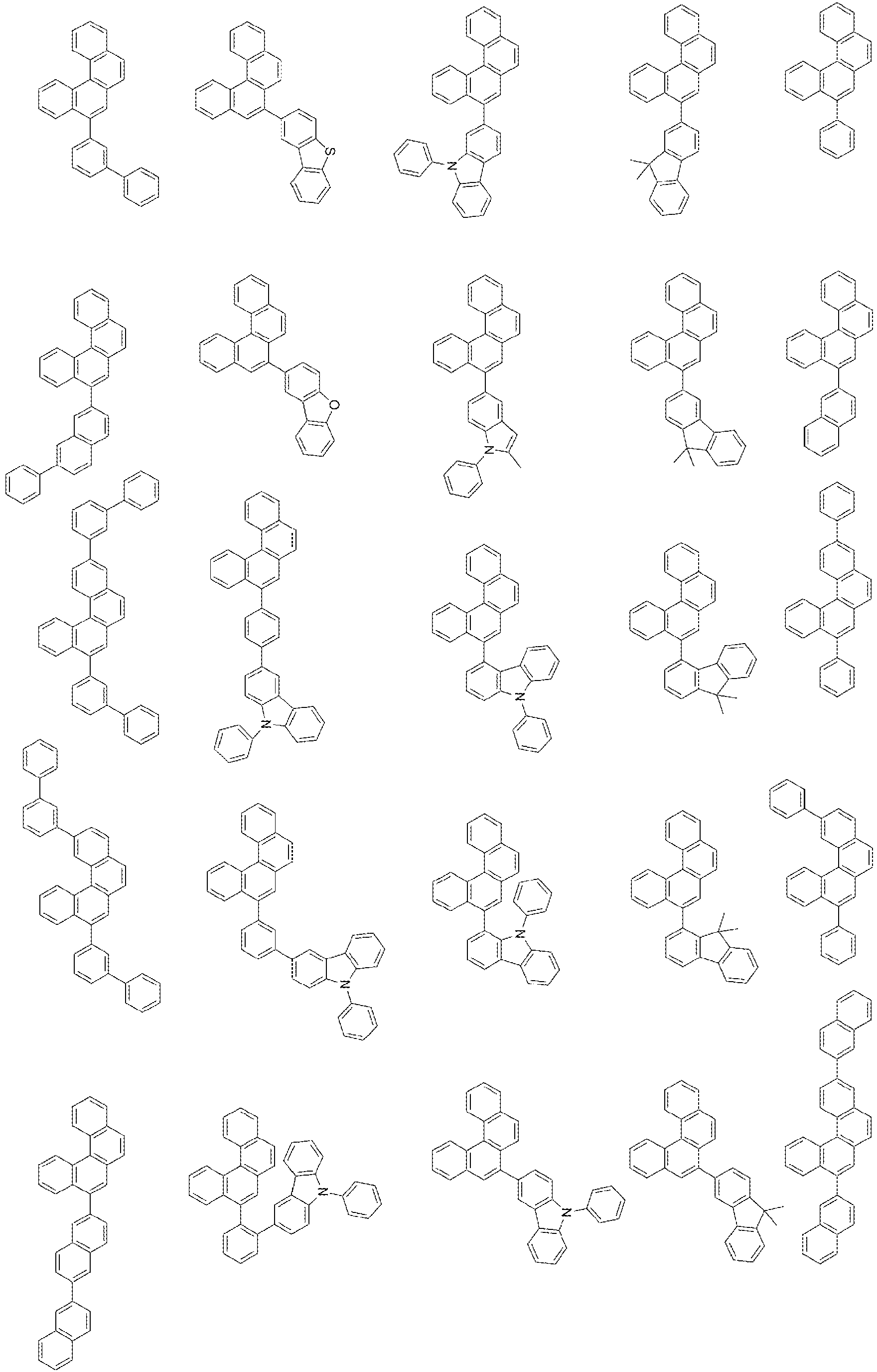

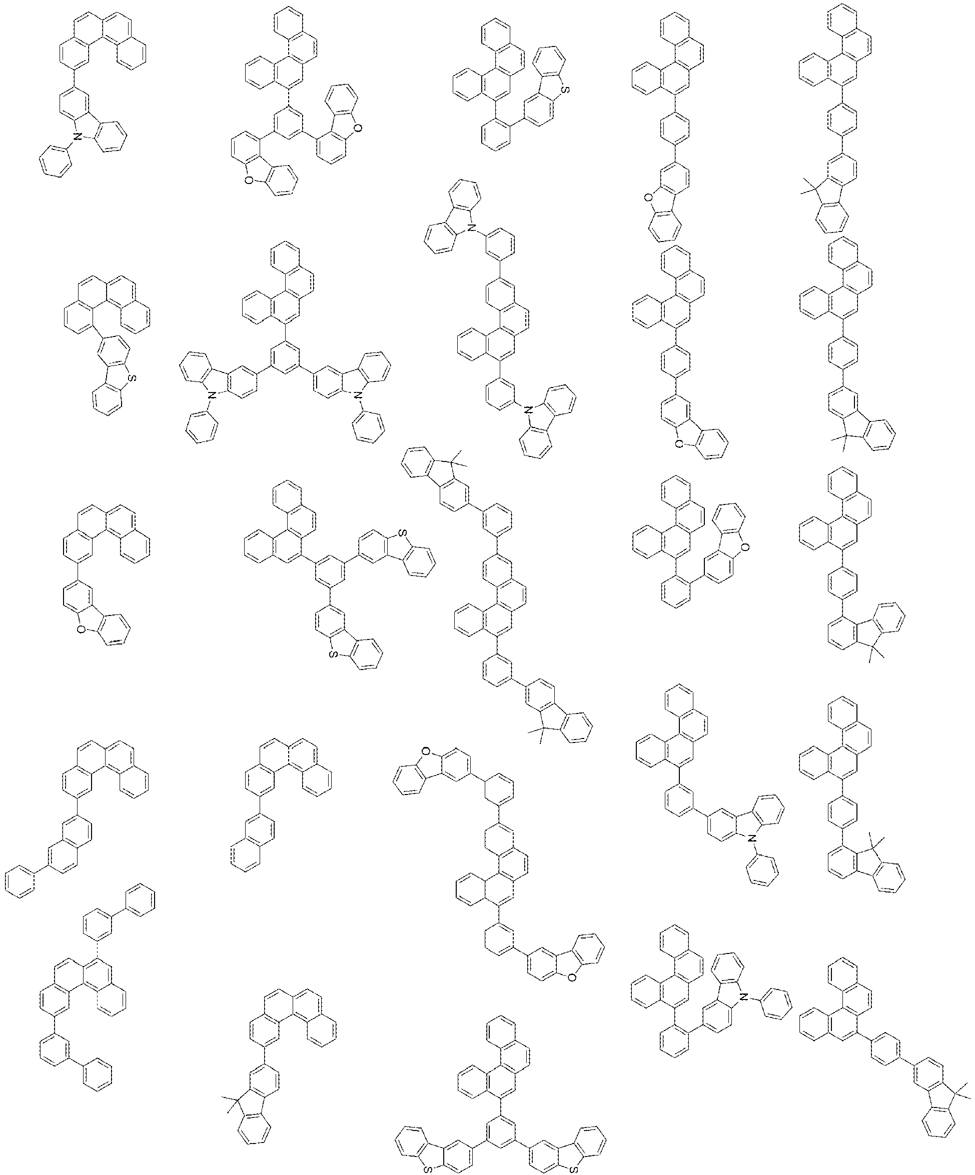

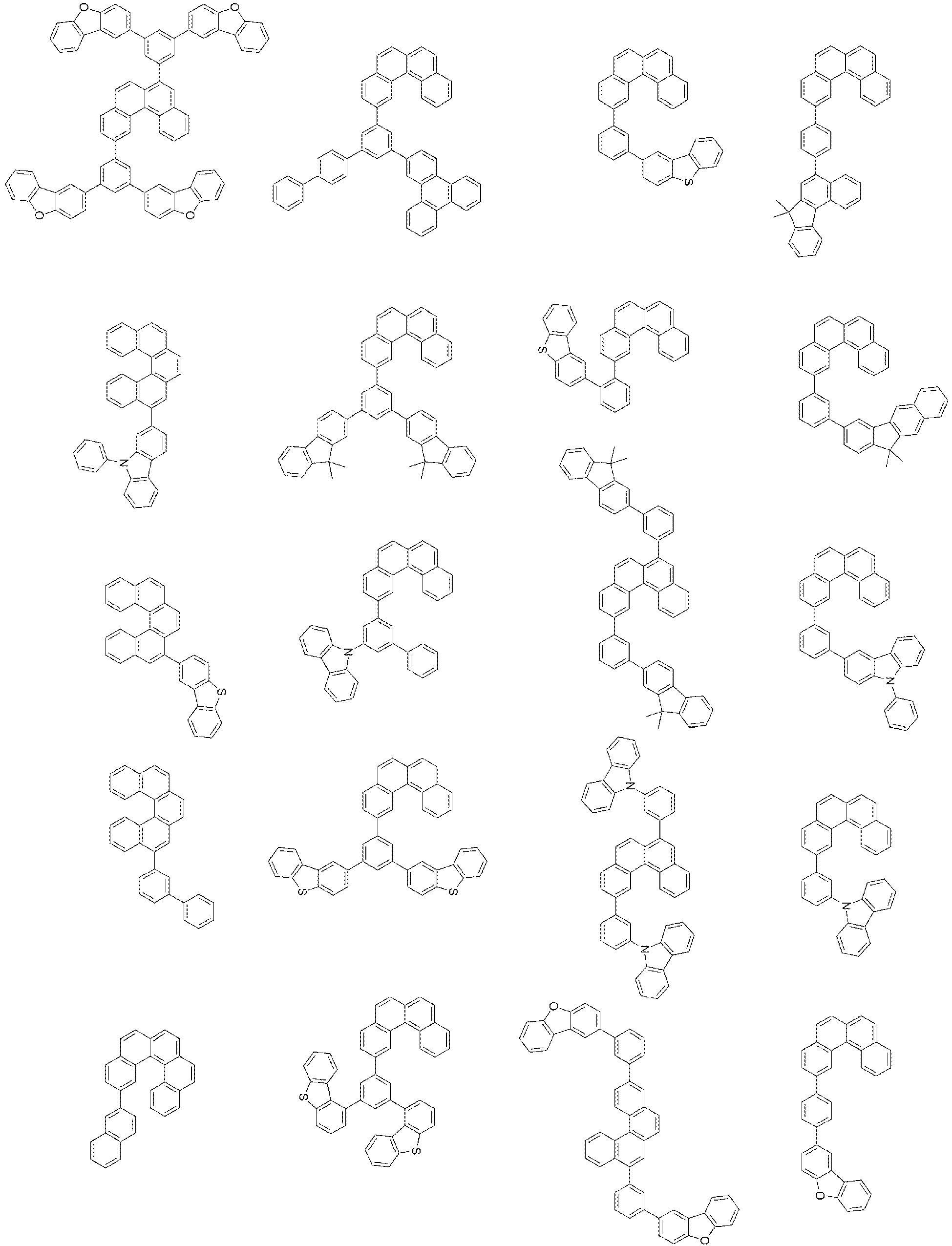

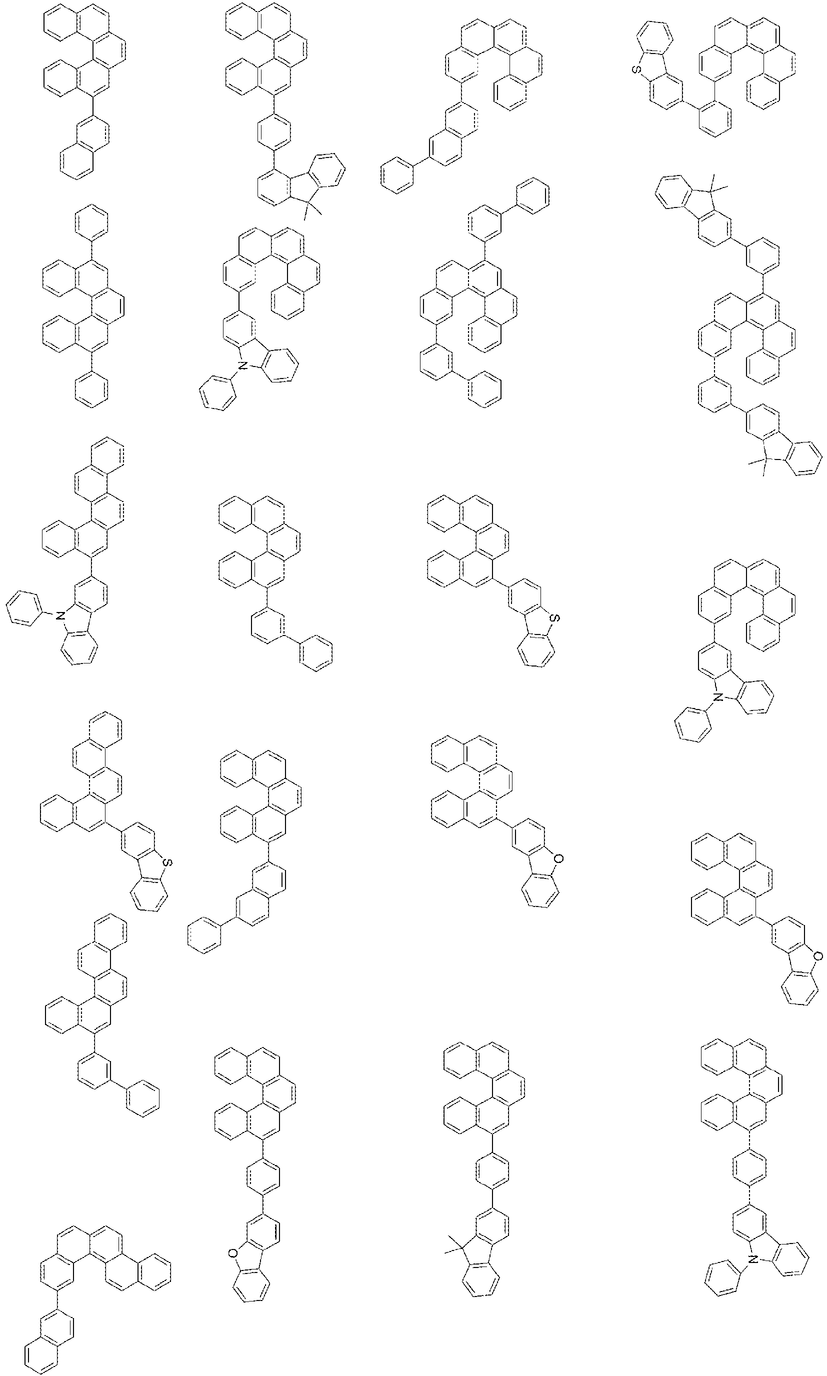

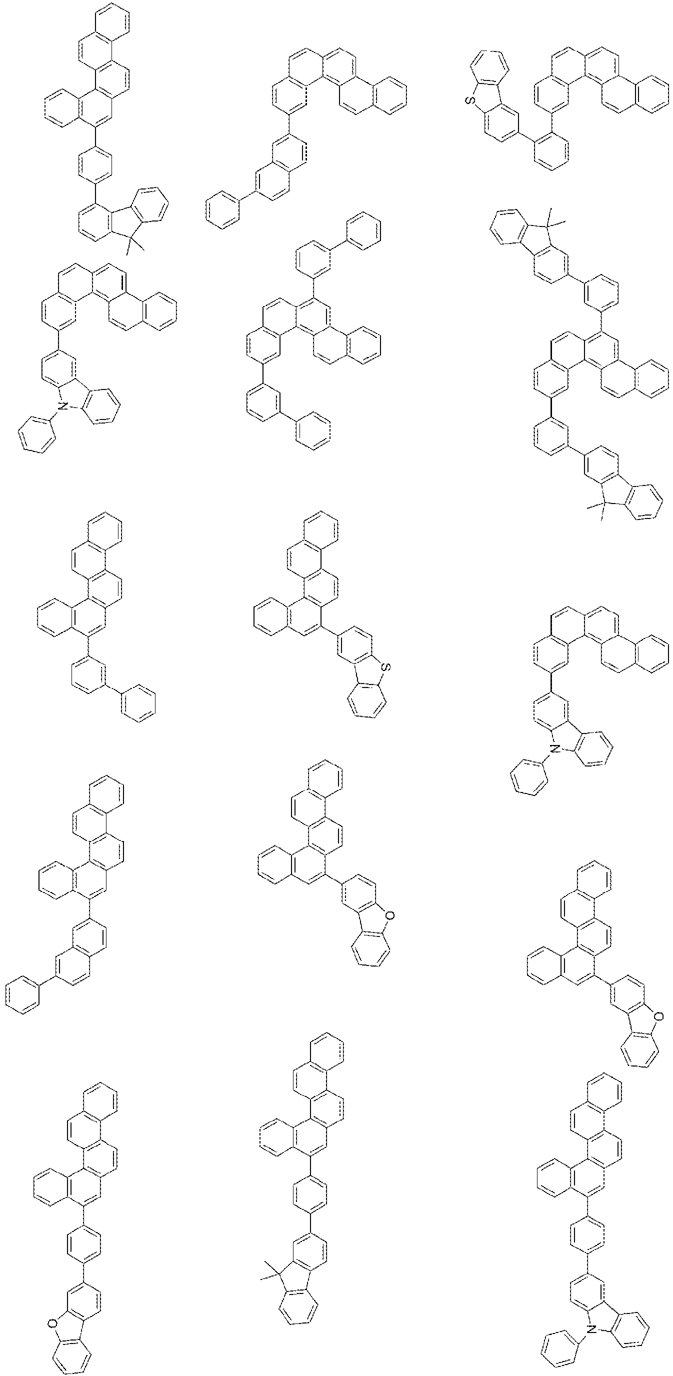

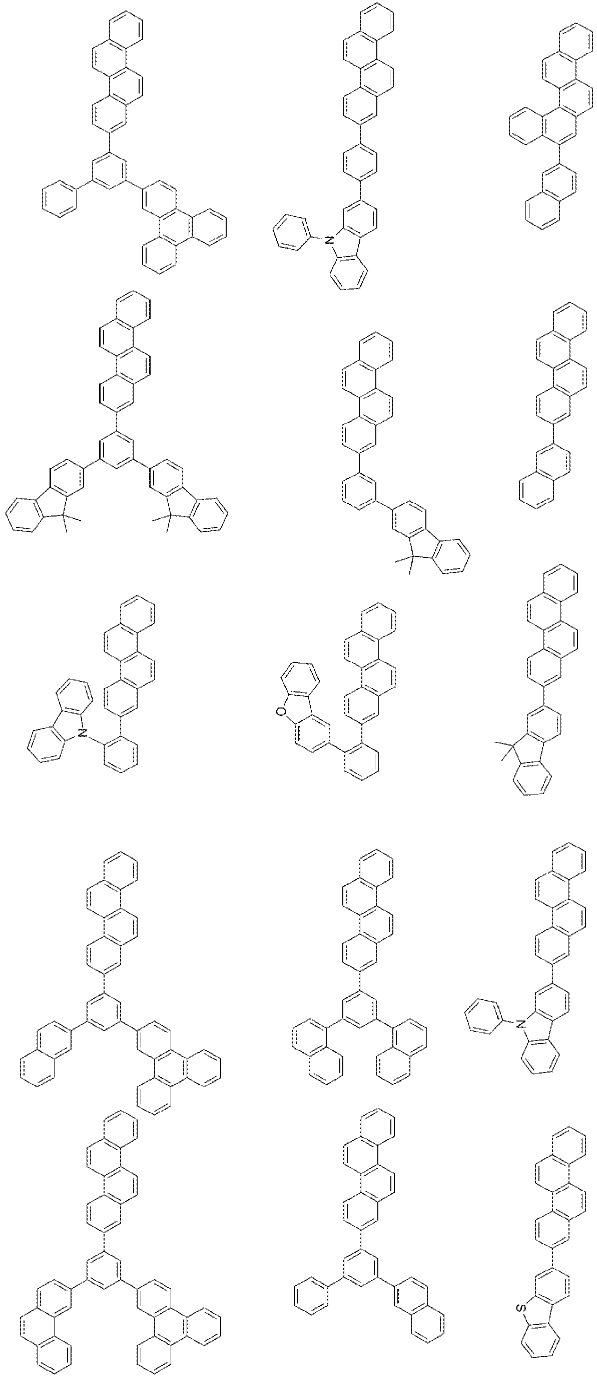

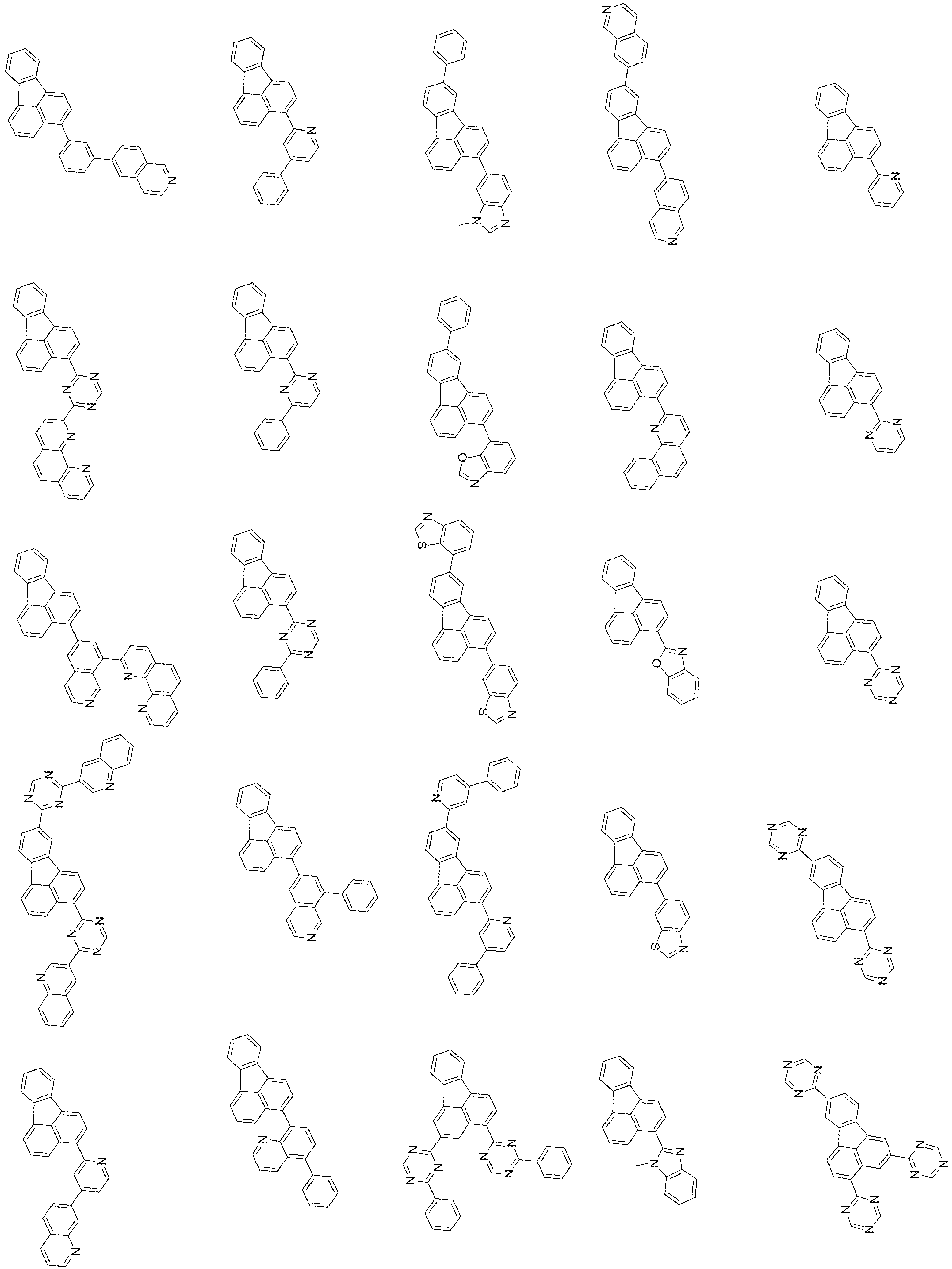

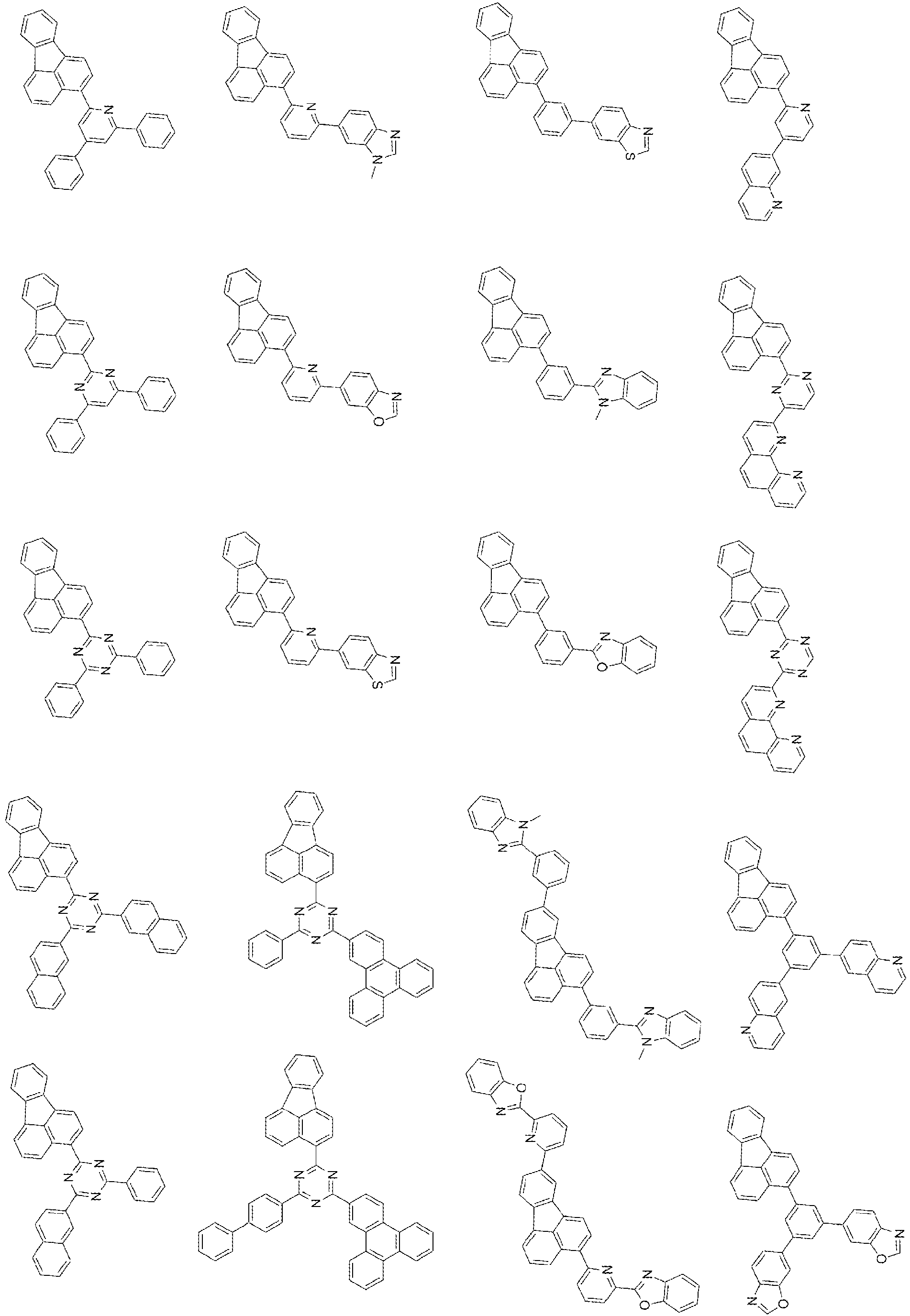

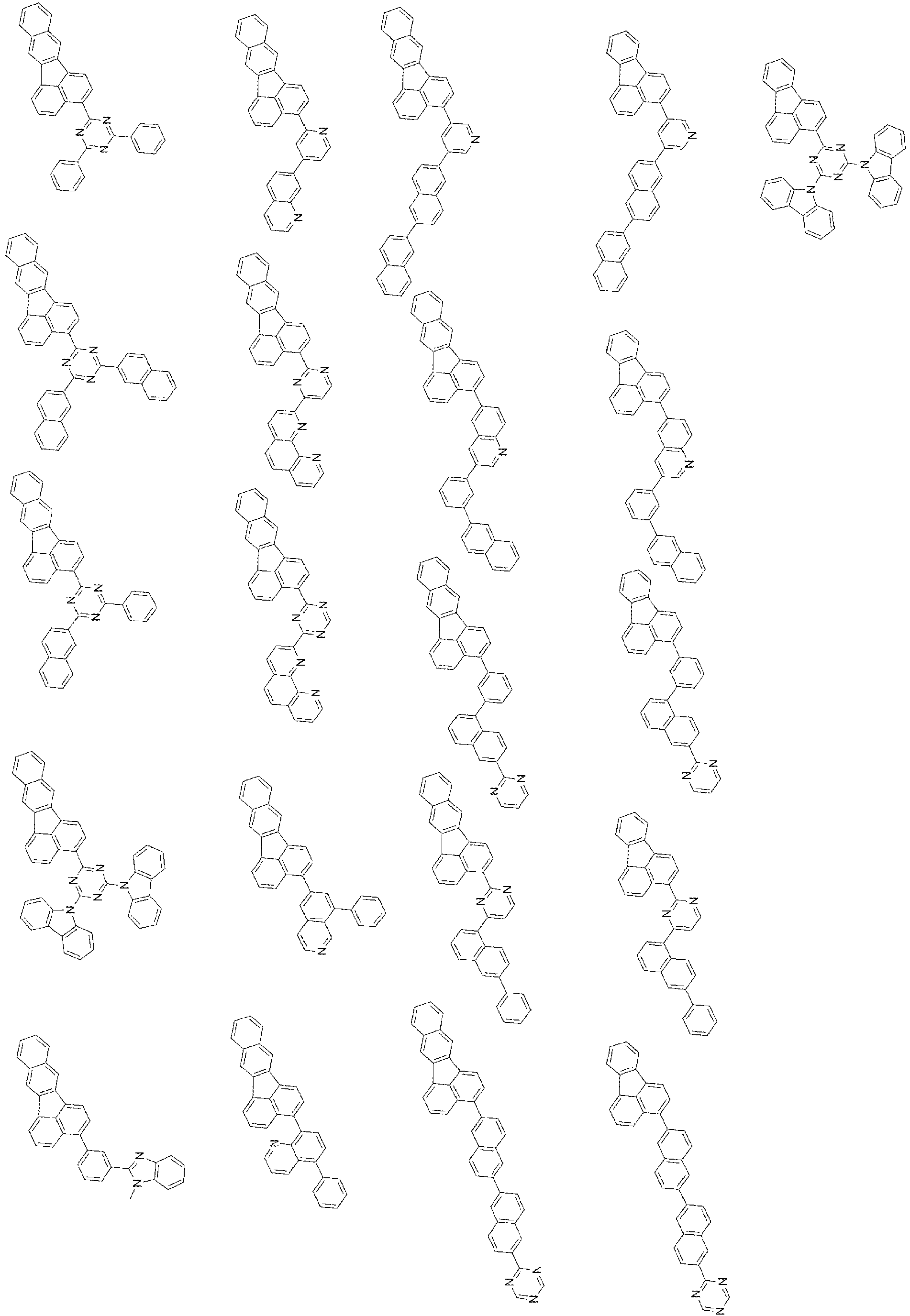

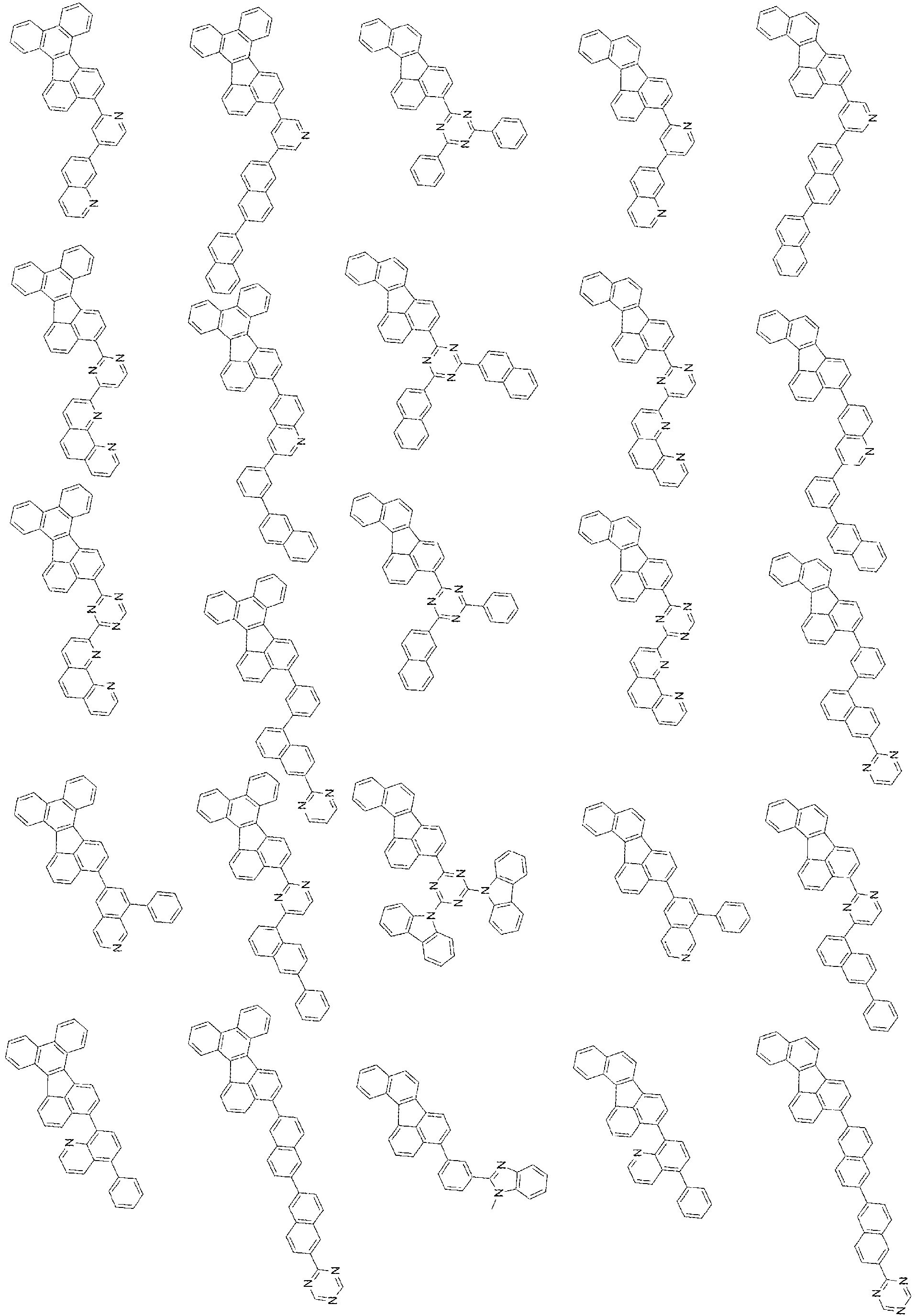

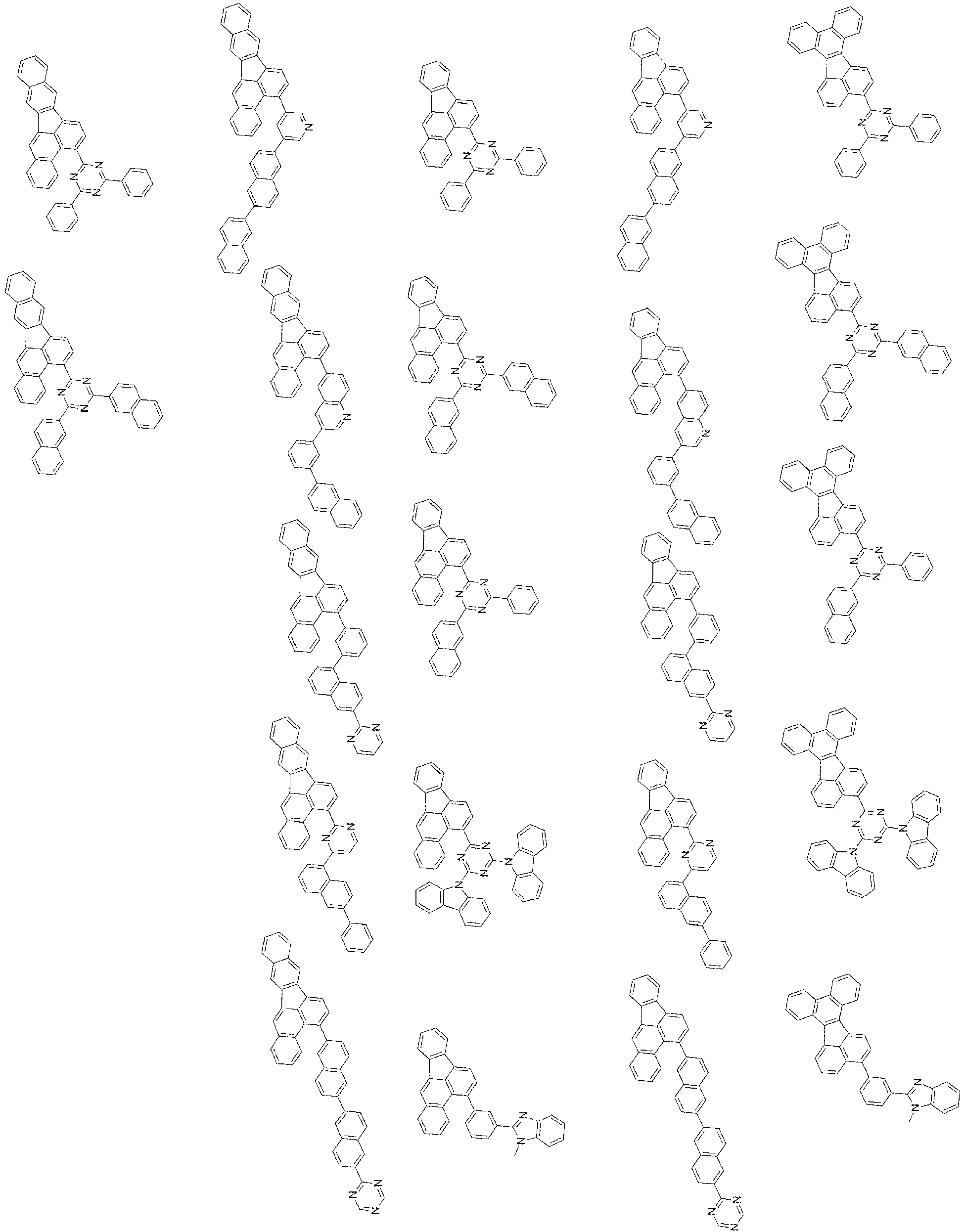

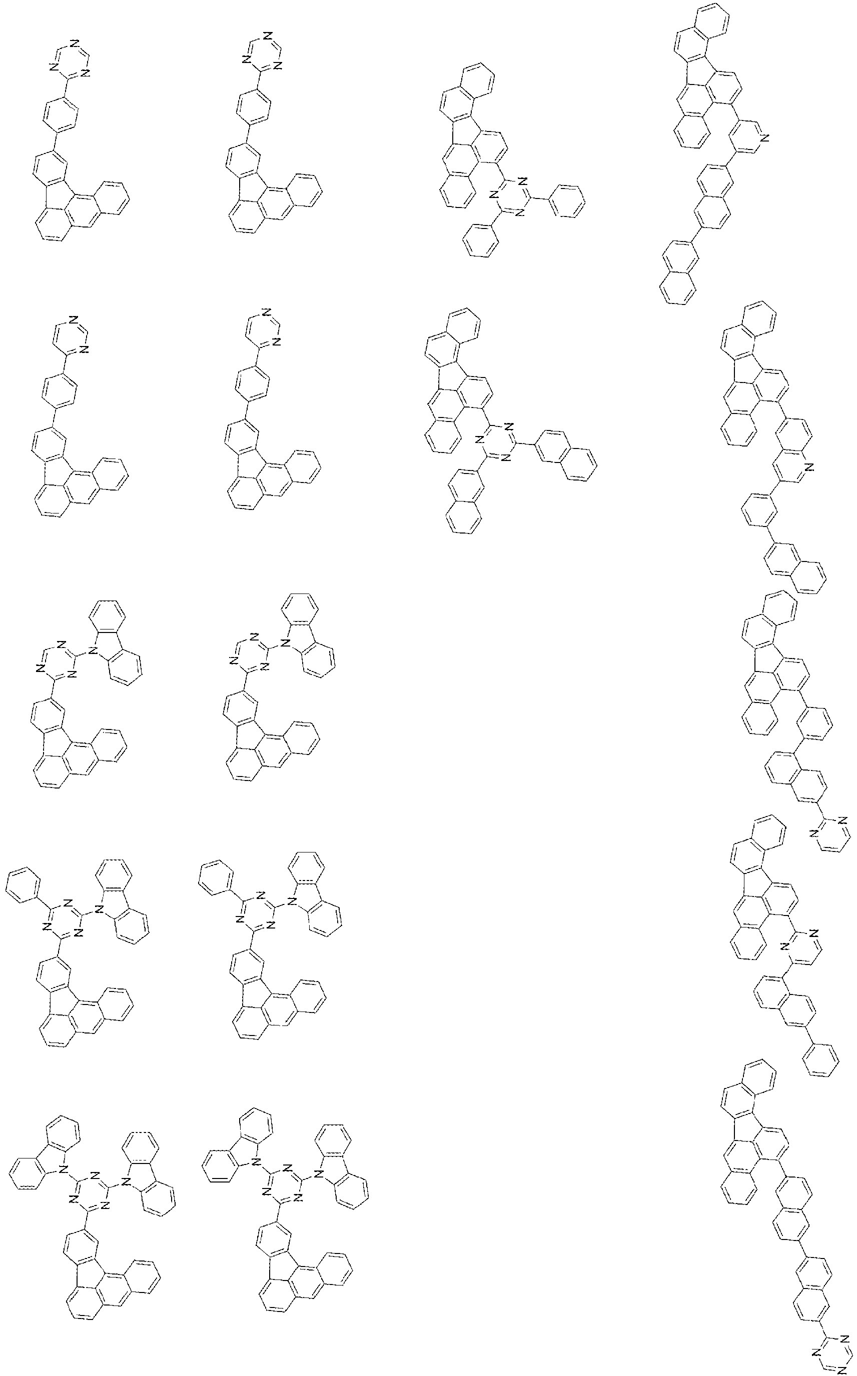

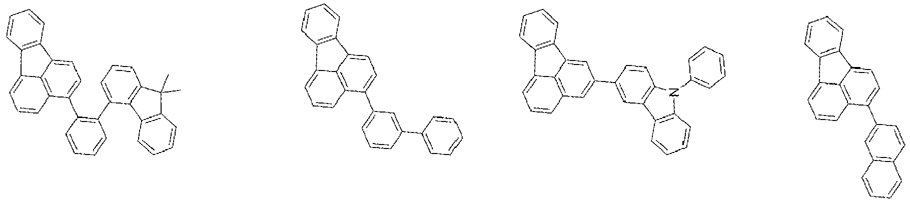

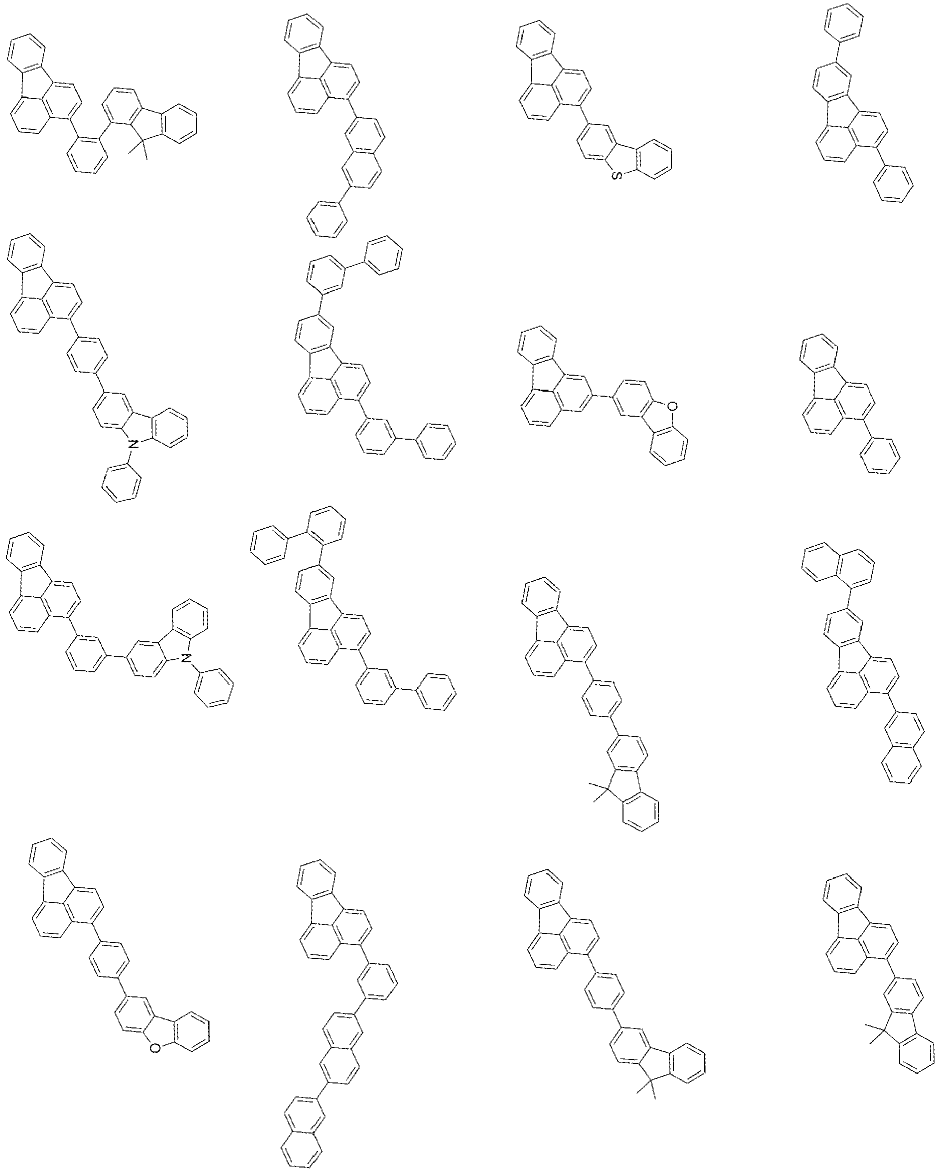

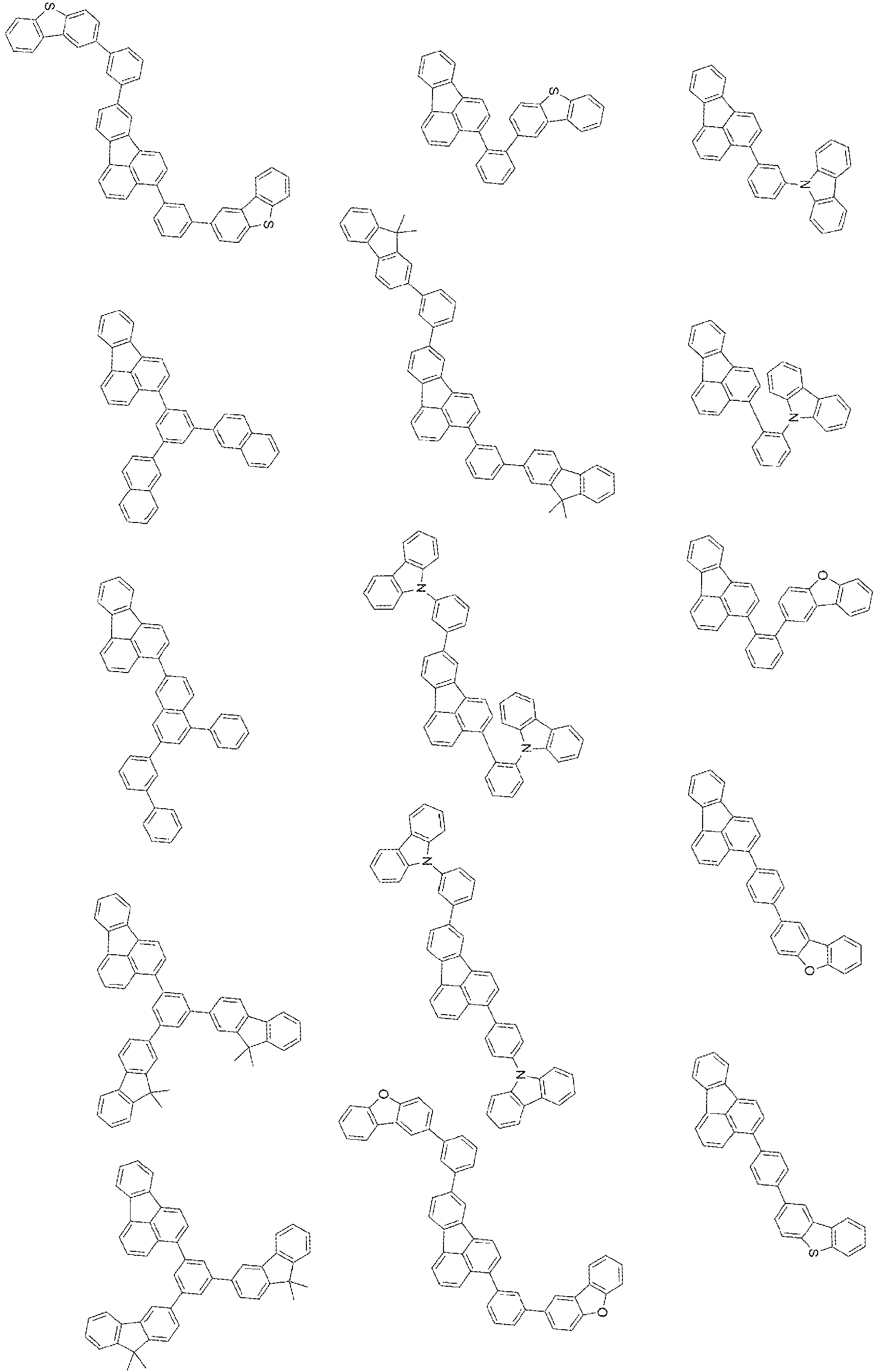

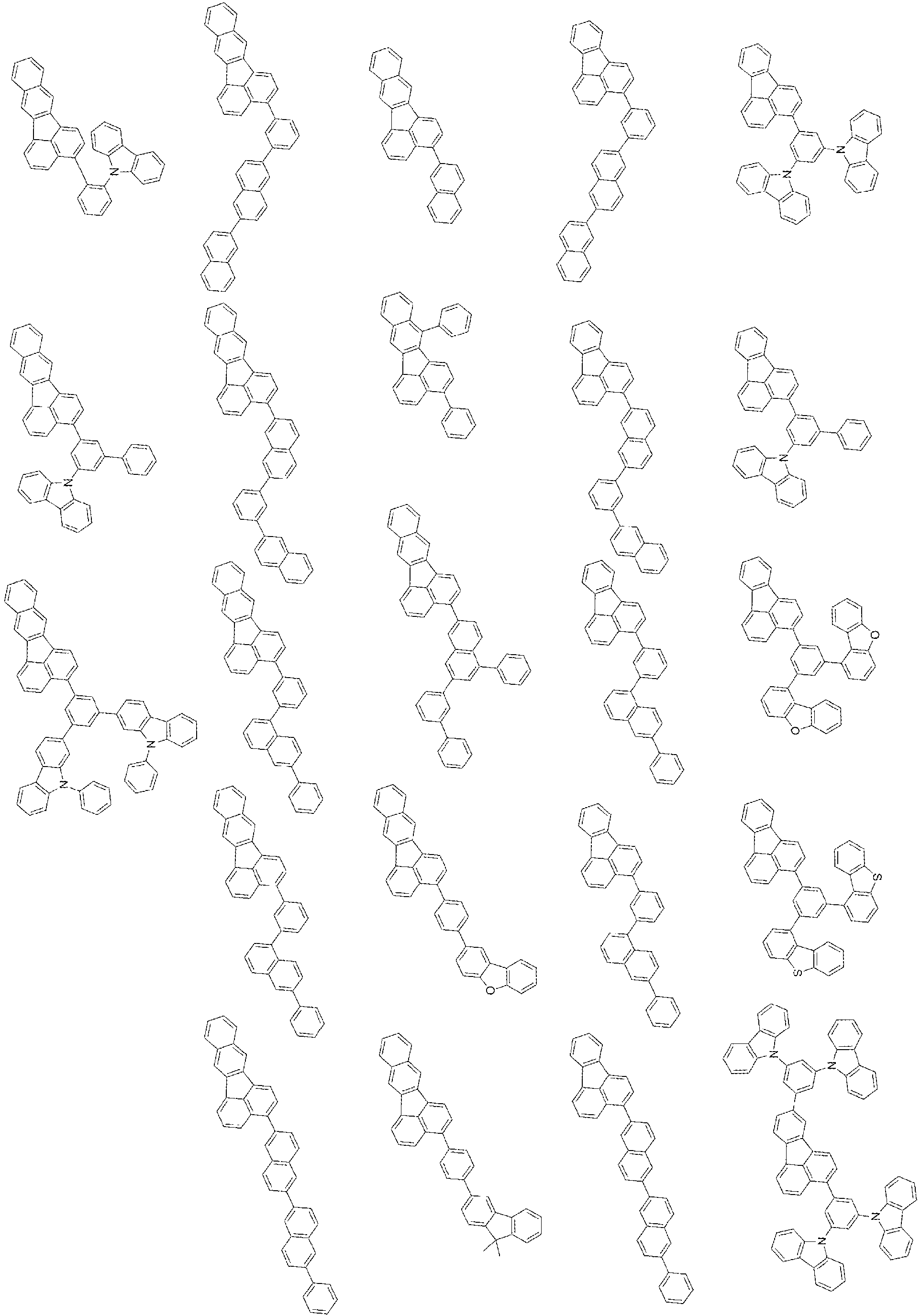

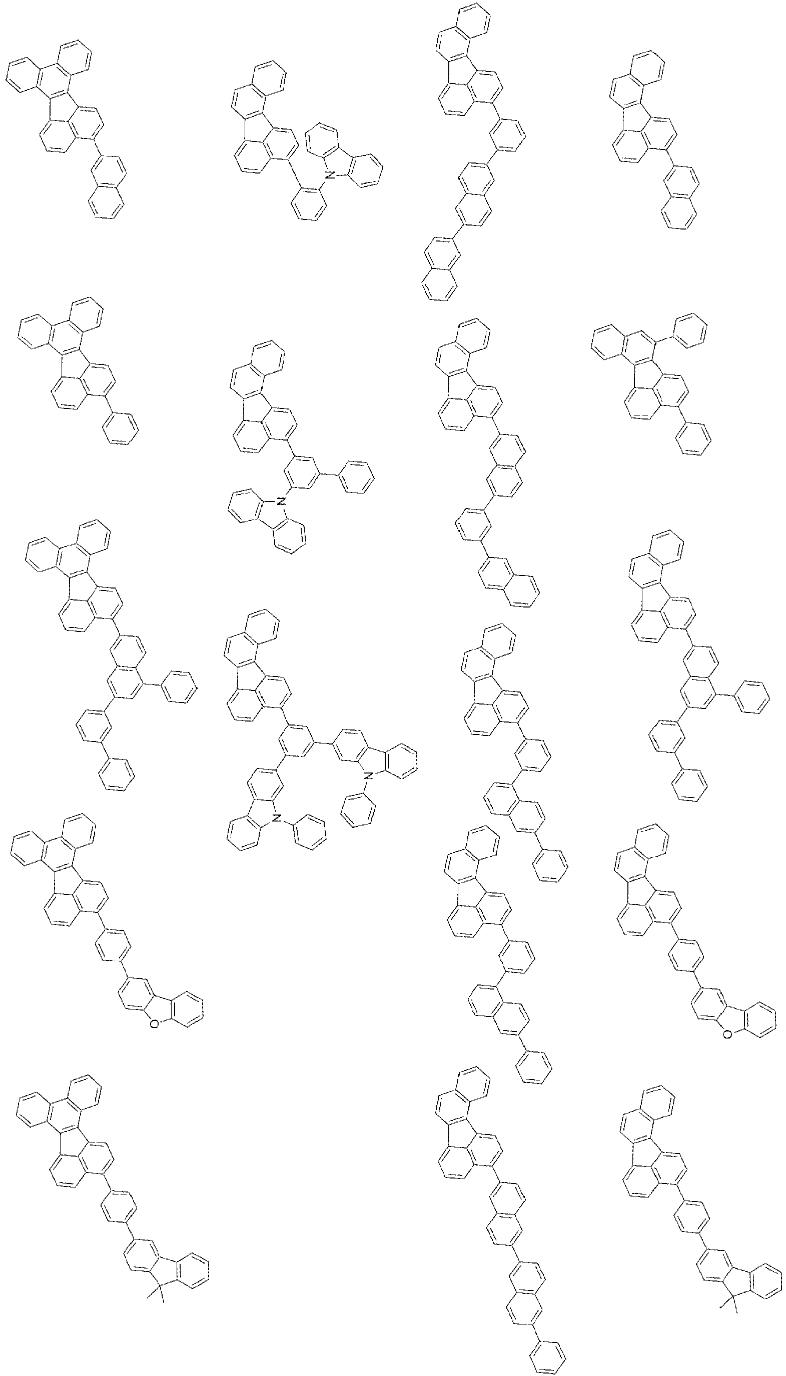

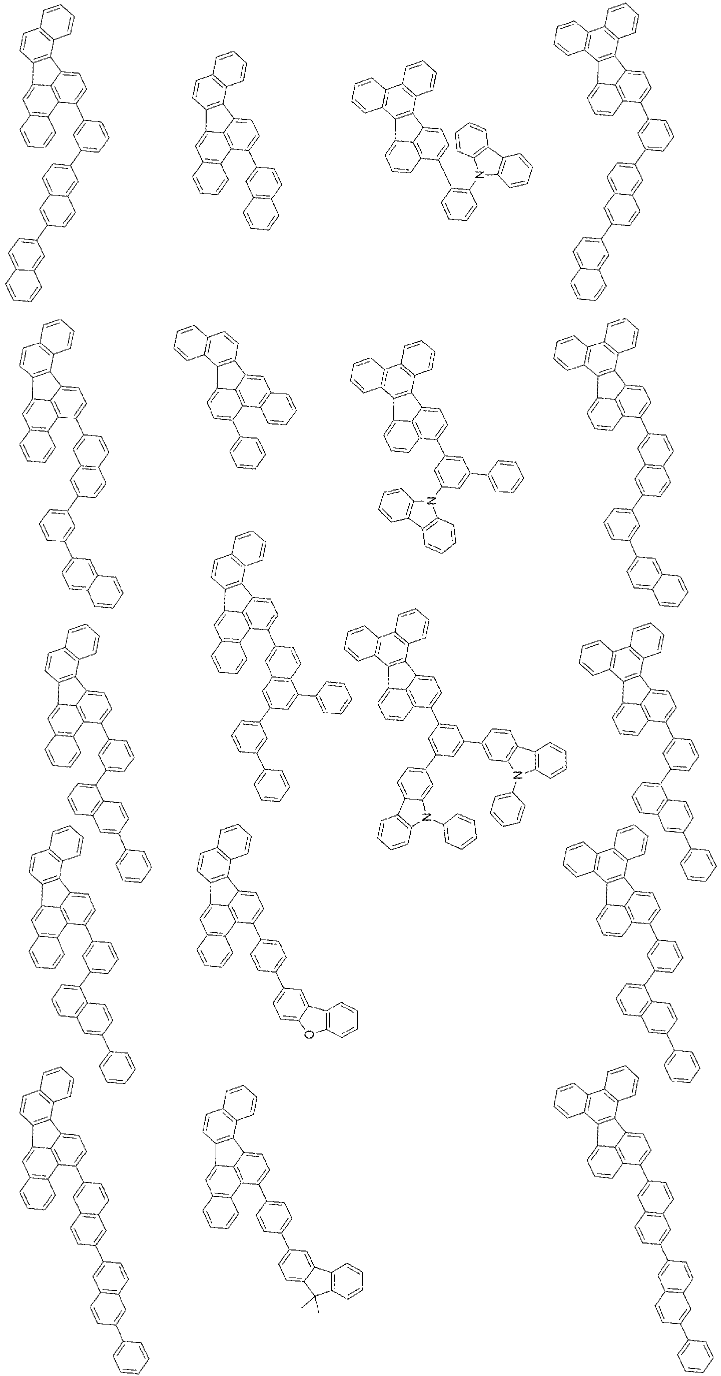

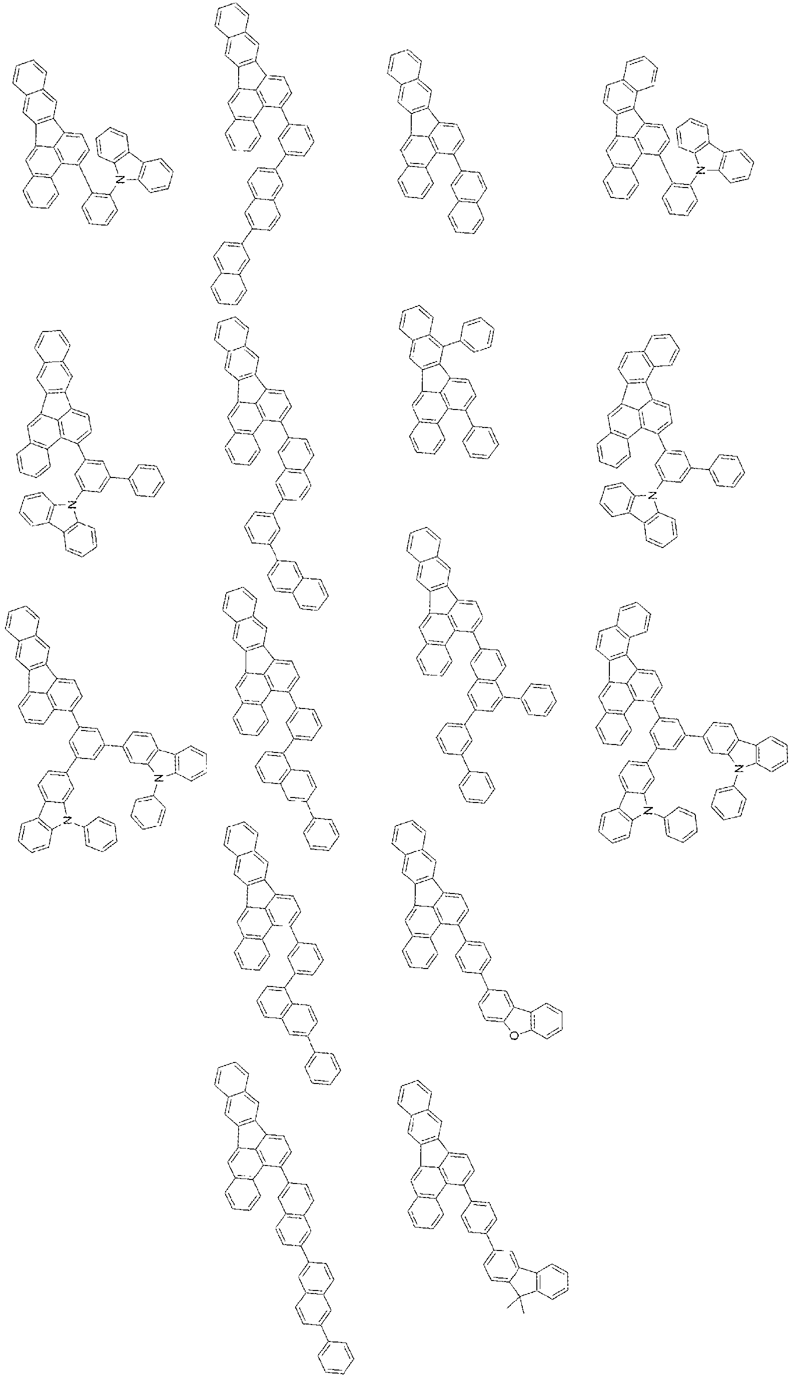

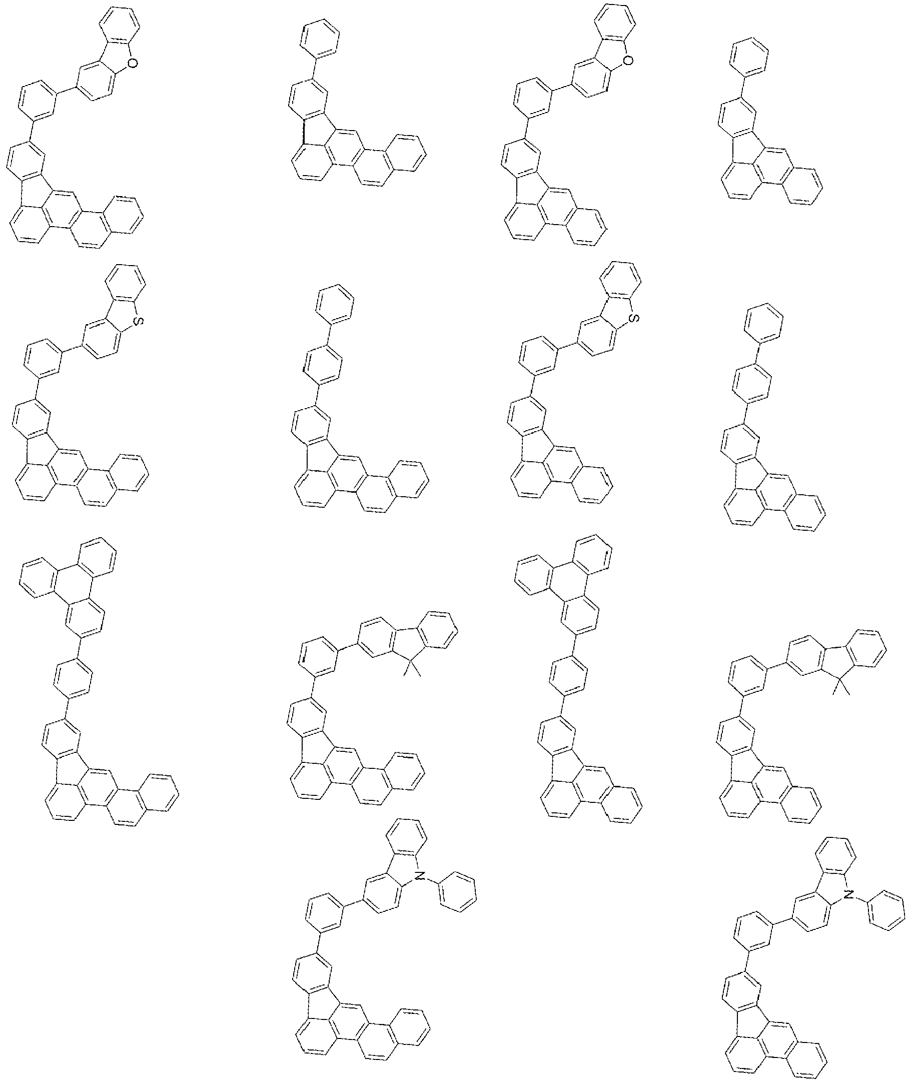

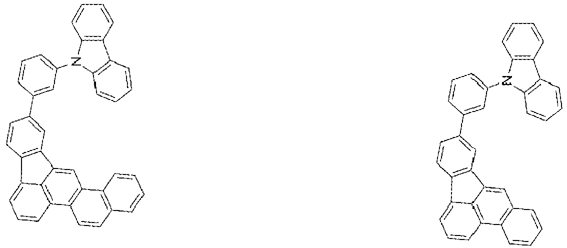

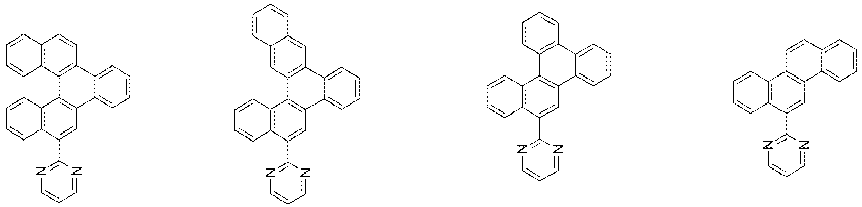

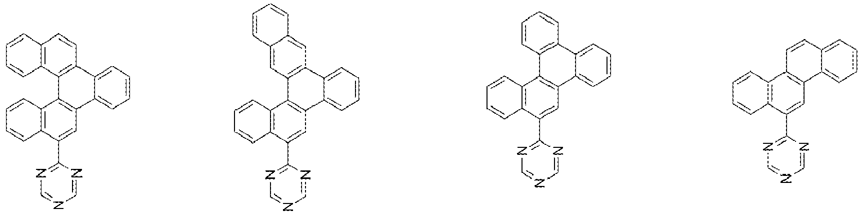

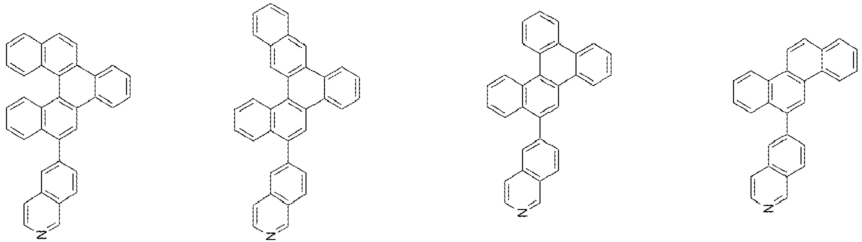

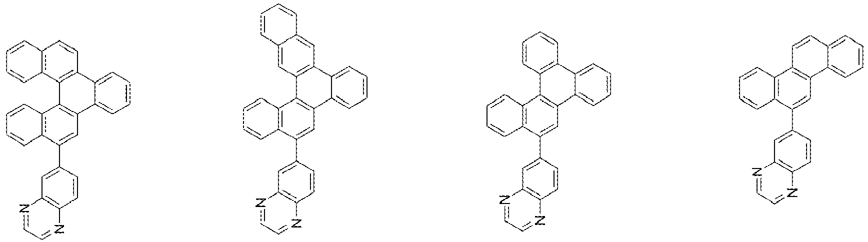

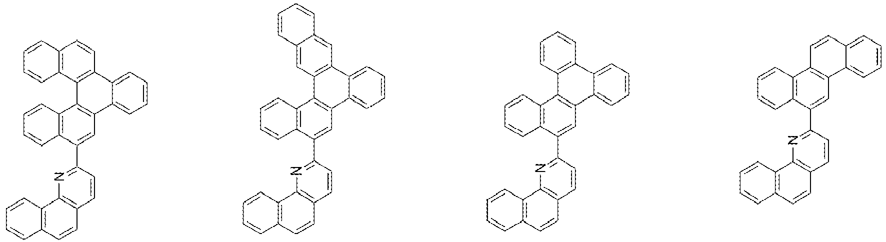

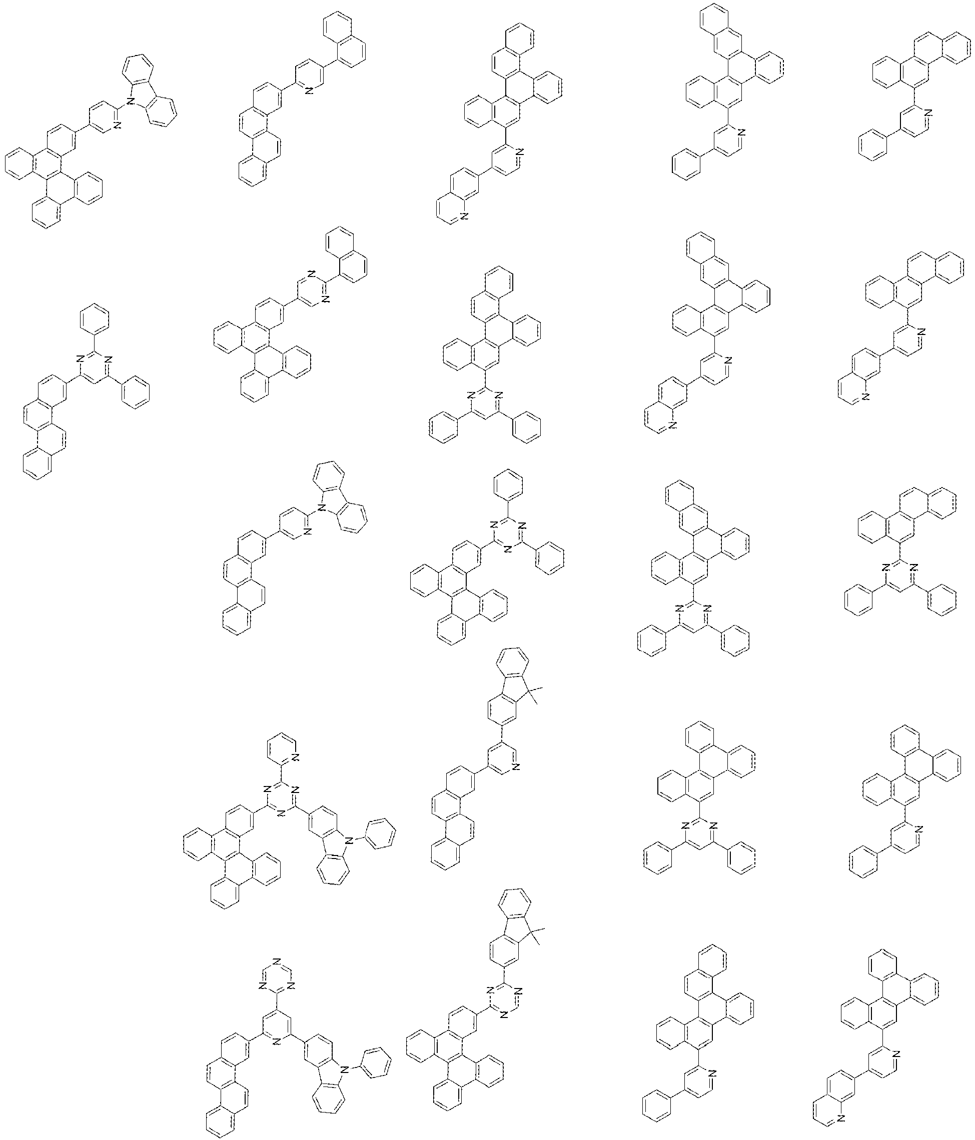

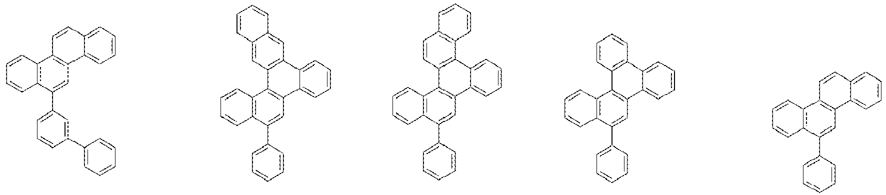

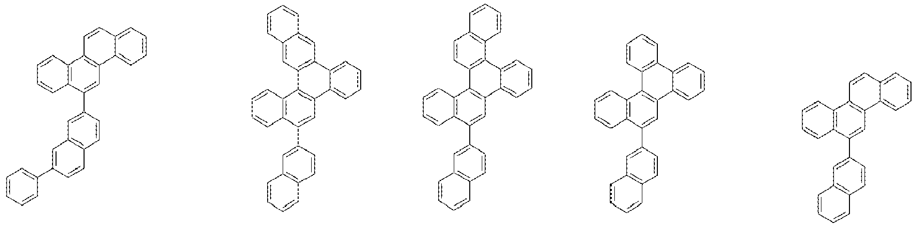

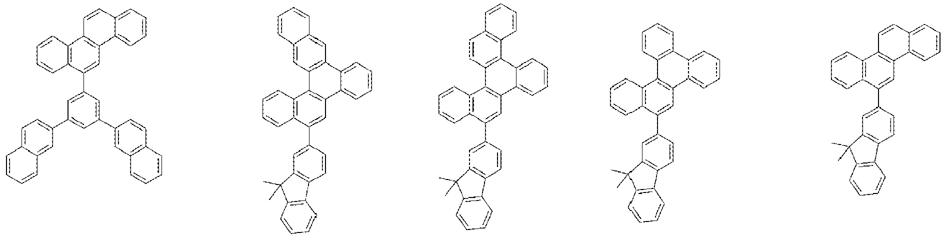

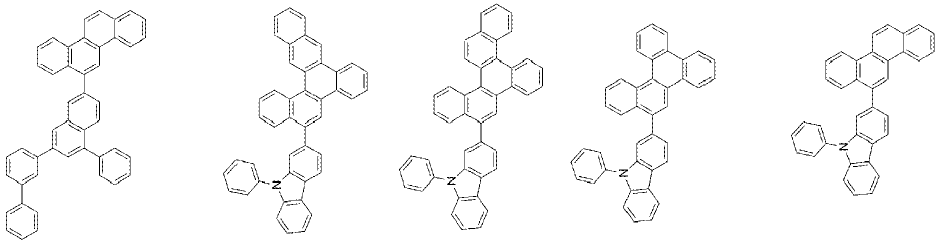

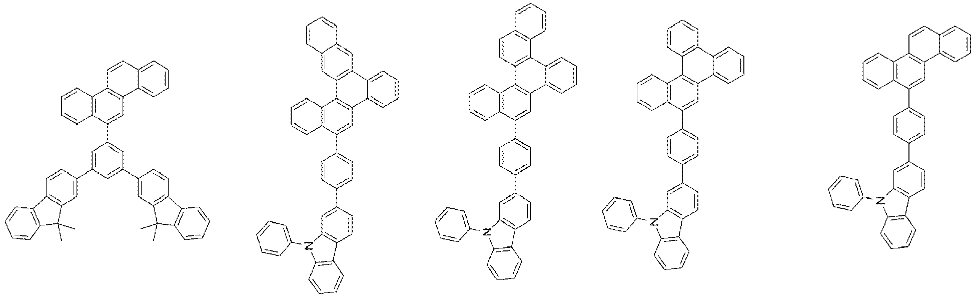

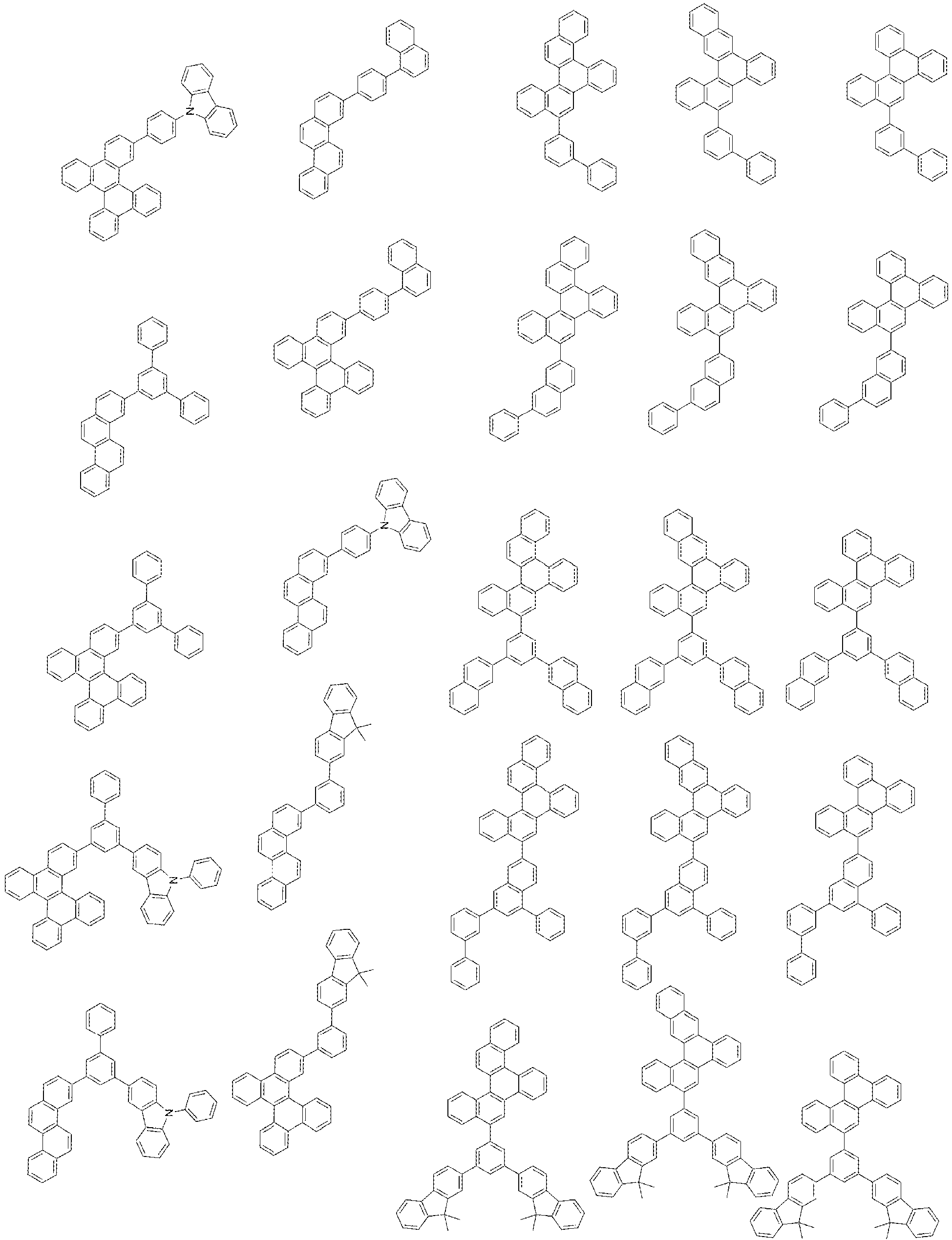

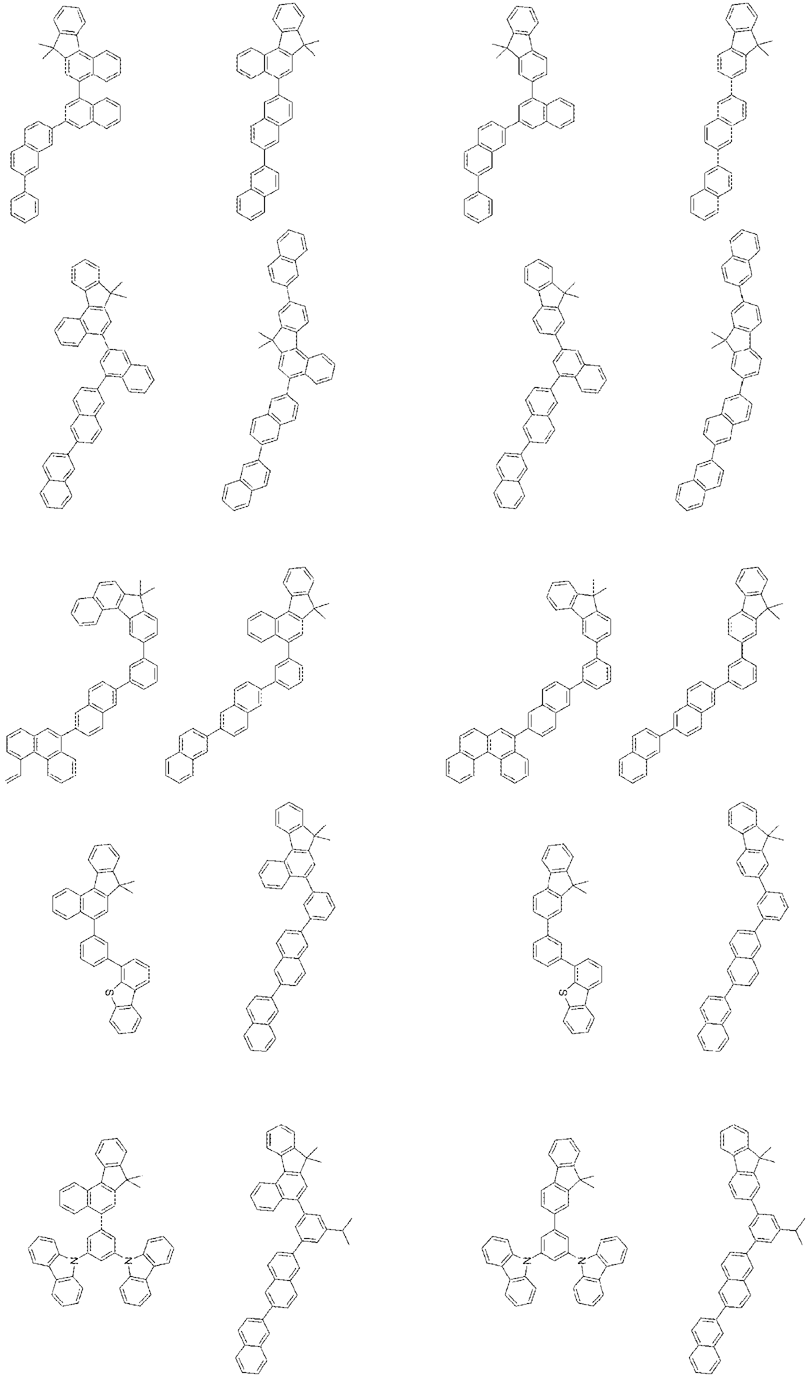

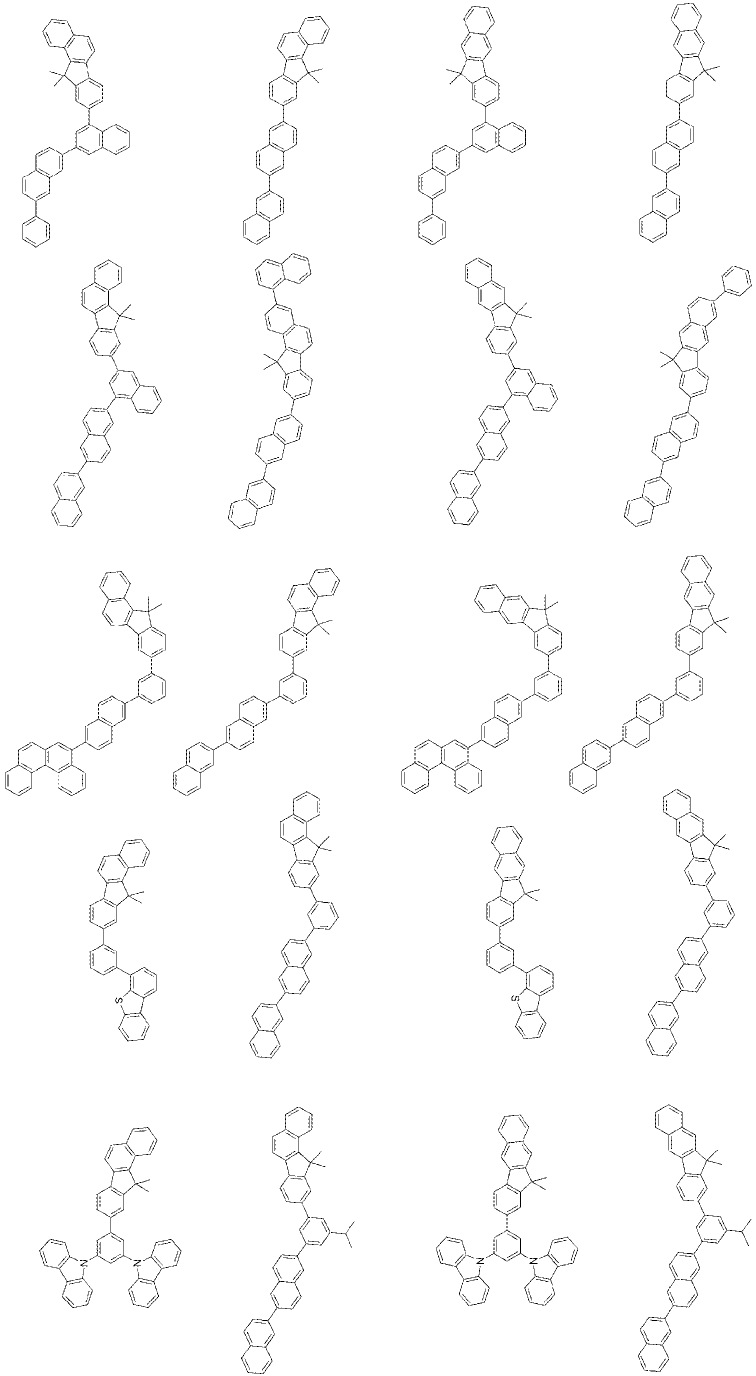

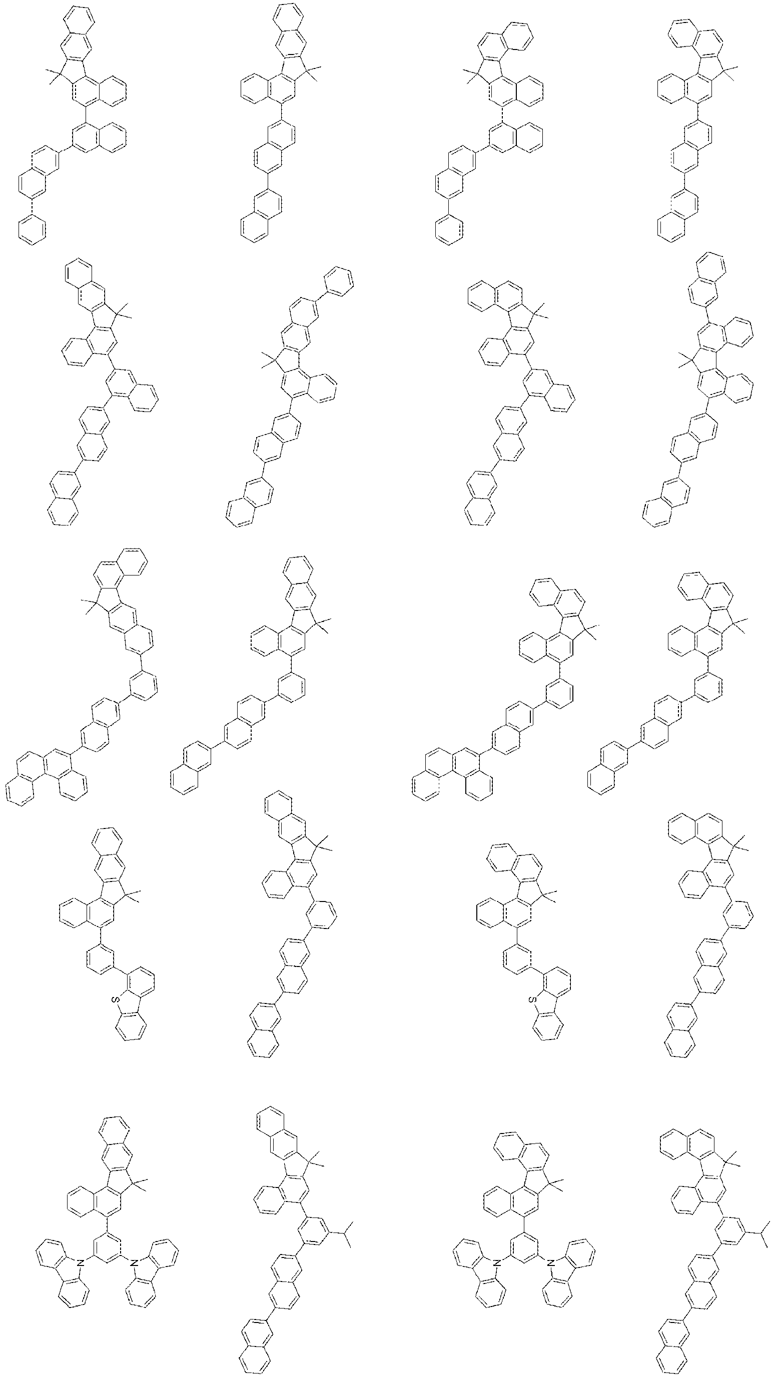

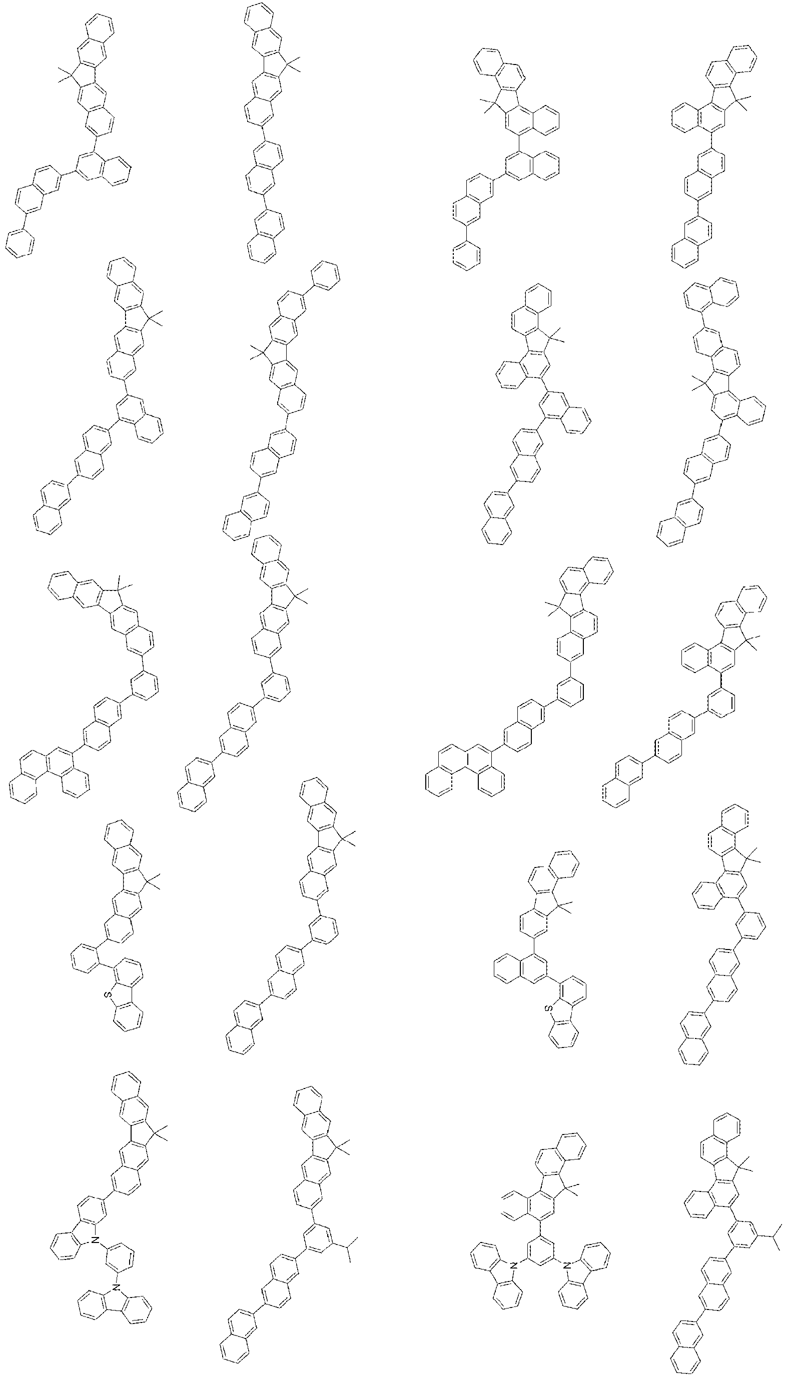

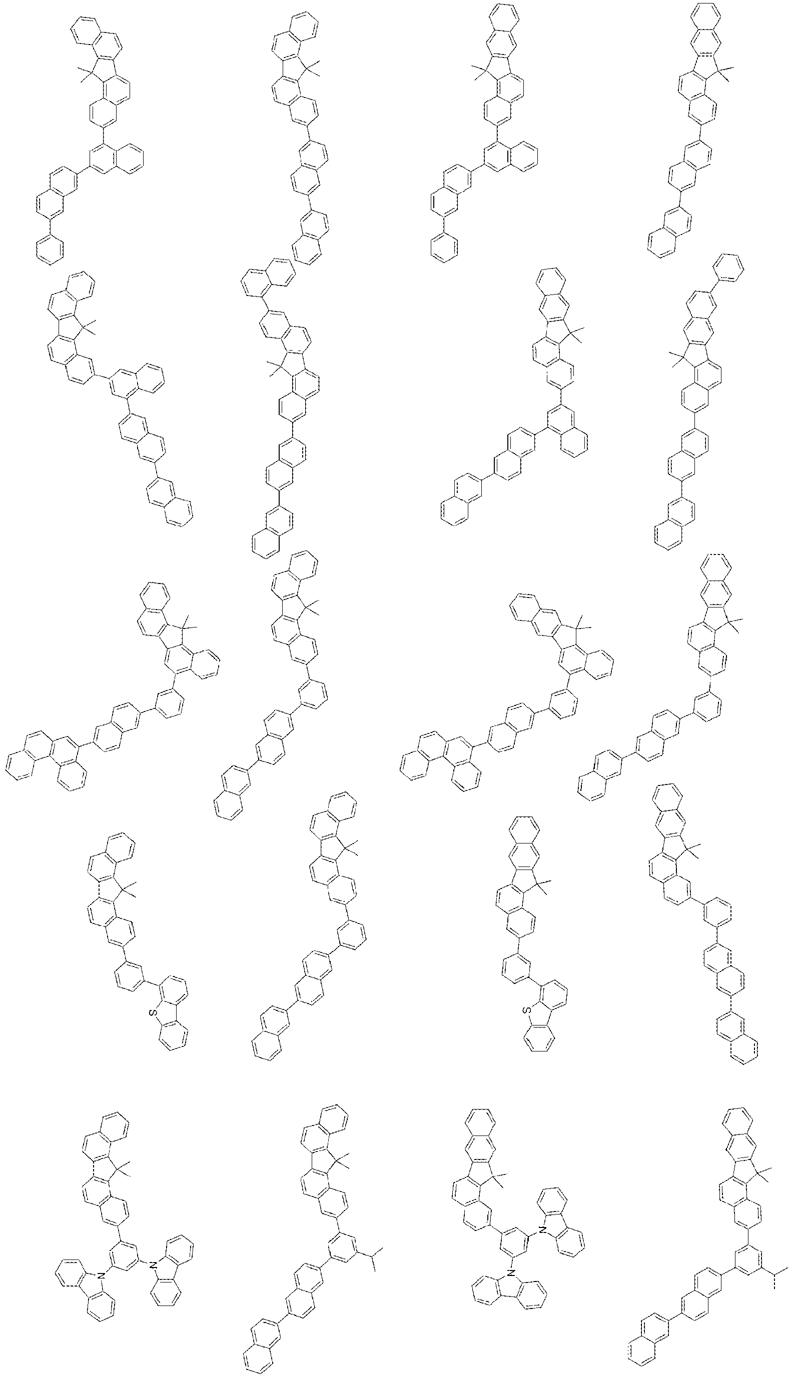

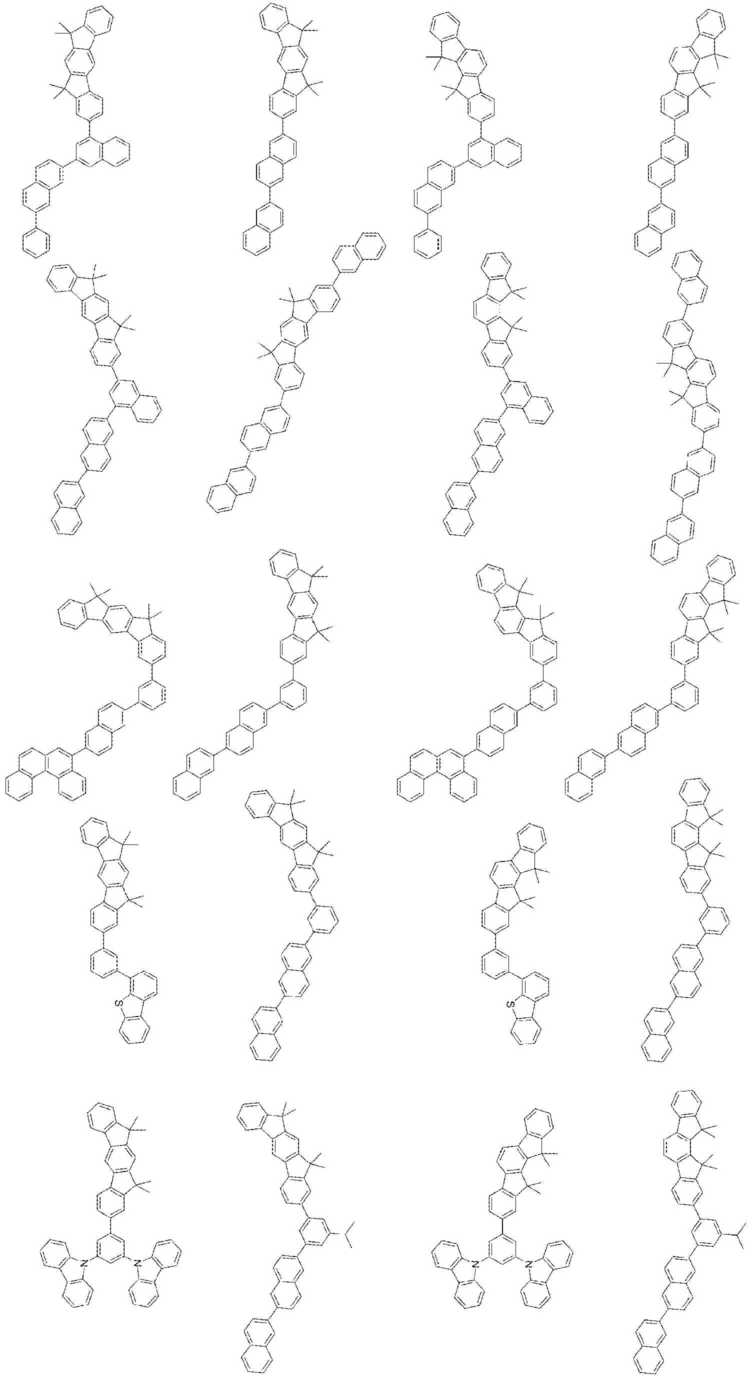

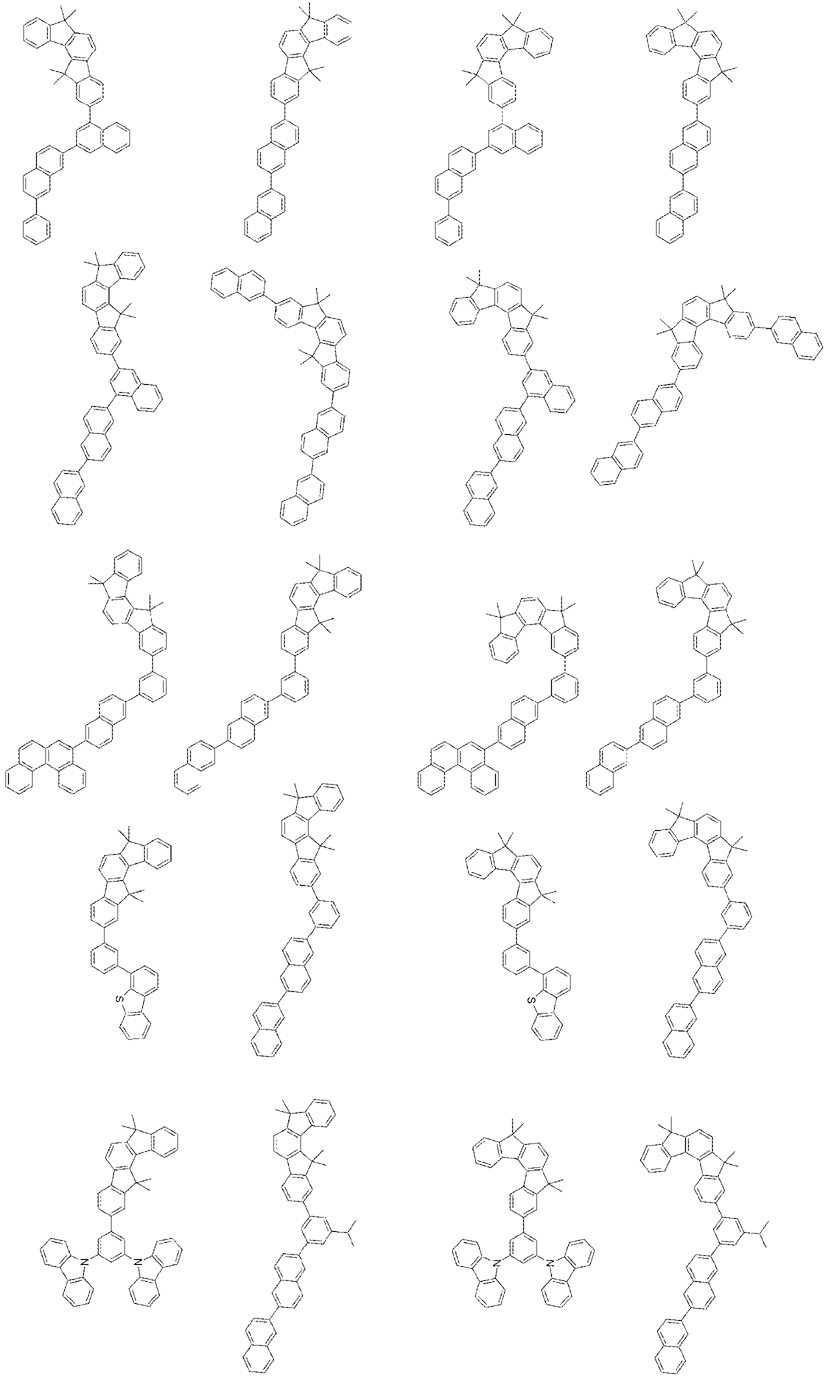

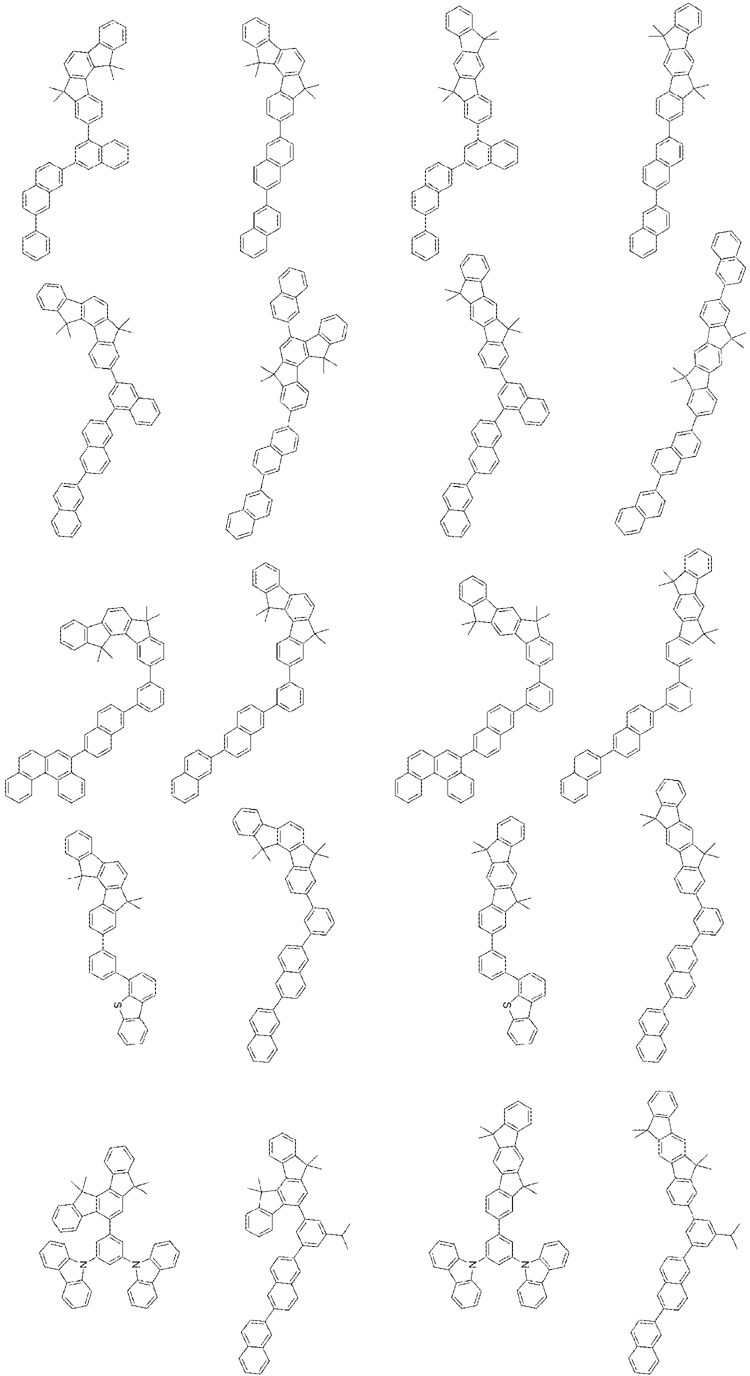

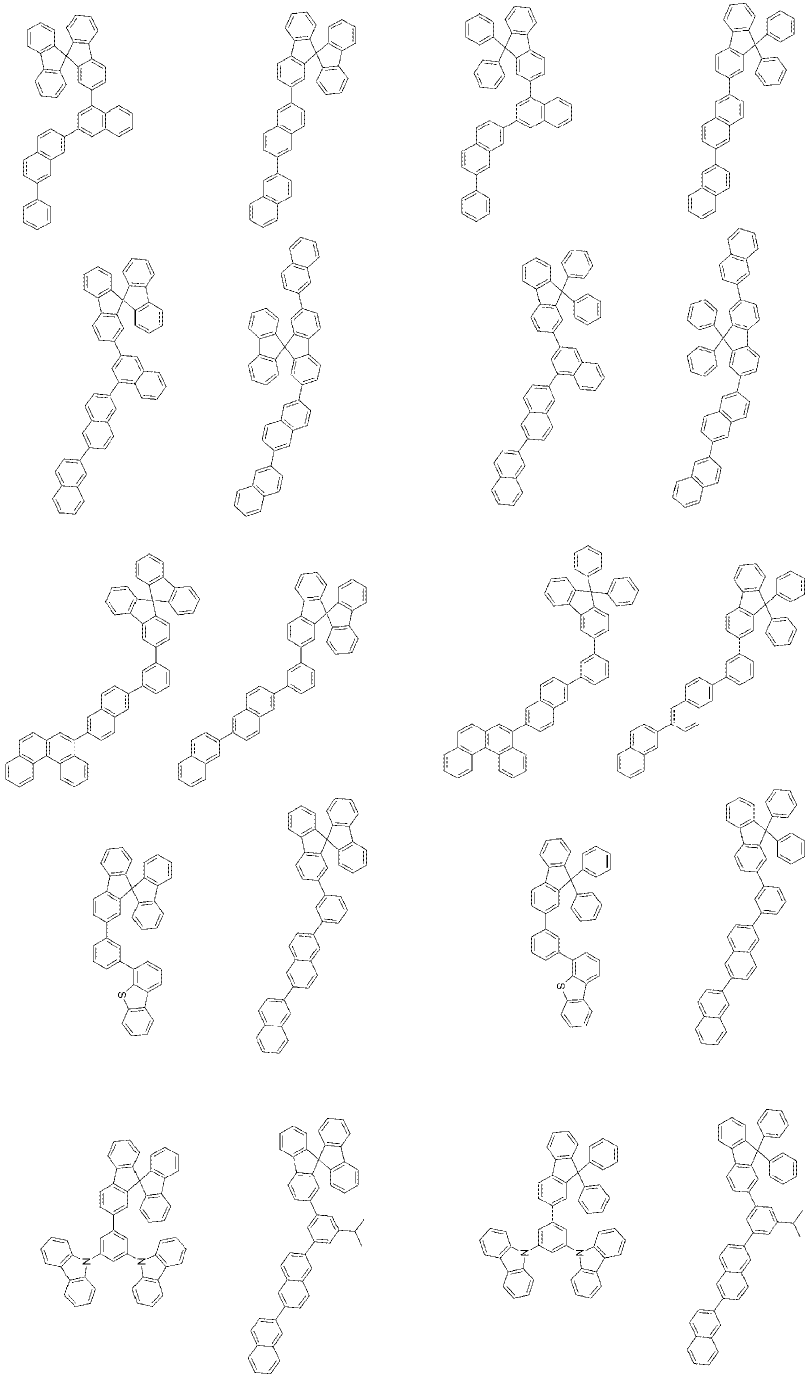

In another exemplary embodiment, the Host I may be selected from Compounds H-1a to H-12a below, but the Host I is not limited thereto:

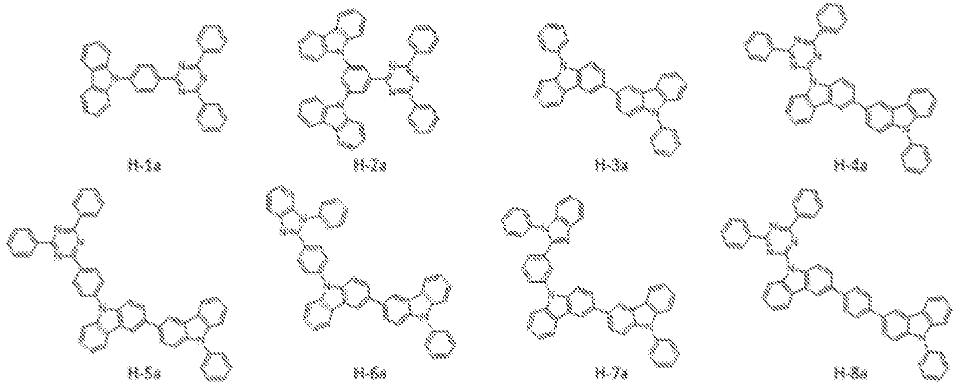

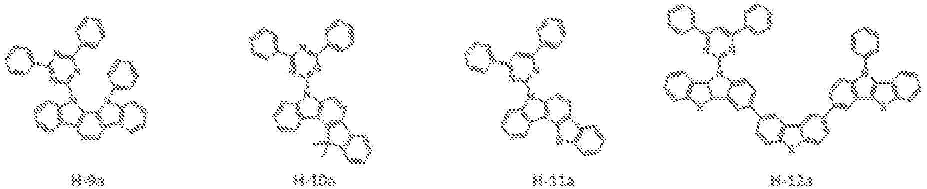

In another exemplary embodiment, the Host I may be selected from Compounds H-1 b to H-11 b below, but the Host I is not limited thereto:

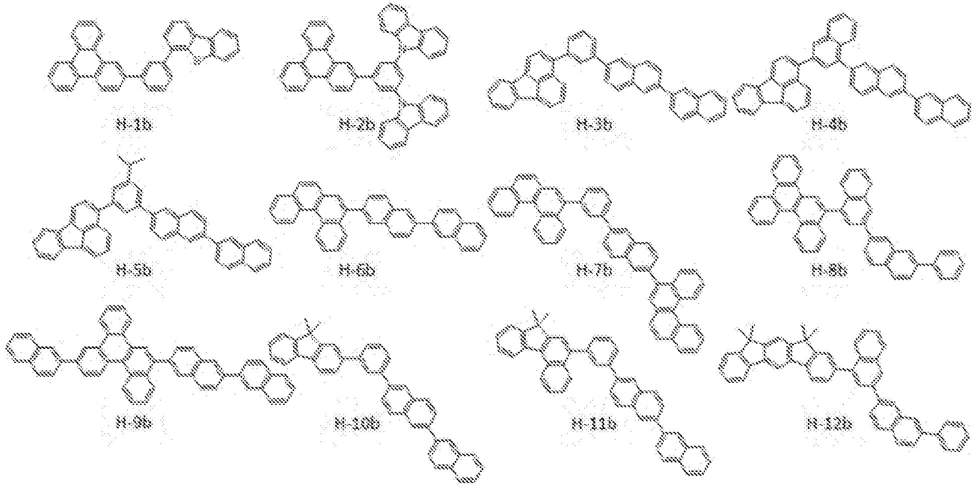

For example, the Host I may have a triplet energy gap of 2.1 eV or more, but the Host I is not limited thereto. When the Host I has a triplet energy gap of 2.1 eV or more, the Host I may have an excited state of triplet excitons of the EML in an efficient manner.

The EML may further include a Host II, and the Host II may be different from Host I, but the Host II is not limited thereto.

For example, the Host II may be selected from compounds below, but the Host II is not limited thereto:

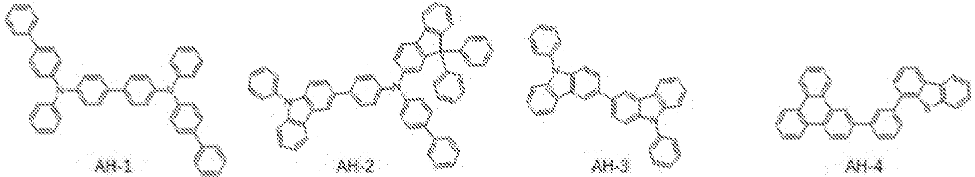

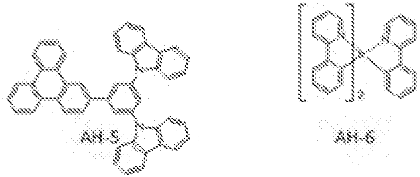

Hereinafter, an exemplary embodiment in which an EML of an organic light-emitting includes the Host I and the Host II will be described (Embodiment 4).

In an exemplary embodiment, the EML may include the Host I and the Host II, wherein the Host I and the Host II may each be independently selected from Formula 11 above.

For example, the Host I and the Host II may be different from each other, but the Host I and the Host II are not limited thereto:

For example, the Host I and the Host II may each be independently selected from Compounds H-1 a to H-12a below, but the Host I and the Host II are not limited thereto:

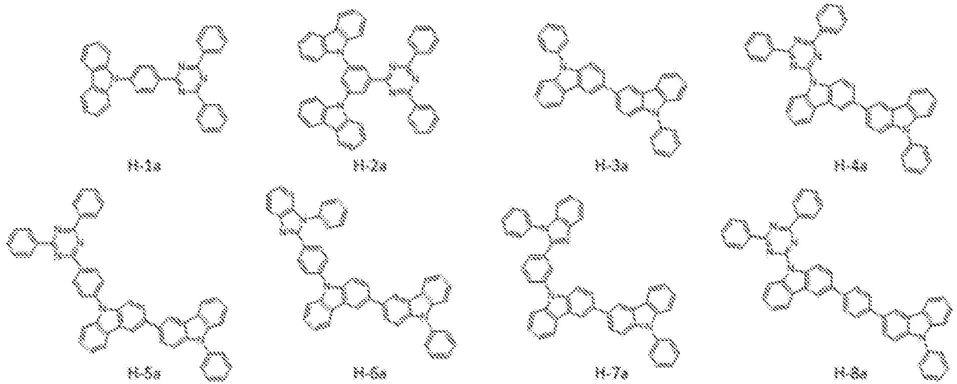

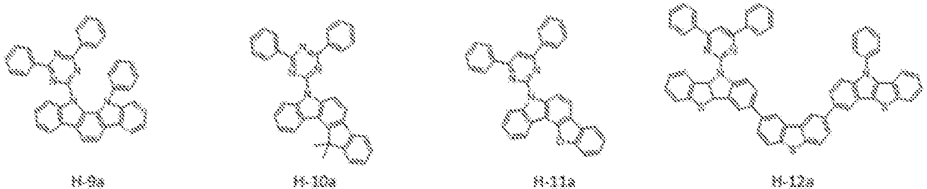

In another exemplary embodiment, the Host I may be selected from Compounds H-1a to H-12a, and the Host II may be selected from Compounds H-1b to H-11b, but the Host I and the Host II are not limited thereto:

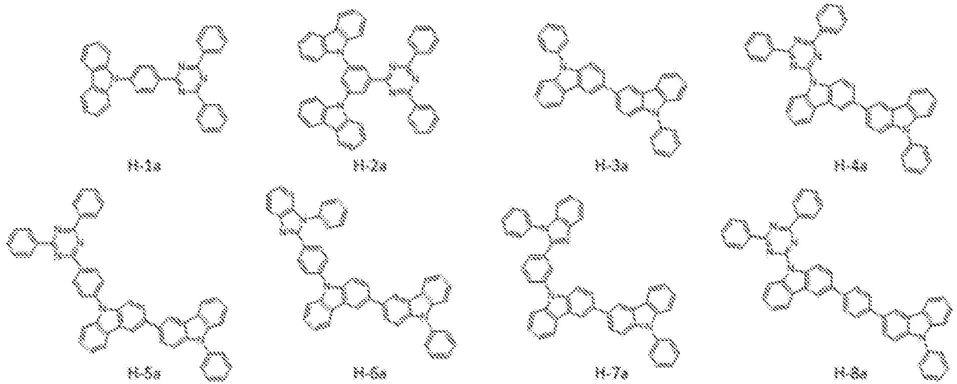

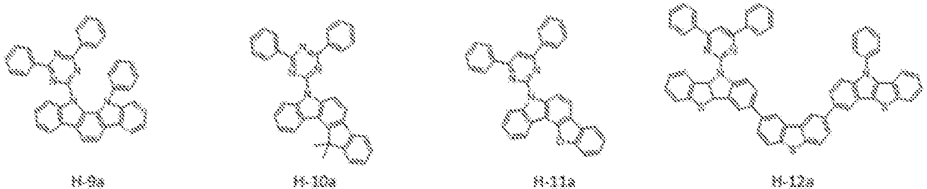

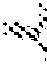

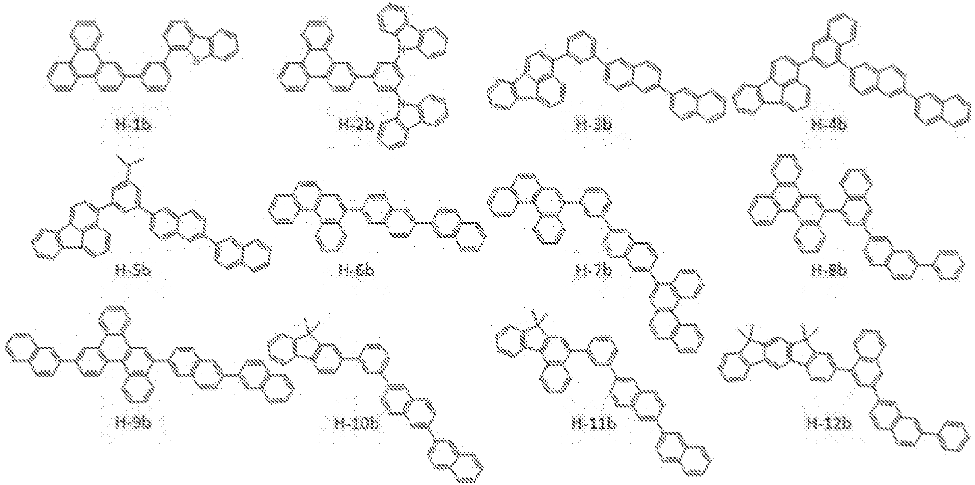

In another exemplary embodiment, the Host I and the Host II may each be independently selected from Compounds H-1b to H-11b, but the Host I and the Host II are not limited thereto:

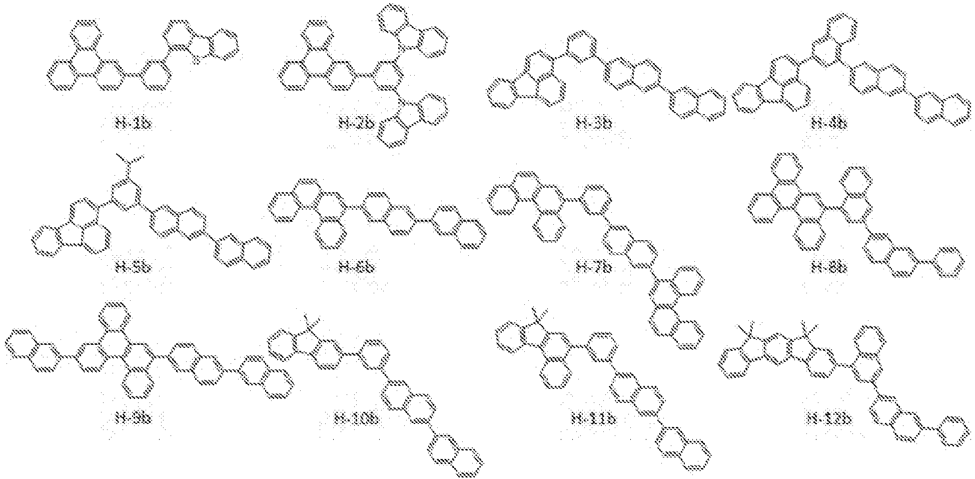

For example, one of the Host I and the Host II may have a triplet energy gap of 2.1 eV or more, but the Host I and the Host II are not limited thereto. When one of the Host I and the Host II has a triplet energy gap of 2.1 eV or more, one of the Host I and the Host II may have an excited state of triplet excitons of the EML in an efficient manner.

A weight ratio of the Host I and the Host II may be in a range of about 1:9 to about 9:1. For example, the weight ratio of the Host I and the Host II may be in a range of about 2:8 to about 8:2. For example, the weight ratio of the Host I and the Host II may be in a range of about 3:7 to about 7:3. For example, the weight ratio of the may be about 5:5.

Alternatively, a volume ratio of the Host I and the Host II may be in a range of about 1:9 to about 9:1. For example, the volume ratio of the Host I and the Host II may be in a range of about 2:8 to about 8:2. For example, the volume ratio of the Host I and the Host II may be in a range of about 3:7 to about 7:3. For example, the volume ratio of the the Host I and the Host II may be about 5:5.

As such, when the weight ratio or the volume ratio of the Host I and the Host II may vary depending upon the electric characteristics and the balance thereof in the organic light-emitting device.

The EML may further include an organometallic compound represented by Formula 7:

<Formula 7> M(L₁)ₙ₇₁(L₂)ₙ₇₂

In Formula 7, M may be selected from iridium (Ir), platinum (Pt), osmium (Os), titanium (Ti), zirconium (Zr), hafnium (Hf), europium (Eu), terbium (Tb), thulium (Tm), and rhodium (Rd).

In an exemplary embodiment, M in Formula 7 may be selected from Os, Ir, and Pt, but M is not limited thereto.

In another exemplary embodiment, M in Formula 7 may be Ir, but M is not limited thereto.

In Formula 7, L₁ may be a ligand represented by Formula 7A, and L₂ may be a ligand represented by Formula, wherein L₁ and L₂ may be different from each other:

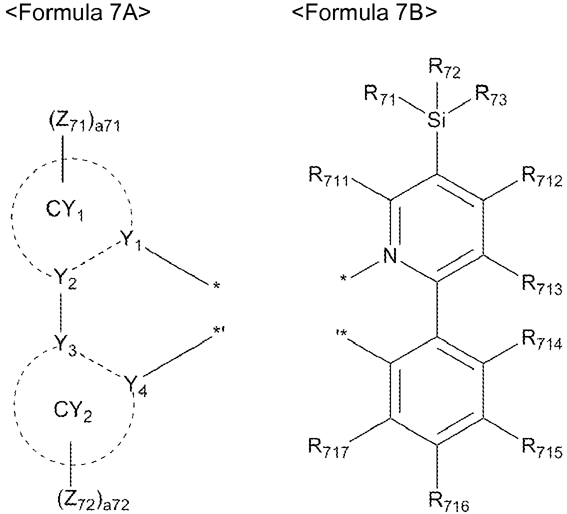

In Formulae 7A and 7B, * and *' may each independently indicate a binding site to M of Formula 1, and substituents may be as defined in the following description.

In Formula 7, n71 and n72 may each be independently 1 or 2, a sum of n71 and n72 (n71+n72) may be 2 or 3, and when n71 is 2 or more, a plurality of L₁s may be identical to or different from each other, and when n72 is 2, two L₂s may be identical to or different from each other.

For example, n71 and n72 may each be independently 1 or 2, and a sum of n71 and n72 (n71 +n72) may be 3, but n71 and n72 are not limited thereto.

In Formula 7A, Y₁ to Y₄ may each be independently C or N, wherein Y₁ and Y₂ may be linked to each other via a single bond or a double bond, and Y₃ and Y₄ may be linked to each other via a single bond or a double bond.

For example, in Formula 7A, Y₁ may be N, and Y₂ to Y₄ may be C, but Y₁ and Y₂ to Y₄ are not limited thereto.

In Formula 7A, CY₁ and CY₂ may each be independently selected from a C₅-C₆₀ cyclic group and a C₂-C₆₀ heterocyclic group, and CY₁ and CY₂ may be optionally linked to each other via a single bond or a first linking group.

In an exemplary embodiment, in Formula 7A, CY₁ and CY₂ may each be independently selected from a benzene, a naphthalene, a fluorene, a spiro-fluorene, an indene, a furan, a thiophene, a carbazole, a benzofuran, a benzothiophene, a dibenzofuran, a dibenzothiophene, a pyrrole, an imidazole, a pyrazole, a thiazole, an isothiazole, an oxazole, an isoxazole, a triazole, a pyridine, a pyrazine, a pyrimidine, a quinoline, an isoquinoline, a benzoquinoline, a quinoxaline, a quinazoline, naphthyridine, an indole, a benzimidazole, a benzoxazole, an isobenzoxazole, an oxadiazole, and a triazine, but CY₁ and CY₂ are not limited thereto.

In another exemplary embodiment, in Formula 7A, CY₁ may be selected from a pyrrole, an imidazole, a pyrazole, a thiazole, an isothiazole, an oxazole, an isoxazole, a triazole, a pyridine, a pyrazine, a pyrimidine, a quinoline, an isoquinoline, a benzoquinoline, a quinoxaline, a quinazoline, naphthyridine, a benzimidazole, a benzoxazole, an isobenzoxazole, an oxadiazole, and a triazine, but CY₁ is not limited thereto.

In another exemplary embodiment, in Formula 7A, CY₁ may be selected from a pyrrole, an imidazole, a pyrazole, a triazole, a pyridine, a pyrimidine, a pyrazine, a quinoline, an isoquinoline, an oxadiazole, and a triazine, but CY₁ is not limited thereto.

In another exemplary embodiment, in Formula 7A, CY₁ may be selected from a pyrrole, an imidazole, a pyrazole, a triazole, a pyridine, a pyrimidine, a pyrazine, and a triazine, but CY₁ is not limited thereto.

In another exemplary embodiment, in Formula 7A, CY₂ may be selected from a benzene, a naphthalene, a fluorene, a carbazole, a furan, a thiophene, a benzofuran, a benzothiophene, a dibenzofuran, a dibenzothiophene, an indole, a pyridine, a pyrazine, a pyrimidine, a quinoline, an isoquinoline, a benzoquinoline, a quinoxaline, a quinazoline, naphthyridine, an indole, an oxadiazole, and a triazine, but CY₂ is not limited thereto.

In another exemplary embodiment, in Formula 7A, CY₂ may be selected from a benzene, a naphthalene, a fluorene, a carbazole, a furan, a thiophene, a benzofuran, a benzothiophene, a dibenzofuran, a dibenzothiophene, an indole, a pyridine, a pyrimidine, a pyrazine, and a triazine, but CY₂ is not limited thereto.

In another exemplary embodiment, in Formula 7A, CY₂ may be selected from a benzene, a pyridine, a pyrimidine, a pyrazine, a triazine, a carbazole, a dibenzofuran, and a dibenzothiophene, but CY₂ is not limited thereto.

In another exemplary embodiment, in Formula 7A,
CY₁ may be selected from a pyrrole, an imidazole, a pyrazole, a triazole, a pyridine, a pyrimidine, a pyrazine, a triazine, a quinoline, an isoquinoline, and an oxadiazole, and
CY₂ may be selected from a benzene, a naphthalene, a fluorene, a carbazole, a furan, a thiophene, a benzofuran, a benzothiophene, a dibenzofuran, a dibenzothiophene, an indole, a pyridine, a pyrimidine, a pyrazine, an oxadiazole, and a triazine, but CY₁ and CY₂ are not limited thereto.

In Formula 7B, R₇₁ to R₇₃ may each be independently selected from:
a C₁-C₁₀ alkyl group; and
a C₁-C₁₀ alkyl group substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, and a phosphoric acid group or a salt thereof, but R₇₁ to R₇₃ are not limited thereto.

In an exemplary embodiment, in Formula 7B, R₇₁ to R₇₃ may each be independently selected from:
a methyl group, an ethyl group, an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, and a tert-decyl group; and
a methyl group, an ethyl group, an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, and a tert-decyl group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, and a phosphoric acid group or a salt thereof, but R₇₁ to R₇₃ are not limited thereto.

In another exemplary embodiment, in Formula 7B, R₇₁ to R₇₃ may each be independently selected from:
a methyl group, an ethyl group, an n-propyl group, an iso-propyl group, an n-butyl group, an iso-butyl group, a sec-butyl group, a tert-butyl group, an n-pentyl group, an isopentyl group, a sec-pentyl group, and a tert-pentyl group; and
a methyl group, an ethyl group, an n-propyl group, an iso-propyl group, an n-butyl group, an iso-butyl group, a sec-butyl group, a tert-butyl group, an n-pentyl group, an isopentyl group, a sec-pentyl group, and a tert-pentyl group, each substituted with at least one of a deuterium, - F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, and a phosphoric acid group or a salt thereof, but R₇₁ to R₇₃ are not limited thereto.

In another exemplary embodiment, in Formula 7B, R₇₁ to R₇₃ may each be independently selected from a methyl group, an ethyl group, an n-propyl group, an iso-propyl group, an n-butyl group, an iso-butyl group, a sec-butyl group, and a tert-butyl group, but R₇₁ to R₇₃ are not limited thereto.

In another exemplary embodiment, in Formula 7B, R₇₁ to R₇₃ may be identical to each other, but R₇₁ to R₇₃ are not limited thereto.

In Formulae 7A and 7B, Z₇₁, Z₇₂ and R₇₁₁ to R₇₁₇ may each be independently selected from:
a hydrogen, a deuterium, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), and -B(Q₆)(Q₇), wherein R₇₁₂ is not a hydrogen, and two adjacent substituents selected from R₇₁₄ to R₇₁₇ are optionally linked to each other to form a condensed ring, and
at least one substituent of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted or unsubstituted monovalent non-aromatic condensed heteropolycyclic group may be selected from:
   a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
   a C₁-C₆₀ alkyl group, a C₂-C₆₀ alkenyl group, a C₂-C₆₀ alkynyl group, and a C₁-C₆₀ alkoxy group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), and -B(Q₁₆)(Q₁₇);
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), and -B(Q₂₆)(Q₂₇); and
   -N(Q₃₁)(Q₃₂), -Si(Q₃₃)(Q₃₄)(Q₃₅), and -B(Q₃₆)(Q₃₇),
   wherein Q₁ to Q₇, Q₁₁ to Q₁₇, Q₂₁ to Q₂₇, and Q₃₁ to Q₃₇ may each be independently selected from a hydrogen, a C₁-C₆₀ alkyl group, a C₁-C₆₀ alkoxy group, a C₆-C₆₀ aryl group, a C₁-C₆₀ heteroaryl group, a monovalent non-aromatic condensed polycyclic group, and a monovalent non-aromatic condensed heteropolycyclic group.

In an exemplary embodiment, in Formulae 7A and 7B, Z₇₁, Z₇₂, and R₇₁₁ to R₇₁₇ may each be independently selected from:
a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a C₁-C₂₀ alkyl group, and a C₁-C₂₀ alkoxy group;
a C₁-C₂₀ alkyl group and a C₁-C₂₀ alkoxy group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a phenyl group, a naphthyl group, a pyridinyl group, and a pyrimidinyl group;
a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group; and
a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group, each substituted with at least one of a deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or or a salt thereof, a phosphoric acid or a salt thereof, a C₁-C₂₀ alkyl group, a C₁-C₂₀ alkoxy group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group, wherein R₇₁₂ is not a hydrogen, but embodiments are not limited thereto.

In another exemplary embodiment, in Formulae 7A and 7B, Z₇₁, Z₇₂, and R₇₁₁ to R₇₁₇ may each be independently selected from:
a hydrogen, -F, a cyano group, a nitro group, -SF₅, a methyl group, an ethyl group, an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, butoxy group, a pentoxy group, a phenyl group, a naphthyl group, a pyridinyl group, and a pyrimidinyl group; and
a methyl group, an ethyl group, an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, butoxy group, a pentoxy group, a phenyl group, a naphthyl group, a pyridinyl group, and a pyrimidinyl group, each substituted with at least one of-F, a cyano group, and a nitro group, wherein R₇₁₂ is not a hydrogen, but embodiments are not limited thereto.

In Formula 7A, a71 indicates the number of Z₇₁, and may be selected from integers of 1 to 5. When a71 is 2 or more, a plurality of Z₇₁s may be identical to or different from each other.

In Formula 1A, a7 indicates the number of Z₇₂, and may be selected from integers of 1 to 5. When a72 is 2 or more, a plurality of Z₇₂s may be identical to or different from each other.

For example, the organometallic compound of Formula 7 may be selected from Compounds PD-1 to PD-192, but the organometallic compound is not limited thereto:

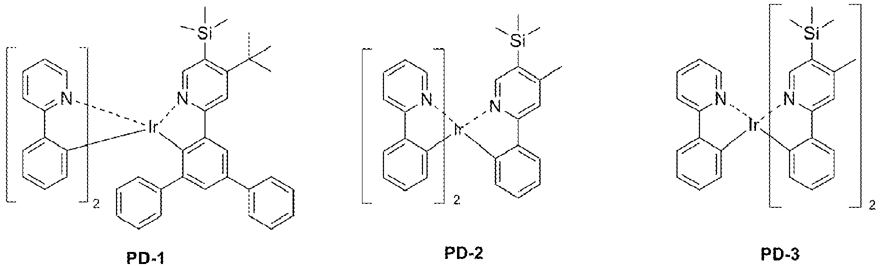

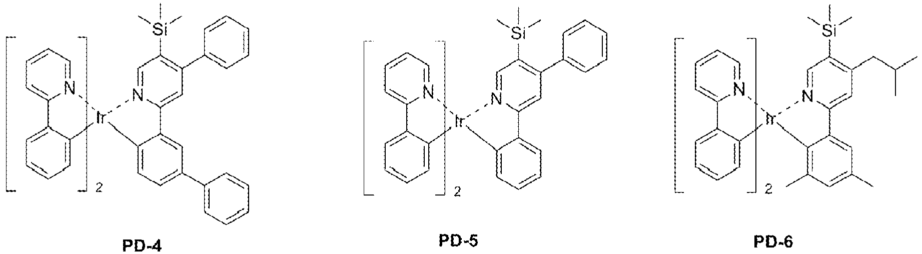

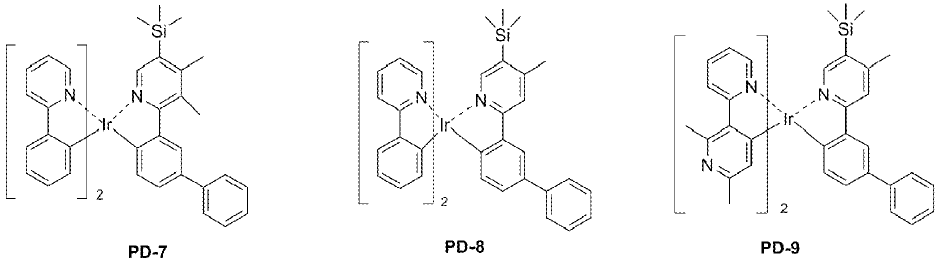

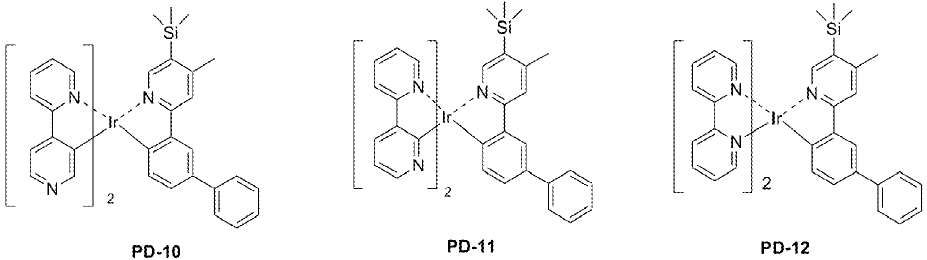

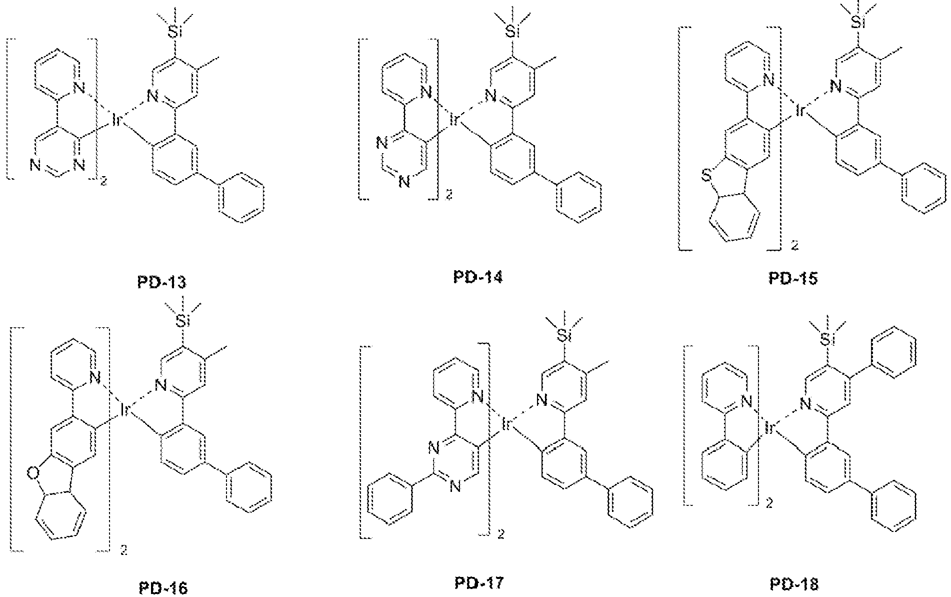

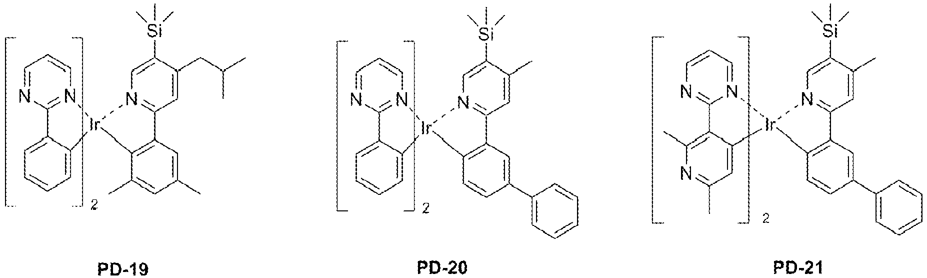

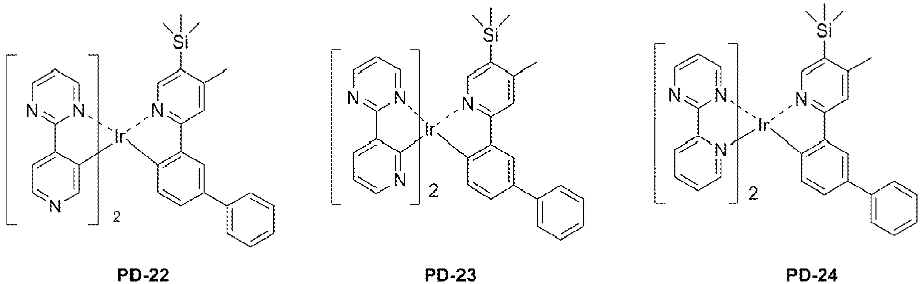

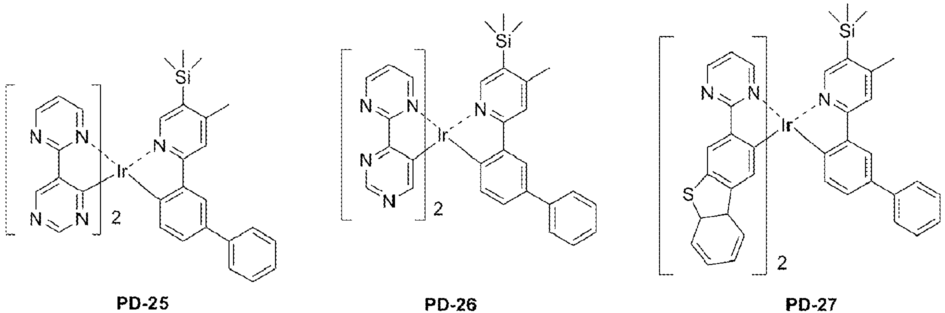

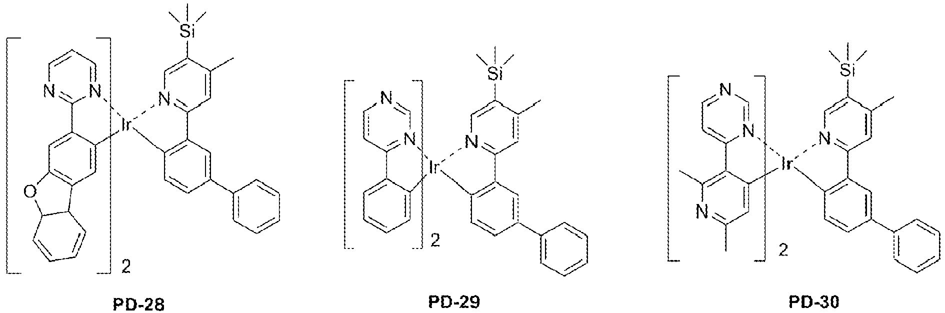

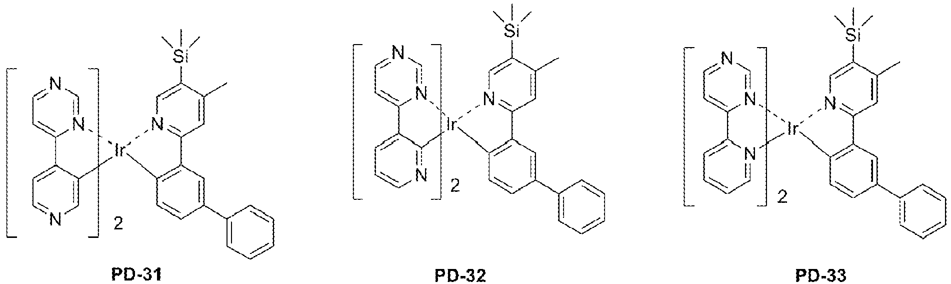

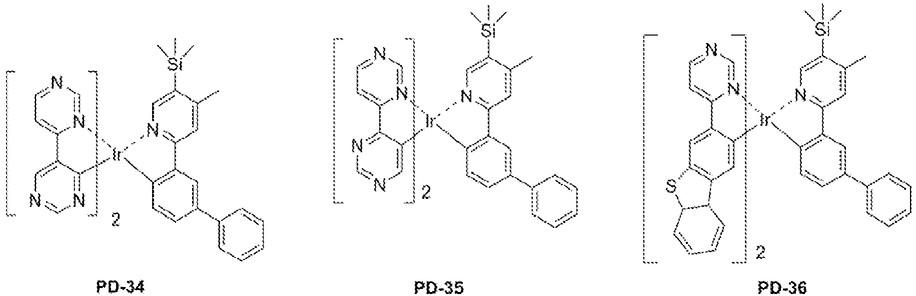

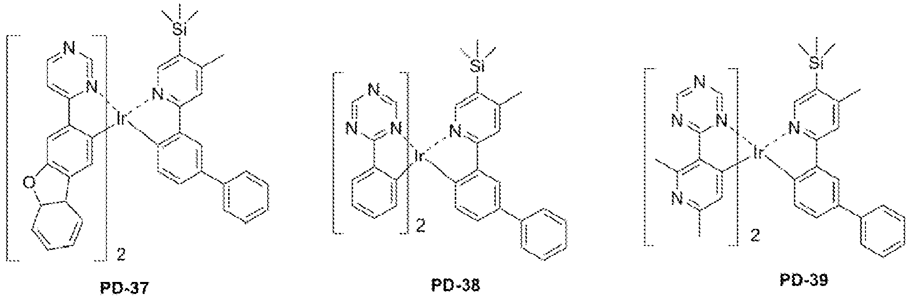

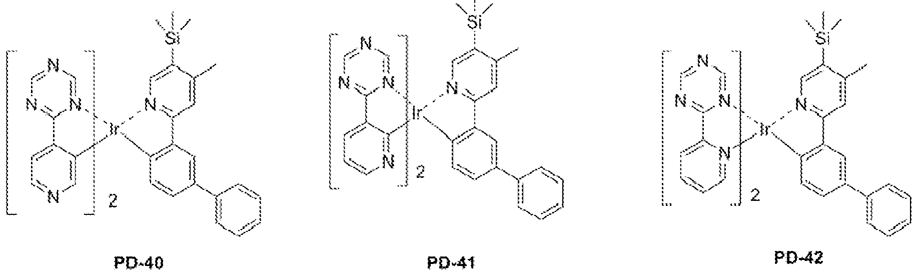

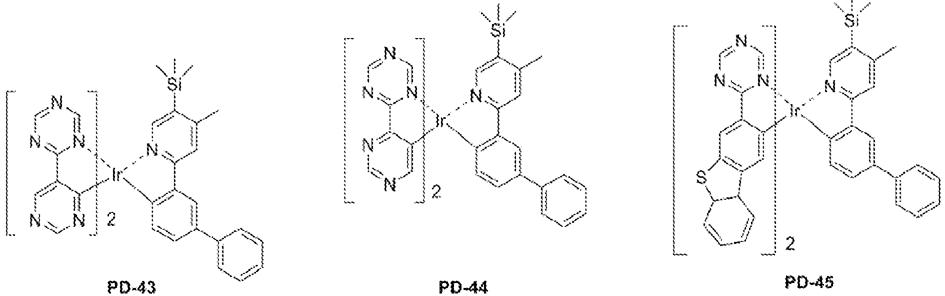

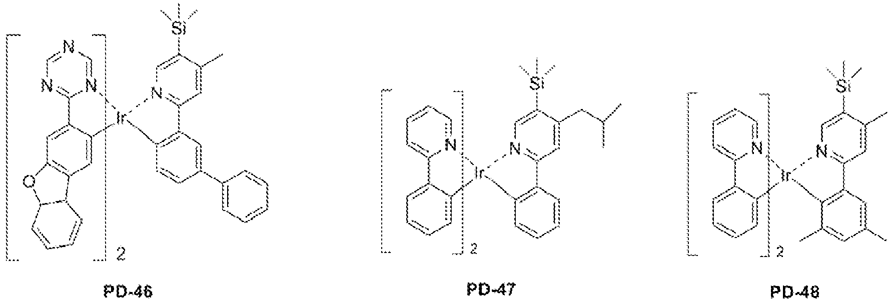

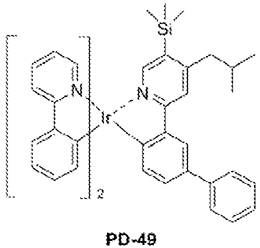

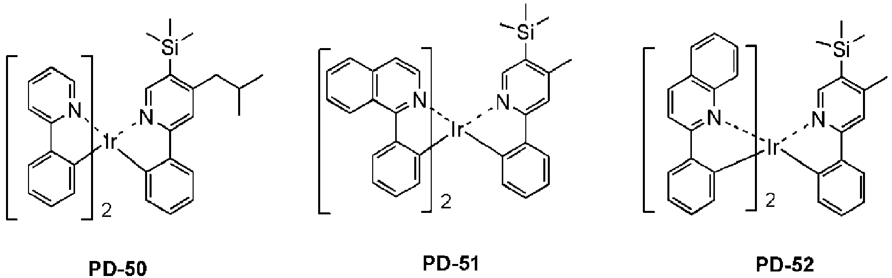

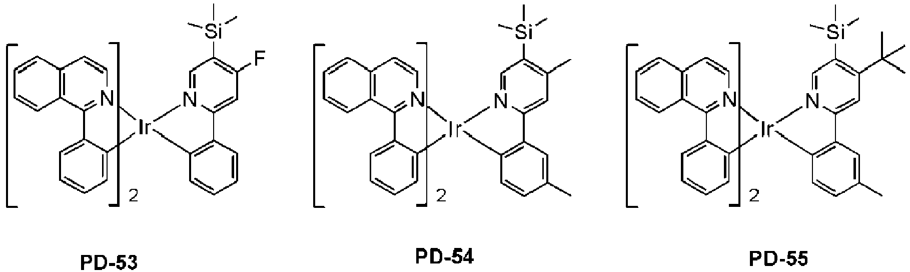

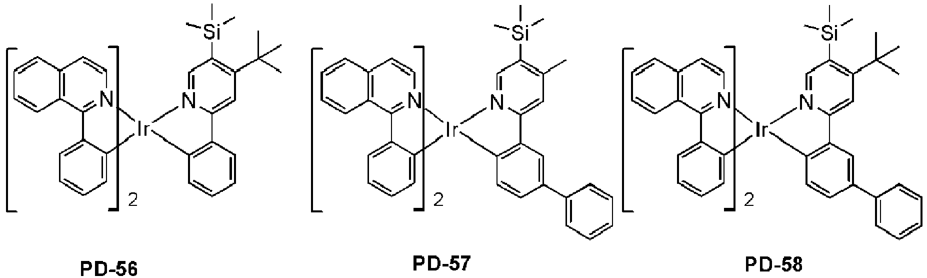

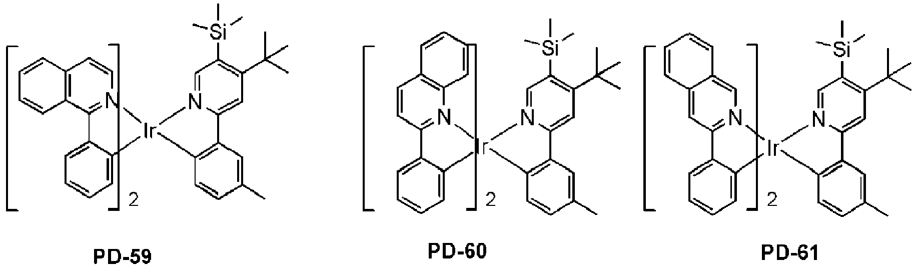

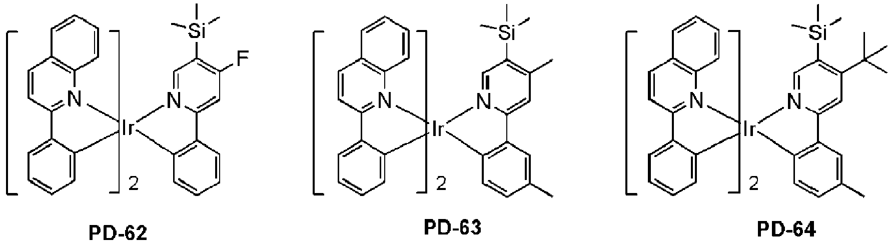

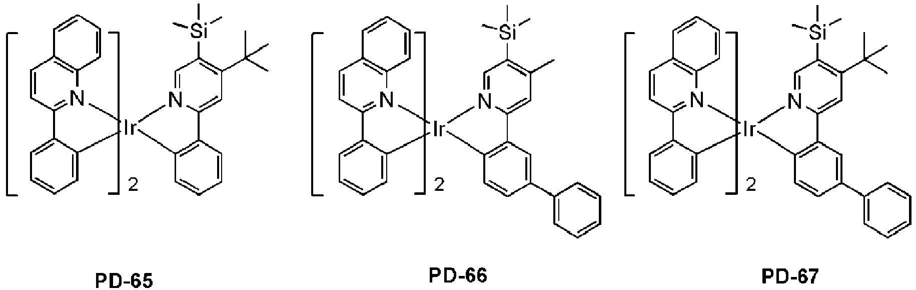

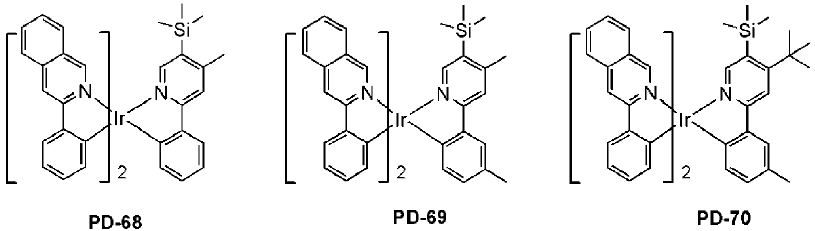

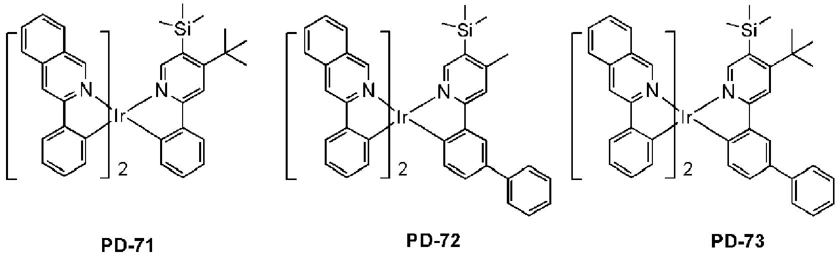

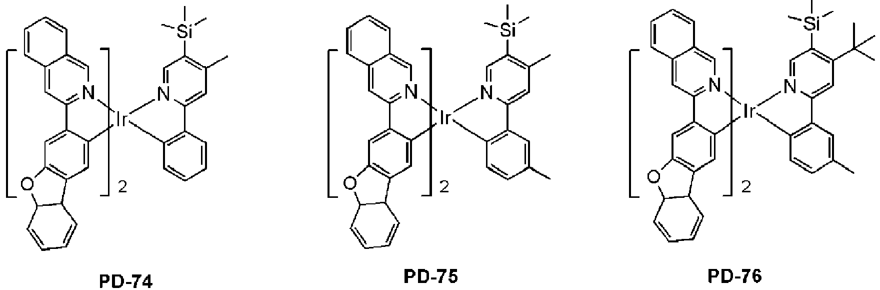

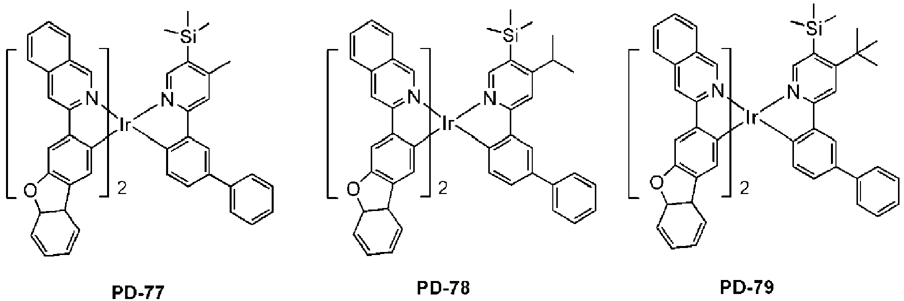

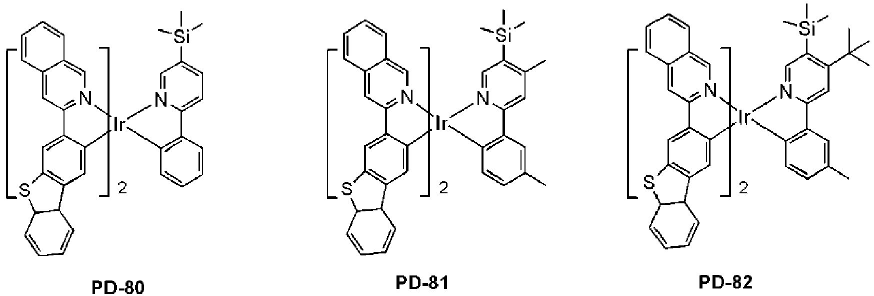

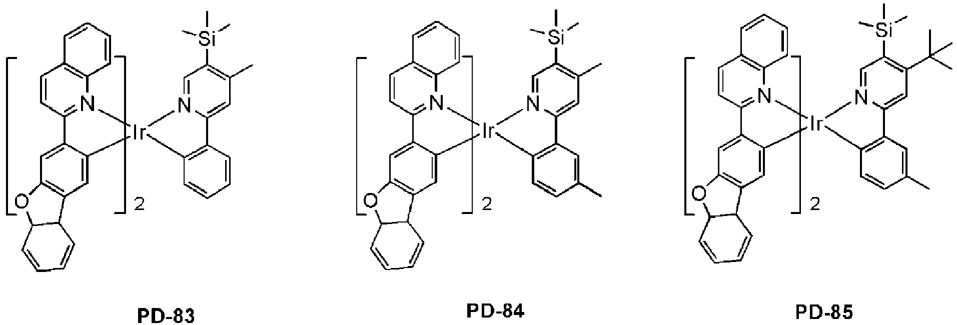

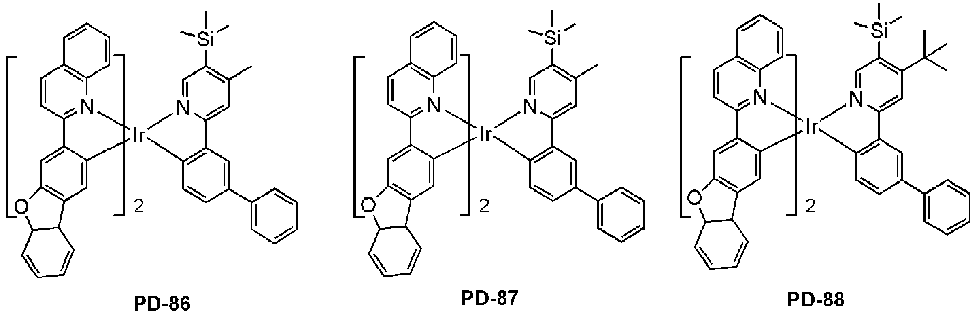

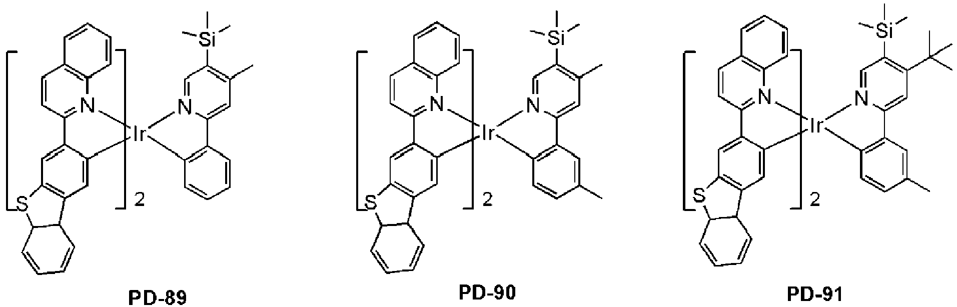

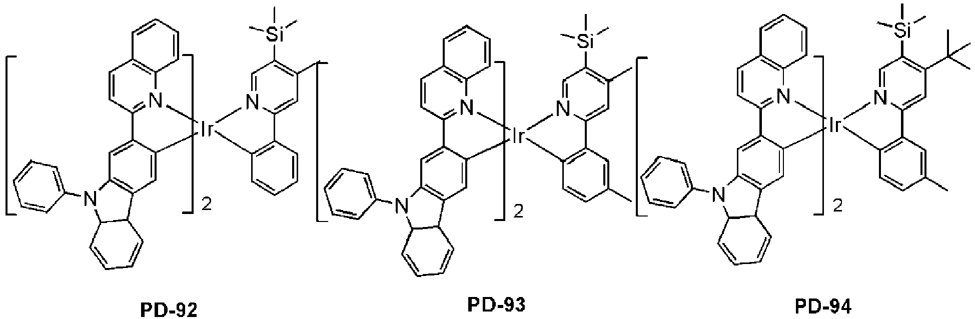

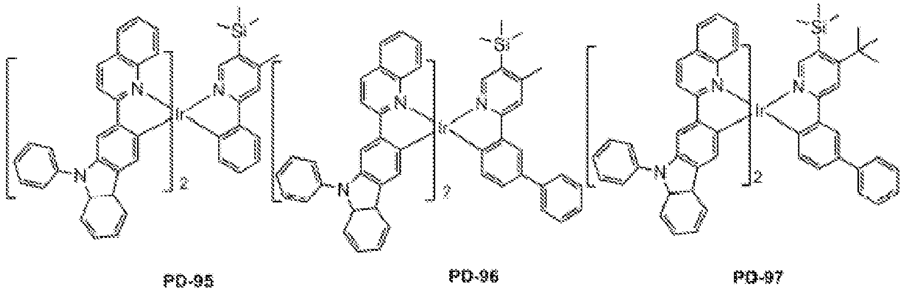

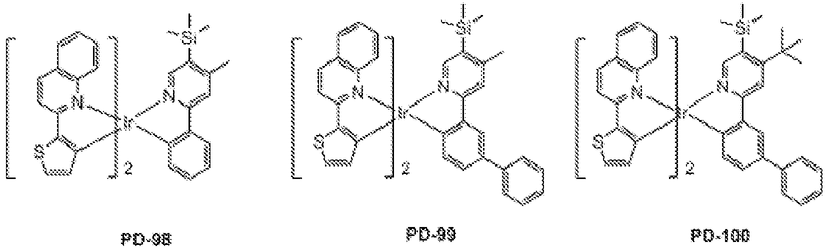

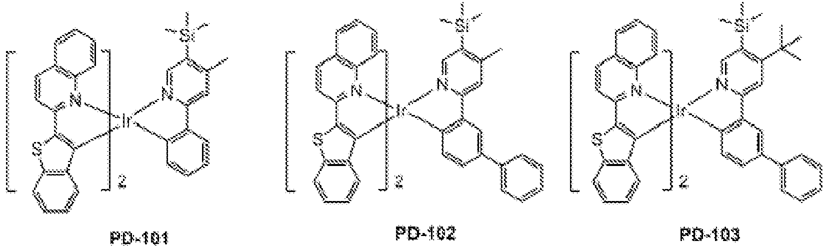

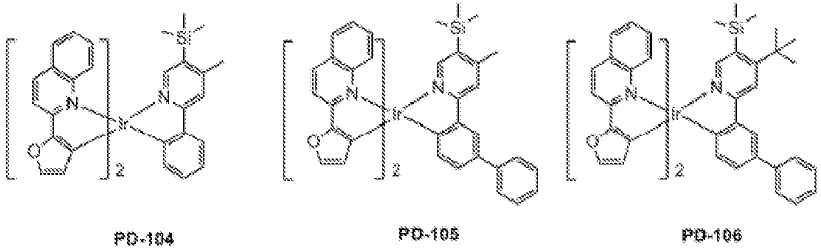

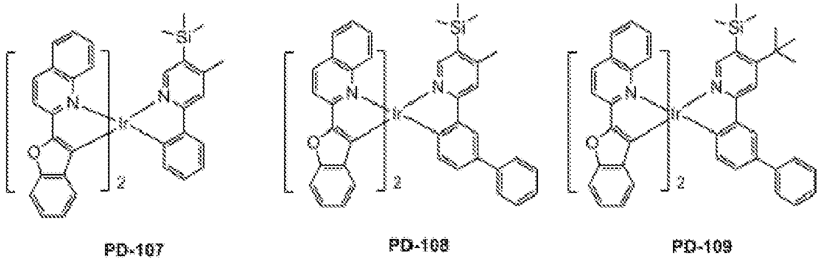

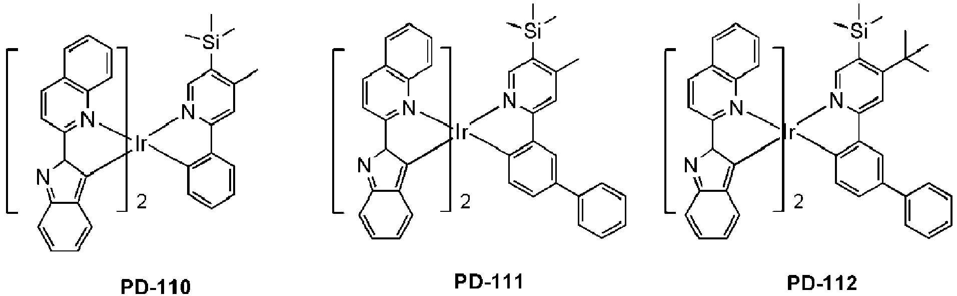

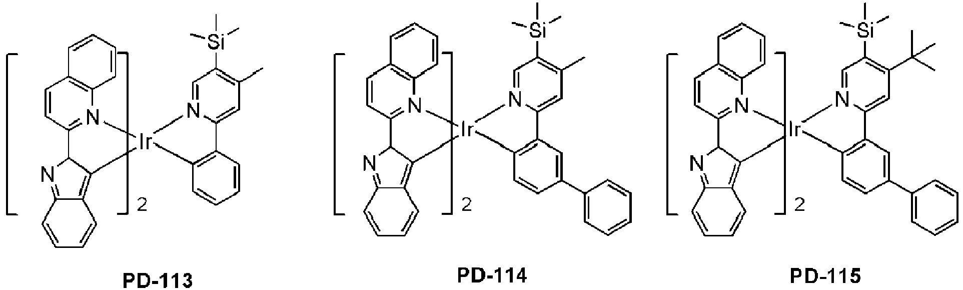

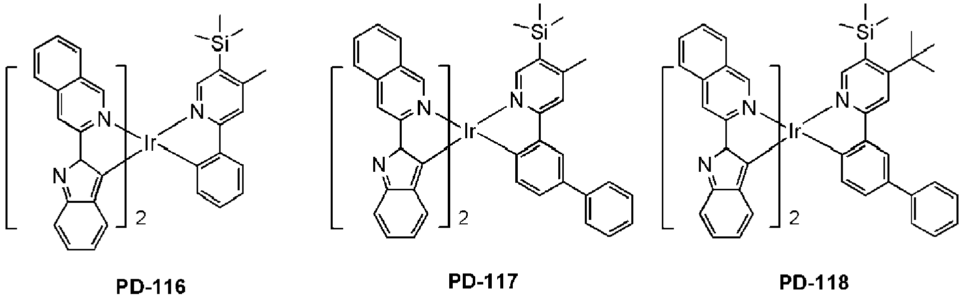

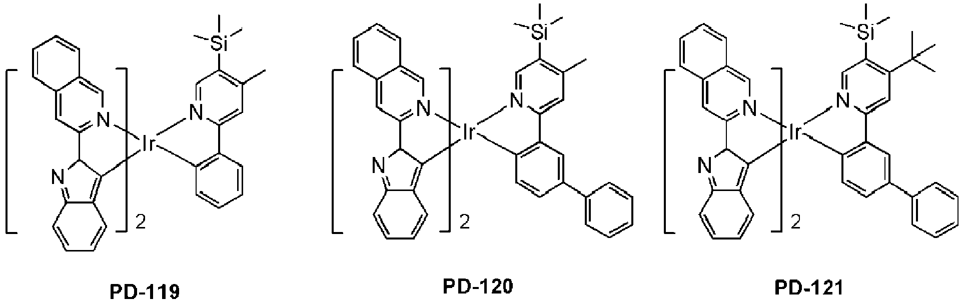

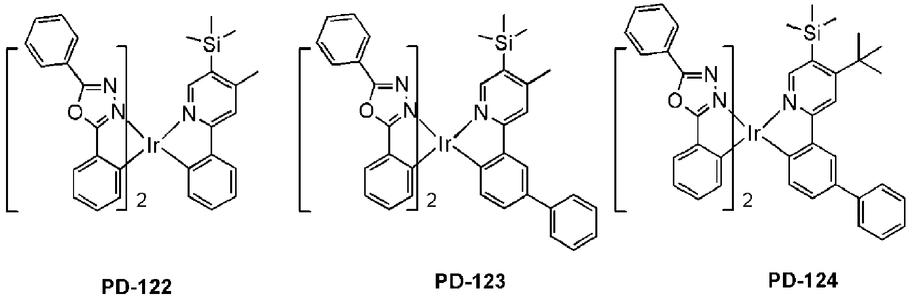

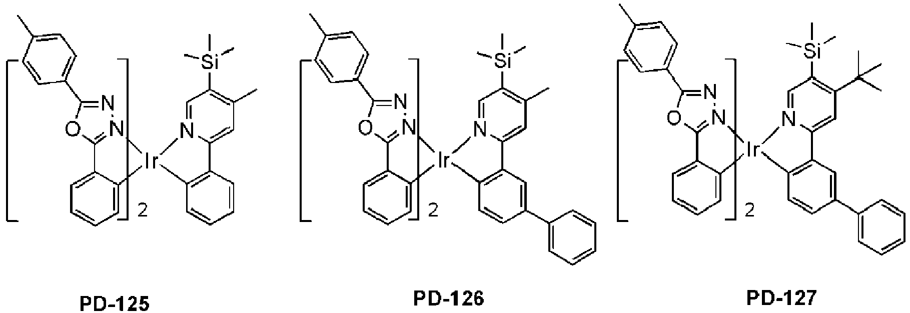

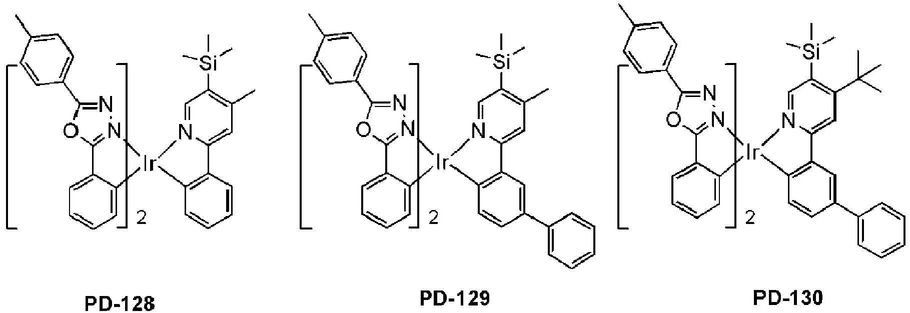

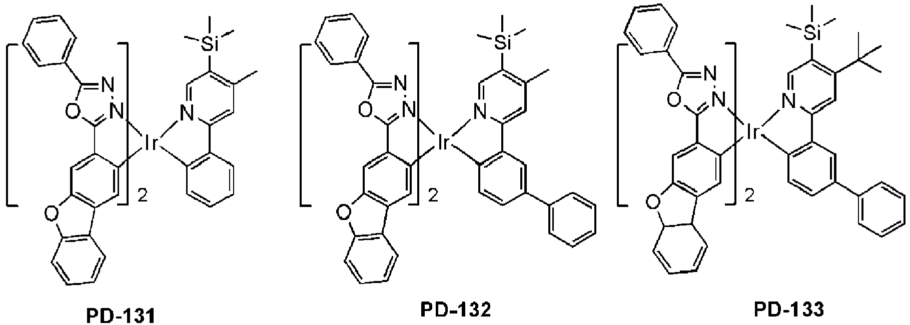

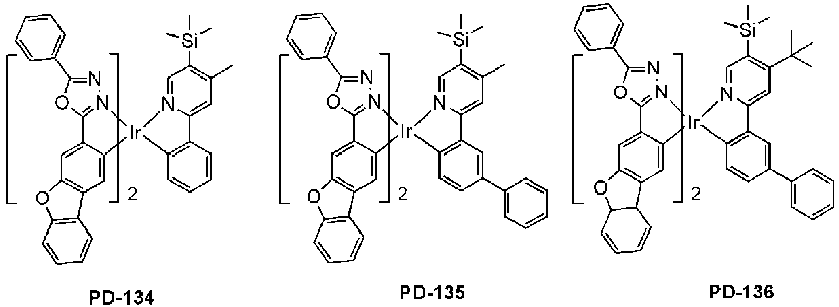

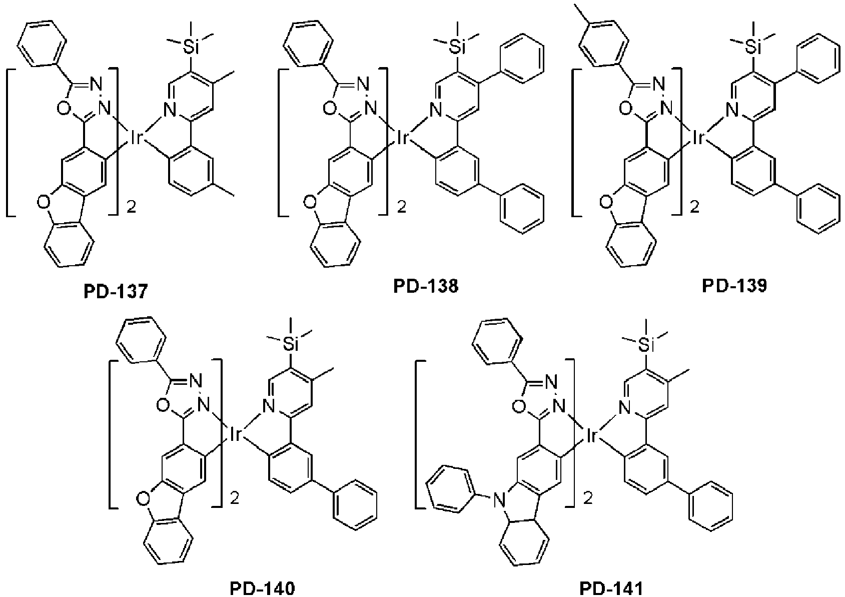

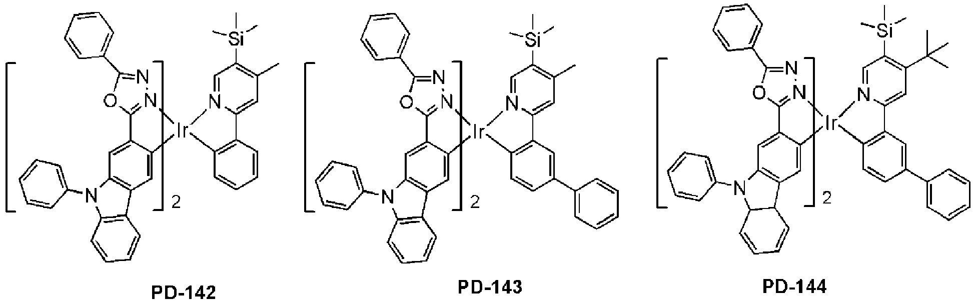

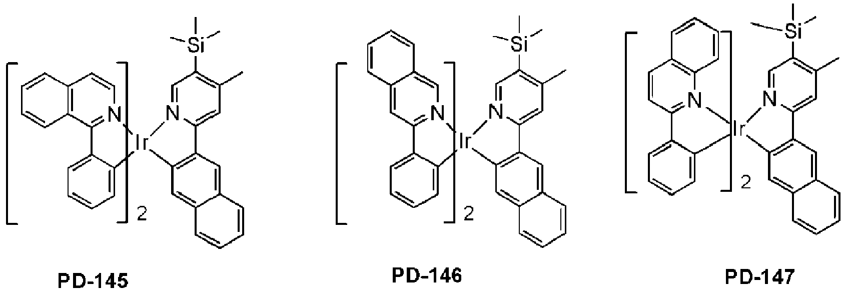

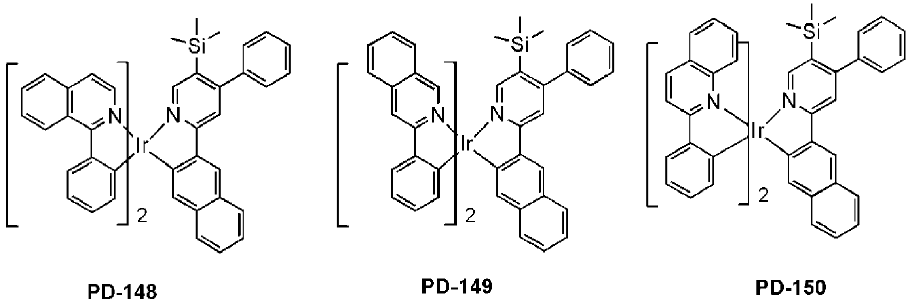

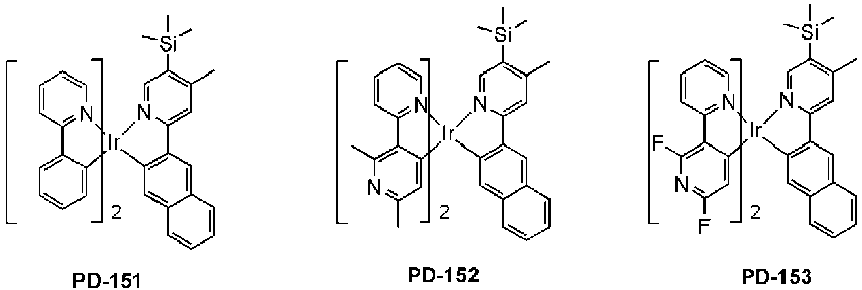

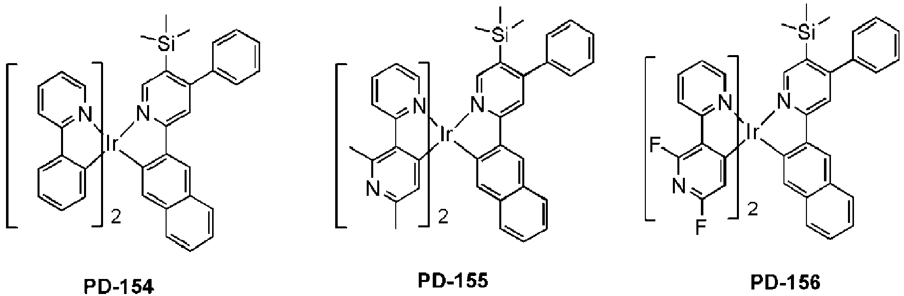

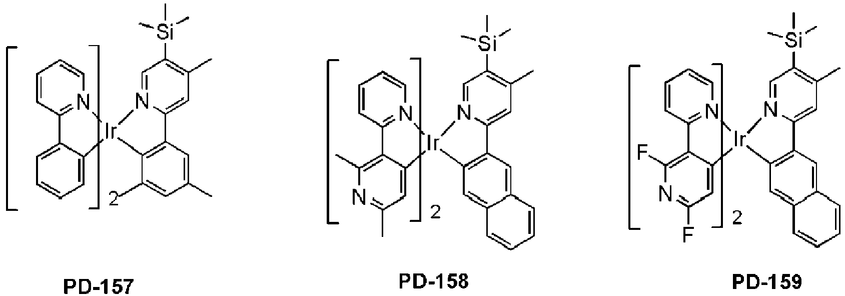

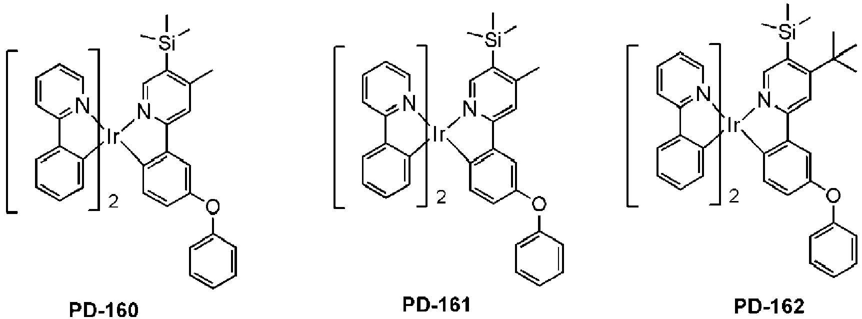

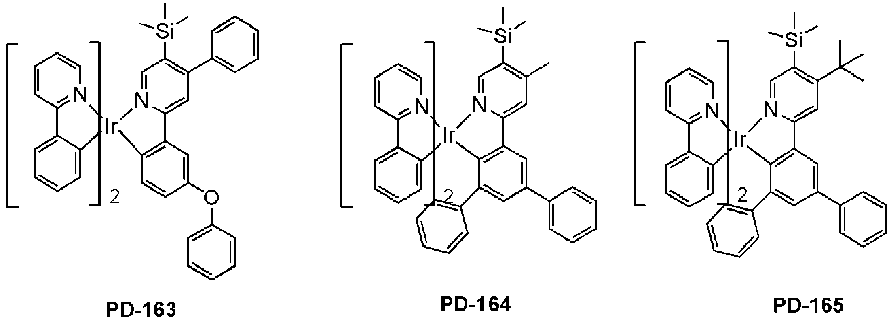

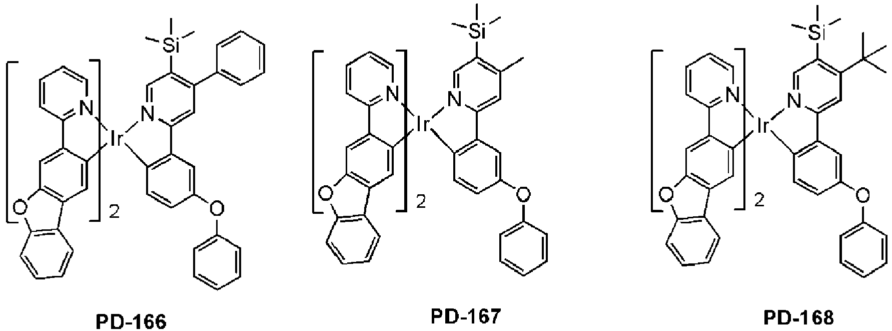

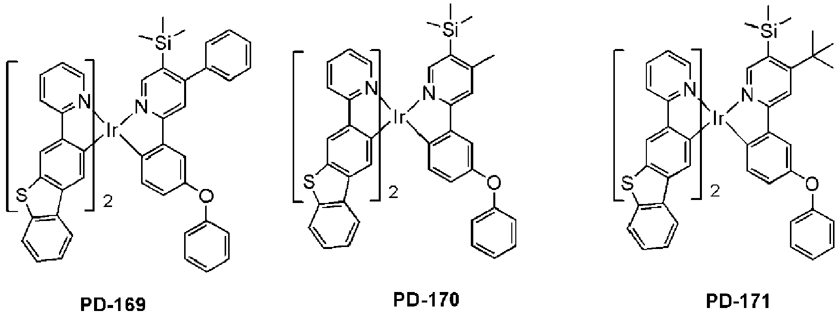

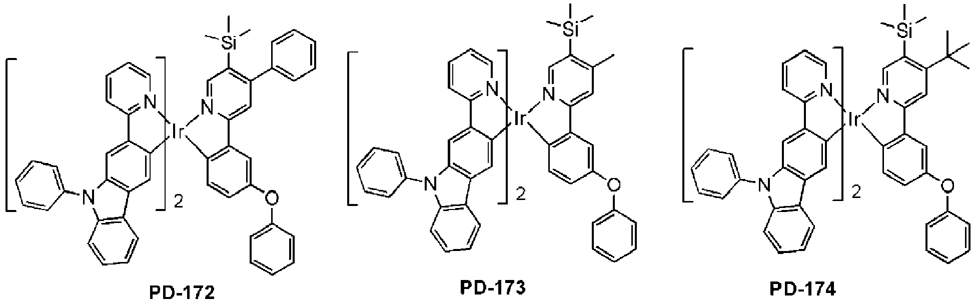

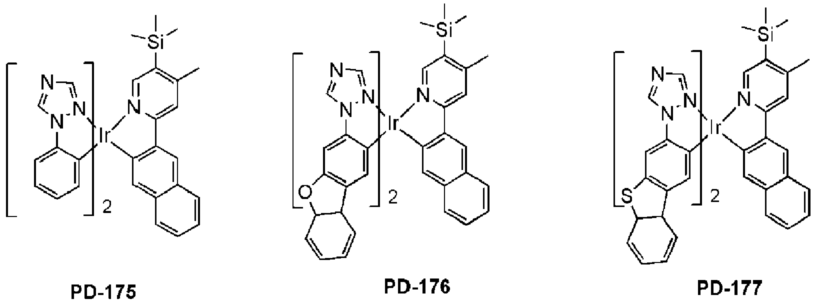

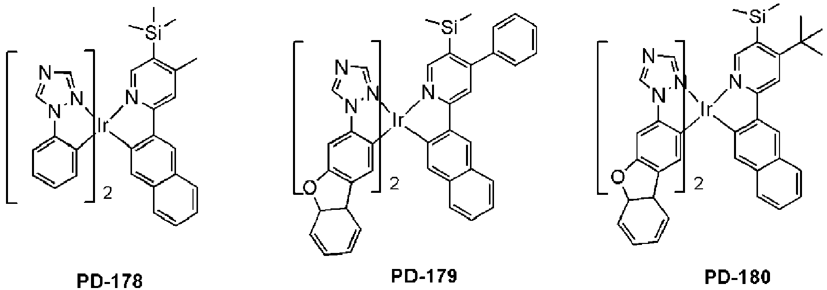

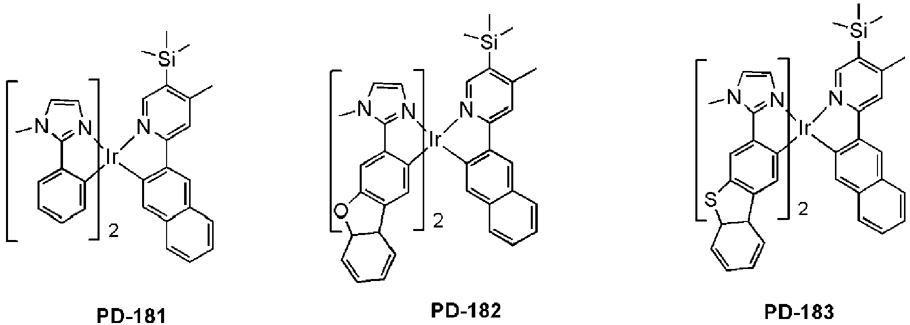

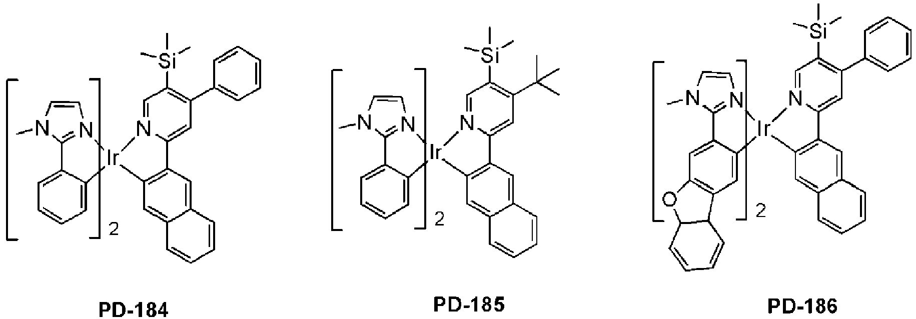

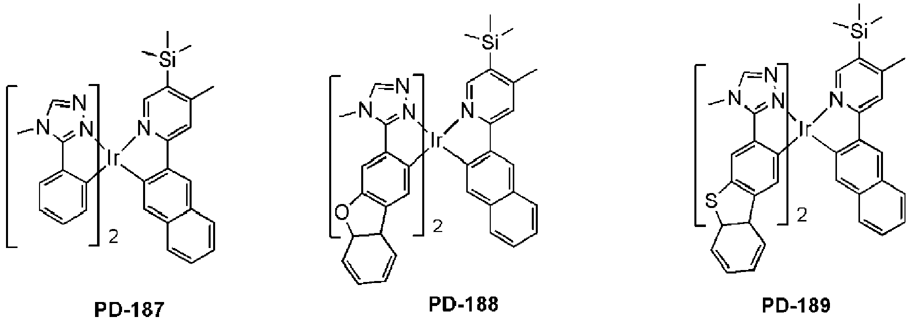

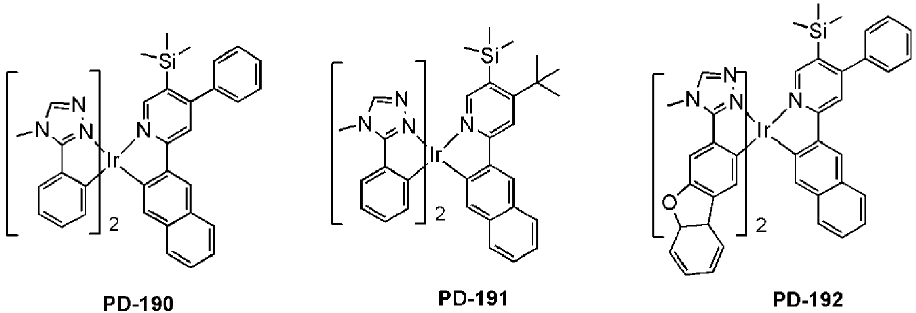

An amount of the organometallic compound included in the EML may be smaller than that of the host (i.e., the amount of the first host or the total amounts of the first host and the second host). For example, the amount of the organometallic compound may be in a range of about 0.01 parts by weight to about 15 parts by weight based on 100 parts by weight of the host, but the amount is not limited thereto. Alternatively, a volume percentage of the organometallic compound included in the EML may be in a range of about 0.01 v% to about 15v%, but the volume percentage is not limited thereto.

The organometallic compound may be a dopant, and may emit green light or red light from the EML.

A thickness of the EML may be in a range of about 100 Å to about 1,000 A , e.g., about 200 Å to about 600 Å. When the thickness of the EML is within these ranges, excellent emission characteristics may be obtained without a substantial increase in driving voltage.

Next, the electron transport region may be disposed on the EML.

The electron transport region may include at least one of an HBL, an ETL, and an EIL, but the electron transport region is not limited thereto.

For example, the electron transport region may have a structure of ETL/EIL or a structure of HBL/ETL/EIL, each of which layers are sequentially stacked in the stated order from the EML, but the structure is not limited thereto.

The electron transport region may include an HBL. When the EML includes a phosphorescent dopant, the HBL may serve as a layer that reduces or prevents triplet excitons or holes from being diffused into the ETL.

When the electron transport region the HBL may be formed on the EML by using various suitable methods, such as vacuum deposition, spin coating, casting, a LB method, ink-jet printing, laser-printing, or LITI. When the HBL is formed by vacuum deposition or by spin coating, the deposition and coating conditions for the HBL may be determined by referring to the deposition and coating conditions for the HIL.

The HBL may include, for example, at least one of BCP and Bphen, but embodiments are not limited thereto.

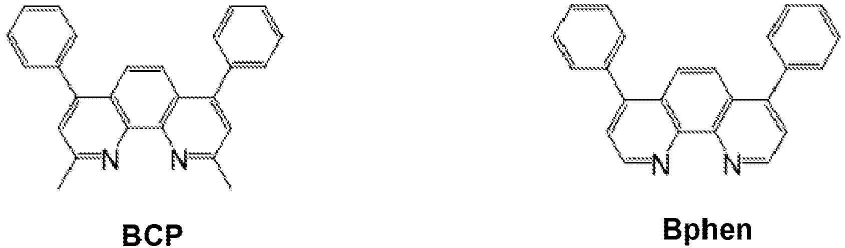

A thickness of the HBL may be in a range of about 20 Å to about 1,000 Å, e.g., about 30 Å to about 300 Å. When the thickness of the HBL is within these ranges, excellent hole blocking characteristics may be obtained without a substantial increase in driving voltage.

The electron transport region may include an ETL, and the ETL may be formed on the EML or on the HBL by using various suitable methods, such as vacuum deposition, spin coating, casting, a LB method, ink-jet printing, laser-printing, or LITI. When the ETL is formed by vacuum deposition or by spin coating, the deoposition and coating conditions for the ETL may be determined by referring to the deposition and coating conditions for the HIL.

The ETL may furthat include at least one of BCP and Bphen above and Alq₃, Balq, TAZ, and NTAZ below:

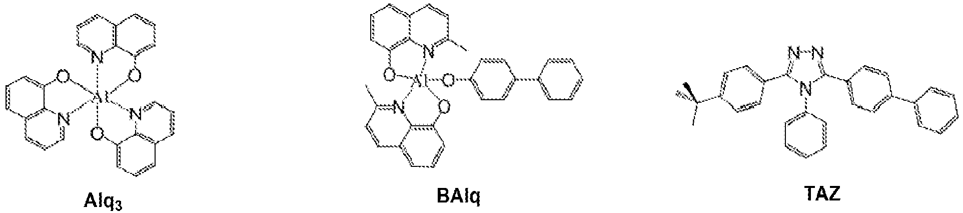

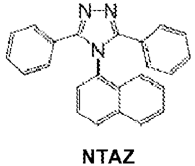

Alternatively, the ETL may include at least one of compounds represented by Formula 601:

<Formula 601 > Ar₆₀₁-[(L₆₀₁)ₓₑ₁-E₆₀₁]ₓₑ₂

In Formula 601, Ar₆₀₁ may be selected fro:
a naphthalene, a heptalene, a fluorenene, a spiro-fluorene, a benzofluorene, a dibenzofluorene, a phenalene, a phenanthrene, an anthracene, a fluoranthene, a triphenylene, a pyrene, a chrysene, a naphthacene, a picene, a perylene, a pentaphene, and an indenoanthracene;
a naphthalene, a heptalene, a fluorene, a spiro-fluorene, a benzofluorene, a dibenzofluorene, a phenalene, a phenanthrene, an anthracene, a fluoranthene, a triphenylene, a pyrene, a chrysene, naphthacene, a picene, a perylene, a pentaphene, and an indenoanthracene, each substituted with at least one of a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, and -Si(Q₃₀₁)(Q₃₀₂)(Q₃₀₃) (wherein Q₃₀₁ to Q₃₀₃ may each be independently selected from a hydrogen, a C₁-C₆₀ alkyl group, a C₂-C₆₀ alkenyl group, a C₆-C₆₀ aryl group, and a C₁-C₆₀ heteroaryl group),
a description of L₆₀₁ may be as referred to in the description provided in connection with L₂₀₁,
E₆₀₁ may be selected from a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, and a dibenzocarbazolyl group; and
a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, and a dibenzocarbazolyl group, each substituted with at least one of a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₁-C₂₀ alkyl group, a C₁-C₂₀ alkoxy group, a phenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, and a dibenzocarbazolyl group,
xe1 may be selected from 0, 1, 2, and 3, and
xe2 may be selected from 1, 2, 3, and 4.

Alternatively, the ETL may include, at least one of compouns represented by Formula 602:

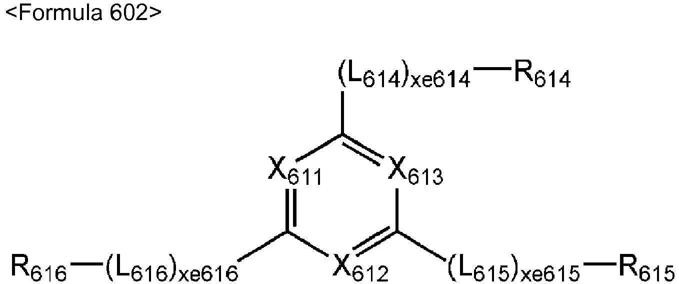

In Formula 602,
X₆₁₁ may be N or C-(L₆₁₁)ₓₑ₆₁₁-R₆₁₁, X₆₁₂ may be N or C-(L₆₁₅)ₓₑ₆₁₂-R₆₁₂, and X₆₁₃ may be N or C-(L₆₁₃)ₓₑ₆₁₃-R₆₁₃, wherein at least one of X₆₁₁ to X₆₁₃ may be N,
descriptions of L₆₁₁ to L₆₁₆ may each be independently as referred to in the description provided in connection with L₂₀₁,

R₆₁₁ to R₆₁₆ may each be independently selected from:
a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group; and
a phenyl group, a naphthyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group, each substituted with at least one of a deuterium, -F, -Cl, -Br, -I, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, an azulenyl group, a fluorenyl group, a spiro-fluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, and a triazinyl group, and
xe611 to xe616 may each be independently selected from 0, 1, 2, and 3.

The compound of Formula 601 and the compound of Formula 602 may each include at least one of Compounds ET1 to ET15:

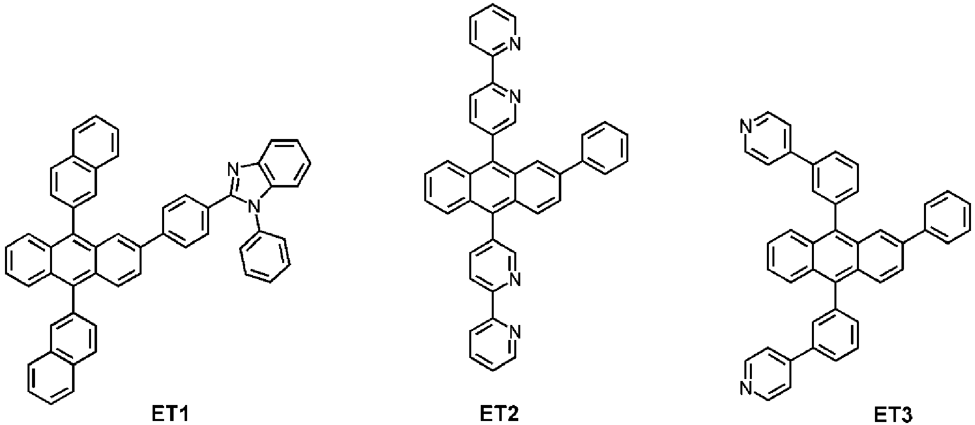

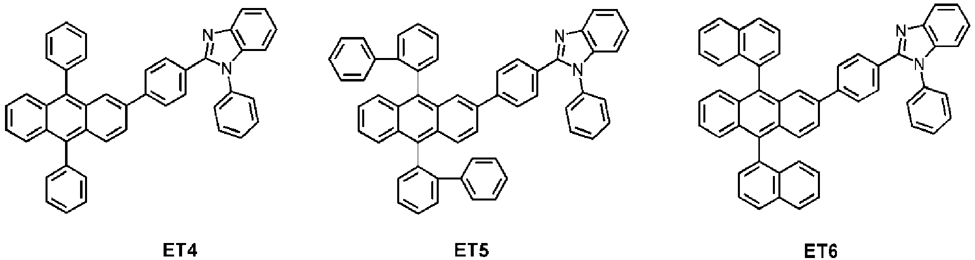

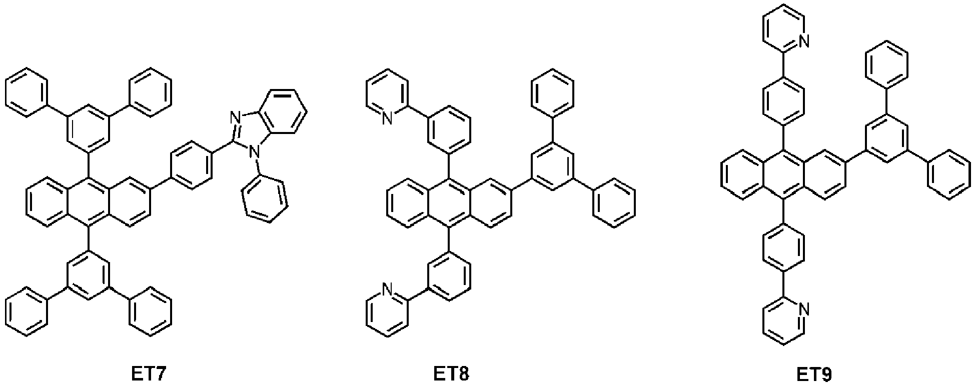

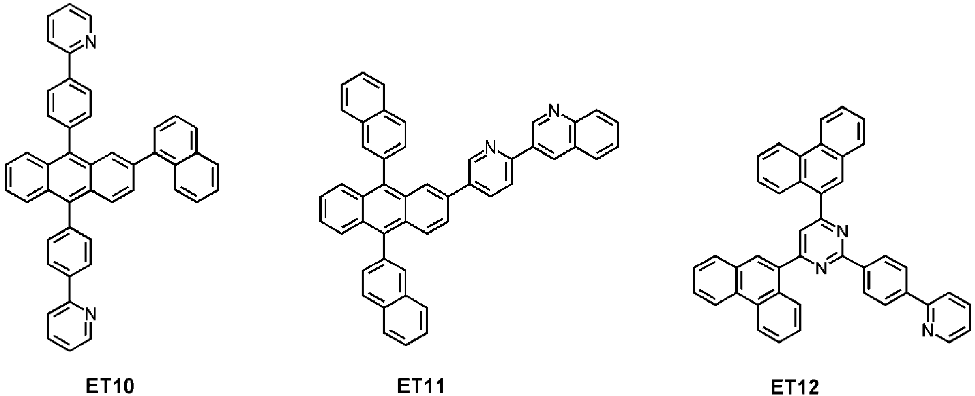

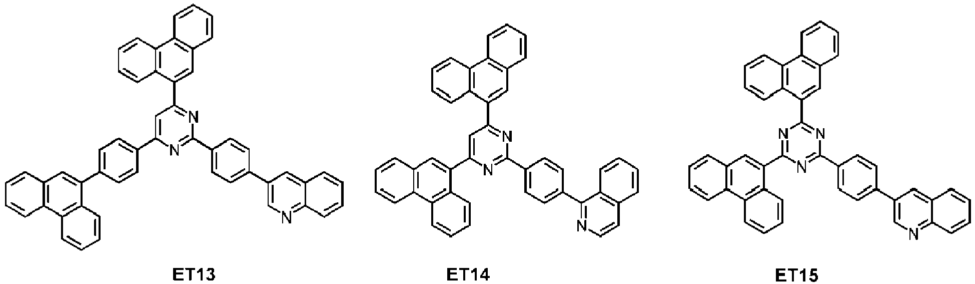

A thickness of the ETL may be in a range of about 100 Å to about 1,000 Å, e.g., about 150 Å to about 500 Å. When the thickness of the ETL is within these ranges, excellent electron transporting characteristics may be obtained without a substantial increase in driving voltage.

The ETL may further include, in addition to the materials described above, a metal-containing material.

The metal-containing material may include a Li complex. The Li complex may include, for example, Compound ET-D1 (e.g., lithium quinolate (LiQ)) or ET-D2.

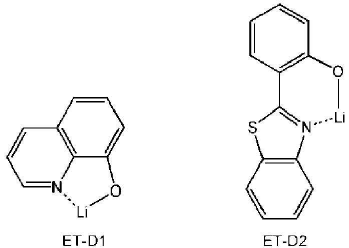

The electron transport region may include an EIL that facilitates electron injection from the second electrode 190.

The EIL may be formed on the ETL by using various suitable methods, such as vacuum deposition, spin coating, casting, a LB method, ink-jet printing, laser-printing, or LITI. When the EIL is formed by vacuum deposition or by spin coating, the deposition and coating conditions for the EIL may be determined by referring to the deposition and coating conditions for the HIL.

The EIL may include at least one selected from LiF, NaCl, CsF, Li₂O, BaO, and LiQ.

A thickness of the EIL may be in a range of about 1 Å to about 100 Å, e.g., about 3 Å to about 90 Å. When the thickness of the EIL is within these ranges, suitable or satisfactory electron injecting characteristics may be obtained without a substantial increase in driving voltage.

The second electrode 190 may be disposed on the electron transport region. The second electrode may be a cathode, which is an electron injection electrode. Here, a material for forming the second electrode 190 may be a material having a relatively low work function, such as a metal, an alloy, an electrically conductive compound, or a mixture thereof. Detailed examples of the material for forming the second electrode 190 may include lithium (Li), magnesium (Mg), aluminum (Al), aluminum-lithium (Al-Li), calcium (Ca), magnesium-indium (Mg-In), and magnesium-silver (Mg-Ag). Alternatively, the material for forming the second electrode 190 may include ITO or IZO. The second electrode 190 may be a reflective electrode, a semi-tranmissive electrode, or a transmissive electrode.

Hereinbefore, the organic light-emitting device 10 is described in connection with FIG. 1, but embodiments are not limited thereto.

The organic light-emitting device 10 may be used in a flat panetl display including a thin film transistor. The thin film transistor may include a gate electrode, source and drain electrodes, a gate insulating film, and an activation layer, wherein one of the source and drain electrodes may be electrically contact with the first electrode 110 of the organic light-emitting device 10. In addition, the activation layer may include cryatalline silicon, amorphous silicon, an organic semiconductor, or an oxide semiconductor, but the activation layer is not limited thereto.

A C₁-C₆₀ alkyl group as used herein refers to a linear or branched aliphatic hydrocarbon monovalent group having 1 to 60 carbon atoms, and detailed examples thereof include a methyl group, an ethyl group, an n-propyl group, an iso-propyl group, an n-butyl group, an iso-butyl group, a sec-butyl group, a tert-butyl group, pentyl group, an iso-amyl group, and a hexyl group. A C₁-C₆₀ alkylene group as used herein refers to a divalent group having the same structure as the C₁-C₆₀ alkyl group.

A C₁-C₆₀ alkoxy group as used herein refers to a monovalent group represented by -OA₁₀₁ (wherein A₁₀₁ is the C₁-C₆₀ alkyl group), and detailed examples thereof include a methoxy group, an ethoxy group, and an isopropyloxy group.

A C₂-C₆₀ alkenyl group as used herein refers to a hydrocarbon group formed by substituting at least one carbon double bond in the middle or terminal end of the C₂-C₆₀ alkyl group, and detailed examples thereof include an ethenyl group, a propenyl group, and a butenyl group. A C₂-C₆₀ alkenylene group as used herein refers to a divalent group having the same structure as the C₂-C₆₀ alkenyl group.

A C₂-C₆₀ alkynyl group 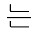 as used herein refers to a hydrocarbon group formed by substituting at least one carbon triple bond in a middle or terminal end of the C₂-C₆₀ alkyl group, and detailed examples thereof are an ethynyl group and a propynyl group. A C₂-C₆₀alkynylene group as used herein refers to a divalent group having the same structure as the C₂-C₆₀ alkynyl group.

A C₃-C₁₀ cycloalkyl group 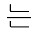 as used herein refers to a monovalent hydrocarbon monocyclic group having 3 to 10 carbon atoms, and detailed examples thereof include a cyclopropyl group, a cyclobutyl group, a cyclopentyl group, a cyclohexyl group, and a cycloheptyl group. A C₃-C₁₀ cycloalkylene group used herein refers to a divalent group having the same structure as the C₃-C₁₀ cycloalkyl group.

A C₁-C₁₀ heterocycloalkyl group as used herein refers to a monovalent monocyclic group having at least one heteroatom selected from N, O, P, and S as a ring-forming atom and 1 to 10 carbon atoms, and detailed examples thereof include a tetrahydrofuranyl group and a tetrahydrothiophenyl group. A C₁-C₁₀ heterocycloalkylene group as used herein refers to a divalent group having the same structure as the C₁-C₁₀ heterocycloalkyl group.

A C₃-C₁₀ cycloalkenyl groupas used herein refers to a monovalent monocyclic group that has 3 to 10 carbon atoms and at least one double bond in the ring thereof and does not have aromaticity (e.g., the ring is not aromatic), and detailed examples thereof include a cyclopentenyl group, a cyclohexenyl group, and a cycloheptenyl group. A C₃-C₁₀ cycloalkenylene group as used herein refers to a divalent group having the same structure as the C₃-C₁₀ cycloalkenyl group.

A C₁-C₁₀ heterocycloalkenyl group as used herein refers to a monovalent monocyclic group that has at least one heteroatom selected from N, O, P, and S as a ring-forming atom, 1 to 10 carbon atoms, and at least one double bond in the ring. Detailed examples of the C₁-C₁₀ heterocycloalkenyl group include a 2,3-hydrofuranyl group and a 2,3-hydrothiophenyl group. A C₁-C₁₀ heterocycloalkenylene group as used herein refers to a divalent group having the same structure as the C₁-C₁₀ heterocycloalkenyl group.

A C₆-C₆₀ aryl group as used herein refers to a monovalent group having a carbocyclic aromatic system having 6 to 60 carbon atoms, and a C₆-C₆₀ arylene group as used herein refers to a divalent group having a carbocyclic aromatic system having 6 to 60 carbon atoms. Detailed examples of the C₆-C₆₀ aryl group include a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, these rings may be fused to each other.

A C₁-C₆₀ heteroaryl groupas used herein refers to a monovalent group having a carbocyclic aromatic system that has at least one heteroatom selected from N, O, P, and S as a ring-forming atom, and 1 to 60 carbon atoms. A C₁-C₆₀ heteroarylene group as used herein refers to a divalent group having a carbocyclic aromatic system that has at least one heteroatom selected from N, O, P, and S as a ring-forming atom, and 1 to 60 carbon atoms. Detailed examples of the C₁-C₆₀ heteroaryl group include a pyridinyl group, a pyrimidinyl group, a pyrazinyl group, a pyridazinyl group, a triazinyl group, a quinolinyl group, and an isoquinolinyl group. When the C₁-C₆₀ heteroaryl group and the C₁-C₆₀ heteroarylene group each include two or more rings, these rings may be fused to each other.

A C₆-C₆₀ aryloxy group as used herein indicates -OA₁₀₂ (wherein A₁₀₂ is the C₆-C₆₀ aryl group), and a C₆-C₆₀ arylthio group as used herein indicates -SA₁₀₃ (wherein A₁₀₃ is the C₆-C₆₀ aryl group).

A monovalent non-aromatic condensed polycyclic group as used herein refers to a monovalent group that has two or more rings condensed to each other, has carbon atoms only as a ring-forming atom, and has non-aromaticity in the entire molecular structure. A detailed example of the monovalent non-aromatic condensed polycyclic group is a fluorenyl group. A divalent non-aromatic condensed polycyclic group as used herein refers to a divalent group having the same structure as the monovalent non-aromatic condensed polycyclic group.

A monovalent non-aromatic condensed heteropolycyclic group as used herein refers to a monovalent group that has two or more rings condensed to each other, has heteroatoms as a ring-forming atom selected from N, O, P, and S, in addition to C, and has non-aromaticity in the entire molecular structure. A detailed example of the monovalent non-aromatic condensed heteropolycyclic group is a carbazolyl group. A divalent non-aromatic condensed heteropolycyclic group used herein refers to a divalent group having the same structure as the monovalent non-aromatic condensed heteropolycyclic group.

The term "Ph" as used herein refers to a phenyl group, the term "Me" as used herein refers to a methyl group, the term "Et" as used herein refers to an ethyl group, and the term "ter-Bu" or "Bu^{t}" as used herein refers to a tert-butyl group.

Hereinafter, an organic light-emitting device according to an exemplary embodiment will be described in detail with reference to Examples below.

### [Examples]

Compounds used in Examples 1 to 4 are as follows:

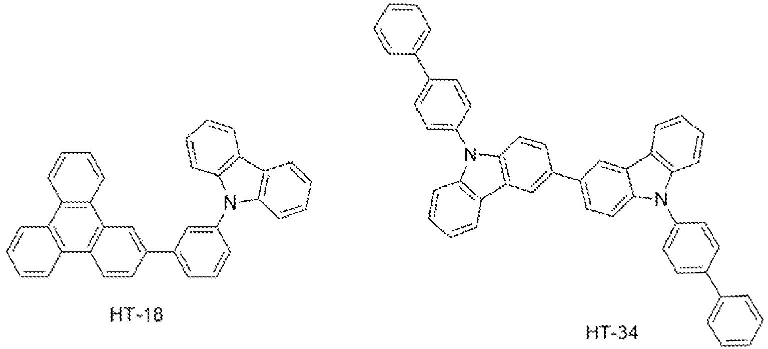

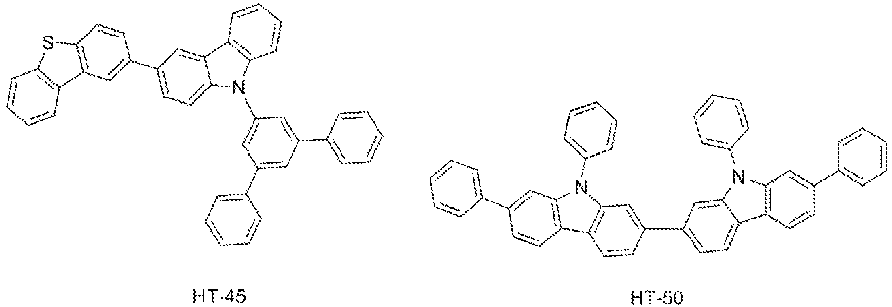

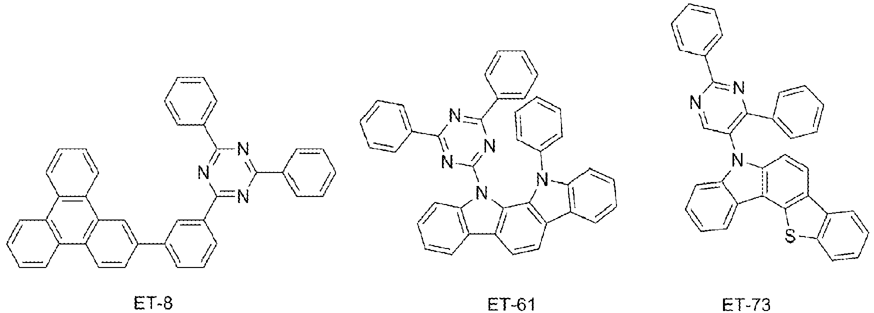

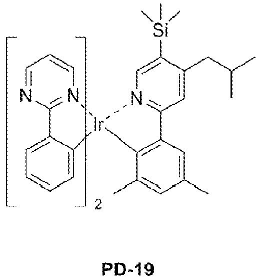

### Example 1

As an anode, a 15 Ω/cm² (1,200 Å) ITO glass substrate (manufactured by Corning company) was cut into a size of 50 mm x 50 mm x 0.7 mm and ultrasonically washed with isopropyl alcohol and pure water, each for 5 minutes. Afterwards, the ITO glass substrate was irradiated by UV light for 30 minutes, cleaned by exposure to ozone, and then, mounted on a vacuum depositor.

m-MTDATA was vacuum-deposited on the substrate to form a hole injection layer (HIL) having a thickness of 300 Å, and then, NPB was vacuum-deposited on the HIL to form a hole transport layer (HTL) having a thickness of 600 Å. Subsequently, Compound 4 was deposited on the HTL to form an auxiliary layer having a thickness of 100 Å.

HT-18, ET-61, and PD-19 were co-deposited on the auxiliary layer at a weight ratio of 72:18:10 to form an emission layer (EML) having a thickness of 300 Å. Subsequently, ET1 and LiF were co-deposited at a weight ratio of 1: 1 on the EML to form an electron transport layer (ETL) having a thickness of 300 Å. LiF was vacuum-deposited on the ETL to form an electron injection layer (EIL) having a thickness of 10 Å, and Al was vacuum-deposited on the EIL to form a cathode having a thickness of 1,000 Å, thereby manufacturing an organic light-emitting device.

### Example 2

An organic light-emitting device was manufactured in the same manner as in Example 1, except that in forming the EML, HT-50 and ET-8 were used instead of HT-18 and ET-61, respectively.

### Example 3

An organic light-emitting device was manufactured in the same manner as in Example 1, except that in forming the EML, HT-34 and ET-73 were used instead of HT-18 and ET-61, respectively.

### Example 4

An organic light-emitting device was manufactured in the same manner as in Example 1, except that in forming the EML, HT-45 was used instead of HT-18.

### Comparative Example 1

An organic light-emitting device was manufactured in the same manner as in Example 1, except that in forming the EML, mCP was used instead of HT-18 and ET-61.

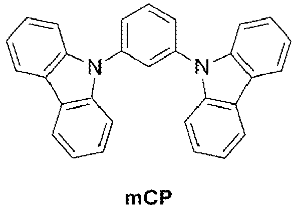

### Comparative Example 2

An organic light-emitting device was manufactured in the same manner as in Example 1, except that in forming the EML, Compound X was used instead of PD-19.

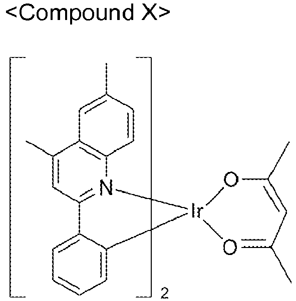

### Evaluation Example 1

Photoluminescence (PL) spectra of PD-19 used in Example 1 and Compound X used in Comparative Example 2 were measured, and the results are shown in FIG. 2.

Referring to FIG. 2, it was confirmed that there was a blue shift of 12 nm in the PL spectra of PD-19 (λₘₐₓ= 512 nm) and Compound X (λₘₐₓ= 524 nm), and that the half-width of PD-19 was decreased from 80 nm to 52 nm (i.e., a decrease of 28 nm).

### Evaluation Example 2

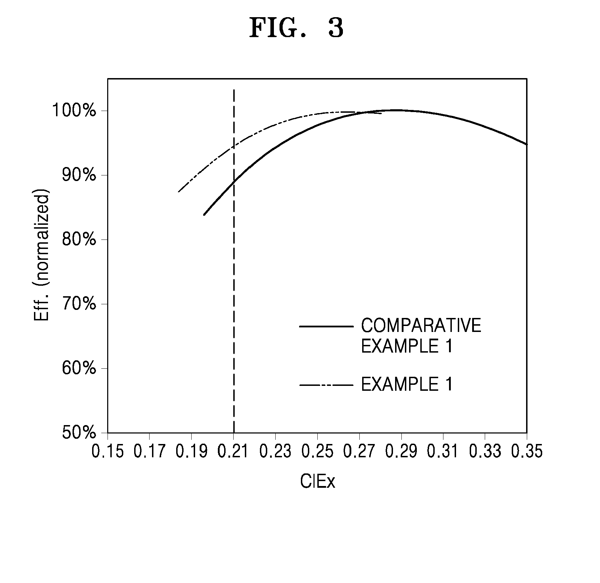

The organic light-emitting devices of Examples 1 to 4 and Comparative Examples 1 and 2 were subjected to measure and evaluate driving voltages, efficiencies, lifespans (at a current density of 1,000 nit), and color coordinates by using a PR650 (Spectroscan) Source Measurement Unit (available from PhotoResearch, Inc.), and the results are shown in Table 1 and FIG. 3. In Table 1, the term "efficiency" refers to a relative efficiency, and the term "lifespan" refers to a relative lifespan, among the organic light-emitting devices.

**[Table 1]**

| Organic light-emitting device | First host | Second host | Dopant | Weight ratio of first host and second host | Driving voltage (V) | Efficiency | Lifespan |
|---|---|---|---|---|---|---|---|
| Example 1 | HT-18 | ET-61 | PD-19 | 8:2 | 5.1 | 1 | 1.3 |
| Example 2 | HT-50 | ET-8 | PD-19 | 8:2 | 4.5 | 1.3 | 1.8 |
| Example 3 | HT-34 | ET-73 | PD-19 | 8:2 | 4.1 | 1.5 | 1.5 |
| Example 4 | HT-45 | ET-73 | PD-19 | 8:2 | 4.2 | 1.6 | 1.6 |
| Comparative Example 1 | mCP | - | PD-19 | - | 4.6 | 1 | 1 |
| Comparative Example 2 | HT-18 | ET-61 | Compound X | 8:2 | 5.0 | 0.7 | 0.6 |

Referring to Table 1, it was confirmed that the organic light-emitting devices of Examples 1 to 4 had improved efficiencies and lifespans compared to those of the organic light-emitting devices of Comparative Examples 1 and 2.

Referring to FIG. 3, it was confirmed that light emitted from the organic light-emitting device manufactured in Example 1 had a shorter wavelength than that of light emitted from the organic light-emitting device manufactured in Comparative Example 2. In addition, it was confirmed that, compared to the organic light-emitting device of Comparative Example 2, light emitted from the organic light-emitting device manufactured in Example 1 was shifted towards short wavelengths based on the light emission wavelengths in CIEx, and there was little change in the efficiency at the x-coordinate value of 0.21. In addition, as shown in Evaluation Example 1, the half-width of PD-19 used in Example 1 was decreased compared to that of Compound X used in Comparative Example 2 (i.e., a decrease from 80 nm to 52 nm), leading to the improvement of the intensity in the actual peak areas. In this regard, the efficiency of the organic light-emitting device would not be decreased.

Hereinafter, compounds used in manufacturing organic light-emitting devices manufactured in Examples 1-1A to 1-33A and Comparative Examples 1-1A to 1-6A are as follows:

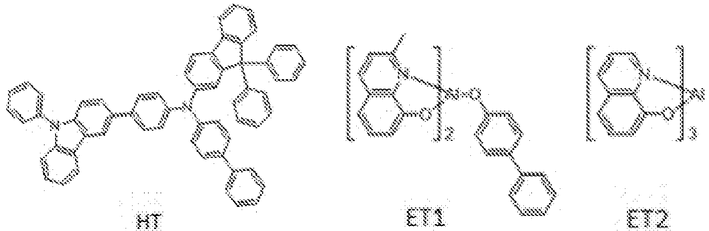

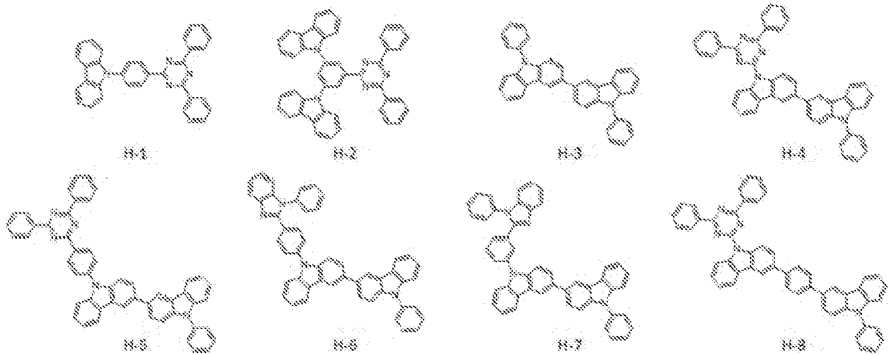

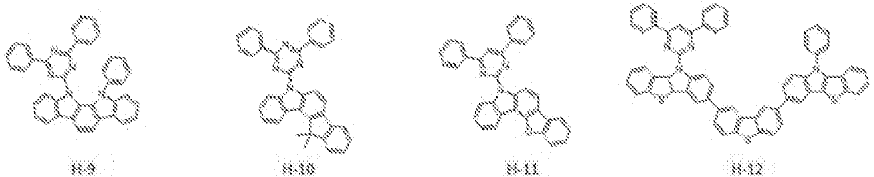

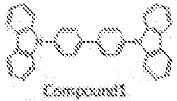

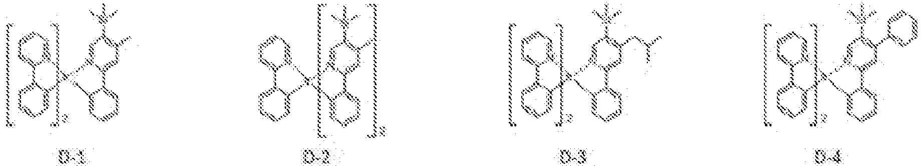

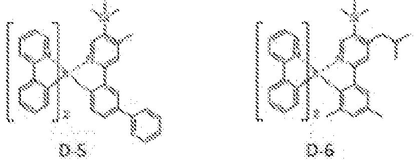

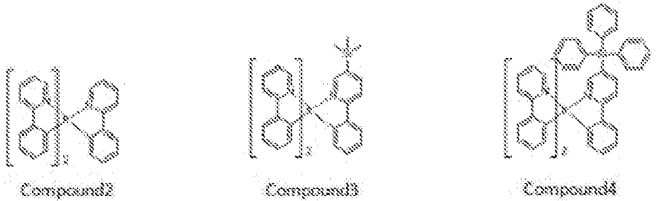

### Example 1-1A

### Glass/ITO(120 nm)/HT(120 nm)/HostDop_7% (30 nm)/ET1(5 nm)/ET2(25 nm)/LiF(0.5 nm)/AI(150 nm)

As an anode, a 15 Ω/cm² (1,200 Å) ITO glass substrate (manufactured by Corning company) was cut into a size of 50 mm x 50 mm x 0.7 mm and ultrasonically washed with isopropyl alcohol and pure water, each for 5 minutes. Afterwards, the ITO glass substrate was irradiated by UV light for 30 minutes, cleaned by exposure to ozone, and then, mounted on a vacuum depositor.

Compound HT was vacuum-deposited on the substrate to form a hole transport region having a thickness of 120 nm.

Compound H-1 and Compound D-1 (7 v%) were co-deposited on the hole transport region to form an EML having a thickness of 30 nm.

Compound ET1 was deposited on the EML to form a buffer layer having a thickness of 5 nm, and Compound ET2 was deposited on the buffer layer to form an electron transport layer (ETL) having a thickness of 25 nm. LiF was deposited on the ETL to form an electron injection layer (EIL) having a thickness of 0.5 nm, thereby preparing an electron transport region.

Al was deposited on the electron transport region to a thickness of 150 nm, thereby manufacturing an organic light-emitting device.

### Examples 1-2A to 1-33A and Comparative Examples 1-1A to 1-6A

Organic light-emitting devices were manufactured in the same manner as in Example 1-1A, except that in forming the EML, host and dopant materials shown in Table 2 were used:

**[Table 2]**

| | Host | Dopant |
|---|---|---|
| Example 1-1A | H-1 | D-1 |
| Example 1-2A | H-1 | D-2 |
| Example 1-3A | H-1 | D-3 |
| Example 1-4A | H-1 | D-4 |
| Example 1-5A | H-1 | D-5 |
| Example 1-6A | H-1 | D-6 |
| Example 1-7A | H-2 | D-1 |
| Example 1-8A | H-2 | D-3 |
| Example 1-9A | H-2 | D-5 |
| Example 1-10A | H-3 | D-1 |
| Example 1-11A | H-3 | D-3 |
| Example 1-12A | H-3 | D-5 |
| Example 1-13A | H-4 | D-1 |
| Example 1-14A | H-4 | D-3 |
| Example 1-15A | H-4 | D-5 |
| Example 1-16A | H-5 | D-1 |
| Example 1-17A | H-5 | D-3 |
| Example 1-18A | H-5 | D-5 |
| Example 1-19A | H-8 | D-1 |
| Example 1-20A | H-8 | D-3 |
| Example 1-21A | H-8 | D-5 |
| Example 1-22A | H-9 | D-1 |
| Example 1-23A | H-9 | D-3 |
| Example 1-24A | H-9 | D-5 |
| Example 1-25A | H-10 | D-1 |
| Example 1-26A | H-10 | D-3 |
| Example 1-27A | H-10 | D-5 |
| Example 1-28A | H-11 | D-1 |
| Example 1-29A | H-11 | D-3 |
| Example 1-30A | H-11 | D-5 |
| Example 1-31A | H-12 | D-1 |
| Example 1-32A | H-12 | D-3 |
| Example 1-33A | H-12 | D-5 |
| Comparative Example 1-1A | Compound 1 | Compound 2 |
| Comparative Example 1-2A | Compound 1 | Compound 3 |
| Comparative Example 1-3A | Compound 1 | Compound 4 |
| Comparative Example 1-4A | Compound 1 | D-1 |
| Comparative Example 1-5A | H-1 | Compound 2 |
| Comparative Example 1-6A | H-2 | Compound 2 |

### Evaluation Example 3

The organic light-emitting devices of Examples 1-1A to 1-33A and Comparative Examples 1-1A to 1-6A were subjected to measure and evaluate efficiencies (@ current density of 10 mA/cm²), lifespan data (@ 50 mA/cm²), and color coordinates, by using an IVL meter (PhotoResearch PR650, Keithley 238), and the results are shown in Table 3. In Table 3, the term "efficiency" refers to a relative efficiency, and the term "lifespan" refers to a relative lifespan, among the organic light-emitting devices.

**[Table 3]**

| | Host | Dopant | Efficiency | Lifespan | Color coordinates (x,y) |
|---|---|---|---|---|---|
| Example 1-1A | H-1 | D-1 | 1.3 | 1.1 | 0.23, 0.69 |
| Example 1-2A | H-1 | D-2 | 1.2 | 1.1 | 0.24, 0.68 |
| Example 1-3A | H-1 | D-3 | 1.3 | 1.2 | 0.22, 0.69 |
| Example 1-4A | H-1 | D-4 | 1.3 | 1.2 | 0.23, 0.68 |
| Example 1-5A | H-1 | D-5 | 1.3 | 1.3 | 0.23, 0.68 |
| Example 1-6A | H-1 | D-6 | 1.2 | 1.2 | 0.22, 0.69 |
| Example 1-7A | H-2 | D-1 | 1.2 | 1.2 | 0.23, 0.69 |
| Example 1-8A | H-2 | D-3 | 1.3 | 1.3 | 0.22, 0.69 |
| Example 1-9A | H-2 | D-5 | 1.2 | 1.3 | 0.23, 0.68 |
| Example 1-10A | H-3 | D-1 | 1.3 | 1.1 | 0.23, 0.69 |
| Example 1-11A | H-3 | D-3 | 1.2 | 1.2 | 0.22, 0.69 |
| Example 1-12A | H-3 | D-5 | 1.3 | 1.3 | 0.23, 0.68 |
| Example 1-13A | H-4 | D-1 | 1.4 | 1.3 | 0.23, 0.69 |
| Example 1-14A | H-4 | D-3 | 1.3 | 1.3 | 0.22, 0.69 |
| Example 1-15A | H-4 | D-5 | 1.4 | 1.4 | 0.23, 0.68 |
| Example 1-16A | H-5 | D-1 | 1.4 | 1.2 | 0.23, 0.69 |
| Example 1-17A | H-5 | D-3 | 1.3 | 1.3 | 0.22, 0.69 |
| Example 1-18A | H-5 | D-5 | 1.3 | 1.4 | 0.23, 0.68 |
| Example 1-19A | H-8 | D-1 | 1.4 | 1.2 | 0.23, 0.69 |
| Example 1-20A | H-8 | D-3 | 1.3 | 1.3 | 0.22, 0.69 |
| Example 1-21A | H-8 | D-5 | 1.3 | 1.3 | 0.23, 0.68 |
| Example 1-22A | H-9 | D-1 | 1.3 | 1.2 | 0.23, 0.69 |
| Example 1-23A | H-9 | D-3 | 1.2 | 1.2 | 0.22, 0.69 |
| Example 1-24A | H-9 | D-5 | 1.2 | 1.2 | 0.23, 0.68 |
| Example 1-25A | H-10 | D-1 | 1.3 | 1.2 | 0.23, 0.69 |
| Example 1-26A | H-10 | D-3 | 1.3 | 1.2 | 0.22, 0.69 |
| Example 1-27A | H-10 | D-5 | 1.3 | 1.2 | 0.23, 0.68 |
| Example 1-28A | H-11 | D-1 | 1.3 | 1.3 | 0.23, 0.69 |
| Example 1-29A | H-11 | D-3 | 1.2 | 1.3 | 0.22, 0.69 |
| Example 1-30A | H-11 | D-5 | 1.3 | 1.4 | 0.23, 0.68 |
| Example 1-31A | H-12 | D-1 | 1.2 | 1.2 | 0.23, 0.69 |
| Example 1-32A | H-12 | D-3 | 1.1 | 1.3 | 0.22, 0.69 |
| Example 1-33A | H-12 | D-5 | 1.2 | 1.2 | 0.23, 0.68 |
| Comparative Example 1-1A | Compound 1 | Compound 2 | 1.0 | 1.0 | 0.30, 0.67 |
| Comparative Example 1-2A | Compound 1 | Compound 3 | 1.1 | 1.1 | 0.26, 0.69 |
| Comparative Example 1-3A | Compound 1 | Compound 4 | 1.1 | 1.1 | 0.28, 0.66 |
| Comparative Example 1-4A | Compound 1 | D-1 | 1.1 | 1.1 | 0.23, 0.69 |
| Comparative Example 1-5A | H-1 | Compound 2 | 1.1 | 1.2 | 0.30, 0.67 |
| Comparative Example 1-6A | H-2 | Compound 2 | 1.0 | 1.1 | 0.30, 0.66 |

Referring to Table 3, it was confirmed that the organic light-emitting devices of Examples 1-1A to 1-33A had improved efficiencies and lifespans compared to those of the organic light-emitting devices of Comparative Examples 1-1A to 1-6A.

Hereinafter, compounds used in manufacturing organic light-emitting devices manufactured in Examples 2-1A to 2-8A and Comparative Examples 2-1A to 2-8A are as follows:

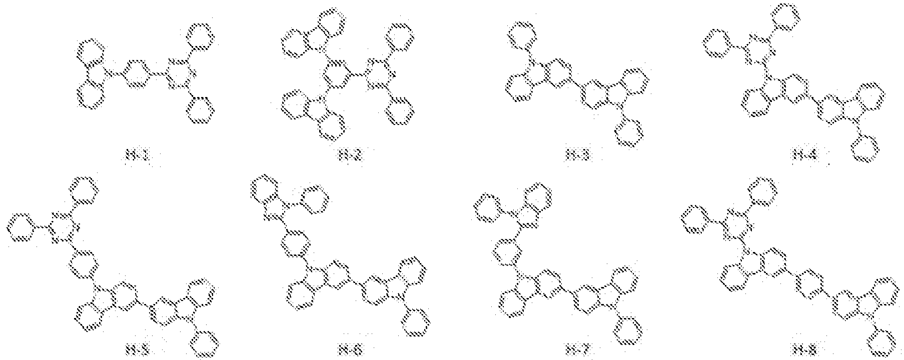

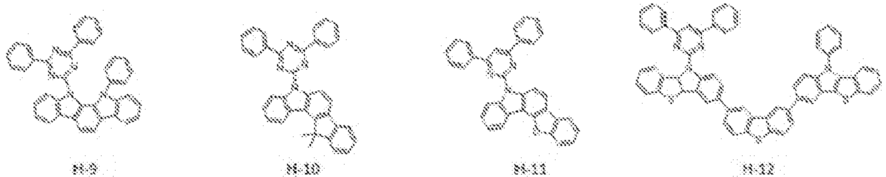

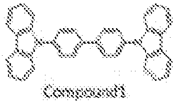

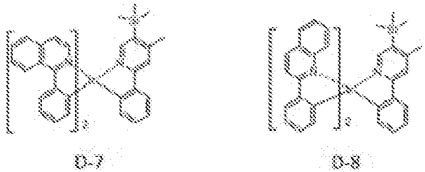

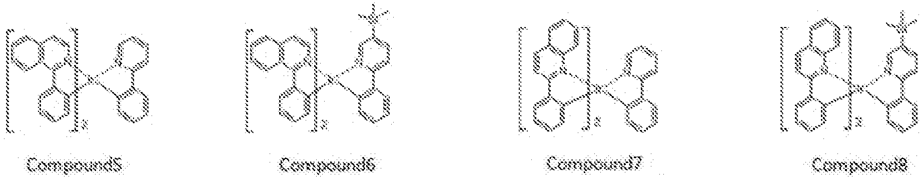

### Examples 2-1A to 2-8A and Comparative Examples 2-1A to 2-8A

Glass/ITO(120 nm)/HT(120 nm)/Host:Dop_1% (30 nm)/ET1(5 nm)/ET2(25 nm)/LiF(0.5 nm)/AI(150 nm)

Organic light-emitting devices were manufactured in the same manner as in Example 1-1A, except that in forming the EML, dopant materials listed in Table 4 were used, and the amounts of the dopants were changed to 1 v%:

**[Table 4]**

| | Host | Dopant |
|---|---|---|
| Example 2-1A | H-1 | D-7 |
| Example 2-2A | H-5 | D-7 |
| Example 2-3A | H-6 | D-7 |
| Example 2-4A | H-7 | D-7 |
| Comparative Example 2-1A | Compound 1 | Compound 5 |
| Comparative Example 2-2A | Compound 1 | Compound 6 |
| Comparative Example 2-3A | Compound 1 | D-7 |
| Comparative Example 2-4A | H-1 | Compound 5 |
| Example 2-5A | H-1 | D-8 |
| Example 2-6A | H-5 | D-8 |
| Example 2-7A | H-6 | D-8 |
| Example 2-8A | H-7 | D-8 |
| Comparative Example 2-5A | Compound 1 | Compound 7 |
| Comparative Example 2-6A | Compound 1 | Compound 8 |
| Comparative Example 2-7A | Compound 1 | D-8 |
| Comparative Example 2-8A | H-1 | Compound 7 |

### Evaluation Example 4

The organic light-emitting devices of Examples 2-1A to 2-8A and Comparative Examples 2-1A to 2-8A were subjected to measure and evaluate efficiencies (@ current density of 10 mA/cm²), lifespan data (@ 50 mA/cm²), and color coordinates, by using an IVL meter (PhotoResearch PR650, Keithley 238), and the results are shown in Table 5. In Table 5, the term "efficiency" refers to a relative efficiency, and the term "lifespan" refers to a relative lifespan, among the organic light-emitting devices.

**[Table 5]**

| | Host | Dopant | Efficieny | Lifespan | Color coordinates (x, y) |
|---|---|---|---|---|---|
| Example 2-1A | H-1 | D-7 | 1.2 | 1.1 | 0.66,0.33 |
| Example 2-2A | H-5 | D-7 | 1.3 | 1.2 | 0.66,0.33 |
| Example 2-3A | H-6 | D-7 | 1.2 | 1.3 | 0.66,0.33 |
| Example 2-4A | H-7 | D-7 | 1.2 | 1.3 | 0.66,0.33 |
| Comparative Example 2-1A | Compound 1 | Compound 5 | 1.0 | 1.0 | 0.64, 0.34 |
| Comparative Example 2-2A | Compound 1 | Compound 6 | 1.1 | 1.0 | 0.65, 0.34 |
| Comparative Example 2-3A | Compound 1 | D-7 | 1.1 | 1.1 | 0.66,0.33 |
| Comparative Example 2-4A | H-1 | Compound 5 | 1.1 | 1.2 | 0.64, 0.34 |
| Example 2-5A | H-1 | D-8 | 1.1 | 1.1 | 0.64, 0.34 |
| Example 2-6A | H-5 | D-8 | 1.2 | 1.2 | 0.64, 0.34 |
| Example 2-7A | H-6 | D-8 | 1.3 | 1.2 | 0.64, 0.34 |
| Example 2-8A | H-7 | D-8 | 1.2 | 1.3 | 0.64, 0.34 |
| Comparative Example 2-5A | Compound 1 | Compound 7 | 1.0 | 1.0 | 0.62,0.35 |
| Comparative Example 2-6A | Compound 1 | Compound 8 | 1.1 | 1.0 | 0.63, 0.34 |
| Comparative Example 2-7A | Compound 1 | D-8 | 1.1 | 1.1 | 0.64, 0.34 |
| Comparative Example 2-8A | H-1 | Compound 7 | 1.1 | 1.1 | 0.62, 0.35 |

Referring to Table 5, it was confirmed that the organic light-emitting devices of Examples 2-1A to 2-8A had improved efficiencies and lifespans compared to those of the organic light-emitting devices of Comparative Examples 2-1A to 2-8A.

Hereinafter, compounds used in manufacturing organic light-emitting devices manufactured in Examples 3-1A to 3-16A and Comparative Example 3-1A to 3-10A are as follows:

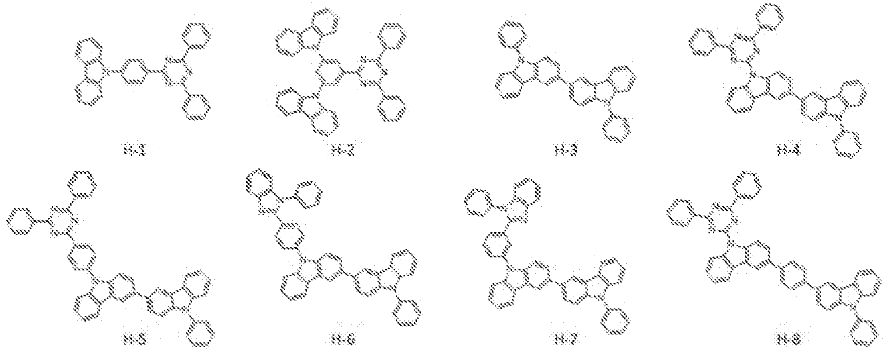

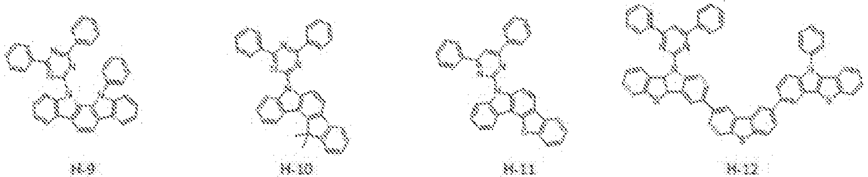

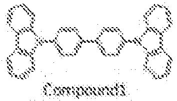

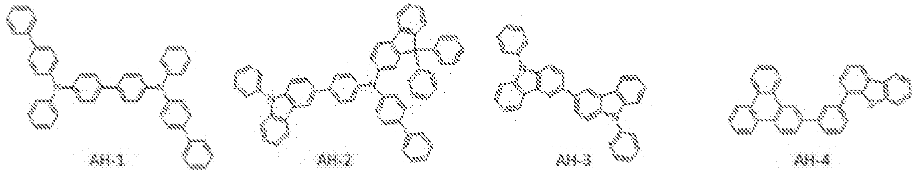

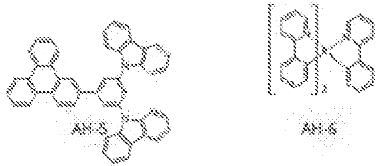

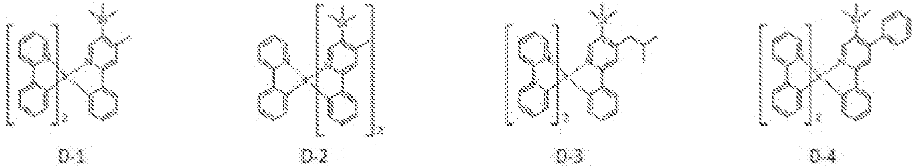

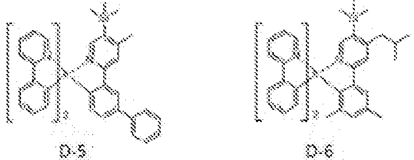

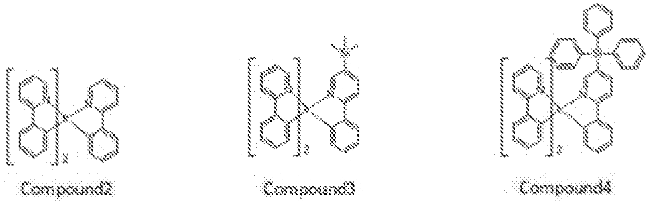

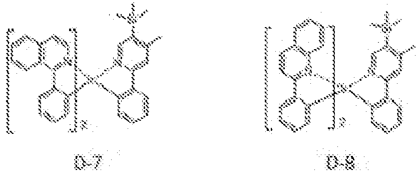

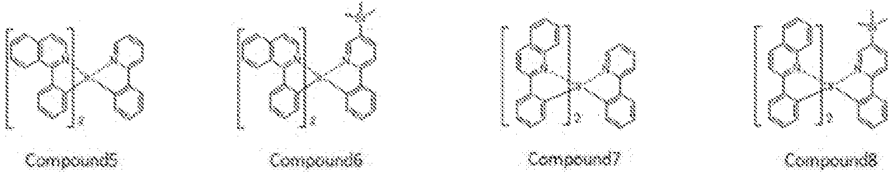

### Example 3-1A to 3-16A and Comparative Example 3-1A to 3-10A

### Glass/ITO(120 nm)/HT(120 nm)/Host1:Host2_10%:Dop_x%(30 nm)/ET1(5 nm)/ET2(25 nm)/LiF(0.5 nm)/AI(150 nm)

Organic light-emitting devices were manufactured in the same manner as in Example 1-1A, except that in forming the EML, host materials listed in Table 6 were used as the first host and the second host (wherein 10 v% of the second host was used), the amounts of the dopants were changed to amounts shown in Table 6, and dopant materials listed in Table 6 were used.

**[Table 6]**

| | First host | Second host | Dopant | Dopant (v%) |
|---|---|---|---|---|
| Example 3-1A | H-4 | AH-1 | D-1 | 7 |
| Example 3-2A | H-4 | AH-2 | D-1 | 7 |
| Example 3-3A | H-4 | AH-3 | D-1 | 7 |
| Example 3-4A | H-4 | AH-4 | D-1 | 7 |
| Example 3-5A | H-9 | AH-1 | D-1 | 7 |
| Example 3-6A | H-9 | AH-2 | D-1 | 7 |
| Example 3-7A | H-9 | AH-3 | D-1 | 7 |
| Example 3-8A | H-9 | AH-4 | D-1 | 7 |
| Comparative Example 3-1A | Compound 1 | - | Compound 2 | 7 |
| Comparative Example 3-2A | Compound 1 | - | Compound 3 | 7 |
| Comparative Example 3-3A | Compound 1 | - | D-1 | 7 |
| Comparative Example 3-4A | H-1 | - | Compound 2 | 7 |
| Comparative Example 3-5A | Compound 1 | AH-3 | Compound 2 | 7 |
| Example 3-9A | H-6 | AH-1 | D-7 | 1 |
| Example 3-10A | H-6 | AH-2 | D-7 | 1 |
| Example 3-11A | H-6 | AH-3 | D-7 | 1 |
| Example 3-12A | H-6 | AH-6 | D-7 | 1 |
| Example 3-13A | H-7 | AH-1 | D-7 | 1 |
| Example 3-14A | H-7 | AH-2 | D-7 | 1 |
| Example 3-15A | H-7 | AH-3 | D-7 | 1 |
| Example 3-16A | H-7 | AH-6 | D-7 | 1 |
| Comparative Example 3-6A | Compound 1 | - | Compound 5 | 1 |
| Comparative Example 3-7A | Compound 1 | - | Compound 6 | 1 |
| Comparative Example 3-8A | Compound 1 | - | D-7 | 1 |
| Comparative Example 3-9A | H-3 | - | Compound 5 | 1 |
| Comparative Example 3-10A | Compound 1 | AH-1 | Compound 5 | 1 |

### Evaluation Example 5

The organic light-emitting devices of Examples 3-1A to 3-16A and Comparative Examples 3-1A to 3-10A were subjected to measure and evaluate efficiencies (@ current density of 10 mA/cm²), lifespan data (@ 50 mA/cm²), and color coordinates, by using an IVL meter (PhotoResearch PR650, Keithley 238), and the results are shown in Table 7. In Table 7, the term "efficiency" refers to a relative efficiency, and the term "lifespan" refers to a relative lifespan, among the organic light-emitting devices.

**[Table 7]**

| | First host | Second host | Dopant | Dopant (v%) | Efficieny | Lifespan | Color coordinates (x, y) |
|---|---|---|---|---|---|---|---|
| Example 3-1A | H-4 | AH-1 | D-1 | 7 | 1.2 | 1.3 | 0.23, 0.69 |
| Example 3-2A | H-4 | AH-2 | D-1 | 7 | 1.3 | 1.3 | 0.23, 0.69 |
| Example 3-3A | H-4 | AH-3 | D-1 | 7 | 1.3 | 1.4 | 0.23, 0.69 |
| Example 3-4A | H-4 | AH-4 | D-1 | 7 | 1.2 | 1.3 | 0.24, 0.69 |
| Example 3-5A | H-9 | AH-1 | D-1 | 7 | 1.1 | 1.2 | 0.23, 0.69 |
| Example 3-6A | H-9 | AH-2 | D-1 | 7 | 1.2 | 1.2 | 0.23, 0.69 |
| Example 3-7A | H-9 | AH-3 | D-1 | 7 | 1.4 | 1.4 | 0.23, 0.69 |
| Example 3-8A | H-9 | AH-4 | D-1 | 7 | 1.5 | 1.4 | 0.24, 0.69 |
| Comparative Example 3-1A | Compound 1 | - | Compound 2 | 7 | 1.0 | 1.0 | 0.30, 0.67 |
| Comparative Example 3-2A | Compound 1 | - | Compound 3 | 7 | 1.1 | 1.1 | 0.26, 0.69 |
| Comparative Example 3-3A | Compound 1 | - | D-1 | 7 | 1.1 | 1.1 | 0.23, 0.69 |
| Comparative Example 3-4A | H-1 | - | Compound 2 | 7 | 1.1 | 1.2 | 0.30, 0.67 |
| Comparative Example 3-5A | Compound 1 | AH-3 | Compound 2 | 7 | 1.0 | 1.2 | 0.30, 0.67 |
| Example 3-9A | H-6 | AH-1 | D-7 | 1 | 1.2 | 1.4 | 0.66, 0.33 |
| Example 3-10A | H-6 | AH-2 | D-7 | 1 | 1.3 | 1.3 | 0.66, 0.33 |
| Example 3-11A | H-6 | AH-3 | D-7 | 1 | 1.3 | 1.3 | 0.66, 0.33 |
| Example 3-12A | H-6 | AH-6 | D-7 | 1 | 1.3 | 1.3 | 0.66, 0.33 |
| Example 3-13A | H-7 | AH-1 | D-7 | 1 | 1.2 | 1.4 | 0.66, 0.33 |
| Example 3-14A | H-7 | AH-2 | D-7 | 1 | 1.3 | 1.3 | 0.66, 0.33 |
| Example 3-15A | H-7 | AH-3 | D-7 | 1 | 1.3 | 1.4 | 0.66, 0.33 |
| Example 3-16A | H-7 | AH-6 | D-7 | 1 | 1.3 | 1.4 | 0.66, 0.33 |
| Comparative Example 3-6A | Compound 1 | - | Compound 5 | 1 | 1.0 | 1.0 | 0.64, 0.34 |
| Comparative Example 3-7A | Compound 1 | - | Compound 6 | 1 | 1.1 | 1.0 | 0.65, 0.34 |
| Comparative Example 3-8A | Compound 1 | - | D-7 | 1 | 1.1 | 1.1 | 0.66, 0.33 |
| Comparative Example 3-9A | H-3 | - | Compound 5 | 1 | 1.1 | 1.2 | 0.64, 0.34 |
| Comparative Example 3-10A | Compound 1 | AH-1 | Compound 5 | 1 | 1.0 | 1.2 | 0.64, 0.34 |

Referring to Table 7, it was confirmed that the organic light-emitting devices of Examples 3-1A to 3-16A had improved efficiencies and lifespans compared to those of the organic light-emitting devices of Comparative Examples 3-1A to 3-10A.

Hereinafter, compounds used in manufacturing organic light-emitting devices manufactured in Examples 1-1 B to 1-12B and Comparative Examples 1-1 B to 1-6B are as follows:

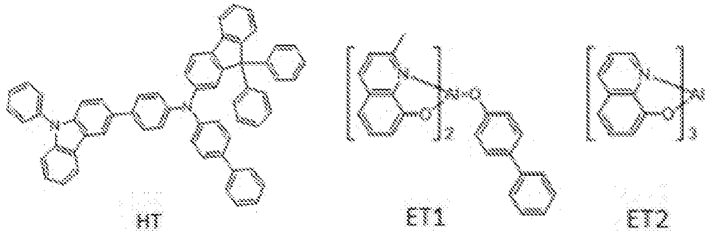

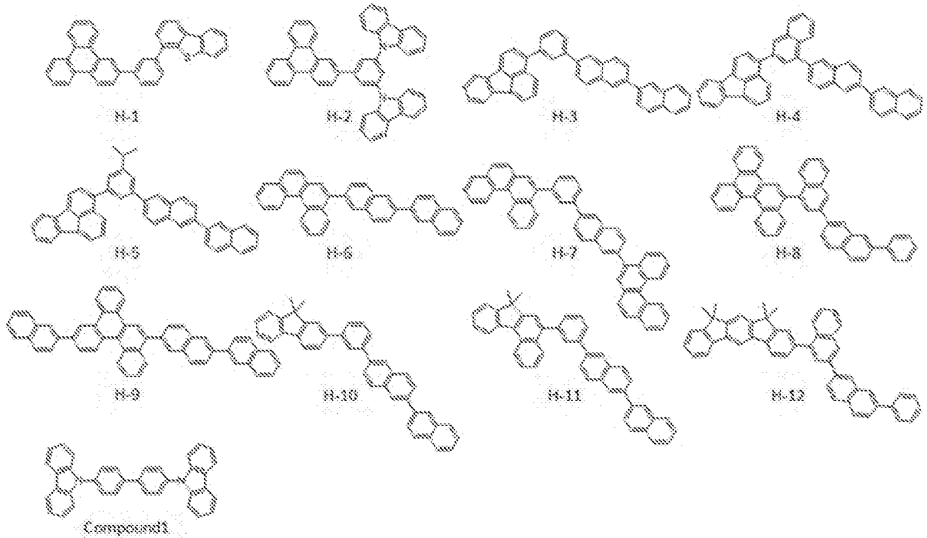

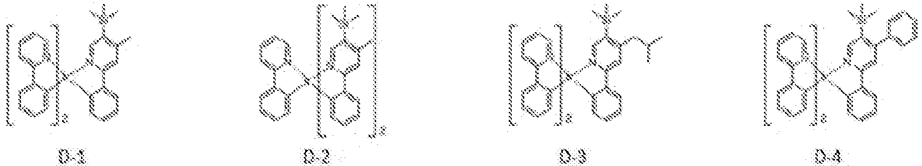

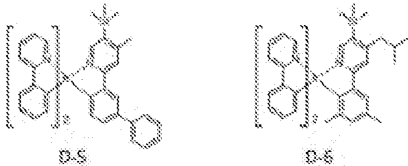

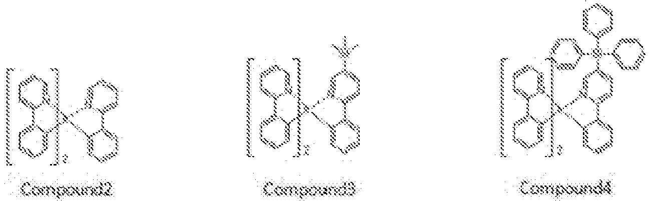

### Example 1-1 B

### Glass/ITO(120 nm)/HT(120 nm)/Host:Dop_7% (30 nm)/ET1(5 nm)/ET2(25 nm)/LiF(0.5 nm)/AI(150 nm)

As an anode, a 15 Ω/cm² (1,200 Å) ITO glass substrate (manufactured by Corning company) was cut into a size of 50 mm x 50 mm x 0.7 mm and ultrasonically washed with isopropyl alcohol and pure water, each for 5 minutes. Afterwards, the ITO glass substrate was irradiated by UV light for 30 minutes, cleaned by exposure to ozone, and then, mounted on a vacuum depositor.

Compound HT was vacuum-deposited on the substrate to form a hole transport region having a thickness of 120 nm.

Compound H-1 and D-1 (7 v%) were copdeposited on the hole transport region to form an EML having a thickness of 30 nm.

Compound ET1 was deposited on the EML to form a buffer layer having a thickness of 5 nm, and Compound ET2 was deposited on the buffer layer to form an electron transport layer (ETL) having a thickness of 25 nm. LiF was deposited on the ETL to form an electron injection layer (EIL) having a thickness of 0.5 nm, thereby preparing an electron transport region.

Al was deposited on the electron transport region to a thickness of 150 nm, thereby manufacturing an organic light-emitting device.

### Examples 1-2B to 1-12B and Comparative Examples 1-1B to 1-6B

Organic light-emitting devices were manufactured in the same manner as in Example 1-1 B, except that in forming the EML, host and dopant materials shown in Table 2 were used:

**[Table 8]**

| | Host | Dopant |
|---|---|---|
| Example 1-1B | H-1 | D-1 |
| Example 1-2B | H-1 | D-2 |
| Example 1-3B | H-1 | D-3 |
| Example 1-4B | H-1 | D-4 |
| Example 1-5B | H-1 | D-5 |
| Example 1-6B | H-1 | D-6 |
| Example 1-7B | H-2 | D-1 |
| Example 1-8B | H-2 | D-2 |
| Example 1-9B | H-2 | D-3 |
| Example 1-10B | H-2 | D-4 |
| Example 1-11B | H-2 | D-5 |
| Example 1-12B | H-2 | D-6 |
| Comparative Example 1-1B | Compound 1 | Compound 2 |
| Comparative Example 1-2B | Compound 1 | Compound 3 |
| Comparative Example 1-3B | Compound 1 | Compound 4 |
| Comparative Example 1-4B | Compound 1 | D-1 |
| Comparative | H-1 | Compound 2 |
| Example 1-5B | | |
| Comparative Example 1-6B | H-2 | Compound 2 |

### Evaluation Example 6

The organic light-emitting devices of Examples 1-1 B to 1-12B and Comparative Examples 1-1 B to 1-6B were subjected to measure and evaluate efficiencies (@ current density of 10 mA/cm²), lifespan data (@ 50 mA/cm²), and color coordinates, by using an IVL meter (PhotoResearch PR650, Keithley 238), and the results are shown in Table 9. In Table 9, the term "efficiency" refers to a relative efficiency, and the term "lifespan" refers to a relative lifespan, among the organic light-emitting devices.

**[Table 9]**

| | Host | Dopant | Efficiency | Lifespan | Color coordinates (x,y) |
|---|---|---|---|---|---|
| Example 1-1B | H-1 | D-1 | 1.2 | 1.2 | 0.23, 0.69 |
| Example 1-2B | H-1 | D-2 | 1.2 | 1.2 | 0.24, 0.68 |
| Example 1-3B | H-1 | D-3 | 1.3 | 1.2 | 0.22, 0.69 |
| Example 1-4B | H-1 | D-4 | 1.3 | 1.3 | 0.23, 0.68 |
| Example 1-5B | H-1 | D-5 | 1.3 | 1.4 | 0.23, 0.68 |
| Example 1-6B | H-1 | D-6 | 1.2 | 1.2 | 0.22, 0.69 |
| Example 1-7B | H-2 | D-1 | 1.2 | 1.1 | 0.23, 0.69 |
| Example 1-8B | H-2 | D-2 | 1.2 | 1.2 | 0.24, 0.68 |
| Example 1-9B | H-2 | D-3 | 1.3 | 1.2 | 0.22, 0.70 |
| Example 1-10B | H-2 | D-4 | 1.2 | 1.3 | 0.23, 0.68 |
| Example 1-11B | H-2 | D-5 | 1.3 | 1.3 | 0.23, 0.68 |
| Example 1-12B | H-2 | D-6 | 1.2 | 1.2 | 0.22, 0.69 |
| Comparative Example 1-1B | Compound 1 | Compound 2 | 1.0 | 1.0 | 0.30, 0.67 |
| Comparative Example 1-2B | Compound 1 | Compound 3 | 1.1 | 1.1 | 0.26, 0.69 |
| Comparative Example 1-3B | Compound 1 | Compound 4 | 1.1 | 1.1 | 0.28, 0.66 |
| Comparative Example 1-4B | Compound 1 | D-1 | 1.1 | 1.1 | 0.23, 0.69 |
| Comparative Example 1-5B | H-1 | Compound 2 | 1.1 | 1.2 | 0.30, 0.67 |
| Comparative Example 1-6B | H-2 | Compound 2 | 1.0 | 1.1 | 0.30,0.66 |

Referring to Table 9, it was confirmed that the organic light-emitting devices of Examples 1-1 B to 1-12B had improved efficiencies and lifespans compared to those of the organic light-emitting devices of Comparative Examples 1-1 B to 1-6B.

Hereinafter, compounds used in manufacturing organic light-emitting devices manufactured in Examples 2-1 B to 2-20B and Comparative Examples 2-1 B to 2-8B are as follows:

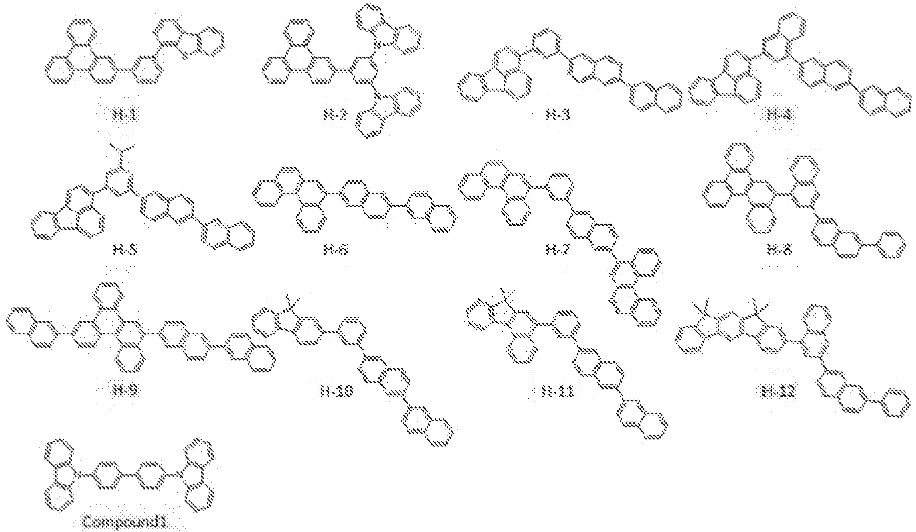

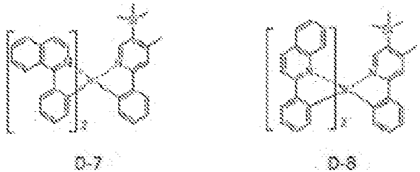

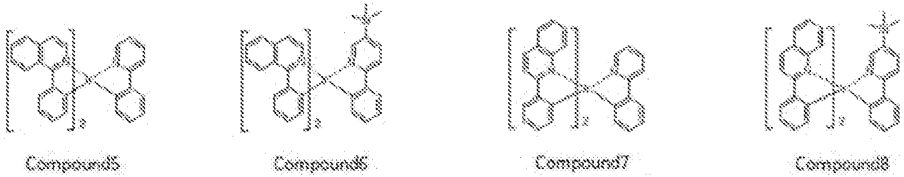

### Example 2-1B to 2-20B and Comparative Example 2-1B to 2-8B

### Glass/ITO(120 nm)/HT(120 nm)/Host:Dop_1% (30 nm)/ET1(5 nm)/ET2(25 nm)/LiF(0.5 nm)/AI(150 nm)

Organic light-emitting devices were manufactured in the same manner as in Example 1-1 B, except that in forming the EML, dopant materials listed in Table 10 were used, and the amounts of the dopants were changed to 1 v%:

**[Table 10]**

| | Host | Dopant |
|---|---|---|
| Example 2-1B | H-3 | D-7 |
| Example 2-2B | H-4 | D-7 |
| Example 2-3B | H-5 | D-7 |
| Example 2-4B | H-6 | D-7 |
| Example 2-5B | H-7 | D-7 |
| Example 2-6B | H-8 | D-7 |
| Example 2-7B | H-9 | D-7 |
| Example 2-8B | H-10 | D-7 |
| Example 2-9B | H-11 | D-7 |
| Example 2-10B | H-12 | D-7 |
| Comparative Example 2-1B | Compound 1 | Compound 5 |
| Comparative Example 2-2B | Compound 1 | Compound 6 |
| Comparative Example 2-3B | Compound 1 | D-7 |
| Comparative Example 2-4B | H-3 | Compound 5 |
| Example 2-11B | H-3 | D-8 |
| Example 2-12B | H-4 | D-8 |
| Example 2-13B | H-5 | D-8 |
| Example 2-14B | H-6 | D-8 |
| Example 2-15B | H-7 | D-8 |
| Example 2-16B | H-8 | D-8 |
| Example 2-17B | H-9 | D-8 |
| Example 2-18B | H-10 | D-8 |
| Example 2-19B | H-11 | D-8 |
| Example 2-20B | H-12 | D-8 |
| Comparative Example 2-5B | Compound 1 | Compound 7 |
| Comparative Example 2-6B | Compound 1 | Compound 8 |
| Comparative Example 2-7B | Compound 1 | D-8 |
| Comparative Example 2-8B | H-3 | Compound 7 |

### Evaluation Example 7

The organic light-emitting devices of Examples 2-1 Bto 2-20B and Comparative Examples 2-1 B to 2-8B were subjected to measure and evaluate efficiencies (@ current density of 10 mA/cm²), lifespan data (@ 50 mA/cm²), and color coordinates, by using an IVL meter (PhotoResearch PR650, Keithley 238), and the results are shown in Table 11. In Table 11, the term "efficiency" refers to a relative efficiency, and the term "lifespan" refers to a relative lifespan, among the organic light-emitting devices.

**[Table 11]**

| | Host | Dopant | Efficieny | Lifespan | Color coordinates (x, y) |
|---|---|---|---|---|---|
| Example 2-1B | H-3 | D-7 | 1.3 | 1.3 | 0.66, 0.33 |
| Example 2-2B | H-4 | D-7 | 1.2 | 1.4 | 0.66, 0.33 |
| Example 2-3B | H-5 | D-7 | 1.3 | 1.2 | 0.66, 0.34 |
| Example 2-4B | H-6 | D-7 | 1.3 | 1.3 | 0.66, 0.33 |
| Example 2-5B | H-7 | D-7 | 1.2 | 1.4 | 0.66, 0.33 |
| Example 2-6B | H-8 | D-7 | 1.3 | 1.2 | 0.66, 0.34 |
| Example 2-7B | H-9 | D-7 | 1.2 | 1.2 | 0.66, 0.33 |
| Example 2-8B | H-10 | D-7 | 1.3 | 1.3 | 0.66, 0.34 |
| Example 2-9B | H-11 | D-7 | 1.3 | 1.2 | 0.66, 0.33 |
| Example 2-10B | H-12 | D-7 | 1.3 | 1.1 | 0.66, 0.33 |
| Comparative Example 2-1B | Compound 1 | Compound 5 | 1.0 | 1.0 | 0.64, 0.34 |
| Comparative Example 2-2B | Compound 1 | Compound 6 | 1.1 | 1.0 | 0.65, 0.34 |
| Comparative Example 2-3B | Compound 1 | D-7 | 1.1 | 1.1 | 0.66, 0.33 |
| Comparative Example 2-4B | H-3 | Compound 5 | 1.1 | 1.2 | 0.64, 0.34 |
| Example 2-11B | H-3 | D-8 | 1.3 | 1.3 | 0.64, 0.34 |
| Example 2-12B | H-4 | D-8 | 1.3 | 1.3 | 0.64, 0.34 |
| Example 2-13B | H-5 | D-8 | 1.2 | 1.4 | 0.65, 0.34 |
| Example 2-14B | H-6 | D-8 | 1.3 | 1.3 | 0.64, 0.34 |
| Example 2-15B | H-7 | D-8 | 1.2 | 1.3 | 0.65, 0.34 |
| Example 2-16B | H-8 | D-8 | 1.3 | 1.2 | 0.64, 0.34 |
| Example 2-17B | H-9 | D-8 | 1.2 | 1.2 | 0.65, 0.34 |
| Example 2-18B | H-10 | D-8 | 1.3 | 1.2 | 0.64, 0.34 |
| Example 2-19B | H-11 | D-8 | 1.2 | 1.2 | 0.64, 0.34 |
| Example 2-20B | H-12 | D-8 | 1.3 | 1.1 | 0.64, 0.34 |
| Comparative Example 2-5B | Compound 1 | Compound 7 | 1.0 | 1.0 | 0.62, 0.35 |
| Comparative Example 2-6B | Compound 1 | Compound 8 | 1.1 | 1.0 | 0.63, 0.34 |
| Comparative Example 2-7B | Compound 1 | D-8 | 1.1 | 1.1 | 0.64, 0.34 |
| Comparative Example 2-8B | H-3 | Compound 7 | 1.1 | 1.2 | 0.62, 0.35 |

Referring to Table 11, it was confirmed that the organic light-emitting devices of Examples 2-1 B to 2-20B had improved efficiencies and lifespans compared to those of the organic light-emitting devices of Comparative Examples 2-1 B to 2-8B.

Hereinafter, compounds used in manufacturing organic light-emitting devices manufactured in Examples 3-1 B to 3-7B and Comparative Examples 3-1 B to 3-10B are as follows:

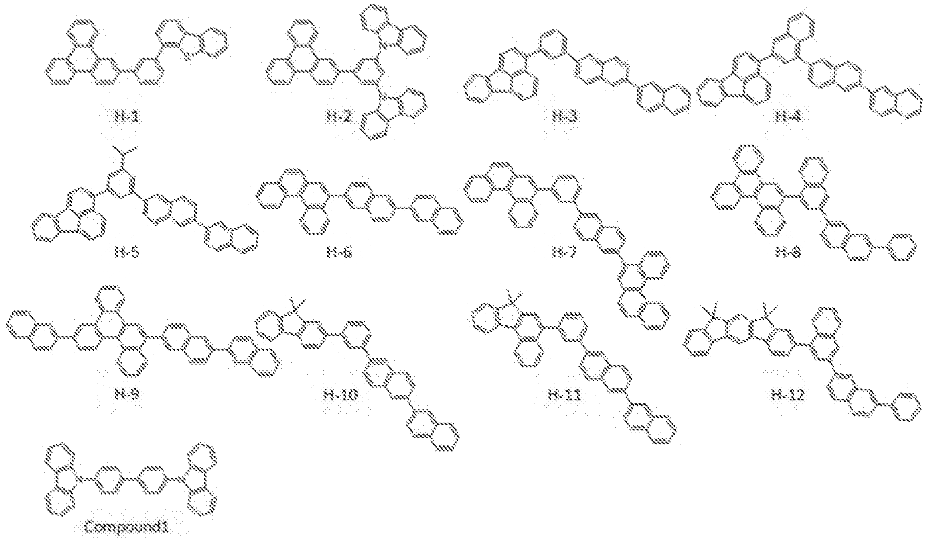

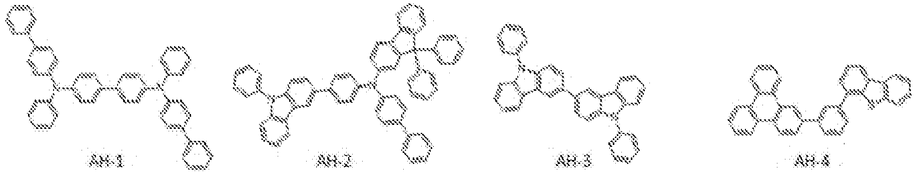

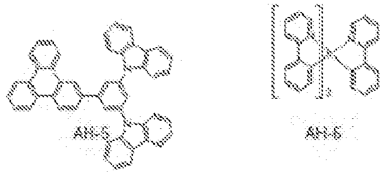

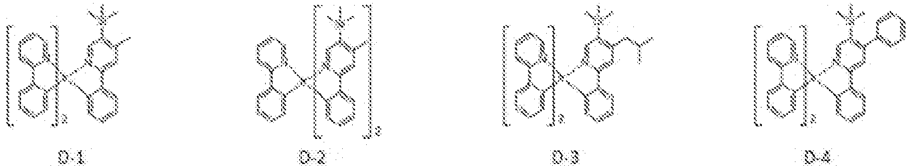

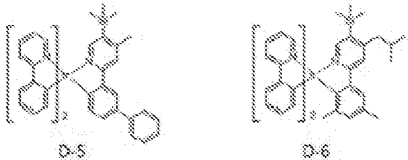

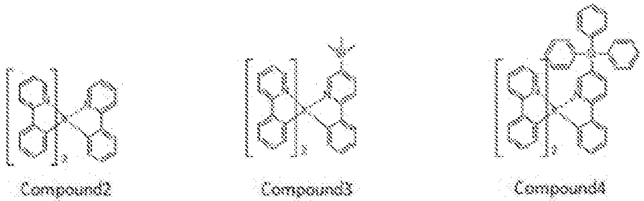

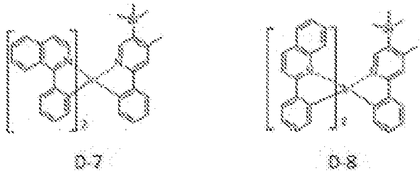

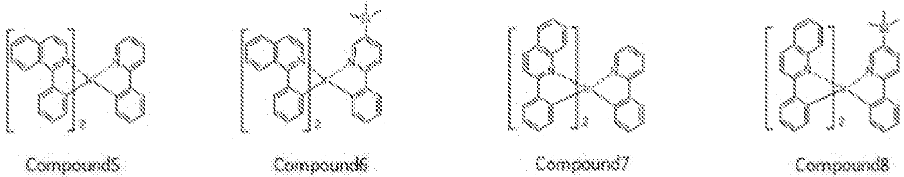

### Example 3-1B to 3-7B and Comparative Example 3-1B to 3-10B

### Glass/ITO(120)/HT(120)/Host1:Host2_10%:Dop_x%(30)/ET1(5)/ET2(25)/LiF(0.5)/Al(150)

Organic light-emitting devices were manufactured in the same manner as in 1-1 B, except that in forming the EML, host materials listed in Table 6 were used as the first host and the second host (wherein 10 v% of the second host was used), the amounts of the dopants were changed to amounts shown in Table 6, and dopant materials listed in Table 12 were used.

**[Table 12]**

| | First host | Second host | Dopant | Dopant (v%) |
|---|---|---|---|---|
| Example 3-1B | H-1 | AH-3 | D-1 | 7 |
| Example 3-2B | H-1 | AH-4 | D-1 | 7 |
| Example 3-3B | H-1 | AH-5 | D-1 | 7 |
| Comparative Example 3-1B | Compound 1 | - | Compound 2 | 7 |
| Comparative Example 3-2B | Compound 1 | - | Compound 3 | 7 |
| Comparative Example 3-3B | Compound 1 | - | D-1 | 7 |
| Comparative Example 3-4B | H-1 | - | Compound 2 | 7 |
| Comparative Example 3-5B | Compound 1 | AH-3 | Compound 2 | 7 |
| Example 3-4B | H-3 | AH-1 | D-7 | 1 |
| Example 3-5B | H-3 | AH-2 | D-7 | 1 |
| Example 3-6B | H-3 | AH-3 | D-7 | 1 |
| Example 3-7B | H-3 | AH-6 | D-7 | 1 |
| Comparative Example 3-6B | Compound 1 | - | Compound 5 | 1 |
| Comparative Example 3-7B | Compound 1 | - | Compound 6 | 1 |
| Comparative Example 3-8B | Compound 1 | - | D-7 | 1 |
| Comparative Example 3-9B | H-3 | - | Compound 5 | 1 |
| Comparative Example 3-10B | Compound 1 | AH-1 | Compound 5 | 1 |

### Evaluation Example 8

The organic light-emitting devices of Examples 3-1 B to 3-7B and Comparative Examples 3-1B to 3-10Bwere subjected to measure and evaluate efficiencies (@ current density of 10 mA/cm²), lifespan data (@ 50 mA/cm²), and color coordinates, by using an IVL meter (PhotoResearch PR650, Keithley 238), and the results are shown in Table 13. In Table 13, the term "efficiency" refers to a relative efficiency, and the term "lifespan" refers to a relative lifespan, among the organic light-emitting devices.

**[Table 13]**

| | First host | Second host | Dopant | Dopant (v%) | Efficieny | Lifespan | Color coordinates (x, y) |
|---|---|---|---|---|---|---|---|
| Example 3-1B | H-1 | AH-3 | D-1 | 7 | 1.2 | 1.3 | 0.23, 0.69 |
| Example 3-2B | H-1 | AH-4 | D-1 | 7 | 1.3 | 1.4 | 0.23, 0.69 |
| Example 3-3B | H-1 | AH-5 | D-1 | 7 | 1.3 | 1.5 | 0.23, 0.69 |
| Comparative Example 3-1B | Compound 1 | - | Compound 2 | 7 | 1.0 | 1.0 | 0.30, 0.67 |
| Comparative Example 3-2B | Compound 1 | - | Compound 3 | 7 | 1.1 | 1.1 | 0.26, 0.69 |
| Comparative Example 3-3B | Compound 1 | - | D-1 | 7 | 1.1 | 1.1 | 0.23, 0.69 |
| Comparative Example 3-4B | H-1 | - | Compound 2 | 7 | 1.1 | 1.2 | 0.30, 0.67 |
| Comparative Example 3-5B | Compound 1 | AH-3 | Compound 2 | 7 | 1.0 | 1.3 | 0.30, 0.67 |
| Example 3-4B | H-3 | AH-1 | D-7 | 1 | 1.2 | 1.3 | 0.66, 0.34 |
| Example 3-5B | H-3 | AH-2 | D-7 | 1 | 1.3 | 1.4 | 0.66, 0.33 |
| Example 3-6B | H-3 | AH-3 | D-7 | 1 | 1.3 | 1.3 | 0.66, 0.33 |
| Example 3-7B | H-3 | AH-6 | D-7 | 1 | 1.3 | 1.4 | 0.66, 0.33 |
| Comparative Example 3-6B | Compound 1 | - | Compound 5 | 1 | 1.0 | 1.0 | 0.64, 0.34 |
| Comparative Example 3-7B | Compound 1 | - | Compound 6 | 1 | 1.1 | 1.0 | 0.65, 0.34 |
| Comparative Example 3-8B | Compound 1 | - | D-7 | 1 | 1.1 | 1.1 | 0.66, 0.33 |
| Comparative Example 3-9B | H-3 | - | Compound 5 | 1 | 1.1 | 1.2 | 0.64, 0.34 |
| Comparative Example 3-10B | Compound 1 | AH-1 | Compound 5 | 1 | 1.1 | 1.2 | 0.64, 0.34 |

Referring to Table 13, it was confirmed that the organic light-emitting devices of Exampless 3-1 B to 3-7B had improved efficiencies and lifespans compared to those of the organic light-emitting devices of Comparative Examples 3-1 B to 3-10B.

Hereinafter, compounds used in manufacturing organic light-emitting devices manufactured in Examples 1-1C to 1-30C and Comparative Examples 1-1C to 1-5C are as follows:

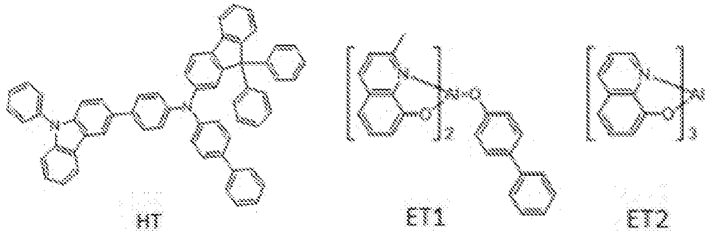

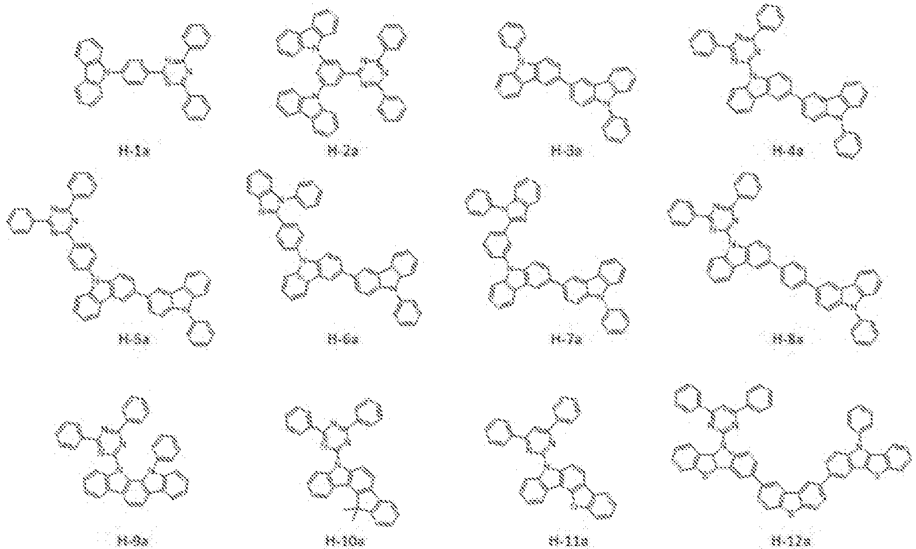

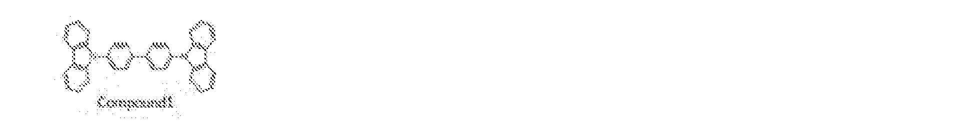

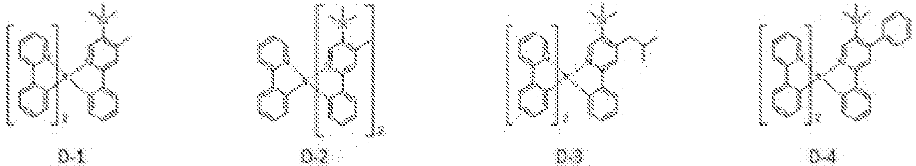

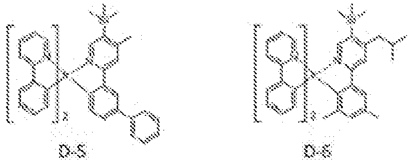

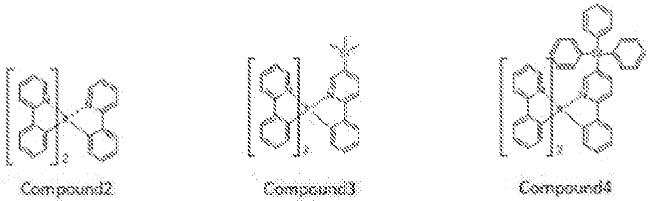

### Example 1-1C

### Glass/ITO(120)/HT(120)/Host1:Host2_10%:Dop_7%(30)/ET1(5)/ET2(25)/LiF(0.5)/Al(150)

As an anode, a 15 Ω/cm² (1,200 Å) ITO glass substrate (manufactured by Corning company) was cut into a size of 50 mm x 50 mm x 0.7 mm and ultrasonically washed with isopropyl alcohol and pure water, each for 5 minutes. Afterwards, the ITO glass substrate was irradiated by UV light for 30 minutes, cleaned by exposure to ozone, and then, mounted on a vacuum depositor.

Compound HT was vacuum-deposited on the substrate to form a hole transport region having a thickness of 120 nm.

Compound H-1a, H3-a (10 v%), and D-1 (7v%) were co-deposited on the hole transport region to form an EML having a thickness of 30 nm.

Compound ET1 was deposited on the EML to form a buffer layer having a thickness of 5 nm, and Compound ET2 was deposited on the buffer layer to form an electron transport layer (ETL) having a thickness of 25 nm. LiF was deposited on the ETL to form an electron injection layer (EIL) having a thickness of 0.5 nm, thereby preparing an electron transport region.

Al was deposited on the electron transport region to a thickness of 150 nm, thereby manufacturing an organic light-emitting device.

### Examples 1-2C to 1-30C and Comparative Examples 1-1C to 1-5C

Organic light-emitting devices were manufactured in the same manner as in Example 1-1C, except that in forming the EML, first host, second host, and dopant materials shown in Table 14 were used:

**[Table 14]**

| | First host | Second host | Dopant |
|---|---|---|---|
| Example 1-1C | H-1a | H-3a | D-1 |
| Example 1-2C | H-1a | H-3a | D-2 |
| Example 1-3C | H-1a | H-3a | D-3 |
| Example 1-4C | H-1a | H-3a | D-4 |
| Example 1-5C | H-1a | H-3a | D-5 |
| Example 1-6C | H-1a | H-3a | D-6 |
| Example 1-7C | H-2a | H-3a | D-1 |
| Example 1-8C | H-2a | H-3a | D-3 |
| Example 1-9C | H-2a | H-3a | D-5 |
| Example 1-10C | H-4a | H-3a | D-1 |
| Example 1-11C | H-4a | H-3a | D-3 |
| Example 1-12C | H-4a | H-3a | D-5 |
| Example 1-13C | H-5a | H-3a | D-1 |
| Example 1-14C | H-5a | H-3a | D-3 |
| Example 1-15C | H-5a | H-3a | D-5 |
| Example 1-16C | H-8a | H-3a | D-1 |
| Example 1-17C | H-8a | H-3a | D-3 |
| Example 1-18C | H-8a | H-3a | D-5 |
| Example 1-19C | H-9a | H-3a | D-1 |
| Example 1-20C | H-9a | H-3a | D-3 |
| Example 1-21C | H-9a | H-3a | D-5 |
| Example 1-22C | H-10a | H-3a | D-1 |
| Example 1-23C | H-10a | H-3a | D-3 |
| Example 1-24C | H-10a | H-3a | D-5 |
| Example 1-25C | H-11a | H-3a | D-1 |
| Example 1-26C | H-11a | H-3a | D-3 |
| Example 1-27C | H-11a | H-3a | D-5 |
| Example 1-28C | H-12a | H-3a | D-1 |
| Example 1-29C | H-12a | H-3a | D-3 |
| Example 1-30C | H-12a | H-3a | D-5 |
| Comparative Example 1-1C | Compound 1 | - | Compound 2 |
| Comparative Example 1-2C | Compound 1 | - | Compound 3 |
| Comparative Example 1-3C | Compound 1 | - | D-1 |
| Comparative Example 1-4C | H-1a | - | Compound 2 |
| Comparative Example 1-5C | Compound 1 | H-3a | Compound 2 |

### Evaluation Example 9

The organic light-emitting devices of Examples 1-1C to 1-30C and Comparative Examples 1-1C to 1-5Cwere subjected to measure and evaluate efficiencies (@ current density of 10 mA/cm²), lifespan data (@ 50 mA/cm²), and color coordinates, by using an IVL meter (PhotoResearch PR650, Keithley 238), and the results are shown in Table 15. In Table 15, the term "efficiency" refers to a relative efficiency, and the term "lifespan" refers to a relative lifespan, among the organic light-emitting devices.

**[Table 15]**

| | First host | Second host | Dopant | Efficiency | Lifespan | Color coordinates (x,y) |
|---|---|---|---|---|---|---|
| Example 1-1C | H-1a | H-3a | D-1 | 1.1 | 1.2 | 0.23, 0.69 |
| Example 1-2C | H-1a | H-3a | D-2 | 1.2 | 1.2 | 0.24, 0.68 |
| Example 1-3C | H-1a | H-3a | D-3 | 1.2 | 1.3 | 0.22, 0.69 |
| Example 1-4C | H-1a | H-3a | D-4 | 1.2 | 1.2 | 0.23, 0.68 |
| Example 1-5C | H-1a | H-3a | D-5 | 1.2 | 1.3 | 0.23, 0.68 |
| Example 1-6C | H-1a | H-3a | D-6 | 1.2 | 1.2 | 0.22, 0.69 |
| Example 1-7C | H-2a | H-3a | D-1 | 1.2 | 1.3 | 0.23, 0.69 |
| Example 1-8C | H-2a | H-3a | D-3 | 1.3 | 1.3 | 0.22, 0.69 |
| Example 1-9C | H-2a | H-3a | D-5 | 1.2 | 1.3 | 0.23, 0.68 |
| Example 1-10C | H-4a | H-3a | D-1 | 1.4 | 1.4 | 0.23, 0.69 |
| Example 1-11C | H-4a | H-3a | D-3 | 1.3 | 1.5 | 0.22, 0.69 |
| Example 1-12C | H-4a | H-3a | D-5 | 1.3 | 1.4 | 0.23, 0.68 |
| Example 1-13C | H-5a | H-3a | D-1 | 1.4 | 1.3 | 0.23, 0.69 |
| Example 1-14C | H-5a | H-3a | D-3 | 1.3 | 1.4 | 0.22, 0.69 |
| Example 1-15C | H-5a | H-3a | D-5 | 1.3 | 1.4 | 0.23, 0.68 |
| Example 1-16C | H-8a | H-3a | D-1 | 1.4 | 1.3 | 0.23, 0.69 |
| Example 1-17C | H-8a | H-3a | D-3 | 1.3 | 1.3 | 0.22, 0.69 |
| Example 1-18C | H-8a | H-3a | D-5 | 1.3 | 1.4 | 0.23, 0.68 |
| Example 1-19C | H-9a | H-3a | D-1 | 1.3 | 1.4 | 0.23, 0.69 |
| Example 1-20C | H-9a | H-3a | D-3 | 1.3 | 1.3 | 0.22, 0.69 |
| Example 1-21C | H-9a | H-3a | D-5 | 1.3 | 1.4 | 0.23, 0.68 |
| Example 1-22C | H-10a | H-3a | D-1 | 1.2 | 1.3 | 0.23, 0.69 |
| Example 1-23C | H-10a | H-3a | D-3 | 1.3 | 1.2 | 0.22, 0.69 |
| Example 1-24C | H-10a | H-3a | D-5 | 1.3 | 1.3 | 0.23, 0.68 |
| Example 1-25C | H-11a | H-3a | D-1 | 1.3 | 1.3 | 0.23, 0.69 |
| Example 1-26C | H-11a | H-3a | D-3 | 1.2 | 1.3 | 0.22, 0.69 |
| Example 1-27C | H-11a | H-3a | D-5 | 1.3 | 1.4 | 0.23, 0.68 |
| Example 1-28C | H-12a | H-3a | D-1 | 1.1 | 1.3 | 0.23, 0.69 |
| Example 1-29C | H-12a | H-3a | D-3 | 1.1 | 1.3 | 0.22, 0.69 |
| Example 1-30C | H-12a | H-3a | D-5 | 1.2 | 1.2 | 0.23, 0.68 |
| Comparative Example 1-1C | Compound 1 | - | Compound 2 | 1.0 | 1.0 | 0.30, 0.67 |
| Comparative Example 1-2C | Compound 1 | - | Compound 3 | 1.1 | 1.1 | 0.26, 0.69 |
| Comparative Example 1-3C | Compound 1 | - | D-1 | 1.1 | 1.1 | 0.23, 0.69 |
| Comparative Example 1-4C | H-1a | - | Compound 2 | 1.1 | 1.2 | 0.30, 0.67 |
| Comparative Example 1-5C | Compound 1 | H-3a | Compound 2 | 1.0 | 1.2 | 0.30, 0.67 |

Referring to Table 15, it was confirmed that the organic light-emitting devices of Examples 1-1C to 1-30C had improved efficiencies and lifespans compared to those of the organic light-emitting devices of Comparative Examples 1-1C to 1-5C.

Hereinafter, compounds used in manufacturing organic light-emitting devices manufactured in Examples 2-1C to 2-8C and Comparative Examples 2-1C to 2-10C are as follows:

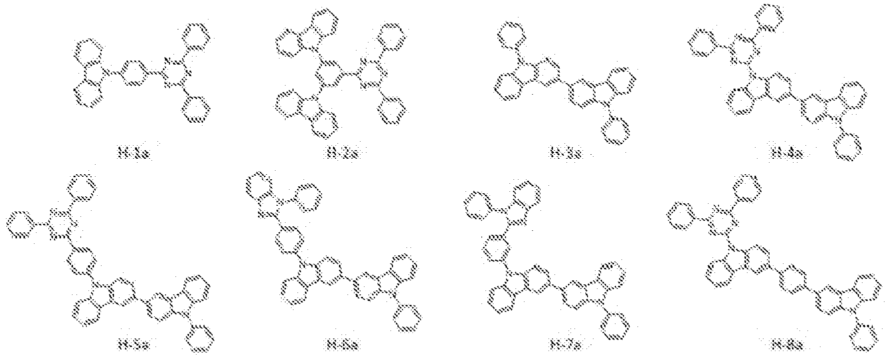

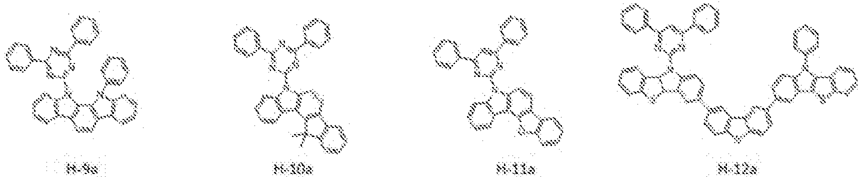

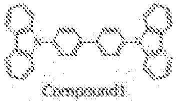

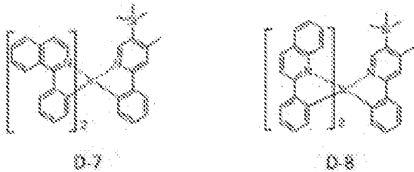

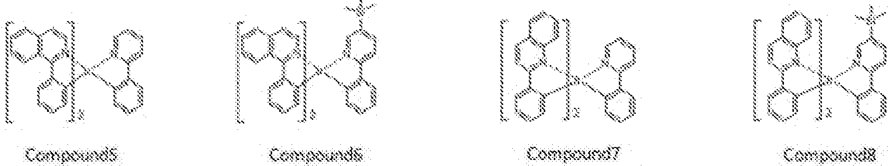

### Examples 2-1C to 2-8C and Comparative Examples 2-1C to 2-10C

### Glass/ITO(120)/HT(120)/Host1:Host2_10%:Dop_1%(30)/ET1(5)/ET2(25)/LiF(0.5)/Al(150)

Organic light-emitting devices were manufactured in the same manner as in Example 1-1C, except that in forming the EML, compounds listed in Table 16 were used as the first hosts, the second hosts, and the dopants, and the amounts of the dopants were changed to 1 v%.

**[Table 16]**

| | First host | Second host | Dopant |
|---|---|---|---|
| Example 2-1C | H-1a | H-4a | D-7 |
| Example 2-2C | H-5a | H-4a | D-7 |
| Example 2-3C | H-6a | H-4a | D-7 |
| Example 2-4C | H-7a | H-4a | D-7 |
| Comparative Example 2-1C | Compound 1 | - | Compound 5 |
| Comparative Example 2-2C | Compound 1 | - | Compound 6 |
| Comparative Example 2-3C | Compound 1 | - | D-7 |
| Comparative Example 2-4C | H-1a | - | Compound 5 |
| Comparative Example 2-5C | Compound 1 | H-4a | Compound 5 |
| Example 2-5C | H-1a | H-4a | D-8 |
| Example 2-6C | H-5a | H-4a | D-8 |
| Example 2-7C | H-6a | H-4a | D-8 |
| Example 2-8C | H-7a | H-4a | D-8 |
| Comparative Example 2-6C | Compound 1 | - | Compound 7 |
| Comparative Example 2-7C | Compound 1 | - | Compound 8 |
| Comparative Example 2-8C | Compound 1 | - | D-7 |
| Comparative Example 2-9C | H-1a | - | Compound 7 |
| Comparative Example 2-10C | Compound 1 | H-4a | Compound 7 |

### Evaluation Example 10

The organic light-emitting devices of Examples 2-1C to 2-8C and Comparative Examples 2-1C to 2-10C were subjected to measure and evaluate efficiencies (@ current density of 10 mA/cm²), lifespan data (@ 50 mA/cm²), and color coordinates, by using an IVL meter (PhotoResearch PR650, Keithley 238), and the results are shown in Table 17. In Table 17, the term "efficiency" refers to a relative efficiency, and the term "lifespan" refers to a relative lifespan, among the organic light-emitting devices.

**[Table 17]**

| | First host | Second host | Dopant | Efficiency | Lifespan | Color coordinates (x,y) |
|---|---|---|---|---|---|---|
| Example 2-1C | H-1a | H-4a | D-7 | 1.2 | 1.2 | 0.66, 0.33 |
| Example 2-2C | H-5a | H-4a | D-7 | 1.2 | 1.3 | 0.66, 0.33 |
| Example 2-3C | H-6a | H-4a | D-7 | 1.2 | 1.4 | 0.66, 0.33 |
| Example 2-4C | H-7a | H-4a | D-7 | 1.2 | 1.4 | 0.66, 0.33 |
| Comparative Example 2-1C | Compound 1 | - | Compound 5 | 1.0 | 1.0 | 0.64, 0.34 |
| Comparative Example 2-2C | Compound 1 | - | Compound 6 | 1.1 | 1.0 | 0.65, 0.34 |
| Comparative Example 2-3C | Compound 1 | - | D-7 | 1.1 | 1.1 | 0.66, 0.33 |
| Comparative Example 2-4C | H-1a | - | Compound 5 | 1.1 | 1.2 | 0.64, 0.34 |
| Comparative Example 2-5C | Compound 1 | H-4a | Compound 5 | 1.0 | 1.2 | 0.64, 0.34 |
| Example 2-5C | H-1a | H-4a | D-8 | 1.2 | 1.2 | 0.64, 0.34 |
| Example 2-6C | H-5a | H-4a | D-8 | 1.3 | 1.2 | 0.64, 0.34 |
| Example 2-7C | H-6a | H-4a | D-8 | 1.3 | 1.3 | 0.64, 0.34 |
| Example 2-8C | H-7a | H-4a | D-8 | 1.2 | 1.3 | 0.64, 0.34 |
| Comparative Example 2-6C | Compound 1 | - | Compound 7 | 1.0 | 1.0 | 0.62, 0.35 |
| Comparative Example 2-7C | Compound 1 | - | Compound 8 | 1.1 | 1.0 | 0.63, 0.34 |
| Comparative Example 2-8C | Compound 1 | - | D-7 | 1.1 | 1.1 | 0.64, 0.34 |
| Comparative Example 2-9C | H-1a | - | Compound 7 | 1.1 | 1.1 | 0.62, 0.35 |
| Comparative Example 2-10C | Compound 1 | H-4a | Compound 7 | 1.0 | 1.2 | 0.62, 0.35 |

Referring to Table 17, it was confirmed that the organic light-emitting devices of Examples 2-1C to 2-8C had improved efficiencies and lifespans compared to those of the organic light-emitting devices of Comparative Examples 2-1C to 2-10C.

Hereinafter, compounds used in manufacturing organic light-emitting devices manufactured in Examples 3-1C to 3-27C and Comparative Examples 3-1C to 3-5C are as follows:

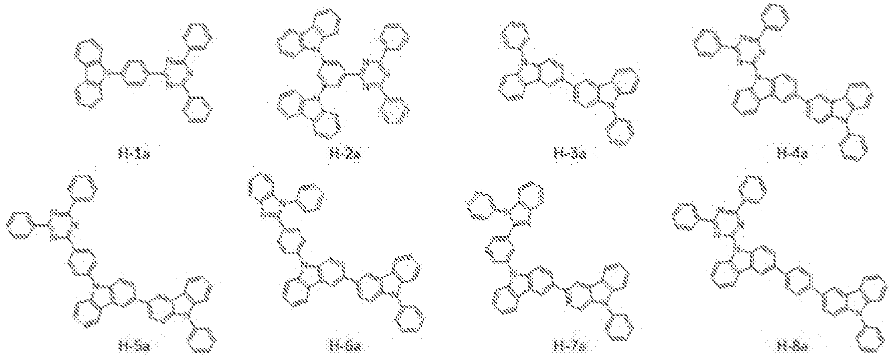

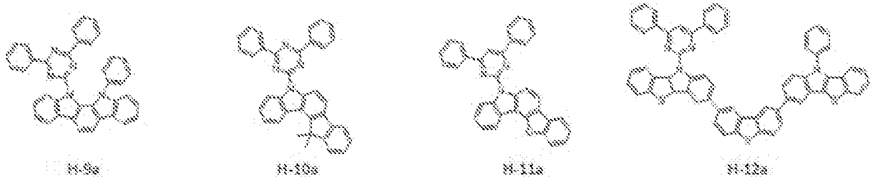

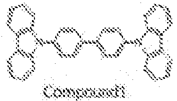

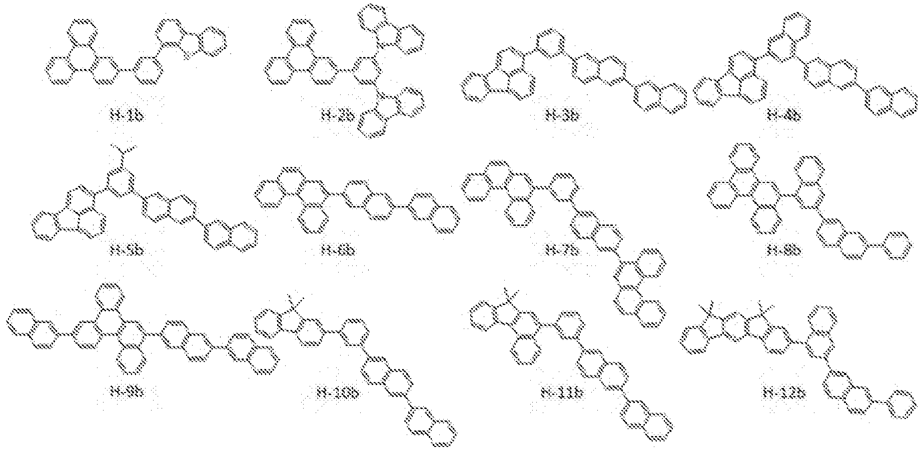

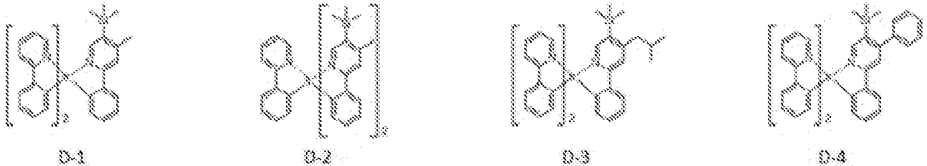

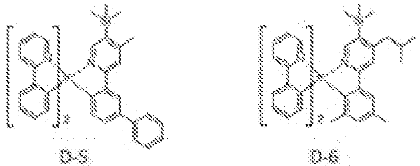

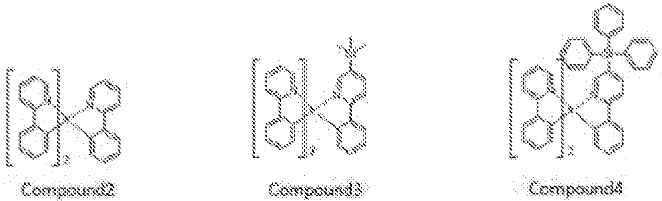

### Examples 3-1C to 3-27C and Comparative Examples 3-1C to 3-5C

### Glass/ITO(120)/HT(120)/Host1:Host2_10%:Dop_7%(30)/ET1(5)/ET2(25)/LiF(0.5)/Al(150)

Organic light-emitting devices were manufactured in the same manner as in Example 1-1C, except that in forming the EML, compunds listed in Table 18 were used as the first hosts, the second hosts, and the dopants:

**[Table 18]**

| | First host | Second host | Dopant |
|---|---|---|---|
| Example 3-1C | H-1b | H-4a | D-1 |
| Example 3-2C | H-1b | H-4a | D-2 |
| Example 3-3C | H-1b | H-4a | D-3 |
| Example 3-4C | H-1b | H-4a | D-4 |
| Example 3-5C | H-1b | H-4a | D-5 |
| Example 3-6C | H-1b | H-4a | D-6 |
| Example 3-7C | H-1b | H-5a | D-1 |
| Example 3-8C | H-1b | H-5a | D-3 |
| Example 3-9C | H-1b | H-5a | D-5 |
| Example 3-10C | H-1b | H-9a | D-1 |
| Example 3-11C | H-1b | H-9a | D-3 |
| Example 3-12C | H-1b | H-9a | D-5 |
| Example 3-13C | H-1b | H-10a | D-1 |
| Example 3-14C | H-1b | H-10a | D-3 |
| Example 3-15C | H-1b | H-10a | D-5 |
| Example 3-16C | H-1b | H-11a | D-1 |
| Example 3-17C | H-1b | H-11a | D-3 |
| Example 3-18C | H-1b | H-11a | D-5 |
| Example 3-19C | H-1b | H-12a | D-1 |
| Example 3-20C | H-1b | H-12a | D-3 |
| Example 3-21C | H-1b | H-12a | D-5 |
| Example 3-22C | H-2b | H-4a | D-1 |
| Example 3-23C | H-2b | H-4a | D-3 |
| Example 3-24C | H-2b | H-4a | D-5 |
| Example 3-25C | H-2b | H-9a | D-1 |
| Example 3-26C | H-2b | H-9a | D-3 |
| Example 3-27C | H-2b | H-9a | D-5 |
| Comparative Example 3-1C | Compound 1 | - | Compound 2 |
| Comparative Example 3-2C | Compound 1 | - | Compound 3 |
| Comparative Example 3-3C | Compound 1 | - | D-1 |
| Comparative Example 3-4C | H-1a | - | Compound 2 |
| Comparative Example 3-5C | Compound 1 | H-3a | Compound 2 |

### Evaluation Example 11

The organic light-emitting devices of Examples 3-1C to 3-27C and Comparative Examples 3-1C to 3-5C were subjected to measure and evaluate efficiencies (@ current density of 10 mA/cm²), lifespan data (@ 50 mA/cm²), and color coordinates, by using an IVL meter (PhotoResearch PR650, Keithley 238), and the results are shown in Table 19. In Table 19, the term "efficiency" refers to a relative efficiency, and the term "lifespan" refers to a relative lifespan, among the organic light-emitting devices.

**[Table 19]**

| | First host | Second host | Dopant | Efficiency | Lifespan | Color coordinates (x,y) |
|---|---|---|---|---|---|---|
| Example 3-1C | H-1b | H-4a | D-1 | 1.3 | 1.3 | 0.23, 0.69 |
| Example 3-2C | H-1b | H-4a | D-2 | 1.3 | 1.4 | 0.24, 0.68 |
| Example 3-3C | H-1b | H-4a | D-3 | 1.2 | 1.4 | 0.22, 0.69 |
| Example 3-4C | H-1b | H-4a | D-4 | 1.3 | 1.3 | 0.23, 0.68 |
| Example 3-5C | H-1b | H-4a | D-5 | 1.3 | 1.4 | 0.23, 0.68 |
| Example 3-6C | H-1b | H-4a | D-6 | 1.2 | 1.3 | 0.22, 0.69 |
| Example 3-7C | H-1b | H-5a | D-1 | 1.3 | 1.4 | 0.23, 0.69 |
| Example 3-8C | H-1b | H-5a | D-3 | 1.4 | 1.4 | 0.22, 0.69 |
| Example 3-9C | H-1b | H-5a | D-5 | 1.3 | 1.5 | 0.23, 0.68 |
| Example 3-10C | H-1b | H-9a | D-1 | 1.3 | 1.4 | 0.23, 0.69 |
| Example 3-11C | H-1b | H-9a | D-3 | 1.2 | 1.3 | 0.22, 0.69 |
| Example 3-12C | H-1b | H-9a | D-5 | 1.3 | 1.4 | 0.23, 0.68 |
| Example 3-13C | H-1b | H-10a | D-1 | 1.3 | 1.3 | 0.23, 0.69 |
| Example 3-14C | H-1b | H-10a | D-3 | 1.3 | 1.2 | 0.22, 0.69 |
| Example 3-15C | H-1b | H-10a | D-5 | 1.4 | 1.3 | 0.23, 0.68 |
| Example 3-16C | H-1b | H-11a | D-1 | 1.2 | 1.4 | 0.23, 0.69 |
| Example 3-17C | H-1b | H-11a | D-3 | 1.2 | 1.3 | 0.22, 0.69 |
| Example 3-18C | H-1b | H-11a | D-5 | 1.3 | 1.4 | 0.23, 0.68 |
| Example 3-19C | H-1b | H-12a | D-1 | 1.2 | 1.3 | 0.23, 0.69 |
| Example 3-20C | H-1b | H-12a | D-3 | 1.1 | 1.2 | 0.22, 0.69 |
| Example 3-21C | H-1b | H-12a | D-5 | 1.2 | 1.3 | 0.23, 0.68 |
| Example 3-22C | H-2b | H-4a | D-1 | 1.2 | 1.2 | 0.23, 0.69 |
| Example 3-23C | H-2b | H-4a | D-3 | 1.1 | 1.3 | 0.22, 0.69 |
| Example 3-24C | H-2b | H-4a | D-5 | 1.2 | 1.3 | 0.23, 0.68 |
| Example 3-25C | H-2b | H-9a | D-1 | 1.2 | 1.2 | 0.23, 0.69 |
| Example 3-26C | H-2b | H-9a | D-3 | 1.2 | 1.2 | 0.22, 0.69 |
| Example 3-27C | H-2b | H-9a | D-5 | 1.3 | 1.2 | 0.23, 0.68 |
| Comparative Example 3-1C | Compound 1 | - | Compound 2 | 1.0 | 1.0 | 0.30, 0.67 |
| Comparative Example 3-2C | Compound 1 | - | Compound 3 | 1.1 | 1.1 | 0.26, 0.69 |
| Comparative Example 3-3C | Compound 1 | - | D-1 | 1.1 | 1.1 | 0.23, 0.69 |
| Comparative Example 3-4C | H-1a | - | Compound 2 | 1.1 | 1.2 | 0.30, 0.67 |
| Comparative Example 3-5C | Compound 1 | H-3a | Compound 2 | 1.0 | 1.2 | 0.30, 0.67 |

Referring to Table 19, it was confirmed that the organic light-emitting devices of Examples 3-1C to 3-27C had improved efficiencies and lifespans compared to those of the organic light-emitting devices of Comparative Examples 3-1C to 3-5C.

Hereinafter, compounds used in manufacturing organic light-emitting devices manufactured in Examples 4-1C to 4-13C and Comparative Examples 4-1C to 4-10C are as follows:

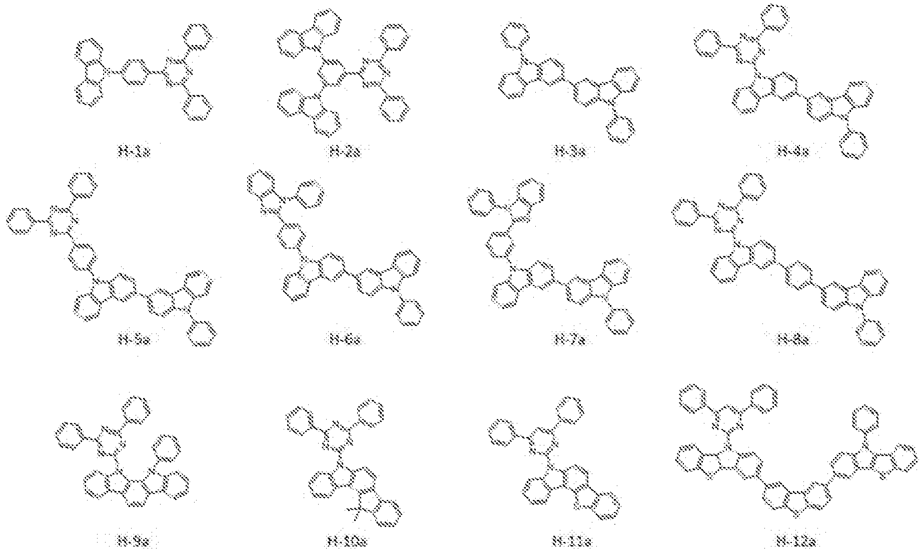

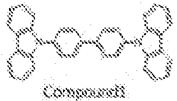

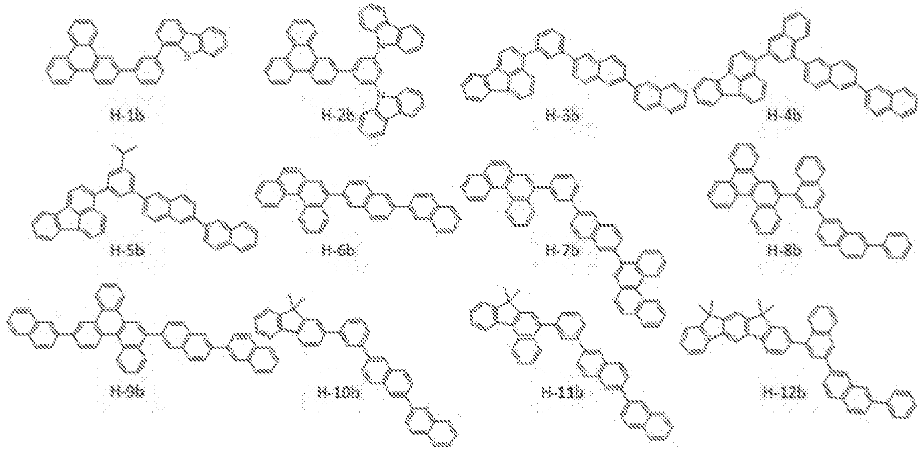

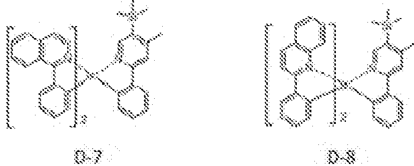

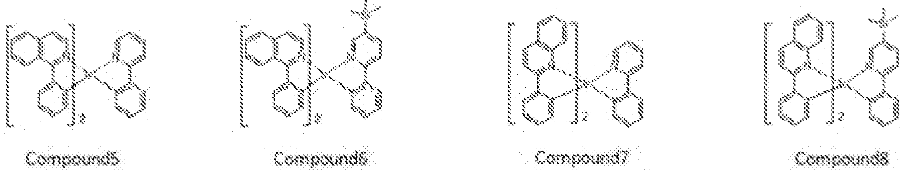

### Example 4-1C to 4-13C and Comparative Example 4-1C to 4-10C

### Glass/ITO(120)/HT(120)/Host1:Host2_10%:Dop_1%(30)/ET1(5)/ET2(25)/LiF(0.5)/AI(150)

Organic light-emitting devices were manufactured in the same manner as in Example 1-1C, except that in forming the EML, compounds listed in Table 20 were used as the first hosts, the second hosts, and the dopants, and the amounts of the dopants were changed to 1 v%.

**[Table 20]**

| | First host | Second host | Dopant |
|---|---|---|---|
| Example 4-1C | H-3b | H-3a | D-7 |
| Example 4-2C | H-4b | H-3a | D-7 |
| Example 4-3C | H-5b | H-3a | D-7 |
| Example 4-4C | H-6b | H-3a | D-7 |
| Example 4-5C | H-7b | H-3a | D-7 |
| Example 4-6C | H-8b | H-3a | D-7 |
| Example 4-7C | H-9b | H-3a | D-7 |
| Example 4-5C | H-10b | H-3a | D-7 |
| Example 4-8C | H-11b | H-3a | D-7 |
| Example 4-9C | H-12b | H-3a | D-7 |
| Comparative Example 4-1C | Compound 1 | - | Compound 5 |
| Comparative Example 4-2C | Compound 1 | - | Compound 6 |
| Comparative Example 4-3C | Compound 1 | - | D-7 |
| Comparative Example 4-4C | H-1a | - | Compound 5 |
| Comparative Example 4-5C | Compound 1 | H-4a | Compound 5 |
| Example 4-10C | H-3b | H-3a | D-8 |
| Example 4-11C | H-6b | H-3a | D-8 |
| Example 4-12C | H-8b | H-3a | D-8 |
| Example 4-13C | H-11b | H-3a | D-8 |
| Comparative Example 4-6C | Compound 1 | - | Compound 7 |
| Comparative Example 4-7C | Compound 1 | - | Compound 8 |
| Comparative Example 4-8C | Compound 1 | - | D-7 |
| Comparative Example 4-9C | H-1a | - | Compound 7 |
| Comparative Example 4-10C | Compound 1 | H-4a | Compound 7 |

### Evaluation Example 12

The organic light-emitting devices of Examples 4-1C to 4-13C and Comparative Examples 4-1C to 4-10C were subjected to measure and evaluate efficiencies (@ current density of 10 mA/cm²), lifespan data (@ 50 mA/cm²), and color coordinates, by using an IVL meter (PhotoResearch PR650, Keithley 238), and the results are shown in Table 21. In Table 21, the term "efficiency" refers to a relative efficiency, and the term "lifespan" refers to a relative lifespan, among the organic light-emitting devices.

**[Table 21]**

| | First host | Second host | Dopant | Efficiency | Lifespan | Color coordinates (x,y) |
|---|---|---|---|---|---|---|
| Example 4-1C | H-3b | H-3a | D-7 | 1.3 | 1.4 | 0.66, 0.33 |
| Example 4-2C | H-4b | H-3a | D-7 | 1.2 | 1.3 | 0.66, 0.33 |
| Example 4-3C | H-5b | H-3a | D-7 | 1.3 | 1.2 | 0.66, 0.34 |
| Example 4-4C | H-6b | H-3a | D-7 | 1.3 | 1.3 | 0.66, 0.33 |
| Example 4-5C | H-7b | H-3a | D-7 | 1.2 | 1.4 | 0.66, 0.33 |
| Example 4-6C | H-8b | H-3a | D-7 | 1.3 | 1.3 | 0.66, 0.34 |
| Example 4-7C | H-9b | H-3a | D-7 | 1.2 | 1.3 | 0.66, 0.33 |
| Example 4-5C | H-10b | H-3a | D-7 | 1.3 | 1.3 | 0.66, 0.34 |
| Example 4-8C | H-11b | H-3a | D-7 | 1.3 | 1.2 | 0.66, 0.33 |
| Example 4-9C | H-12b | H-3a | D-7 | 1.3 | 1.2 | 0.66, 0.33 |
| Comparative Example 4-1C | Compound 1 | - | Compound 5 | 1.0 | 1.0 | 0.64, 0.34 |
| Comparative Example 4-2C | Compound 1 | - | Compound 6 | 1.1 | 1.0 | 0.65, 0.34 |
| Comparative Example 4-3C | Compound 1 | - | D-7 | 1.1 | 1.1 | 0.66, 0.33 |
| Comparative Example 4-4C | H-1a | - | Compound 5 | 1.1 | 1.2 | 0.64, 0.34 |
| Comparative Example 4-5C | Compound 1 | H-4a | Compound 5 | 1.0 | 1.2 | 0.64, 0.34 |
| Example 4-10C | H-3b | H-3a | D-8 | 1.3 | 1.3 | 0.64, 0.34 |
| Example 4-11C | H-6b | H-3a | D-8 | 1.3 | 1.2 | 0.64, 0.34 |
| Example 4-12C | H-8b | H-3a | D-8 | 1.3 | 1.3 | 0.64, 0.34 |
| Example 4-13C | H-11b | H-3a | D-8 | 1.2 | 1.2 | 0.64, 0.34 |
| Comparative Example 4-6C | Compound 1 | - | Compound 7 | 1.0 | 1.0 | 0.62, 0.35 |
| Comparative Example 4-7C | Compound 1 | - | Compound 8 | 1.1 | 1.0 | 0.63, 0.34 |
| Comparative Example 4-8C | Compound 1 | - | D-7 | 1.1 | 1.1 | 0.64, 0.34 |
| Comparative Example 4-9C | H-1a | - | Compound 7 | 1.1 | 1.1 | 0.62, 0.35 |
| Comparative Example 4-10C | Compound 1 | H-4a | Compound 7 | 1.1 | 1.2 | 0.62, 0.35 |

Referring to Table 21, it was confirmed that the organic light-emitting devices of Examples 4-1C to 4-13C had improved efficiencies and lifespans compared to those of the organic light-emitting devices of Comparative Examples 4-1C to 4-10C.

Hereinafter, compounds used in manufacturing organic light-emitting devices manufactured in Examples 5-1C to 5-8C and Comparative Examples 5-1C to 5-10C are as follows:

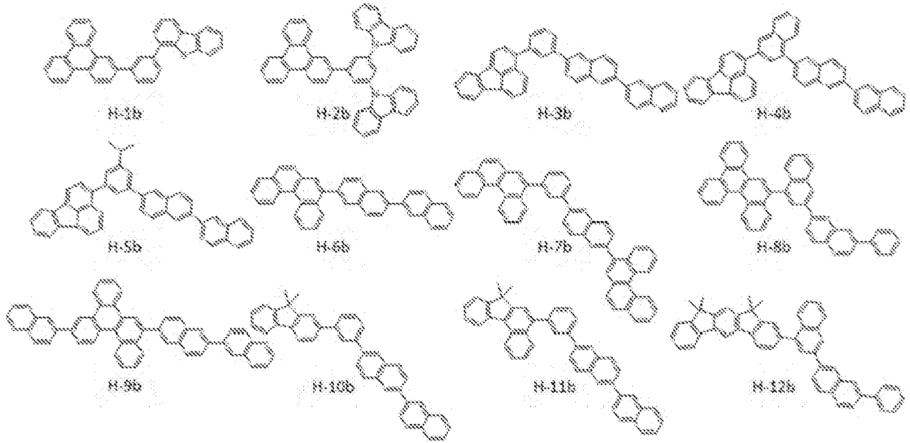

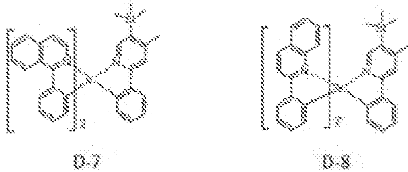

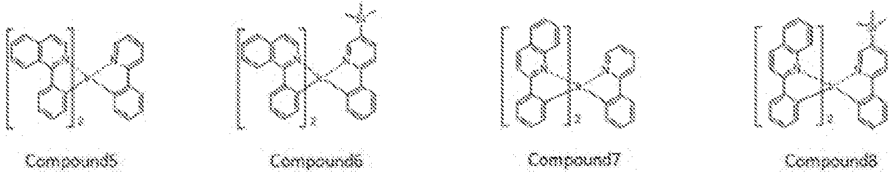

### Examples 5-1C to 5-8C and Comparative Examples 5-1C to 5-10C

### Glass/ITO(120)/HT(120)/Host1:Host2_10%:Dop_1%(30)/ET1(5)/ET2(25)/LiF(0.5)/Al(150)

Organic light-emitting devices were manufactured in the same manner as in Example 1-1C, except that in forming the EML, compounds listed in Table 22 were used as the first hosts, the second hosts, and the dopants, and the amounts of the dopants were changed to 1 v%.

**[Table 22]**

| | First host | Second host | Dopant |
|---|---|---|---|
| Example 5-1 | H-3b | H-2b | D-7 |
| Example 5-2 | H-6b | H-2b | D-7 |
| Example 5-3 | H-8b | H-2b | D-7 |
| Example 5-4 | H-11b | H-2b | D-7 |
| Comparative Example 5-1 | Compound 1 | - | Compound 5 |
| Comparative Example 5-2 | Compound 1 | - | Compound 6 |
| Comparative Example 5-3 | Compound 1 | - | D-7 |
| Comparative Example 5-4 | H-3b | - | Compound 5 |
| Comparative Example 5-5 | Compound 1 | H-2b | Compound 5 |
| Example 5-5 | H-3b | H-2b | D-8 |
| Example 5-6 | H-6b | H-2b | D-8 |
| Example 5-7 | H-8b | H-2b | D-8 |
| Example 5-8 | H-11b | H-2b | D-8 |
| Comparative Example 5-6 | Compound 1 | - | Compound 7 |
| Comparative Example 5-7 | Compound 1 | - | Compound 8 |
| Comparative Example 5-8 | Compound 1 | - | D-7 |
| Comparative Example 5-9 | H-3b | - | Compound 7 |
| Comparative Example 5-10 | Compound 1 | H-2b | Compound 7 |

### Evaluation Example 12

The organic light-emitting devices of Examples 5-1C to 5-8C and Comparative Examples 5-1C to 5-10Cwere subjected to measure and evaluate efficiencies (@ current density of 10 mA/cm²), lifespan data (@ 50 mA/cm²), and color coordinates, by using an IVL meter (PhotoResearch PR650, Keithley 238), and the results are shown in Table 23. In Table 23, the term "efficiency" refers to a relative efficiency, and the term "lifespan" refers to a relative lifespan, among the organic light-emitting devices.

**[Table 23]**

| | First host | Second host | Dopant | Efficiency | Lifespan | Color coordinates (x,y) |
|---|---|---|---|---|---|---|
| Example 5-1C | H-3b | H-2b | D-7 | 1.3 | 1.4 | 0.66, 0.33 |
| Example 5-2C | H-6b | H-2b | D-7 | 1.3 | 1.3 | 0.66, 0.33 |
| Example 5-3C | H-8b | H-2b | D-7 | 1.3 | 1.3 | 0.66, 0.34 |
| Example 5-4C | H-11b | H-2b | D-7 | 1.3 | 1.2 | 0.66, 0.33 |
| Comparative Example 5-1C | Compound 1 | - | Compound 5 | 1.0 | 1.0 | 0.64, 0.34 |
| Comparative Example 5-2C | Compound 1 | - | Compound 6 | 1.1 | 1.0 | 0.65, 0.34 |
| Comparative Example 5-3C | Compound 1 | - | D-7 | 1.1 | 1.1 | 0.66, 0.33 |
| Comparative Example 5-4C | H-3b | - | Compound 5 | 1.1 | 1.1 | 0.64, 0.34 |
| Comparative Example 5-5C | Compound 1 | H-2b | Compound 5 | 1.0 | 1.1 | 0.64, 0.34 |
| Example 5-5C | H-3b | H-2b | D-8 | 1.3 | 1.3 | 0.64, 0.34 |
| Example 5-6C | H-6b | H-2b | D-8 | 1.2 | 1.3 | 0.64, 0.34 |
| Example 5-7C | H-8b | H-2b | D-8 | 1.3 | 1.2 | 0.64, 0.34 |
| Example 5-8C | H-11b | H-2b | D-8 | 1.2 | 1.3 | 0.64, 0.34 |
| Comparative Example 5-6C | Compound 1 | - | Compound 7 | 1.0 | 1.0 | 0.62, 0.35 |
| Comparative Example 5-7C | Compound 1 | - | Compound 8 | 1.1 | 1.0 | 0.63, 0.34 |
| Comparative Example 5-8C | Compound 1 | - | D-7 | 1.1 | 1.1 | 0.64, 0.34 |
| Comparative Example 5-9C | H-3b | - | Compound 7 | 1.1 | 1.1 | 0.62, 0.35 |
| Comparative Example 5-10C | Compound 1 | H-2b | Compound 7 | 1.0 | 1.2 | 0.62, 0.35 |

Referring to Table 23, it was confirmed that the organic light-emitting devices of Examples 5-1C to 5-8C had improved efficiencies and lifespans compared to those of the organic light-emitting devices of Comparative Examples 5-1C to 5-10C.

According to one or more embodiments of the present inventive concept, organic light-emitting devices have excellent high efficiency long lifespan characteristics, show little change in the efficiency at an x-coordinate value of 0.21.

## Claims

1. An organic light-emitting device comprising:
a first electrode;
a second electrode; and
an organic layer disposed between the first electrode and the second electrode and comprising an emission layer,
wherein the emission layer comprises and a dopant and at least one of a first host and a second host,
the first host and second host are each independently represented by one of Formulae 1 and 2, wherein the first host and the second host are different from each other, and
the dopant is represented by Formula 7:
<Formula 1> Ar₁₁⁅(L₁₁)ₐ₁₁-(R₁₁)b₁₁]ₙ₁₁
<Formula 2> Ar₂₁⁅(L₂₁)a₂₁-(R₂₁)b₂₁]ₙ₂₁
<Formula 7> M(L₁)ₙ₇₁(L₂)ₙ₇₂ 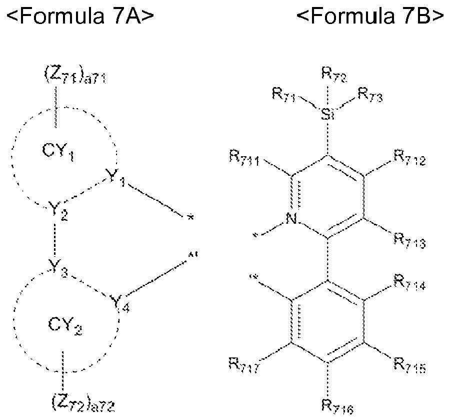
wherein, in Formulae above,
Ar₁₁ and Ar₂₁ are each independently selected from a substituted or unsubstituted C₄-C₃₀ pyrrolidine-based core and a substituted or unsubstituted C₇-C₃₀ condensed polycyclic-based core,
L₁₁ and L₂₁ are each independently selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
a11 and a21 are each independently selected from 0, 1, 2, and 3;
R₁₁ is a hole-transporting group, and R₂₁ is an electron-transporting group,
b11 and b21 are each independently selected from 1, 2, and 3,
n11 and n21 are each independently selected from 1, 2, 3, and 4,
M is selected from iridium (Ir), platinum (Pt), osmium (Os), titanium (Ti), zirconium (Zr), hafnium (Hf), europium (Eu), terbium (Tb), thulium (Tm), and rhodium (Rd),
L₁ is a ligand selected from Formula 7A above, and L₂ is a ligand selected from Formula 7B above, wherein L₁ are L₂ are different from each other,
n71 and n72 are each independently 1 or 2, a sum of n71 and n72 (n71+n72) is 2 or 3, and when n71 is 2, two L₁s are identical to or different from each other, and when n72 is 2, two L₂s are identical to or different from each other,
Y₁ to Y₄ are each independently C or N, wherein Y₁ and Y₂ are linked to each other via a single bond or a double bond, and Y₃ and Y₄ are linked to each other via a single bond or a double bond,
CY₁ and CY₂ are each independently selected from a C₅-C₆₀ cyclic group and a C₂-C₆₀ heterocyclic group, wherein CY₁ and CY₂ are optionally linked to each other via a single bond or a first linking group,
R₇₁ to R₇₃ are each independently selected from:
a C₁-C₁₀ alkyl group; and
a C₁-C₁₀ alkyl group substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, and a phosphoric acid group or a salt thereof,
Z₇₁, Z₇₂, and R₇₁₁ to R₇₁₇ are each independently selected from a hydrogen, a deuterium, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, - N(Q₁)(Q₂), -Si(Q₃)(Q₄)(Q₅), and -B(Q₆)(Q₇), wherein R₇₁₂ is not a hydrogen, and two adjacent substituents selected from R₇₁₄ to R₇₁₇ are optionally linked to each other to form a condensed ring,
a71 and a72 are each independently selected from integers of 1 to 5, and when a71 is 2 or more, a plurality of Z₇₁s are identical to or different from each other, and when a72 is 2 or more, a plurality of Z₇₂s are identical to or different from each other,
* and *' each independently indicate a binding site to M of Formula 1, and
at least one substituent of the substituted C₄-C₃₀ pyrrolidine-based core, the substituted C₇-C₃₀ condensed polycyclic-based core, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the subtstituted divalent non-aromatic condensed polycyclic group, the subtstituted divalent non-aromatic condensed heteropolycyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted or unsubstituted monovalent non-aromatic condensed heteropolycyclic group is selected from:
a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), and -B(Q₁₆)(Q₁₇),
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), and -B(Q₂₆)(Q₂₇); and
-N(Q₃₁)(Q₃₂), -Si(Q₃₃)(Q₃₄)(Q₃₅), and -B(Q₃₆)(Q₃₇),
wherein Q₁ to Q₇, Q₁₁ to Q₁₇, Q₂₁ to Q₂₇, and Q₃₁ to Q₃₇ are each independently selected from a hydrogen, a C₁-C₆₀ alkyl group, a C₁-C₆₀ alkoxy group, a C₆-C₆₀ aryl group, a C₁-C₆₀ heteroaryl group, a monovalent non-aromatic condensed polycyclic group, and a monovalent non-aromatic condensed heteropolycyclic group.

2. The organic light-emitting device of claim 1, wherein Ar₁₁ is a group represented by one of Formulae 8A-1 to 8A-4, 8B-1 to 8B-19, and 8C-1 to 8C-19, and
Ar₂₁ is a group represented by one of Formulae 9A-1 to 9A-4, 9B-1 to 9B-19, and 9C-1 to 9C-19: 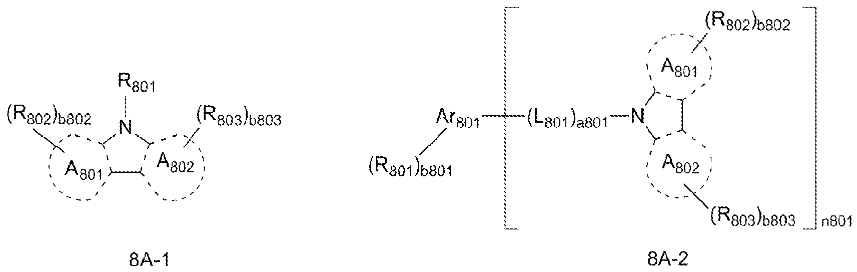 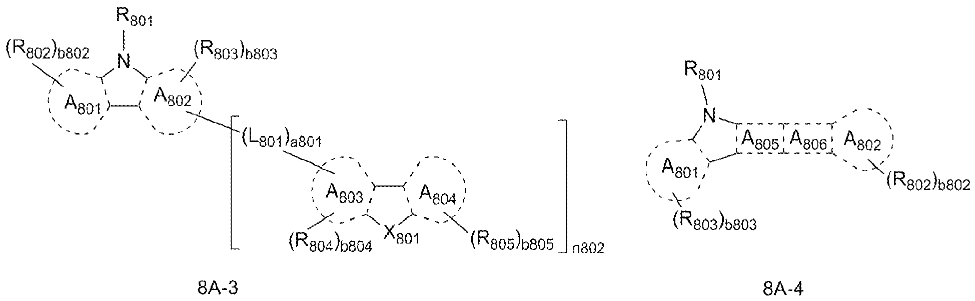 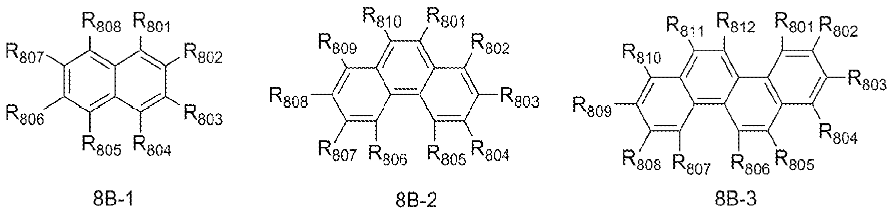 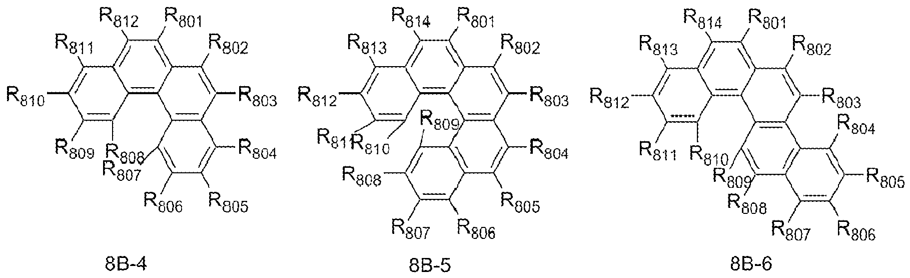 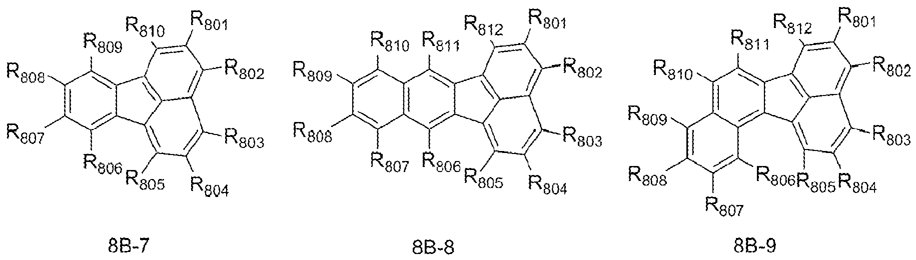 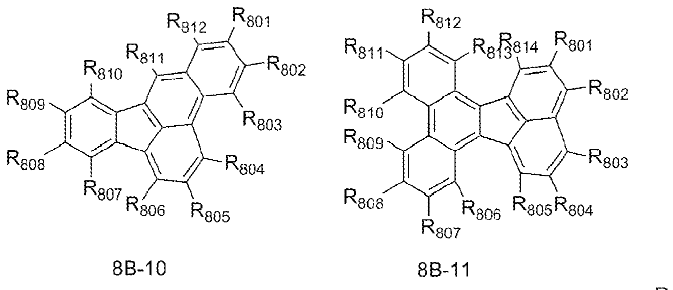 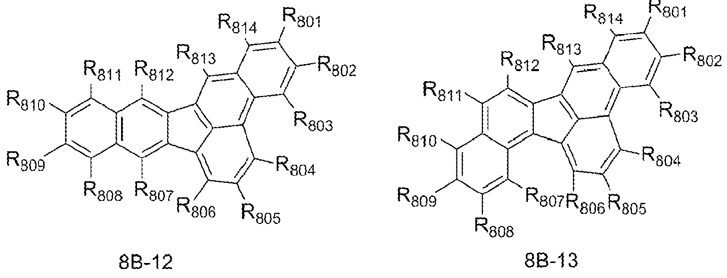 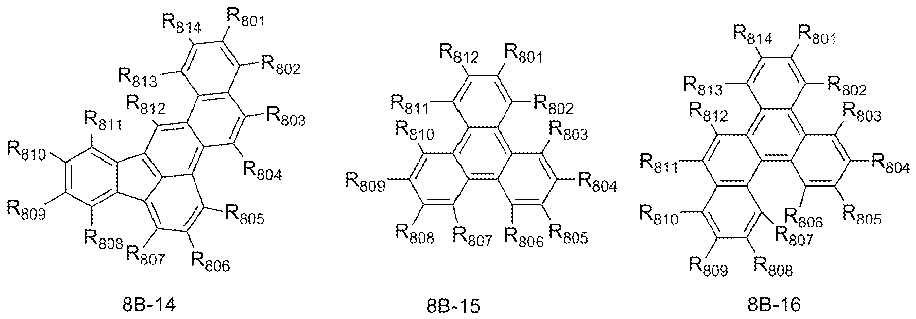 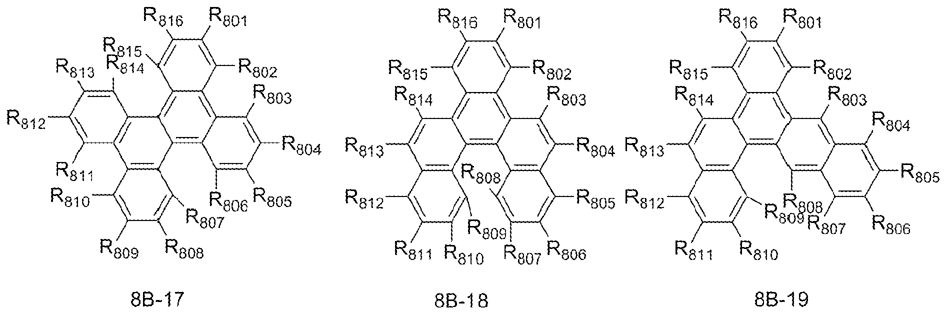 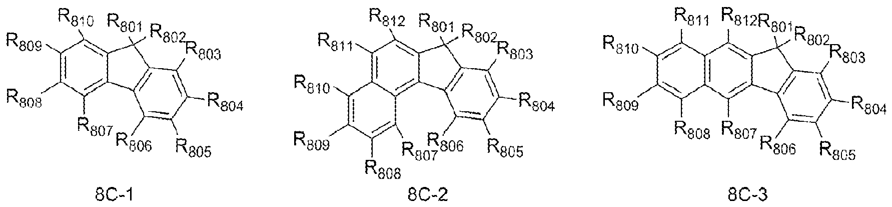 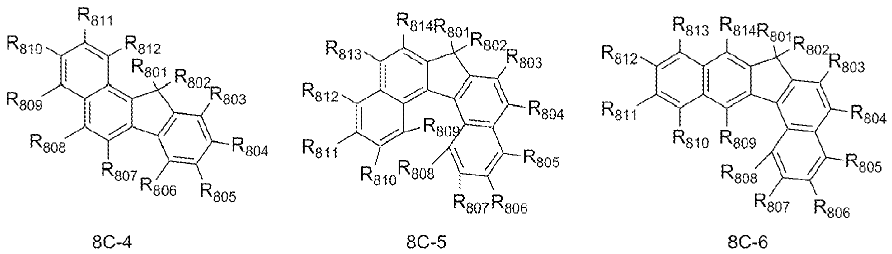 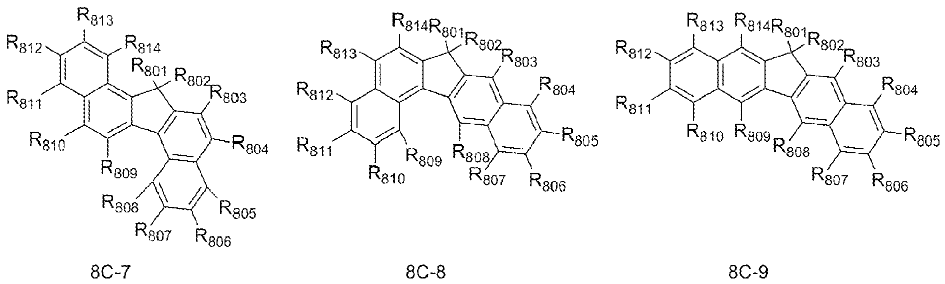 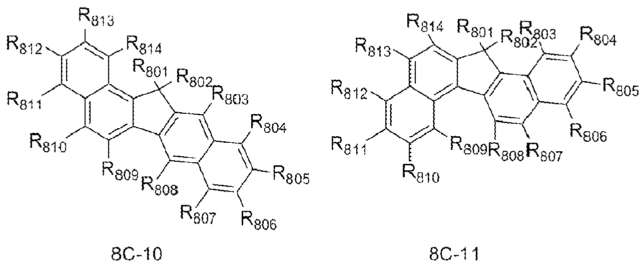 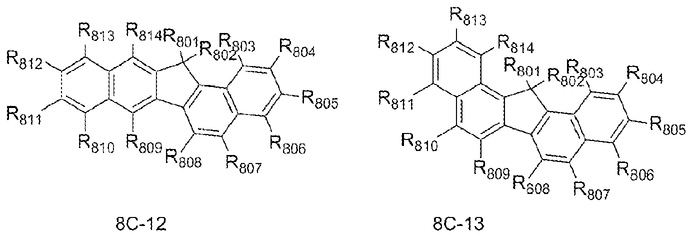 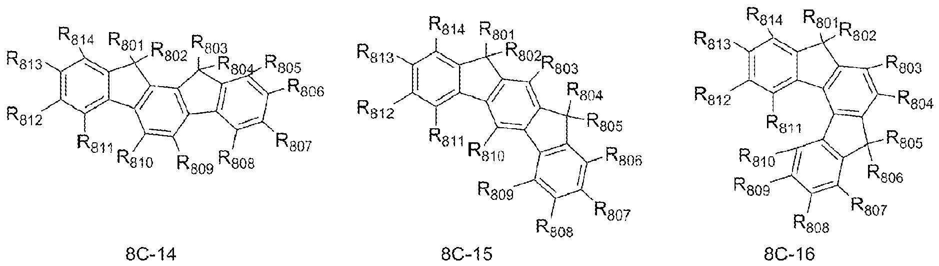 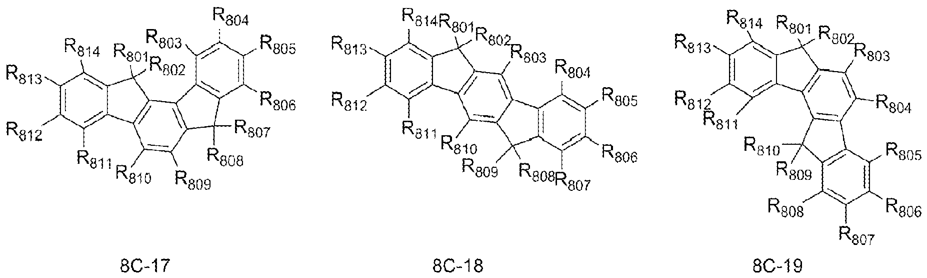 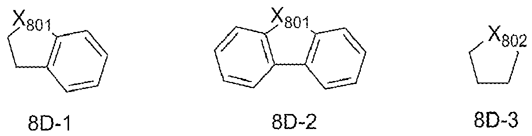 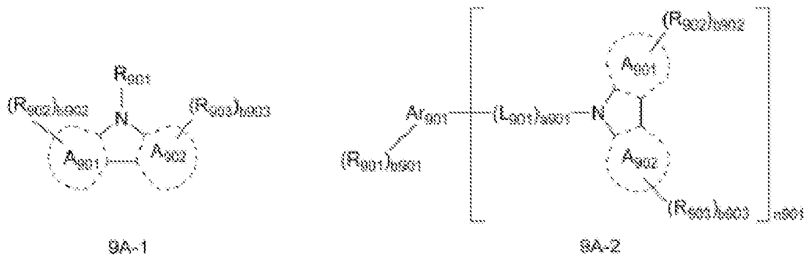 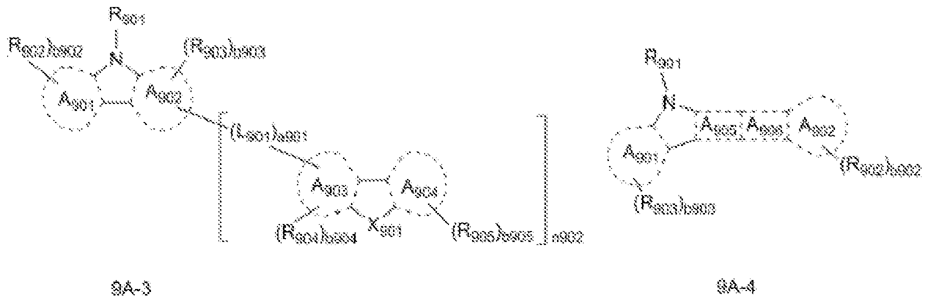 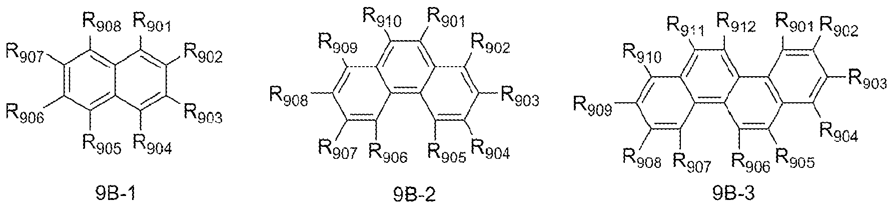 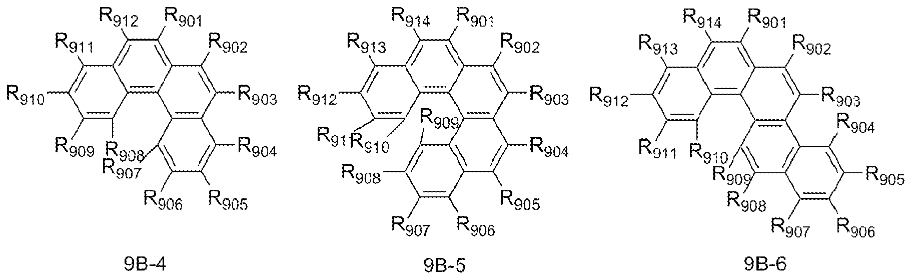 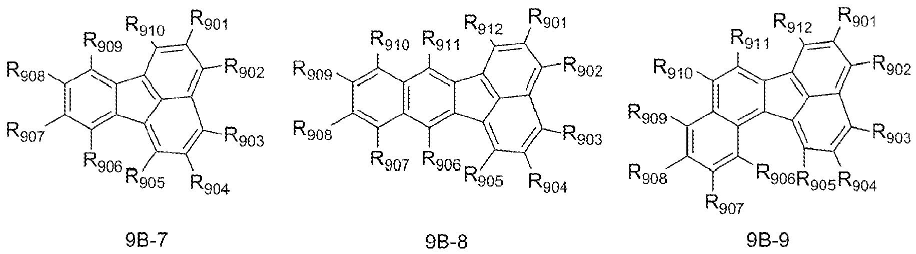 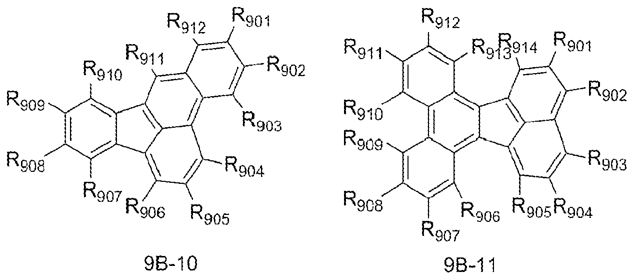 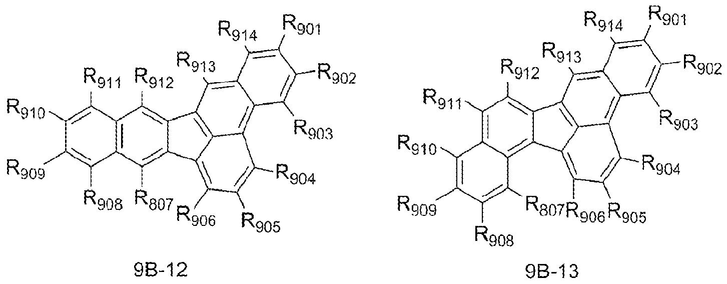 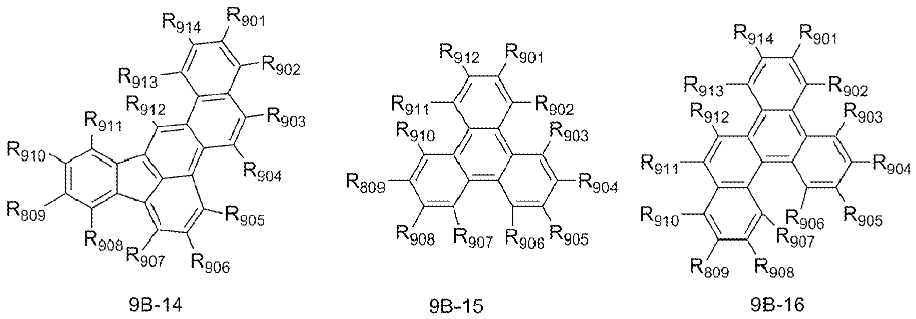 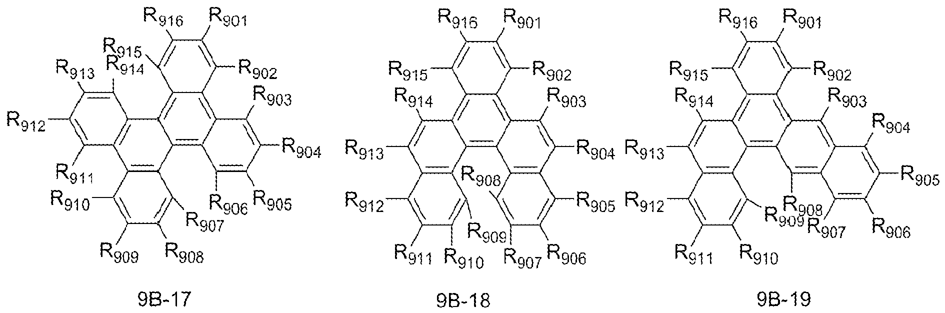 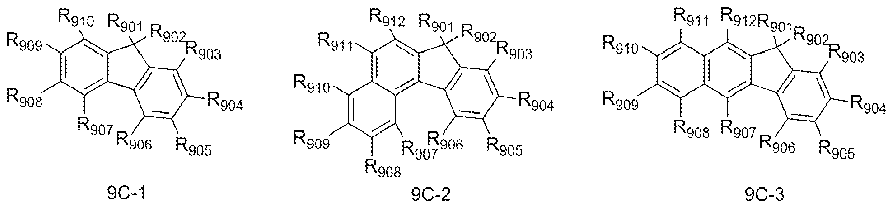 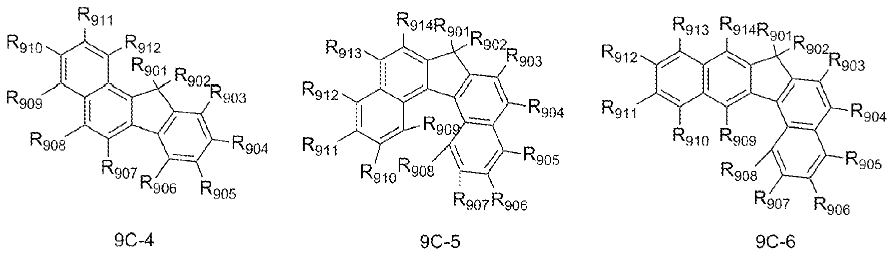 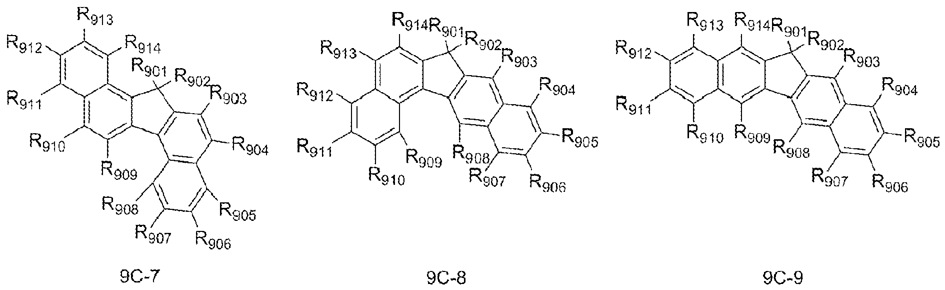 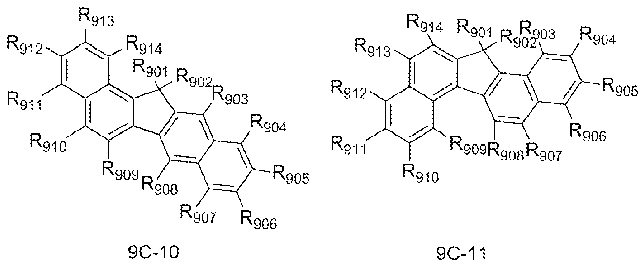 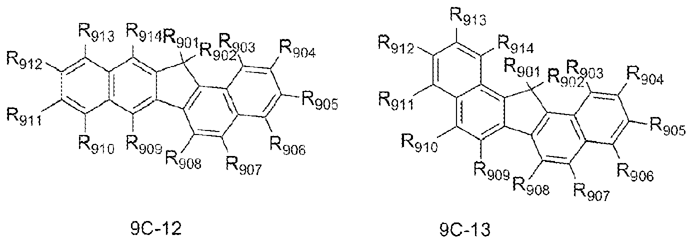 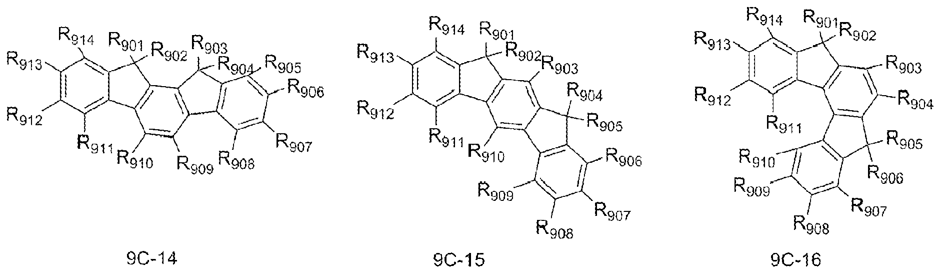 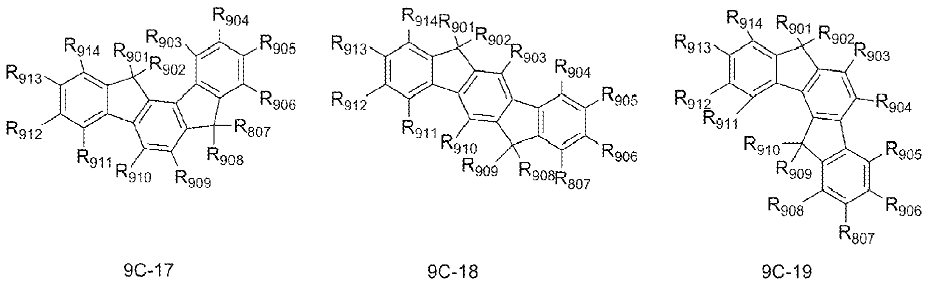 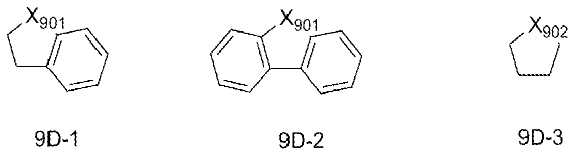 In Formulae above,
Ar₈₀₁ and Ar₉₀₁ are each independently selected from a substituted or unsubstituted C₃-C₁₀ cycloalkane group, a substituted or unsubstituted C₁-C₁₀ heterocycloalkane group, a substituted or unsubstituted C₃-C₁₀ cycloalkene group, a substituted or unsubstituted C₁-C₁₀ heterocycloalkene group, a substituted or unsubstituted C₆-C₆₀ arene group, a substituted or unsubstituted C₁-C₆₀ heteroarene group, a substituted or unsubstituted non-aromatic condensed polycyclic group, and a substituted or unsubstituted non-aromatic condensed heteropolycyclic group,
L₈₀₁ and L₉₀₁ are each independently selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
a801 and a901 are each independently selected from 0, 1, 2, and 3,
A₈₀₁ to A₈₀₄ are each independently selected from a benzene, a naphthalene, a pyridine, a pyrimidine, a pyrazine, a quinoline, an isoquinoline, a 2,6-naphthyridine, a 1,8-naphthyridine, a 1,5-naphthyridine, a 1,6-naphthyridine, a 1,7-naphthyridine, a 2,7-naphthyridine, a quinoxaline, a phthalazine, a quinazoline, a group represented by Formula 8D-1 above, and a group represented by Formula 8D-2 above, and A₉₀₁ to A₉₀₄ are each independently selected from a benzene, a naphthalene, a pyridine, a pyrimidine, a pyrazine, a quinoline, an isoquinoline, a 2,6-naphthyridine, a 1,8-naphthyridine, a 1,5-naphthyridine, a 1,6-naphthyridine, a 1,7-naphthyridine, a 2,7-naphthyridine, a quinoxaline, a phthalazine, a quinazoline, a group represented by Formula 9D-1 above, and a group represented by Formula 9D-2 above,
A₈₀₅ and A₈₀₅ are each independently selected from a benzene and a naphthalene,
A₈₀₆ is represented by Formula 8D-3 above, and A₉₀₆ is represented by Formula 9D-3 above,
X₈₀₁ and X₈₀₂ are each independently selected from N(R₈₀₆), O, S, C(R₈₀₆)(R₈₀₇), Si(R₈₀₆)(R₈₀₇), B(R₈₀₆), P(R₈₀₆), and P(=O)(R₈₀₆), and X₉₀₁ and X₉₀₂ are each independently selected from N(R₉₀₆), O, S, C(R₉₀₆)(R₉₀₇), Si(R₉₀₆)(R₉₀₇), B(R₉₀₆), P(R₉₀₆), and P(=O)(R₉₀₆),
R₈₀₁ to R₈₁₆ are each independently selected from *-[(L₁₁)ₐ₁₁-(R₁₁)_{b11}], a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, wherein R₈₀₁ to R₈₁₆ in the number of n11 may be *-[(L₁₁)ₐ₁₁-(R₁₁)_{b11}],
R₉₀₁ to R₉₁₆ are each independently selected from *-[(L₂₁)ₐ₂₁-(R₂₁)_{b21}], a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, wherein R₉₀₁ to R₉₁₆ in the number of n21 is *-[(L₂₁)ₐ₂₁-(R₂₁)_{b21}],
b801 to b805 and b901 to b905 are each independently selected from 1, 2, 3, and 4,
n801 and n901 are each independently selected from 2, 3, and 4,
n802 and n902 are each independently selected from 1, 2, and 3, and
at least one substituent of the substituted C₃-C₁₀ cycloalkane group, the substituted C₁-C₁₀ heterocycloalkane group, the substituted C₃-C₁₀ cycloalkene group, the substituted C₁-C₁₀ heterocycloalkene group, the substituted C₆-C₆₀ arene group, the substituted C₁-C₆₀ heteroarene group, the substituted non-aromatic condensed polycyclic group, the substituted non-aromatic condensed heteropolycyclic group, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the subtstituted divalent non-aromatic condensed polycyclic group, the subtstituted divalent non-aromatic condensed heteropolycyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted or unsubstituted monovalent non-aromatic condensed heteropolycyclic group may be selected from:
a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with a t least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), and -B(Q₁₆)(Q₁₇);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), and -B(Q₂₆)(Q₂₇); and
-N(Q₃₁)(Q₃₂), -Si(Q₃₃)(Q₃₄)(Q₃₅), and -B(Q₃₆)(Q₃₇),
wherein Q₁₁ to Q₁₇, Q₂₁ to Q₂₇, and Q₃₁ to Q₃₇ are each independently selected from a hydrogen, a C₁-C₆₀ alkyl group, a C₁-C₆₀ alkoxy group, a C₆-C₆₀ aryl group, a C₁-C₆₀ heteroaryl group, a monovalent non-aromatic condensed polycyclic group, and a monovalent non-aromatic condensed heteropolycyclic group.

3. The organic light-emitting device of claim 1, wherein L₁₁ and L₂₁ are each independently selected from a phenylene group, a pentalenylene group, an indenylene group, a naphthylene group, an azulenylene group, a heptalenylene group, an indacenylene group, an acenaphthylene group, a fluorenylene group, a spiro-fluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a pyrrolylene group, a thiophenylene group, a furanylene group, an imidazolylene group, a pyrazolylene group, a thiazolylene group, an isothiazolylene group, an oxazolylene group, an isoxazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzimidazolylene group, a benzofuranylene group, a benzothiophenylene group, an isobenzothiazolylene group, a benzoxazolylene group, an isobenzoxazolylene group, a triazolylene group, a tetrazolylene group, an oxadiazolylene group, a triazinylene group, a dibenzofuranylene group, a dibenzothiophenylene group, a benzocarbazolylene group, and a dibenzocarbazolylene group; and
a phenylene group, a pentalenylene group, an indenylene group, a naphthylene group, an azulenylene group, a heptalenylene group, an indacenylene group, an acenaphthylene group, a fluorenylene group, a spiro-fluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a pyrrolylene group, a thiophenylene group, a furanylene group, an imidazolylene group, a pyrazolylene group, a thiazolylene group, an isothiazolylene group, an oxazolylene group, an isoxazolylene group, a pyridinylene group, a pyrazinylene group, a pyrimidinylene group, a pyridazinylene group, an isoindolylene group, an indolylene group, an indazolylene group, a purinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a carbazolylene group, a phenanthridinylene group, an acridinylene group, a phenanthrolinylene group, a phenazinylene group, a benzimidazolylene group, a benzofuranylene group, a benzothiophenylene group, an isobenzothiazolylene group, a benzoxazolylene group, an isobenzoxazolylene group, a triazolylene group, a tetrazolylene group, an oxadiazolylene group, a triazinylene group, a dibenzofuranylene group, a dibenzothiophenylene group, a benzocarbazolylene group, and a dibenzocarbazolylene group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a pentalenyl group, an indenyl group, a naphthyl group, an azulenyl group, a heptalenyl group, an indacenyl group, an acenaphthyl group, a fluorenyl group, a spiro-fluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, a thiadiazolyl group, and an imidazopyridinyl group.

4. The organic light-emitting device of claim 1, wherein R₁₁ is selected from a phenyl group, a naphthyl group, a fluorenyl group, a benzofluorenyl group, a dibenzofluorenyl group, a phenanthrenyl group, an anthracenyl group, a triphenylenyl group, a benzofuranyl group, a benzothiophenyl group, a dibenzofuranyl group, a dibenzothiophenyl group, a carbazolyl group, and -N(R₅₆)(R₅₇);
a phenyl group, a naphthyl group, a fluorenyl group, a benzofluorenyl group, a dibenzofluorenyl group, a phenanthrenyl group, an anthracenyl group, a triphenylenyl group, a benzofuranyl group, a benzothiophenyl group, a dibenzofuranyl group, a dibenzothiophenyl group, and a carbazolyl group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₆-C₂₀ aryl group, a C₁-C₂₀ heteroaryl group, a monovalent non-aromatic condensed polycyclic group, a monovalent non-aromatic condensed heteropolycyclic group, -N(Q₄₁)(Q₄₂), -Si(Q₄₃)(Q₄₄)(Q₄₅), and -B(Q₄₆)(Q₄₇); and
a phenyl group, a naphthyl group, a fluorenyl group, a benzofluorenyl group, a dibenzofluorenyl group, a phenanthrenyl group, an anthracenyl group, a triphenylenyl group, a benzofuranyl group, a benzothiophenyl group, a dibenzofuranyl group, a dibenzothiophenyl group, and a carbazolyl group, each substituted with at least one of a C₆-C₂₀ aryl group, a C₁-C₂₀ heteroaryl group, a monovalent non-aromatic condensed polycyclic group, and a monovalent non-aromatic condensed heteropolycyclic group that are each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group and C₁-C₂₀ alkoxy group;
R₅₆ and R₅₇ each independently a C₁-C₂₀ alkyl group, a C₆-C₂₀ aryl group, a C₁-C₂₀ heteroaryl group, a monovalent non-aromatic condensed polycyclic group, and a monovalent non-aromatic condensed heteropolycyclic group; and
a C₁-C₂₀ alkyl group, a C₆-C₂₀ aryl group, a C₁-C₂₀ heteroaryl group, a monovalent non-aromatic condensed polycyclic group, and a monovalent non-aromatic condensed heteropolycyclic group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₆-C₂₀ aryl group, a C₁-C₂₀ heteroaryl group, a monovalent non-aromatic condensed polycyclic group, and a monovalent non-aromatic condensed heteropolycyclic group,,
wherein Q₄₁ to Q₄₇ are each independently selected from a C₁-C₂₀ alkyl group, a C₆-C₂₀ aryl group, a C₁-C₂₀ heteroaryl group, a monovalent non-aromatic condensed polycyclic group, and a monovalent non-aromatic condensed heteropolycyclic group.

5. The organic light-emitting device of claim 1, wherein R₂₁ is selected from:
a pyrrolyl group, an indolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a quinolinyl group, an isoquinolinyl group, a benzoquinolinyl group, a naphthyridinyl group, a quinoxalinyl group, a quinazolinyl group, a phenanthridinyl group, an acridinyl group, a phenanthrolinyl group, a phenazinyl group, a benzothiazolyl group, a benzoxazolyl group, a benzimidazolyl group, a triazolyl group, a triazinyl group, an imidazopyridinyl group, an imidazopyrimidinyl group, a pyridobenzofuranyl group, a pyrimidobenzofuranyl group, a pyridobenzothiophenyl group, and a pyrimidobenzothiophenyl group;
a pyrrolyl group, an indolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a quinolinyl group, an isoquinolinyl group, a benzoquinolinyl group, a naphthyridinyl group, a quinoxalinyl group, a quinazolinyl group, a phenanthridinyl group, an acridinyl group, a phenanthrolinyl group, a phenazinyl group, a benzothiazolyl group, a benzoxazolyl group, a benzimidazolyl group, a triazolyl group, a triazinyl group, an imidazopyridinyl group, an imidazopyrimidinyl group, a pyridobenzofuranyl group, a pyrimidobenzofuranyl group, a pyridobenzothiophenyl group, and a pyrimidobenzothiophenyl group, each substituted with at least one of a deuterium, -F, -CI, - Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₆-C₂₀ aryl group, a C₁-C₂₀ heteroaryl group, a monovalent non-aromatic condensed polycyclic group, a monovalent non-aromatic condensed heteropolycyclic group, -N(Q₄₁)(Q₄₂), -Si(Q₄₃)(Q₄₄)(Q₄₅), and -B(Q₄₆)(Q₄₇); and
a pyrrolyl group, an indolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a quinolinyl group, an isoquinolinyl group, a benzoquinolinyl group, a naphthyridinyl group, a quinoxalinyl group, a quinazolinyl group, a phenanthridinyl group, an acridinyl group, a phenanthrolinyl group, a phenazinyl group, a benzothiazolyl group, a benzoxazolyl group, a benzimidazolyl group, a triazolyl group, a triazinyl group, an imidazopyridinyl group, an imidazopyrimidinyl group, a pyridobenzofuranyl group, a pyrimidobenzofuranyl group, a pyridobenzothiophenyl group, and a pyrimidobenzothiophenyl group, each substituted with at least one of a C₆-C₂₀ aryl group, a C₁-C₂₀ heteroaryl group, a monovalent non-aromatic condensed polycyclic group, and a monovalent non-aromatic condensed heteropolycyclic group that are each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, and a C₁-C₂₀ alkoxy group,
wherein Q₄₁ to Q₄₇ are each independently selected from a C₁-C₂₀ alkyl group, a C₆-C₂₀ aryl group, a C₁-C₂₀ heteroaryl group, a monovalent non-aromatic condensed polycyclic group, and a monovalent non-aromatic condensed heteropolycyclic group.

6. The organic light-emitting device of claim 1, wherein M is iridum (Ir).

7. The organic light-emitting device of claim 1, wherein n71 and n72 are each independently 1 or 2, and a sum of n71 and n72 (n71+n72) is 3, organic light-emitting device.

8. The organic light-emitting device of claim 1, wherein CY₁ and CY₂ are each independently selected from a benzene, a naphthalene, a fluorene, a spiro-fluorene, an indene, a furan, a thiophene, a carbazole, a benzofuran, a benzothiophene, a dibenzofuran, a dibenzothiophene, a pyrrole, an imidazole, a pyrazole, a thiazole, an isothiazole, an oxazole, an isoxazole, a triazole, a pyridine, a pyrazine, a pyrimidine, a quinoline, an isoquinoline, a benzoquinoline, a quinoxaline, a quinazoline, naphthyridine, an indole, a benzimidazole, a benzoxazole, an isobenzoxazole, an oxadiazole, and a triazine.

9. The organic light-emitting device of claim 1, wherein CY₁ is selected from a pyrrole, an imidazole, a pyrazole, a triazole, a pyridine, a pyrimidine, a pyrazine, a triazine, a quinoline, an isoquinoline, and, an oxadiazole, and
CY₂ is selected from a benzene, a naphthalene, a fluorene, a carbazole, a furan, a thiophene, a benzofuran, a benzothiophene, a dibenzofuran, a dibenzothiophene, an indole, a pyridine, a pyrimidine, a pyrazine, an oxadiazole, and a triazine.

10. The organic light-emitting device of claim 1, wherein R₇₁ to R₇₃ are each independently selected from:
a methyl group, an ethyl group, an n-propyl group, an iso-propyl group, an n-butyl group, an iso-butyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, and a tert-decyl group; and
a methyl group, an ethyl group, an n-propyl group, an iso-propyl group, an n-butyl group, an iso-butyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, and a tert-decyl group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, and a phosphoric acid group or a salt thereof.

11. The organic light-emitting device of claim 1, wherein Z₇₁, Z₇₂, and R₇₁₁ to R₇₁₇ are each independently selected from:
a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a C₁-C₂₀ alkyl group, and a C₁-C₂₀ alkoxy group;
a C₁-C₂₀ alkyl group and a C₁-C₂₀ alkoxy group, each substituted with at least one of a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a phenyl group, a naphthyl group, a pyridinyl group, and a pyrimidinyl group;
a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group; and
a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group, each substituted with at least one of a deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or or a salt thereof, a phosphoric acid or a salt thereof, a C₁-C₂₀ alkyl group, a C₁-C₂₀ alkoxy group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group,
wherein R₇₁₂ is not a hydrogen.

12. The organic light-emitting device of claim 1, wherein the first host is represented by Formula 1 above, and the second host is represented by Formula 2 above.
